(19) Europäisches Patentamt
European Patent Office
Office européen des brevets

(11) EP 4 737 449 A1

(12) **EUROPEAN PATENT APPLICATION**
published in accordance with Art. 153(4) EPC

(43) Date of publication:
**06.05.2026 Bulletin 2026/19**

(21) Application number: **24830815.7**

(22) Date of filing: **26.06.2024**

(51) International Patent Classification (IPC):
***C07D 401/12*** *(2006.01)* ***C07D 471/04*** *(2006.01)*
***A61K 31/454*** *(2006.01)* ***A61K 31/4545*** *(2006.01)*
***A61P 35/00*** *(2006.01)* ***A61P 35/02*** *(2006.01)*

(52) Cooperative Patent Classification (CPC):
**A61K 31/454; A61K 31/4545; A61P 35/00; A61P 35/02; C07D 401/12; C07D 471/04**

(86) International application number:
**PCT/CN2024/101613**

(87) International publication number:
**WO 2025/002177 (02.01.2025 Gazette 2025/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.06.2023 CN 202310767412**
**03.08.2023 CN 202310972386**
**08.09.2023 CN 202311158861**
**23.10.2023 CN 202311378644**
**22.11.2023 CN 202311566589**
**06.02.2024 CN 202410171815**

(71) Applicant: **Changchun Genescience Pharmaceutical Co., Ltd.**
**Changchun, Jilin 130012 (CN)**

(72) Inventors:
- **GENG, Kaijun**
  **Changchun, Jilin 130012 (CN)**
- **LU, Biao**
  **Changchun, Jilin 130012 (CN)**
- **YANG, Fanglong**
  **Changchun, Jilin 130012 (CN)**
- **WANG, Siqin**
  **Changchun, Jilin 130012 (CN)**
- **JIN, Lei**
  **Changchun, Jilin 130012 (CN)**

(74) Representative: **Gille Hrabal Partnerschaftsgesellschaft mbB**
**Patentanwälte**
**Brucknerstraße 20**
**40593 Düsseldorf (DE)**

(54) **P53-Y220C SELECTIVE SMALL-MOLECULAR REACTIVATOR COMPOUND, PHARMACEUTICAL COMPOSITION AND USE THEREOF**

(57) Provided in the present invention are compounds shown as formula (I) and racemates, stereoisomers, tautomers, nitrogen oxides, solvates, polymorphs, metabolites, esters, prodrugs or pharmaceutically acceptable salts thereof, which have good p53-Y220C mutant activation effect, can be used for treating tumor diseases containing p53-Y220C mutant proteins, and can be used for preparing drugs for such disorders or diseases.

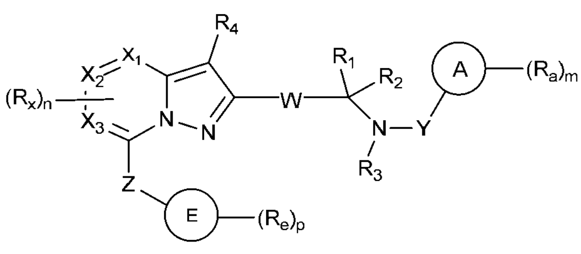

(I)

EP 4 737 449 A1

## Description

The present invention claims priority to prior applications including Patent Application No. 202310767412.7 filed with the China National Intellectual Property Administration on June 27, 2023, entitled "p53-Y220C Selective Small-Molecule Reactivator Compound, Pharmaceutical Composition and Use Thereof"; Patent Application No. 202310972386.1 filed with the China National Intellectual Property Administration on August 3, 2023, entitled "p53-Y220C Selective Small-Molecule Reactivator Compound, Pharmaceutical Composition and Use Thereof"; Patent Application No. 202311158861.8 filed with the China National Intellectual Property Administration on September 8, 2023, entitled "p53-Y220C Selective Small-Molecule Reactivator Compound, Pharmaceutical Composition and Use Thereof"; Patent Application No. 202311378644.X filed with the China National Intellectual Property Administration on October 23, 2023, entitled "p53-Y220C Selective Small-Molecule Reactivator Compound, Pharmaceutical Composition and Use Thereof"; Patent Application No. 202311566589.7 filed with the China National Intellectual Property Administration on November 22, 2023, entitled "p53-Y220C Selective Small-Molecule Reactivator Compound, Pharmaceutical Composition and Use Thereof"; and Patent Application No. 202410171815.X filed with the China National Intellectual Property Administration on February 6, 2024, entitled "p53-Y220C Selective Small-Molecule Reactivator Compound, Pharmaceutical Composition and Use Thereof". The above prior applications are incorporated herein by reference in their entirety.

## TECHNICAL FIELD

The present invention belongs to the field of pharmaceutical compounds, and specifically, relates to p53-Y220C selective small-molecule reactivator compounds, pharmaceutical compositions and uses thereof.

## BACKGROUND

Tumor is a set of related diseases characterized by uncontrolled proliferation of tumor cells that may metastasize throughout the body. Tumor cell proliferation is dependent on the proto-oncogene and the cancer suppressor gene, and gene mutation can lead to abnormal activation or activity inhibition of these two genes, which further promote uncontrolled cell division. As a well-known cancer suppressor gene, TP53 is known as the "Genome Guardian". TP53 is a member of the p53-like transcription factor family which includes three members, TP53, TP63 and TP73 genes encoding p53, p63 and p73 proteins, respectively. They have similar structures: their DNA binding domains are almost identical and can all bind to similar DNA-specific sequences, regulating the transcription of the same gene and some different genes; their C-terminal domains differ in size, sequence and function, regulating DNA binding and transcription and mediating protein interactions; their N-terminal sequences encode at least two different transcription activation domains. Similar to p53, p63 is also a key transcription factor that responds to DNA damage by inducing apoptosis, acts on the skull, face, limbs, and central nervous system, and participates in the generation and regeneration of squamous cell epithelium throughout the body. The production of ciliary epithelial cells requires p73, which acts on male germ cells, the immune system, the hearing system, the trachea, the lungs, the central nervous system, etc.

The p53 cancer suppressor factor is mainly distributed in the nucleoplasm of cells. As a sensor of cell stress, it can respond to a variety of cell stresses, including ultraviolet radiation, hypoxia, oncogene activation and DNA damage. Upon activation, p53 binds to specific DNA sequences in the form of a tetramer, mediates downstream gene transcription, and inhibits cancer progression through a variety of mechanisms, such as regulation of cell cycle, apoptosis, aging, stem cell differentiation, metabolism (lowering glycosynthesis), ROS and mitochondria, and DNA damage repair. p53 can activate proteins involved in the above pathways, including, for example, Fas/Apol, KILLER/DR5, Bax, Puma, Noxa, Bid, caspase-3, caspase-6, caspase-7, caspase-8, caspase-9 and p21. In addition, p53 can also inhibit the transcription of a variety of genes, including c-MYC, Cyclin B, VEGF, RAD51 and hTERT.

Due to its key role in tumor suppression, TP53 gene is the most frequently mutated gene in human cancers, with p53 mutations present in nearly 50% of malignant tumors. Contrary to the mutations of other cancer suppressor genes such as RB1, APC, PTEN, most TP53 mutations are missense mutations in up to 75% of cases. Most missense mutations are located in the DNA binding domain of p53, resulting in abnormal folding of the protein sequence required for DNA recognition and binding. p53 may be mutated in many amino acid sites, such as Vall43, Hisl68, Argl75, Tyr220, Gly245, Arg248, Arg299, Phe270, Arg273 and Arg282. Accordingly, p53 mutations that can abrogate wild-type p53 activity include, for example, R175H, Y220C, G245S, R248Q, R248W, R273H and R282H. These p53 mutations can both distort the structure and destabilize the thermodynamics of DNA binding sites. Mutated TP53 loses the cancer suppressor transcriptional activity of wild-type p53 through loss of homozygosity; exhibits dominant negative effects through loss of heterozygosity, and gain of function such as increased binding to p63 and p73, thus promoting tumor occurrence and development. Therefore, mutant p53 proteins can be viewed as a group of heterogeneous proteins with varying degrees of loss of normal tumor suppressor function and gain of oncogenic properties (GOF).

Although the activity of different p53 mutants varies, TP53 missense mutants can be viewed as proto-oncogenes that promote tumor migration, drug resistance leading to a poor prognosis, and thus becoming therapeutic targets for drug development. Among others, the oncogenic p53 Y220C mutant exhibits a typical structure particularly suitable for the development of small-molecule stabilizers. It is the ninth most common p53 missense mutation found in cancer, with approximately 100,000 new cancer cases occurring worldwide each year. Mutation of Tyr220 to Cys results in a narrow hydrophobic cleft on the surface of the p53 DBD, which reduces its thermal stability to about 4 kcal/mol. Wild-type p53 is moderately stable and melts and denatures at 44°C, whereas the Y220C mutant protein rapidly unfolds from its folded state and denatures at physiological body temperature, effectively eliminating the cancer suppressor signal of wild-type p53 and driving tumorigenesis. Importantly, the hydrophobic cleft caused by the Y220C mutation is away from the surface of the p53 protein involved in DNA recognition or protein interactions, allowing the development of small-molecule drugs without interfering with its binding to the natural DNA substrate. The Y220C mutant is a temperature-sensitive mutant that binds to DNA at lower temperatures and denatures at body temperature. When a small-molecule compound selectively binds to p53-Y220C mutant, the compound can stabilize the Y220C mutant to reduce the possibility of denaturation of the p53 protein at body temperature, convert p53 from an unfolded state to a folded state, and restore the wild-type conformation and transcriptional activity of p53.

Studies have shown that several small molecules can bind to the hydrophobic cleft of the Y220C mutant, such as PK083, PK7088, PK5196, PC14586, but the effective concentration is still relatively high, so whether this is due to an off-target effect cannot be excluded. Therefore, there is still a need to develop better small molecules that can bind to the Y220C mutant more specifically, restore the structure and transcriptional activity of the wild-type p53 better, and inhibit tumors more effectively.

## SUMMARY OF THE INVENTION

To solve the problems found in the prior art, the present invention provides a compound shown as formula (I), and a racemate, a stereoisomer, a tautomer, an isotope-labeled counterpart, a nitrogen oxide, a solvate, a polymorph, a metabolite, an ester, a prodrug or a pharmaceutically acceptable salt thereof:

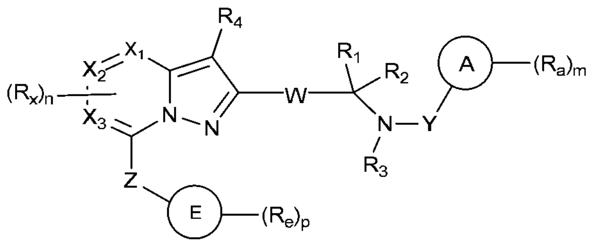

Formula (I)

wherein $R_1$ and $R_2$ are the same or different, and are independently selected from H, halogen, $C_{1-12}$ alkyl, or $C_{1-12}$ alkoxy;

$R_3$ is selected from H, or $C_{1-12}$ alkyl;

$R_4$ is selected from H, halogen, cyano, $C_{1-12}$ alkyl, $C_{2-12}$ alkenyl, $C_{2-12}$ alkynyl, $C_{1-12}$ alkoxy, $C_{3-12}$ cycloalkyl, halo-$C_{1-12}$ alkyl, halo-$C_{1-12}$ alkoxy, halo-$C_{3-12}$ cycloalkyl, cyano-$C_{1-12}$ alkyl, or cyano-$C_{1-12}$ alkoxy;

W is selected from $C_{2-6}$ alkenylene, $C_{2-6}$ alkynylene, $C_{3-14}$ cycloalkylene, $C_{6-10}$ arylene, or 5-10 membered heteroarylene;

ring A is selected from $C_{3-14}$ cycloalkyl, 3-14 membered heterocyclyl, $C_{6-10}$aryl, or 5-10 membered heteroaryl;

$R_a$ is selected from H, CN, oxo (=O), halogen, OH, or the following groups unsubstituted or optionally substituted with one, two or more $R_{a1}$: $C_{1-12}$ alkyl, $C_{1-12}$ alkoxy, $C_{3-12}$ cycloalkyl, 3-14 membered heterocyclyl, $C_{6-10}$ aryl, 5-10 membered heteroaryl, $-C(O)N(R_{a11})(R_{a12})$, $-N(R_{a13})(R_{a14})$, $-S(O)_2-R_{a15}$, $-S(O)(=NR_{a16})(R_{a17})$, or $-P(O)(R_{a18})(R_{a19})$; each $R_{a1}$ is the same or different and is independently selected from H, OH, CN, halogen, or the following groups unsubstituted or optionally substituted with one, two or more $R_{a2}$: $C_{1-12}$ alkyl, $C_{1-12}$ alkoxy, $C_{3-12}$ cycloalkyl, 3-14 membered heterocyclyl, $C_{6-10}$ aryl, 5-10 membered heteroaryl, $-NH_2$, or $-S(O)_2-C_{1-12}$ alkyl; each $R_{a2}$ is the same or different and is independently selected from H, OH, $NH_2$, CN, halogen, $C_{1-6}$ alkyl, or $C_{1-6}$ alkoxy; $R_{a11}$, $R_{a12}$, $R_{a13}$, $R_{a14}$, $R_{a15}$, $R_{a16}$, $R_{a17}$, $R_{a18}$, and $R_{a19}$ are the same or different and are independently selected from H, $C_{1-12}$ alkyl, $C_{3-7}$ cycloalkyl, or 3-8 membered heterocyclyl; m is selected from 0, 1, 2, 3, 4, or 5;

Z is absent, or is selected from NH, S, O, $C_{1-6}$ alkylene, or $C_{1-6}$ alkylene-NH;

ring E is selected from 3-14 membered heterocyclyl, or $C_{3-12}$ cycloalkyl;

$R_e$ is selected from H, halogen, or the following groups unsubstituted or optionally substituted with one, two or more $R_{e1}$: $C_{1-12}$ alkyl, halo-$C_{1-12}$ alkyl, deuterated $C_{1-12}$ alkyl, $C_{1-12}$ alkoxy, $C_{3-12}$ cycloalkyl, 3-14 membered heterocyclyl, $C_{1-12}$ alkyl-NH-, or $(C_{1-12}$ alkyl$)_2$-N-; each $R_{e1}$ is the same or different and is independently selected from H, OH, $NH_2$, CN, halogen, $C_{1-6}$ alkyl, or $C_{1-6}$ alkoxy; or, two $R_e$ attached to the same carbon atom together with the atoms to which they are respectively attached form a $C_{3-12}$ cycloalkane ring, or a 3-14 membered heterocycle; or, two $R_e$ attached to different carbon atoms together with the atoms to which they are respectively attached form a 3-14 membered heterocycle; p is selected from 0, 1, 2, 3, 4, or 5;

$X_1$, $X_2$, and $X_3$ are the same or different, and are independently selected from CH or N, and at least two of $X_1$, $X_2$, and $X_3$ are CH;

$R_x$ is selected from H, CN, halogen, $C_{1-12}$ alkyl, or $C_{1-12}$ alkoxy; n is selected from 0, 1, 2 or 3;

Y is selected from C(O), C(O)NH, C(S), or $SO_2$.

According to some embodiments, $R_1$, $R_2$, and $R_3$ are the same or different and are independently selected from H, or $C_{1-6}$ alkyl.

According to some embodiments, $R_1$, $R_2$, and $R_3$ are all H.

According to some embodiments, $R_4$ is selected from $C_{1-6}$ alkyl, $C_{2-6}$ alkenyl, halo-$C_{1-6}$ alkyl, or cyano-$C_{1-6}$ alkyl.

According to some embodiments, $R_4$ is selected from $C_{1-6}$ alkyl, halo-$C_{1-6}$ alkyl, or cyano-$C_{1-6}$ alkyl.

According to some embodiments, $R_4$ is selected from methyl, ethyl, propyl, isopropyl, vinyl, cyanomethyl, 2-fluoroethyl, 2,2-difluoroethyl, or 2,2,2-trifluoroethyl.

According to some embodiments, $R_4$ is selected from methyl, ethyl, propyl, isopropyl, cyanomethyl, 2-fluoroethyl, 2,2-difluoroethyl, or 2,2,2-trifluoroethyl.

According to some embodiments, W is selected from $C_{2-4}$ alkenylene, $C_{2-4}$ alkynylene, $C_{3-6}$ cycloalkylene, $C_{6-10}$ arylene, or 5-6 membered heteroarylene.

According to some embodiments, W is selected from:

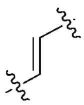, 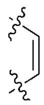, 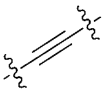, 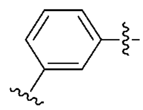,

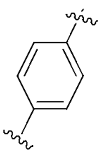, 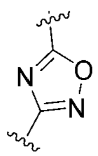, 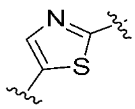, 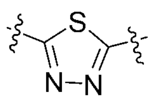, 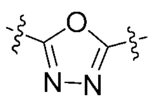, 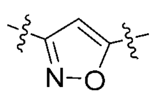, 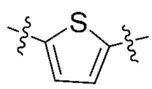, 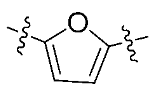,

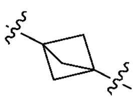, 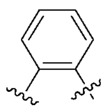, 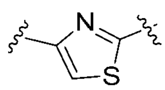, 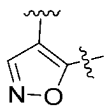, 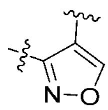, 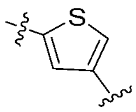, or 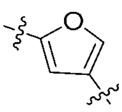.

According to some embodiments, ring A is selected from phenyl, 5-6 membered heteroaryl, 8-9 membered heteroaryl, 5-10 membered heterocyclyl, or $C_{3-6}$ membered cycloalkyl.

According to some embodiments, ring A is selected from phenyl, 5-6 membered heteroaryl, 8-9 membered heteroaryl, 8-9 membered heterocyclyl, or $C_{3-6}$ membered cycloalkyl.

According to some embodiments, ring A is selected from:

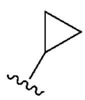, 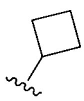, 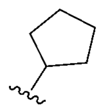, 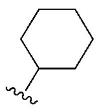,

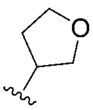 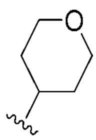, 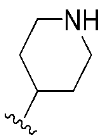, 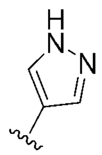, 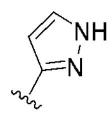, 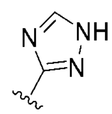, 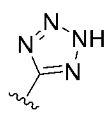, 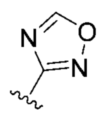, 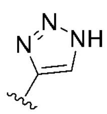, 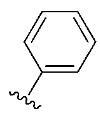,

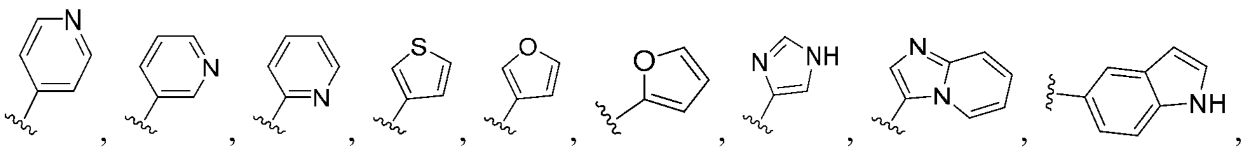

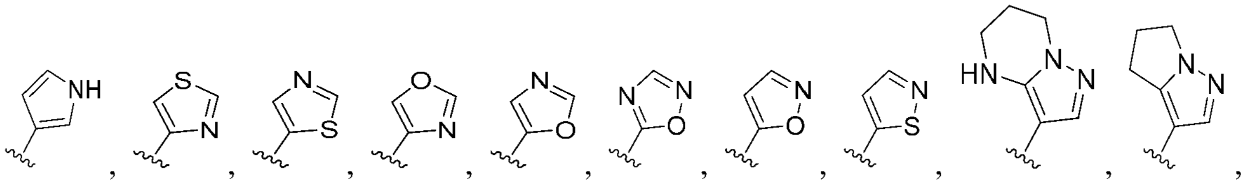

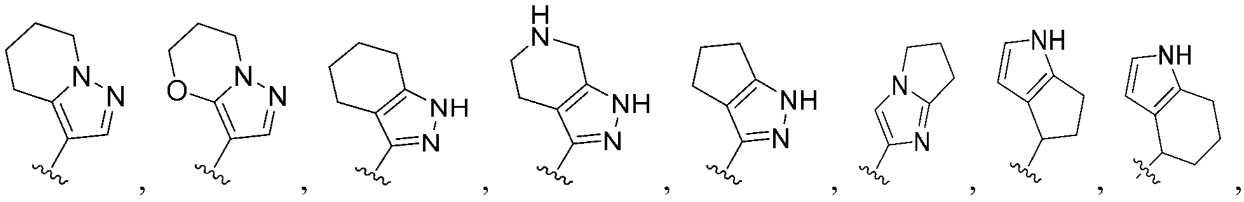

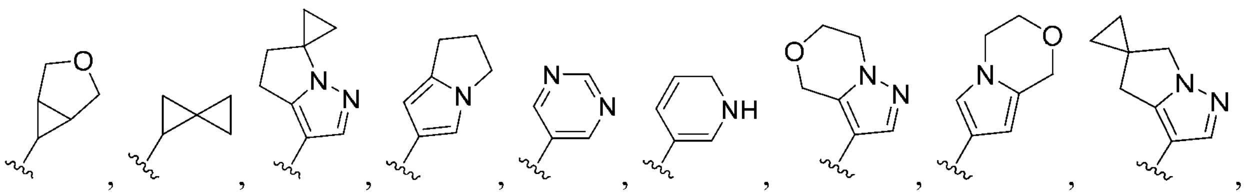

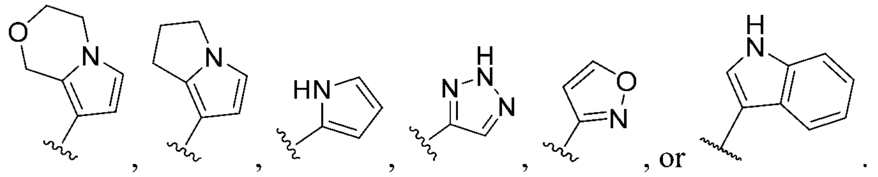

According to some embodiments, $R_a$ is selected from H, CN, oxo (=O), halogen, OH, or the following groups unsubstituted or optionally substituted with one, two or more $R_{a1}$: $C_{1-6}$ alkyl, $C_{1-6}$ alkoxy, $C_{3-6}$ cycloalkyl, 4-7 membered heterocyclyl, phenyl, 5-6 membered heteroaryl, - C(O)(NHC$_{1-6}$ alkyl), -S(O)$_2$-C$_{1-6}$ alkyl, -S(O)(-NH)(C$_{1-6}$ alkyl), -S(O)(NC$_{1-6}$ alkyl)(C$_{1-6}$ alkyl), - P(O)(C$_{1-6}$ alkyl)(C$_{1-6}$ alkyl), or -NH$_2$; each $R_{a1}$ is the same or different and is independently selected from H, OH, CN, halogen, $C_{1-6}$ alkyl, halo-C$_{1-6}$ alkyl, $C_{1-6}$ alkoxy, $C_{3-6}$ cycloalkyl, 5-6 membered heterocyclyl, phenyl, 5-6 membered heteroaryl, cyano-C$_{1-6}$ alkyl, cyano-C$_{1-6}$ alkoxy, $C_{1-6}$ alkyl-NH-, ($C_{1-6}$ alkyl)$_2$-N-, -S(O)$_2$-C$_{1-6}$ alkyl, $C_{1-6}$ alkyl-O-C$_{1-6}$ alkyl, or hydroxy-C$_{1-6}$ alkyl.

According to some embodiments, $R_a$ is selected from H, F, Cl, CN, oxo (=O), OH, or the following groups unsubstituted or optionally substituted with one, two or more $R_{a1}$: methyl, ethyl, propyl, isopropyl, tert-butyl, cyclopropyl, cyclobutyl, cyclopentyl, cyclohexyl, methoxy, isopropoxy, propoxy, phenyl, pyridyl, benzyl, piperidyl,

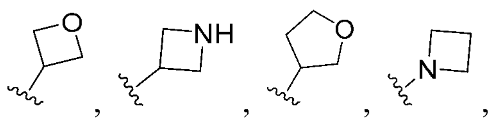

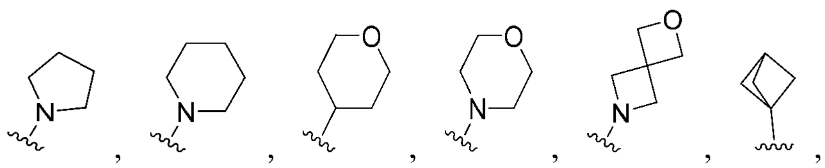

$-C(O)NHCH_3$, $-S(O)_2-CH_3$, - $S(O)(=NH)(CH_3)$, $-P(O)(CH_3)(CH_3)$, or $-NH_2$; each $R_{a1}$ is the same or different and is independently selected from H, CN, OH, F, Cl, methyl, ethyl, methoxy, trifluoromethyl, cyclopropyl, cyclobutyl, cyclopentyl, $-CH_2OCH_3$, $-CH_2CH_2OCH_3$, $-CH_2OH$, $-CH_2CN$, $-N(CH_3)_2$, $-S(O)_2-CH_3$,

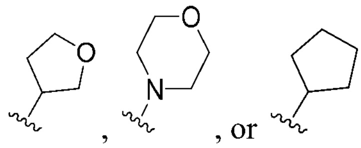, , or .

According to some embodiments, $R_a$ is selected from H, F, Cl, CN, oxo (=O), OH, $NH_2$, methylamino, dimethylamino, methyl, ethyl, isopropyl, tert-butyl, $-C(CH_3)_2CH_2CH_3$, - $CH_2C(CH_3)_3$, cyclopropyl, cyclobutyl, cyclopentyl, cyclohexyl, phenyl, pyridyl, benzyl, 2,2-difluoroethyl, 2,2,2-trifluoroethyl, monofluoromethyl, difluoromethyl, trifluoromethyl, trifluoromethoxy, methoxy, isopropyloxy, 2-hydroxyethyl,

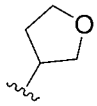, 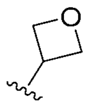, 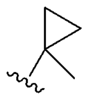, 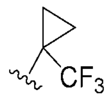,

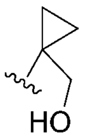, 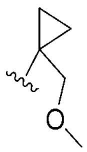, 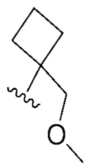, 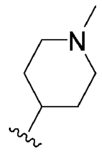, 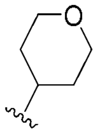, 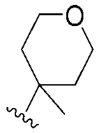, 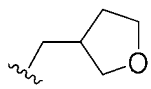, 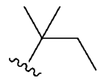, 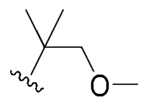, 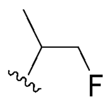,

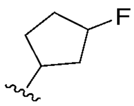, 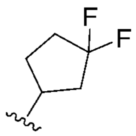, 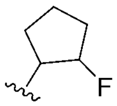, 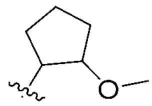, 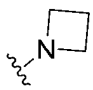, 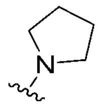, 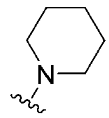, 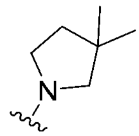,

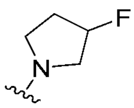, 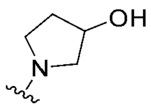, 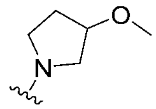, 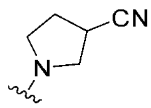, 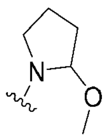, 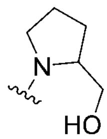, 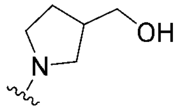, 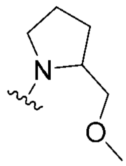,

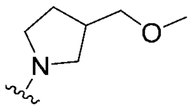, 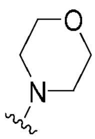, 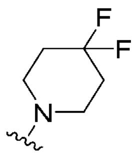, 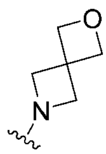, 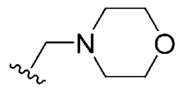, 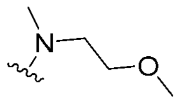, 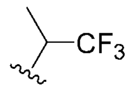,

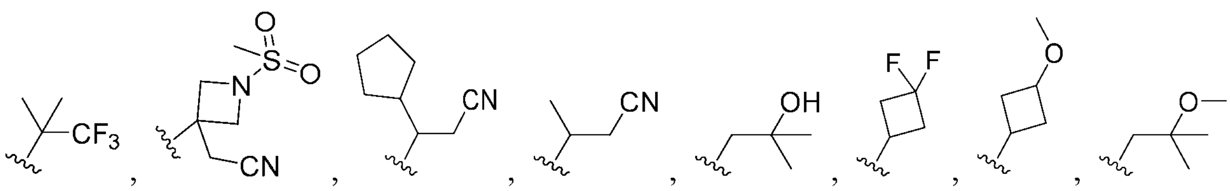, , , , , , , ,

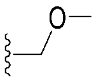, 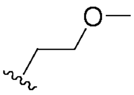, 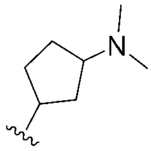, 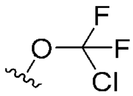, 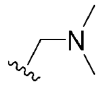, 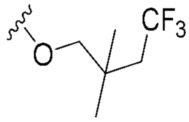, 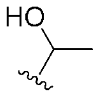, 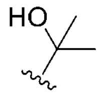, 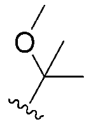,

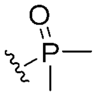, 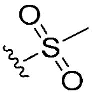, 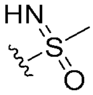, 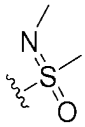, 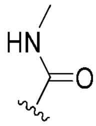, 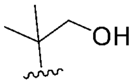, 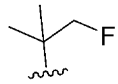, 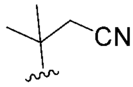, 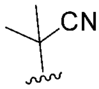, 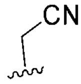,

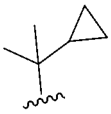, 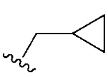, 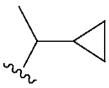, 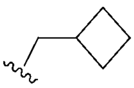, 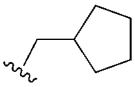, 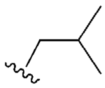, 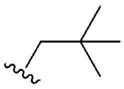, 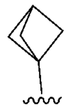, 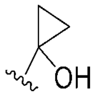,

or

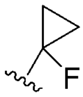.

According to some embodiments,

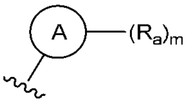

is selected from

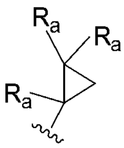, 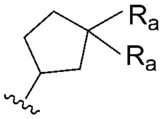,

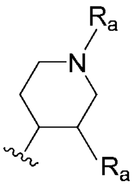, 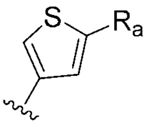, 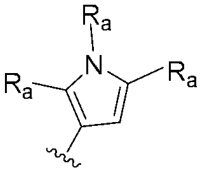, 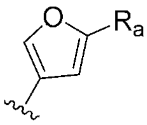, 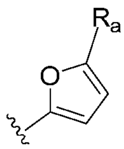, 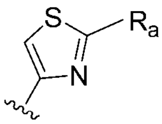, 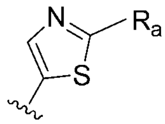,

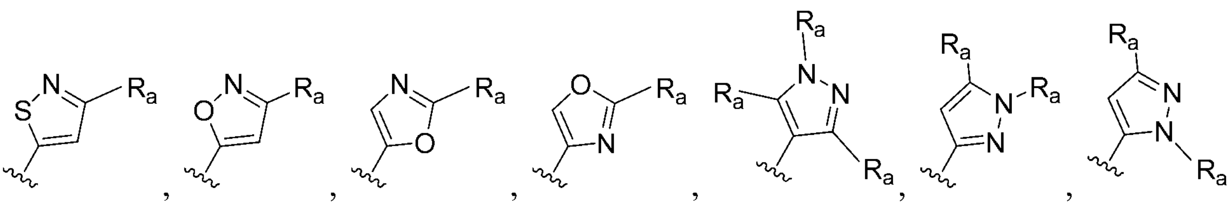, , , , , , ,

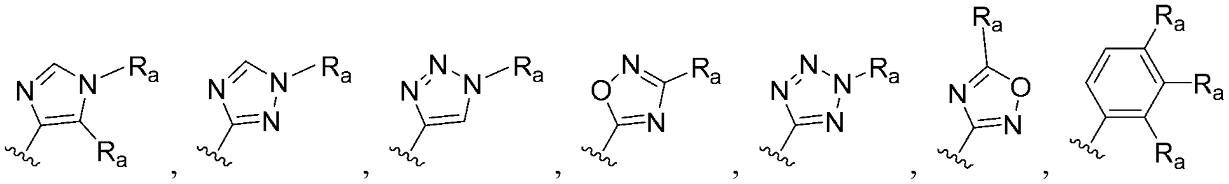

, , , , , , ,

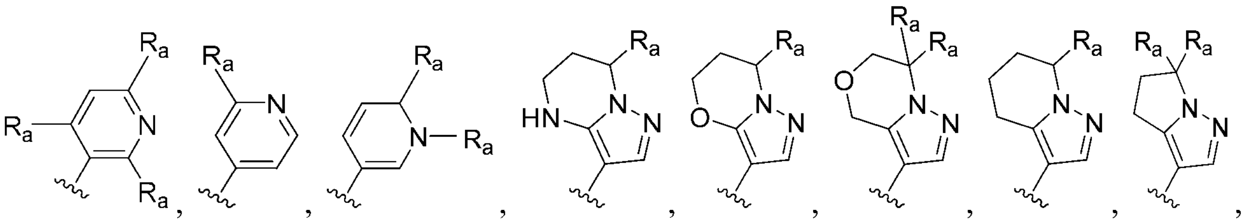

, , , , , , , , , ,

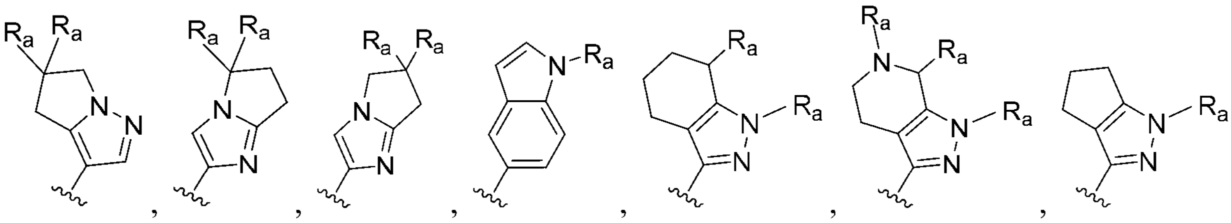

, , , , , , ,

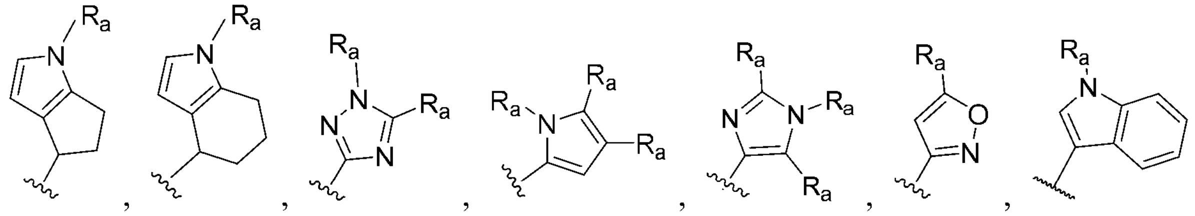

, , , , , , ,

or

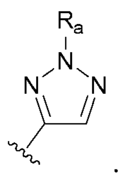

.

According to some embodiments, Z is absent or is selected from NH, S, O, $CH_2$, or $CH_2NH$.

According to some embodiments, ring E is selected from 5-9 membered heterocyclyl or $C_{5-6}$ cycloalkyl.

According to some embodiments, ring E is selected from 6-9 membered heterocyclyl or $C_{5-6}$ cycloalkyl.

According to some embodiments, ring E is selected from

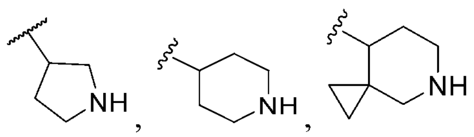

, , ,

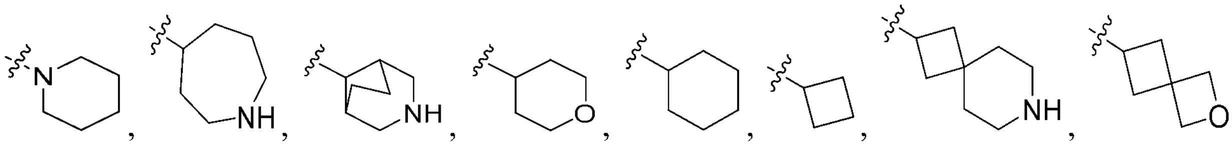

, , , , , , , ,

or

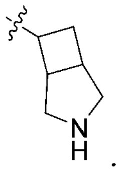.

According to some embodiments, ring E is selected from

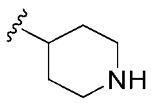, 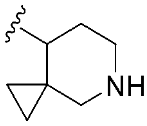, 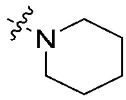,

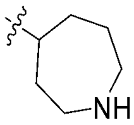, 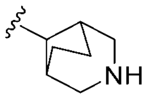, 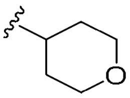, 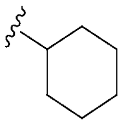, 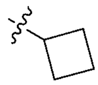, 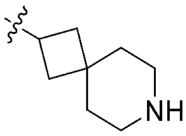, 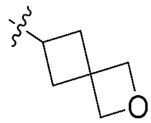, or 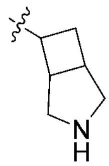.

According to some embodiments, $R_e$ is selected from H, halogen, or the following groups unsubstituted or optionally substituted with one, two or more $R_{e1}$: $C_{1-6}$ alkyl, halo-$C_{1-6}$ alkyl, deuterated $C_{1-6}$ alkyl, $C_{1-6}$ alkoxy, $C_{3-6}$ cycloalkyl, 4-9 membered heterocyclyl, or ($C_{1-6}$ alkyl)$_2$-N-; each $R_{e1}$ is the same or different and is independently selected from OH, halogen, $C_{1-3}$ alkyl, or $C_{1-3}$ alkoxy; or two $R_e$ together with the atoms to which they are respectively attached form a $C_{3-6}$ cycloalkane ring.

According to some embodiments, $R_e$ is selected from H, halogen, $C_{1-6}$ alkyl, halo-$C_{1-6}$ alkyl, deuterated $C_{1-6}$ alkyl, $C_{1-6}$ alkoxy, $C_{3-6}$ cycloalkyl, 5-6 membered heterocyclyl, or ($C_{1-6}$ alkyl)$_2$-N-; or, two $R_e$ together with the atoms to which they are respectively attached form a $C_{3-6}$ cycloalkane ring;

According to some embodiments, $R_e$ is selected from H, F, Cl, methyl, deuterated methyl ($CD_3$), trifluoromethyl, ethyl, isopropyl, cyclopropyl, methoxy, dimethylamino,

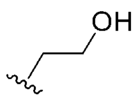, 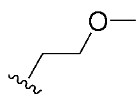,

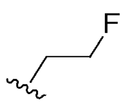, 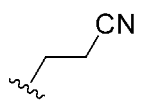, 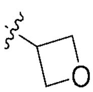, 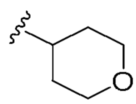, 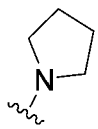, 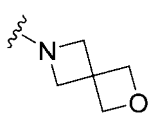, or 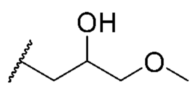;

or, two $R_e$ attached to the same carbon atom together with the atoms to which they are respectively attached form a cyclopropane ring,

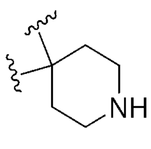, or 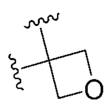;

or, two $R_e$ attached to different carbon atoms together with the atoms to which they are respectively attached form

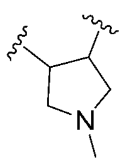.

According to some embodiments, $R_e$ is selected from H, F, Cl, methyl, deuterated methyl ($CD_3$), trifluoromethyl, ethyl, isopropyl, cyclopropyl, methoxy, dimethylamino,

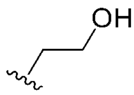 , 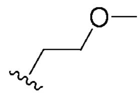 ,

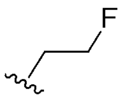 , 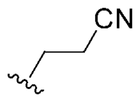 , 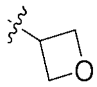 , 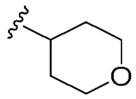 , 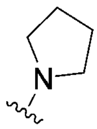 , or 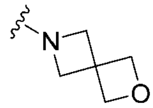 ;

or, two $R_e$ attached to the same carbon atom together with the atoms to which they are respectively attached form a cyclopropane ring,

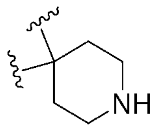 , or 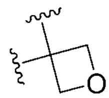 ;

or, two $R_e$ attached to different carbon atoms together with the atoms to which they are respectively attached form

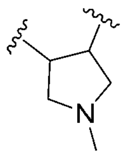 .

According to some embodiments, $X_1$, $X_2$, and $X_3$ are all CH, or one of $X_1$, $X_2$, and $X_3$ is N.

According to some embodiments, $R_x$ is selected from H, CN, halogen, $C_{1-6}$ alkyl, or $C_{1-6}$ alkoxy.

According to some embodiments, $R_x$ is selected from H, F, Cl, CN, methoxy, or methyl.

According to some embodiments, the compound shown as formula (I) has a structure shown below:

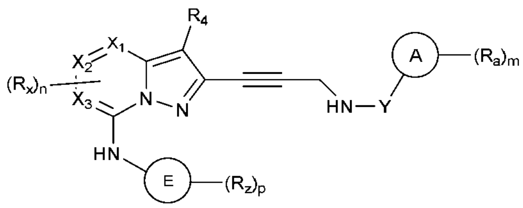

IA-1 ,

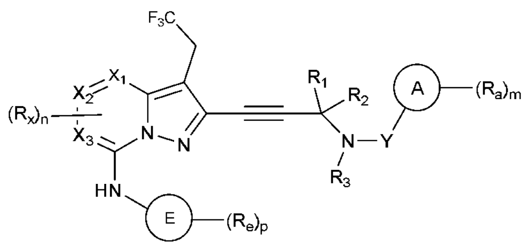

IA-2 ,

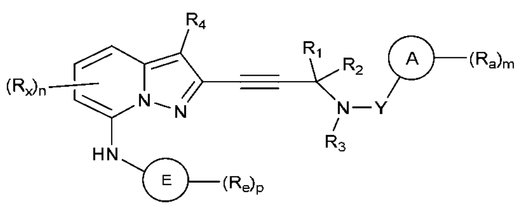

IA-3 ,

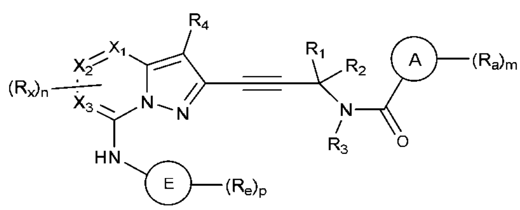

IA-4 ,

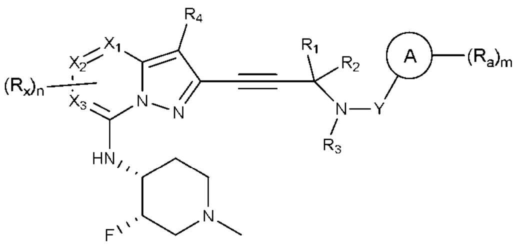
IA-5
,

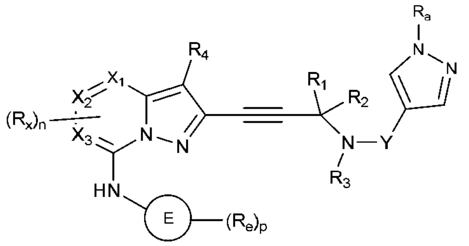
IA-6
,

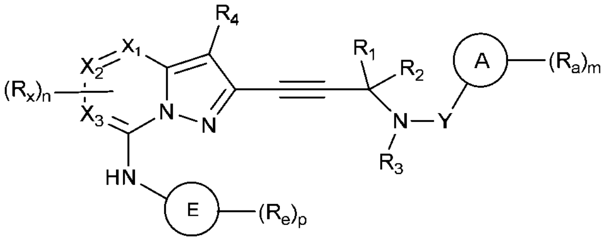
IA-7
,

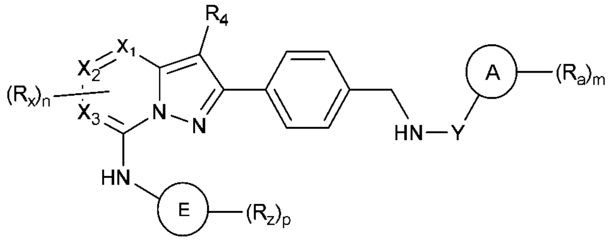
IA-8
,

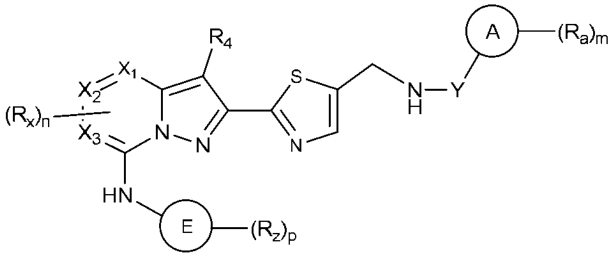
IA-9
or

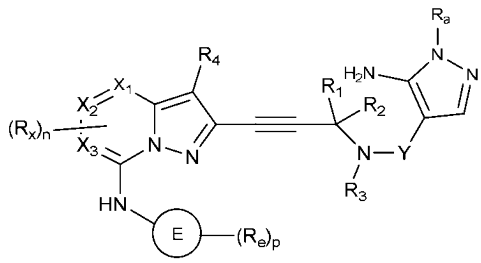
IA-10
;

wherein ring A, ring E, $X_1$, $X_2$, $X_3$, Y, $R_1$, $R_2$, $R_3$, $R_4$, $R_a$, $R_e$, $R_x$, m, n, and p are as defined in the present invention.

According to some embodiments, the compound shown as formula (I) has a structure shown below:

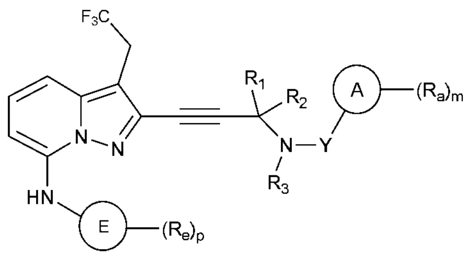
IB-1
,

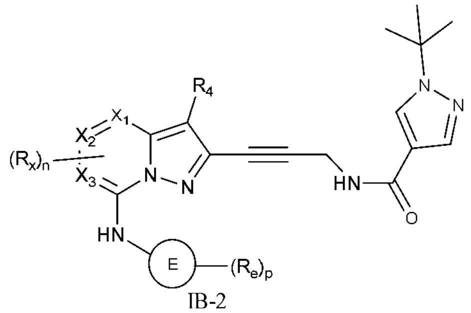
IB-2

or

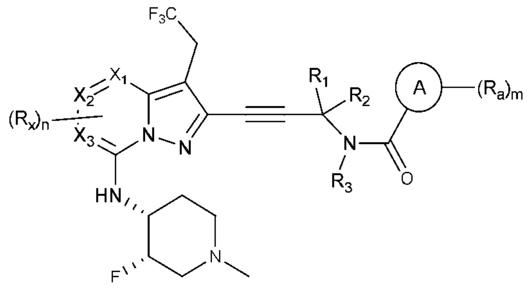

IB-3

;

wherein ring A, ring E, $X_1$, $X_2$, $X_3$, Y, $R_1$, $R_2$, $R_3$, $R_4$, $R_a$, $R_e$, $R_x$, m, n, and p are as defined in the present invention.

According to some embodiments, the compound shown as formula (I) has a structure shown below:

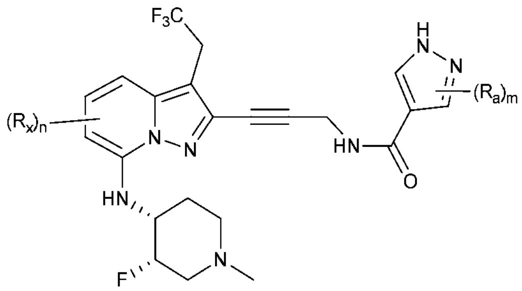

II

wherein $R_a$, $R_x$, m, and n are as defined in the present invention.

According to some embodiments, the compound shown as formula (I) has a structure shown below:

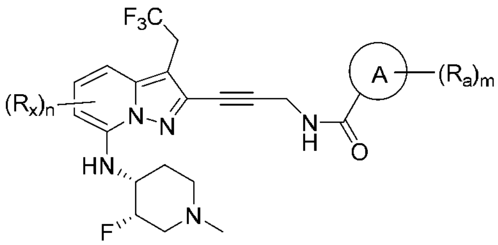

III

wherein ring A, $R_a$, $R_x$, m, and n are as defined in the present invention.

According to some embodiments, the compound shown as formula (I) has a structure shown below:

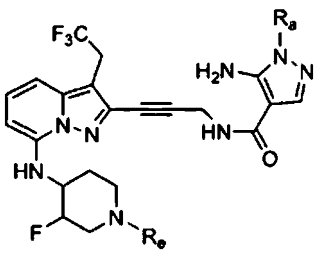

IV

or

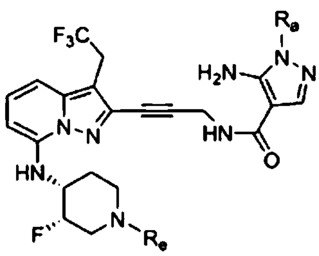

IV-1

wherein $R_a$ and $R_e$ are as defined in the present invention.

According to some embodiments, among the compound shown as formula (I) and the racemate, stereoisomer, tautomer, isotope-labeled counterpart, nitrogen oxide, solvate, polymorph, metabolite, ester, prodrug or pharmaceutically acceptable salt thereof, the exemplary and non-limiting examples of the compound shown as formula (I) are as follows:

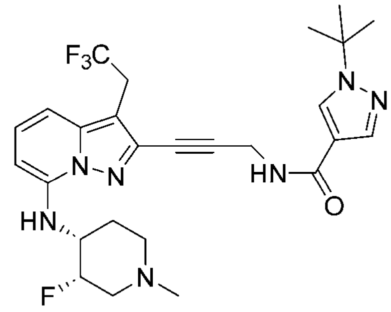
001 ,

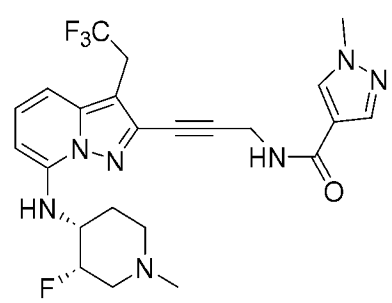
002 ,

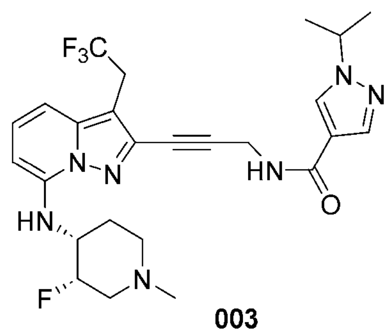
003 ,

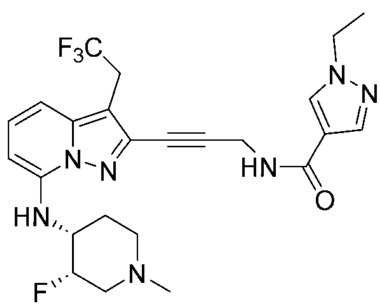
004 ,

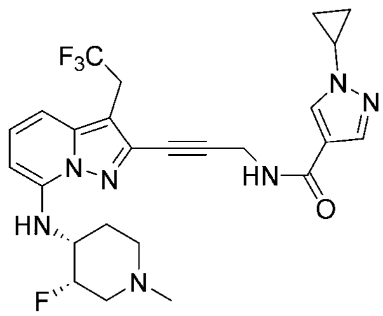
005 ,

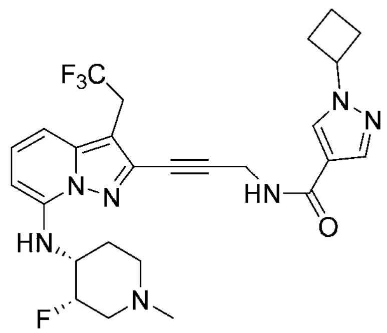
006 ,

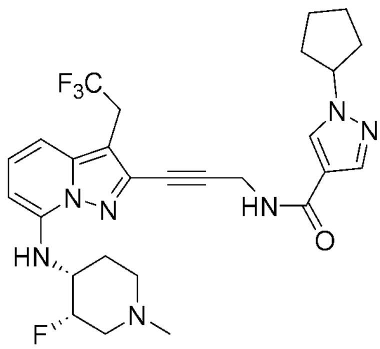
007 ,

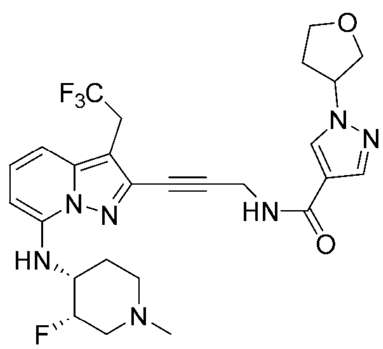
008 ,

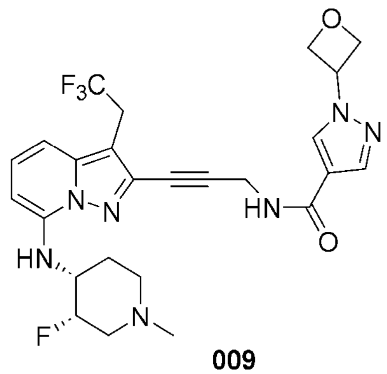
009 ,

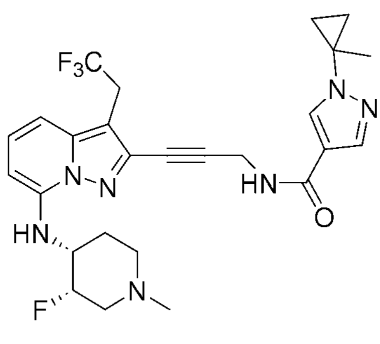
010 ,

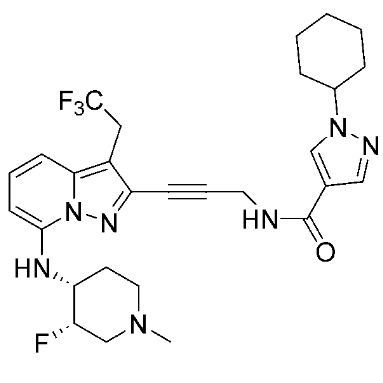
011 ,

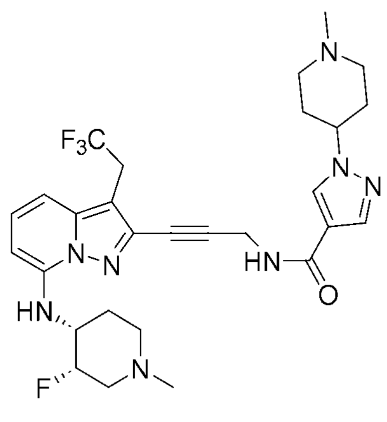
012 ,

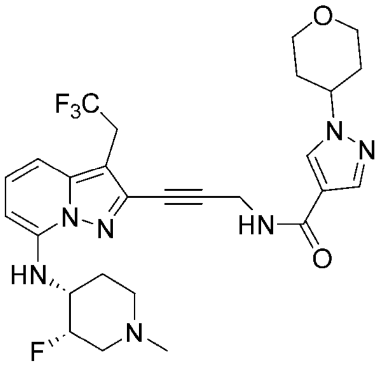

**013** ,

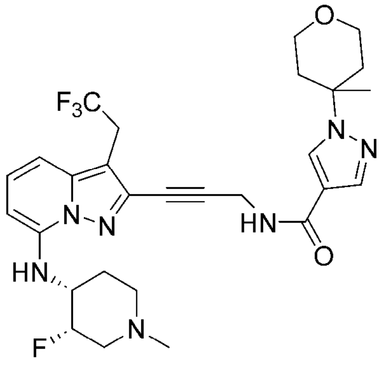

**014** ,

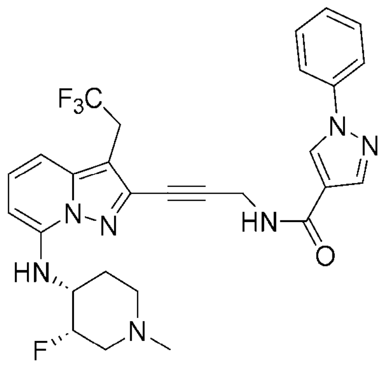

**015** ,

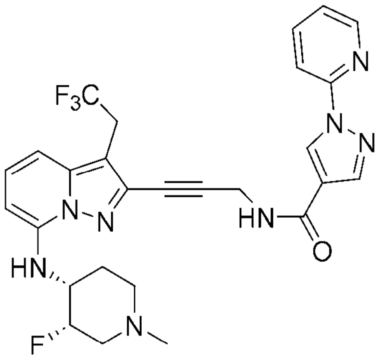

**016** ,

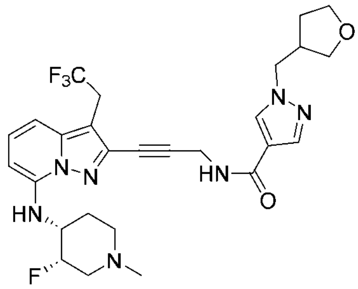

**017** ,

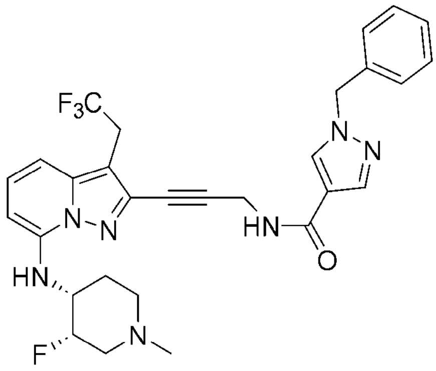

**018** ,

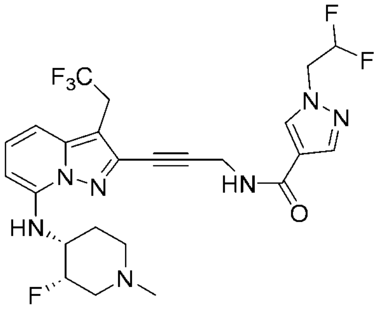

**019** ,

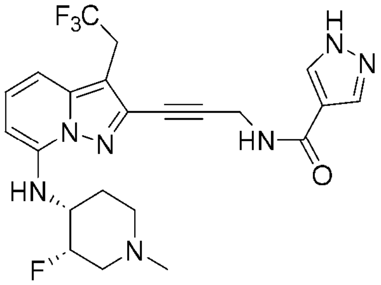

**020** ,

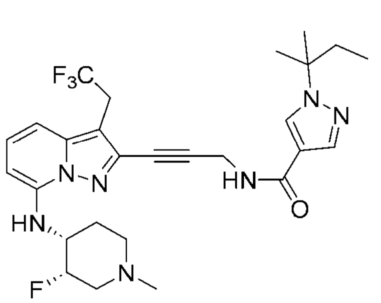

**021** ,

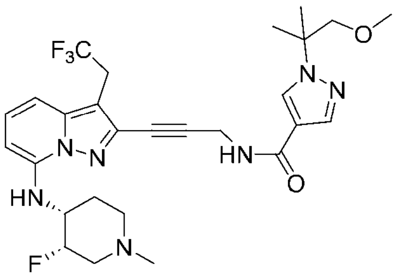

**022** ,

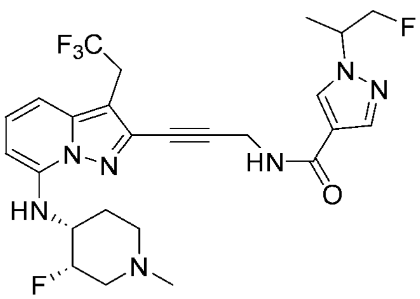

**023** ,

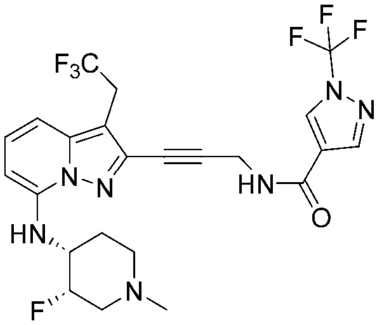

**024** ,

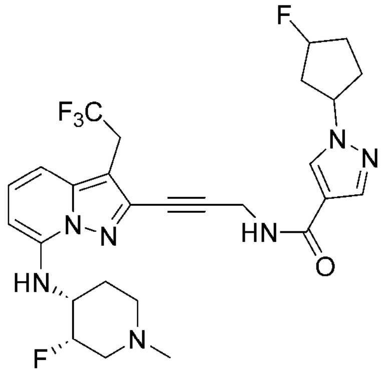

**025** ,

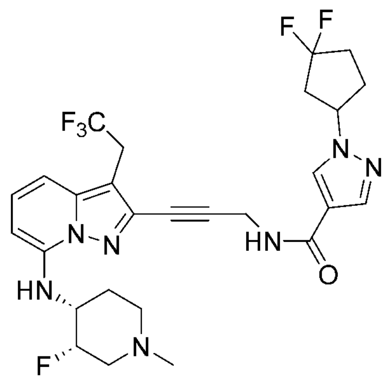

**026** ,

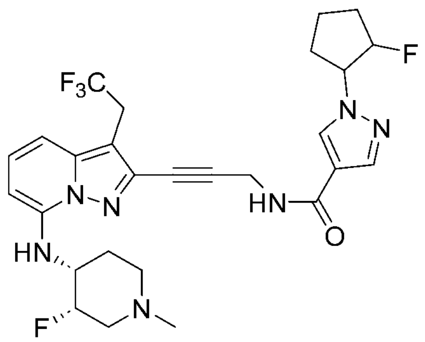

**027** ,

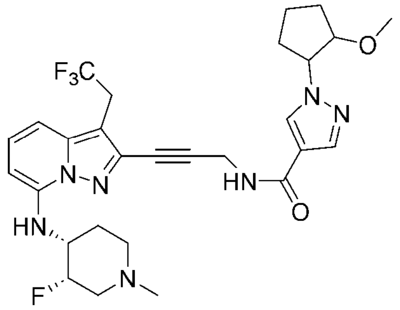

**028** ,

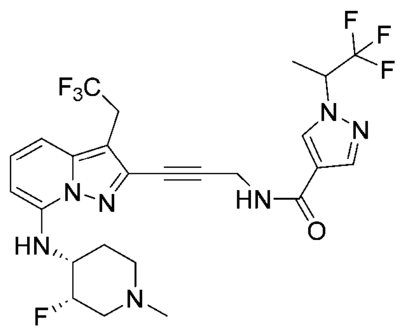

**029** ,

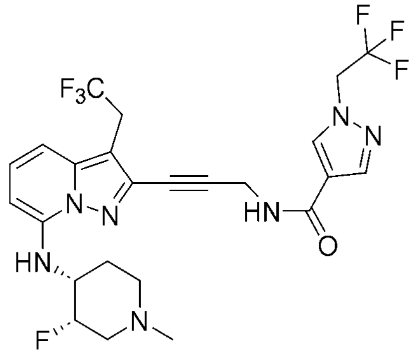

**030** ,

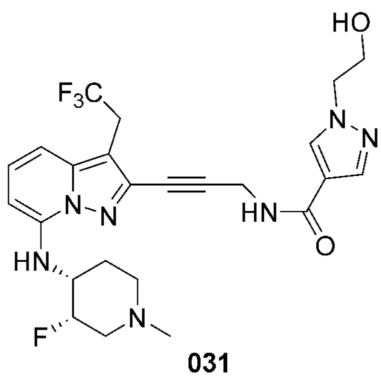

**031** ,

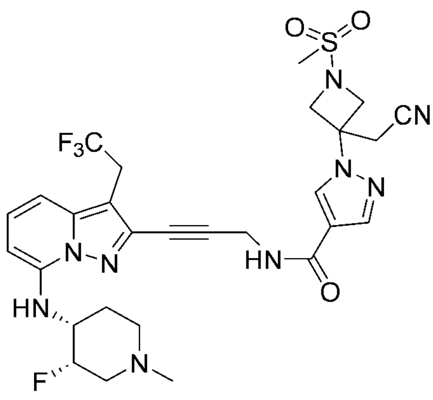

**032** ,

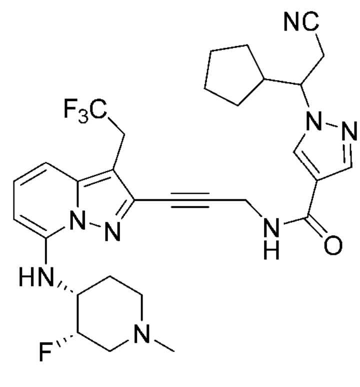

**033** ,

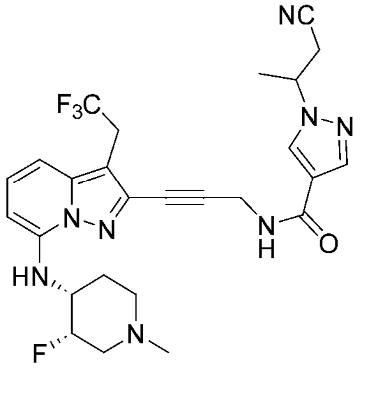

**034** ,

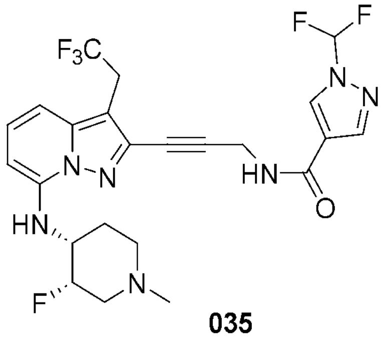

**035** ,

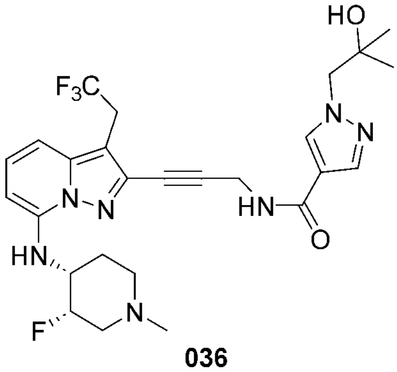

**036** ,

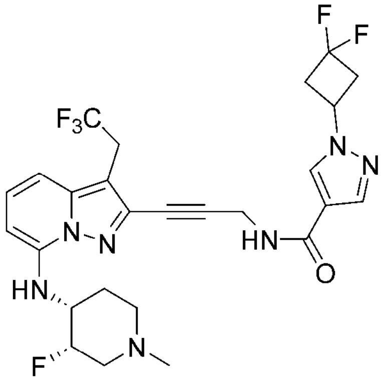

**037** ,

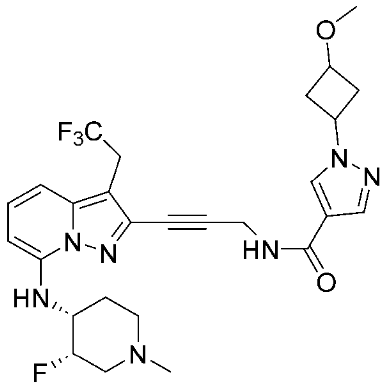

**038** ,

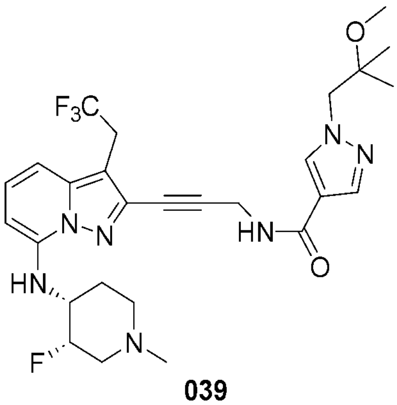

**039** ,

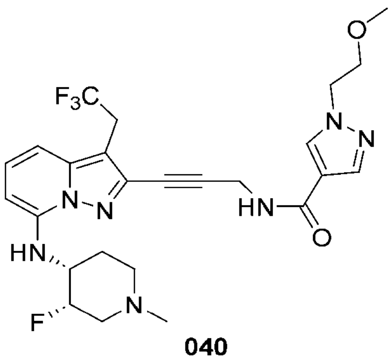

**040** ,

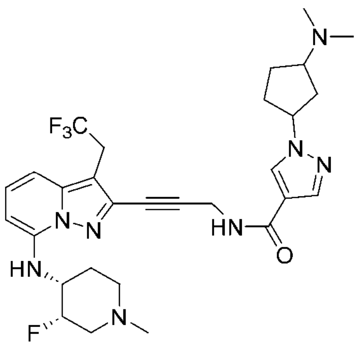

**041** ,

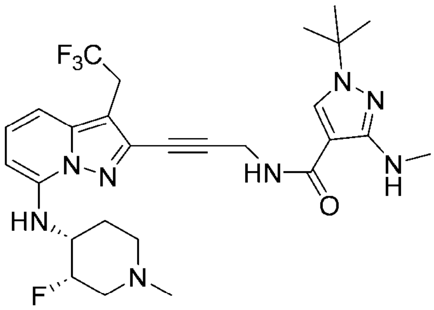

**042** ,

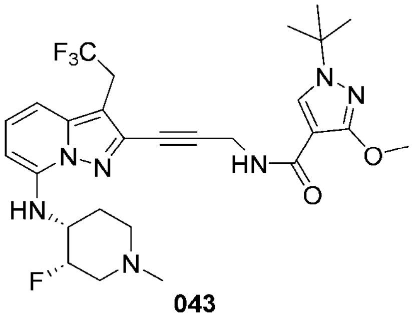

**043** ,

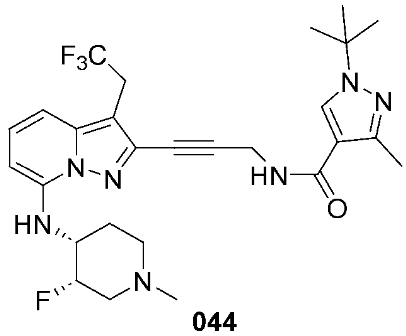

**044** ,

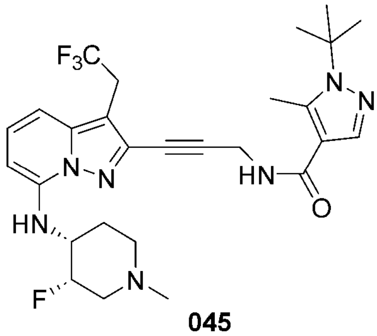

**045** ,

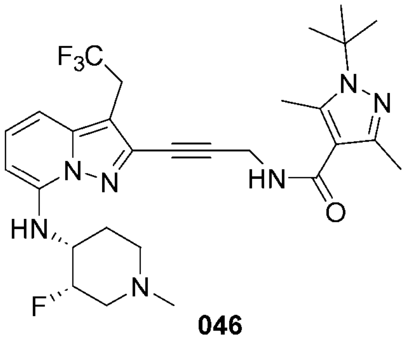

**046**,

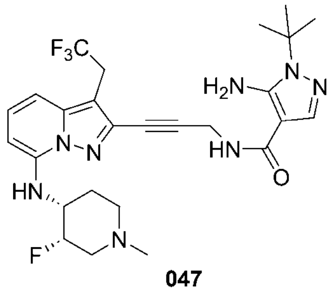

**047**,

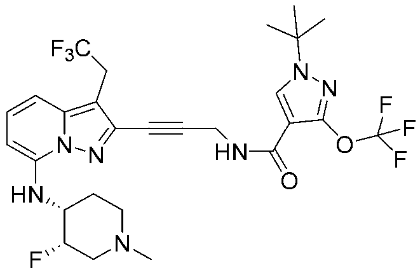

**048**,

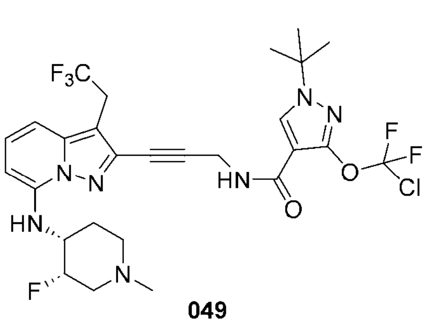

**049**,

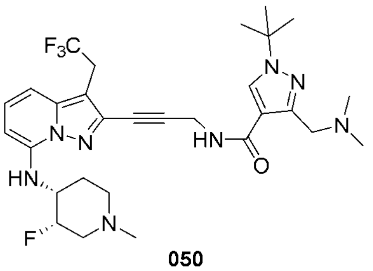

**050**,

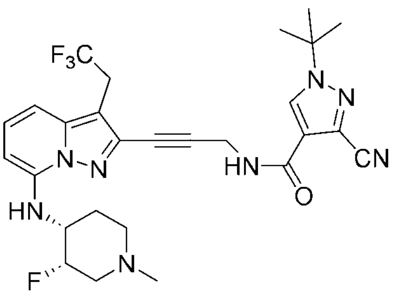

**051**,

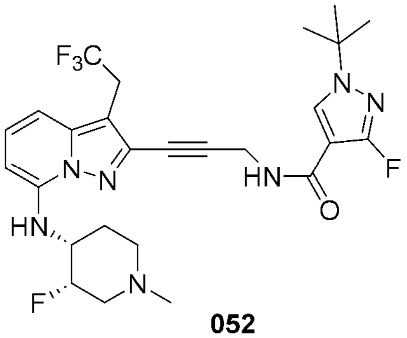

**052**,

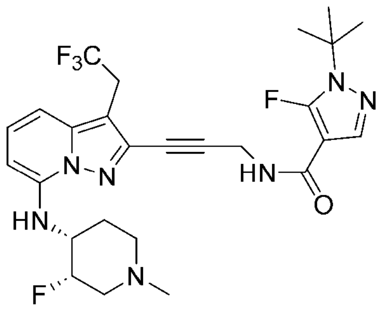

**053**,

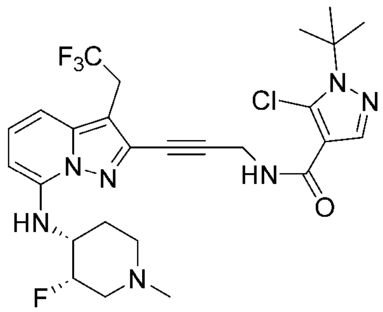

**054**,

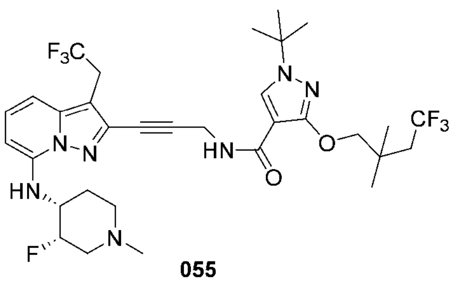

**055**,

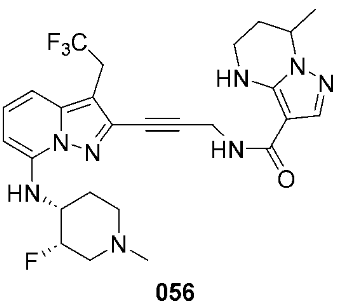

**056**,

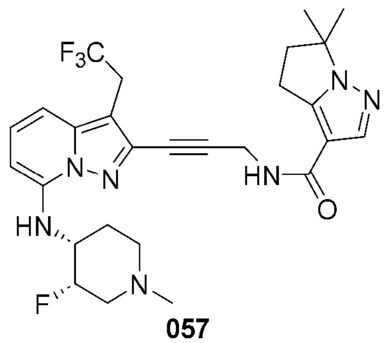

**057**,

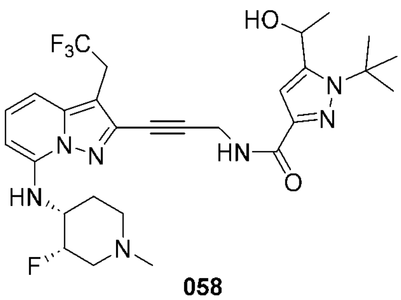
058 ,
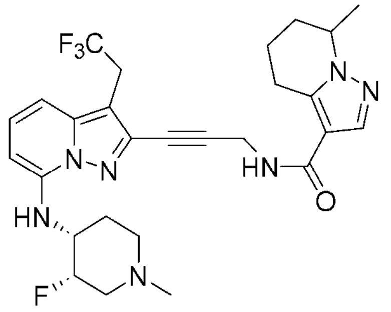
059 ,
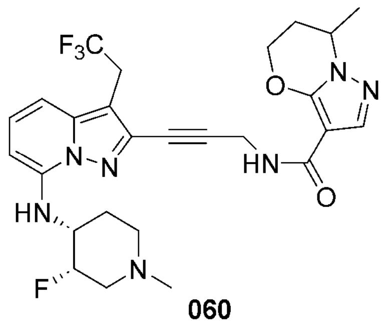
060 ,

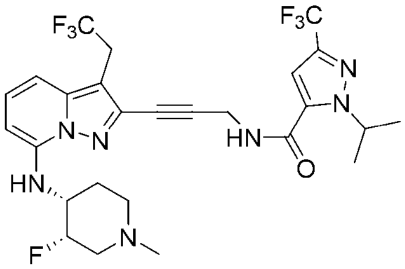
061 ,
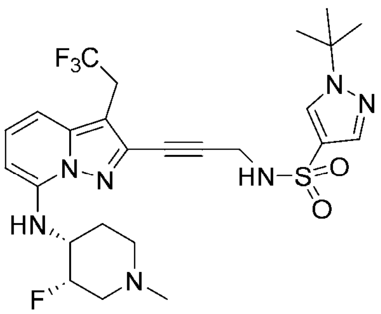
062 ,
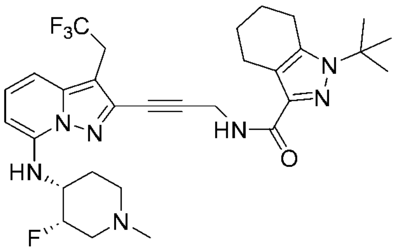
063 ,

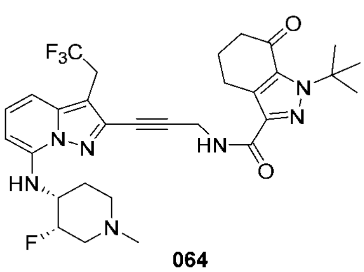
064 ,
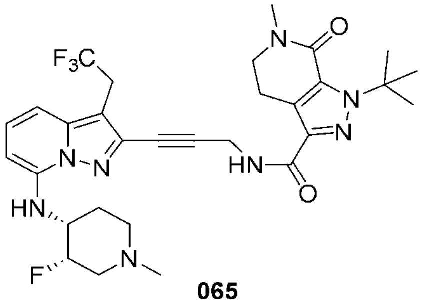
065 ,
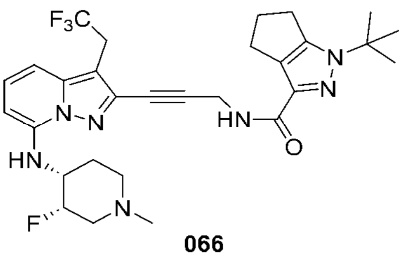
066 ,

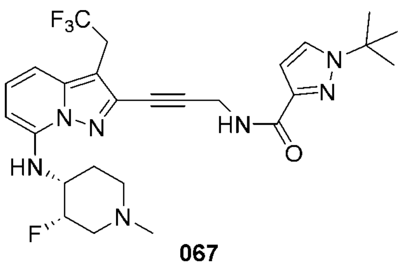
067 ,
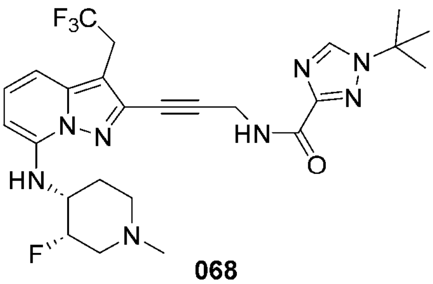
068 ,
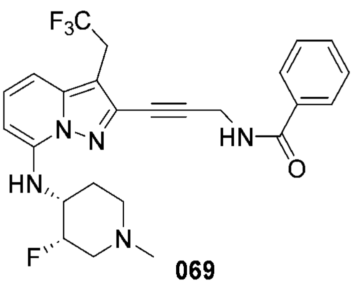
069 ,

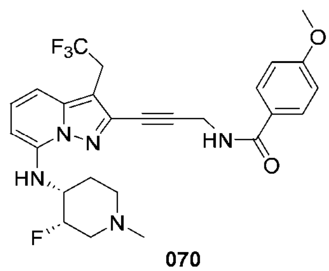

070 ,

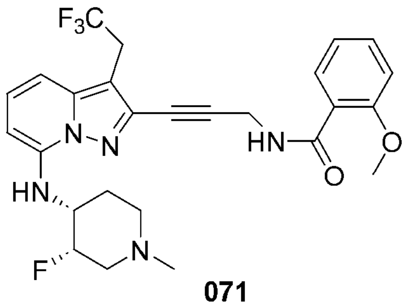

071 ,

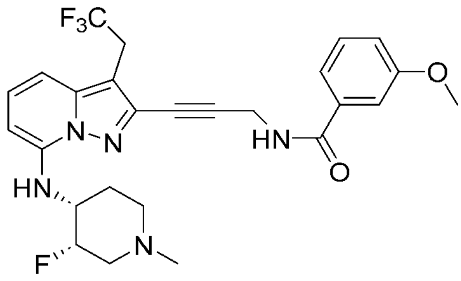

072 ,

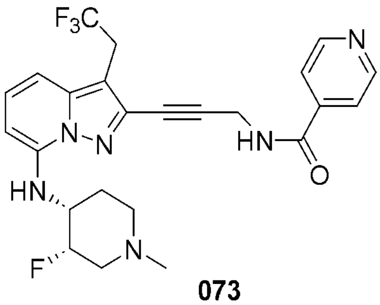

073 ,

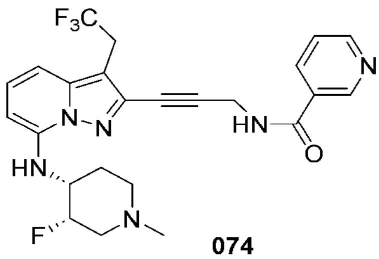

074 ,

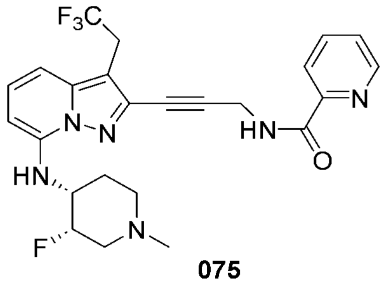

075 ,

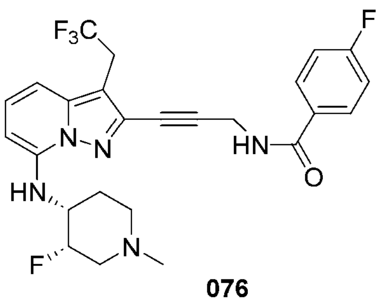

076 ,

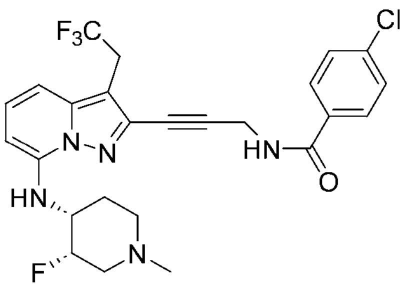

077 ,

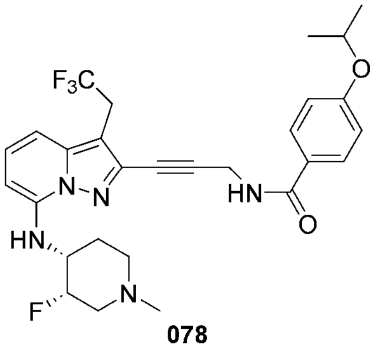

078 ,

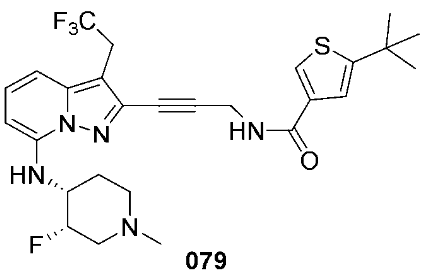

079 ,

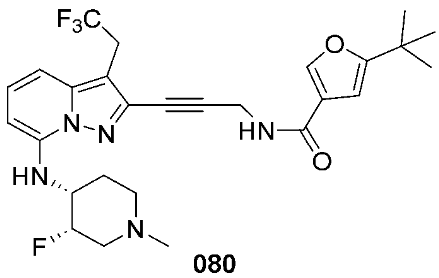

080 ,

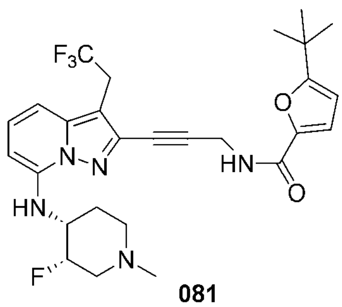

081 ,

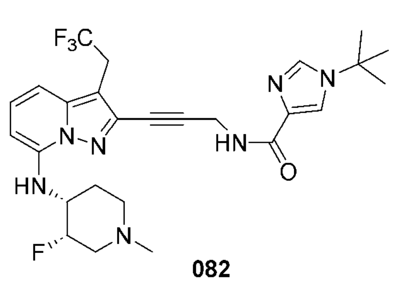
082 ,

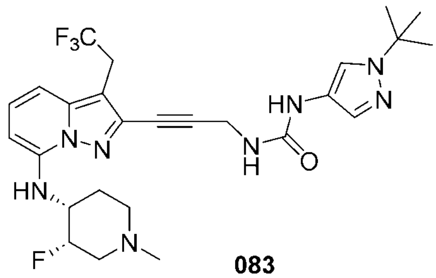
083 ,

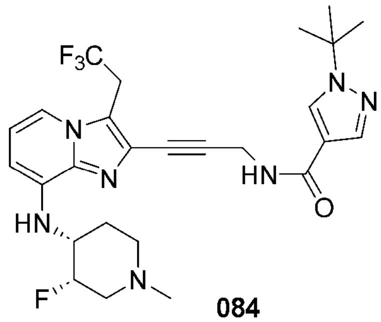
084 ,

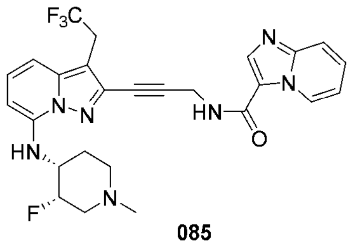
085 ,

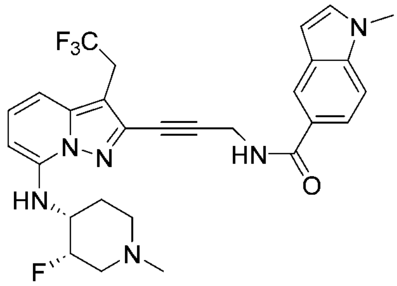
086 ,

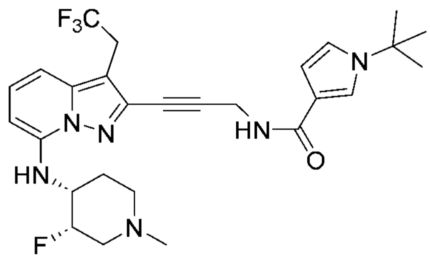
087 ,

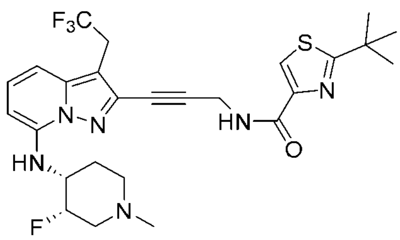
088 ,

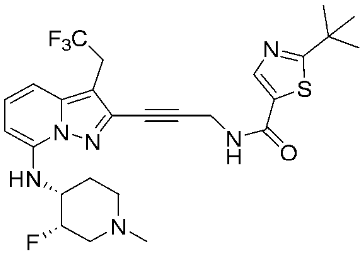
089 ,

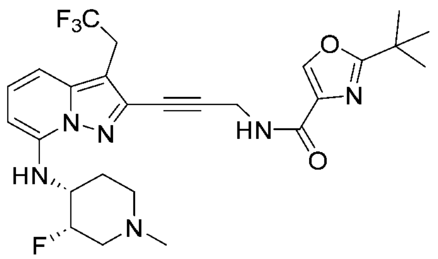
090 ,

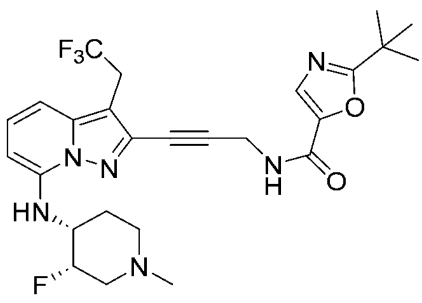
091 ,

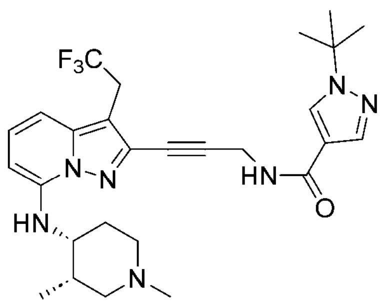
092 ,

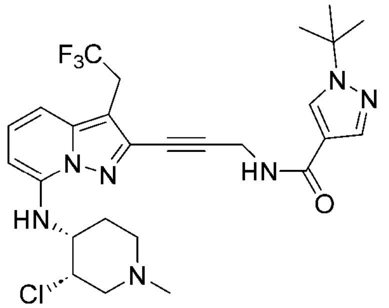
093 ,

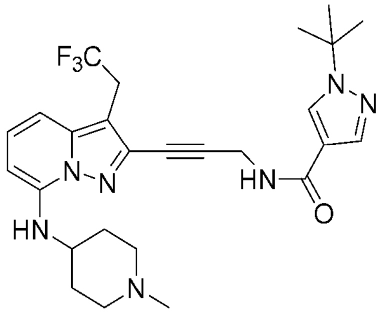

**094** ,

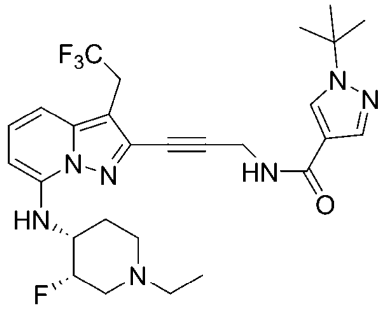

**095** ,

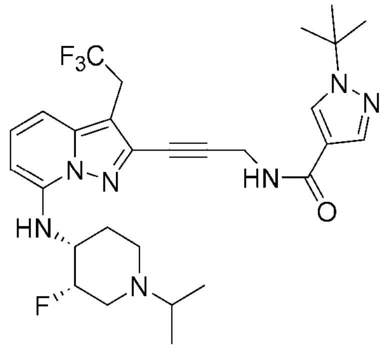

**096** ,

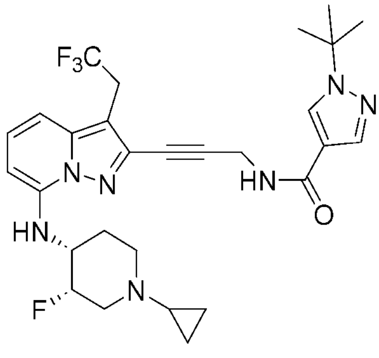

**097** ,

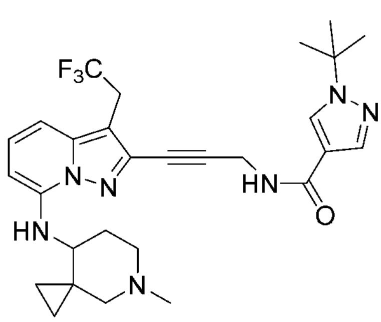

**098** ,

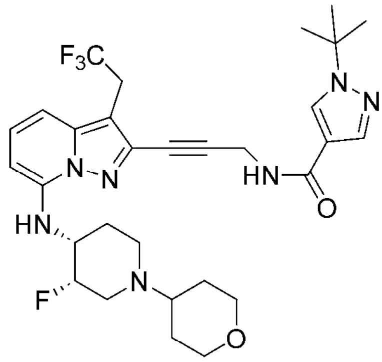

**099** ,

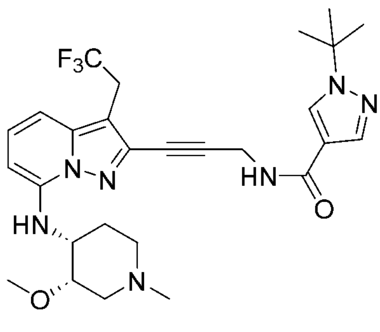

**100** ,

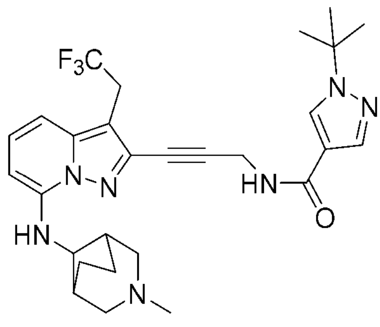

**101** ,

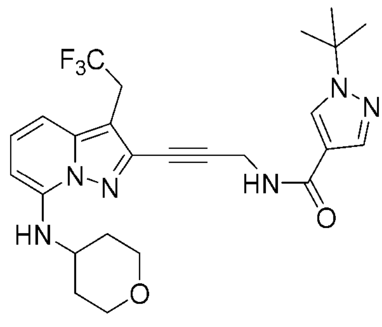

**102** ,

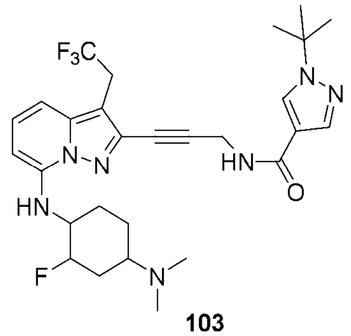

**103** ,

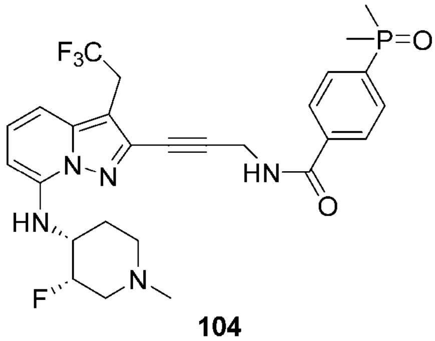

**104** ,

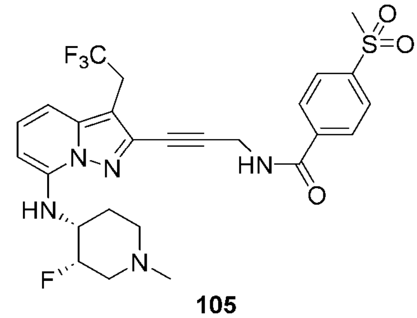

**105** ,

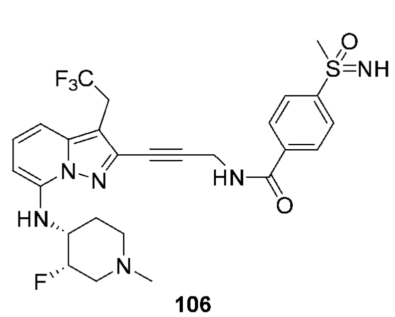

106 ,

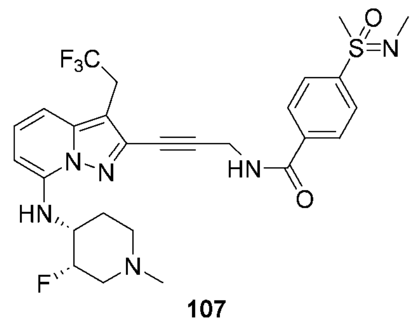

107 ,

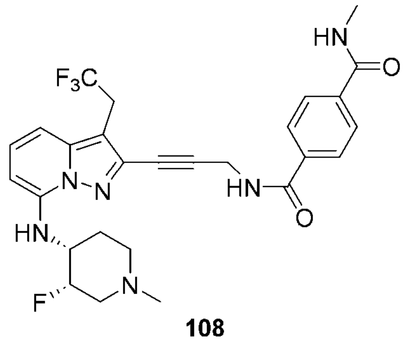

108 ,

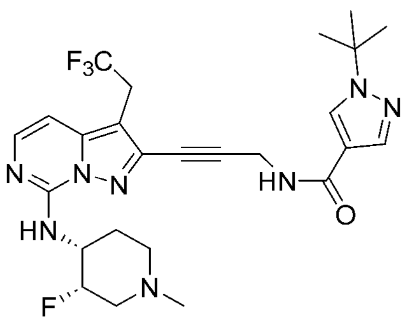

109 ,

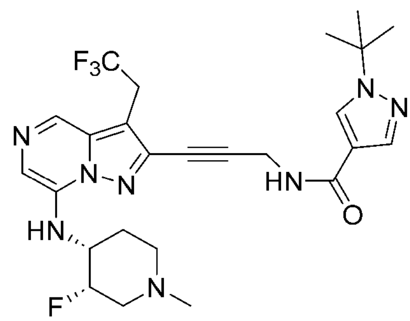

110 ,

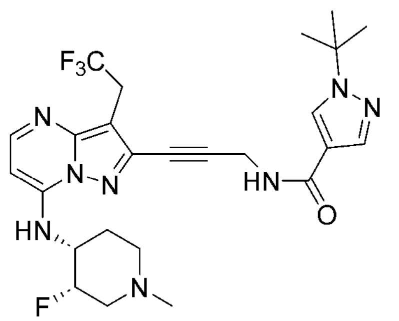

111 ,

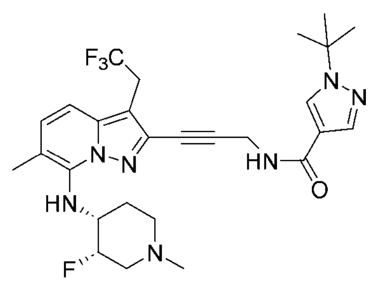

112 ,

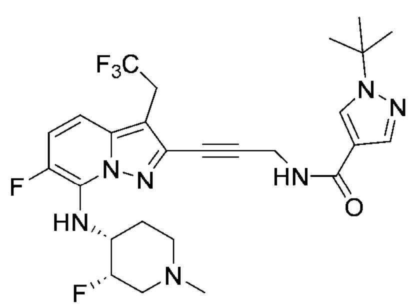

113 ,

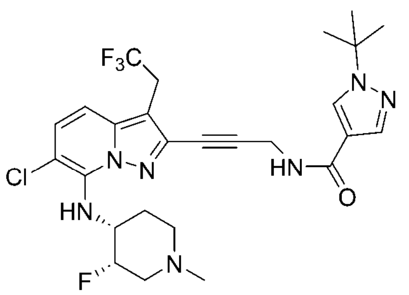

114 ,

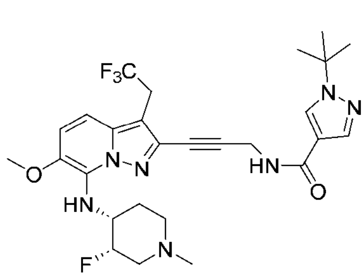

115 ,

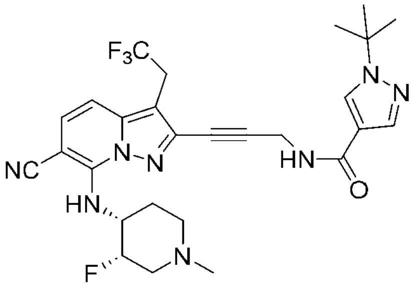

116 ,

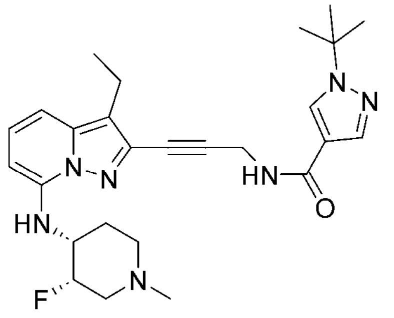

117 ,

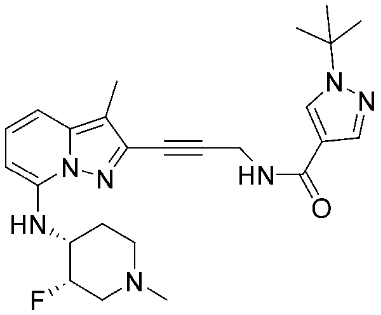
118 ,

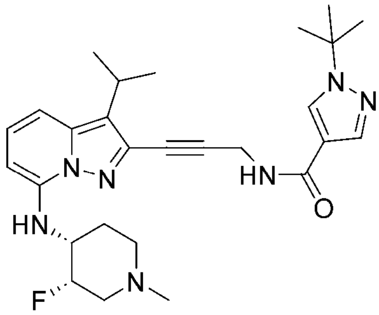
119 ,

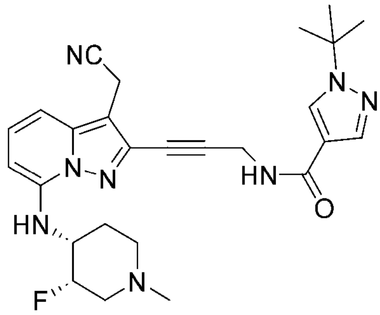
120 ,

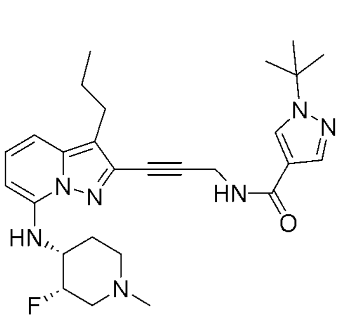
121 ,

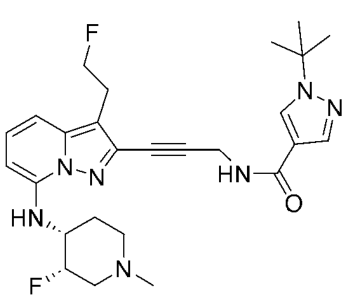
122 ,

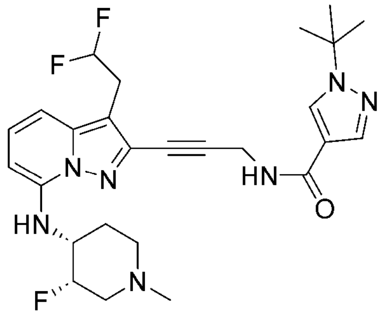
123 ,

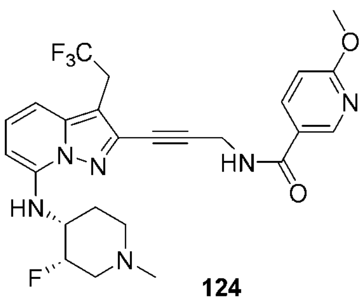
124 ,

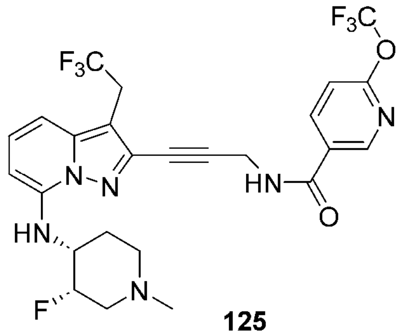
125 ,

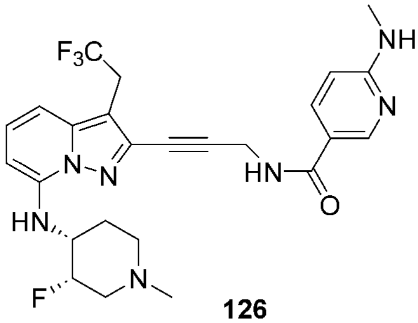
126 ,

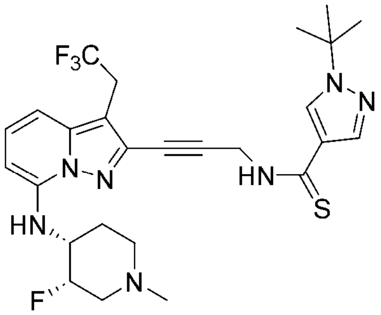
127 ,

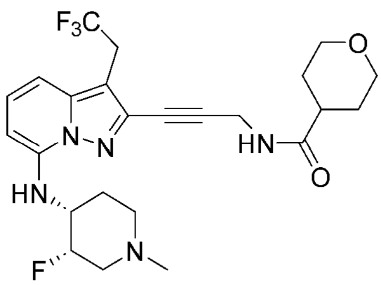
128 ,

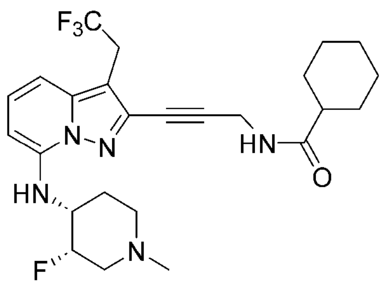
129 ,

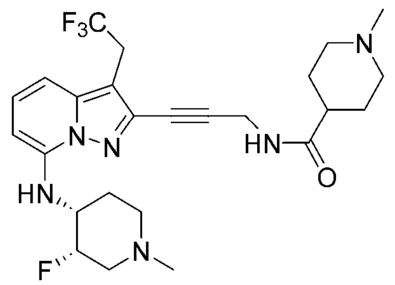
130 ,
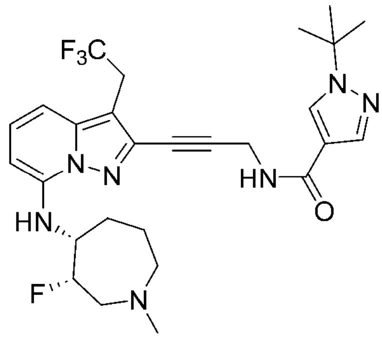
131 ,
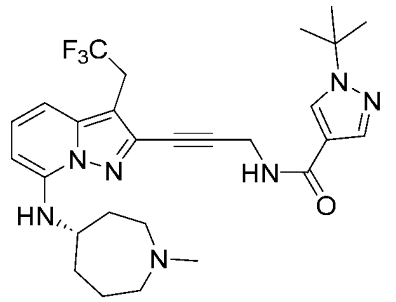
132 ,

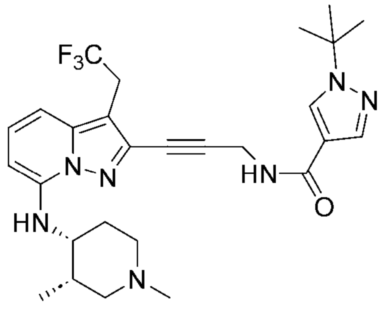
133 ,
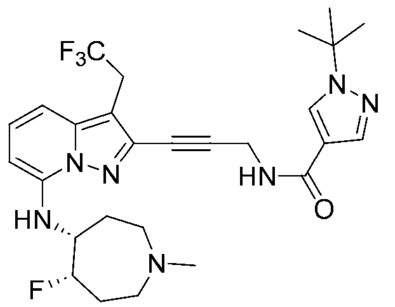
134 ,
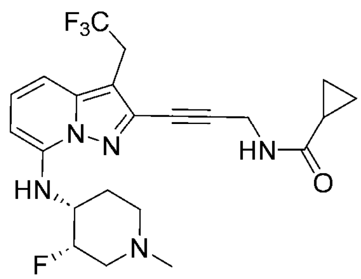
135 ,

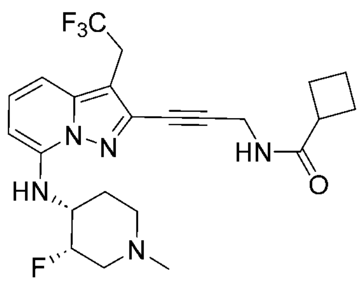
136 ,
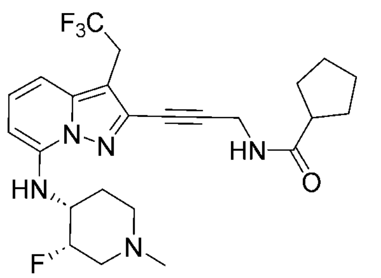
137 ,
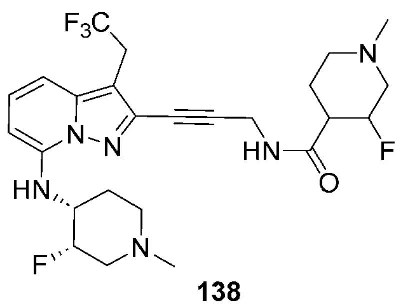
138 ,

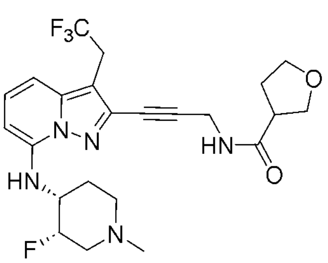
139 ,
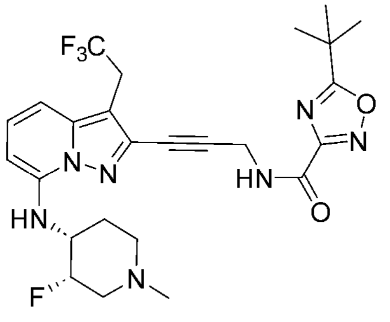
140 ,
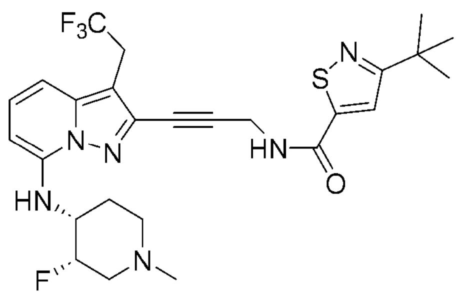
141 ,

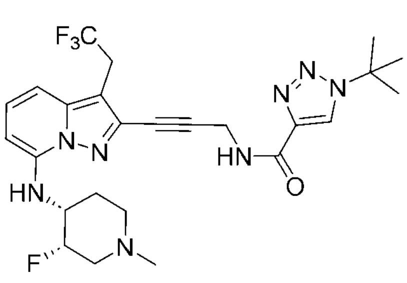
142 ,
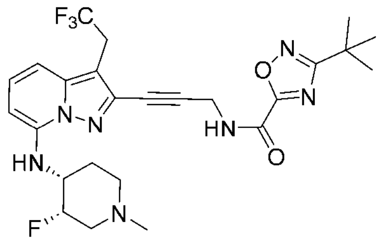
143 ,
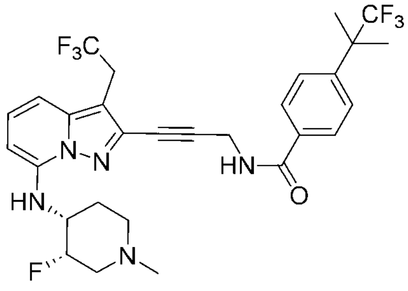
144 ,

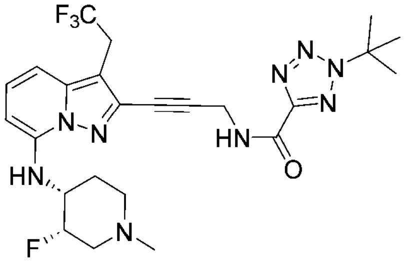
145 ,
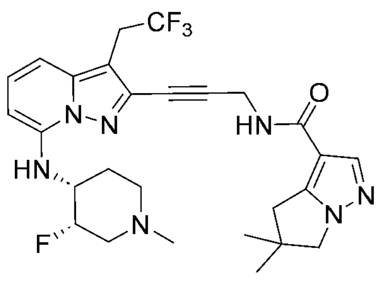
146 ,
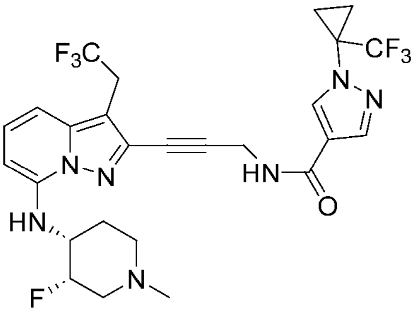
147 ,

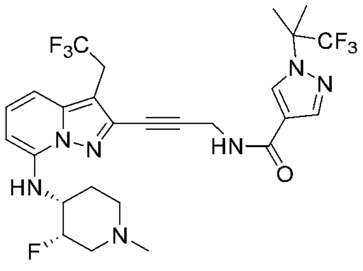
148 ,
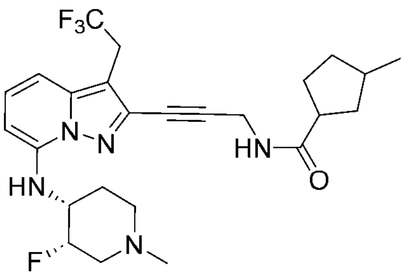
149 ,
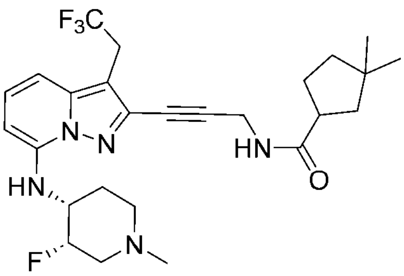
150 ,

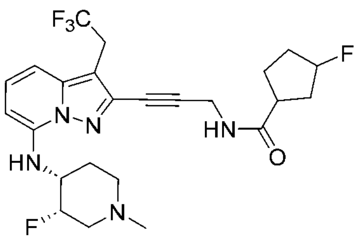
151 ,
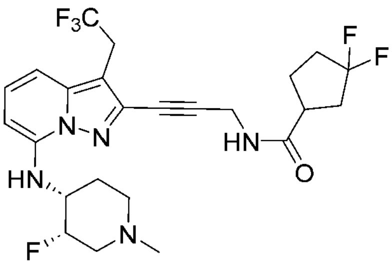
152 ,
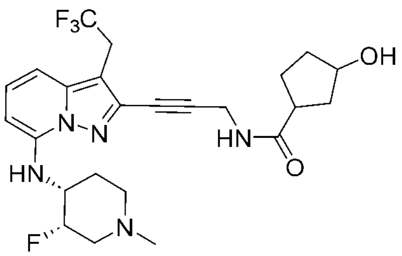
153 ,

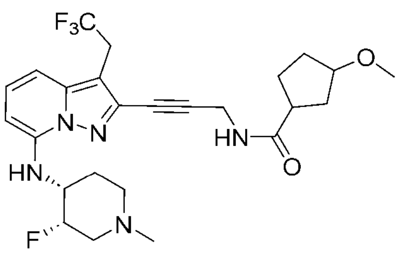
154 ,
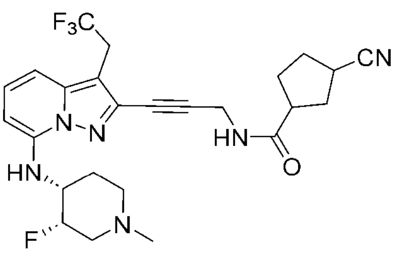
155 ,
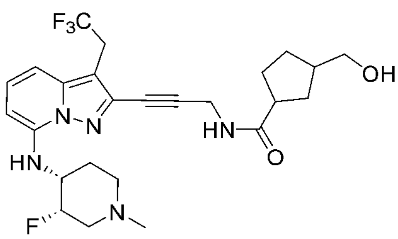
156 ,

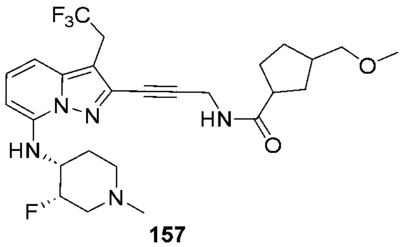

157,

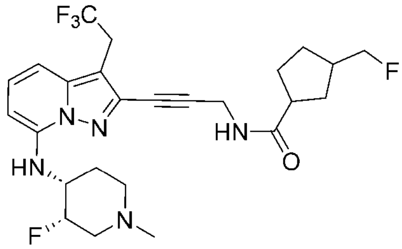

158,

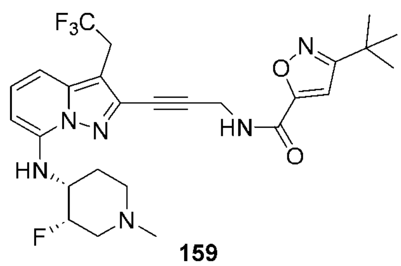

159,

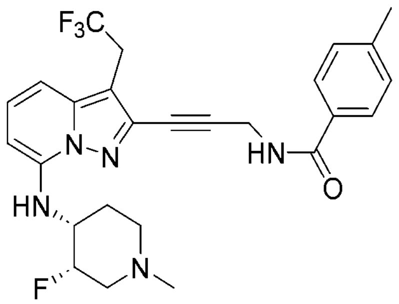

160,

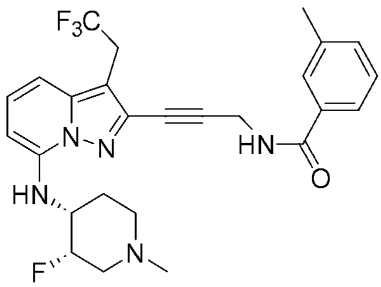

161,

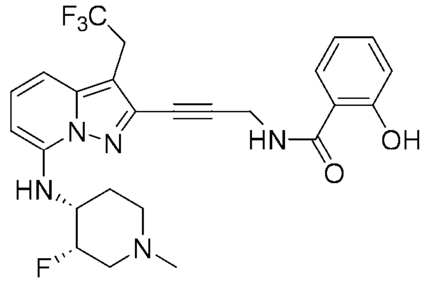

162,

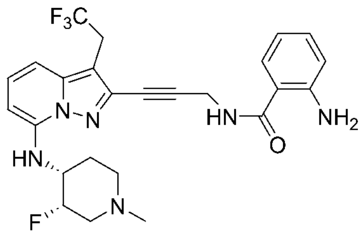

163,

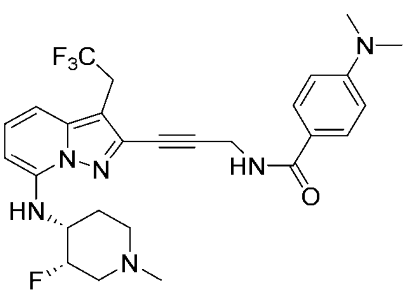

164,

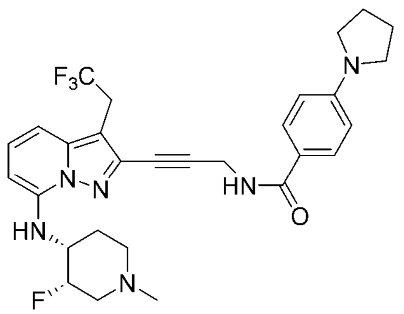

165,

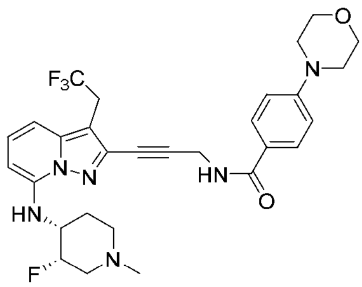

166,

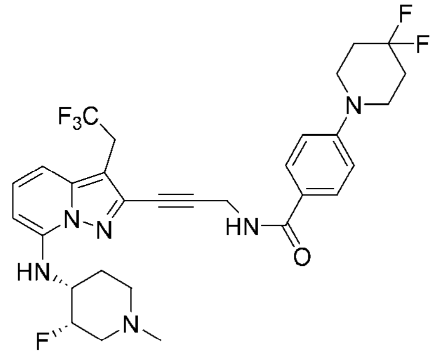

167,

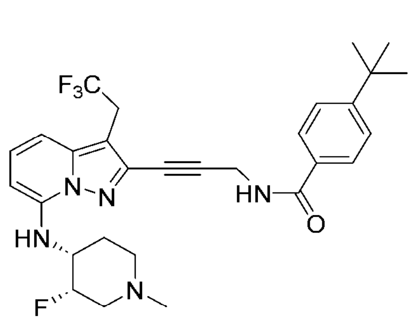

168,

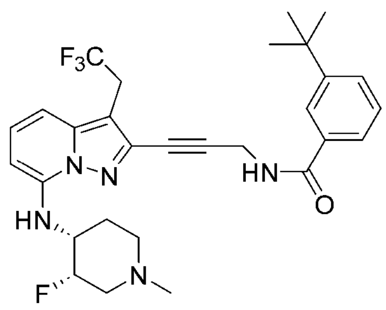

**169**,

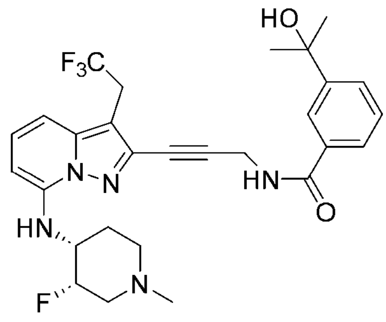

**170**,

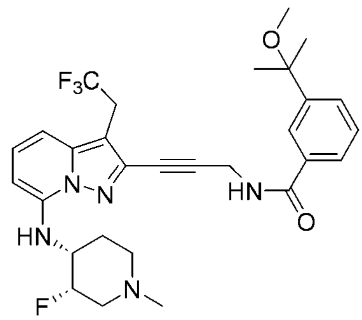

**171**,

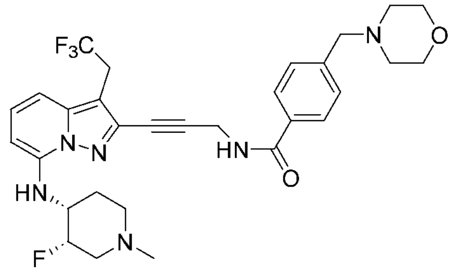

**172**,

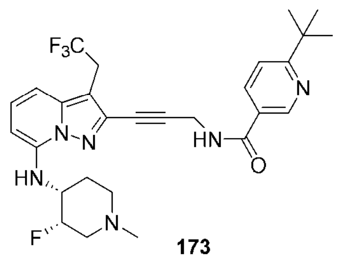

**173**,

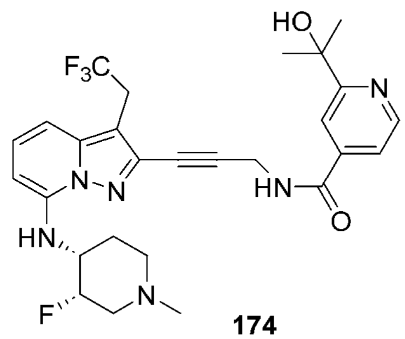

**174**,

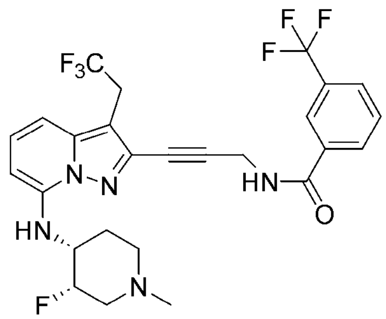

**175**,

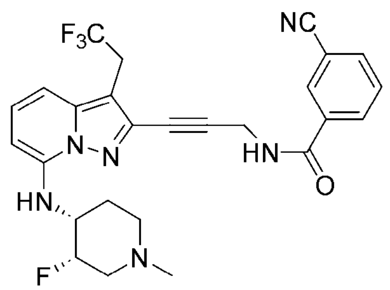

**176**,

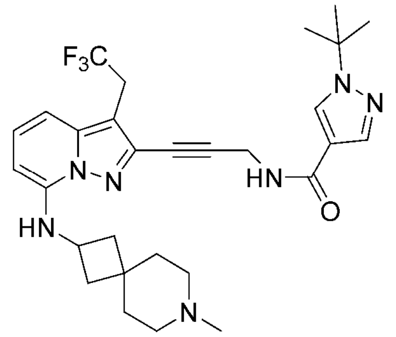

**177**,

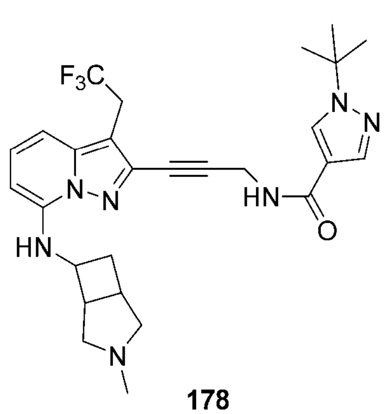

**178**,

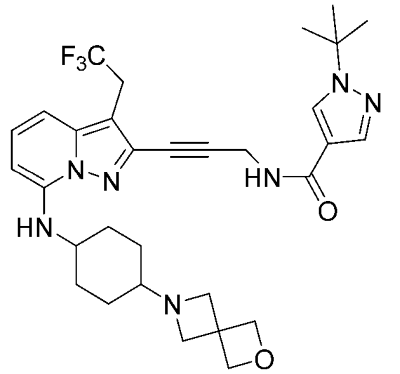

**179**,

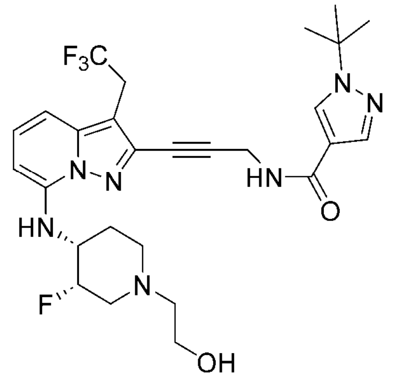

**180**,

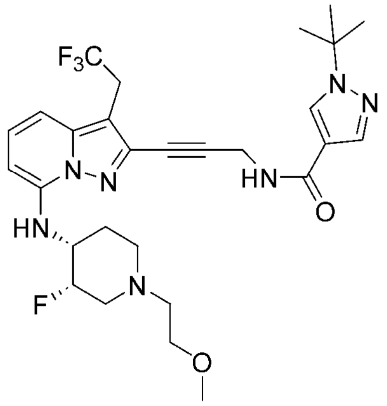
**181** ,

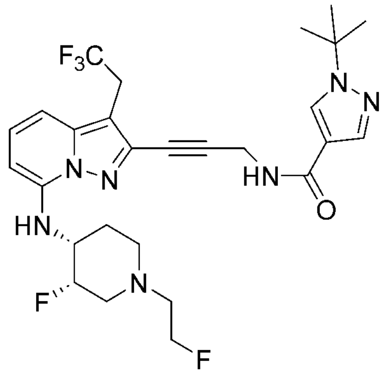
**182** ,

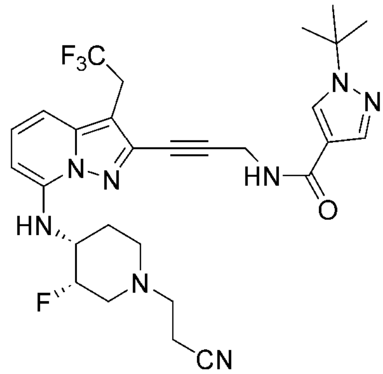
**183** ,

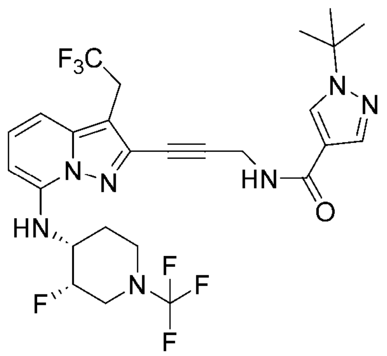
**184** ,

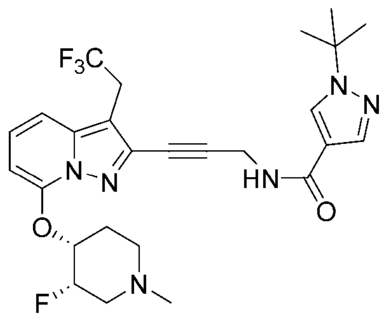
**185** ,

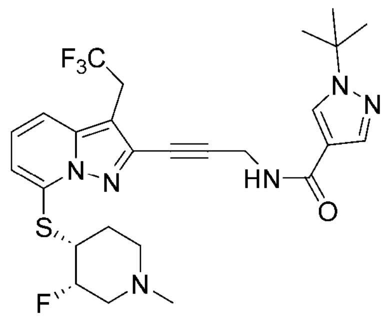
**186** ,

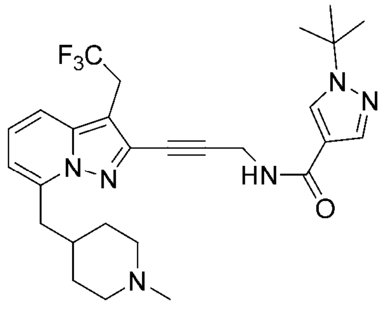
**187** ,

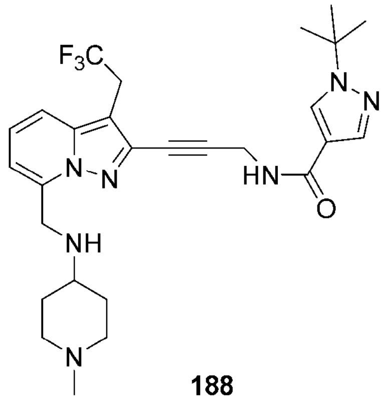
**188** ,

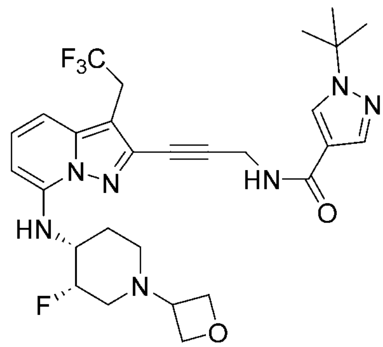
**189** ,

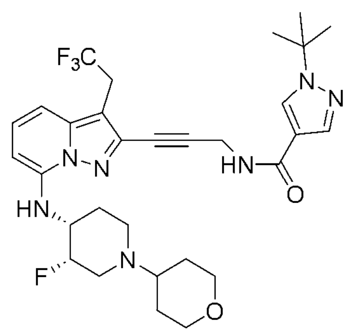
**190** ,

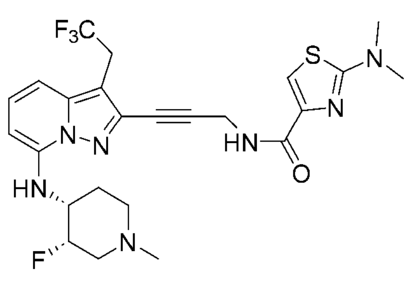
**191** ,

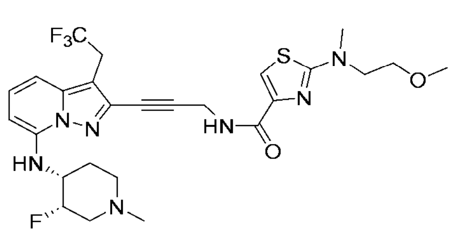
**192** ,

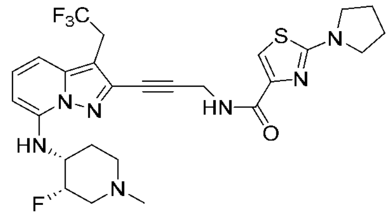

**193** ,

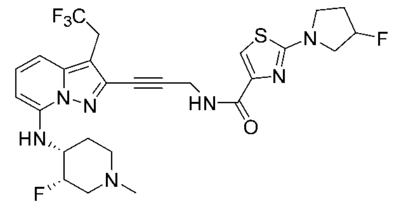

**194** ,

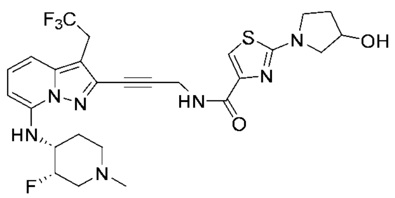

**195** ,

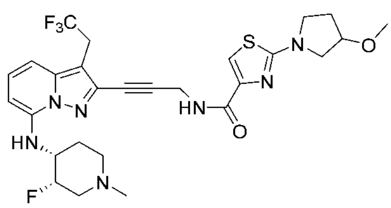

**196** ,

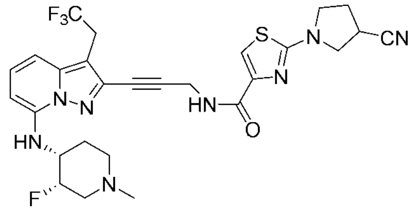

**197** ,

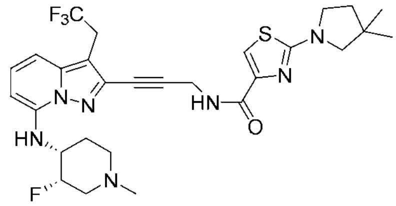

**198** ,

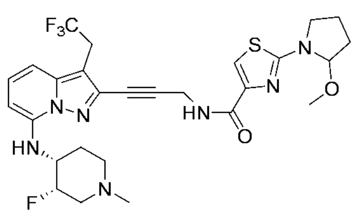

**199** ,

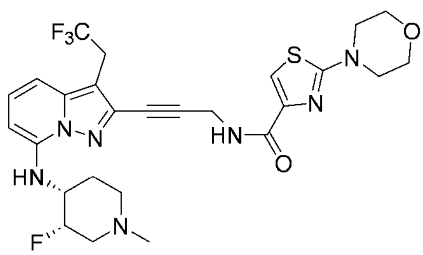

**200** ,

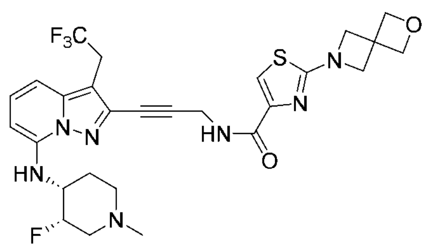

**201** ,

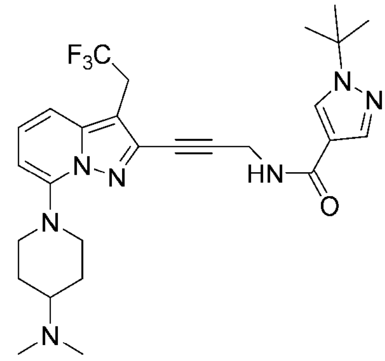

**202** ,

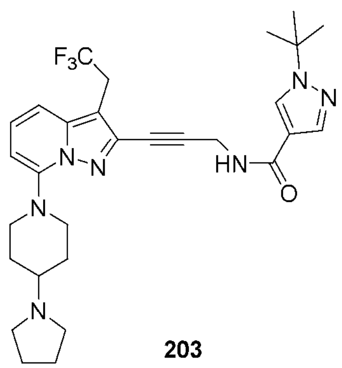

**203** ,

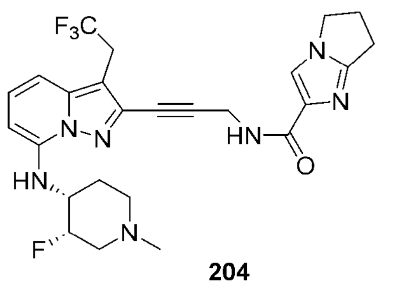

**204** ,

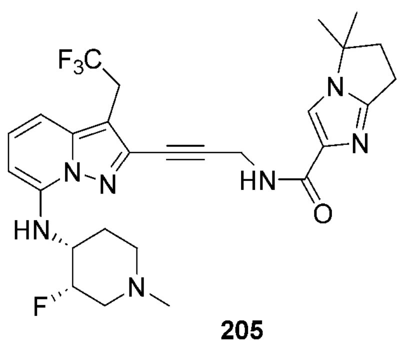

**205** ,

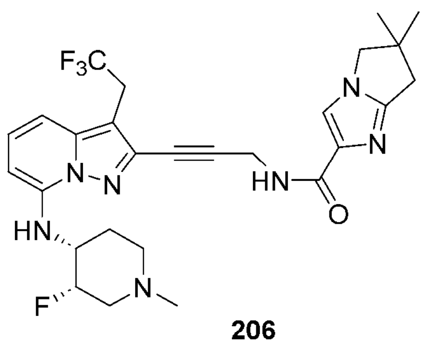

**206** ,

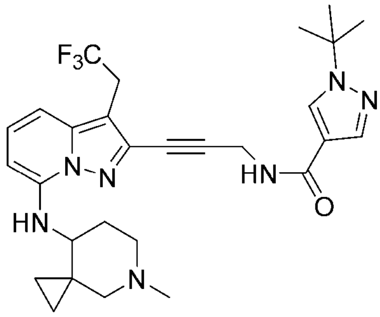

**207** ,

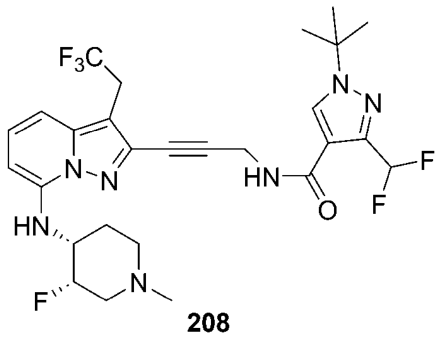
208 ,

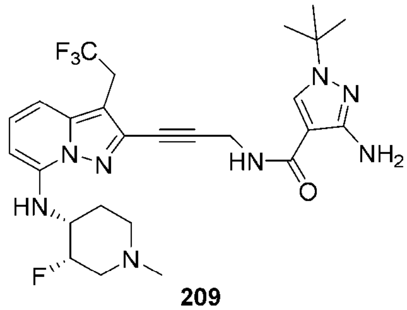
209 ,

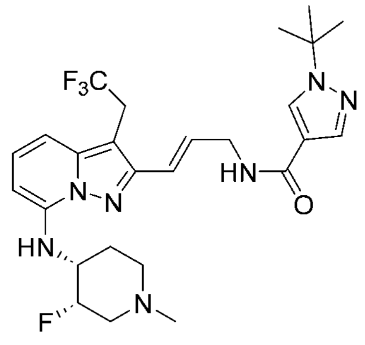
210 ,

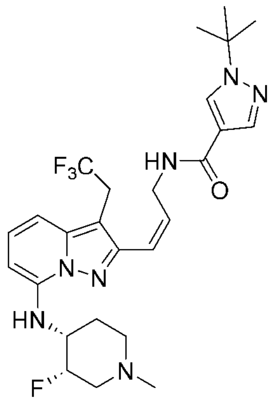
211 ,

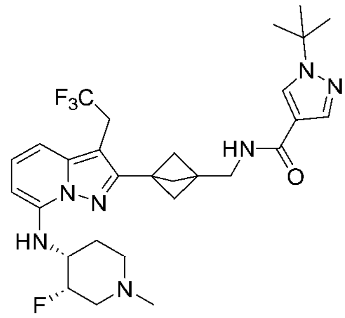
212 ,

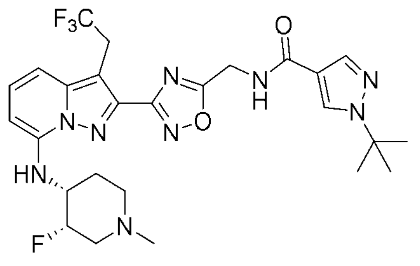
213 ,

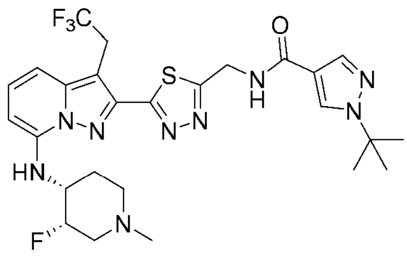
214 ,

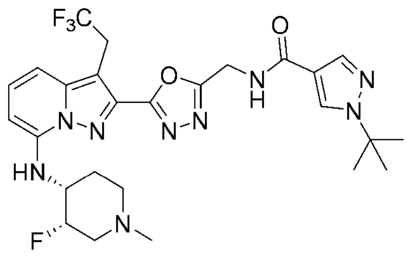
215 ,

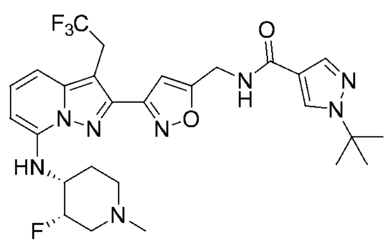
216 ,

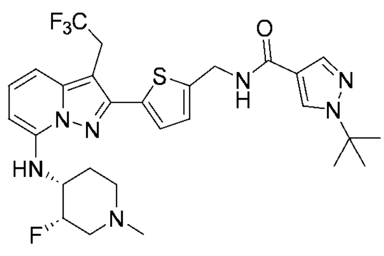
217 ,

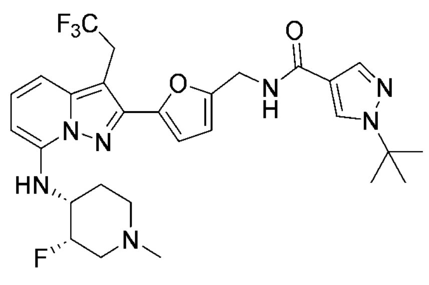
218 ,

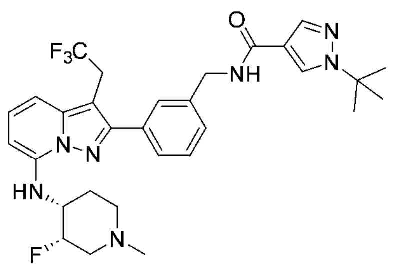
219 ,

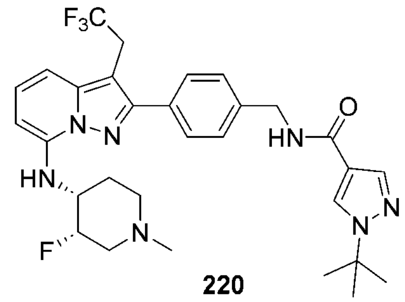
220,

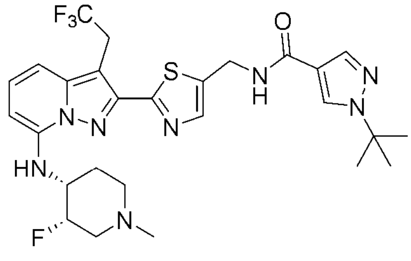
221,

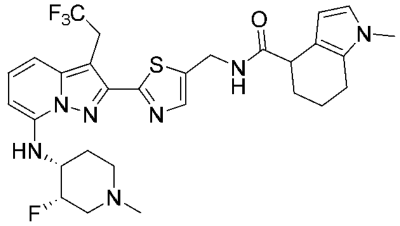
222,

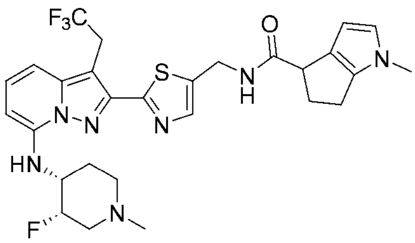
223,

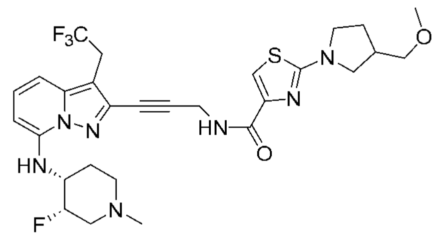
224,

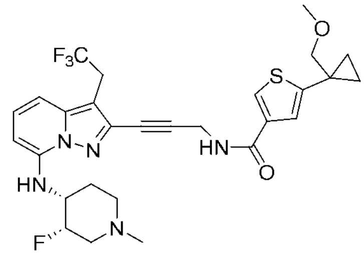
225,

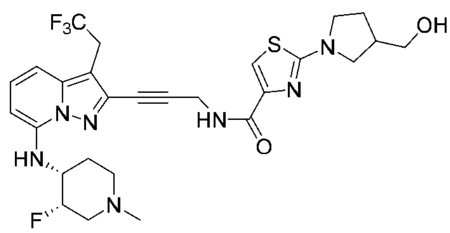
226,

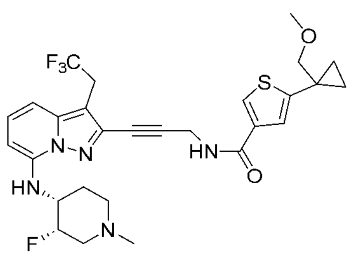
227,

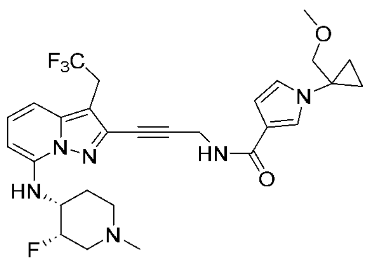
228,

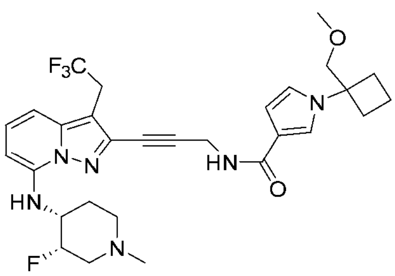
229,

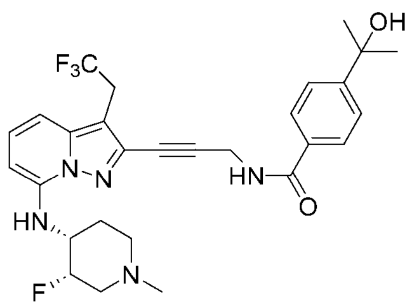
230,

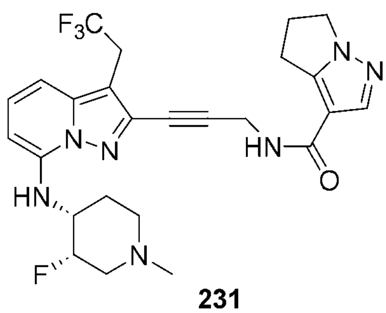
231,

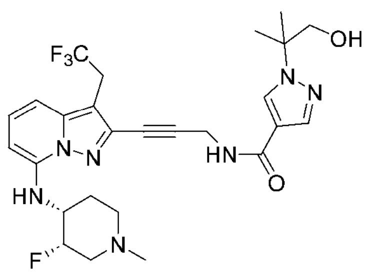
232
,
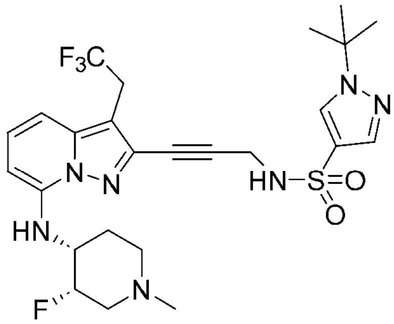
233
,
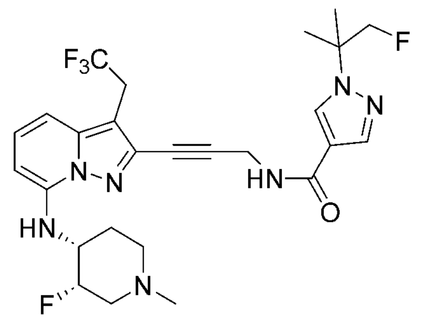
234
,

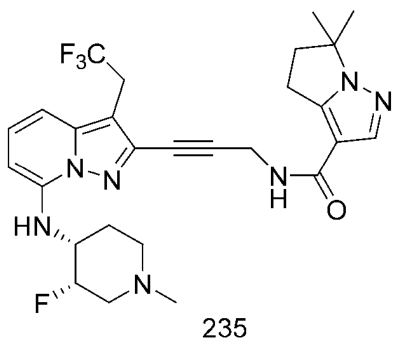
235
,
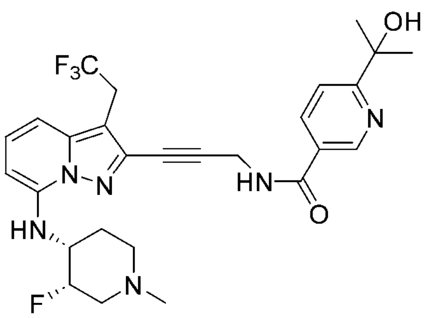
236
,
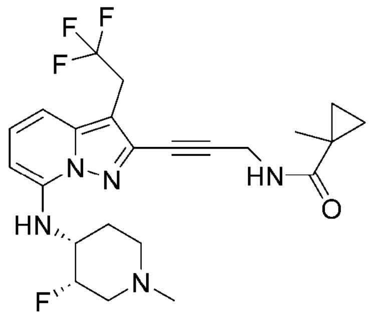
237
,

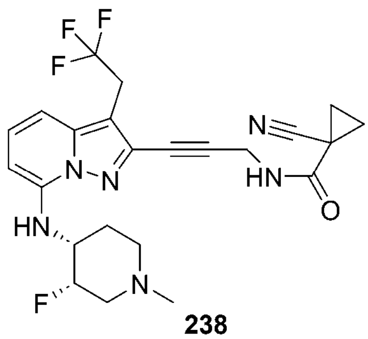
238
,
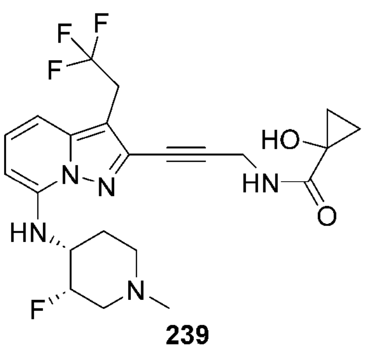
239
,
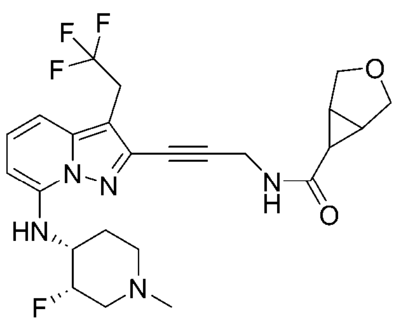
240
,

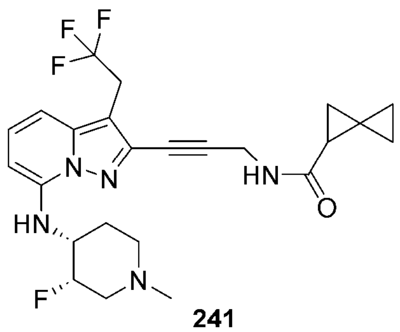
241
,
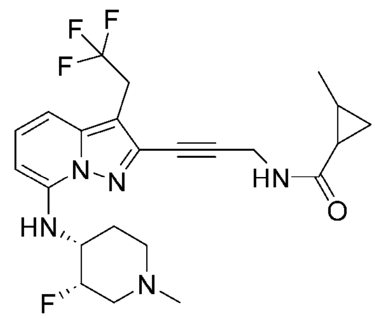
242
,
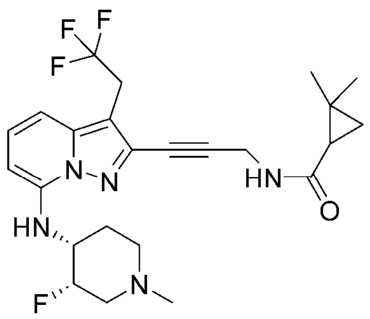
243
,

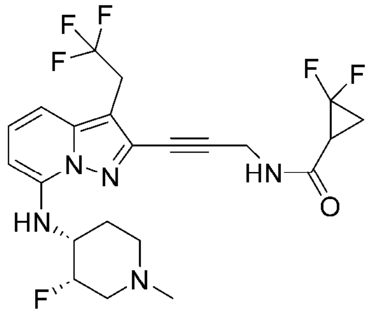
244 ,

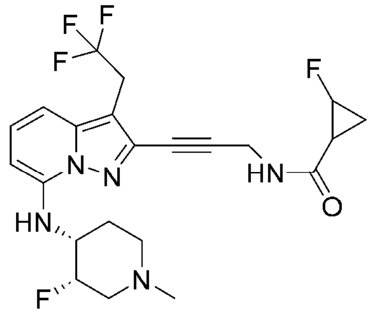
245 ,

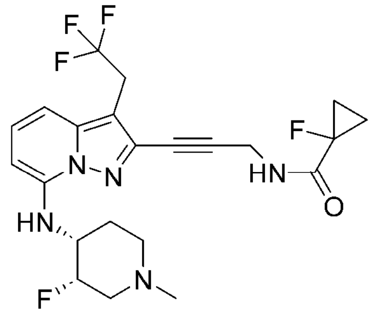
246 ,

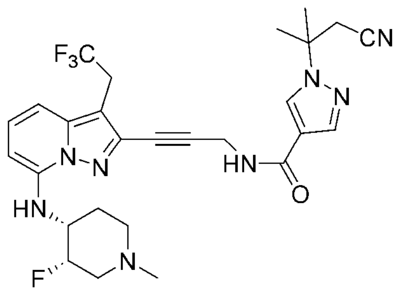
247 ,

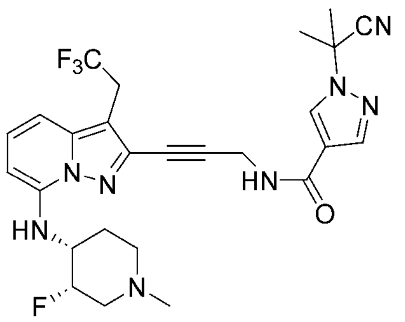
248 ,

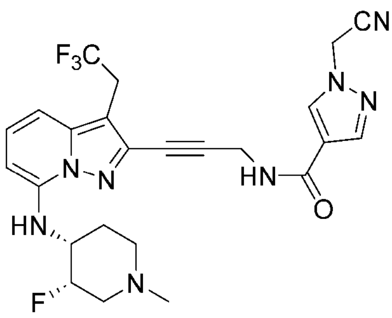
249 ,

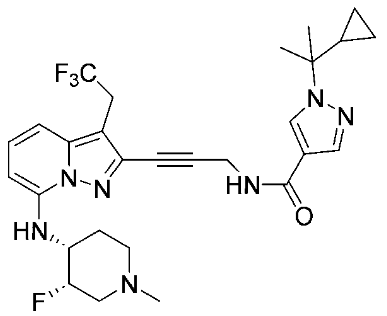
250 ,

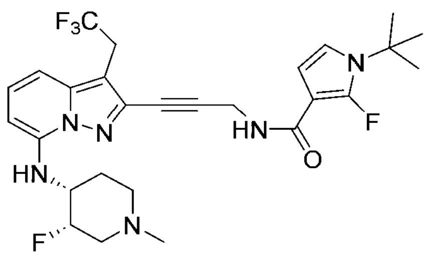
251 ,

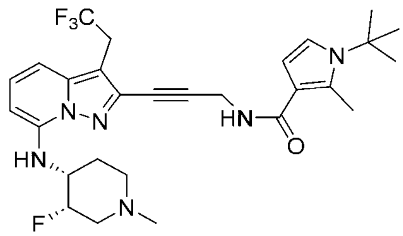
252 ,

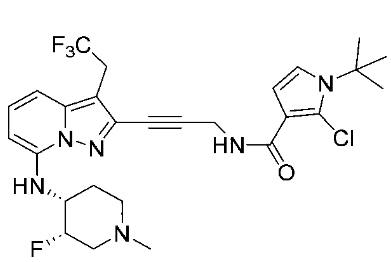
253 ,

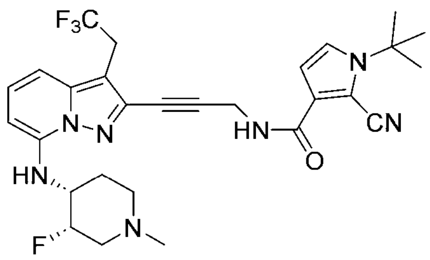
254 ,

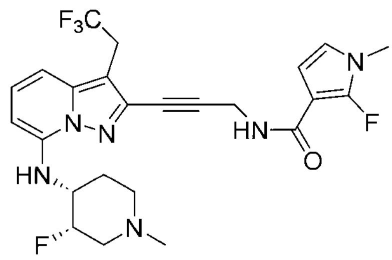
255 ,

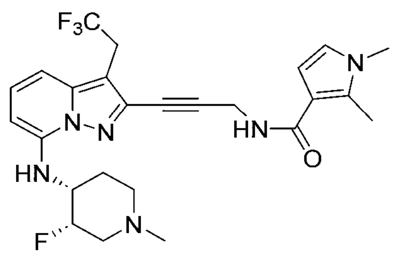
**256** ,

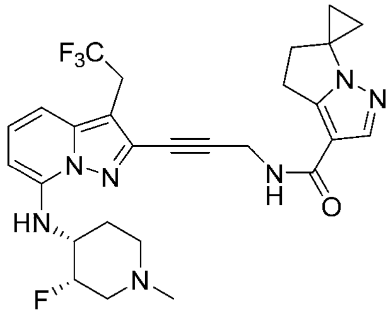
257 ,

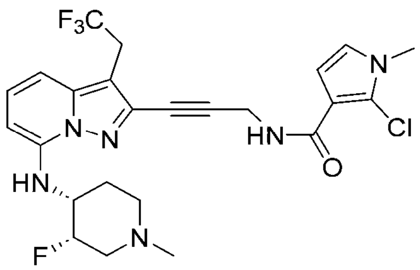
**258** ,

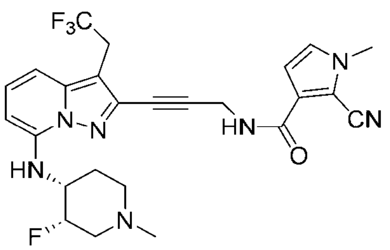
**259** ,

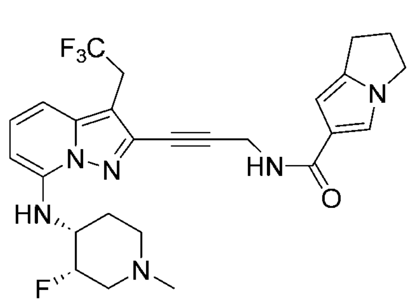
**260** ,

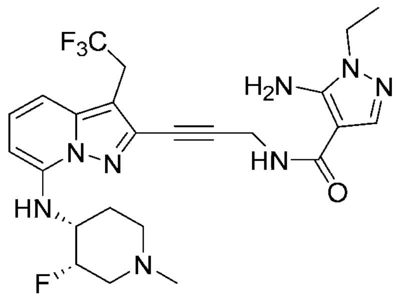
**261** ,

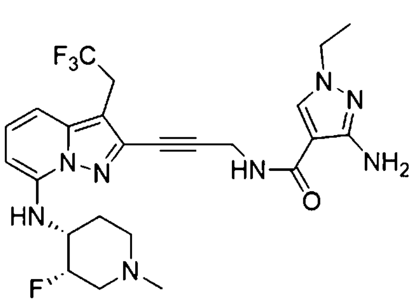
**262** ,

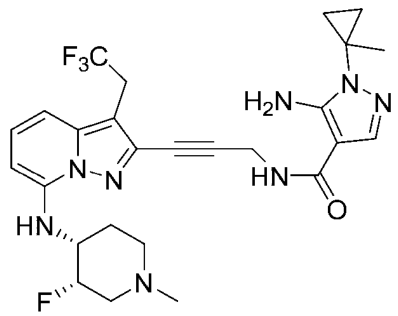
263 ,

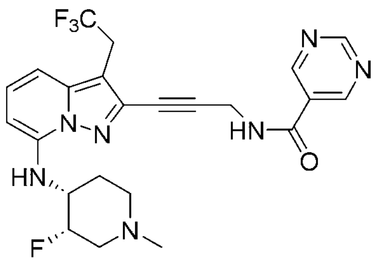
**264** ,

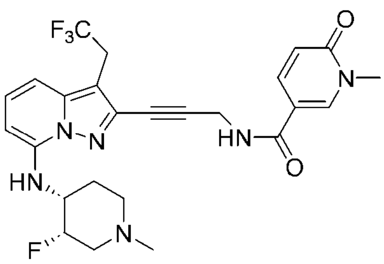
**265** ,

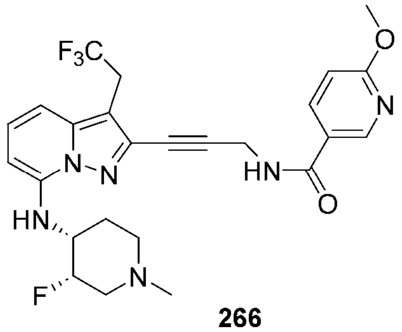
**266** ,

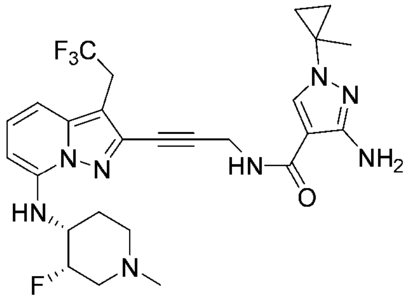
267 ,

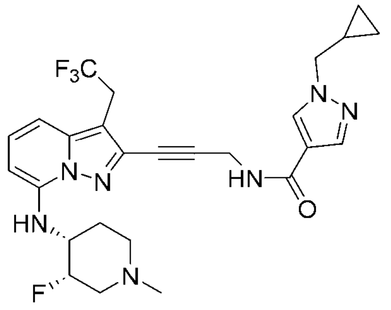
**268** ,

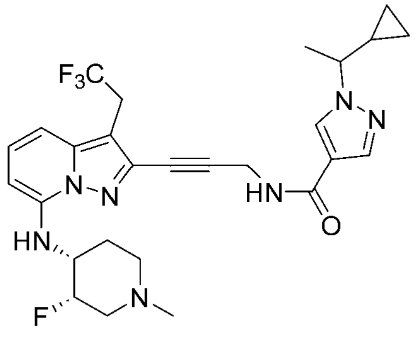
**269** ,

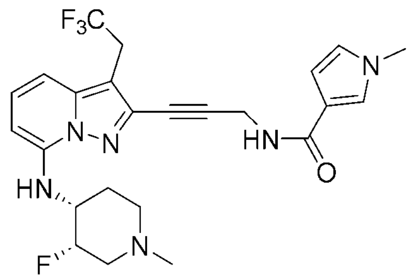
**270** ,

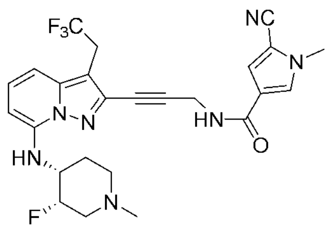
**271** ,

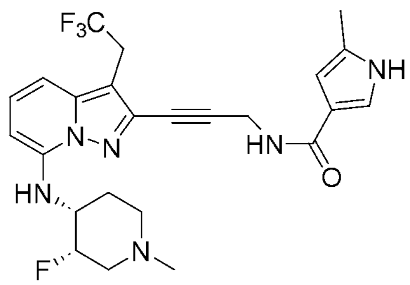
**272** ,

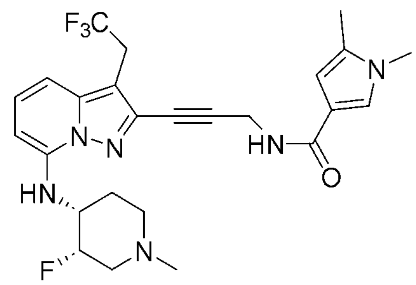
**273** ,

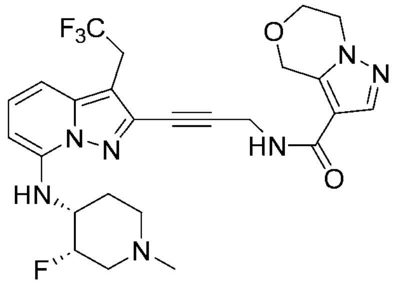
**274** ,

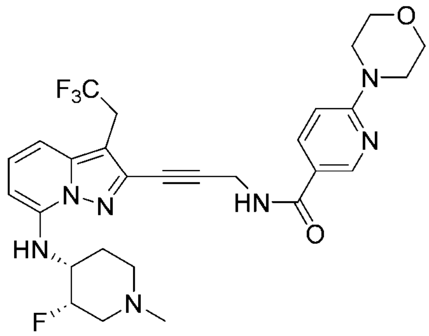
**275** ,

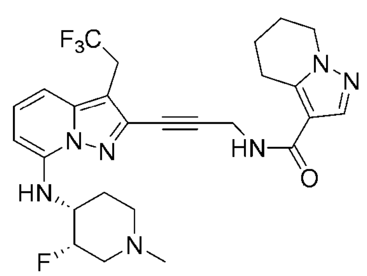
**276** ,

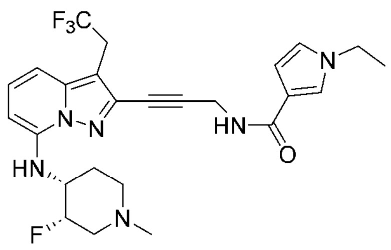
**277** ,

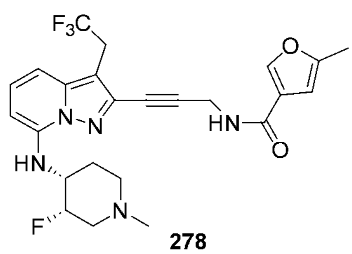
**278** ,

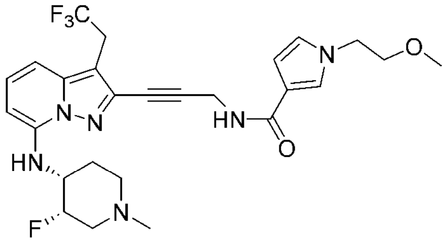
**279** ,

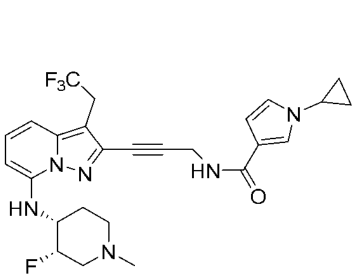
280,

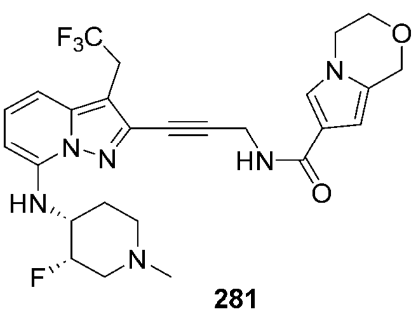
281,

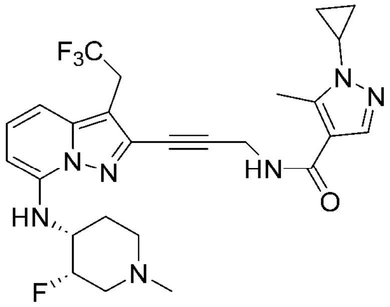
282,

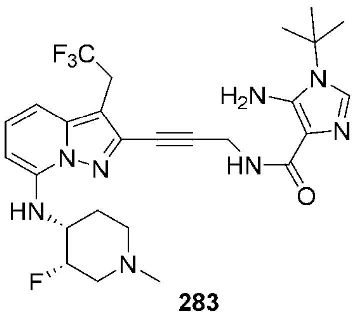
283,

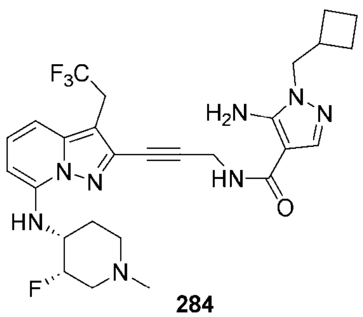
284,

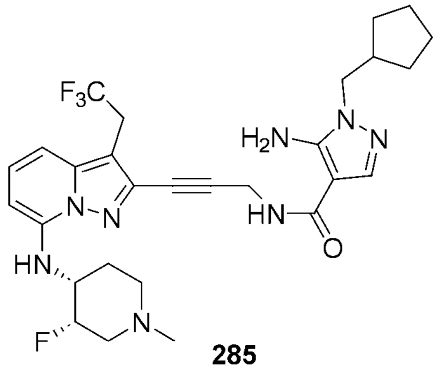
285,

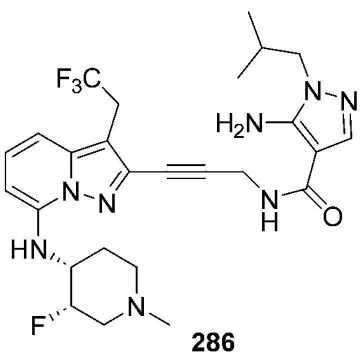
286,

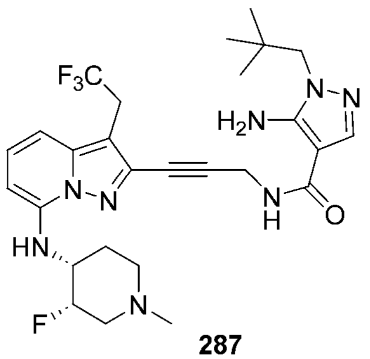
287,

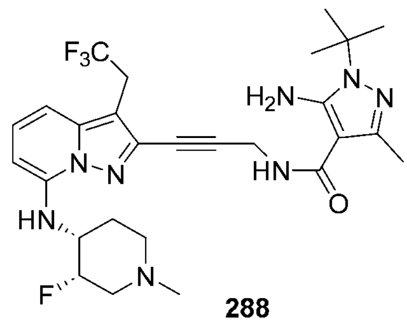
288,

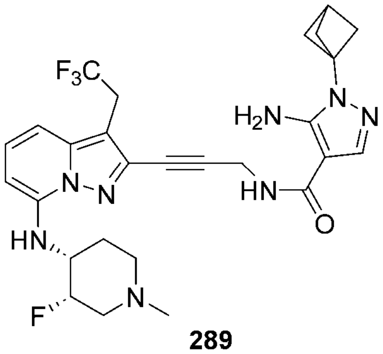
289,

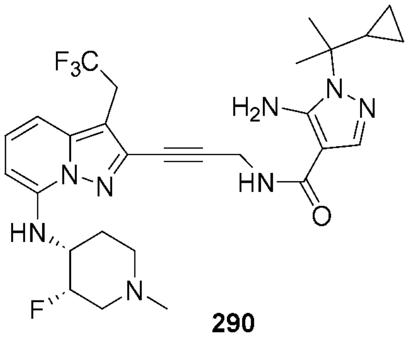
290,

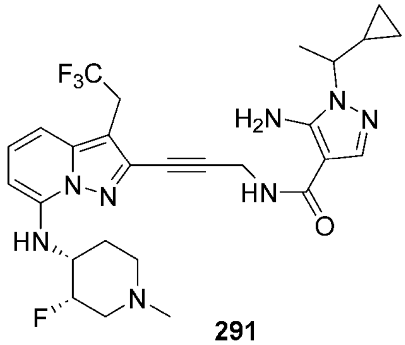
291,

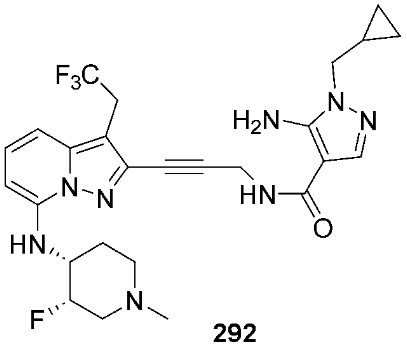
292 ,
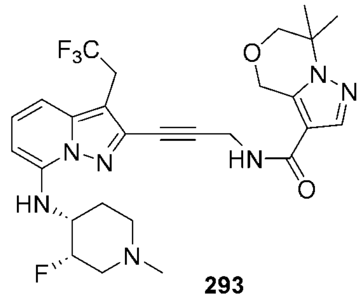
293 ,
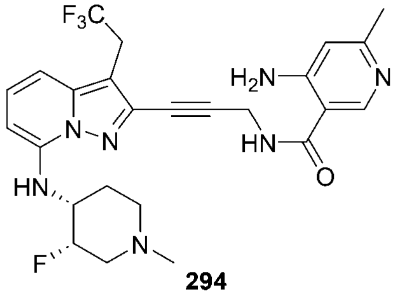
294 ,

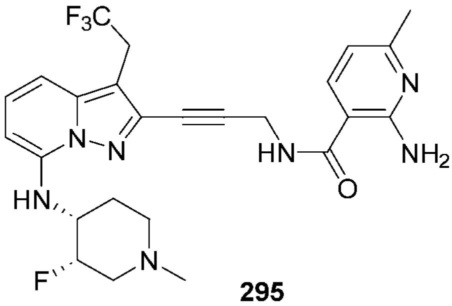
295 ,
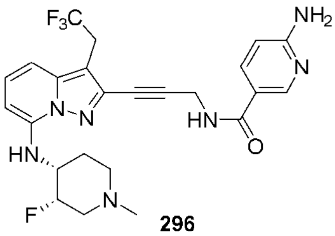
296 ,
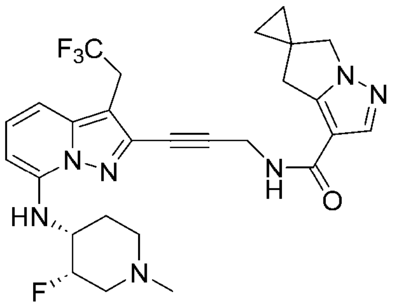
297 ,

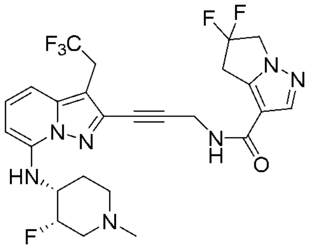
298 ,
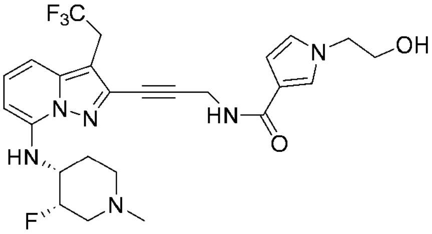
299 ,
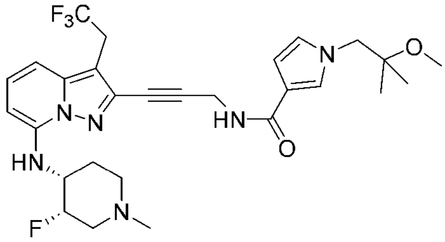
300 ,

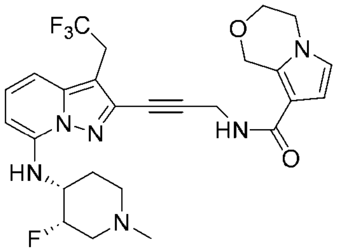
301 ,
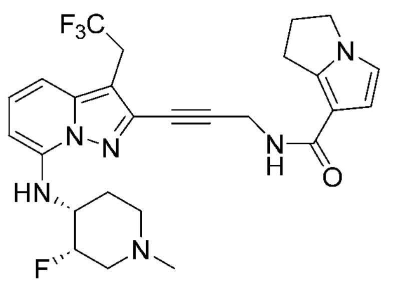
302 ,
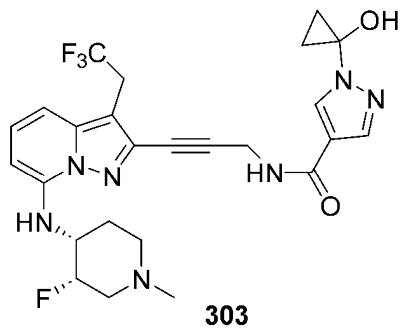
303 ,

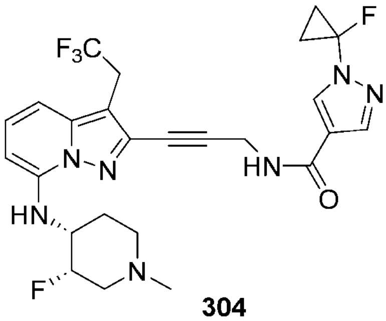
**304** ,
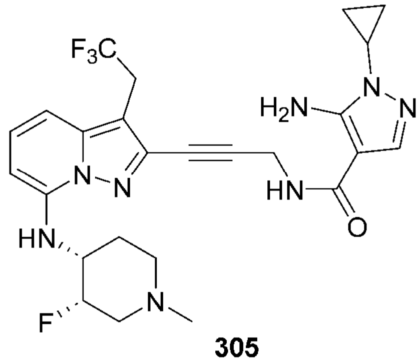
**305** ,
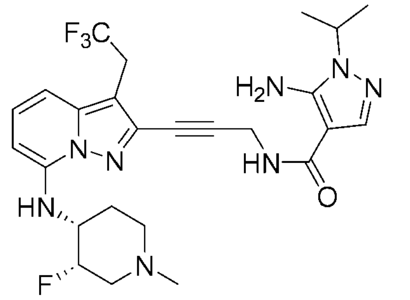
**306** ,

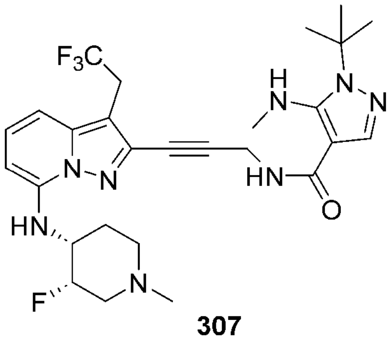
**307** ,
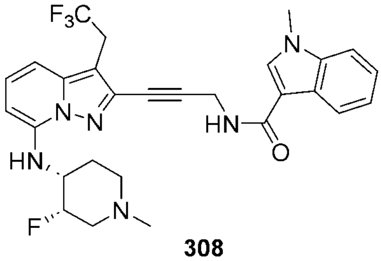
**308** ,
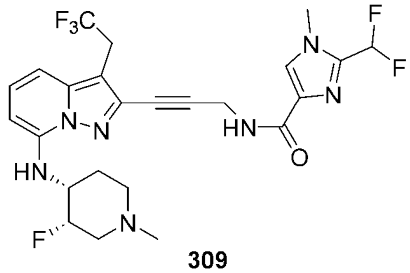
**309** ,

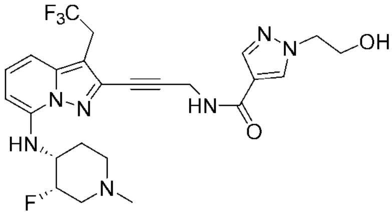
**310** ,
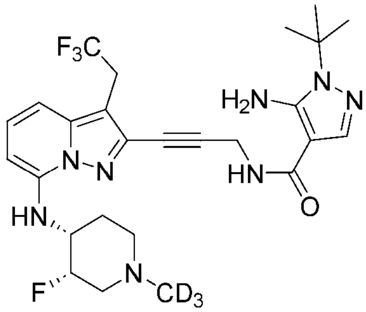
**311** ,
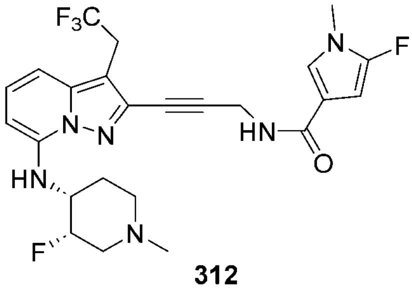
**312** ,

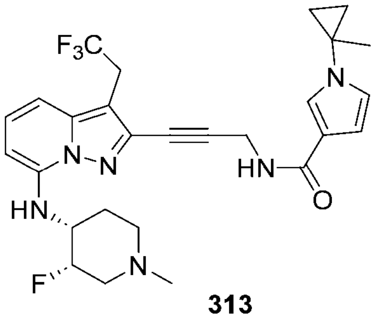
**313** ,
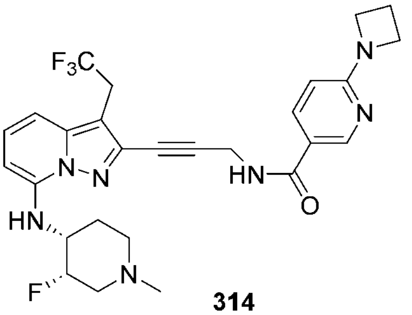
**314** ,
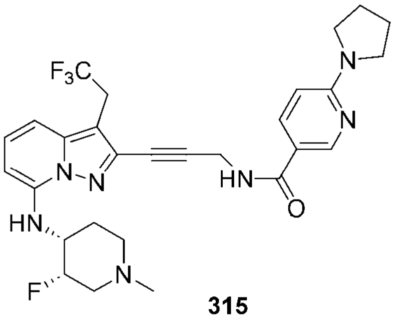
**315** ,

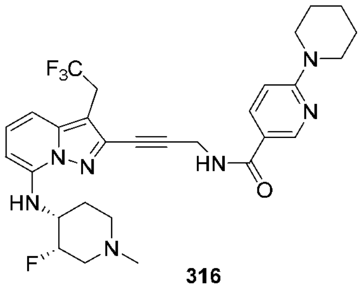
316,

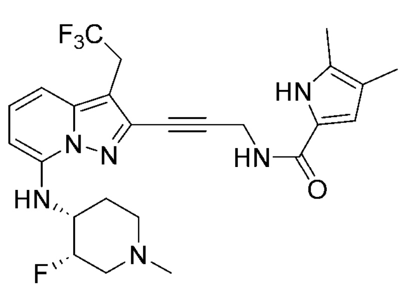
317,

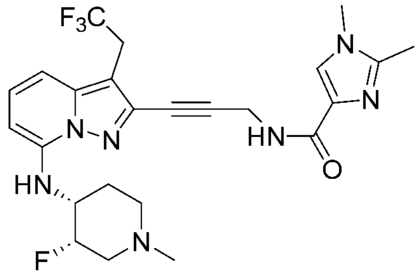
318,

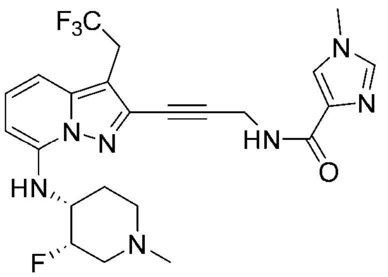
319,

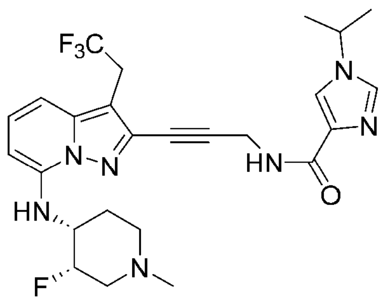
320,

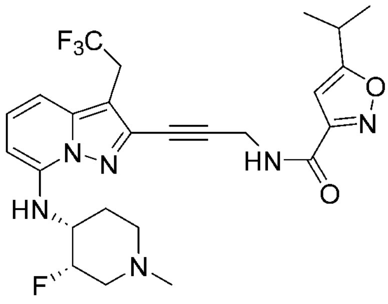
321,

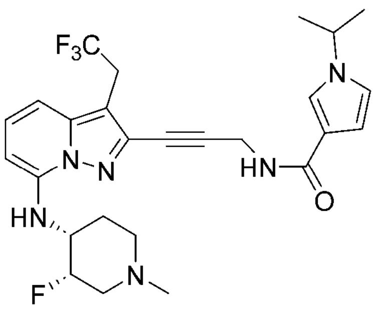
322,

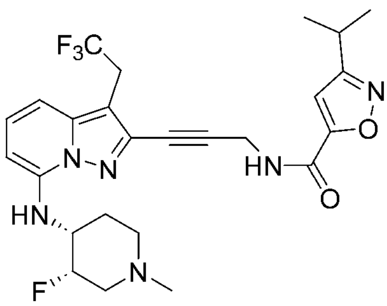
323,

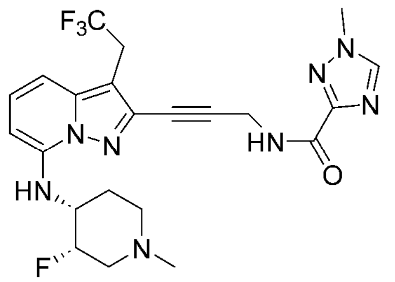
324,

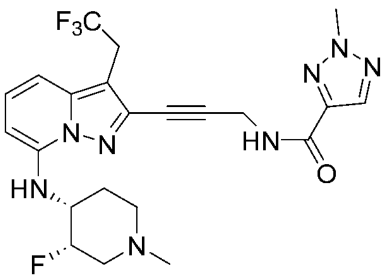
325,

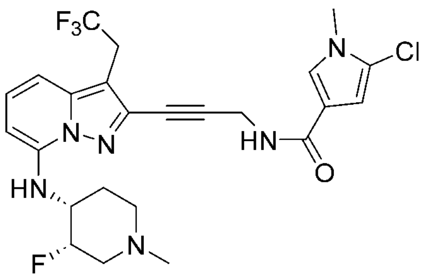
326,

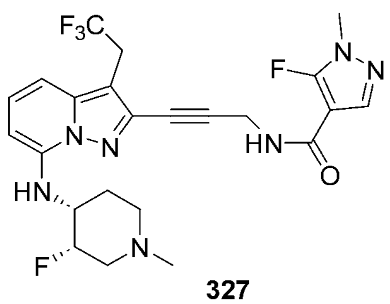
327,

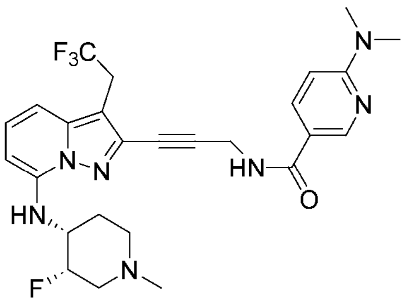

**328**,

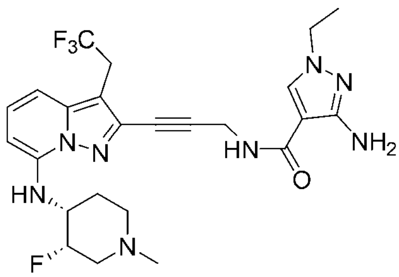

**329**,

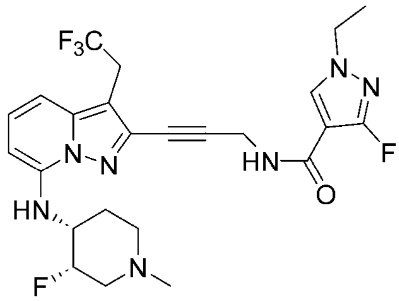

**330**,

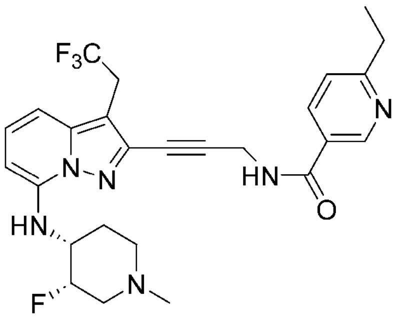

**331**,

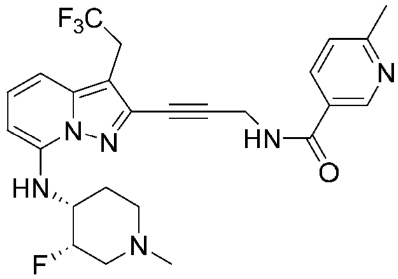

**332**,

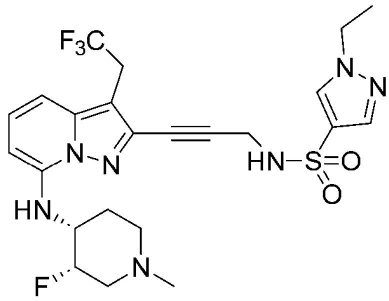

**333**,

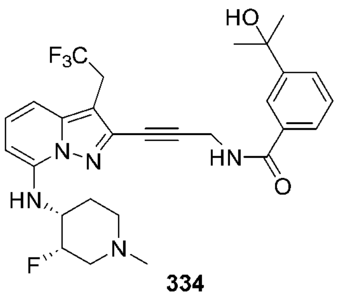

**334**,

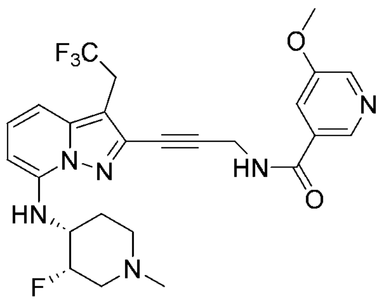

**335**,

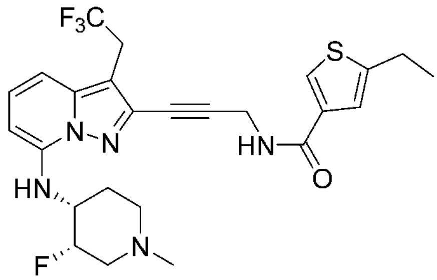

**336**,

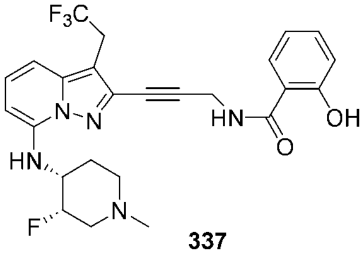

**337**,

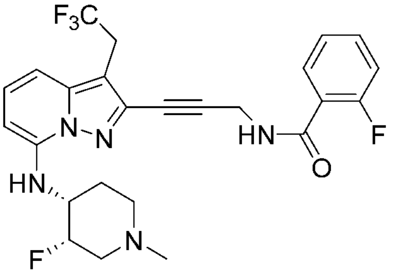

**338**,

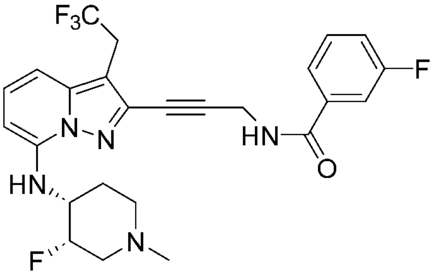

**339**,

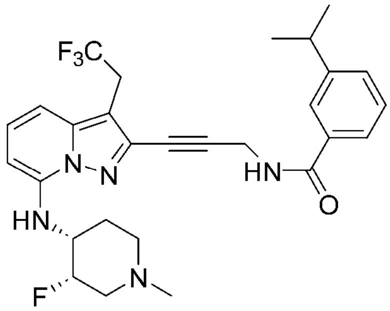
**340** ,

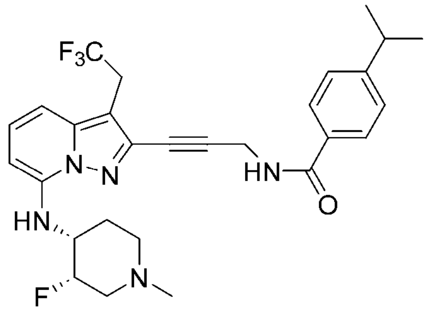
**341** ,

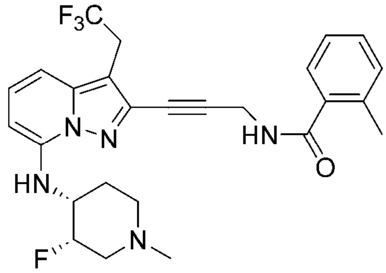
**342** ,

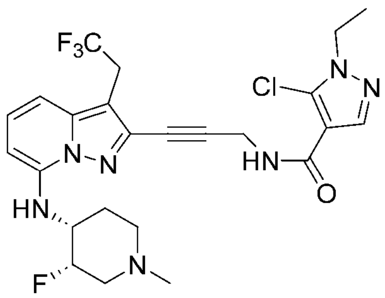
**343** ,

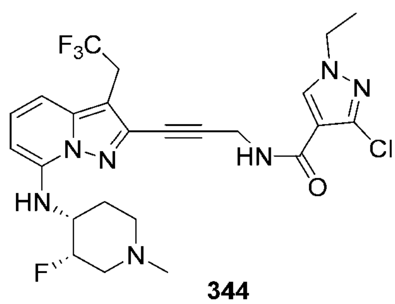
**344** ,

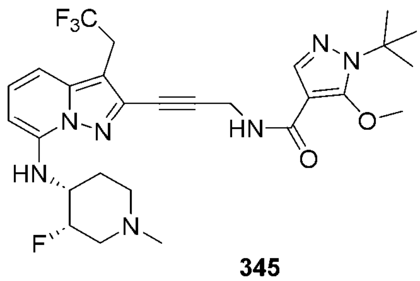
**345** ,

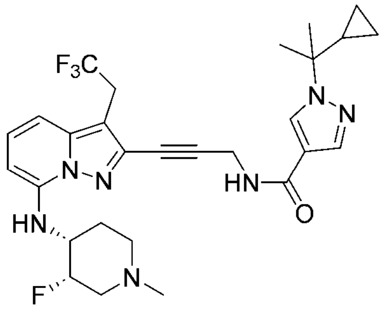
**346** ,

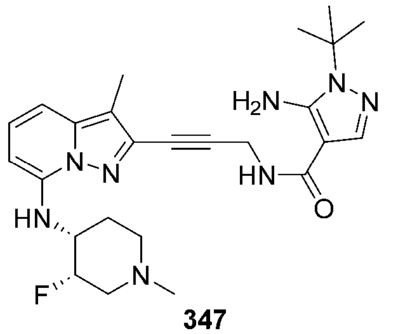
**347** ,

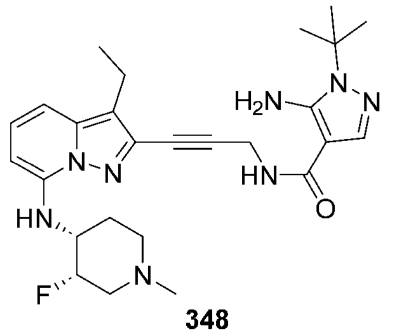
**348** ,

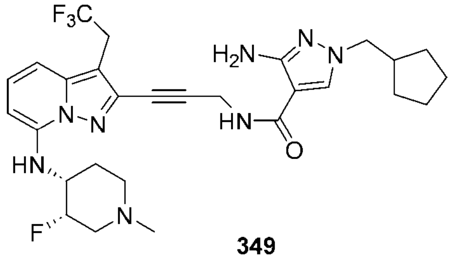
**349** ,

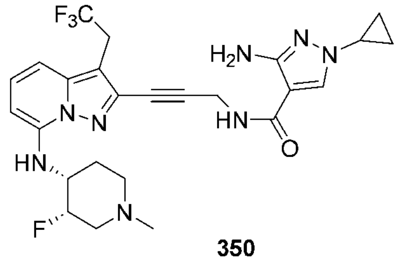
**350** ,

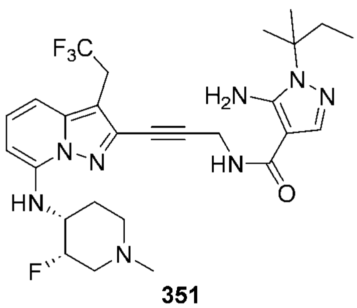
**351** ,

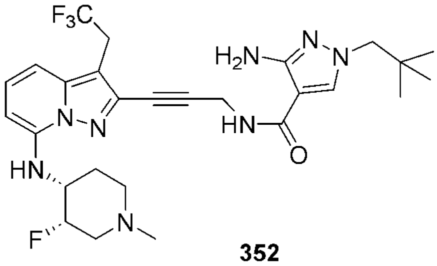
352 ,
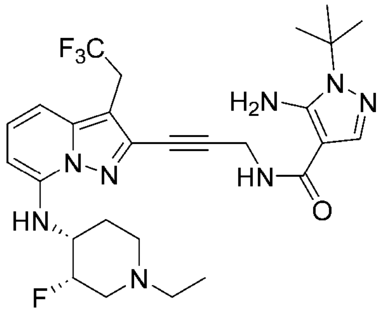
353 ,
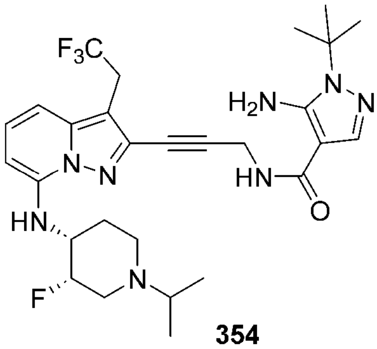
354 ,

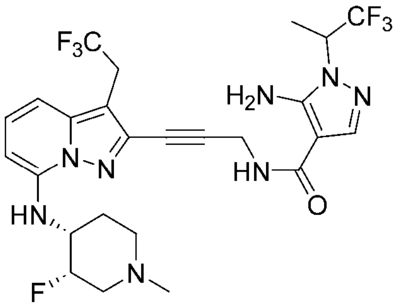
355 ,
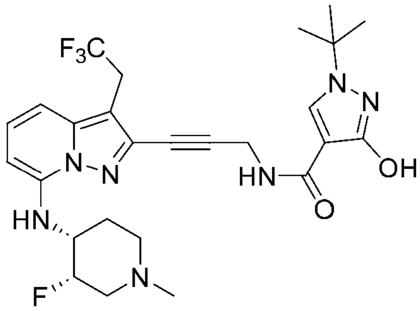
356 ,
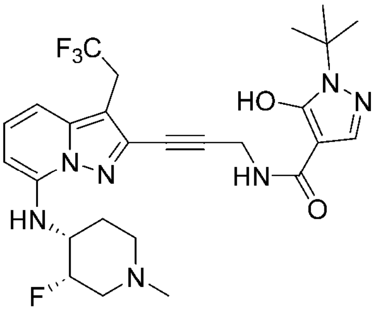
357 ,

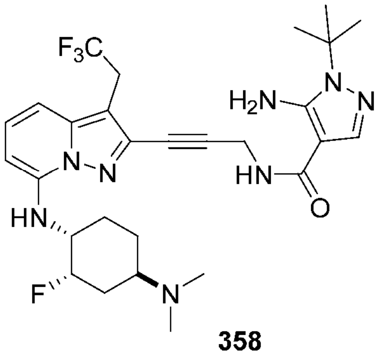
358 ,
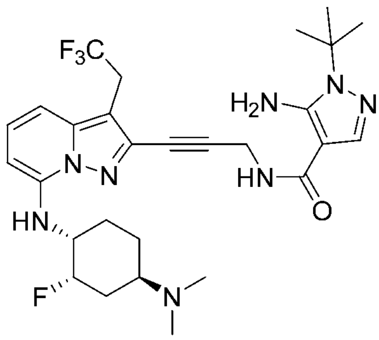
,
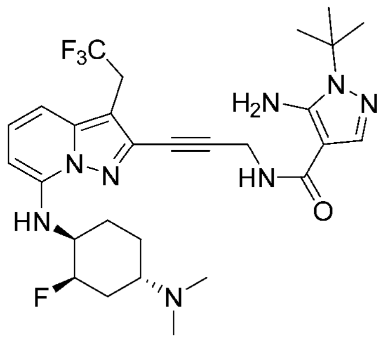
,

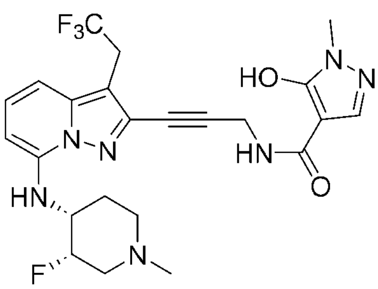
359 ,
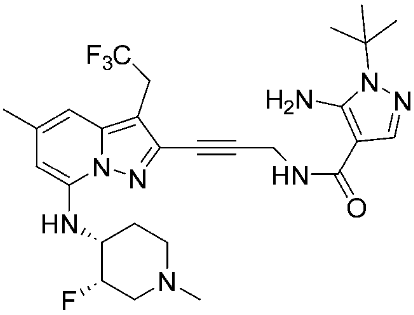
360 ,
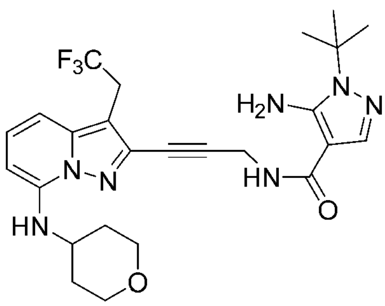
361 ,

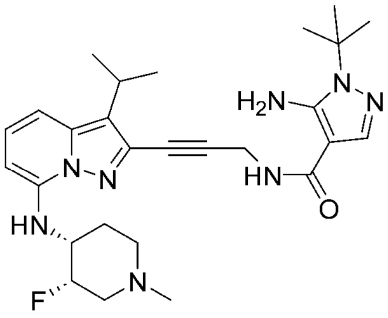

**362** ,

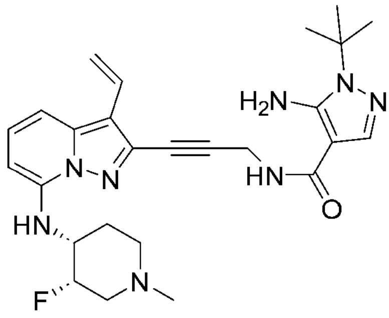

**363** ,

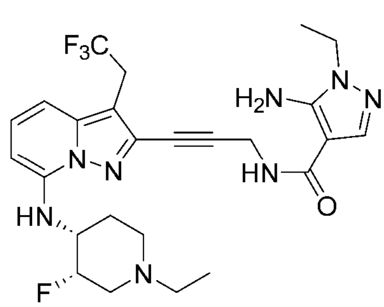

**364** ,

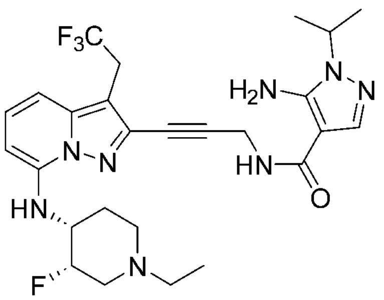

**365** ,

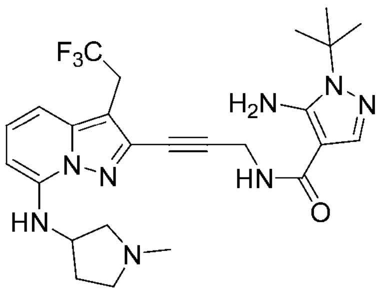

**366** ,

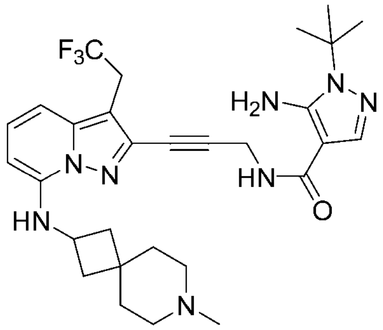

**367** ,

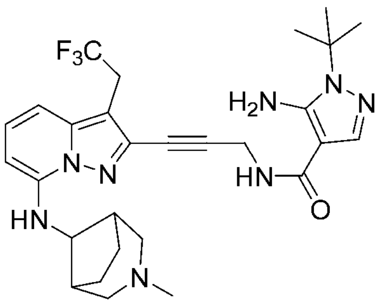

**368** ,

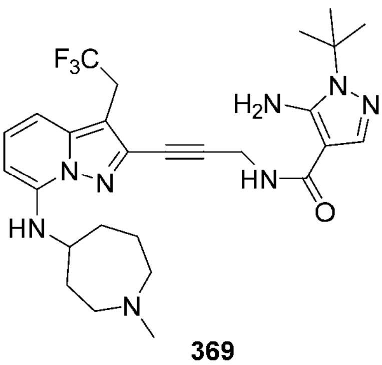

**369** ,

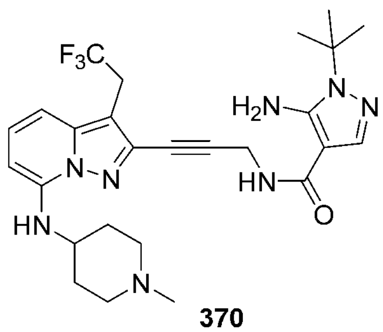

**370** ,

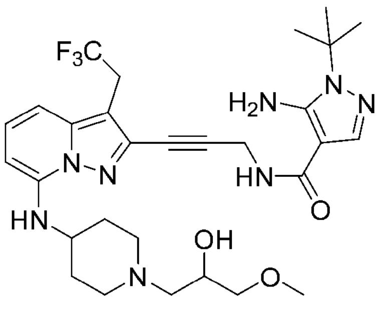

**371** ,

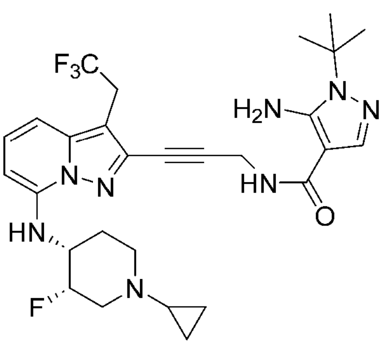

**372** ,

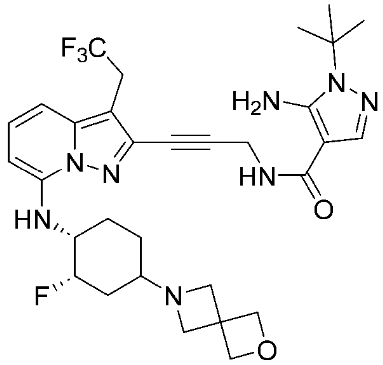

**373** ,

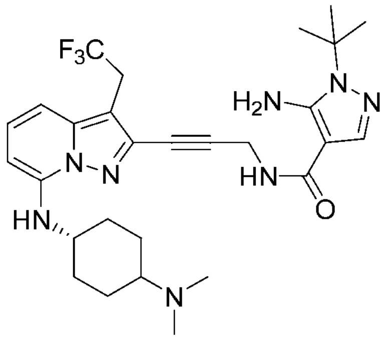

374

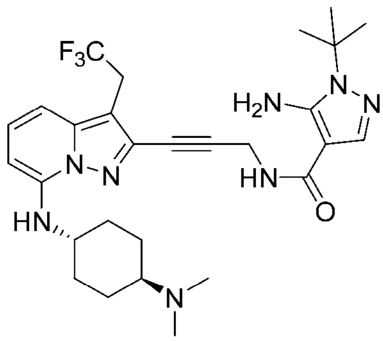

,

or

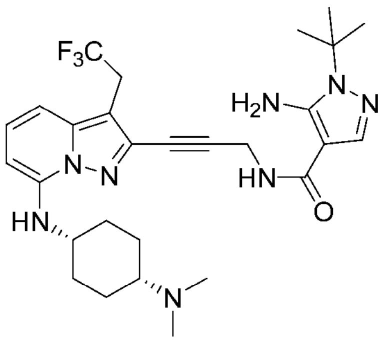

.

The present invention further provides a method for preparing the compound shown as formula (I), which comprises the following step: allowing compound 1 to react with compound 2 to give the compound shown as formula (I);

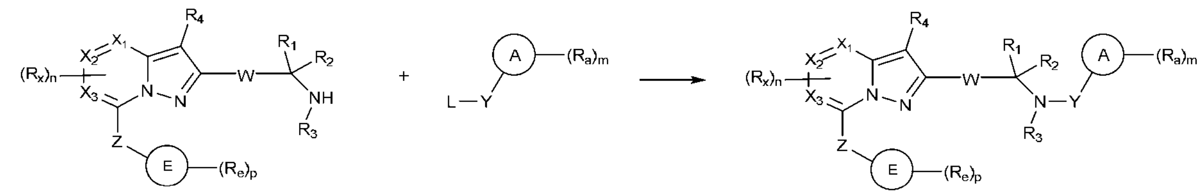

1 2 (I)

wherein ring A, ring E, $X_1$, $X_2$, $X_3$, W, Y, Z, $R_1$, $R_2$, $R_3$, $R_4$, $R_a$, $R_e$, $R_x$, m, n, and p are as defined in the present invention.

The present invention further provides a pharmaceutical composition comprising a therapeutically effective amount of at least one of the compound shown as formula (I), and a racemate, a stereoisomer, a tautomer, an isotope-labeled counterpart, a nitrogen oxide, a solvate, a polymorph, a metabolite, an ester, a prodrug or a pharmaceutically acceptable salt thereof.

According to embodiments of the present invention, the pharmaceutical composition further comprises one or more pharmaceutically acceptable excipients.

According to embodiments of the present invention, the pharmaceutical composition may further comprise one or more other therapeutic agents.

The present invention further provides a method for treating tumor diseases, which comprises administering to a patient a therapeutically effective amount of at least one of the compound shown as formula (I), and a racemate, a stereoisomer, a tautomer, an isotope-labeled counterpart, a nitrogen oxide, a solvate, a polymorph, a metabolite, an ester, a prodrug or a pharmaceutically acceptable salt thereof.

According to embodiments of the present invention, the tumor is a tumor containing p53-Y220C mutant.

The present invention further provides a method for treating tumor diseases, comprising administering to a patient a prophylactically or therapeutically effective amount of the above pharmaceutical composition.

In some embodiment, the patient includes a mammal, preferably a human.

The present invention further provides at least one of a compound shown as formula (I), and a racemate, a stereoisomer, a tautomer, an isotope-labeled counterpart, a nitrogen oxide, a solvate, a polymorph, a metabolite, an ester, a prodrug or a pharmaceutically acceptable salt thereof, or a pharmaceutical composition thereof for use in treating tumor diseases.

The present invention further provides use of at least one of a compound shown as formula (I), and a racemate, a stereoisomer, a tautomer, an isotope-labeled counterpart, a nitrogen oxide, a solvate, a polymorph, a metabolite, an ester, a prodrug or a pharmaceutically acceptable salt thereof in the preparation of a drug.

According to embodiments of the present invention, the use can be use in the preparation of drugs against tumors containing p53-Y220C mutant, such as in the preparation of p53-Y220C reactivator drugs.

According to an embodiment of the present invention, the tumors containing p53-Y220C mutant include acute lymphocytic leukemia, acute myeloid leukemia, adrenocortical carcinoma, AIDS-related cancer, AIDS-related lymphoma, anal cancer, appendix cancer, astrocytoma, basal cell carcinoma, bile duct cancer, bladder cancer, bone cancer, brain tumors such as cerebellar astrocytoma, cerebral astrocytoma/malignant glioma, ependymoma, medulloblastoma, supratentorial primitive cell tumor, neuroectodermal tumor, visual pathway and hypothalamic glioma, breast cancer, bronchial adenoma, Burkitt's lymphoma, cancer of unknown primary, central nervous system lymphoma, cerebellar

astrocytoma, cervical cancer, childhood cancers, chronic lymphocytic leukemia, chronic myelocytic leukemia, chronic myeloproliferative disorders, colon cancer, cutaneous T-cell lymphoma, desmoplastic small round cell tumor, endometrial cancer, ependymoma, esophageal cancer, Ewing's sarcoma, germ cell tumors, gallbladder cancer, gastric cancer, gastrointestinal carcinoid tumors, gastrointestinal stromal tumors, gliomas, hairy cell leukemia, head and neck cancer, heart cancer, hepatocellular carcinoma, Hodgkin lymphoma, hypopharyngeal cancer, intraocular melanoma, islet cell carcinoma, Kaposi's sarcoma, kidney cancer, laryngeal cancer, lip and oral cancer, liposarcoma, liver cancer, lung cancer such as non-small cell lung cancer and small cell lung cancer, lymphoma, leukemia, macroglobulinemia, malignant bone fibrous histiocytoma/osteosarcoma, medulloblastoma, melanoma, mesothelioma, metastatic squamous cell carcinoma with occult primary, oral cancer, multiple endocrine neoplasms syndrome, myelodysplastic syndrome, myeloid leukemia, nasal and paranasal sinus cancer, nasopharyngeal cancer, neuroblastoma, non-Hodgkin lymphoma, non-small cell lung cancer, oral cancer, oropharyngeal cancer, osteosarcoma/malignant fibrous histiocytoma of bone, ovarian cancer, ovarian epithelial cancer, ovarian germ cell tumor, pancreatic cancer, pancreatic islet cell carcinoma, paranasal sinus and nasal cancer, parathyroid cancer, penile cancer, pharyngeal cancer, pheochromocytoma, pineal astrocytoma, pineal germ cell tumor, pituitary adenoma, pleuropulmonary blastoma, plasmacytoma, primary central nervous system lymphoma, prostate cancer, rectal cancer, renal cell carcinoma, transitional cell carcinoma of renal pelvis and ureter, retinoblastoma, rhabdomyosarcoma, salivary gland carcinoma, sarcoma, skin cancer, cutaneous Merkel cell carcinoma, small intestine cancer, soft tissue sarcoma, squamous cell carcinoma, gastric cancer, T-cell lymphoma, laryngeal cancer, thymoma, thymic carcinoma, thyroid cancer, gestational trophoblastic tumor, cancer of unknown primary site, urethral cancer, uterine sarcoma, vaginal cancer, vulvar cancer, Waldenstrom's macroglobulinemia, and nephroblastoma.

## Beneficial Effects

The compound of the present invention has good p53-Y220C mutant activation effect and can effectively restore the activity of the p53-Y220C mutant protein, thereby treating tumor diseases comprising p53-Y220C mutant proteins, as well as preparing drugs for use in such conditions or diseases. In addition, the compound of the present invention with a completely new structure obtained through structural optimization has better anti-tumor effects on various tumor models than the compounds in the prior art, and has improved pharmacokinetic properties and safety.

## Definition and Explanation of Terms

Unless otherwise indicated, the definitions of groups and terms recorded in the specification and claims of the present application, including their definitions as examples, exemplary definitions, preferred definitions, definitions recorded in tables, definitions of specific compounds in examples, etc., may be arbitrarily combined and associated with each other. Such combined and associated group definitions and compound structures should be understood to be within the scope of the specification and/or claims of the present application.
Unless otherwise indicated, the numerical ranges recorded in the present specification and claims are equivalent to at least recording each specific integer value therein. For example, the numerical range "1-14" is equivalent to recording each integer value in the numerical range "1-14", namely 1, 2, 3, 4, 5, 6, 7, 8, 9, 10, 11, 12, 13, 14.
The term "optional" (or "optionally") in the general formula definitions of the present application means a situation of being substituted with zero, one or more substituents, for example "optionally substituted with one, two or more R" means that it may not be substituted with R (unsubstituted) or may be selectively substituted with one, two or more R.
"More" means three or more, for example, 3, 4, 5, 6, 7, 8, 9 or 10.
The term "$C_{1-12}$ alkyl" is understood to mean linear and branched alkyl groups having 1 to 12 carbon atoms, "$C_{1-8}$ alkyl" means linear and branched alkyl groups having 1, 2, 3, 4, 5, 6, 7, or 8 carbon atoms, and "$C_{1-6}$ alkyl" means linear and branched alkyl groups having 1, 2, 3, 4, 5, or 6 carbon atoms. The alkyl is, for example, methyl, ethyl, propyl, butyl, pentyl, hexyl, iso-propyl, isobutyl, s-butyl, t-butyl, iso-pentyl, 2-methylbutyl, 1-methylbutyl, 1-ethylpropyl, 1,2-dimethylpropyl, neo-pentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl, or isomers thereof.
The term "$C_{2-12}$ alkenyl" is understood to mean straight or branched monovalent hydrocarbon groups having 1 to 12 carbon atoms, which contain one or more double bonds and have 2, 3, 4, 5, 6, 7, 8, 9, 10, 11 or 12 carbon atoms. For example, they have 2, 3, 4, 5, 6, 7 or 8 carbon atoms (i.e. $C_{2-8}$ alkenyl), for example, 2, 3, 4, 5 or 6 carbon atoms (i.e. $C_{2-6}$ alkenyl), 2 or 3 carbon atoms (i.e. $C_{2-3}$ alkenyl). It will be understood that where the alkenyl comprises more than one double bond, the double bonds may be separated from each other or conjugated. The alkenyl is, for example, vinyl, allyl, (E)-2-methylvinyl, (Z)-2-methylvinyl, (E)-but-2-enyl, (Z)-but-2-enyl, (E)-but-1-enyl, (Z)-but-1-enyl, pent-4-enyl, (E)-pent-3-enyl, (Z)-pent-3-enyl, (E)-pent-2-enyl, (Z)-pent-2-enyl, (E)-pent-1-enyl, (Z)-pent-1-enyl, hex-5-enyl, (E)-hex-4-enyl, (Z)-hex-4-enyl, (E)-hex-3-enyl, (Z)-hex-3-enyl, (E)-hex-2-enyl, (Z)-hex-2-enyl, (E)-hex-1-enyl, (Z)-hex-1-enyl, isopropenyl, 2-methylprop-2-enyl, 1-methylprop-2-enyl, 2-methylprop-1-enyl, (E)-1-methylprop-1-enyl,

(Z)-1-methylprop-1-enyl, 3-methylbut-3-enyl, 2-methylbut-3-enyl, 1-methylbut-3-enyl, 3-methylbut-2-enyl, (E)-2-methylbut-2-enyl, (Z)-2-methylbut-2-enyl, (E)-1-methylbut-2-enyl, (Z)-1-methylbut-2-enyl, (E)-3-methylbut-1-enyl, (Z)-3-methylbut-1-enyl, (E)-2-methylbut-1-enyl, (Z)-2-methylbut-1-enyl, (E)-1-methylbut-1-enyl, (Z)-1-methylbut-1-enyl, 1,1-dimethylprop-2-enyl, 1-ethylprop-1-enyl, 1-propyvinyl, or 1-isopropylvinyl.

The term "$C_{2-12}$ alkynyl" is understood to mean linear or branched monovalent hydrocarbon groups having 1 to 12 carbon atoms, which comprise one or more triple bonds and have 2, 3, 4, 5, 6, 7, 8, 9, 10, 11 or 12 carbon atoms, for example, 2, 3, 4, 5, 6, 7, or 8 carbon atoms (i.e. "$C_{2-8}$ alkynyl"), 2, 3, 4, 5, or 6 carbon atoms (i.e. "$C_{2-6}$ alkynyl"), or 2 or 3 carbon atoms (i.e. "$C_{2-3}$ alkynyl"). The alkynyl is, for example, ethynyl, prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl, or 3,3-dimethylbut-1-ynyl. In particular, the alkynyl is ethynyl, prop-1-ynyl, or prop-2-ynyl.

The term "$C_{3-12}$ cycloalkyl" is understood to mean saturated monovalent monocyclic, bicyclic (such as fused, bridged, spiro) hydrocarbon ring or tricyclic alkanes having 3 to 12 carbon atoms, preferably "$C_{3-10}$ cycloalkyl", more preferably "$C_{3-8}$ cycloalkyl". The term "$C_{3-12}$ cycloalkyl" should be understood to mean a saturated monovalent monocyclic, bicyclic (such as bridged, spiro) hydrocarbon ring or tricyclic alkane having 3, 4, 5, 6, 7, 8, 9, 10, 11 or 12 carbon atoms. The $C_{3-12}$ cycloalkyl may be a monocyclic hydrocarbon group, such as cyclopropyl, cyclobutyl, cyclopentyl, cyclohexyl, cycloheptyl, cyclooctyl, cyclononyl or cyclodecyl, or a bicyclic hydrocarbon group, such as bornyl, indolyl, hexahydroindolyl, tetrahydronaphthyl, decahydronaphthyl, bicyclo[2.1.1]hexyl, bicyclo[2.2.1]heptyl, bicyclo[2.2.1]heptenyl, 6,6-dimethylbicyclo[3.1.1]heptyl, 2,6,6-trimethylbicyclo[3.1.1]heptyl, bicyclo[2.2.2]octyl, 2,7-diazaspiro[3,5]nonanyl, 2,6-diazaspiro[3,4]octanyl, or a tricyclic hydrocarbon group, such as adamantyl.

The term "$C_{6-14}$ aryl" is understood to preferably mean a monovalent aromatic or partially aromatic monocyclic, bicyclic or tricyclic hydrocarbon ring having 6 to 14 carbon atoms, which may be a single aromatic ring or a fused polyaromatic ring, preferably "$C_{6-10}$ aryl". The term "$C_{6-14}$ aryl" should be understood as preferably representing a monovalent aromatic or partially aromatic monocyclic, bicyclic or tricyclic hydrocarbon ring ("$C_{6-14}$ aryl") having 6, 7, 8, 9, 10, 11, 12, 13, or 14 carbon atoms, in particular a ring having 6 carbon atoms ("$C_6$ aryl"), such as a phenyl; or biphenyl, or a ring having 9 carbon atoms ("$C_9$ aryl"), such as indanyl or indenyl, or a ring having 10 carbon atoms ("$C_{10}$ aryl"), such as tetrahydronaphthyl, dihydronaphthyl, or naphthyl, or a ring having 13 carbon atoms ("$C_{13}$ aryl"), such as fluorenyl, or a ring having 14 carbon atoms ("$C_{14}$ aryl"), such as anthracenyl. When the $C_{6-20}$ aryl is substituted, it can be monosubstituted or polysubstituted. Furthermore, there is no limitation on the substitution site, and for example, substitution may be at the ortho, para or meta position.

The term "5-14 membered heteroaryl" is understood to include monovalent monocyclic, bicyclic (such as fused, bridged, spiro) or tricyclic aromatic ring systems which have 5 to 14 ring atoms and comprise 1 to 5 heteroatoms independently selected from N, O and S, for example "5-10 membered heteroaryl". The term "5- to 14-membered heteroaryl" should be understood to include monovalent monocyclic, bicyclic or tricyclic aromatic ring systems, which have 5, 6, 7, 8, 9, 10, 11, 12, 13 or 14 ring atoms, in particular 5 or 6 or 9 or 10 carbon atoms, and comprise 1 to 5, preferably 1 to 3 heteroatoms that are each independently selected from N, O and S, and which, in addition, in each case may be benzo-fused. "Heteroaryl" also means a group in which a heteroaromatic ring is fused with one or more aryl, alicyclic, or heterocyclic rings, wherein the radical or site of attachment is on the heteroaromatic ring. Non-limiting examples include 1-, 2-, 3-, 5-, 6-, 7- or 8-indolizinyl, 1-, 3-, 4-, 5-, 6-, or 7-isoindolyl, 2-, 3-, 4-, 5-, 6- or 7-indolyl, 2-, 3-, 4-, 5-, 6-, or 7-indazolyl, 2-, 4-, 5-, 6-, 7- or 8-purinyl, 1-, 2-, 3-, 4-, 6-, 7-, 8- or 9-quinolizinyl, 2-, 3- , 4-, 5-, 6-, 7- or 8-quinolinyl, 1-, 3-, 4-, 5-, 6-, 7- or 8-isoquinolinyl, 1-, 4-, 5-, 6-, 7- or 8-phthalazinyl, 2-, 3-, 4-, 5- or 6-naphthyridinyl, 2-, 3-, 5-, 6-, 7- or 8-quinazolinyl, 3-, 4-, 5-, 6-, 7- or 8-cinnolinyl, 2-, 4-, 6- or 7-pteridinyl, 1-, 2-, 3-, 4-, 5-, 6-, 7- or 8-4aH carbazolyl, 1-, 2-, 3-, 4-, 5-, 6-, 7- or 8-carbazolyl, 1-, 3-, 4-, 5-, 6-, 7-, 8- or 9-carbolinyl, 1-, 2-, 3-, 4-, 6-, 7-, 8-, 9- or 10-phenanthridinyl, 1-, 2-, 3-, 4-, 5-, 6-, 7-, 8- or 9-acridinyl, 1-, 2-, 4-, 5-, 6-, 7-, 8- or 9-pyridyl, 2-, 3-, 4-, 5-, 6-, 8-, 9- or 10-phenanthrolinyl, 1-, 2-, 3-, 4-, 6-, 7-, 8- or 9-phenazinyl, 1-, 2-, 3-, 4-, 6-, 7-, 8-, 9- or 10-phenothiazinyl, 1-, 2-, 3-, 4-, 6-, 7-, 8-, 9- or 10-phenazinyl , 2-, 3-, 4-, 5-, 6- or 1-, 3-, 4-, 5-, 6-, 7-, 8-, 9- or 10-benzoisoquinolyl, 2-, 3-, 4- or thieno[2,3-b]furanyl, 2-, 3-, 5-, 6-, 7-, 8-, 9-, 10- or 11-7H-pyrazino [2,3-c]carbazolyl, 2-, 3-, 5-, 6- or 7-2H-furo[3,2-b]pyranyl , 2-, 3-, 4-, 5-, 7-, or 8-5H-pyridino[2,3-d]-o-oxazinyl, 1-, 3-, or 5-1H-pyrazolo[4,3-d]-oxazolyl, 2-, 4-, or 54H-imidazo[4,5-d]thiazolyl, 3-, 5-, or 8-pyrazino[2,3-d]pyridazinyl, 2-, 3-, 5-, or 6-imidazo[2,1-b]thiazolyl, 1-, 3-, 6-, 7-, 8-, or 9-furo[3,4-c]cinnolinyl, 1-, 2-, 3-, 4-, 5-, 6-, 8-, 9-, 10 or 11-4H-pyridino[2,3-c] carbazolyl, 2-, 3-, 6- or 7-imidazo[1,2-b][1,2,4]triazinyl, 7-benzo[b]thienyl, 2-, 4-, 5-, 6- or 7-benzoxazolyl, 2-, 4-, 5-, 6- or 7-benzimidazolyl, 2-, 4- , 4-, 5-, 6- or 7-benzothiazolyl, 1-, 2-, 4-, 5-, 6-, 7-, 8- or 9-benzoxapinyl, 2-, 4-, 5-, 6-, 7- or 8-benzoxazinyl, 1-, 2-, 3-, 5-, 6-, 7-, 8-, 9-, 10- or 11-4H-pyrrolo[1,2-b][2]benzazapinyl. A typical fused heteroaryl includes, but is not limited to, 2-, 3-, 4-, 5-, 6-, 7-, or 8-quinolyl, 1-, 3-, 4-, 5-, 6-, 7-, or 8-isoquinolyl, 2-, 3-, 4-, 5-, 6-, or 7-indolyl, 2-, 3-, 4-, 5-, 6-, or 7-benzo[b]thienyl, 2-, 4-, 5-, 6-, or 7-benzoxazolyl, 2-, 4-, 5-, 6-, or 7-benzimidazolyl, and 2-, 4-, 5-, 6-, or 7-benzothiazolyl. When the 5- to 14-membered heteroaryl is linked to other groups to constitute the compound of the present

invention, either the carbon atom on the 5- to 14-membered heteroaryl ring can be linked to other groups, or the heteroatom on the 5- to 14-membered heteroaryl ring can be linked to other groups. When the 5- to 14-membered heteroaryl is substituted, it can be monosubstituted or polysubstituted. Further, the substitution site is not limited. For example, hydrogen attached to a carbon atom on the heteroaryl ring may be substituted, or hydrogen attached to a heteroatom on the heteroaryl ring may be substituted.

The term "carbon ring" refers to a saturated or unsaturated non-aromatic monocyclic or polycyclic (such as bicyclic) hydrocarbon ring (for example a monocyclic ring such as a cyclopropane ring, a cyclobutane ring, a cyclopentane ring, a cyclohexane ring, a cycloheptane ring, a cyclooctane ring, a cyclononane ring, or a bicyclic ring, including a spirocyclic, fused or bridged system (such as a bicyclo[11.1]pentane ring, a bicyclo[2.2.1]heptane ring, a bicyclo[3.2.1]octane ring or a bicyclo[5.2.0]nonane ring, a decalin ring), which may be optionally substituted with 1 or more (such as 1, 2 or 3) suitable substituents. The term "3-6 membered carbon ring" refers to a carbon ring containing 3, 4, 5, or 6 ring-forming carbon atoms.

Unless otherwise defined, the term "3-14 membered heterocyclyl" refers to a saturated or unsaturated non-aromatic ring or ring system, for example, a 4-, 5-, 6- or 7-membered monocyclic ring, a 7-, 8-, 9-, 10-, 11- or 12-membered bicyclic ring (e.g. fused, bridged, spiro) or a 10-, 11-, 12-, 13- or 14-membered tricyclic ring system, and contains at least one, for example 1, 2, 3, 4, 5 or more heteroatoms selected from O, S and N, wherein N and S may also be optionally oxidized to various oxidation states to form nitrogen oxides, -S(O)- or $-S(O)_2-$. Preferably, the heterocyclyl may be selected from "3- to 10-membered heterocyclyl". The term "3-10 membered heterocyclyl" means a saturated or unsaturated non-aromatic ring or ring system and contains at least one heteroatom selected from O, S and N. The heterocyclyl may be attached to the rest of the molecule via any of the carbon atoms or the nitrogen atom, if present. The heterocyclyl may include a fused or bridged ring and a spirocyclic ring. In particular, the heterocyclyl may include, but is not limited to: a 4-membered ring, such as azetidinyl, oxetanyl; a 5-membered ring, such as tetrahydrofuranyl, dioxolyl, pyrrolidinyl, imidazolidinyl, pyrazolidinyl, pyrrolinyl; or a 6-membered ring, such as tetrahydropyranyl, piperidinyl, morpholinyl, dithianyl, thiomorpholinyl, piperazinyl or trithianyl; or a 7-membered ring, such as diazepanyl. Optionally, the heterocyclyl may be benzo-fused. The heterocyclyl may be bicyclic, such as but not limited to a 5,5-membered ring, such as a hexahydrocyclopenta[c]pyrrol-2(1H)-yl ring, or a 5,6-membered bicyclic ring, such as a hexahydropyrrolo[1,2-a]pyrazin-2(1H)-yl ring. The heterocyclyl may be partially unsaturated, tht is, it may contain one or more double bonds, such as but not limited to dihydrofuranyl, dihydropyranyl, 2,5-dihydro-1H-pyrrolyl, 4H-[1,3,4]thiadiazinyl, 1,2,3,5-tetrahydrooxazolyl or 4H-[1,4]thiazinyl, or it may be benzo-fused, such as but not limited to dihydroisoquinolinyl. When the 3-14 membered heterocyclyl is linked to other groups to constitute the compound of the present invention, either the carbon atom on the 3-14 membered heterocyclyl can be linked to other groups, or the heterocyclic atom on the 3-14 membered heterocyclyl can be linked to other groups. For example, when the 3-14 membered heterocyclyl is selected from piperazinyl, the nitrogen atom on the piperazinyl may be linked to other groups. Alternatively, when the 3-14 membered heterocyclyl is selected from piperidinyl, the nitrogen atom on the piperidinyl ring and the carbon atom at the para position thereof may be linked to other groups.

The term "spiro ring" refers to a ring system in which two rings share one ring-forming atom.

The term "fused ring" refers to a ring system in which two rings share 2 ring-forming atoms.

The term "bridged ring" refers to a ring system in which two rings share more than 3 ring-forming atoms.

The term "halogen" represents fluorine, chlorine, bromine, and iodine.

"Halo" means being substituted with one or more halogens.

The term "haloalkyl" refers to alkyl substituted with one or more halogens, wherein the alkyl is as defined above.

The term "oxo" refers to a substitution with oxygen (=O) formed when a carbon atom, nitrogen atom or sulfur atom in a substituent is oxidized.

The term "alkylamino" refers to -NH-(alkyl) or $-N-(alkyl)_2$, wherein the alkyl is as defined above. Non-limiting examples of alkylamino comprise: methylamino, ethylamino, propylamino, iso-propylamino, butylamino, dimethylamino, methylethylamino, diethylamino, dipropylamino, methylpropylamino, di-iso-propylamino, dibutylamino, and the like.

The "hydroxyalkyl" refers to alkyl substituted with one or more hydroxy groups, wherein the alkyl is as defined above. Non-limiting examples of hydroxyalkyl groups include hydroxymethyl, hydroxyethyl, hydroxypropyl, hydroxymethylpropyl or dihydroxypropyl, etc.

The term "alkoxy" refers to -O-(alkyl), wherein the alkyl is as defined above. Non-limiting examples of alkoxy comprise: methoxy, ethoxy, propoxy, and butoxy. The alkoxy may be optionally substituted or unsubstituted, and when it is substituted, the substituent is preferably one or more radicals independently selected from alkyl, alkenyl, alkynyl, alkoxy, alkylamino, halogen, sulfhydryl, hydroxyl, nitro, cyano, cycloalkyl, heterocycloalkyl, aryl, heteroaryl, cycloalkoxy, or heterocycloalkoxy.

The terms "alkyleneoxy" and "oxyalkylene" refer to -alkylene-O- or -O-alkylene-, wherein the alkylene represents a linear or branched saturated divalent hydrocarbon radical. For the definition of the number of carbon atoms in the "alkylene", the above definition for the "alkyl" applies. Those skilled in the art can understand that alkyleneoxy or oxyalkylene can be attached to the rest of the molecule containing it in any direction, that is, they can be used

interchangeably.

The term "N-oxide" refers to a compound formed by oxidation of a nitrogen atom in the structure of tertiary amine or nitrogen-containing (aromatic) heterocyclic compound.

Unless otherwise indicated, the heterocyclyl, heterocyclylene, heteroaryl, or heteroarylene comprises all possible isomeric forms thereof, such as positional isomers thereof. Therefore, for some illustrative non-limiting examples, the substitution or bonding to other radicals may be comprised at, e.g., 1, 2, or more of its 1-, 2-, 3-, 4-, 5-, 6-, 7-, 8-, 9-, 10-, 11-, 12-C (if present), comprising pyridin-2-yl, pyridyliden-2-yl, pyridin-3-yl, pyridyliden-3-yl, pyridin-4-yl, and pyridyliden-4-yl; and the thienyl or thienylenyl comprises thien-2-yl, thienylen-2-yl, thien-3-yl, and thienylen-3-yl; pyrazol-1-yl, pyrazol-3-yl, pyrazol-4-yl, and pyrazol-5-yl.

The compounds of the present disclosure may exist in different tautomeric forms, and all such forms are encompassed within the scope of the present disclosure. The term "tautomer" or "tautomeric form" refers to structural isomers that exist in equilibrium and are readily converted from one isomeric form to another. It includes all possible tautomers, i.e., in the form of a single isomer or in the form of mixtures of the tautomers in any ratio. Non-limiting examples include: keto-enol, imine-enamine, lactam-lactim, and the like.

Wavy lines (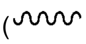) intersecting with chemical bonds are used to indicate positions where a group is linked to other atoms in the molecular structure. For example, "

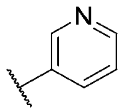

" indicates connection to the 3-position of pyridyl. When the connection position of the group is not fixed, taking pyridyl as an example, it can be represented by "

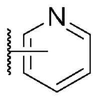

", which means that it can be connected to any connectable position on the pyridyl. As another example, "

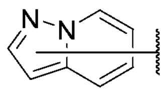

" indicates that it can be connected to any connectable position on the heteroaromatic ring, for example, it can be connected to any of the 4 carbon atoms on the right pyridine ring of the heteroaromatic group, or it can be connected to any one of the carbon atoms on the left pyrazole ring. Unless otherwise stated, similar expressions in the present application shall be interpreted the same as above.

In the chemical structure of the compounds described in the present invention, the bond "

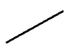

" indicates an unspecified configuration, and "

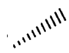

" or "

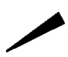

" indicates an absolute configuration, that is, if stereoisomers exist in the chemical structure, the bond ".

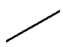

" can be ",

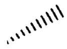

" or"

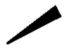

" , or can comprise both "

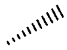

" and "

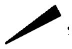

" configurations.

In the present invention, the compound involved also comprises an isotopically labeled compound, which is the same as the compound of Formula I, but in which one or more atoms are replaced by atoms having an atomic mass or mass number different from the atomic mass or mass number usually occurring in nature. Exemplary isotopes that can be incorporated into the compound of the present invention comprise isotopes of H, C, N, O, S, F and Cl, such as $^{2}H$, $^{3}H$, $^{13}C$, $^{11}C$, $^{14}C$, $^{15}N$, $^{18}O$, $^{17}O$, $^{32}P$, $^{35}S$, $^{18}F$ and $^{36}Cl$. The compound of the present invention containing the aforementioned isotopes and/or other isotopes of other atoms and a prodrug thereof, or a pharmaceutically acceptable salt of the compound or the prodrug are covered in the scope of the present invention. Some isotopically labeled compounds of the present invention, for example, compounds incorporated with radioactive isotopes (such as $^{3}H$ and $^{14}C$) can be used in the distribution determination of drugs and/or substrates in tissues. The isotopes tritium (that is, $^{3}H$) and carbon 14 (that is, $^{14}C$) are particularly preferred because of their easy preparation and detectability. Furthermore, the substitution with a heavier isotope (such as deuterium, that is, $^{2}H$ or D) can provide some therapeutic advantages due to the greater metabolic stability (for example, increased in-vivo half-life or reduced dosage requirement), and is thus preferred in some cases. In the present invention, in case that hydrogen in a substituent is not explicitly indicated to be deuterium or tritium, it does not mean that deuterium or tritium is excluded, but deuterium or tritium can be contained as well.

Those skilled in the art will understand that the compound shown as formula (I) may exist in the form of various pharmaceutically acceptable salts. If the compounds have basic centers, they can form acid addition salts; if they have acidic centers, they can form base addition salts; if the compounds contain both acidic centers (e.g. carboxyl) and basic centers (e.g. amino), they can also form inner salts.

The compounds of the present invention may be present in the form of solvates, such as hydrates, wherein the compounds of the present invention comprise polar solvents, such as water, methanol or ethanol, as structural elements of the lattice of the compounds. The polar solvent, especially water, may be present in a stoichiometric or non-stoichiometric amount.

Depending on their molecular structure, the compounds of the present invention may be chiral and may therefore exist in various enantiomeric forms. These compounds may therefore exist in racemic or optically active forms. The compounds of the present invention encompass isomers in which the chiral carbons are each in R or S configuration, or mixtures and racemates thereof. The compounds of the present invention or their intermediates can be separated into enantiomeric compounds by chemical or physical methods known to those skilled in the art, or used in synthesis in this form. In the case of racemic amines, diastereomers are prepared from the mixture by reaction with an optically active resolving reagent. Examples of suitable resolving reagents are optically active acids, such as the R and S forms of tartaric acid, diacetyltartaric acid, dibenzoyltartaric acid, mandelic acid, malic acid, lactic acid, appropriate N-protected amino acids (e.g. N-benzoylproline or N-phenylsulfonylproline) or various optically active camphorsulfonic acids. Chromatographic enantiomeric resolution can also be advantageously carried out by means of optically active resolving reagents, such as dinitrobenzoylphenylglycine, cellulose triacetate or other carbohydrate derivatives or chirally derivatized methacrylate polymers immobilized on silica gel. Suitable eluents for this purpose are aqueous or alcoholic solvent mixtures, for example, hexane/isopropanol/acetonitrile.

The corresponding stable isomers can be separated according to known methods, such as by extraction, filtration or column chromatography.

The term "patient" refers to any animal, including mammals, preferably mice, rats, other rodents, rabbits, dogs,

cats, pigs, cows, sheep, horses or primates, and most preferably humans.

The term "therapeutically effective amount" refers to the amount of an active compound or drug which causes a biological or medical response sought by a researcher, veterinarian, physician or other clinician in a tissue, system, animal, individual or human, and which comprises one or more of the following: (1) prevention of disease: for example, prevention of a disease, disorder or condition in an individual who is susceptible to the disease, disorder or condition but has not yet experienced or developed the pathology or symptoms of the disease. (2) Inhibition of disease: for example, inhibition of a disease, disorder or condition (i.e., prevention of further progression of pathology and/or symptoms) in an individual undergoing or exhibiting the pathology or symptoms of the disease, disorder or condition. (3) Alleviation of disease: for example, alleviation of a disease, disorder or condition (i.e., reversion of pathology and/or symptoms) in an individual undergoing or exhibiting the pathology or symptoms of the disease, disorder or condition.

## BRIEF DESCRIPTION OF THE DRAWINGS

Fig. 1: Tumor growth curve of experimental animals in NUGC-3 human gastric cancer subcutaneous xenograft model.
Fig. 2: Tumor growth curve of experimental animals in human-derived lung cancer LU5269 subcutaneous xenograft female NOD/SCID model.

## DETAILED DESCRIPTION OF THE INVENTION

The technical solution of the present invention will be further described in detail below in conjunction with specific embodiments. It should be understood that the following embodiments are only intended to exemplify and explain the present invention and should not be construed as limiting the scope of protection of the present invention. All technologies realized based on the above contents of the present invention are included in the scope that the present invention intends to protect.

Unless otherwise indicated, the raw materials and reagents used in the following examples are commercially available or may be prepared by known methods.

The structures of the compounds in the present invention are determined by nuclear magnetic resonance (NMR) or/and liquid chromatography mass spectrometry (LC-MS). NMR chemical shifts ($\delta$) are given in parts per million (ppm). The NMR measurement is performed by using Bruker AVANCE-400 nuclear magnetic resonance spectrometer, with deuterated dimethyl sulfoxide (DMSO-d6), deuterated methanol ($CD_3OD$) or deuterated chloroform ($CDCl_3$) as determination solvents, and with tetramethylsilane (TMS) as an internal standard.

An Agilent 1200 Infinity Series mass spectrometer is used for liquid chromatography mass spectrometry (LC-MS) measurements. The HPLC measurement is performed using an Agilent 1200DAD high pressure liquid chromatograph (Sunfire C18 150×4.6 mm column) and a Waters 2695-2996 high pressure liquid chromatograph (Gimini C18 150×4.6 mm column).

Yantai Huanghai HSGF254 or Qingdao Marine Chemical GF254 silica gel plate is used as the silica gel plate for thin layer chromatography, the specification used for TLC is from 0.15 mm to 0.20 mm, and the specification of the thin layer chromatography for separation and purification of products is from 0.4 mm to 0.5 mm. Yantai Huanghai silica gel of 200-300 mesh is generally used as the carrier for column chromatography.

Unless otherwise indicated, all reactions in the present invention are carried out under continuous magnetic stirring in a dry nitrogen or argon atmosphere in a dry solvent at a reaction temperature expressed in degrees centigrade.

## Example 1

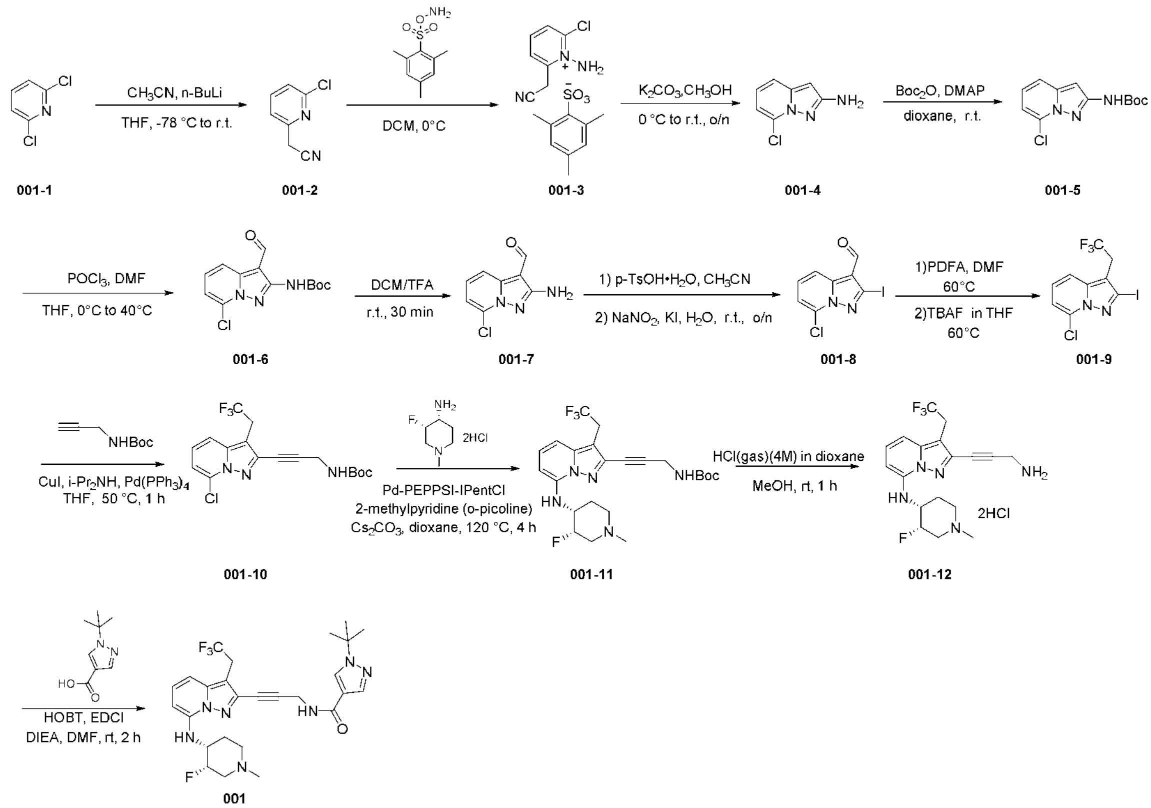


001-1, 001-2, 001-3, 001-4, 001-5, 001-6, 001-7, 001-8, 001-9, 001-10, 001-11, 001-12, 001

**Step 1: Synthesis of compound 001-2**

A solution of acetonitrile (4.72 g, 114.872 mmol, 3.4 eq) in tetrahydrofuran (150 mL) was added to a reaction flask, and n-butyl lithium (44 mL) was added dropwise at -78°C, and the mixture was further stirred for 1 h. Then, a solution of 2,6-dichloropyridine (compound **001-1,** 5 g, 33.786 mmol, 1 eq) in tetrahydrofuran (50 mL) was added dropwise, and the system was further reacted at -78°C for 2 h, and then warmed to room temperature and further reacted for 30 min. After the reaction was completed, the system was quenched with water and extracted with ethyl acetate (3×300 mL). The organic phases were combined, backwashed with saturated sodium chloride solution, dried over anhydrous sodium sulfate, and filtered, and the filtrate was concentrated under reduced pressure. The resulting crude product was purified by silica gel column chromatography (petroleum ether/ethyl acetate = 1:1) to give compound **001-2** (4.66 g, 89.49%).

$^1H$ NMR (400 MHz, DMSO-$d_6$) $\delta$ 7.92 (t, $J$ = 7.8 Hz, 1H), 7.52 (dd, $J$ = 8.0, 0.7 Hz, 1H), 7.45 (dd, $J$ = 7.6, 0.7 Hz, 1H), 4.26 (s, 2H).

**Step 2: Synthesis of compound 001-3**

2-(6-chloropyridin-2-yl)acetonitrile (compound **001-2,** 5 g, 32.770 mmol, 1 eq) was added to a reaction flask and dissolved in dichloromethane (100 mL). A solution of 2-[(aminooxy)sulfonyl]-1,3,5-trimethylbenzene (14.11 g, 65.546 mmol, 2.00 eq) in dichloromethane(70 mL) was added at 0°C and the mixture was further stirred at 0°C for 2 h. After the reaction was completed, diethyl ether was added and the solvent was removed by spin drying to give crude product **001-3** (7 g, 40.65%), which was directly used in the next step.

**Step 3: Synthesis of compound 001-4**

1-amino-2-chloro-6-(cyanomethyl)pyridin-1-ium-2,4,6-trimethylbenzenesulfonate (compound **001-3,** 8 g, 21.748 mmol, 1 eq) was added to a reaction flask and dissolved in methanol (80.0 mL). Potassium carbonate (6.01 g, 43.496 mmol, 2.00 eq) was added in ice bath and then the system was warmed to room temperature to react overnight. After the reaction was completed, the solvent was removed by spin drying, water was added and the system was extracted with dichloromethane (3 × 100 mL). The organic phases were combined, backwashed with saturated sodium chloride solution, dried over anhydrous sodium sulfate, and filtered, and the filtrate was concentrated under reduced pressure. The resulting crude product was purified by silica gel column chromatography (dichloromethane/methanol = 50:1) to give compound **001-4** (1.7 g, 39.64%).

$^{1}$H NMR (400 MHz, DMSO-$d_6$) $\delta$ 7.28 (dd, *J* = 8.8, 1.2 Hz, 1H), 7.01 (dd, *J* = 8.8, 7.3 Hz, 1H), 6.75 (dd, *J* = 7.3, 1.2 Hz, 1H), 5.80 (s, 1H), 5.57 (s, 2H).

**Step 4: Synthesis of compound 001-5**

7-chloropyrazolo[1,5-a]pyridin-2-amine (compound **001-4,** 1.4 g, 8.353 mmol, 1 eq), 4-dimethylaminopyridine (102.05 mg, 0.835 mmol, 0.1 eq), and di-tert-butyl dicarbonate (2.01 g, 9.188 mmol, 1.1 eq) were added to a reaction flask in sequence, dissolved in 1,4-dioxane (20 mL), and reacted at room temperature for 3 h. After the reaction was completed, the system was diluted with water and extracted with dichloromethane (3×20 mL). The organic phases were combined, backwashed with saturated sodium chloride solution, dried over anhydrous sodium sulfate, and filtered, and the filtrate was concentrated under reduced pressure. The resulting crude product was purified by silica gel column chromatography (petroleum ether/ethyl acetate = 10:1) to give compound **001-5** (634 mg, 22.68%).
LCMS: (ESI, m/z): 267.85 $[M+H]^+$.

**Step 5: Synthesis of compound 001-6**

*N*,*N*-dimethylformamide (344.03 mg, 4.706 mmol, 2 eq) and tetrahydrofuran (13 mL) were added to a reaction flask, and phosphorus oxychloride (1.08 g, 7.059 mmol, 3 eq) was added at 0°C. The system was reacted for 30 min, and then tert-butyl (7-chloropyridino[1,5-a]pyridin-2-yl)carbamate (compound **001-5,** 630 mg, 2.353 mmol, 1 eq) was added. The system was heated to 40°C and reacted for 30 min. After the reaction was completed, saturated sodium carbonate solution was added to quench the reaction, and the mixture was extracted with dichloromethane (3×20 mL). The organic phases were combined, backwashed with saturated sodium chloride solution, dried over anhydrous sodium sulfate, filtered, and the filtrate was concentrated under reduced pressure to directly give compound **001-6** (791 mg, 90.93%).
LCMS: (ESI, m/z): 296.00 $[M+H]^+$.

**Step 6: Synthesis of compound 001-7**

Tert-butyl (7-chloro-3-formylpyridino[1,5-a]pyridin-2-yl)carbamate (compound **001-6,** 740 mg, 2.502 mmol, 1 eq) was added to a reaction flask, dissolved in dichloromethane (5 mL) and trifluoroacetic acid (5 mL), and reacted at room temperature for 30 min. After the reaction was completed, the solvent was directly removed by spin drying to give crude product **001-7** (800 mg, 138.92%), which was directly used in the next step.
LCMS: (ESI, m/z): 196.00 $[M+H]^+$.

**Step 7: Synthesis of compound 001-8**

2-amino-7-chloropyrazolo[1,5-a]pyridin-3-carboxaldehyde (compound **001-7,** 490 mg, 2.505 mmol, 1 eq) and p-toluenesulfonic acid hydrate (1.43 g, 7.515 mmol, 3 eq) were added to a reaction flask and dissolved in acetonitrile (8 mL). Sodium nitrite (345.66 mg, 5.010 mmol, 2 eq) and potassium iodide (1.04 g, 6.262 mmol, 2.5 eq) were added to another reaction flask and dissolved in water (2 mL), and the mixed aqueous solution was then added dropwise to the previous acetonitrile solution. The system was reacted at room temperature overnight. After the reaction was completed, the system was quenched with saturated sodium thiosulfate aqueous solution and extracted with ethyl acetate (3×10 mL). The organic phases were combined, backwashed with saturated sodium chloride solution, dried over anhydrous sodium sulfate, and filtered, and the filtrate was concentrated under reduced pressure. The resulting crude product was purified by silica gel column chromatography (petroleum ether/ethyl acetate = 10:1) to give compound **001-8** (180 mg, 23.21%).
$^{1}$H NMR (400 MHz, DMSO-$d_6$) $\delta$ 9.76 (s, 1H), 8.22 (dd, *J* = 8.7, 1.2 Hz, 1H), 7.73 (dd, *J* = 8.7, 7.5 Hz, 1H), 7.54 (dd, *J* = 7.6, 1.2 Hz, 1H).

**Step 8: Synthesis of compound 001-9**

7-chloro-2-iodopyrazolo[1,5-a]pyridin-3-carboxaldehyde (compound **001-8,** 160 mg, 0.522 mmol, 1 eq) and (triphenylphosphonium) difluoroacetate (372.01 mg, 1.044 mmol, 2 eq) were added to a reaction flask, dissolved in *N*,*N*-dimethylformamide (8.0 mL), and reacted at 60°C for 2 h. Then tetrabutylammonium fluoride solution (1 M in tetrahydrofuran, 1.566 mL, 1.566 mmol, 3.00 eq) was added and the reaction was continued for 1 h. After the reaction was completed, the system was diluted with ethyl acetate, then water was added and the system was extracted with ethyl acetate (3×10 mL). The organic phases were combined, backwashed with saturated sodium chloride solution, dried over anhydrous sodium sulfate, and filtered, and the filtrate was concentrated under reduced pressure. The resulting crude product was purified by silica gel column chromatography (petroleum ether/ethyl acetate = 10:1) to give compound **001-9** (57 mg, 29.98%).

$^1H$ NMR (400 MHz, DMSO-$d_6$) $\delta$ 7.85 - 7.82 (m, 1H), 7.38 (dd, *J* = 8.9, 7.3 Hz, 1H), 7.25 (dd, *J* = 7.3, 1.1 Hz, 1H), 3.78 (q, *J* = 11.0 Hz, 2H).

**Step 9: Synthesis of compound 001-10**

Under nitrogen protection, at 50°C, 7-choro-2-iodo-3-(2,2,2-trifluoroethyl)pyrazolo[1,5-a]pyridine (compound **001-9,** 400 mg, 1.110 mmol, 1 eq), N-Boc-aminopropyne (189.42 mg, 1.221 mmol, 1.1 eq), cuprous iodide (21.13 mg, 0.111 mmol, 0.1 eq), palladium tetrakis(triphenylphosphine) (128.22 mg, 0.111 mmol, 0.1 eq) and diisopropylamine (1.12 g, 11.100 mmol, 10 eq) were dissolved in tetrahydrofuran (6 mL) and stirred to react for 1 h. After the reaction was completed, the reaction mixture was quenched with water and extracted with ethyl acetate (3×30 mL) at room temperature. The organic phases were combined and backwashed with saturated sodium chloride (2×20 ml), dried over anhydrous sodium sulfate, filtered, and the filtrate was concentrated under reduced pressure. The resulting crude product was purified by silica gel column chromatography (petroleum ether/ethyl acetate = 1:9) to give compound **001-10** (400 mg, 92.96%).
LCMS: (ESI, m/z): 387.95 $[M+H]^+$.

**Step 10: Synthesis of compound 001-11**

Under nitrogen protection, compound tert-butyl {3-[7-chloro-3-(2,2,2-trifluoroethyl)pyrazolo[1,5-a]pyridin-2-yl] prop-2-yn-1-yl}carbamate (compound **001-10,** 380 mg, 0.980 mmol, 1 eq), (3S,4R)-3-fluoro-1-methylpiperidin-4-amine dihydrochloride (240.92 mg, 1.176 mmol, 1.2 eq), (SP-4-1)-[1,3-bis[2,6-bis(1-ethylpropyl)phenyl]-4,5-dichloro-1,3-dihydro-*2H*-imidazol-2-ylidene]dichloro(2-methylpyridine)palladium (82.43 mg, 0.098 mmol, 0.1 eq) and cesium carbonate (1.277 g, 3.920 mmol, 4 eq) were dissolved in 1,4-dioxane (4 mL), stirred to react at 120°C for 4 h. After the reaction was completed, the system was cooled to room temperature and diluted with water. The reaction mixture was extracted with ethyl acetate (3×50 mL). The organic phases were combined, backwashed with saturated sodium chloride solution (1×50 mL), dried over anhydrous sodium sulfate, filtered, and the filtrate was concentrated under reduced pressure. The resulting crude product was purified by silica gel column chromatography (dichloromethane/methanol=10:1) to give compound **001-11** (380 mg, 80.20%).
LCMS: (ESI, m/z): 483.90 $[M+H]^+$.

**Step 11: Synthesis of compound 001-12**

At room temperature, tert-butyl N-[3-(7-{[(3S,4R)-3-fluoro-1-methylpiperidin-4-yl]amino}-3-(2,2,2-trifluoroethyl) pyrazolo[1,5-a]pyridin-2-yl)prop-2-yn-1-yl]carbamate (compound **001-11,** 100 mg, 0.207 mmol, 1 eq) was added to a reaction flask, dissolved in methanol (2 mL), and 4 M HCl/1,4-dioxane (2 mL) was added dropwise at 0°C. The system was allowed to react at room temperature for 1 h. After the reaction was completed, the resulting residue was concentrated in vacuum to give compound **001-12.**
LCMS: (ESI, m/z): 383.85 $[M+H]^+$.

**Step 12: Synthesis of 1-tert-butyl-N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-1*H*-pyrazole-4-carboxamide (compound 001)**

At room temperature, N-hydroxybenzotriazole (22.21 mg, 0.0164 mmol, 1.5 eq), 1-(3-dimethylaminopropyl)-3-ethylcarbodiimide hydrochloride (31.51 mg, 0.164 mmol, 1.5 eq), N,N-diisopropylethylamine (42.49 mg, 0.329 mmol, 3 eq) and N,N-dimethylformamide (3 mL) were added to a reaction flask and stirred for 5 min, and 2-(3-aminoprop-1-yn-1-yl)-N-((3S,4R)-3-fluoro-1-methylpiperidin-4-yl)-3-(2,2,2-trifluoroethyl)pyrazolo[1,5-a]pyridin-7-amine dihydrochloride (compound **001-12,** 50 mg, 0.110 mmol, 1 eq) and 1-tert-butyl-1H-pyrazole-4-carboxylic acid (22.12 mg, 0.156 mmol, 1.32 eq) were added. The system was stirred at room temperature to react for 2 h. After the reaction was completed, the reaction mixture was extracted with ethyl acetate (3×30 mL). The organic phases were combined, backwashed with saturated sodium chloride solution (3×30 mL), dried over anhydrous sodium sulfate, filtered, and the filtrate was concentrated under reduced pressure. The crude product was purified by HPLC with the following conditions (column specifications: Sunfire C18 5 μm, 30 mm×150 mm; mobile phase A: water (0.1% ammonium bicarbonate), mobile phase B: acetonitrile; flow rate: 60 mL/min; elution gradient: 32% to 55% in 10 min; detection wavelength: UV 254 nm/220 nm; retention time (min): 9.73), to give compound **1-tert-butyl-N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-1*H*-pyrazole-4-carboxamide 001** (19.18 mg, 27.23%).
LCMS: (ESI, m/z): 534.25 $[M+H]^+$.
$^1H$ NMR (400 MHz, DMSO) $\delta$ 8.62 (t, J = 5.5 Hz, 1H), 8.32 (s, 1H), 7.90 (s, 1H), 7.28 (t, J = 8.2 Hz, 1H), 7.02 (d, J =

8.7 Hz, 1H), 6.32 (d, J = 7.6 Hz, 1H), 6.22 (d, J = 9.0 Hz, 1H), 4.93 (s, 0.5H), 4.81 (s, 0.5H), 4.34 (d, J = 5.5 Hz, 2H), 3.94 - 3.68 (m, 3H), 3.05 (t, J = 10.9 Hz, 1H), 2.78 (d, J = 11.3 Hz, 1H), 2.38 - 2.23 (m, 1H), 2.20 (s, 3H), 2.13 (t, J = 10.6 Hz, 1H), 1.99 - 1.80 (m, 2H), 1.53 (s, 9H).

Example 2

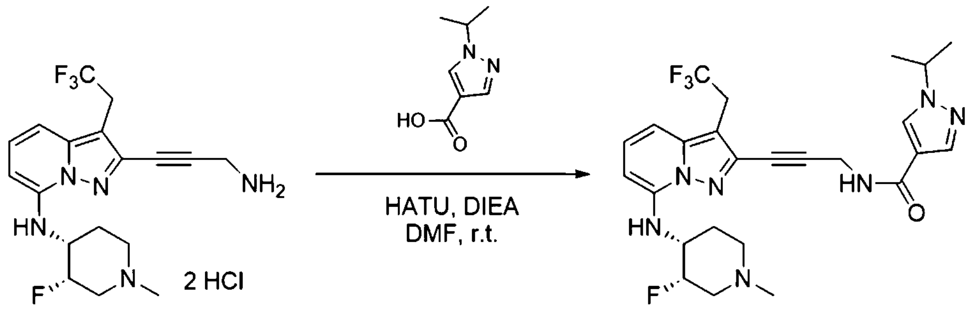


**001-12** **003**

**Step 1: Synthesis of N-[3-(7-{[(3S,4R)-3-fluoro-1-methylpiperidin-4-yl]amino}-3-(2,2,2-trifluoroethyl)pyrazolo [1,5-a]pyridin-2-yl)prop-2-yn-1-yl)-1-propyl]-1*H*-pyrazole-4-carboxamide (compound 003)**

At room temperature, a solution of 1-isopropyl-1*H*-pyrazole-4-carboxylic acid (16.2 mg, 0.106 mmol, 1.2 eq), *N*, *N*-diisopropylethylamine (56.65 mg, 0.440 mmol, 5 eq) and O-(7-azabenzotriazol-1-yl)-*N*,*N*,*N'*,*N'*-tetramethyluronium hexafluorophosphate (2-(7-azobenzotriazole)-*N*,*N*,*N'*,*N'*-tetramethyluronium hexafluorophosphate) (40 mg, 0.106 mmol, 1.2 eq) in N,N-dimethylformamide (1 mL) was stirred to react for 30 min, then 2-(3-aminoprop-1-yn-1-y1)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 40 mg, 0.104 mmol, 1 eq) was added, and the mixture was stirred to react for 2 h. After the reaction was completed, the reaction mixture was quenched with water and extracted with ethyl acetate (3 × 10 mL) at room temperature. The organic phases were combined, backwashed with saturated sodium chloride solution (3 × 10 mL), dried over anhydrous sodium sulfate, filtered, and the filtrate was concentrated under reduced pressure. The crude product was purified by HPLC with the following conditions (column specifications: Sunfire C18 5μm, 30 mm × 150 mm; mobile phase A: water (0.1% ammonium bicarbonate), mobile phase B: acetonitrile; flow rate: 60 mL/min; elution gradient: 27% to 54% in 10 min; detection wavelength: UV 254 nm/220 nm; retention time (min): 8.48) to give compound **N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl)-1-propyl]-1*H*-pyrazole-4-carboxamide 003** (16.65 mg, 36.45%).

LCMS: (ESI, m/z): 520.15 $[M+H]^+$.

$^1$H NMR (400 MHz, DMSO-$d_6$) δ 8.65 (t, J = 5.5 Hz, 1H), 8.26 (s, 1H), 7.89 (s, 1H), 7.29 (t, J = 8.23 Hz, 1H), 7.03 (d, J = 8.7 Hz, 1H), 6.32 (d, J = 7.6 Hz, 1H), 6.25 (d, J = 9.0 Hz, 1H), 4.95 (s, 0.5H), 4.82 (s, 0.5H), 4.51 (hept, *J* = 6.7 Hz, 1H), 4.32 (d, *J* = 5.6 Hz, 2H),3.94 - 3.83 (m, 1H), 3.76 (q, J = 11.1 Hz, 2H), 3.08 (t, J = 11.0 Hz, 1H), 2.81 (d, J = 11.4 Hz, 1H), 2.40 - 2.25 (m, 1H), 2.22 (s, 3H), 2.17 (t, J = 10.8 Hz, 1H), 2.02 - 1.80 (m, 2H), 1.42 (d, J = 6.7 Hz, 6H).

**Example 3**

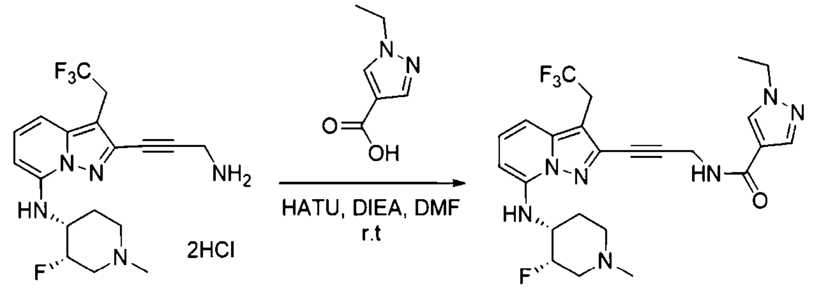


001-12 004

**Step 1: Synthesis of 1-ethyl-N-[3-(7-{[(3S,4R)-3-fluoro-1-methylpiperidin-4-yl]amino}-3-(2,2,2-trifluoroethyl) pyrazolo[1,5-a]pyridin-2-yl)prop-2-yn-1-yl]-1*H*-pyrazole-4-carboxamide (compound 004)**

Under nitrogen protection at room temperature, to a solution of 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 40 mg, 0.088 mmol, 1 eq) and 1-ethyl-1*H*-pyrazole-4-carboxylic acid (18.43 mg, 0.132 mmol, 1.5 eq) in *N*,*N*-dimethylformamide (0.8 mL) was added *N*,*N*-diisopropylethylamine (56.65 mg, 0.440 mmol, 5 eq), and the mixture was stirred to react for 1 h. Next, O-(7-azabenzotriazol-1-yl)-*N*,*N*,*N'*,*N'*-tetramethyluronium hexafluorophosphate (2-(7-azobenzotriazole)-*N*,*N*,*N'*,*N'*-tetramethyluronium hexafluorophosphate) (50.00 mg, 0.132 mmol, 1.5 eq) was added and the system was further stirred for 1 h. After the reaction was completed, methanol (1.0 mL) was added to quench the reaction. The crude product was purified by HPLC with the following conditions (column specifications: YMC Triart C18 ExRs 5 μm, 30 mm × 150 mm; mobile phase A: water (10 mmol/L ammonium bicarbonate), mobile phase B: acetonitrile; flow rate: 60 mL/min; elution gradient: 31% B to 54% B in 10 min; detection wavelength: UV 254 nm/220 nm; retention time (min): 8.63), to give compound **1-ethyl-N-[3-(7-{[(3S,4R)-3-fluoro-1-methylpiperidin-4-yl] amino}-3-(2,2,2-trifluoroethyl)pyrazolo [1,5-a]pyrazin-2-yl)prop-2-yn-1-yl]-1*H*-pyrazole-4-carboxamide 004** (21.27 mg, 47.61%).

LCMS: (ESI, m/z): 506.10 $[M+H]^+$.

$^1$H NMR (400 MHz, DMSO-$d_6$) δ 8.65 (t, *J* = 5.6 Hz, 1H), 8.22 (d, *J* = 0.7 Hz, 1H), 7.89 (d, *J* = 0.7 Hz, 1H), 7.28 (dd, *J* = 8.7, 7.6 Hz, 1H), 7.03 (d, *J* = 8.7 Hz, 1H), 6.32 (d, *J* = 7.3 Hz, 1H), 6.23 (d, *J* = 9.1 Hz, 1H), 4.87 (d, *J* = 49.6 Hz, 1H), 4.33 (d, *J* = 5.6 Hz, 2H), 4.15 (q, *J* = 7.3 Hz, 2H), 3.92 - 3.81 (m, 1H), 3.75 (q, *J* = 11.2 Hz, 2H), 3.05 (t, J = 10.9 Hz, 1H), 2.78 (d, *J* = 11.3 Hz, 1H), 2.37 - 2.22 (m, 1H), 2.20 (s, 3H), 2.18 - 2.10 (m, 1H), 1.99-1.70 (m, 2H), 1.37 (t, *J* = 7.3 Hz, 3H).

Example 4

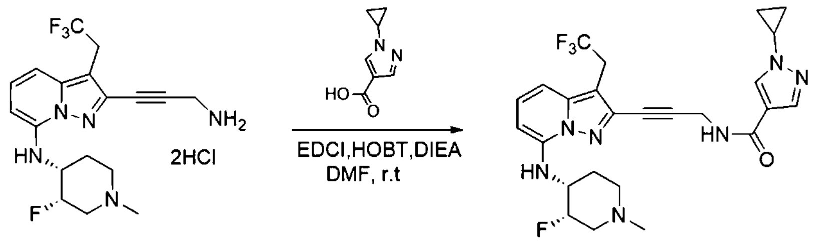


001-12 005

**Step 1: Synthesis of 1-cyclopropyl-N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-1*H*-pyrazole-4-carboxamide (compound 005)**

Under nitrogen protection, 1-cyclopropyl-1*H*-pyrazole-4-carboxylic acid (14.29 mg, 0.094 mmol, 1.2 eq), 1-ethyl-(3-dimethylaminopropyl)carbodiimide hydrochloride (22.5 mg, 0.117 mmol, 1.5 eq), 1-hydroxy-benzotriazole (15.86 mg, 0.117 mmol, 1.5 eq), *N*,*N*-diisopropylethylamine (50.57 mg, 0.390 mmol, 5 eq) were added to a reaction flask, dissolved in *N*,*N*-dimethylformamide (2 mL) and stirred at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 30 mg, 0.078 mmol, 1 eq) was added, and the system was stirred to react at room temperature for 1 h. After the reaction was completed, water was added to the reaction mixture and the system was extracted with ethyl acetate (20 mL × 2). The organic phases were combined, backwashed with saturated sodium chloride solution (10 mL ×

3), dried over anhydrous sodium sulfate, and filtered, and the filtrate was concentrated under reduced pressure. The crude product was purified by HPLC with the following conditions (column specifications: YMC Triart C18 ExRs 5 μm, 30 mm × 150 mm; mobile phase A: water (10 mmol/L ammonium bicarbonate), mobile phase B: acetonitrile; flow rate: 60 mL/min; elution gradient: 33% B to 60% B in 10 min; detection wavelength: UV 254 nm/220 nm; retention time (min): 7.95) to give compound **1-cyclopropyl-N-[3-(7-{[(3S,4R)-3-fluoro-1-methylpiperidin-4-yl]amino}-3-(2,2,2-trifluoroethyl)pyrazolo[1,5-a]pyridin-2-yl)-prop-2-yn-1-yl]-1*H*-pyrazole-4-carboxamide 005** (17.74 mg, 43.63%).

LCMS: (ESI, m/z): 517.85 $[M+H]^+$.

$^1H$ NMR (400 MHz, DMSO-$d_6$) $\delta$ 8.63 (t, *J* = 5.6 Hz, 1H), 8.26 (s, 1H), 7.86 (s, 1H), 7.28 (dd, *J* = 8.7, 7.6 Hz, 1H), 7.03 (d, *J* = 8.6 Hz, 1H), 6.32 (d, J = 7.5 Hz, 1H), 6.23 (d, *J* = 9.0 Hz, 1H), 4.88 (d, *J* = 49.2 Hz, 1H), 4.33 (d, *J* = 5.6 Hz, 2H), 3.87 - 3.70 (m, 4H), 3.06 (t, *J* = 11.3 Hz, 1H), 2.79 (d, *J* = 11.4 Hz, 1H), 2.39 - 2.23 (m, 1H), 2.21 (s, 3H), 2.15 (t, J = 10.5 Hz, 1H), 1.99 - 1.80 (m, 2H), 1.09 - 0.92 (m, 4H).

**Example 5**

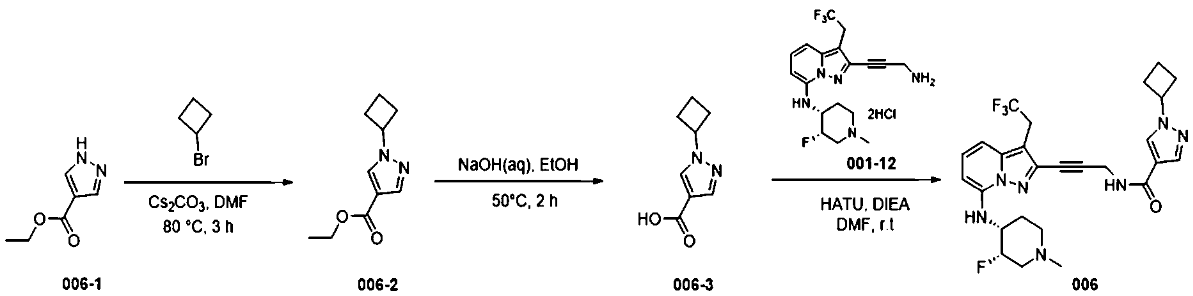


006-1 006-2 006-3 006

**Step 1: Synthesis of compound 006-2**

Ethyl 1*H*-pyrazole-4-carboxylate (compound **006-1,** 1 g, 7.136 mmol, 1 eq), cyclobutyl bromide (1.93 g, 14.272 mmol, 2 eq), and cesium carbonate (6.97 g, 21.408 mmol, 3 eq) were added to a reaction flask, dissolved in *N*,*N*-dimethylformamide (10 mL), and allowed to react at 80°C for 3 h. After the reaction was completed, the reaction mixture was poured into water and extracted with ethyl acetate (3 × 30 mL). The organic phases were combined, backwashed with saturated sodium chloride solution (1 × 30 mL), dried over anhydrous sodium sulfate, filtered, and the filtrate was concentrated under reduced pressure. The resulting crude product was purified by silica gel column chromatography (petroleum ether/ethyl acetate = 5:1) to give compound **006-2** (1.5 g, 97.40%).

LCMS: (ESI, m/z): 195.10 $[M+H]^+$.

**Step 2: Synthesis of compound 006-3**

To a reaction flask, ethyl 1-cyclobutyl-1*H*-pyrazole-4-carboxylate (compound **006-2,** 200 mg, 1.030 mmol, 1 eq) was added and dissolved in ethanol (2 mL). Then, sodium hydroxide (10M, 2 mL, 10 mmol, 10 eq) was added and the mixture was allowed to react at 50°C for 2 h. After the reaction was completed, the solvent was removed by spin drying, and some water was added. The pH was adjusted to 2 using 1M aqueous hydrochloric acid solution. The reaction mixture was extracted with ethyl acetate (3 × 30 mL). The organic phases were combined, backwashed with saturated sodium chloride solution (1 × 30 mL), dried over anhydrous sodium sulfate and filtered. The filtrate was concentrated under reduced pressure to give crude compound **006-3** (190 mg).

**Step 3: Synthesis of 1-cyclobutyl-N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-1*H*-pyrazole-4-carboxamide (compound 006)**

1-cyclobutyl-1*H*-pyrazole 4-carboxylic acid (compound **006-3,** 30 mg, 0.181 mmol, 1 eq), 2-(3-aminoprop-1-yn-1-yl)-N-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 69.21 mg, 0.181 mmol, 1 eq), *O*-(7-azabenzotriazol-1-yl)-*N*,*N*,*N'*,*N'*-tetramethyluronium hexafluorophosphate (102.96 mg, 0.271 mmol, 1.5 eq), and *N*,*N*-diisopropylethylamine (116.66 mg, 0.905 mmol, 5 eq) were added to a reaction flask, dissolved in N,N-dimethylformamide (3 mL) and stirred at room temperature for 1 h. The reaction was quenched with water, and the reaction mixture was extracted with ethyl acetate (3 × 10 mL). The organic phases were combined, backwashed with saturated sodium chloride solution (1 × 10 mL), and dried over sodium sulfate. The resulting

mixture was filtered, and then the filtrate was concentrated under reduced pressure. The crude product was purified by HPLC with the following conditions (column specifications: XBridge BEH C18 OBD Prep Column 130, 5 μm, 30 mm × 150 mm; mobile phase A: water (10 mmol/L ammonium bicarbonate), B: acetonitrile; flow rate: 60 mL/min; gradient: 36% B 56% B in 10 min; wavelength: UV 254 nm/220 nm; retention time (min): 9.2) to give compound **1-cyclobutyl-N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-1*H*-pyrazole-4-carboxamide 006.**

LCMS: (ESI, m/z): 531.85 $[M+H]^+$.

$^1H$ NMR (400 MHz, DMSO-$d_6$) $\delta$ 8.64 (t, *J* = 5.6 Hz, 1H), 8.29 (s, 1H), 7.91 (s, 1H), 7.28 (t, *J* = 8.2 Hz, 1H), 7.02 (d, *J* = 8.7 Hz, 1H), 6.32 (d, *J* = 7.6 Hz, 1H), 6.22 (d, *J* = 9.1 Hz, 1H), 4.95 - 4.79 (m, 2H), 4.33 (d, *J* = 5.5 Hz, 2H), 3.91 - 3.69 (m, 3H), 3.05 (t, J = 11.0 Hz, 1H), 2.78 (d, *J* = 11.5 Hz, 1H), 2.49 - 2.29 (m, 5H), 2.20 (s, 3H), 2.13 (t, *J* = 10.8 Hz, 1H), 1.96 - 1.72 (m, 4H).

**Example 6**

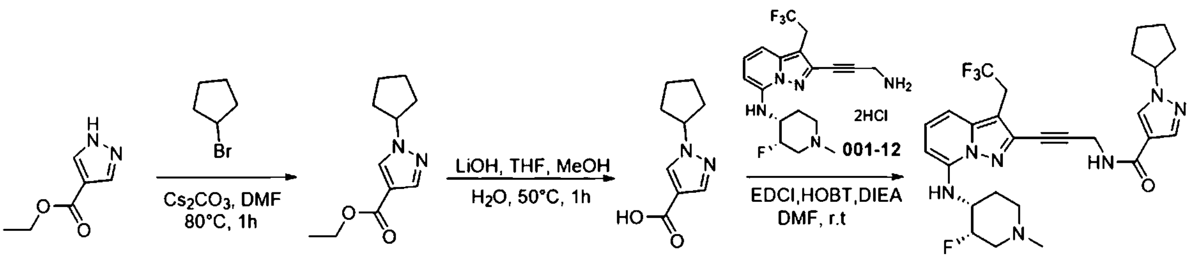


006-1 007-1 007-2 007

**Step 1: Synthesis of compound 007-1**

Under nitrogen protection, ethyl 1*H*-pyrazole-4-carboxylate (compound **006-1,** 500 mg, 3.568 mmol, 1 eq), bromocyclopentane (1063.43 mg, 7.136 mmol, 2 eq), and cesium carbonate (3487.38 mg, 10.704 mmol, 3 eq) were added to a reaction flask, dissolved in *N*,*N*-dimethylformamide (5 mL), warmed to 80°C and stirred to react for 1 h. After the reaction was completed, the system was extracted with ethyl acetate (2 × 20 mL). The organic phases were combined, backwashed with saturated sodium chloride solution (3 × 10 mL), dried over anhydrous sodium sulfate, and filtered, and the filtrate was concentrated under reduced pressure. The resulting residue was purified by silica gel column chromatography (dichloromethane/anhydrous methanol = 20:1) to give compound **007-1** (450 mg, 57.29%).

LCMS: (ESI, m/z): 209.05 $[M+H]^+$.

**Step 2: Synthesis of compound 007-2**

Under nitrogen protection, ethyl 1-cyclopentyl-1*H*-pyrazole-4-carboxylate (compound **007-1,** 200 mg, 0.960 mmol, 1 eq) and lithium hydroxide (115.00 mg, 4.800 mmol, 5 eq) were added to a reaction flask, and dissolved in tetrahydrofuran (1 mL)/water (1 mL)/anhydrous methanol (1 mL). The system was warmed to 50°C and stirred to react for 1 h. After the reaction was completed, the reaction mixture was neutralized to pH=7 with 1 mol/L hydrochloric acid aqueous solution. The mixture was concentrated under reduced pressure. To the resulting residue was added 10 mL of tetrahydrofuran, the system was stirred for 5 min, filtered, and the filtrate was concentrated under reduced pressure to give compound **007-2** (95 mg, 54.29%).

LCMS: (ESI, m/z): 181.00 $[M+H]^+$.

**Step 3: Synthesis of 1-cyclopentyl-N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-1*H*-pyrazole-4-carboxamide (compound 007)**

Under nitrogen protection, 1-cyclopentyl-1*H*-pyrazole-4-carboxylic acid (compound **007-2,** 14.1 mg, 0.078 mmol, 1.5 eq), 1-ethyl-(3-dimethylaminopropyl)carbodiimide hydrochloride (15 mg, 0.078 mmol, 1.5 eq), 1-hydroxybenzotriazole (10.6 mg, 0.078 mmol, 1.5 eq), *N*, *N*-diisopropylethylamine (33.7 mg, 0.260 mmol, 5 eq) were added to a reaction flask, dissolved in *N*,*N*-dimethylformamide (2 mL) and stirred at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.052 mmol, 1 eq) was added, and the system was allowed to react for 1 h.

After the reaction was completed, the system was extracted with ethyl acetate (2 × 20 mL). The organic phases were combined, backwashed with saturated sodium chloride solution (3 × 10 mL), dried over anhydrous sodium sulfate, and filtered, and the filtrate was concentrated under reduced pressure. The crude product was purified by HPLC with the following conditions (XBridge BEH C18 OBD Prep Column 130Å, 5 μm, 30 mm ×150 mm; mobile phase A: water (10 mmol/L ammonium bicarbonate), mobile phase B: acetonitrile; flow rate: 60 mL/min; elution gradient: 34% B to 60% B in 10 min; detection wavelength: UV 254 nm/220 nm; retention time (min): 7.02) to give compound **1-cyclopentyl-N-[3-(7-{[(3S,4R)-3-fluoro-1-methylpiperidin-4-yl]amino}-3-(2,2,2-trifluoroethyl)pyrazolo[1,5-a]pyridin-2-yl)-prop-2-yn-1-yl]-1*H*-pyrazole-4-carboxamide 007** (7.1 mg, 24.37%).

LCMS: (ESI, m/z): 545.90 $[M+H]^+$.

$^1$H NMR (400 MHz, DMSO-$d_6$) δ 8.62 (t, *J* = 5.6 Hz, 1H), 8.25 (s, 1H), 7.88 (s, 1H), 7.28 (dd, *J* = 8.8, 7.6 Hz, 1H), 7.02 (d, *J* = 8.8 Hz, 1H), 6.32 (dd, *J* = 7.3 Hz, 1H), 6.22 (d, *J* = 9.0 Hz, 1H), 4.87 (d, *J* = 49.5 Hz, 1H), 4.77 - 4.66 (m, 1H), 4.33 (d, *J* = 5.6 Hz, 2H), 3.93 - 3.81 (m, 1H), 3.75 (q, *J* = 11.1 Hz, 2H), 3.05 (t, *J* = 11.5 Hz, 1H), 2.78 (d, *J* = 11.6 Hz, 1H), 2.37 - 2.29 (m, 1H), 2.26 - 2.15 (m, 4H), 2.15 - 2.04 (m, 3H), 1.96 - 1.70 (m, 6H), 1.69 - 1.57 (m, 2H).

## Example 7

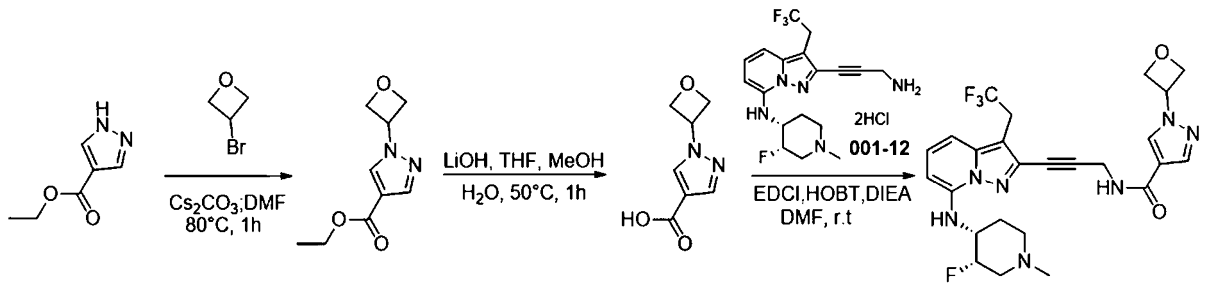


**006-1** **009-1** **009-2** **009**

### Step 1: Synthesis of compound 009-1

Under nitrogen protection, ethyl 1*H*-pyrazole-4-carboxylate (compound **006-1,** 500 mg, 3.568 mmol, 1 eq), 3-bromo-oxetane (977.41 mg, 7.136 mmol, 2 eq)), and cesium carbonate (3487.38 mg, 10.704 mmol, 3 eq) were added to a reaction flask, dissolved in *N*,*N*-dimethylformamide (5 mL), warmed to 80°C and stirred to react for 1 h. After the reaction was completed, the system was extracted with ethyl acetate (20 mL × 2). The organic phases were combined, backwashed with saturated sodium chloride solution (10 mL × 3), and dried over anhydrous sodium sulfate. The system was filtered, and the filtrate was concentrated under reduced pressure. The resulting crude product was purified by silica gel column chromatography (dichloromethane/anhydrous methanol = 18:1) to give compound **009-1** (480 mg, 64.25%).

LCMS: (ESI, m/z): 197.00 $[M+H]^+$.

### Step 2: Synthesis of compound 009-2

Under nitrogen protection, ethyl 1-(oxetan-3-yl)-1*H*-pyrazole-4-carboxylate (compound **009-1**, 200 mg, 0.960 mmol, 1 eq) and lithium hydroxide (115.00 mg, 4.800 mmol, 5 eq) were added to a reaction flask, and dissolved in tetrahydrofuran (1 mL)/water (1 mL)/anhydrous methanol (1 mL). The system was warmed to 50°C and stirred to react for 1 h. After the reaction was completed, the reaction mixture was neutralized to pH=7 with 1 mol/L hydrochloric acid aqueous solution. The mixture was concentrated under reduced pressure. To the resulting residue was added 10 mL of tetrahydrofuran, and the system was stirred for 5 min, and filtered. The filtrate was concentrated under reduced pressure to give compound **009-2** (95 mg, 54.29%).

LCMS: (ESI, m/z): 169.00 $[M+H]^+$.

### Step 3: Synthesis of N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-1-(oxetan-3-yl)-1*H*-pyrazole-4-carboxamide (compound 009)

Under nitrogen protection, 1-(oxetan-3-yl)-1*H*-pyrazole-4-carboxylic acid (compound **009-2,** 13.2 mg, 0.078 mmol, 1.5 eq), 1-ethyl-(3-dimethylaminopropyl) carbodiimide hydrochloride (15 mg, 0.078 mmol, 1.5 eq), 1-hydroxybenzotriazole (10.6 mg, 0.078 mmol, 1.5 eq), and *N*, *N*-diisopropylethylamine (33.7 mg, 0.260 mmol, 5 eq) were added to a reaction flask, and dissolved in *N*,*N*-dimethylformamide (2 mL). The mixture was stirred at room temperature for 10 min.

Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.052 mmol, 1 eq) was added and the mixture was stirred to react at room temperature for 1 h. The desired product was found by LC-MS. The reaction mixture was extracted with ethyl acetate (20 mL × 2). The organic phases were combined, backwashed with saturated sodium chloride solution (10 mL × 3), and dried over anhydrous sodium sulfate. The resulting mixture was filtered, and then the filtrate was concentrated under reduced pressure. The crude product was purified by HPLC with the following conditions (column specifications: Sunfire C18 5 μm, 30 mm × 150 mm; mobile phase A: water (0.1% formic acid), mobile phase B: acetonitrile; flow rate: 60 mL/min; elution gradient: 5% B to 29% B in 10 min; detection wavelength: UV 254 nm/220 nm; retention time (min): 8.53) to give compound **N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl)-1-(oxetan-3-yl)-1*H*-pyrazole-4-carboxamide 009** (5.79 mg, 20.80%).

LCMS: (ESI, m/z): 534.05 $[M+H]^+$.

$^1$H NMR (400 MHz, DMSO-$d_6$) $\delta$ 8.75 - 8.70 (m, 1H), 8.37 (s, 1H), 8.04 (s, 1H), 7.28 (t, *J* = 8.3 Hz, 1H), 7.03 (d, *J* = 8.6 Hz, 1H), 6.32 (d, *J* = 7.7 Hz, 1H), 6.23 (d, *J* = 8.7 Hz, 1H), 5.61 (m, 1H), 4.92 (t, J = 7.1 Hz, 2H), 4.87 (t, J = 6.4 Hz, 2H), 4.82 (s, 1H), 4.34 (d, *J* = 5.4 Hz, 2H), 3.92 - 3.81 (m, 1H), 3.75 (q, *J* = 11.2 Hz, 2H), 3.06 (m, 1H), 2.80 (d, *J* = 11.3 Hz, 1H), 2.41 - 2.28 (m, 1H), 2.22 (d, *J* = 2.1 Hz, 3H), 2.14 (m, 1H), 1.94 - 1.83 (m, 2H).

## Example 8

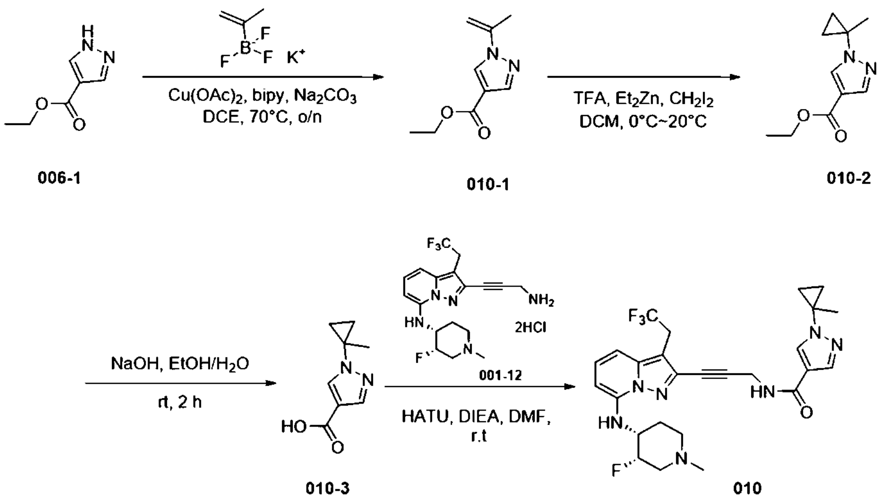


006-1 010-1 010-2 010-3 010

### Step 1: Synthesis of compound 010-1

At room temperature, ethyl 1*H*-pyrazole-4-carboxylate (compound **006-1,** 1 g, 7.136 mmol, 1 eq), potassium isopropenyl trifluoroborate (2.11 g, 14.272 mmol, 2 eq), 2,2'-bipyridine (2.23 g, 14.272 mmol, 2 eq), copper acetate (2.59 g, 14.272 mmol, 2 eq) and sodium carbonate (1.51 g, 14.272 mmol, 2 eq) were added to a reaction flask, and dissolved in dichloroethane (10 mL). Oxygen was introduced and the reaction was carried out at 70°C overnight. After the reaction was completed, the reaction mixture was quenched with a saturated solution of ethylenediamine tetraacetic acid disodium salt, filtered, and the filtrate was extracted with ethyl acetate (3 × 30 mL). The organic phases were combined, backwashed with a saturated sodium chloride solution (3 × 20 mL), and dried over anhydrous sodium sulfate. The system was filtered and the filtrate was concentrated under reduced pressure. The resulting residue was purified by silica gel column chromatography (petroleum ether/ethyl acetate = 5:1) to give compound **010-1** (900 mg, 69.99%).

LCMS: (ESI, m/z): 180.95 $[M+H]^+$.

### Step 2: Synthesis of compound 010-2

Under nitrogen protection, diethyl zinc (2.74 g, 22.195 mmol, 5 eq) was added to a solution of trifluoroacetic acid (2.53 g, 22.195 mmol, 5 eq) in dichloromethane (10 mL) at 0°C. After the mixture was stirred to react for 20 min, diiodomethane (11.89 g, 44.390 mmol, 10 eq) was added dropwise at 0°C. After the mixture was stirred to react for 20 min, a solution of ethyl 1-(prop-1-en-2-yl)-1*H*-pyrazole-4-carboxylate (compound **010-1,** 800 mg, 4.439 mmol, 1 eq) in dichloromethane (3 mL) was added dropwise at 0°C. The system was stirred to react at 20°C for 16 h. After the reaction was completed, the reaction mixture was quenched with water and extracted with ethyl acetate (3 × 30 mL). The organic

phases were combined, backwashed with saturated sodium chloride solution (3 × 30 mL), dried over anhydrous sodium sulfate, filtered, and the filtrate was concentrated under reduced pressure. The crude product was purified by HPLC with the following conditions (column specifications: Sunfire C18 5 μm, 30 mm × 150 mm; mobile phase A: water (0.1% formic acid), mobile phase B: acetonitrile; flow rate: 60 mL/min; elution gradient: 2% to 55% in 10 min; detection wavelength: UV 254 nm/220 nm; retention time (min): 9.73) to give compound **010-2** (90 mg, 10.44%).

LCMS: (ESI, m/z): 195.45 $[M+H]^+$.

**Step 3: Synthesis of compound 010-3**

Under nitrogen protection, at room temperature, a solution of ethyl 1-(1-methylcyclopropyl)-1*H*-pyrazole-4-carboxylate (compound **010-2,** 70 mg, 0.360 mmol, 1 eq) and sodium hydroxide (28.83 mg, 0.720 mmol, 2 eq) in ethanol (2 mL) and water (2 mL) was stirred for 2 hours. After the reaction was completed, the reaction mixture was diluted with water at room temperature, acidified to pH=5 with 1 mol/L hydrochloric acid solution, and extracted with ethyl acetate (3 × 20 mL). The organic phases were combined, backwashed with saturated sodium chloride solution (1 × 30 mL), dried over anhydrous sodium sulfate, filtered, and the filtrate was concentrated under reduced pressure to give compound **010-3** (50 mg, 83.49%) without further purification.

LCMS: (ESI, m/z): 167.40 $[M+H]^+$.

**Step 4: Synthesis of N-[3-(7-{[(3S,4R)-3-fluoro-1-methylpiperidin-4-yl]amino}-3-(2,2,2-trifluoroethyl)pyrazolo [1,5-a]pyridin-2-yl)prop-2-yn-1-yl]-1-(1-methylcyclopropyl)-1*H*-pyrazole-4-carboxamide (compound 010)**

At room temperature, a solution of 1-(1-methylcycopropyl)-1*H*-pyrazole-4-carboxylic acid (compound **010-3,** 10 mg, 0.060 mmol, 1 eq), O-(7-azabenzothiazol-1-yl)-*N,N,N',N'*-tetramethyluronium hexafluorophosphate (2-(7-azobenzothiazole)-*N,N,N',N'*-tetramethyluronium hexafluorophosphate) (27.46 mg, 0.072 mmol, 1.2 eq) and *N,N*-diisopropylethylamine (38.89 mg, 0.300 mmol, 5 eq) in *N,N*-dimethylformamide (1 mL) was stirred to react for 30 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 27.46 mg, 0.060 mmol, 1 eq) was added and the system was stirred to react for 2 h. After the reaction was completed, the reaction mixture was quenched with water and extracted with ethyl acetate (3 × 30 mL). The organic phases were combined, backwashed with saturated sodium chloride solution (3 × 30 mL), dried over anhydrous sodium sulfate, filtered, and the filtrate was concentrated under reduced pressure. The crude product was purified by HPLC with the following conditions (column specifications: Sunfire C18 5 μm, 30 mm × 150 mm; mobile phase A: water (0.1% ammonium bicarbonate), mobile phase B: acetonitrile; flow rate: 60 mL/min; elution gradient: 35% to 57% in 10 min; detection wavelength: UV 254 nm/220 nm; retention time (min): 8.52), to give compound **N-[3-(7-1[(3S,4R)-3-fluoro-1-methylpiperidin-4-yl]amino)-3-(2,2,2-trifluoroethyl)pyrazolo[1,5-a]pyridin-2-yl)prop-2-yn-1-yl]-1-(1-methylcyclopropyl)-1*H*-pyrazole-4-carboxamide 010** (15.49 mg, 48.18%).

LCMS: (ESI, m/z): 532.10 $[M+H]^+$.

$^1$H NMR (400 MHz, DMSO-$d_6$) δ 8.63 (t, J = 5.5 Hz, 1H), 8.29 (s, 1H), 7.87 (s, 1H), 7.27 (t, J = 8.0 Hz, 1H), 7.03 (d, J = 8.7 Hz, 1H), 6.32 (d, J = 7.5 Hz, 1H), 6.22 (d, J = 9.0 Hz, 1H), 4.87 (d, J = 49.8 Hz, 1H), 4.33 (d, J = 5.5 Hz, 2H), 3.92 - 3.80 (m, 1H), 3.75 (q, 11.2 Hz, 2H), 3.05 (t, J = 10.6 Hz, 1H), 2.78 (d, J = 11.0 Hz, 1H), 2.36 - 2.24 (m, 1H), 2.20 (s, 3H), 2.13 (t, J = 10.5 Hz, 1H), 1.99 - 1.79 (m, 2H), 1.57 (s, 3H), 1.19 (q, J = 5.3 Hz, 2H), 0.93 (q, J = 5.2 Hz, 2H).

**Example 9**

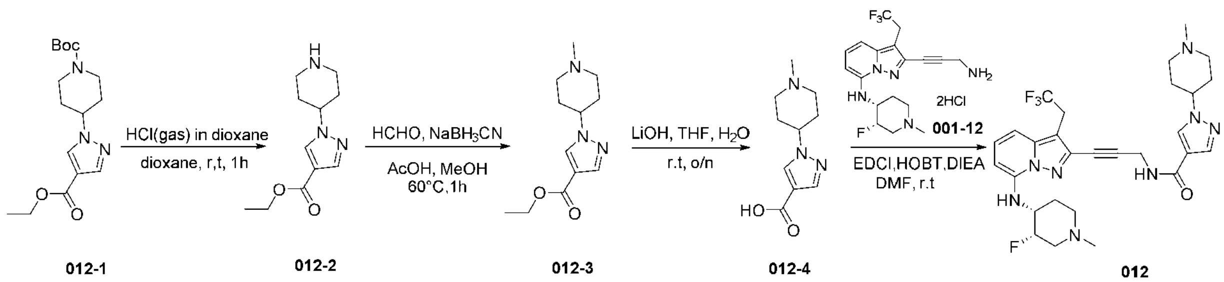


012-1 012-2 012-3 012-4 012

**Step 1: Synthesis of compound 012-2**

Under nitrogen protection, tert-butyl 4-(4-(ethoxycarbonyl)-1*H*-pyrazole-1-yl)piperidine-1-carboxylate (compound **012-1,** 300 mg, 0.928 mmol, 1 eq) was added to a reaction flask, dissolved in 1,4-dioxane (4 mL), and a solution of 4M hydrogen chloride solution in 1,4-dioxane (4 mL) was added. The mixture was stirred to react at room temperature for 1 h. After the reaction was completed, the reaction mixture was basified to pH=8 with saturated sodium carbonate solution, the mixture was concentrated under reduced pressure, water was added, and the system was extracted with dichloromethane (20 mL × 2). The organic phases were combined, backwashed with saturated sodium chloride solution (20 mL × 1), dried over anhydrous sodium sulfate, filtered, and the filtrate was concentrated under reduced pressure. Compound **012-2** (180 mg, 85.08%) was obtained.

LCMS: (ESI, m/z): 224.45 $[M+H]^+$.

**Step 2: Synthesis of compound 012-3**

Under nitrogen protection, ethyl 1-(piperidin-4-yl)-1*H*-pyrazole-4-carboxylate (compound **012-2,** 150 mg, 0.672 mmol, 1 eq), sodium cyanoborohydride (84.43 mg, 1.344 mmol, 2 eq), 37% formaldehyde aqueous solution (40.34 mg, 1.344 mmol, 2 eq), and acetic acid (0.04 mL, 0.067 mmol, 0.1 eq) were added to a reaction flask, dissolved in methanol (5 mL), the system was warmed to 60°C and stirred to react for 1 h. After the reaction was completed, the reaction mixture was quenched with saturated ammonium chloride (10 mL) at 0°C, concentrated under reduced pressure, and the resulting residue was purified by reverse phase column chromatography with the following conditions: C18 column, mobile phase: water (0.1% ammonium bicarbonate) and acetonitrile, 10% to 20% gradient in 10 min, detection wavelength: UV 254 nm. Compound **012-3** (50 mg, 30.64%) was obtained.

LCMS: (ESI, m/z): 238.00 $[M+H]^+$.

**Step 3: Synthesis of compound 012-4**

Under nitrogen protection, ethyl 1-(1-methylpiperidin-4-yl)-1*H*-pyrazole-4-carboxylate (compound **012-3,** 50 mg, 0.211 mmol, 1 eq) and lithium hydroxide (10 mg, 0.422 mmol, 2 eq) were added to a reaction flask, dissolved in water (1.0 mL) and tetrahydrofuran (1.0 mL), and stirred to react at room temperature overnight. After the reaction was completed, the reaction mixture was concentrated under reduced pressure to give compound **012-4** (45 mg, 94.51%).

LCMS: (ESI, m/z): 210.05 $[M+H]^+$.

**Step 4: Synthesis of N-[3-(7-{[(3S,4R)-3-fluoro-1-methylpiperidin-4-yl]amino}-3-(2,2,2-trifluoroethyl)pyrazolo [1,5-a]pyridin-2-yl)prop-2-yn-1-yl]-1-(1-methylpiperidin-4-yl)-1*H*-pyrazole-4-carboxamide (compound 012)**

Under nitrogen protection, 1-(1-methylpiperidin-4-yl)-1*H*-pyrazole-4-carboxylic acid (compound **012-4,** 13.8 mg, 0.066 mmol, 1.5 eq), 1-ethyl-(3-dimethylaminopropyl)carbodiimide hydrochloride (15 mg, 0.078 mmol, 1.5 eq), 1-hydroxybenzotriazole (10.6 mg, 0.078 mmol, 1.5 eq), and *N,N*-diisopropylethylamine (33.7 mg, 0.260 mmol, 5 eq) were added to a reaction flask, dissolved in *N*,*N*-dimethylformamide (2 mL) and stirred at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.052 mmol, 1 eq) was added, and the system was stirred to react at room temperature for 1 h. After the reaction was completed, water was added, and the system was extracted with ethyl acetate (20 mL × 2). The organic phases were combined, backwashed with saturated sodium chloride solution (10 mL × 3), dried over anhydrous sodium sulfate, and filtered, and the filtrate was concentrated under reduced pressure. The crude product was purified by HPLC with the following conditions (column specifications: Xselect CSHTM Prep C18 5μm 30*150mm OBD; mobile phase A: water (0.1% formic acid), mobile phase B: acetonitrile; flow rate: 60 mL/min; elution gradient: 5% B to 35% B in 10 min; detection wavelength: UV 254 nm/220 nm; retention time (min): 4.5) to give the carboxylate of compound **N-[3-(7-{[(3S,4R)-3-fluoro-1-methylpiperidin-4-yl]amino}-3-(2,2,2-trifluoroethyl)pyrazolo[1,5-a]pyridin-2-yl)-prop-2-yn-1-yl]-1-(1-methylpiperidin-4-yl)-1*H*-pyrazole-4-carboxamide 012** (2.24 mg, 8.69%).

$^1$H NMR (400 MHz, Chloroform-d) δ 8.01 (s, 1H), 7.81 (s, 1H), 7.18 (t, J = 7.8 Hz, 1H), 6.88 (d, J = 8.7 Hz, 1H), 6.33 (t, *J* = 5.5 Hz, 1H), 6.20 (d, J = 9.2 Hz, 1H), 5.93 (d, J = 7.5 Hz, 1H), 4.88 (d, J = 48.8 Hz, 1H), 4.53 (d, J = 5.2 Hz, 1H), 4.34 - 4.23 (m, 1H), 3.70 - 3.51 (m, 3H), 3.30 - 3.20 (m, 1H), 2.98 (d, J = 12.2 Hz, 1H), 2.57 (s, 1H), 2.53 (s, 3H), 2.37 (s, 3H), 2.34 - 2.19 (m, 6H), 2.16 - 2.02 (m, 3H).

**Example 10**

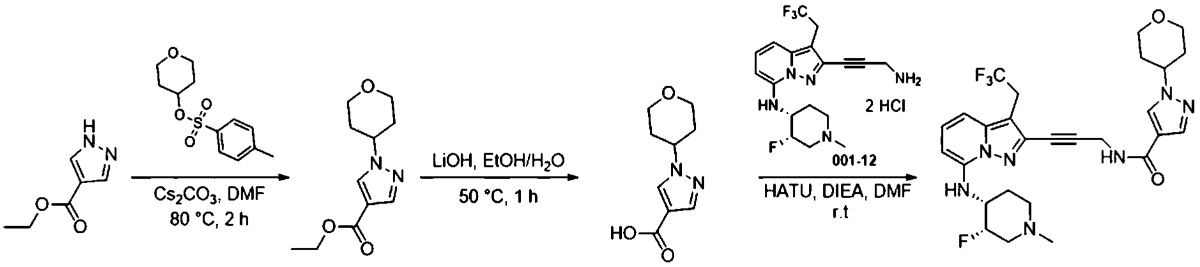


006-1 013-1 013-2 013

**Step 1: Synthesis of compound 013-1**

At 80°C, a solution of ethyl 1*H*-pyrazole-4-carboxylate (compound **006-1,** 400 mg, 2.854 mmol, 1 eq), 4-methylbenzenesulfonic acid oxyalkyl-4-yl ester (877.92 mg, 3.425 mmol, 1.2 eq) and cesium carbonate (2.789 g, 8.562 mmol, 3 eq) in *N,N*-dimethylformamide (10 mL) was stirred to react for 2 h. After the reaction was completed, the reaction mixture was quenched with water at room temperature and extracted with ethyl acetate (3 × 30 mL). The organic phases were combined, backwashed with saturated sodium chloride solution (3 × 30 mL), dried over anhydrous sodium sulfate, and filtered, and the filtrate was concentrated under reduced pressure. The resulting residue was purified by silica gel column chromatography (petroleum ether/ethyl acetate = 5:1) to give compound **013-1** (300 mg, 46.87%).

LCMS: (ESI, m/z): 224.85 $[M+H]^+$.

**Step 2: Synthesis of compound 013-2**

At 50°C, a solution of ethyl 1-(tetrahydro-2*H*-pyran-4-yl)-1*H*-pyrazole-4-carboxylate (compound **013-1,** 250 mg, 1.115 mmol, 1 eq) and lithium hydroxide (53.4 mg, 2.230 mmol, 2 eq) in ethanol (3 mL) and water (3 mL) was stirred to react for 1 h. After the reaction was completed, the reaction mixture was diluted with water at room temperature, acidified to PH=5 with 1 M hydrochloric acid solution, and extracted with ethyl acetate (3 × 20 mL). The organic phases were combined, backwashed with saturated sodium chloride solution (1 × 20 mL), dried over anhydrous sodium sulfate, filtered, and the filtrate was concentrated under reduced pressure to give compound **013-2** (160 mg, 73.15%) without further purification.

LCMS: (ESI, m/z): 197.05 $[M+H]^+$.

**Step 3: Synthesis of N-[3-(7-{[(3S,4R)-3-fluoro-1-methylpiperidin-4-yl]amino}-3-(2,2,2-trifluoroethyl)pyrazolo [1,5-a]pyridin-2-yl)prop-2-yn-1-yl]-1-((tetrahydro-2H-pyran-4-yl))-1*H*-pyrazole-4-carboxamide (compound 013)**

At room temperature, a solution of 1-(tetrahydro-2*H*-pyran-4-yl)-1*H*-pyrazole-4-carboxylic acid (compound **013-2,** 14.04 mg, 0.071 mmol, 1 eq), *N,N*-diisopropylethylamine (46.11 mg, 0.355 mmol, 5 eq) and O-(7-azabenzothiazol-1-yl)-*N,N,N',N'*-tetramethyluronium hexafluorophosphate (2-(7-azobenzothiazole)-*N,N,N',N'*-tetramethyluronium hexafluorophosphate) (32.56 mg, 0.085 mmol, 1.2 eq) in *N,N*-dimethylformamide (1 mL) was stirred to react for 30 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 32.56 mg, 0.071 mmol, 1 eq) was added and the system was stirred to react at room temperature for 2 h. After the reaction was completed, the reaction mixture was quenched with water and extracted with ethyl acetate (3 × 30 mL). The organic phases were combined, backwashed with saturated sodium chloride solution (3 × 30 mL), dried over anhydrous sodium sulfate, filtered, and the filtrate was concentrated under reduced pressure. The crude product was purified by HPLC with the following conditions (column specifications: Sunfire C18 5 μm, 30 mm × 150 mm; mobile phase A: water (0.1% ammonium bicarbonate), mobile phase B: acetonitrile; flow rate: 60 mL/min; elution gradient: 31% to 61% in 10 min; detection wavelength: UV 254 nm/220 nm; retention time (min): 7.45), to give compound **N-[3-(7-{[(3S,4R)-3-fluoro-1-methylpiperidin-4-yl]amino}-3-(2,2,2-trifluoroethyl)pyrazolo[1,5-a] pyridin-2-yl)prop-2-yn-1-yl]-1-(tetrahydro-2H-pyran-4-yl)-1H-pyrazole-4-carboxamide 013** (28.05 mg, 70.00%).

LCMS: (ESI, m/z): 562.15 $[M+H]^+$.

$^1$H NMR (400 MHz, DMSO-$d_6$) δ 8.67 (t, J = 5.3 Hz, 1H), 8.29 (s, 1H), 7.92 (s, 1H), 7.29 (t, J = 8.2 Hz, 1H), 7.03 (d, J = 8.7 Hz, 1H), 6.32 (d, J = 7.6 Hz, 1H), 6.23 (d, J = 9.0 Hz, 1H), 4.94 (s, 0.5H), 4.81 (s, 0.5H), 4.44 (m, 1H), 4.34 (d, J = 5.4 Hz, 2H), 3.98 - 3.92 (m, 2H), 3.90 - 3.82 (m, 1H), 3.75 (m, 2H), 3.54 - 3.41 (m, 2H), 3.06 (t, J = 10.6 Hz, 1H), 2.78 (d, J = 10.8 Hz, 1H), 2.37 - 2.24 (m, 1H), 2.20 (s, 3H), 2.14 (t, J = 10.7 Hz, 1H), 2.03 - 1.80 (m, 6H).

**Example 11**

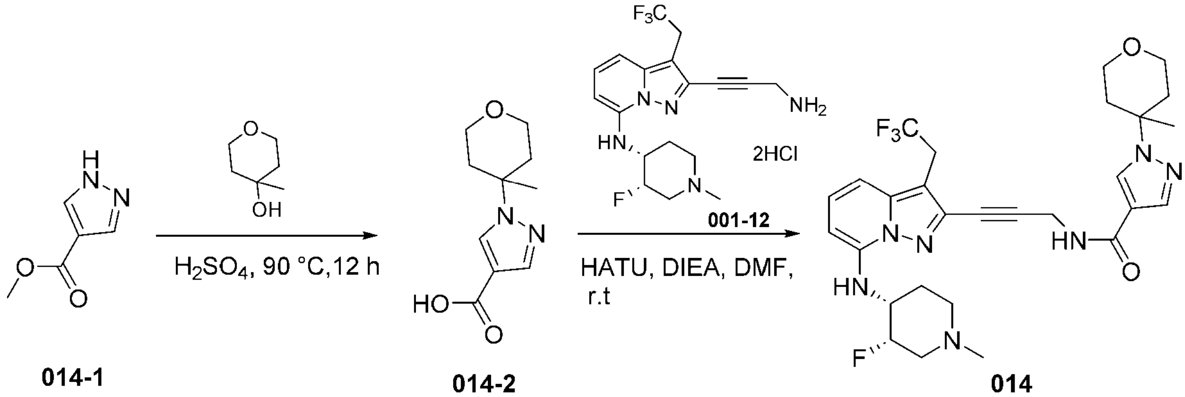


**014-1** **014-2** **014

**Step** 1: **Synthesis of compound 014-2**

Under nitrogen protection, at 90°C, a solution of methyl 1*H*-pyrazole-4-carboxylate (compound **014-1,** 600 mg, 4.758 mmol, 1 eq) and 4-methyltetrahydro-2*H*-pyran-4-ol (3868.47 mg, 33.306 mmol, 7 eq) in sulfuric acid (269 μL) was stirred to react for 12 h. After the reaction was completed, the reaction mixture was concentrated in vacuum. The crude product was purified by HPLC with the following conditions (column specifications: Sunfire C18 5 μm, 30 mm × 150 mm; mobile phase A: water (0.1% trifluoroacetic acid), mobile phase B: acetonitrile; flow rate: 40 mL/min; elution gradient: 5% to 28% in 10 min; detection wavelength: UV 254 nm/220 nm; retention time (min): 7.08) to give compound **014-2** (120 mg, 12.00%).

LCMS: (ESI, m/z): 211.05 [M+H]+.

**Step 2: Synthesis of 3-fluoro-N-[3-(7-{[(3S,4R)-3-fluoro-1-methylpiperidin-4-yl]amino}-3-(2,2,2-trifluoroethyl) pyrazolo[1,5-a]pyridin-2-yl)prop-2-yn-1-yl]-1H-pyrazole-4-carboxamide (compound 014)**

At room temperature, a solution of 1-(4-methyltetrahydro-2*H*-pyran-4-yl)-1*H*-pyrazole-4-carboxylic acid (compound **014-2,** 15.38 mg, 0.073 mmol, 1 eq), *N,N*-diisopropylethylamine (47.28 mg, 0.365 mmol, 5 eq) and O-(7-azabenzothiazol-1-yl)-*N,N,N',N'*-tetramethyluronium hexafluorophosphate (2-(7-azobenzothiazole)-*N*, and *N,N',N'*-tetramethyluronium hexafluorophosphate) (33.38 mg, 0.088 mmol, 1.2 eq) in *N,N*-dimethylformamide (1 mL) was stirred to react for 30 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 40.06 mg, 0.088 mmol, 1.2 eq) was added and the system was stirred at room temperature for 2 h. After the reaction was completed, the reaction mixture was quenched with water and extracted with ethyl acetate (3 × 10 mL). The organic phases were combined, backwashed with saturated sodium chloride solution (3 × 10 mL), dried over anhydrous sodium sulfate, filtered, and the filtrate was concentrated under reduced pressure. The crude product was purified by HPLC with the following conditions (column specifications: Sunfire C18 5 μm, 30 mm × 150 mm; mobile phase A: water (0.1% ammonium bicarbonate), mobile phase B: acetonitrile; flow rate: 60 mL/min; elution gradient: 24% to 50% in 10 min; detection wavelength: UV 254 nm/220 nm; retention time (min): 8.8), to give compound **3-fluoro-N-[3-(7-{[(3S,4R)-3-fluoro-1-methylpiperidin-4-yl] amino}-3-(2,2,2-trifluoroethyl)pyrazolo [1,5-a]pyridin-2-yl)prop-2-yn-1-yl]-1*H*-pyrazole-4-carboxamide 014.**

LCMS: (ESI, m/z): 576.15 [M+H]+.

$^1$H NMR (400 MHz, DMSO-$d_6$) δ 8.64 (t, *J* = 5.6 Hz, 1H), 8.40 (s, 1H), 7.95 (s, 1H), 7.28 (dd, *J* = 8.8, 7.6 Hz, 1H), 7.03 (d, *J* = 8.7 Hz, 1H), 6.32 (d, J = 7.5 Hz, 1H), 6.22 (d, *J* = 9.1 Hz, 1H), 4.87 *(d, J* = 49.4 Hz, 1H), 4.35 (d, *J* = 5.6 Hz, 2H), 3.92 - 3.64 (m, 5H), 3.45 (ddd, *J* = 11.5, 8.0, 3.2 Hz, 2H), 3.06 (t, J = 9.7 Hz, 1H), 2.78 (d, *J* = 11.6 Hz, 1H), 2.37 - 2.22 (m, 3H), 2.20 (s, 3H), 2.14 (td, *J* = 11.5, 2.4 Hz, 1H), 1.88 (m, 4H), 1.44 (s, 3H).

**Example 12**

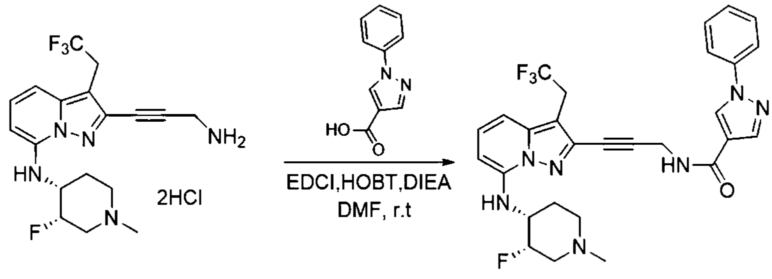


001-12 015

**Step 1: Synthesis of *N*-[3-(7-{[(3S,4R)-3-fluoro-l-methylpiperidin-4-yl]amino}-3-(2,2,2-trifluoroethyl)pyrazolo [1,5-*a*]pyridin-2-yl)prop-2-yn-1-yl]-1-phenyl-1*H*-pyrazole-4-carboxamide (compound 015)**

Under nitrogen protection, 1-phenyl-1*H*-pyrazole-4-carboxylic acid (14.73 mg, 0.078 mmol, 1.5 eq), 1-ethyl-(3-dimethylaminopropyl)carbodiimide hydrochloride (15.00 mg, 0.078 mmol, 1.5 eq), 1-hydroxy-benzotriazole (10.57 mg, 0.078 mmol, 1.5 eq), *N,N*-diisopropylethylamine (33.71 mg, 0.260 mmol, 5 eq) were added to a reaction flask, dissolved in *N,N*-dimethylformamide (2 mL) and stirred at room temperature for 10 min. Then, 2-(3-aminoprop-1-yn-1-yl)-*N*-((3S,4R)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.052 mmol, 1 eq) was added, and the system was stirred to react at room temperature for 1 h. After the reaction was completed, water was added to the reaction mixture and the system was extracted with ethyl acetate (20 mL × 2). The organic phases were combined, backwashed with saturated sodium chloride solution (10 mL × 3), dried over anhydrous sodium sulfate, and filtered, and the filtrate was concentrated under reduced pressure. The crude product was purified by HPLC with the following conditions (column specifications: YMC Triart C18 ExRs 5 μm, 30 mm × 150 mm; mobile phase A: water (10 mmol/L ammonium bicarbonate), mobile phase B: acetonitrile; flow rate: 60 mL/min; elution gradient: 41% B to 67% B in 10 min; detection wavelength: UV 254 nm/220 nm; retention time (min): 8.55) to give compound ***N*-[3-(7-{ [(3 S,4R)-3-fluoro-1-methylpiperidin-4-yl] amino}-3-(2,2,2-trifluoroethyl)pyrazolo [1,5-*a*]pyridin-2-yl)prop-2-yn-1-yl]-1-phenyl-1*H*-pyrazole-4-carboxamide 015** (11.42 mg, 39.47%).

LCMS: (ESI, m/z): 553.90 $[M+H]^+$.

$^1H$ NMR (400 MHz, DMSO-$d_6$) δ 8.98 (s, 1H), 8.85 (t, *J* = 5.5 Hz, 1H), 8.19 (s, 1H), 7.87 (d, J = 7.9 Hz, 2H), 7.54 *(t, J* = 7.9 Hz, 2H), 7.37 *(t, J* = 7.4 Hz, 1H), 7.29 (t, *J* = 8.1 Hz, 1H), 7.03 (d, *J* = 8.7 Hz, 1H), 6.32 *(d, J* = 7.6 Hz, 1H), 6.22 (d, *J* = 9.0 Hz, 1H), 4.88 (d, *J* = 49.4 Hz, 1H), 4.40 (d, *J* = 5.5 Hz, 2H), 3.94 - 3.82 (m, 1H), 3.77 (q, *J* = 11.0 Hz, 2H), 3.06 (t, *J* = 11.7 Hz, 1H), 2.78 (d, *J* = 11.6 Hz, 1H), 2.37 - 2.22 (m, 1H), 2.20 (s, 3H), 2.14 (t, J = 10.5 Hz, 1H), 1.99 - 1.81 (m, 2H).

**Example 13**

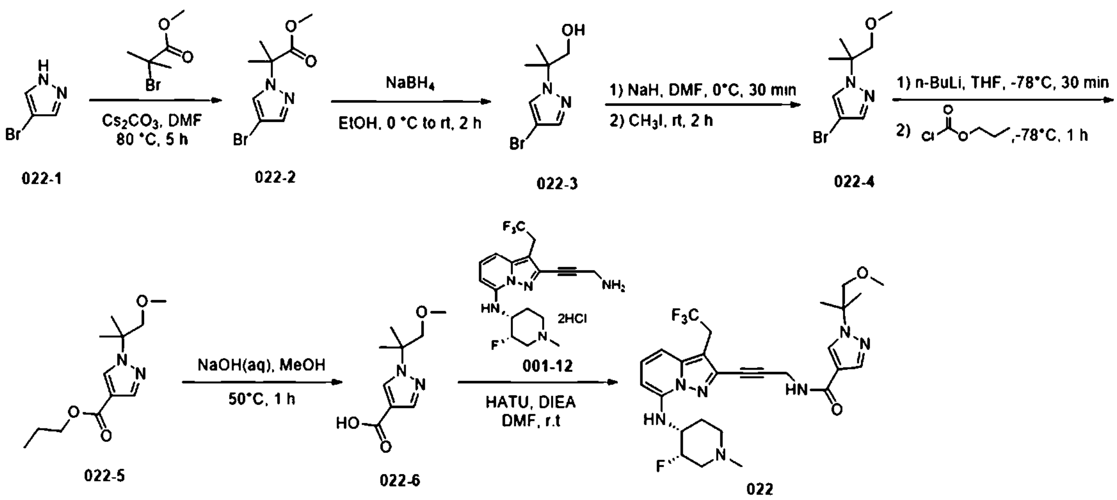


022-1 022-2 022-3 022-4

022-5 022-6 001-12 022

**Step** 1: **Synthesis of compound 022-2**

4-bromo-1*H*-pyrazole (compound **022-1,** 1 g, 6.804 mmol, 1 eq), methyl 2-bromoisobutyrate (2.46 g, 13.608

mmol, 2 eq), cesium carbonate (6.65 g, 20.412 mmol, 3 eq) were added to a reaction flask in sequence, dissolved in *N,N*-dimethylformamide (10 mL), and allowed to react at 80°C for 5 h. After the reaction was completed, the reaction mixture was poured into water and extracted with ethyl acetate (3 × 30 mL). The organic phases were combined, backwashed with saturated sodium chloride solution (1 × 30 mL), dried over anhydrous sodium sulfate, filtered, and the filtrate was concentrated under reduced pressure. The resulting residue was purified by silica gel column chromatography (petroleum ether/ethyl acetate = 5:1) to give compound **022-2** (1.4 g, 79.11%). LCMS: (ESI, m/z): 246.85 $[M+H]^+$.

**Step 2: Synthesis of compound 022-3**

Methyl 2-(4-bromo-1*H*-pyrazole-1-yl)-2-isobutyrate (compound **022-2,** 1.15 g, 4.654 mmol, 1 eq) was added to a reaction flask and dissolved in ethanol (23.0 mL). Sodium borohydride (528.2 mg, 13.962 mmol, 3 eq) was added at 0°C and the system was warmed to room temperature to further react for 2 h. Water was added to quench the reaction. The reaction mixture was extracted with ethyl acetate (3 × 20 mL). The organic phases were combined, backwashed with saturated sodium chloride solution (2 × 10 mL), dried over anhydrous sodium sulfate and filtered. The filtrate was concentrated under reduced pressure to give compound **022-3** (1 g, 93.17%).
LCMS: (ESI, m/z): 218.95 $[M+H]^+$.

**Step** 3: **Synthesis of compound 022-4**

2-(4-bromo-1*H*-pyrazole-1-yl)-2-methylpropan-1-ol (compound **022-3,** 900 mg, 4.108 mmol, 1 eq) was added to a reaction flask and dissolved in *N,N*-dimethylformamide (15 mL). Sodium hydride (821.54 mg, 20.540 mmol, 5 eq, 60%) was added at 0°C and the system was stirred for 30 min. Then, iodomethane (1166.19 mg, 8.216 mmol, 2 eq) was added and the system was warmed to room temperature to further react for 2 h. Saturated ammonium chloride was added to quench the reaction, and the mixture was extracted with ethyl acetate (3 × 30 mL). The organic phases were combined, backwashed with saturated sodium chloride solution (1 × 10 mL), dried over anhydrous sodium sulfate, filtered, and the filtrate was concentrated under reduced pressure. The resulting residue was purified by silica gel column chromatography (petroleum ether/ethyl acetate = 10:1) to give compound **022-4** (900 mg, 89.28%).
LCMS: (ESI, m/z): 232.90 $[M+H]^+$.

**Step 4: Synthesis of compound 022-5**

Under nitrogen protection, 4-bromo-1-(1-methoxy-2-methylpropan-2-yl)-1*H*-pyrazole (compound **022-4,** 700 mg, 3.003 mmol, 1 eq) was added to a reaction flask and dissolved in tetrahydrofuran (25 mL). At -78°C, n-butyl lithium (2.4 mL, 2 eq, 2.5M) was added dropwise and the mixture was stirred for 30 min. Then, propyl chlorocarbonate (331.2 mg, 2.703 mmol, 0.9 eq) was added and the mixture was stirred for another 1 h. After the reaction was completed, the reaction mixture was poured into saturated ammonium chloride solution and extracted with ethyl acetate (3 × 150 mL). The organic phases were combined, backwashed with saturated sodium chloride solution (1 × 150 mL), dried over anhydrous sodium sulfate, filtered, and the filtrate was concentrated under reduced pressure. The resulting residue was purified by silica gel column chromatography (petroleum ether/ethyl acetate = 10:1) to give compound **022-5** (220 mg, 27.44%).
LCMS: (ESI, m/z): 241.30 $[M+H]^+$.

**Step 5: Synthesis of compound 022-6**

To a reaction flask, propyl 1-(1-methoxy-2-methylpropan-2-yl)-1*H*-pyrazole-4-carboxylate (compound **022-5,** 200 mg, 0.832 mmol, 1 eq) was added and dissolved in methanol (2 mL). Then, sodium hydroxide (10M) (1.6 mL, 8.32 mmol, 10 eq) was added and the mixture was allowed to react at 50°C for 1 h. After the reaction was completed, the solvent was directly removed by spin drying, and water was added. The pH was adjusted to 4 using 1M hydrochloric acid. The system was extracted with ethyl acetate (3 × 10 mL). The organic phases were combined, backwashed with saturated sodium chloride solution (1 × 10 mL), dried over anhydrous sodium sulfate and filtered. The filtrate was concentrated under reduced pressure to directly give compound **022-6** (180 mg, 98.20%).
LCMS: (ESI, m/z): 199.10 $[M+H]^+$.

**Step 6: Synthesis of N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-1-(1-methoxy-2-methylpropan-2-yl)-1*H*-pyrazole-4-carboxamide (compound 022)**

1-(1-methoxy-2-methylpropan-2-yl)-1*H*-pyrazole-4-carboxylic acid (compound **022-6,** 30 mg, 0.151 mmol, 1 eq), 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 58.03 mg, 0.151 mmol, 1 eq), O-(7-azabenzotriazol-1-yl)-*N*,*N*,*N'*,*N'*-tetramethyluronium hexafluorophosphate (86.32 mg, 0.226 mmol, 1.5 eq) and *N,N*-diisopropylethylamine (97.8 mg, 0.755 mmol, 5 eq) were added to a reaction flask, dissolved in *N,N*-dimethylformamide (3 mL) and allowed to react at room temperature for 1 h. After the reaction was completed, water was added to the reaction mixture, and the system was extracted with ethyl acetate (20 mL × 2). The organic phases were combined, backwashed with saturated sodium chloride solution (30 mL × 1), dried over anhydrous sodium sulfate, filtered, and the filtrate was concentrated under reduced pressure. The crude product was purified by HPLC with the following conditions (column specifications: YMC Triart C18 ExRs 5 μm, 30 mm × 150 mm; mobile phase A: water (10 mmol/L ammonium bicarbonate), mobile phase B: acetonitrile; flow rate: 60 mL/min; gradient: 36% B to 58% B in 8 min; wavelength: UV 254 nm/220 nm; retention time (min): 8.12), to give compound **N-[3-(7-{[(3S,4R)-3-fluoro-1-methylpiperidin-4-yl]amino}-3-(2,2,**2-**trifluoroethyl)pyrazolo[1,5-a]pyridin-2-yl)prop-2-yn-1-yl]-1-(1-methoxy-2-methylpropan-2-yl)-1*H*-pyrazole-4-carboxamide 022** (12.28 mg, 14.14%).

LCMS: (ESI, m/z): 564.25 $[M+H]^+$.

$^1H$ NMR (400 MHz, DMSO-$d_6$) δ 8.62 (t, J = 5.5 Hz, 1H), 8.27 (s, 1H), 7.90 (s, 1H), 7.28 (t, *J* = 8.2 Hz, 1H), 7.02 (d, J = 8.7 Hz, 1H), 6.31 (d, J = 7.5 Hz, 1H), 6.22 (d, J = 9.1 Hz, , 1H), 4.87 (d, J = 49.3 Hz, 1H), 4.34 (d, J = 5.5 Hz, 2H), 3.84 (m, 1H), 3.75 (q, *J* = 11.2 Hz, 2H), 3.55 (s, 2H), 3.17 (s,3H), 3.05 (t, J = 10.4 Hz, 1H), 2.78 (d, J = 11.1 Hz, 1H), 2.36 - 2.25 (m, 1H), 2.21 (s, 3H), 2.13 (t, J = 11.2 Hz, 1H), 1.99 - 1.80 (m, 2H), 1.50 (s, 6H).

**Example 14**

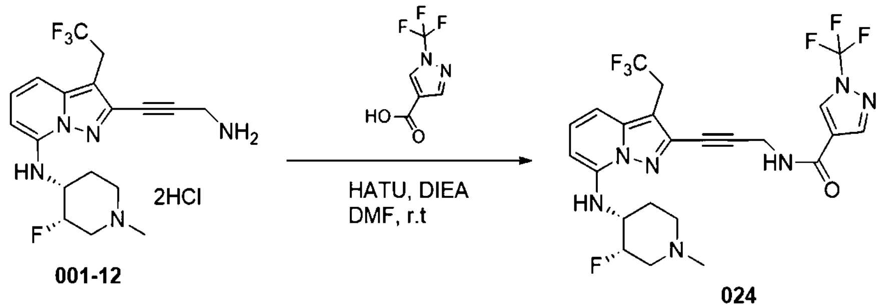


**001-12** **024**

**Step 1: Synthesis of N-[3-(7-{[(3S,4R)-3-fluoro-1-methylpiperidin-4-yl]amino}-3-(2,2,2-trifluoroethyl)pyrazolo [1,5-a]pyridin-2-yl)prop-2-yn-1-yl]-1-(trifluoromethyl)-1*H*-pyrazole-4-carboxamide (compound 024)**

At room temperature, a solution of 1-(trifluoromethyl)-1*H*-pyrazole-4-carboxylic acid (16.91 mg, 0.094 mmol, 1.2 eq), *N,N*-diisopropylethylamine (20.23 mg, 0.156 mmol, 2 eq) and O-(7-azabenzotriazol-1-yl)-*N,N,N',N'*-tetramethyluronium hexafluorophosphate (2-(7-azobenzotriazole)-*N,N,N',N'*-tetramethyluronium hexafluorophosphate) (35.7 mg, 0.094 mmol, 1.2 eq) in N,N-dimethylformamide (1 mL) was stirred to react for 30 min, then 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 30 mg, 0.078 mmol, 1 eq) was added. The system was stirred for 2 h. After the reaction was completed, the reaction mixture was quenched with water and extracted with ethyl acetate (3 × 10 mL). The organic phases were combined, backwashed with saturated sodium chloride solution (3 × 10 mL), dried over anhydrous sodium sulfate, filtered, and the filtrate was concentrated under reduced pressure. The crude product was purified by HPLC with the following conditions (column specifications: Sunfire C18 5 μm, 30 mm × 150 mm; mobile phase A: water (0.1% ammonium bicarbonate), mobile phase B: acetonitrile; flow rate: 60 mL/min; elution gradient: 42% to 66% in 10 min; detection wavelength: UV 254 nm/220 nm; retention time (min): 9.08) to give compound **N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-1-(trifluoromethyl)-1*H*-pyrazole-4-carboxamide 024** (14.57 mg, 33.97%).

LCMS: (ESI, m/z): 546.05 $[M+H]^+$.

$^1H$ NMR (400 MHz, DMSO-$d_6$) δ 9.04 (t, *J* = 5.5 Hz, 1H), 8.97 (s, 1H), 8.33 (3, 1H), 7.28 (dd, *J* = 8.7, 7.6 Hz, 1H), 7.02 (d, J = 8.6 Hz, 1H), 6.32 (d, J = 7.4 Hz, 1H), 6.20 (d, *J* = 9.1 Hz, 1H), 4.87 (d, *J* = 49.4 Hz, 1H), 4.38 (d, *J* = 5.5 Hz, 2H), 3.92-3.82 (m, 1H), 3.75 (q, *J* = 11.1 Hz, 2H), 3.05 (t, *J*= 11.4 Hz, 1H), 2.78 (d, *J* = 11.5 Hz, 1H), 2.29 (dd, J = 38.7, 12.9 Hz, 1H), 2.20 (s, 3H), 2.14 (t, J = 10.3 Hz, 1H), 1.98 - 1.80 (m, 2H).

**Example 15**

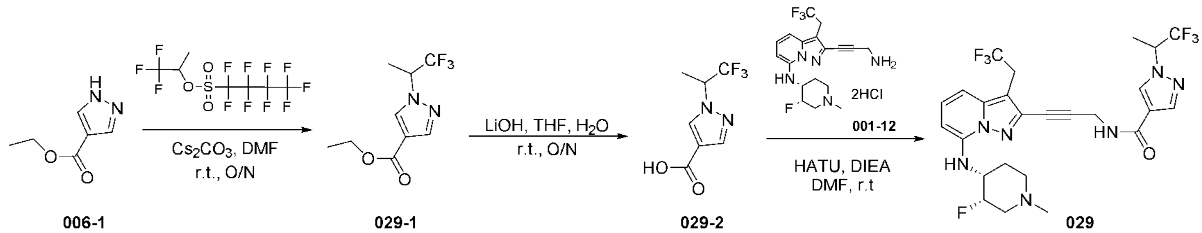


006-1 029-1 029-2 029

**Step 1: Synthesis of compound 029-1**

Under nitrogen protection, at room temperature, ethyl 1*H*-pyrazole-4-carboxylate (compound **006-1,** 100 mg, 0.714 mmol, 1 eq), 2,2,2- trifluoro-1-methyl ethyl 1,1,2,2,3,3,4,4,4-nonafluorobutan-1-sulfonate (565.36 mg, 1.428 mmol, 2 eq), and cesium carbonate (697.48 mg, 2.142 mmol, 3 eq) were added to a reaction flask, dissolved in *N,N*-dimethylformamide (2 mL), and stirred to react overnight. After the reaction was completed, the reaction mixture was poured into water (10 mL) and extracted with ethyl acetate (2 × 10 mL). The organic phases were combined, backwashed with saturated sodium chloride solution (2 × 10 mL), dried over anhydrous sodium sulfate, filtered, and the filtrate was concentrated under reduced pressure to give compound **029-1** (120 mg, 71.20%).

LCMS: (ESI, m/z): 237.00 $[M+H]^+$.

**Step 2: Synthesis of compound 029-2**

At room temperature, ethyl 1-(1,1,1-trifluoropropyl-2-yl)-1*H*-pyrazole-4-carboxylate (compound **029-1,** 100 mg, 0.423 mmol, 1 eq) and lithium hydroxide (15.21 mg, 0.634 mmol, 1.5 eq) were added to a reaction flask, dissolved in tetrahydrofuran (2 mL) and water (1 mL), and stirred to react overnight. The resulting residue was concentrated under reduced pressure to give compound **029-2** (80 mg, 90.78%).

LCMS: (ESI, m/z): 209.35 $[M+H]^+$.

**Step 3: Synthesis of N-[3-(7-{[(3S,4R)-3-fluoro-1-methylpiperidin-4-yl]amino}-3-(2,2,2-trifluoroethyl)pyrazolo [1,5-a]pyridin-2-yl)prop-2-yn-1-yl]-1-(1,1,1-trifluoroprop-2-yl)-1*H*-pyrazole-4-carboxamide (compound 029)**

At room temperature, 1-(1,1,1-trifluoroprop-2-yl)-1*H*-pyrazole-4-carboxylic acid **029-2** (18.25 mg, 0.088 mmol, 2 eq), O-(7-azabenzothiazol-1-yl)-*N,N,N',N'*-tetramethyluronium hexafluorophosphate (16.67 mg, 0.044 mmol, 1 eq), and *N,N*-diisopropylethylamine (22.66 mg, 0.176 mmol, 4 eq) were added to a reaction flask, dissolved in *N,N*-dimethylformamide (1 mL) and stirred to react for 20 min. Then, 2-(3-aminoprop-1-yn-1-yl)-*N*-((3S,4R)-3-fluoro-1-methylpiperidin-4-yl)-3-(2,2,2-trifluoroethyl)pyrazolo[1,5-*a*]yridin-7-amine dihydrochloride (compound **001-12,** 20 mg, 0.044 mmol, 1 eq) was added in portions. The system was further stirred at room temperature for 1 h. After the reaction was completed, the reaction mixture was poured into water (10 mL) and extracted with ethyl acetate (2 × 10 mL). The organic phases were combined, backwashed with saturated sodium chloride solution (2 × 10 mL), dried over anhydrous sodium sulfate, filtered, and the filtrate was concentrated under reduced pressure. The crude product was purified by HPLC with the following conditions (column specifications: YMC Triart C18 ExRs 5 μm, 30 mm × 150 mm; mobile phase A: water (10 mmol/L ammonium bicarbonate), mobile phase B: acetonitrile; flow rate: 60 mL/min; gradient: 37% B to 67% B in 10 min; wavelength: UV 254 nm/220 nm; retention time (min): 8.22), to give compound N-[3-(7-**{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-1-(1,1,1-trifluoroprop-2-yl)-1*H*-pyrazole-4-carboxamide 029** (5.01 mg, 19.89%).

LCMS: (ESI, m/z): 573.85 $[M+H]^+$.

$^1$H NMR (400 MHz, DMSO-$d_6$) $\delta$ 8.81 (t, *J* = 5.6 Hz, 1H), 8.42 (s, 1H), 8.03 (s, 1H), 7.28 (t, J = 8.0 Hz, 1H), 7.03 (d, *J* = 8.7 Hz, 1H), 6.32 (d, *J* = 7.6 Hz, 1H), 6.23 (d, *J* = 9.0 Hz, 1H), 5.48 (h, *J* = 7.2 Hz, 1H), 4.87 (d, *J* = 48.0 Hz, 1H), 4.35 (d, *J* = 5.5 Hz, 2H), 3.92 - 3.81 (m, 1H), 3.75 (q, *J* = 11.1 Hz, 2H), 3.06 (t, *J* = 11.7 Hz, 1H), 2.78 (d, *J* = 11.5 Hz, 1H), 2.37 - 2.22 (m, 1H), 2.20 (s, 3H), 2.14 (t, J = 10.2 Hz, 1H), 1.88 - 1.81 (m, 2H), 1.68 (d, *J* = 7.0 Hz, 3H).

**Example 16**

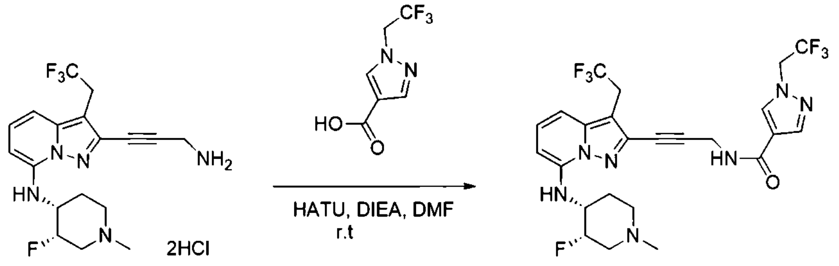


001-12 030

**Step** 1: **Synthesis of N-[3-(7-{[(3S,4R)-3-fluoro-1-methylpiperidin-4-yl]amino}-3-(2,2,2-trifluoroethyl)pyridino [1,5-a]pyridin-2-yl)prop-2-yn-1-yl]-1-(2,2,2-trifluoroethyl)-1H-pyrazole-4-carboxamide (compound 030)**

At room temperature, 1-(2,2,2-trifluoroethyl)-1*H*-pyrazole-4-carboxylic acid (15.31 mg, 0.079 mmol, 1.2 eq), *N,N*-diisopropylethylamine (0.1 mL, 0.660 mmol, 10 eq) and O-(7-azabenzothiazol-1-yl)-*N,N,N',N'*-tetramethyluronium hexafluorophosphate (25.0 mg, 0.066 mmol, 1 eq) were added to a reaction flask, dissolved in *N,N*-dimethylformamide (2 mL) and stirred to react at room temperature for 10 min. Then, 2-(3-aminoprop-1-yn-1-yl)-*N*((3*S*,4*R*)-3-fluoro-1-methylpiperidin-4-yl)-3-(2,2,2-trifluoroethyl)pyrazolo[1,5-*a*]yridin-7-amine dihydrochloride (compound **001-12,** 30 mg, 0.066 mmol, 1 eq) was added. The system was stirred at room temperature for 1 h. After the reaction was completed, the system was diluted with ethyl acetate (5 mL), washed with saturated sodium chloride solution (10 mL × 3), dried over anhydrous sodium sulfate, filtered and concentrated under reduced pressure. The crude product was purified by HPLC with the following conditions (column specifications: XBridge BEH Shield RP18 5 μm, 30 mm × 150 mm; mobile phase A: water (10 mmol/L ammonium bicarbonate), mobile phase B: acetonitrile; flow rate: 60 mL/min; elution gradient: 32% B to 57% B in 8 min; wavelength: UV 254 nm/220 nm; retention time (min): 7.83), to give compound **N-[3-(7-{[(3S,4R)-3-fluoro-1-methylpiperidin-4-yl]amino}-3-(2,2,2-trifluoroethyl)pyridino[1,5-a]pyridin-2-yl)prop-2-yn-1-yl]-1-(2,2,2-trifluoroethyl)-1H-pyrazole-4-carboxamide 330** (5.06 mg, 13.56%).

LCMS: (ESI, m/z): 559.85 $[M+H]^+$.

$^1H$ NMR (400 MHz, Methanol-$d_4$) $\delta$ 8.17 (s, 1H), 7.91 (s, 1H), 7.16 (dd, *J* = 8.8, 7.6 Hz, 1H), 6.85 (d, *J* = 8.7 Hz, 1H), 6.10 (d, J = 7.3 Hz, 1H), 4.92 (q, *J* = 8.7 Hz, 2H), 4.33 (s, 2H), 3.74 (dt, *J* = 27.9, 7.5 Hz, 1H), 3.55 (q, *J* = 10.8 Hz, 2H), 3.13 *(t, J* = 11.9 Hz, 1H), 2.83 (d, *J* = 11.6 Hz, 1H), 2.33 (dd, *J* = 37.4, 13.3 Hz, 1H), 2.20-2.16 (m, 4H), 1.96 - 1.88 (m, 2H).

**Example 17**

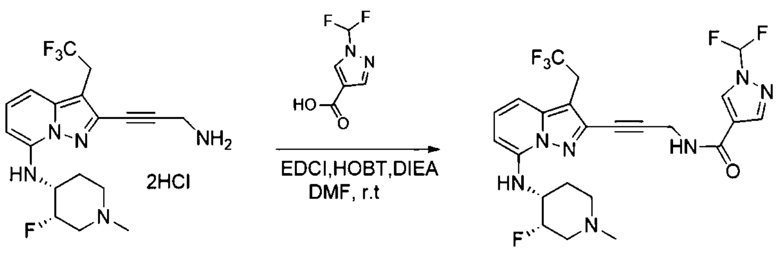


001-12 035

**Step 1: Synthesis of 1-(difluoromethyl)-N-(3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-1*H*-pyrazole-4-carboxamide (compound 035)**

Under nitrogen protection, 1-(difluoromethyl)-1*H*-pyrazole-4-carboxylic acid (15.22 mg, 0.094 mmol, 1.2 eq), 1-ethyl-(3-dimethylaminopropyl) carbodiimide hydrochloride (22.5 mg, 0.117 mmol, 1.5 eq), 1-hydroxy-benzotriazole (15.86 mg, 0.117 mmol, 1.5 eq), and *N, N*-diisopropylethylamine (50.57 mg, 0.390 mmol, 5 eq) were added to a reaction flask, and dissolved in *N,N*-dimethylformamide (2 mL). The mixture was stirred at room temperature for 10 min. Then, 2-(3-aminoprop-1-yn-1-yl)-*N*-((3S,4R)-3-fluoro-1-methylpiperidin-4-yl)-3-(2,2,2-trifluoroethyl)pyrazolo[1,5-*a*]pyridin-7-amine dihydrochloride (compound **001-12,** 30 mg, 0.078 mmol, 1 eq) was added, and the mixture was stirred to react for 1 h. After the reaction was completed, water was added to the reaction mixture, and the system was extracted with ethyl acetate (20 mL × 2). The organic phases were combined, backwashed with saturated sodium chloride solution (10 mL × 3), dried over anhydrous sodium sulfate, filtered, and the filtrate was concentrated under reduced pressure. The crude product was

purified by HPLC with the following conditions (column specifications: XBridge BEH Shield RP18 5 μm, 30 mm *150 mm; mobile phase A: water (10 mmol/L ammonium bicarbonate), mobile phase B: acetonitrile; flow rate: 60 mL/min; elution gradient: 30% B to 57% B in 8 min; detection wavelength: UV 254 nm/220 nm; retention time (min): 7.79) to give compound 1-(difluoromethyl)-N-(3-(7-{[(3S,4R)-3-fluoro-1-**methylpiperidin-4-yl] amino}-3-(2,2,2-trifluoroethyl)pyrazolo [1,5-a] pyridin-2-yl)prop-2-yn-1-yl]-1*H*-pyrazole-4-carboxamide 035** (19.49 mg, 47.08%).

LCMS: (ESI, m/z): 527.85 $[M+H]^+$.

$^1H$ NMR (400 MHz, DMSO-$d_6$) $\delta$ 8.97 (t, *J* = 5.5 Hz, 1H), 8.72 (s, 1H), 8.20 (s, 1H), 7.87 (t, *J* = 58.8 Hz, 1H), 7.29 (dd, *J* = 8.8, 7.6 Hz, 1H), 7.03 (d, *J* = 8.6 Hz, 1H), 6.32 (d, J = 7.4 Hz, 1H), 6.22 *(d, J* = 9.0 Hz, 1H), 4.88 (d, *J* = 49.4 Hz, 1H), 4.37 (d, *J* = 5.5 Hz, 2H), 3.94 - 3.80 (m, 1H), 3.76 (q, *J* = 11.1 Hz, 2H), 3.07 (t, *J* = 11.4 Hz, 1H), 2.79 (d, *J* = 11.4 Hz, 1H), 2.39 - 2.24 (m, 1H), 2.21 (s, 3H), 2.19 - 2.12 (m, 1H), 1.99 - 1.81 (m, 2H).

**Example 18**

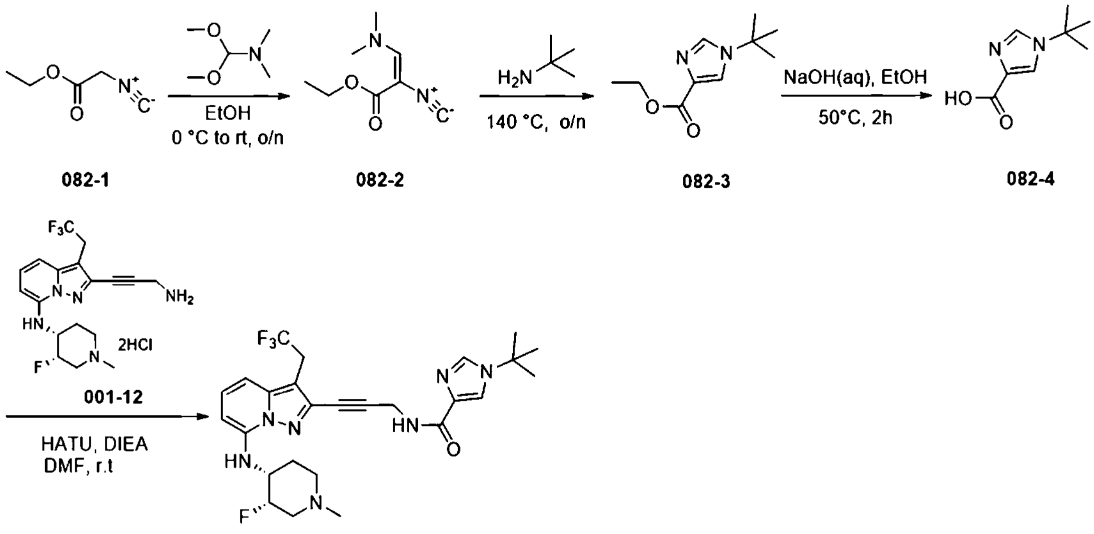


082-1 082-2 082-3 082-4 001-12 082

**Step** 1: **Synthesis of compound 082-2**

Under nitrogen protection, ethyl 2-isocyanoacetate (compound **082-1,** 5 g, 44.202 mmol, 1 eq) was added to a reaction flask, dissolved in anhydrous ethanol (50.0 mL), and (dimethoxymethyl)dimethylamine (10.53 g, 88.404 mmol, 2 eq) was added dropwise at 0°C. The mixture was stirred at room temperature overnight. After the reaction was completed, the solvent was directly removed by spin drying. The resulting residue was purified by silica gel column chromatography (petroleum ether/ethyl acetate = 5:1) to give compound **082-2** (5.6 g, 71.56%)

LCMS: (ESI, m/z): 169.10 $[M+H]^+$.

$^1H$ NMR (400 MHz, DMSO-$d_6$) $\delta$ 7.31 (s, 1H), 4.11 (q, *J* = 7.1 Hz, 2H), 3.19 (s, 6H), 1.20 (t, *J*= 7.1 Hz, 3H).

**Step 2: Synthesis of compound 082-3**

Ethyl (2*E*)-3-(dimethylamino)-2-isocyanoproppy-2-enoate (compound **082-2,** 1 g, 5.945 mmol, 1 eq) and tert-butylamine (2.5 mL, 0.034 mmol, 4 eq) were added to a reaction flask, and stirred at 140°C overnight. After the reaction was completed, the reaction mixture was poured into ice water, and extracted with ethyl acetate (3 × 50 mL). The organic phases were combined, backwashed with saturated sodium chloride solution (3 × 50 mL), dried over anhydrous sodium sulfate, and filtered. The filtrate was concentrated under reduced pressure. The resulting residue was purified by silica gel column chromatography (ethyl acetate) to give compound **082-3** (600 mg, 48.85%).

LCMS: (ESI, m/z): 196.90 $[M+H]^+$.

**Step 3: Synthesis of compound 082-4**

Ethyl 1-tert-Butyl-1*H*-imidazole-4-carboxylate (compound **082-3,** 200 mg, 1.019 mmol, 1 eq), sodium hydroxide aqueous solution (5M, 2 mL, 10 eq) and anhydrous ethanol (2 mL) were added to a reaction flask and allowed to react at

50°C for 2 h. After the reaction was completed, 3M dilute hydrochloric acid was added to adjust the pH to neutral, and the solvent was removed by spin drying to give compound **082-4** (50 mg, 27.71%).

LCMS: (ESI, m/z): 169.05 [M+H]+.

**Step 4: Synthesis of 1-tert-butyl-N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-1*H*-imidazole-4-carboxamide (compound 082)**

1-tert-butyl-1*H*-imidazole-4-carboxylic acid (compound **082-4,** 35 mg, 0.208 mmol, 1 eq), 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 79.78 mg, 0.208 mmol, 1 eq), *O*-(7-azabenzotriazol-1-yl)-*N,N,N',N'*-tetramethyluronium hexafluorophosphate (2-(7-azobenzotriazole)-*N,N,N',N'*-tetramethyluronium hexafluorophosphate) (118.68 mg, 0.312 mmol, 1.5 eq), and *N,N*-diisopropylethylamine (134.48 mg, 1.040 mmol, 5 eq) were added to a reaction flask, dissolved in *N,N*-dimethylformamide (4 mL), and allowed to react at room temperature for 2 h. After the reaction was completed, the reaction mixture was quenched with water and extracted with ethyl acetate (3 × 10 mL). The organic phases were combined, backwashed with saturated sodium chloride solution (3 × 10 mL), dried over anhydrous sodium sulfate, filtered, and the filtrate was concentrated under reduced pressure. The crude product was purified by HPLC with the following conditions (column specifications: Kinetex 5 μm EVO C18, 30mm × 150 mm; mobile phase A: water (10 mmol/L ammonium bicarbonate), mobile phase B: acetonitrile; flow rate: 60 mL/min; elution gradient: 27% B to 58% B in 8 min; detection wavelength: UV 254 nm/220 nm; retention time (min): 7.02), to give compound **1-tert-butyl-N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-1H-imidazole-4-carboxamide 082** (19.97 mg, 17.95%).

LCMS: (ESI, m/z): 533.95 [M+H]+.

$^1$H NMR (400 MHz, DMSO-*d*$_6$) δ 8.45 (t, J = 5.9 Hz, 1H), 7.90 - 7.85 (m, 2H), 7.27 (t, J = 8.2 Hz, 1H), 7.01 (d, J = 8.7 Hz, 1H), 6.30 (d, J = 7.5 Hz, 1H), 6.25 (d, J = 9.1 Hz, 1H), 4.86 (d, J = 50.1 Hz, 1H), 4.30 (d, J = 6.0 Hz, 2H), 3.83 (m, 1H), 3.75 (dd, J = 22.0, 11.0 Hz, 2H), 3.03 (t, J = 9.9 Hz, 1H), 2.77 (d, J = 11.2 Hz, 1H), 2.36 - 2.23 (m, 1H), 2.20 (s, 3H), 2.17 - 2.09 (m, 1H), 2.00 - 1.80 (m, 2H), 1.53 (s, 9H).

Example 19

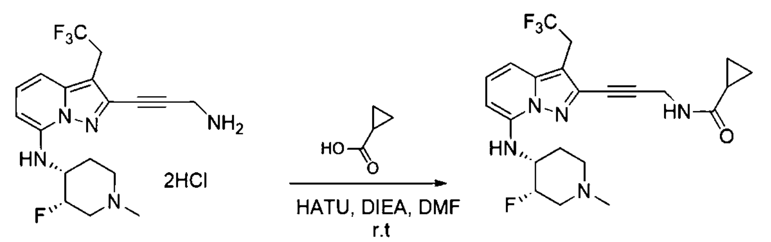


001-12 135

**Step 1: Synthesis of N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]cycloproplycarboxamide (compound 135)**

At room temperature, cyclopropyl carboxylic acid (4.53 mg, 0.053 mmol, 1.2 eq), *N,N*-diisopropylethylamine (28.32 mg, 0.220 mmol, 5 eq) and O-(7-azabenzotriazol-1-yl)-*N,N,N',N'*-tetramethyluronium hexafluorophosphate (20.0 mg, 0.053 mmol, 1.2 eq) were added to a reaction flask, dissolved in *N,N*-dimethylformamide (1 mL), and stirred to react for 30 min. Then, 2-(3-aminoprop-1-yn-1-yl)-*N* ((3S,4R)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.044 mmol, 1 eq) was added and the system was stirred to react for 2 h. After the reaction was completed, the reaction mixture was quenched with water and extracted with ethyl acetate (3 × 10 mL). The organic phases were combined, backwashed with saturated sodium chloride solution (3 × 10 mL), dried over anhydrous sodium sulfate, filtered, and the filtrate was concentrated under reduced pressure. The crude product was purified by HPLC with the following conditions (column specifications: Sunfire C18 5 μm, 30 mm × 150 mm; mobile phase A: water (0.1% ammonium bicarbonate), mobile phase B: acetonitrile; flow rate: 60 mL/min; elution gradient: 27% to 57% in 10 min; detection wavelength: UV 254 nm/220 nm; retention time (min): 7.93), to give compound **N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]cycloproplycarboxamide 135** (4.46 mg, 22.43%).

LCMS: (ESI, m/z): 452.05 [M+H]+.

$^1$H NMR (400 MHz, DMSO-*d6*) δ 8.64 (t, *J* = 5.5 Hz, 1H), 7.29 (dd, *J* = 8.7, 7.6 Hz, 1H), 7.03 (d, *J* = 8.7 Hz, 1H), 6.32 (d, *J* = 7.6 Hz, 1H), 6.22 (d, *J* = 9.0 Hz, 1H), 4.88 (d, *J* = 49.5 Hz, 1H), 4.20 (d, *J* = 5.5 Hz, 2H), 3.93 - 3.66 (m, 3H), 3.18 - 2.99 (m, 1H), 2.78 (d, *J* = 11.2 Hz, 1H), 2.36 (s, 1H), 2.21 (s, 3H), 2.17 - 2.10 (m, 1H), 1.99 - 1.81 (m, 2H), 1.60 (tt, *J* = 7.6, 4.9 Hz, 1H), 0.70 (tt, *J* = 7.9, 2.9 Hz, 4H).

**Example 20**

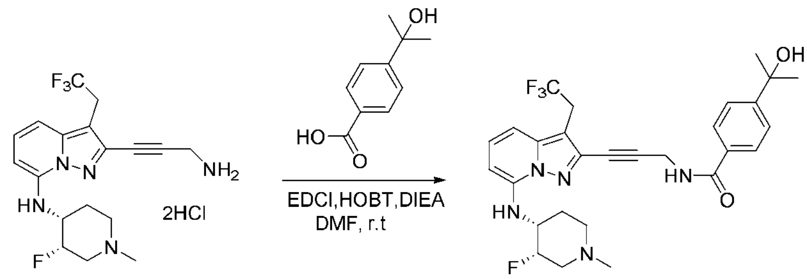


**001-12** **230**

**Step 1: Synthesis of N-[3-(7-{[(3S,4R)-3-fluoro-1-methylpiperidin-4-yl]amino}-3-(2,2,2-trifluoroethyl)pyrazolo [1,5-a]pyridin-2-yl)prop-2-yn-1-yl]-4-(2-hydroxyprop-2-yl)benzamide (compound 230)**

Under nitrogen protection, 4-(2-hydroxyprop-2-yl)benzoic acid (11.85 mg, 0.066 mmol, 1.5 eq), 1-ethyl-(3-dimethylaminopropyl)carbodiimide hydrochloride (12.6 mg, 0.066 mmol, 1.5 eq), 1-hydroxy-benzotriazole (8.88 mg, 0.066 mmol, 1.5 eq), and *N, N*-diisopropylethylamine (28.32 mg, 0.220 mmol, 5 eq) were added to a reaction flask, dissolved in *N, N*-dimethylformamide (2 mL) and stirred at room temperature for 10 min. Then, 2-(3-aminoprop-1-yn-1-yl)-*N*-((3S,4R)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.044 mmol, 1 eq) was added, and the system was stirred to react at room temperature for 1 h. After the reaction was completed, the reaction mixture was quenched with water and extracted with ethyl acetate (20 mL × 2). The organic phases were combined, backwashed with saturated sodium chloride solution (10 mL × 3), dried over anhydrous sodium sulfate, and filtered, and the filtrate was concentrated under reduced pressure. The crude product was purified by HPLC with the following conditions (column specifications: Xselect CSHTM Prep C18 5 μm 30*150mm OBD; mobile phase A: water (0.1% formic acid), mobile phase B: acetonitrile; flow rate: 60 mL/min; elution gradient: 10% B to 35% B in 10 min; detection wavelength: UV 254 nm/220 nm; retention time (min): 7.78) to give the carboxylate of compound **N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-4-(2-hydroxyprop-2-yl)benzamide 230** (15.33 mg, 63.40%)。

LCMS: (ESI, m/z): 546.20 [M+H]$^+$.

$^1$H NMR (400 MHz, DMSO-*d*$_6$) δ 8.99 (t, *J* = 5.5 Hz, 1H), 7.83 (d, *J* = 8.1 Hz, 2H), 7.56 (d, *J* = 8.1 Hz, 2H), 7.28 *(t, J* = 8.2 Hz, 1H), 7.02 (d, *J* = 8.7 Hz, 1H), 6.31 (d, *J* = 7.6 Hz, 1H), 6.24 (d, *J* = 9.0 Hz, 1H), 5.10 (s, 1H), 4.88 (d, *J* = 49.5 Hz, 1H), 4.38 (d, *J* = 5.5 Hz, 2H), 3.94 - 3.82 (m, 1H), 3.76 (q, *J* = 11.3 Hz, 2H), 3.08 (t, *J* = 11.6 Hz, 1H), 2.80 (d, *J* = 11.5 Hz, 1H), 2.41 - 2.25 (m, 1H), 2.22 (s, 3H), 2.16 (m, 1H), 1.99 - 1.83 (m, 2H), 1.44 (s, 6H).

**Example 21**

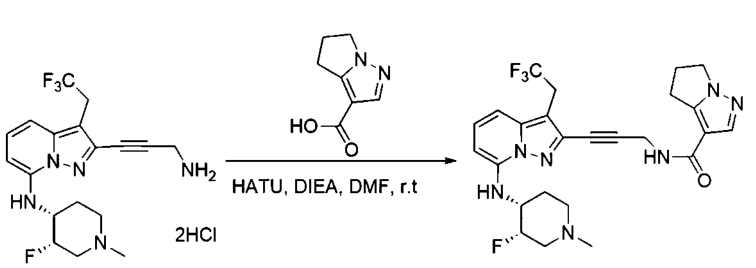


**001-12** **231**

**Step 1: Synthesis of N-[3-(7-{[(3S,4R)-3-fluoro-1-methylpiperidin-4-yl]amino}-3-(2,2,2-trifluoroethyl)pyrazolo [1,5-a]pyridin-2-yl)prop-2-yn-1-yl]-5,6-dihydro-4*H*-pyrrolo[1,2-b]pyrazole-3-carboxamide (compound 231)**

Under nitrogen protection at room temperature, to a solution of 2-(3-aminoprop-1-yn-1-yl)-N-((3*S*,4*R*)-3-fluoro-1-methylpiperidin-4-yl)-3-(2,2,2-trifluoroethyl)pyrazolo[1,5-*a*]pyridin-7-amine dihydrochloride **(001-12,** 40 mg, 0.088 mmol, 1 eq) and 5,6-dihydro-4*H*-pyrrolo[1,2-*b*]pyrazole-3-carboxamide (23.66 mg, 0.156 mmol, 1.5 eq) in N,N-dimethylformamide (0.8 mL) were added **N,N**-diisopropylethylamine (56.65 mg, 0.440 mmol, 5 eq) and O-(7-azabenzotriazol-1-yl)-**N,N,N',N'**-tetramethyluronium hexafluorophosphate (2-(7-azobenzotriazole)-*N,N,N',N'*-tetramethyluronium hexafluorophosphate) (50.00 mg, 0.132 mmol, 1.5 eq), and the mixture was further stirred for 1 h. After the reaction was completed, methanol (1.0 mL) was added to the reaction mixture for quenching. The crude product was purified by HPLC with the following conditions (column specifications: YMC Triart C18 ExRs 5 μm, 30 mm × 150 mm; mobile phase A: water (10 mmol/L ammonium bicarbonate), mobile phase B: acetonitrile; flow rate: 60 mL/min; elution gradient: 30% B to 53% B in 10 min; detection wavelength: UV 254 nm/220 nm; retention time (min): 8.67), to give compound **N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-5,6-dihydro-4*H*-pyrrolo[1,2-b]pyrazole-3-carboxamide 231** (4.78 mg, 8.49%).

LCMS: (ESI, m/z): 517.85 $[M+H]^+$.

$^1H$ NMR (400 MHz, DMSO-$d_6$) δ 8.45 (t, *J* = 5.6 Hz, 1H), 7.91 (s, 1H), 7.28 (dd, *J* = 8.8, 7.6 Hz, 1H), 7.02 (d, *J* = 8.6 Hz, 1H), 6.32 (d, J = 7.5 Hz, 1H), 6.23 *(d, J* = 9.0 Hz, 1H), 4.87 (d, *J* = 49.5 Hz, 1H), 4.31 (d, *J* = 5.6 Hz, 2H), 4.07 (t, *J* = 7.3 Hz, 2H), 3.92 - 3.81 (m, 1H), 3.75 (q, *J* = 11.2 Hz, 2H), 3.07 (t, *J* = 12.0 Hz, 1H), 2.99 (t, *J* = 7.4 Hz, 2H), 2.78 (d, *J* = 11.4 Hz, 1H), 2.57 (t, *J* = 7.3 Hz, 2H), 2.37 - 2.23 (m, 1H), 2.20 (s, 3H), 2.14 (t, *J* = 11.2 Hz, 1H), 1.99 - 1.80 (m, 2H).

Example 22

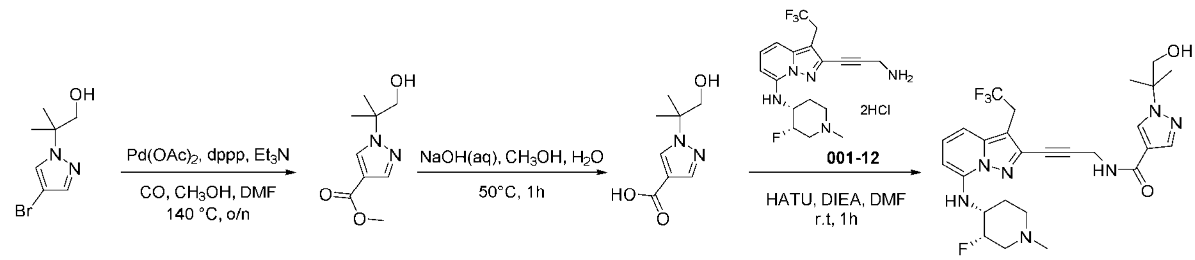


022-3 232-1 232-2 232

**Step** 1: **Synthesis of compound 232-1**

2-(4-bromo-1*H*-pyrazole-1-yl)-2-methylpropan-1-ol (compound **022-3,** 1.1 g, 5.021 mmol, 1 eq), palladium acetate (112.7 mg, 0.502 mmol, 0.1 eq), 1,3-bis(diphenylphosphino)propane (207.1 mg, 0.502 mmol, 0.1 eq), and triethylamine (5.5 mL) were added to a reaction flask, and then *N,N*-dimethylformamide (13.2 mL) and anhydrous methanol (13.2 mL) were added. Carbon monoxide was introduced to 0.4 MPa and the system was allowed to react at 140°C overnight. After the reaction was completed, the mixture was filtered, water was added, and the mixture was extracted with ethyl acetate (3 × 50 mL). The organic phases were combined and backwashed with saturated sodium chloride solution (3 × 30 mL), dried over anhydrous sodium sulfate, filtered, and the filtrate was concentrated under reduced pressure. The resulting residue was purified by reverse phase column chromatography with the following conditions (80gC18 reverse phase column, mobile phase: water (0.1% ammonium bicarbonate) and acetonitrile, 5% to 30% gradient in 30 min, flow rate: 60 mL/min, detection wavelength: UV 254 nm) to give compound **232-1** (300 mg, 28.64%).

LCMS: (ESI, m/z): 199.05 $[M+H]^+$.

**Step 2: Synthesis of compound 232-2**

Under nitrogen protection, at room temperature, to a solution of methyl 1-(1-hydroxy-2-methylpropyl)-1*H*-pyrazole-4-carboxylate (compound **232-1,** 100 mg, 0.504 mmol, 1 eq) in methanol (1.0 mL) and water (1.0 mL) was added sodium hydroxide (40.36 mg, 1.008 mmol, 2 eq), and the mixture was stirred at 50°C for 1 h. After the reaction was completed, the reaction mixture was acidified to pH=6 with dilute hydrochloric acid solution, and then extracted with ethyl acetate and dichloromethane (3:1) (5 × 20 mL). The organic phases were combined, backwashed with saturated brine (2

× 20 mL), dried over anhydrous sodium sulfate, filtered, and the filtrate was concentrated under reduced pressure to give compound **232-2** (50 mg, 53.81%).

LCMS: (ESI, m/z): 184.95 [M+H]+.

**Step 3: Synthesis of N-[3-(7-{[(3S,4R)-3-fluoro-1-methylpiperidin-4-yl]amino}-3-(2,2,2-trifluoroethyl)pyrazolo [1,5-a]pyridin-2-yl)prop-2-yn-1-yl]-1-(1-hydroxy-2-methylprop-2-yl)-1*H*-pyrazole-4-carboxamide (compound 232)**

Under nitrogen protection at room temperature, to a solution of 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.044 mmol, 1 eq) in *N,N*-dimethylformamide (1.0 mL) were added 1-(1-hydroxy-2-methylpropyl)-1*H*-pyrazole-4-carboxylic acid (compound **232-2,** 12.11 mg, 0.066 mmol, 1.5 eq) and *N,N*-diisopropylethylamine (28.32 mg, 0.220 mmol, 5 eq). After the mixture was stirred to react for 30 min, at room temperature, O-(7-azabenzotriazol-1-yl)-*N,N,N',N'*-tetramethyluronium hexafluorophosphate (16.67 mg, 0.044 mmol, 1 eq) was added and the mixture was further stirred for 1 h. After the reaction was completed, the mixture was quenched at room temperature with methanol (1.0 mL). The crude product was purified by HPLC with the following conditions (column specifications: Sunfire C18 5 μm, 30 mm × 150 mm; mobile phase A: water (0.1% formic acid), mobile phase B: acetonitrile; flow rate: 60 mL/min; elution gradient: 5-5% B to 65% B in 10 min; detection wavelength: UV 254 nm/220 nm; retention time (min): 6.8), to give the carboxylate of compound **N-[3-(7-{[(3S,4R)-3-fluoro-1-methylpiperidin-4-yl] amino}-3-(2,2,2-trifluoroethyl)pyrazolo [1,5-a] pyridin-2-yl)prop-2-yn-1-yl]-1-(1-hydroxy-2-methylprop-2-yl)-1*H*-pyrazole-4-carboxamide 232** (6.43 mg, 26.37%).

LCMS: (ESI, m/z): 550.25 [M+H]+.

$^1$H NMR (400 MHz, DMSO-$d_6$) $\delta$ 8.62 (t, *J* = 5.6 Hz, 1H), 8.28 (s, 1H), 8.14 (s, 1H), 7.89 (s, 1H), 7.28 (dd, *J* = 8.8, 7.6 Hz, 1H), 7.03 (d, *J* = 8.6 Hz, 1H), 6.32 (d, *J* = 7.6 Hz, 1H), 6.24 (d, *J* = 9.0 Hz, 1H), 5.00 (t, *J* = 5.6 Hz, 1H), 4.88 (d, *J* = 49.4 Hz, 1H), 4.33 (d, *J* = 5.6 Hz, 2H), 3.95 - 3.81 (m, 1H), 3.75 (q, J = 11.0 Hz, 1H), 3.56 (d, *J* = 5.5 Hz, 2H), 3.07 (t, *J* = 11.6 Hz, 1H), 2.79 (d, *J* = 11.4 Hz, 1H), 2.39 - 2.24 (m, 1H), 2.21 (s, 3H), 2.14 (t, *J* = 11.4 Hz, 1H), 1.98 - 1.82 (m, 2H), 1.47 (s, 6H).

**Example 23**

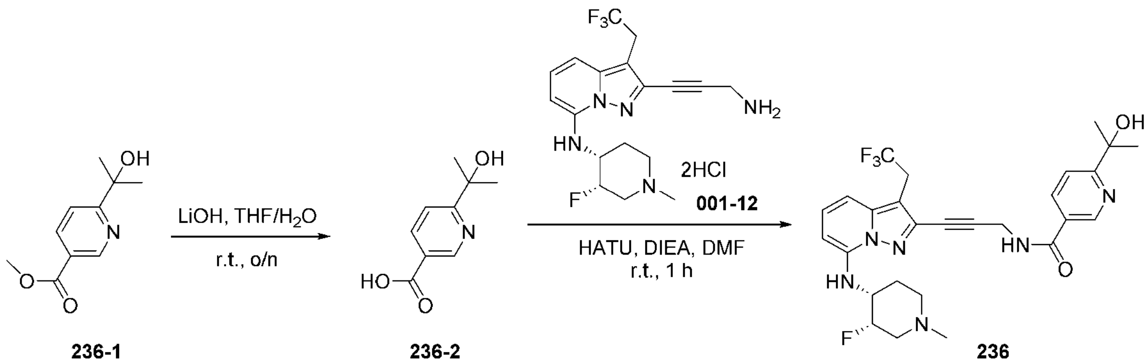


236-1 236-2 236

**Step** 1: **Synthesis** of **compound** 236-2

At room temperature, methyl 6-(2-hydroxypropyl-2-yl)pyridine-3-carboxylate (compound **236-1,** 20 mg, 0.102 mmol, 1 eq) and lithium hydroxide (4.91 mg, 0.204 mmol, 2 eq) were added to a reaction flask, dissolved in tetrahydrofuran (0.5 mL) and water (0.5 mL), and stirred at room temperature overnight. After the reaction was completed, the mixture was concentrated under reduced pressure to give crude compound **236-2.** The resulting mixture was not further purified and directly used in the next step.

LCMS: (ESI, m/z): 182.00 [M+H]+.

**Step 2: Synthesis of N-[3-(7-{[(3S,4R)-3-fluoro-1-methylpiperidin-4-yl]amino}-3-(2,2,2-trifluoroethyl)pyrazolo [1,5-a]pyridin-2-yl)prop-2-yn-1-yl]-6-(2-hydroxyprop-2-yl)pyridine-3-carboxamide (compound 236)**

At room temperature, 6-(2-hydroxypropyl-2-yl)pyridine-3-carboxylic acid (compound **236-2,** 18 mg, 0.099 mmol, 1.2 eq), *N,N*-diisopropylethylamine (0.14 mL, 0.825 mmol, 10 eq), and O-(7-azabenzotriazol-1-yl)-*N,N,N',N'*-tetramethyluronium hexafluorophosphate (37.78 mg, 0.083 mmol, 1 eq) were added to a reaction flask, dissolved in *N,N*-dimethylformamide (2 mL) and stirred at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 37.78 mg, 0.083 mmol, 1 eq) was added and the mixture was stirred at room temperature for 1 h. After the reaction was completed, the reaction mixture was diluted with ethyl acetate (5 mL), washed with saturated sodium chloride solution (20 mL × 3), dried over anhydrous sodium sulfate, filtered, and the filtrate was concentrated under reduced pressure. The crude product was purified by HPLC with the following conditions (column specifications: Xselect CSHTM Prep C18 5μm 30 × 150mm OBD; mobile phase A: water (0.1% formic acid), mobile phase B: acetonitrile; flow rate: 60 mL/min; elution gradient: 5% B to 32% B in 10 min; detection wavelength: UV 254 nm/220 nm; retention time (min): 8.05), to give the carboxylate of compound **N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-6-(2-hydroxyprop-2-yl)pyridine-3-carboxamide 236** (4.77 mg, 10.32%).

LCMS: (ESI, m/z): 546.90 $[M+H]^+$.

$^1H$ NMR (400 MHz, DMSO-$d_6$) $\delta$ 9.24 (t, *J* = 5.5 Hz, 1H), 8.94 (dd, *J* = 2.3, 0.9 Hz, 1H), 8.21 (dd, *J* = 8.3, 2.3 Hz, 1H), 7.76 (dd, *J* = 8.3, 0.9 Hz, 1H), 7.29 (dd, *J* = 8.8, 7.6 Hz, 1H), 7.03 (d, *J* = 8.7 Hz, 1H), 6.41 - 6.29 (m, 1H), 6.23 (d, *J* = 9.1 Hz, 1H), 5.34 (s, 1H), 4.88 (d, *J* = 49.3 Hz, 1H), 4.42 (d, *J* = 5.5 Hz, 2H), 3.77 (q, *J* = 11.0 Hz, 3H), 3.06 (t, *J* = 11.6 Hz, 1H), 2.79 (d, *J* = 11.5 Hz, 1H), 2.36 (s, 1H), 2.21 (s, 4H), 2.01 - 1.89 (m, 1H), 1.89 - 1.77 (m, 1H), 1.45 (s, 6H).

**Example 24**

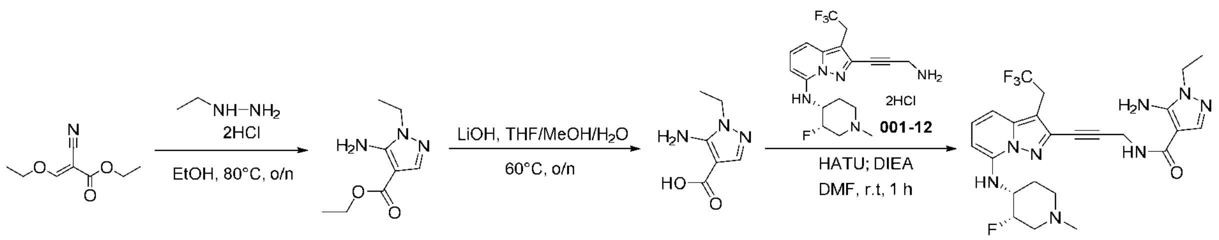


261-1 261-2 261-3 261

**Step 1: Synthesis of compound 261-2**

A solution of ethyl (E)-2-cyano-3-ethoxyacrylate (compound **261-1,** 25.44 g, 150.353 mmol, 1.00 eq), ethylhydrazine dihydrochloride (20 g, 150.353 mmol, 1 eq) and sodium acetate (12.33 g, 150.304 mmol, 1.00 eq) in anhydrous ethanol (200 mL) was stirred at 80°C overnight. After the reaction was completed, the reaction mixture was spin-dried, and ethyl acetate and water were added. The organic phase was separated, dried over anhydrous sodium sulfate, filtered, and the filtrate was concentrated under reduced pressure. The resulting residue was purified by silica gel column chromatography (petroleum ether/ethyl acetate = 1:1) to give **261-2** (8 g, 29.04%).

LCMS: (ESI, m/z): 183.95 $[M+H]^+$.

**Step 2: Synthesis of compound 261-3**

At 60°C, ethyl 5-amino-1-ethyl-1*H*-pyrazole-4-carboxylate (compound **261-2,** 8 g, 43.666 mmol, 1 eq) and lithium hydroxide (5.23 g, 218.330 mmol, 5 eq) were added to a reaction flask, then dissolved in methanol (24 mL), tetrahydrofuran (24 mL) and water (24 mL) and stirred to react overnight. After the reaction was completed, the organic phase was removed by spinning, the system was acidified to pH=5 with hydrochloric acid solution (2M), filtered, and the filter cake was collected and washed with water (20 mL × 2) to give compound **261-3** (3.2 g, 47.23%).

LCMS: (ESI, m/z): 155.85 $[M+H]^+$.

**Step 3: Synthesis of 5-amino-1-ethyl-N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-1*H*-pyrazole-4-carboxamide (compound 261)**

At room temperature, a solution of 5-amino-1-ethyl-1*H*-pyrazole-4-carboxylic acid (compound **261-3,** 2 g, 12.890 mmol, 1 eq) and 2-(7-azabenzotriazole)-*N,N,N',N'*-tetramethyluronium hexafluorophosphate (4.90 g, 12.890 mmol, 1.00 eq), and *N,N*-diisopropylethylamine (6.66 g, 51.560 mmol, 4 eq) in *N,N*-dimethylformamide (80.00 mL) was stirred to react for 20 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 5.88 g, 12.890 mmol, 1 eq) was added and mixture was further

stirred for 1 h. After the reaction was completed, the reaction mixture was concentrated under reduced pressure, and the crude product was purified by HPLC with the following conditions (column specifications: Ultimate 5 μm AQ-C18 30mm * 150 mm; mobile phase A: water (0.1% formic acid), mobile phase B: methanol; flow rate: 100 mL/min; elution gradient: 15% B to 30% B in 20 min; detection wavelength: 254 nm/220 nm; retention time (min): 18.5), to give the carboxylate of **5-amino-1-ethyl-N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-1*H*-pyrazole-4-carboxamide 261** (748 mg, 10.13%).

LCMS: (ESI, m/z): 521.10 $[M+H]^+$.

$^1$H NMR (400 MHz, DMSO-$d_6$, *ppm)* $\delta$ 8.26 (t, *J*= 5.7 Hz, 1H), 7.68 (s, 1H), 7.28 (dd, *J* = 8.8, 7.6 Hz, 1H), 7.02 (d, *J* = 8.7 Hz, 1H), 6.32 (d, J = 7.4 Hz, 1H), 6.28 (d, J = 8.8 Hz, 1H), 6.22 (s, 2H), 4.87 (d, *J= 49.5* Hz, 1H), 4.29 (d, J = 5.6 Hz, 2H), 3.89 (q, J = 7.1 Hz, 2H), 3.84 - 3.68 (m, 3H), 3.05 (t, J = 11.3 Hz, 1H), 2.78 (d, J = 11.4 Hz, 1H), 2.39 - 2.23 (m, 1H), 2.20 (s, 3H), 2.14 (t, J = 11.4 Hz, 1H), 1.99 - 1.90 (m, 1H), 1.89 - 1.81 (m, 1H), 1.21 (t, J = 7.1 Hz, 3H).

**Example 25**

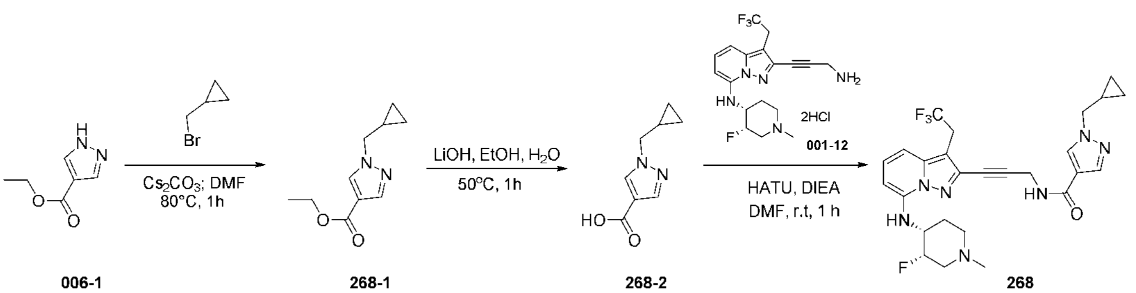


006-1 268-1 268-2 268

**Step 1: Synthesis of compound 268-1**

Under nitrogen protection, ethyl 1*H*-pyrazole-4-carboxylate (compound **006-1,** 500 mg, 3.568 mmol, 1 eq), (bromomethyl)cycpropane (963.34 mg, 7.136 mmol, 2 eq) and cesium carbonate (3487.38 mg, 10.704 mmol, 3 eq) were added to a reaction flask, and dissolved in *N,N*-dimethylformamide (5 mL, 64.608 mmol). The system was warmed to 80°C and stirred to react for 1 h. After the reaction was completed, the reaction mixture was extracted with ethyl acetate (20 mL × 2). The organic phases were combined, backwashed with saturated sodium chloride solution (20 mL × 3), dried over anhydrous sodium sulfate and filtered. The filtrate was concentrated under reduced pressure to give compound **268-1** (400 mg, 53.97%).

LCMS: (ESI, m/z): 195.00 $[M+H]^+$.

**Step 2: Synthesis of compound 268-2**

Under nitrogen protection, ethyl 1-(cyclopropylmethyl)-1*H*-pyrazole-4-carboxylate (compound **268-1,** 200 mg, 1.030 mmol, 1 eq), lithium hydroxide (49.32 mg, 2.060 mmol, 2 eq) were added to a reaction flask, and dissolved in anhydrous ethanol (1 mL) and water (1 mL). The system was warmed to 50°C and stirred to react for 1 h. After the reaction was completed, the reaction mixture was concentrated under reduced pressure. To the crude product was added 10 mL of tetrahydrofuran, the system was stirred for 10 min, and then filtered, and the filtrate was concentrated under reduced pressure to give compound **268-2** (50 mg, 27.76%).

LCMS: (ESI, m/z): 167.05 $[M+H]^+$.

**Step 3: Synthesis of 1-(cyclopropylmethyl)-N-[3-(7-{[(3S,4R)-3-fluoro-1-methylpiperidin-*4-yl]amino}-3-(2,2,2-trifluoroethyl)pyrazolo[1,5-a]pyridin-2-yl)prop-2-yn-1-yl]-1H*-pyrazole-4-carboxamide (compound 268)**

Under nitrogen protection, 1-(cyclopropylmethyl)-1*H*-pyrazole-4-carboxylic acid (compound **268-2,** 10.93 mg, 0.066 mmol, 1.5 eq), 2-(7-azabenzotriazole)-*N,N,N',N'*-tetramethyluronium hexafluorophosphate (16.67 mg, 0.044 mmol, 1 eq), and *N,N*-diisopropylethylamine (28.32 mg, 0.220 mmol, 5 eq) were added to a reaction flask, dissolved in *N,N*-dimethylformamide (1.33 mL, 17.296 mmol), and stirred at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.044 mmol, 1.00 eq) was added and the mixture was stirred to react at room temperature for 1 h. After the reaction was completed, the system was extracted with ethyl acetate (20 mL × 2). The organic phases

were combined, backwashed with saturated sodium chloride solution (10 mL × 3), dried over anhydrous sodium sulfate, filtered, and the filtrate was concentrated under reduced pressure. The crude product was purified by HPLC with the following conditions (column specifications: Kinetex 5 μm EVO C18, 30mm * 150 mm; mobile phase A: water (10 mmol/L ammonium bicarbonate), mobile phase B: acetonitrile; flow rate: 60 mL/min; elution gradient: 31% B to 51% B in 8 min; detection wavelength: UV 254 nm/226 nm; retention time (min): 7.03), to give compound **1-(cyclopropylmethyl)-N-[3-(7-{ [(3 S,4R)-3-fluoro-1-methylpiperidin-4-yl] amino}-3-(2,2,2-trifluoroethyl)pyrazolo [1,5-a]pyridin-2-yl)prop-2-yn-1-yl]-1H-pyrazole-4-carboxamide 268.**

LCMS: (ESI, m/z): 532.20 [M+H]+.

$^1$H NMR (400 MHz, DMSO-$d_6$, *ppm)* δ 8.69 (t, *J* = 5.6 Hz, 1H), 8.27 (s, 1H), 7.89 (s, 1H), 7.29 (dd, *J* = 8.8, 7.6 Hz, 1H), 7.03 (d, J = 8.6 Hz, 1H), 6.32 (d, J = 7.4 Hz, 1H), 6.28 (d, J = 9.0 Hz, 1H), 4.87 (d, *J* = 49.3 Hz, 1H), 4.34 (d, *J* = 5.6 Hz, 2H), 3.99 (d, *J* = 7.1 Hz, 2H), 3.94 - 3.81 (m, 1H), 3.76 (q, *J* = 11.1 Hz, 2H), 3.05 (t, *J* = 11.5 Hz, 1H), 2.78 (d, *J* = 11.1 Hz, 1H), 2.39 (dd, J = 38.7, 13.5 Hz, 1H), 2.20 (s, 3H), 2.16 - 2.10 (m, 1H), 1.97 - 1.82 (m, 2H), 1.28 - 1.19 (m, 1H), 0.57 - 0.52 (m, 2H), 0.40 - 0.33 (m, 2H).

## Example 26

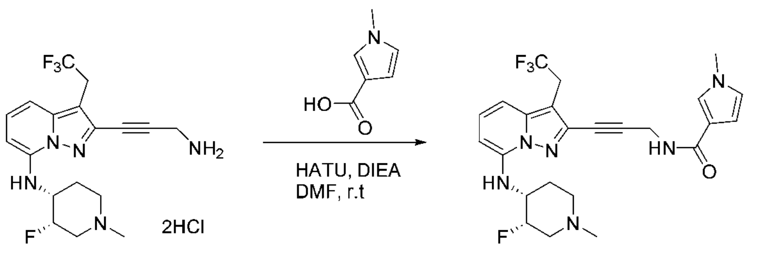


001-12

270

### Step 1: Synthesis of N-[3-(7-{[(3S,4R)-3-fluoro-1-methylpiperidin-4-yl]amino}-3-(2,2,2-trifluoroethyl)pyrazolo [1,5-a]pyridin-2-yl)prop-2-yn-1-yl]-1-methyl-1H-pyrrole-3-carboxamide (compound 270)

Under nitrogen protection, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.044 mmol, 1 eq) and 1-methylpyrrole-3-carboxylic acid (8.23 mg, 0.066 mmol, 1.5 eq) were added to a reaction flask, and dissolved in *N*,*N*-dimethylformamide (0.8 mL). Then, N,N-diisopropylethylamine (28.32 mg, 0.220 mmol, 5 eq) was added dropwise, and the mixture was stirred to react for 2 h. Then, at room temperature, O-(7-azabenzotriazol-1-yl)-N,N,N',N'-tetramethyluronium hexafluorophosphate (16.67 mg, 0.044 mmol, 1 eq) was added and the system was further stirred for 1 h. After the reaction was completed, methanol (1.0 mL) was added to quench the reaction. The solvent was removed by spin drying. The crude product was purified by HPLC with the following conditions (column specifications: Kinetex 5 μm EVO C18 ,30mm × 150 mm; mobile phase A: water (10 mmol/L ammonium bicarbonate), mobile phase B: acetonitrile; flow rate: 60 mL/min; elution gradient: 30% B to 48% B in 8 min; detection wavelength: UV 254 nm/220 nm; retention time (min): 7.12), to give compound **N-[3-(7-{ [(3 S,4R)-3-fluoro-1-methylpiperidin-4-yl] amino}-3-(2,2,2-trifluoroethyl)pyrazolo [1,5-a]pyridin-2-yl)prop-2-yn-1-yl]-1-methyl-1*H*-pyrrole-3-carboxamide 270** (8.18 mg, 38.05%). LCMS: (ESI, m/z): 491.20 [M+H]+.

$^1$H NMR (400 MHz, DMSO-$d_6$, *ppm)* δ 8.31 (t, *J* = 5.7 Hz, 1H), 7.32 - 7.25 (m, 2H), 7.02 (d, *J* = 8.4 Hz, 1H), 6.71 (t, *J* = 2.5 Hz, 1H), 6.47 (dd, *J* = 2.9, 1.8 Hz, 1H), 6.31 (d, *J* = 7.6 Hz, 1H), 6.24 *(d, J* = 9.0 Hz, 1H), 4.87 (d, *J* = 49.5 Hz, 1H), 4.29 (d, *J* = 5.7 Hz, 2H), 3.92 - 3.81 (m, 1H), 3.75 *(q, J* = 11.2 Hz, 2H), 3.63 (s, 3H), 3.05 (t, *J* = 11.4 Hz, 1H), 2.78 (d, *J* = 11.1 Hz, 1H), 2.39 - 2.25 (m, 1H), 2.20 (s, 3H), 2.13 (t, *J* = 11.1 Hz, 1H), 1.99 - 1.80 (m, 2H).

## Example 27

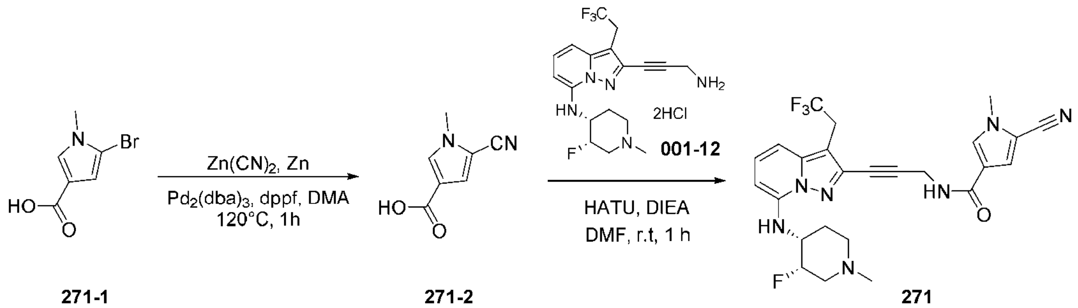


271-1 271-2 271

**Step 1: Synthesis of compound 271-2**

Under nitrogen protection, 5-bromo-1-methyl-1*H*-pyrrole-3-carboxylic acid (compound **271-**1, 100 mg, 0.490 mmol, 1 eq), zinc cyanide (57.55 mg, 0.490 mmol, 1 eq), tris(dibenzylideneacetone)dipalladium (44.88 mg, 0.049 mmol, 0.1 eq), 1,1'-bis(diphenylphosphino)ferrocene (27.07 mg, 0.049 mmol, 0.1 eq), and zinc powder (6.41 mg, 0.098 mmol, 0.2 eq) were added to a reaction flask, and dissolved in *N,N*-dimethylacetamide (2 mL, 21.510 mmol). The system was warmed to 120°C and stirred to react for 1 h. After the reaction was completed, the mixture was filtered and the filter cake was washed with ethyl acetate (10 mL). The resulting filtrate was washed with saturated sodium chloride solution (20 mL × 3), dried over anhydrous sodium sulfate, filtered, and the filtrate was concentrated under reduced pressure. The resulting crude product was purified by silica gel column chromatography (dichloromethane/anhydrous methanol = 12:1) to give compound **271-2** (40 mg, 27.34%).

LCMS: (ESI, m/z): 149.25 $[M+H]^+$.

**Step 2: Synthesis of 5-cyano-N-[3-(7-{[(3S,4R)-3-fluoro-1-methylpiperidin-4-yl]amino}-3-(2,2,2-trifluoroethyl) pyrazolo[1,5-a]pyridin-2-yl)prop-2-yn-1-yl]-1-methyl-1*H*-pyrrole-3-carboxamide (compound 271)**

Under nitrogen protection, 5-cyano-1-methyl-1*H*-pyrrole-3-carboxylic acid (compound **271-2,** 9.87 mg, 0.066 mmol, 1.5 eq), 2-(7-azabenzotriazole)-*N,N,N',N'*-tetramethyluronium hexafluorophosphate (16.67 mg, 0.044 mmol, 1 eq), and *N,N*-diisopropylethylamine (28.32 mg, 0.220 mmol, 5 eq) were added to a reaction flask, dissolved in *N,N*-dimethylformamide (1 mL, 12.922 mmol), and stirred at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.044 mmol, 1.00 eq) was added and the mixture was stirred to react at room temperature for 1 h. After the reaction was completed, the system was extracted with ethyl acetate (20 mL × 2). The organic phases were combined, backwashed with saturated sodium chloride solution (10 mL × 3), dried over anhydrous sodium sulfate, filtered, and the filtrate was concentrated under reduced pressure. The crude product was purified by HPLC with the following conditions (column specifications: Kinetex 5 μm EVO C18, 30mm * 150 mm; mobile phase A: water (10 mmol/L ammonium bicarbonate), mobile phase B: acetonitrile; flow rate: 60 mL/min; elution gradient: 31% B to 51% B in 7 min; detection wavelength: UV 254 nm/220 nm; retention time (min): 7.02), to give compound **5-cyano-N-[3-(7-{[(3S,4R)-3-fluoro-1-methylpiperidin-4-yl]amino}-3-(2,2,2-trifluoroethyl)pyrazolo[1,5-a]pyridin-2-yl) prop-2-yn-1-yl]-1-methyl-1*H*-pyrrole-3-carboxamide 271.**

LCMS: (ESI, m/z): 516.05 $[M+H]^+$.

$^1H$ NMR (400 MHz, DMSO-$d_6$, *ppm)* δ 8.69 (t, *J* = 5.6 Hz, 1H), 7.73 (d, *J* = 1.8 Hz, 1H), 7.36 (d, *J* = 1.8 Hz, 1H), 7.29 (dd, *J* = 8.8, 7.6 Hz, 1H), 7.03 (d, *J* = 8.7 Hz, 1H), 6.32 (d, J = 7.4 Hz, 1H), 6.23 (d, *J* = 9.0 Hz, 1H), 4.87 (d, *J* = 49.4 Hz, 1H), 4.33 (d, *J* = 5.6 Hz, 2H), 3.82 - 3.70 (m, 6H), 3.06 (t, *J* = 11.5 Hz, 1H), 2.78 (d, *J* = 11.3 Hz, 1H), 2.36 - 2.24 (m, 1H), 2.20 (s, 3H), 2.12 (t, J = 10.9 Hz, 1H), 1.97 - 1.82 (m, 2H).

Example 28

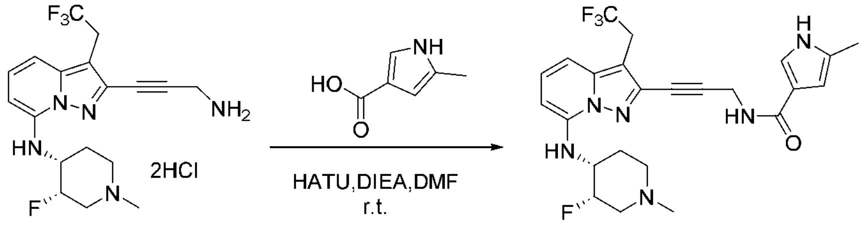


001-12

272

**Step 1: Synthesis of N-[3-(7-{[(3S,4R)-3-fluoro-1-methylpiperidin-4-yl]amino}-3-(2,2,2-trifluoroethyl)pyrazolo [1,5-a]pyridin-2-yl)prop-2-yn-1-yl]-5-methyl-1*H*-pyrrole-3-carboxamide (compound 272)**

At room temperature, 5-methylpyrrole-3-carboxylic acid (9.87 mg, 0.079 mmol, 1.2 eq), *N,N*-diisopropylethylamine (33.99 mg, 0.264 mmol, 4 eq), and O-(7-azabenzotriazol-1-yl)-*N,N,N',N'*-tetramethyluronium hexafluorophosphate (30 mg, 0.066 mmol, 1 eq) were added to a reaction flask, dissolved in N,N-dimethylformamide (2 mL) and stirred at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 30 mg, 0.066 mmol, 1 eq) was added and the mixture was stirred at room temperature for 1 h. After the reaction was completed, the system was diluted with ethyl acetate (5 mL), washed with saturated sodium chloride solution (10 mL × 3), dried over anhydrous sodium sulfate, filtered, and the filtrate was concentrated under reduced pressure. The crude product was purified by HPLC with the following conditions (column specifications: Sunfire C18 5 μm, 30 mm * 150 mm; mobile phase A: water (0.1% formic acid), mobile phase B: acetonitrile; flow rate: 60 mL/min; elution gradient: 6% B to 32% B in 7 min; detection wavelength: UV 254 nm/220 nm; retention time (min): 7.03), to give the carboxylate of compound **N-[3-(7-{[(3S,4R)-3-fluoro-1-methylpiperidin-4-yl] amino}-3-(2,2,2-trifluoroethyl)pyrazolo[1,5-a]pyridin-2-yl)prop-2-yn-1-yl]-5-methyl-1*H*-pyrrole-3-carboxamide 272** (5.4 mg, 16.68%).

LCMS: (ESI, m/z): 491.05 $[M+H]^+$.

$^1H$ NMR (400 MHz, DMSO-$d_6$, *ppm)* $\delta$ 10.93 (s, 1H), 8.19 (t, *J* =5.7 Hz, 1H), 7.28 (dd, *J* = 8.7, 7.6 Hz, 1H), 7.18 (dd, *J* = 2.9, 1.7 Hz, 1H), 7.02 (d, *J* = 8.7 Hz, 1H), 6.31 (d, J = 7.5 Hz, 1H), 6.27 (d, J = 8.9 Hz, 1H), 6.17 (s, 1H), 4.88 (d, *J* = 49.3 Hz, 1H), 4.28 (d, *J* = 5.7 Hz, 2H), 3.96 - 3.81 (m, 1H), 3.75 (q, *J* = 11.4 Hz, 2H), 3.08 (t, *J* = 11.3 Hz, 1H), 2.80 (d, *J* = 11.3 Hz, 1H), 2.43 - 2.27 (m, 1H), 2.22 (s, 3H), 2.16 (s, 4H), 2.01 - 2.79 (m, 2H).

**Example 29**

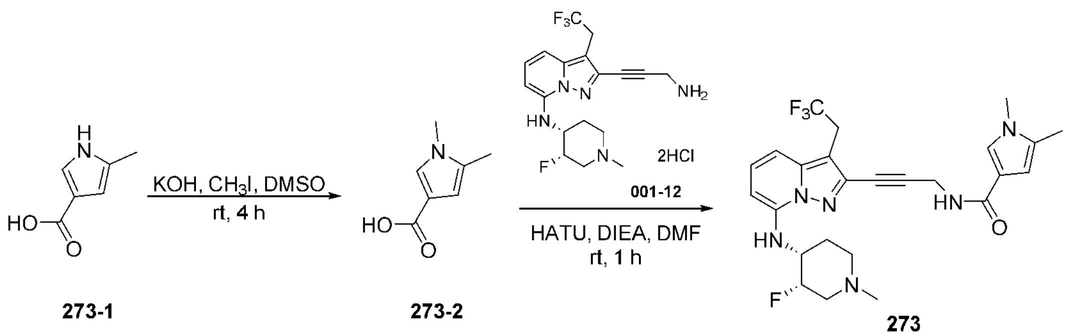


273-1 273-2 273

**Step 1: Synthesis of compound 273-2**

At room temperature, 5-methyl-1*H*-pyrrole-3-carboxylic acid (compound **273-1,** 200 mg, 1.598 mmol, 1 eq) was added to a reaction flask, dissolved in dimethyl sulfoxide (3 mL), and then potassium hydroxide (717.42 mg, 12.784 mmol, 8 eq) was added. After the mixture was stirred to react for 50 min, iodomethane (181.5 mg, 1.278 mmol, 0.8 eq) was added dropwise, and the system was further stirred for 4 h. After the reaction was completed, the reaction mixture was diluted with water (20 mL), extracted with ethyl acetate (3 × 20 mL), and the aqueous phase was retained. The aqueous phase was acidified to pH=1 with 4 mol/L hydrochloric acid, backwashed with ethyl acetate (3 × 20 mL), and the organic phase was dried over anhydrous sodium sulfate, filtered, and the filtrate was concentrated under reduced pressure to give crude compound **273-2** (170 mg, 61.15%). The crude product was directly used in the next step.

LCMS: (ESI, m/z): 140.45 [M+H]+.

**Step 2: Synthesis of N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-1,5-dimethyl-1*H*-pyrrole-3-carboxamide (compound 273)**

At room temperature, 1,5-dimethyl-1*H*-pyrrole-3-carboxylic acid (compound **273-2,** 9.15 mg, 0.066 mmol, 1 eq), O-(7-azabenzotriazol-1-yl)-*N*,*N*,*N'*,*N'*-tetramethyluronium hexafluorophosphate (25.0 mg, 0.066 mmol, 1 eq), and diisopropylethylamine (34 mg, 0.264 mmol, 4 eq) were added to a reaction flask, dissolved in *N,N*-dimethylformamide (1 mL), and stirred to react for 30 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 30 mg, 0.066 mmol, 1 eq) was added and the mixture was allowed to react for 1 h. Then, the reaction mixture was diluted with water (20 mL) and extracted with ethyl acetate (3 × 20 mL). The organic phases were combined, backwashed with saturated brine (3 × 20 mL), dried over anhydrous sodium sulfate, filtered, and the filtrate was concentrated under reduced pressure. The crude product was purified by HPLC with the following conditions (column specifications: XBridge BEH C18 OBD Prep Column 130, 5 μm, 30 mm * 150 mm; mobile phase A: water (17 mmol/L ammonium bicarbonate), mobile phase B: acetonitrile; flow rate: 60 mL/min; elution gradient: 36% B to 54% B in 7 min; detection wavelength: UV 254 nm/220 nm; retention time (min): 5.98), to give compound **N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-1,5-dimethyl-1*H*-pyrrole-3-carboxamide 273** (8.81 mg, 26.21%).
LCMS: (ESI, m/z): 505.10 [M+H]+.

**Example 30**

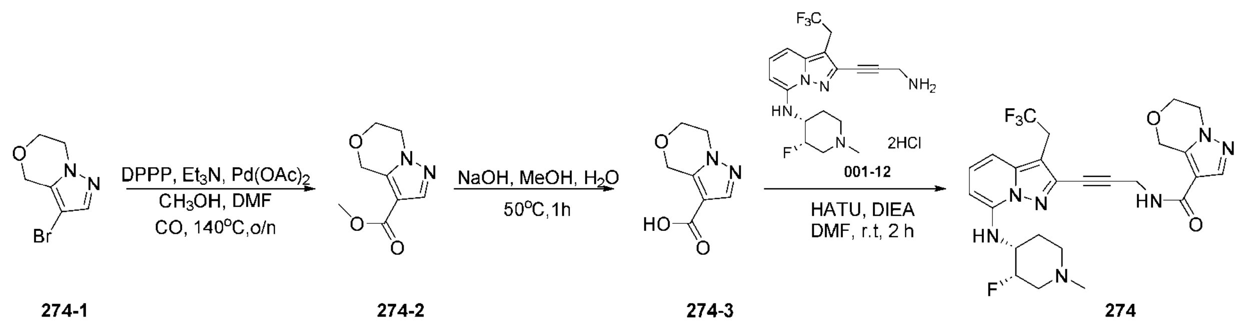


**274-1** **274-2** **274-3** **274**

**Step 1: Synthesis of compound 274-2**

At room temperature, 3-bromo-6,7-dihydro-4*H*-pyrazolo[3,2-*c*][1,4]oxazine (compound **274-1, 200** mg, 0.985 mmol, 1 eq), 1,3-bis(diphenylphosphino)propane (40.63 mg, 0.099 mmol, 0.1 eq), palladium acetate (22.11 mg, 0.099 mmol, 0.1 eq), anhydrous methanol (2.0 mL), *N,N*-dimethylformamide (2.0 mL) and triethylamine (1.0 mL) were added to a 50 mL autoclave. Thereafter, carbon monoxide (3-4 MPa) was introduced to the system. Then the system was further stirred at 140°C overnight, the resulting mixture was concentrated under reduced pressure, and the crude product was purified by reverse phase column chromatography with the following conditions: (40gC18 reverse phase column, mobile phase: water (0.1% trifluoroacetic acid) and acetonitrile, 5% to 30% gradient in 30 min, detection wavelength: UV 254 nm), to give compound **274-2** (110 mg, 61.30%).
LCMS: (ESI, m/z): 183.05 [M+H]+.

**Step 2: Synthesis of compound 274-3**

Under nitrogen protection, to a solution of methyl 6,7-dihydro-4*H*-pyrazolo[3,2-c][1,4]oxazine-3-carboxylate (compound **274-2,** 50 mg, 0.274 mmol, 1 eq) in water (0.5 mL) and methanol (1.0 mL) was added sodium hydroxide (21.95 mg, 0.548 mmol, 2 eq), and the mixture was stirred at 50°C for 1 h. After the reaction was completed, the reaction mixture was acidified to pH=6 with hydrochloric acid solution, and the resulting residue was concentrated under reduced pressure. The reaction mixture was dissolved in tetrahydrofuran (2 mL), filtered, and the filter cake was washed with tetrahydrofuran (3 × 1 mL). The filtrate was concentrated under reduced pressure to give compound **274-3** (40 mg, 86.67%).
LCMS: (ESI, m/z): 169.05 [M+H]+.

**Step 2: Synthesis of N-[3-(7-{[(3S,4R)-3-fluoro-1-methylpiperidin-4-yl]amino}-3-(2,2,2-**trifluoroethyl)pyrazolo[1,5-a]pyridin-2-yl)prop-2-yn-1-yl]-6,7-dihydro-4H-pyrazolo[5,1-**c][1,4]oxazine-3-carboxamide (compound 274)**

Under nitrogen protection, to a solution of 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.044 mmol, 1 eq) and 6,7-dihydro-4H-pyrazolo[3,2-*c*][1,4]oxazine-3-carboxylic acid (compound **274-3,** 11.06 mg, 0.066 mmol, 1.5 eq) in *N,N*-dimethylformamide (0.8 mL) was added *N,N*-diisopropylethylamine (28.32 mg, 0.220 mmol, 5 eq) dropwise, and the mixture was stirred to react for 1 h. Then, O-(7-azabenzotriazol-1-yl)-*N,N,N',N'*-tetramethyluronium hexafluorophosphate (16.67 mg, 0.044 mmol, 1 eq) was added and the system was stirred for another 1 h. After the reaction was completed, methanol (1.0 mL) was added to quench the reaction. The solvent was removed by spin drying. The crude product was purified by HPLC with the following conditions (column specifications: XBridge BEH C18 OBD Prep Column 130, 5 μm, 30 mm × 150 mm; mobile phase A: water (17 mmol/L ammonium bicarbonate), mobile phase B: acetonitrile; flow rate: 60 mL/min; elution gradient: 30% B to 48% B in 8 min; detection wavelength: UV 254 nm/220 nm; retention time (min): 6.98), to give compound N-[3-**(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-6,7-dihydro-4*H*-pyrazolo[5,1-c][1,4]oxazine-3-carboxamide 274.**

LCMS: (ESI, m/z): 534.10 $[M+H]^+$.

$^1H$ NMR (400 MHz, DMSO-$d_6$, *ppm*) δ 8.71 (t, *J* = 5.6 Hz, 1H), 8.00 (s, 1H), 7.29 (t, *J* = 8.2 Hz, 1H), 7.03 (d, *J* = 8.7 Hz, 1H), 6.32 (d, *J* = 7.7 Hz, 1H), 6.22 (d, *J* = 9.1 Hz, 1H), 4.98 (s, 2H), 4.87 (d, *J* = 49.4 Hz, 1H), 4.32 (d, *J* = 5.6 Hz, 2H), 4.13 (t, *J* = 5.0 Hz, 2H), 4.04 (t, J = 5.0 Hz, 2H), 3.92 - 3.82 (m, 1H), 3.75 (q, *J* = 11.2 Hz, 2H), 3.05 (t, *J* = 11.5 Hz, 1H), 2.78 (d, *J* = 11.4 Hz, 1H), 2.36 - 2.21 (m, 1H), , 2.20 (s, 3H), 2.13 (t, J = 10.5 Hz, 1H), 1.99 - 1.79 (m, 1H).

**Example 31**

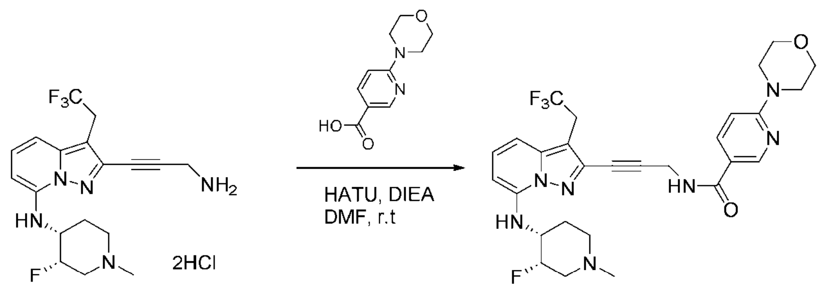


**001-12** **275**

**Step 1: Synthesis of N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-(6-morpholin-4-yl)pyridine-3-carboxamide (compound 275)**

Under nitrogen protection, to a solution of 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.044 mmol, 1 eq) and 6-(morpholin-4-yl)pyridine-3-carboxylic acid (13.69 mg, 0.066 mmol, 1.5 eq) in *N,N*-dimethylformamide (0.8 mL) was added *N,N*-diisopropylethylamine (28.32 mg, 0.220 mmol, 5 eq) dropwise, and the mixture was stirred to react for 1 h. Then, at room temperature, O-(7-azabenzotriazol-1-yl)-*N,N,N',N'*-tetramethyluronium hexafluorophosphate (16.67 mg, 0.044 mmol, 1 eq) was added and the system was further stirred for 1 h. After the reaction was completed, methanol (1.0 mL) was added to quench the reaction. The solvent was removed by spin drying. The crude product was purified by HPLC with the following conditions (column specifications: XBridge BEH Shield RP18 5 μm, 30 mm × 150 mm; mobile phase A: water (10 mmol/L ammonium bicarbonate), mobile phase B: acetonitrile; flow rate: 60 mL/min; elution gradient: 34% B to 54% B in 8 min; detection wavelength: UV 254 nm/220 nm; retention time (min): 7.65), to give compound **N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-(6-morpholin-4-yl)pyridine-3-carboxamide 275** (14.07 mg, 55.63%).

LCMS: (ESI, m/z): 574.10 $[M+H]^+$.

$^1H$ NMR (400 MHz, DMSO-$d_6$, *ppm*) δ 8.88 *(t, J* = 5.5 Hz, 1H), 8.66 (d, *J* = 2.4 Hz, 1H), 8.01 (dd, *J* = 9.0, 2.5 Hz, 1H), 7.28 (dd, *J* = 8.8, 7.6 Hz, 1H), 7.02 (d, *J* = 8.5 Hz, 1H), 6.88 (d, *J* = 9.0 Hz, 1H), 6.32 (d, J = 7.5 Hz, 1H), 6.23 *(d, J* = 9.1 Hz, 1H), 4.87 (d, *J* = 49.5 Hz, 1H), 4.37 (d, *J* = 5.5 Hz, 2H), 3.92 - 3.82 (m, 1H), 3.75 (q, *J* = 11.1 Hz, 2H), 3.69 (t, *J* = 4.9 Hz, 4H), 3.57 (t, *J* = 4.9, 4H), 3.05 *(t, J* = 11.3 Hz, 1H), 2.78 (d, *J* = 11.5 Hz, 1H), 2.36 - 2.23 (m, 1H), 2.20 (s, 3H), 2.13 (t, *J* = 11.0 Hz, 1H), 1.99 - 1.80 (m, 2H).

**Example 32**

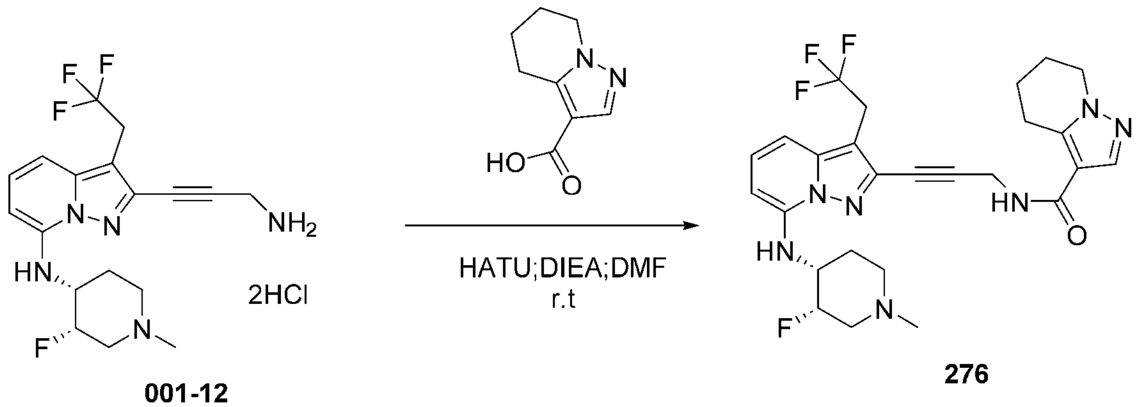


**001-12**

**276**

**Step 1: Synthesis of N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-4,5,6,7-tetrahydropyrazolo[1,5-a]pyridine-3-carboxamide (compound 276)**

At room temperature, 4,5,6,7-tetrahydropyrazolo[1,5-*a*]pyridine-3-carboxylic acid (8.74 mg, 0.053 mmol, 1.2 eq), *N,N*-diisopropylethylamine (0.04 mL, 0.220 mmol, 5 eq), and O-(7-azabenzotriazol-1-yl)-*N*,*N*,*N'*,*N'*-tetramethyluronium hexafluorophosphate (16.67 mg, 0.044 mmol, 1 eq) were added to a reaction flask, dissolved in *N,N*-dimethylformamide (2 mL) and stirred at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.044 mmol, 1 eq) was added and the mixture was stirred for 1 h. After the reaction was completed, the system was diluted with ethyl acetate (5 mL), the reaction mixture was washed with saturated sodium chloride solution (10 mL × 3), dried over anhydrous sodium sulfate, filtered, and the filtrate was concentrated under reduced pressure. The crude product was purified by HPLC with the following conditions (column specifications: XBridge BEH C18 OBD Prep Column 130, 5 μm, 30 mm × 150 mm; mobile phase A: water (17 mmol/L ammonium bicarbonate), mobile phase B: acetonitrile; flow rate: 60 mL/min; elution gradient: 33% B to 53% B in 7 min; detection wavelength: UV 254 nm/220 nm; retention time (min): 6.07), to give compound **N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-4,5,6,7-tetrahydropyrazolo[1,5-a]pyridine-3-carboxamide 276** (5.43 mg, 22.33%).

LCMS: (ESI, m/z): 531.85 $[M+H]^+$.

$^1H$ NMR (400 MHz, DMSO-$d_6$, *ppm)* $\delta$ 8.50 (t, *J* = 5.6 Hz, 1H), 7.91 (s, 1H), 7.29 (t, *J* = 8.2 Hz, 1H), 7.03 (d, *J* = 8.7 Hz, 1H), 6.32 (d, *J* = 7.6 Hz, 1H), 6.23 (d, *J* = 8.9 Hz, 1H), 4.88 (d, *J* = 49.4 Hz, 1H), 4.31 (d, *J* = 5.6 Hz, 2H), 4.06 (t, *J* = 6.0 Hz, 2H), 3.94 - 3.83 (m, 1H), 3.85 (q, J = 11.2 Hz, 2H), 3.06 (t, *J* = 11.2 Hz, 1H), 2.99 (t, *J* = 6.4 Hz, 2H), 2.78 (d, *J* = 10.8 Hz, 1H), 2.38 - 2.23 (m, 1H), 2.20 (s, 3H), 2.09 - 2.08 (m, 1H), 2.00 - 1.71 (m, 6H).

**Example 33**

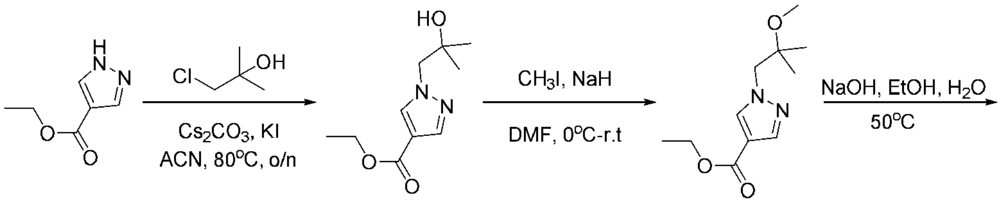


006-1 039-1 039-2

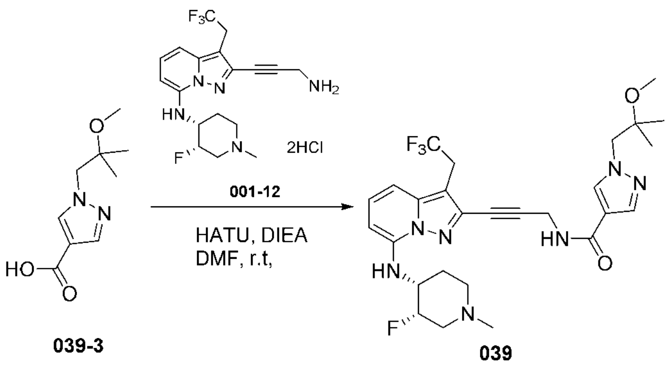


039-3 039

**Step** 1: **Synthesis of compound 039-1**

Under nitrogen protection, to a solution of ethyl 1*H*-pyrazole-4-carboxylate (compound **006-1,** 300 mg, 2.141 mmol, 1 eq) and 1-chloro-2-methyl-2-propanol (348.62 mg, 3.212 mmol, 1.5 eq) in acetonitrile (4.5 mL) were added cesium carbonate (2.09 g, 6.423 mmol, 3 eq) and potassium iodide (177.68 mg, 1.071 mmol, 0.5 eq) in portions, and the mixture was stirred at 80°C overnight. After the reaction was completed, the reaction mixture was concentrated under reduced pressure and filtered. The filter cake was washed with acetonitrile (3 × 10 mL), and the filtrate was concentrated under reduced pressure. The resulting residue was purified by silica gel column chromatography (dichloromethane/anhydrous methanol = 10:1) to give compound **039-1** (450 mg, 84.89%).

LCMS: (ESI, m/z): 213.00 $[M+H]^+$.

**Step 2: Synthesis of compound 039-2**

Under nitrogen protection, at 0°C, to a solution of ethyl 1-(2-hydroxy-2-methylpropyl)-1*H*-pyrazole-4-carboxylate (compound **039-1,** 200 mg, 0.942 mmol, 1 eq) in *N*,*N*-dimethylformamide (4.0 mL) was added sodium hydride (45.23 mg, 1.884 mmol, 2 eq), and the mixture was stirred to react for 30 min. Then, iodomethane (267.5 mg, 1.884 mmol, 2 eq) was added dropwise and the mixture was further stirred at room temperature for 2 h. After the reaction was completed, the reaction mixture was added to ice water (20 mL) at room temperature for quenching, and extracted with ethyl acetate (3 × 20 mL). The organic phases were combined, backwashed with saturated brine (3 × 10 mL), dried over anhydrous sodium sulfate, filtered, and the filtrate was concentrated under reduced pressure. The resulting crude product was purified by silica gel column chromatography (dichloromethane/methanol = 20:1) to give compound **039-2** (130 mg, 60.97%).

LCMS: (ESI, m/z): 227.00 $[M+H]^+$.

**Step 3: Synthesis of compound 039-3**

Under nitrogen protection, at room temperature, to a solution of ethyl 1-(2-methoxy-2-methylpropyl)-1*H*-pyrazole-4-carboxylate (compound **039-2,** 130 mg, 0.575 mmol, 1 eq) in ethanol (1.5 mL), a solution of sodium hydroxide (45.96 mg, 1.150 mmol, 2 eq) in water (0.5 mL) was added dropwise. The mixture was further stirred at 50°C for 1 h. After the reaction was completed, the reaction mixture was acidified to pH=5 with hydrochloric acid solution. The resulting residue was concentrated under reduced pressure, dissolved in tetrahydrofuran (3.0 mL), filtered, the filter cake was washed with tetrahydrofuran (3 × 3 mL), and the filtrate was concentrated under reduced pressure to give compound **039-3** (100 mg, 87.81%).

LCMS: (ESI, m/z): 199.00 $[M+H]^+$.

**Step 4: Synthesis of N-[3-(7-{[(3S,4R)-3-fluoro-1-methylpiperidin-4-yl]amino}-3-(2,2,2-trifluoroethyl)pyrazolo [1,5-a]pyridin-2-yl)prop-2-yn-1-yl]-1-(2-methoxy-2-methylpropyl)-1*H*-pyrazole-4-carboxamide (compound 039)**

Under nitrogen protection at room temperature, to a solution of 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.044 mmol, 1 eq) and 1-(2-methoxy-2-methylpropyl)-1*H*-pyrazole-4-carboxylic acid (compound **039-3,** 13.03 mg, 0.066 mmol, 1.5 eq) in *N,N*-dimethylformamide (0.8 mL) was added *N,N*-diisopropylethylamine (28.32 mg, 0.220 mmol, 5 eq) dropwise. The mixture was stirred to react for 1 h, and at room temperature, O-(7-azabenzotriazol-1-yl)-*N,N,N',N'*-tetramethyluronium hexafluorophosphate (16.67 mg, 0.044 mmol, 1 eq) was added and the mixture was further stirred for 1 h. After the reaction was completed, the reaction mixture was quenched at room temperature with methanol (1.0 mL) and concentrated under reduced pressure. The crude product was purified by HPLC with the following conditions (column specifications: YMC Triart C18 ExRs 5 μm, 30 mm × 150 mm; mobile phase A: water (10 mmol/L ammonium bicarbonate), mobile phase B: acetonitrile; flow rate: 60 mL/min; elution gradient: 33% B to 63% B in 8 min; detection wavelength: UV 254 nm/220 nm; retention time (min): 7.1), to give compound **N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-1-(2-methoxy-2-methylpropyl)-1*H*-pyrazole-4-carboxamide 039** (20.93 mg, 83.80%).

LCMS: (ESI, m/z): 564.15 $[M+H]^+$.

$^1$H NMR (400 MHz, DMSO-$d_6$, *ppm)* $\delta$ 8.70 (t, *J* = 5.5 Hz, 1H), 8.11 (s, 1H), 7.86 (s, 1H), 7.28 (dd, *J* = 8.8, 7.6 Hz, 1H), 7.02 (d, *J* = 8.7 Hz, 1H), 6.32 (d, J = 7.4 Hz, 1H), 6.24 (d, *J* = 9.0 Hz, 1H), 4.87 (d, *J* = 49.6 Hz, 1H), 4.33 (d, *J* = 5.6 Hz, 2H), 4.15 (s, 2H), 3.94 - 3.82 (m, 1H), 3.75 (q, *J* = 11.1 Hz, 2H), 3.18 (s, 3H), 3.04 (t, *J* = 10.1, 1H), 2.78 (d, *J* = 11.4 Hz, 1H), 2.36 - 2.23 (m, 1H), 2.20 (s, 3H), 2.13 (t, *J* = 11.1 Hz, 1H), 1.99 - 1.80 (m, 2H), 1.07 (s, 6H).

**Example 34**

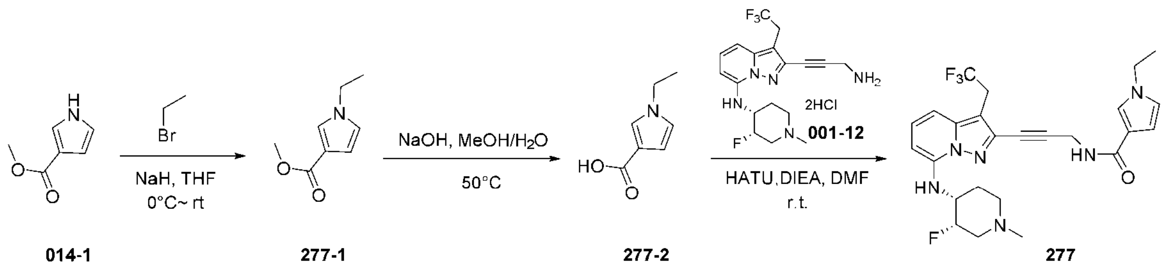


014-1 277-1 277-2 277

**Step 1: Synthesis of compound 277-1**

Under nitrogen protection, at 0°C, to a solution of methyl pyrazole-4-carboxylate (compound **014-1,** 200 mg, 1.598 mmol, 1 eq) in tetrahydrofuran (5 mL) was added sodium hydride (76.72 mg, 3.196 mmol, 2 eq), and the mixture was stirred to react at room temperature for 30 min. Then, bromoethane (348.34 mg, 3.196 mmol, 2 eq) dissolved in tetrahydrofuran (2 mL) was added dropwise at 0°C and the reaction was carried out at room temperature for 2 h. After the reaction was completed, at room temperature, the reaction mixture was quenched with saturated ammonium chloride aqueous solution and extracted with ethyl acetate (3 × 20 mL). The organic phases were combined, backwashed with saturated sodium chloride solution (1 × 20 mL), dried over anhydrous sodium sulfate, and filtered. The filtrate was concentrated under reduced pressure. The resulting residue was purified by silica gel column chromatography (petroleum ether/ethyl acetate = 9:1) to give compound **277-1** (240 mg, 98.02%).

LCMS: (ESI, m/z): 154.10 $[M+H]^+$.

**Step 2: Synthesis of compound 277-2**

At 50°C, a solution of methyl 1-ethyl-1H-pyrrole-3-carboxylate (compound **277-1,** 20 mg, 0.131 mmol, 1 eq) and sodium hydroxide (5.22 mg, 0.131 mmol, 1 eq) in methanol (1 mL) and water (1 mL) was added to a reaction flask and stirred to react for 1 h. After the reaction was completed, the reaction mixture was adjusted to pH=5 with 1 mol/L hydrochloric acid solution and concentrated in vacuum to give compound **277-2** (15 mg, 82.56%).

LCMS: (ESI, m/z): 140.15 $[M+H]^+$.

**Step 3: Synthesis of 1-ethyl-N-[3-(7-{[(3S,4R)-3-fluoro-1-methylpiperidin-4-yl]amino}-3-(2,2,2-trifluoroethyl) pyrazolo[1,5-a]pyridin-2-yl)prop-2-yn-1-yl]-1H-pyrrole-3-carboxylic acid amide (compound 277)**

At room temperature, a solution of 1-ethyl-1*H*-pyrrole-3-carboxylic acid (compound **277-2,** 6.1 mg, 0.044 mmol, 1 eq), O-(7-azabenzotriazol-1-yl)-*N,N,N',N'*-tetramethyluronium hexafluorophosphate (16.67 mg, 0.044 mmol, 1 eq) and *N,N*-diisopropylethylamine (28.32 mg, 0.220 mmol, 5 eq) in *N,N*-dimethylformamide (1 mL) was added to a reaction flask, and stirred to react for 30 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.044 mmol, 1 eq) was added and the system was stirred to react at room temperature for 2 h. After the reaction was completed, the reaction mixture was quenched with water at room temperature and extracted with ethyl acetate (3 × 30 mL). The organic phases were combined, backwashed with saturated sodium chloride solution (3 × 30 mL), dried over anhydrous sodium sulfate, filtered, and the filtrate was concentrated under reduced pressure. The crude product was purified by HPLC with the following conditions (column specifications: Sunfire C18 5 μm, 30 mm × 150 mm; mobile phase A: water (0.1% ammonium bicarbonate), mobile phase B: acetonitrile; flow rate: 60 mL/min; elution gradient: 36% to 51% in 7 min; detection wavelength: UV 254 nm/220 nm; retention time (min): 6.22), to give compound **1-ethyl-N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-1*H*H-pyrrole-3-carboxylic acid amide 277** (13.84 mg, 62.27%).

LCMS: (ESI, m/z): 505.15 $[M+H]^+$.

$^1$H NMR (400 MHz, DMSO-$d_6$, *ppm)* δ 8.31 *(t, J* = 5.7 Hz, 1H), 7.38 (t, *J* = 2.0 Hz, 1H), 7.28 (dd, *J* = 8.8, 7.6 Hz, 1H), 7.02 (d, *J* = 8.6 Hz, 1H), 6.79 (t, *J* = 2.5 Hz, 1H), 6.48 (dd, *J* = 2.9, 1.8 Hz, 1H), 6.31 (d, J = 7.3 Hz, 1H), 6.25 *(d, J* = 9.0 Hz, 1H), 4.87 (d, *J* = 49.1 Hz, 1H), 4.29 (d, *J* = 5.6 Hz, 2H), 3.93 (q, *J* = 7.2 Hz, 2H), 3.88 - 3.80 (m, 1H), 3.75 (q, *J* = 11.2 Hz, 2H), 3.05 (t, *J* = 11.8 Hz, 1H), 2.78 (d, *J* = 11.4 Hz, 1H), 2.37 - 2.23 (m, 1H), 2.20 (s, 3H), 2.12 (t, *J* = 11.1 Hz, 1H), 2.00 - 1.87 (m, 1H), 1.86 - 1.79 (m, 1H), 1.32 (t, *J* = 7.3 Hz, 3H).

## Example 35

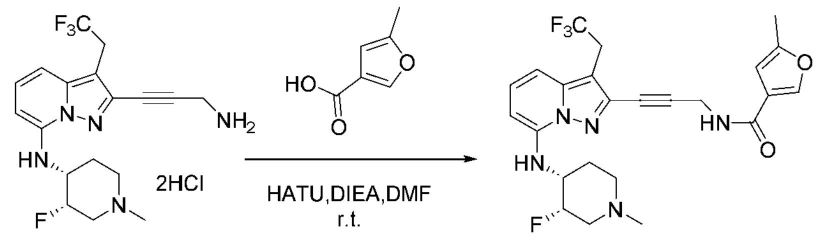


**001-12** **278**

**Step 1: Synthesis of N-[3-(7-{[(3S,4R)-3-fluoro-1-methylpiperidin-4-yl]amino}-3-(2,2,2-trifluoroethyl)pyrazolo [1,5-a]pyridin-2-yl)prop-2-yn-1-yl]-5-methylfuran-3-carboxamide (compound 278)**

At room temperature, a solution of 5-methylfuran-3-carboxylic acid (8.29 mg, 0.066 mmol, 1.5 eq), *N,N*-diisopropylethylamine (0.03 mL, 0.176 mmol, 4 eq), and O-(7-azabenzotriazol-1-yl)-*N,N,N',N'*-tetramethyluronium hexafluorophosphate (16.67 mg, 0.044 mmol, 1 eq) in *N,N*-dimethylformamide (1 mL) was added to a reaction flask and stirred at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.044 mmol, 1.00 eq) was added to the above system, and the system was stirred for 1 h. After the reaction was completed, the reaction mixture was diluted with ethyl acetate (5 mL), washed with saturated sodium chloride solution (10 mL × 3), dried over anhydrous sodium sulfate, filtered, and the filtrate was concentrated under reduced pressure. The crude product was purified by HPLC with the following conditions (column specifications: Kinetex 5 μm EVO C18, 30mm * 150 mm; mobile phase A: water (10 mmol/L ammonium bicarbonate), mobile phase B: acetonitrile; flow rate: 60 mL/min; elution gradient: 31% B to 51% B in 7 min; detection wavelength: UV 254 nm/220 nm; retention time (min): 6.98), to give compound **N-[3-(7-{[(3S,4R)-3-fluoro-1-methylpiperidin-4-yl] amino}-3-(2,2,2-trifluoroethyl)pyrazolo [1,5-a] pyridin-2-yl)prop-2-yn-1-yl]-5-methylfuran-3-carboxamide 278** (5.2 mg, 23.87%).

LCMS: (ESI, m/z): 492.05 $[M+H]^+$.

$^1$H NMR (400 MHz, DMSO-$d_6$, *ppm)* δ 8.70 (t, *J* = 5.6 Hz, 1H), 8.05 (s, 1H), 7.29 (t, J = 8.2 Hz), 7.03 (d, *J* = 8.7 Hz, 1H), 6.48 (s, 1H), 6.32 (d, *J* = 7.7 Hz, 1H), 6.23 (d, *J* = 9.0 Hz, 1H), 4.87 (d, *J*= 49.5 Hz, 1H), 4.32 (d, *J* = 5.6 Hz, 2H), 3.98 -

3.82 (m, 1H), 3.75 (q, *J* = 11.2 Hz, 2H), 3.05 (t, J = 10.8 Hz, 1H), 2.78 *(d, J*= 11.4 Hz, 1H), 2.33 (d, *J* = 12.9 Hz, 1H), 2.29 - 2.25 (m, 3H), 2.20 (s, 3H), 2.14 (t, J = 10.5 Hz, 1H), 1.99 - 1.80 (m, 2H).

Example 36

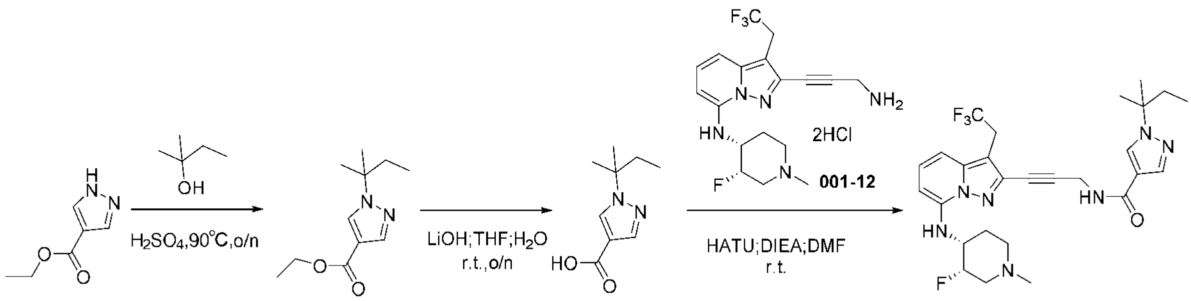


**006-1** **021-1** **021-2** **021**

**Step** 1: **Synthesis of compound 021-1**

Under nitrogen protection, at room temperature, a solution of ethyl 1*H* pyrazole-4-carboxylate (compound **006-1,** 500 mg, 3.568 mmol, 1 eq), concentrated sulfuric acid (220 uL, 4.086 mmol, 1.15 eq, 99%) and 2-methyl-2-butanol (3.06 mL, 27.652 mmol, 7.75 eq) was added to a reaction flask and stirred at 90°C overnight. After the reaction was completed, the reaction mixture was concentrated in vacuum. The resulting residue was purified by reverse phase column chromatography with the following conditions (C18 column, mobile phase: petroleum ether and ethyl acetate, 0% to 30% gradient in 15 min, detection wavelength: UV 254 nm) to give compound **021-1** (270 mg, 41.53%).
LCMS: (ESI, m/z): 211.00 $[M+H]^+$.

**Step 2: Synthesis of compound 021-2**

At room temperature, a solution of ethyl 1-(2-methylbutan-2-yl)pyrazole-4-carboxylate (compound **021-1,** 270 mg, 1.284 mmol, 1 eq) and lithium hydroxide (61.5 mg, 2.568 mmol, 2 eq) in tetrahydrofuran (2 mL) and water (1 mL) was added to a reaction flask and stirred overnight at room temperature. After the reaction was completed, the reaction mixture was concentrated in vacuum, the residue was dissolved in tetrahydrofuran (10 mL), filtered, and the filtrate was concentrated in vacuum to give compound **021-2** (240 mg, 93.44%).
LCMS: (ESI, m/z): 183.00 $[M+H]^+$.

**Step 2: Synthesis of N-[3-(7-{[(3S,4R)-3-fluoro-1-methylpiperidin-4-yl]amino}-3-(2,2,2-trifluoroethyl)pyrazolo [1,5-a]pyridin-2-yl)prop-2-yn-1-yl]-1-(2-methylbutan-2-yl)-1H-pyrazole-4-carboxamide (compound 021)**

At room temperature, a solution of 1-(2-methylbutan-2-yl)pyrazole-4-carboxylic acid (compound **021-2,** 23.96 mg, 0.132 mmol, 2 eq), *N,N*-diisopropylethylamine (33.99 mg, 0.264 mmol, 4 eq), and O-(7-azabenzotriazol-1-yl)-*N*,*N*,*N'*,*N'*-tetramethyluronium hexafluorophosphate (25.0 mg, 0.066 mmol, 1 eq) in *N,N*-dimethylformamide (1.2 mL) was added to a reaction flask and stirred for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 30 mg, 0.066 mmol, 1.00 eq) was added, and the system was stirred for 1 h. After the reaction was completed, the reaction mixture was diluted with ethyl acetate (10 mL), washed with saturated sodium chloride solution (10 mL × 3), dried over anhydrous sodium sulfate, filtered, and the filtrate was concentrated under reduced pressure. The crude product was purified by HPLC with the following conditions (column specifications: Kinetex 5 μm EVO C18, 30mm * 150 mm; mobile phase A: water (10 mmol/L ammonium bicarbonate), mobile phase B: acetonitrile; flow rate: 60 mL/min; elution gradient: 35% B to 54% B in 7 min; detection wavelength: UV 254 nm/226 nm; retention time (min): 6.55), to give compound **N-[3-(7-{[(3S,4R)-3-fluoro-1-methylpiperidin-4-yl] amino}-3-(2,2,2-trifluoroethyl)pyrazolo [1,5-a] pyridin-2-yl)prop-2-yn-1-yl]-1-(2-methylbutan-2-yl)-1*H*-pyrazole-4-carboxamide 021** (14.31 mg, 39.55%).
LCMS: (ESI, m/z): 548.25 $[M+H]^+$.
$^1H$ NMR (400 MHz, DMSO-$d_6$, *ppm)* δ 8.63 (t, *J* = 5.5 Hz, 1H), 8.29 (s, 1H), 7.91 (s, 1H), 7.29 (dd, *J* = 8.8, 7.6 Hz, 1H), 7.03 (d, *J* = 8.7 Hz, 1H), 6.32 (d, J = 7.5 Hz, 1H), 6.23 (d, *J* = 9.0 Hz, 1H), 4.87 *(d, J* = 49.5 Hz, 1H), 4.34 (d, *J* = 5.6 Hz, 2H), 3.93 - 3.81 (m, 1H), 3.76 *(q, J* = 11.1 Hz, 2H), 3.05 (t, *J* = 11.0 Hz, 1H), 2.78 (d, *J* = 11.3 Hz, 1H), 2.38 - 2.22 (m, 1H), 2.20

(s, 3H), 2.13 (t, J = 10.6 Hz, 1H), 2.01 - 1.88 (m, 1H), 1.86 - 1.77 (m, 3H), 1.50 (s, 6H), 0.59 (t, *J* = 7.4 Hz, 3H).

**Example 37**

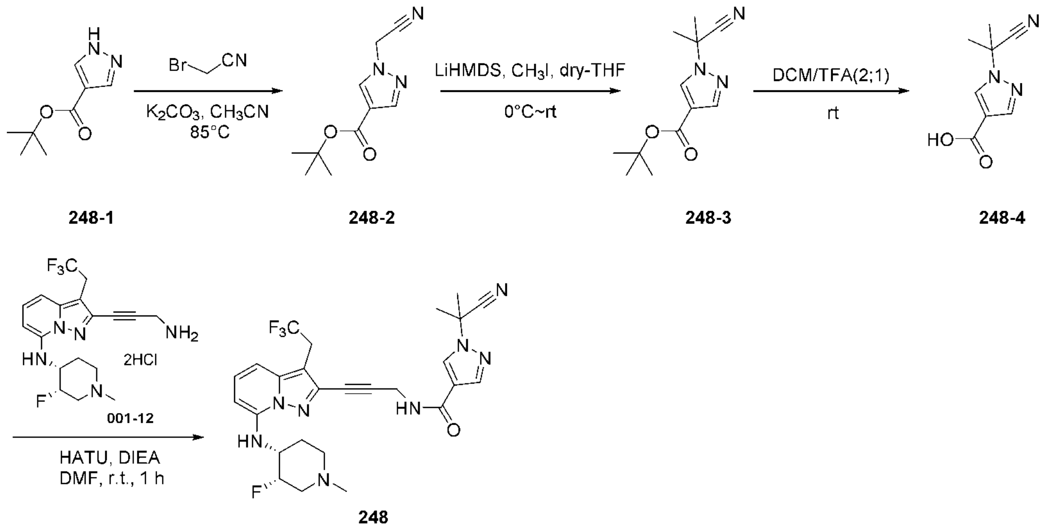


**248-1** **248-2** **248-3** **248-4** **001-12** **248**

**Step 1: Synthesis of compound 248-2**

To a solution of tert-butyl 1*H*-pyrazole-4-carboxylate (compound **248-1,** 200 mg, 1.189 mmol, 1 eq) in acetonitrile (2 mL) were added bromoacetonitrile (171.16 mg, 1.427 mmol, 1.2 eq) and potassium carbonate (493.01 mg, 3.567 mmol, 3 eq) at room temperature. The mixture was stirred at 85°C for 2 h. After the reaction was completed, the mixture was filtered, the filter cake was washed with acetonitrile (3 × 50 mL), the filtrate was concentrated under reduced pressure, the crude product was diluted with water (50 mL), the aqueous phase was extracted with ethyl acetate (3 × 50 mL), the organic phases were combined, dried over anhydrous sodium sulfate, filtered, and the filtrate was concentrated under reduced pressure to give crude compound **248-2** (265 mg), which was directly used in the next step without further purification.

LCMS: (ESI, m/z): 208.00 $[M+H]^+$.

**Step 2: Synthesis of compound 248-3**

At 0°C, to a solution of tert-butyl 1-(cyanomethyl)-1*H*-pyrazole-4-carboxylate (compound **248-2,** 260 mg, 1.255 mmol, 1 eq) in anhydrous tetrahydrofuran (3 mL) was added iodomethane (890.4 mg, 6.275 mmol, 5 eq), and then lithium bis(trimethylsilyl)amide (629.81 mg, 3.765 mmol, 3 eq) was slowly added dropwise. The mixture was warmed to room temperature and allowed to react for 1 h. After the reaction was completed, the reaction mixture was quenched with saturated ammonium chloride at 0°C, diluted with water (20 mL), and extracted with ethyl acetate (3 × 20 mL). The organic phases were combined, backwashed with saturated sodium chloride solution (3 × 20 mL), dried over anhydrous sodium sulfate, filtered, and the filtrate was concentrated under reduced pressure. The crude product was purified by reverse phase column chromatography with the following conditions (column specifications: 40 g, mobile phase: 0.001% trifluoroacetic acid and acetonitrile, 10% to 80% gradient in 15 min, detection wavelength: UV 254 nm), to give compound **248-3** (190 mg, 61.79%).

LCMS: (ESI, m/z): 236.00 $[M+H]^+$.

**Step 3: Synthesis of compound 248-4**

At room temperature, to a solution of tert-butyl 1-(1-cyano-1-methylethyl)-1*H*-pyrazole-4-carboxylate (compound **248-3,** 30 mg, 0.128 mmol, 1 eq) in dichloromethane (1.5 mL) was added trifluoroacetic acid (0.5 mL), and the mixture was stirred to react for 30 min. After the reaction was completed, the reaction solution was concentrated under reduced pressure to give a crude compound **248-4** (30 mg, 59.70%). The crude product was directly used in the next step without further purification.

LCMS: (ESI, m/z): 180.00 $[M+H]^+$.

**Step 3: Synthesis of 1-(1-cyano-1-methylethyl)-N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-1*H*-pyrazole-4-carboxamide (compound 248)**

At room temperature, to a solution of 1-(1-cyano-1-methylethyl)-1*H*-pyrazole-4-carboxylic acid (compound **248-2,** 11.78 mg, 0.066 mmol, 1 eq) in *N,N*-dimethylformamide (1 mL) were added O-(7-azabenzotriazol-1-yl)-*N*,*N*,*N'*,*N'*-tetramethyluronium hexafluorophosphate (25.0 mg, 0.066 mmol, 1 eq) and diisopropylethylamine (33.99 mg, 0.264 mmol, 4 eq), and the mixture was stirred to react for 30 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 30 mg, 0.066 mmol, 1 eq) was added and the mixture was allowed to further react for 1 h. After the reaction was completed, the reaction mixture was diluted with water (20 mL) and extracted with ethyl acetate (3 × 20 mL). The organic phases were combined, backwashed with saturated brine (3 × 20 mL), dried over anhydrous sodium sulfate, filtered, and the filtrate was concentrated under reduced pressure. The crude product was purified by HPLC with the following conditions (column specifications: XBridge BEH Shield RP18 5 μm, 30 mm *150 mm; mobile phase A: water (10 mmol/L ammonium bicarbonate), mobile phase B: acetonitrile; flow rate: 60 mL/min; elution gradient: 36% B to 54% B in 7 min; detection wavelength: UV 254 nm/220 nm; retention time (min): 7.17), to give compound **1-(1-cyano-1-methylethyl)-N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-1H-pyrazole-4-carboxamide 248** (15.7 mg, 43.41%).

LCMS: (ESI, m/z): 545.25 $[M+H]^+$.

$^1H$ NMR (400 MHz, DMSO-d6) δ 8.81 (t, J = 5.5, 5.5 Hz, 1H), 8.53 (s, 1H), 8.07 (s, 1H), 7.29 (t, J = 8.8, 7.6 Hz, 1H), 7.03 (d, J = 8.7 Hz, 1H), 6.32 (d, J = 7.4 Hz, 1H), 6.21 (d, J = 9.1 Hz, 1H), 4.87 (d, J = 49.5 Hz, 1H), 4.36 (d, J = 5.5 Hz, 2H), 3.93 - 3.82 (m, 1H), 3.76 (q, J = 11.2 Hz, 2H), 3.05 (t, J = 10.8 Hz, 1H), 2.78 (d, J = 11.0 Hz, 1H), 2.25 - 2.37 (m, 1H), 2.20 (s, 3H), 2.13 (t, J = 10.3 Hz, 1H), 1.99 (s, 6H), 1.92 (kdd, J = 11.9, 3.5 Hz, 1H), 1.82 - 1.88 (m, 1H).

## Example 38

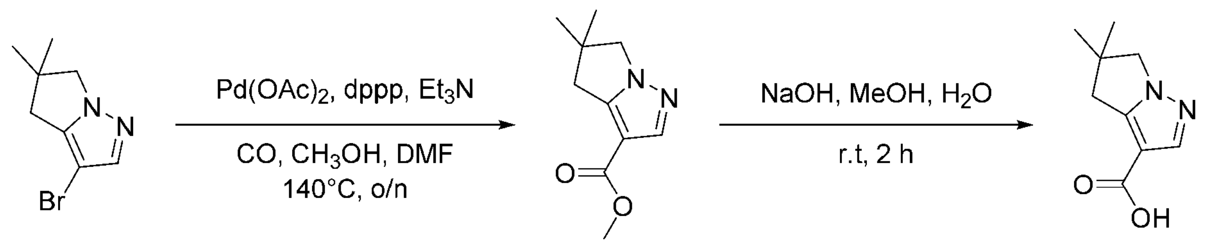


146-1 146-2 146-3

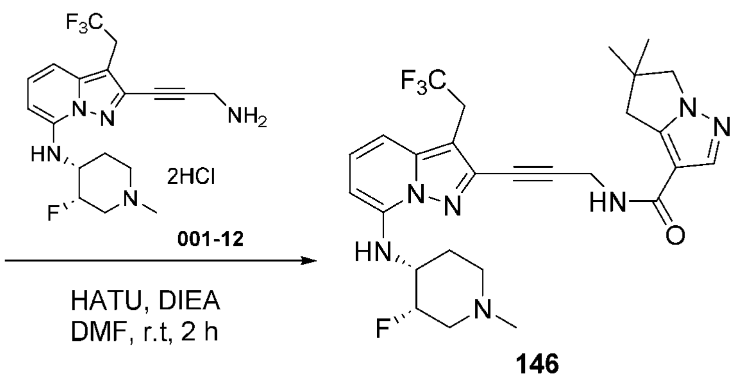


146

**Step 1: Synthesis of compound 146-2**

At room temperature, 3-bromo-5,5-dimethyl-5,6-dihydro-4*H*-pyrrolo[1,2-b]pyrazole (compound **146-1,** 250 mg, 1.162 mmol, 1 eq), palladium acetate (13.05 mg, 0.058 mmol, 0.05 eq), 1,3-bis(diphenylphosphine)propane (23.97 mg, 0.058 mmol, 0.05 eq), triethylamine (823.3 mg, 8.134 mmol, 7 eq), and methanol (2 mL) were added to a reaction flask. Then, N,N-dimethylformamide (2 mL) was added, and carbon monoxide was introduced, the pressure was 4 MPa, and the reaction was carried out at 140°C overnight. After the reaction was completed, the reaction mixture was quenched with

water at room temperature, and extracted with ethyl acetate (3 × 10 mL). The organic phases were combined, backwashed with saturated sodium chloride solution (3 × 10 mL), dried over anhydrous sodium sulfate, and filtered. The filtrate was concentrated under reduced pressure, and the crude product was purified by reverse phase column chromatography with the following conditions (40gC18 reverse phase column, mobile phase: water (0.1% trifluoroacetic acid) and acetonitrile, 5% to 30% gradient in 10 min, detection wavelength: UV 254 nm), to give compound **146-2** (100 mg, 44.30%).
LCMS: (ESI, m/z): 195.05 $[M+H]^+$.

**Step 2: Synthesis of compound 146-3**

At room temperature, a solution of methyl 5,5-dimethyl-5,6-dihydro-4*H*-pyrrolo[1,2-b]pyrazole-3-carboxylate (compound **146-2,** 50 mg, 0.257 mmol, 1 eq) and sodium hydroxide (20.59 mg, 0.514 mmol, 2 eq) in methanol (1 mL) and water (1 mL) was stirred for 2 h. After the reaction was completed, the reaction mixture was acidified to pH=5 with 1 mol/L hydrochloric acid solution, then extracted with ethyl acetate (3 × 10 mL). The organic phases were combined, backwashed with saturated sodium chloride solution (1 × 10 mL), dried over anhydrous sodium sulfate and filtered. The filtrate was concentrated under reduced pressure to give compound **146-3** (40 mg, 86.23%).
LCMS: (ESI, m/z): 181.10 $[M+H]^+$.

**Step 3: Synthesis of N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-5,5-dimethyl-5,6-dihydro-4H-pyrrolo[1,2-b]-1*H*pyrazole-3-carboxamide (compound 146)**

At room temperature, a solution of 5,5-dimethyl-5,6-dihydro-4*H*-pyrrolo[1,2-b]pyrazole-3-carboxylic acid (compound **146-3,** 7.9 mg, 0.044 mmol, 1 eq), O-(7-azabenzotriazol-1-yl)-N,N,N',N'-tetramethyluronium hexafluorophosphate (16.67 mg, 0.044 mmol, 1 eq) and N,N-diisopropylethylamine (28.32 mg, 0.220 mmol, 5 eq) in N,N-dimethylformamide (1 mL) was stirred to react for 30 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.044 mmol, 1 eq) was added and the system was further stirred to react for 2 h. After the reaction was completed, the reaction mixture was quenched with water at room temperature and extracted with ethyl acetate (3 × 10 mL). The organic phases were combined, backwashed with saturated sodium chloride solution (3 × 10 mL), dried over anhydrous sodium sulfate, filtered, and the filtrate was concentrated under reduced pressure. The crude product was purified by HPLC with the following conditions (column specifications: Sunfire C18 5 μm, 30 mm × 150 mm; mobile phase A: water (0.1% ammonium bicarbonate), mobile phase B: acetonitrile; flow rate: 60 mL/min; elution gradient: 32% to 58% in 8 min; detection wavelength: UV 254 nm/220 nm; retention time (min): 7.63), to give compound **N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-5,5-dimethyl-5,6-dihydro-4H-pyrrolo-[1,2-b]pyrazole-3-carboxamide 146** (14.94 mg, 61.91%).
LCMS: (ESI, m/z): 546.15 $[M+H]^+$.
$^1$H NMR (400 MHz, DMSO-$d_6$, *ppm)* δ 8.47 *(t, J* = 5.6 Hz, 1H), 7.91 (s, 1H), 7.28 (dd, *J* = 8.7, 7.6 Hz, 1H), 7.03 (d, *J* = 8.6 Hz, 1H), 6.32 (d, *J* = 7.6 Hz, 1H), 6.24 (d, *J* = 9.0 Hz, 1H), 4.87 (d, *J* = 49.4 Hz, 1H), 4.30 (d, *J* = 5.6 Hz, 2H), 3.85 (s, 3H), 3.76 (q, *J* = 11.1 Hz, 2H), 3.05 (t, *J* = 11.4 Hz, 1H), 2.84 (s, 2H), 2.78 (d, *J* = 11.4 Hz, 1H), 2.36 - 2.21 (m, 1H), 2.20 (s, 3H), 2.13 (t, *J* = 10.6 Hz, 1H), 1.99 - 1.78 (m, 2H), 1.22 (s, 6H).

**Example 39**

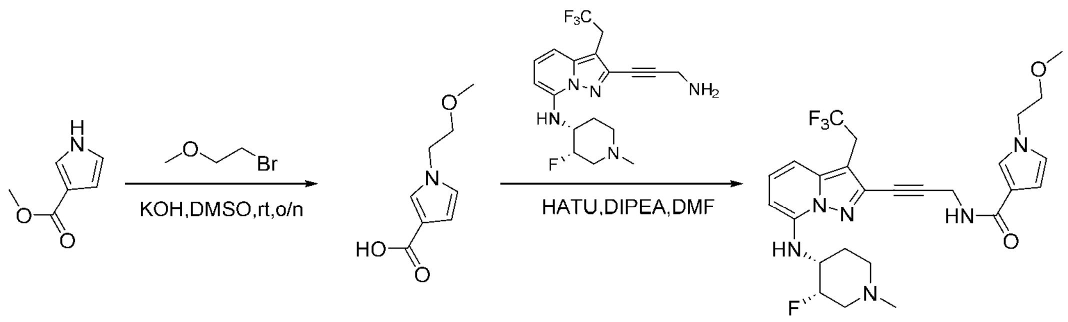


**014-1** **279-1** **279**

**Step 1: Synthesis of compound 279-2**

Under air protection, a solution of methyl pyrazole-4-carboxylate (compound **014-1,** 500 mg, 3.996 mmol, 1 eq) and 2-bromoethyl methyl ether (1.11 g, 7.992 mmol, 2 eq) in dimethyl sulfoxide (5 mL) was stirred to react at room temperature overnight. After the reaction was completed, the reaction mixture was extracted with dichloromethane (3 × 10 mL), the organic phases were combined, dried over anhydrous sodium sulfate, filtered, and the filtrate was concentrated under reduced pressure. Compound **279-1** (600 mg, 81.96%) was obtained.
LCMS: (ESI, m/z): 170.00 $[M+H]^+$.

**Step 2: Synthesis of N-[3-(7-{[(3S,4R)-3-fluoro-1-methylpiperidin-4-yl]amino}-3-(2,2,2-trifluoroethyl)pyrazolo [1,5-a]pyridin-2-yl)prop-2-yn-1-yl]-1-(methoxyethyl)-1H-pyrrole-3-carboxamide (compound 279)**

At room temperature, to a solution of 1-(2-methoxyethyl)pyrrole-3-carboxylic acid (compound **279-1,** 6.62 mg, 0.039 mmol, 1.5 eq) in *N,N*-dimethylformamide (1 mL) was added 2-(7-azobenzothiazole)-*N,N,N',N'*-tetramethyluronium hexafluorophosphate (19.84 mg, 0.052 mmol, 2 eq), and the mixture was stirred to react for 10 min. Then, (3*S*,4*R*)-N-[2-(3-aminoprop-1-yn-1-yl)-3-(2,2,2-trifluoroethyl)pyrazolo[1,5-*a*]pyridin-7-yl]-3-fluoro-1-methylpiperidin-4-amine (10 mg, 0.026 mmol, 1.00 eq) was added and the mixture was stirred to react for 90 min. The reaction mixture was extracted with ethyl acetate (3 × 10 mL). The organic phases were combined, dried over anhydrous sodium sulfate, filtered, and the filtrate was concentrated under reduced pressure. The crude product was purified by HPLC with the following conditions (column specifications: Sunfire C18 5 μm, 30 mm * 150 mm; mobile phase A: water (0.1% formic acid), mobile phase B: acetonitrile; flow rate: 60 mL/min; elution gradient: 17% B to 35% B in 7 min; detection wavelength: UV 254 nm/220 nm; retention time (min): 6.42), to give compound **N-[3-(7-{ [(3 S,4R)-3-fluoro-1-methylpiperidin-4-yl] amino}-3-(2,2,2-trifluoroethyl)pyrazolo [1,5-a]pyridin-2-yl)prop-2-yn-1-yl]-1-(methoxyethyl)-1H-pyrrole-3-carboxamide 279** (6.14 mg, 18.00%).
LCMS: (ESI, m/z): 535.00 $[M+H]^+$.
$^1$H NMR (400 MHz, DMSO-$d_6$, *ppm)* δ 8.33 (t, *J* = 5.6 Hz, 1H), 7.36 (t, *J* = 1.9 Hz, 1H), 7.28 (t, *J* = 8.2 Hz, 1H), 7.02 (d, *J* = 8.6 Hz, 1H), 6.77 (t, *J* = 2.6 Hz, 1H), 6.47 (dd, *J* = 2.7, 1.8 Hz, 1H), 6.31 (dd, *J* = 7.9 Hz, 1H), 6.27 (d, *J* = 8.9 Hz, 1H), 4.88 (d, *J* = 49.6 Hz, 1H), 4.29 (d, *J* = 5.5 Hz, 2H), 4.06 (t, *J* = 5.2 Hz, 2H), 3.91 - 3.83 (m, 1H), 3.75 (q, *J* = 11.3 Hz, 2H), 3.58 *(t, J* = 5.2 Hz, 2H), 3.23 (s, 3H), 3.13 - 3.02 (m, 1H), 2.80 (d, *J* = 10.2 Hz, 1H), 2.41 - 2.26 (m, 1H), 2.22 (s, 3H), 2.15 (t, *J* = 10.5 Hz, 1H), 2.02 - 1.90 (m, 1H), 1.88 - 1.79 (m, 1H).

**Example 40**

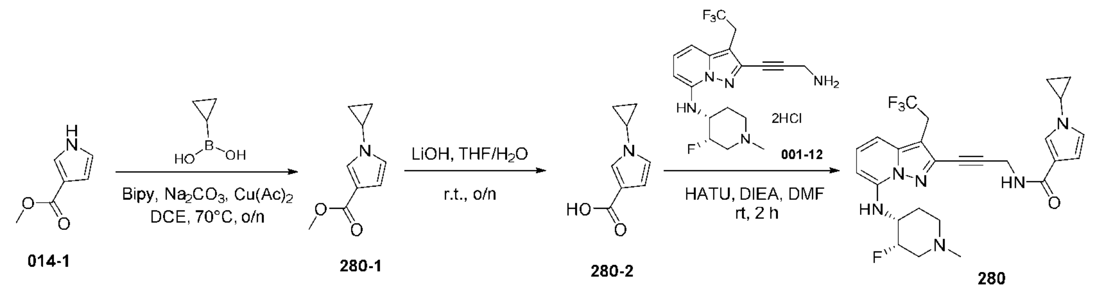


014-1 280-1 280-2 280

**Step 1: Synthesis of compound 280-1**

Under oxygen protection, at 70°C, a solution of methyl pyrazole-4-carboxylate (compound **014-1,** 500 mg, 3.996 mmol, 1 eq), sodium carbonate (423.52 mg, 3.996 mmol, 1 eq), cyclopropylboric acid (686.5 mg, 7.992 mmol, 2 eq), anhydrous copper acetate (725.8 mg, 3.996 mmol, 1 eq) and 2,2'-bipyridine (624.1 mg, 3.996 mmol, 1 eq) in 1,2-dichloroethane (5 mL) was stirred to react overnight. After the reaction was completed, the reaction mixture was extracted with ethyl acetate (3 × 20 mL), dried over anhydrous sodium sulfate, filtered, and the filtrate was concentrated under reduced pressure. The residue was purified by silica gel column chromatography (petroleum ether/ethyl acetate = 5:1) to give compound **280-1** (250 mg, 37.87%).
LCMS: (ESI, m/z): 166.00 $[M+H]^+$.

**Step 2: Synthesis of compound 280-2**

To a solution of methyl 1-cyclopropylpyrrol-3-carboxylate (compound **280-1,** 50 mg, 0.303 mmol, 1 eq) in methanol (0.5 mL)/water (0.5 mL) was added lithium hydroxide (21.75 mg, 0.909 mmol, 3 eq), and the mixture was stirred to react at 50°C for 1 h. After the reaction was completed, the reaction mixture was acidified with hydrochloric acid solution to pH=3, and then extracted with ethyl acetate (3 × 10 mL). The organic phases were combined, dried over anhydrous sodium sulfate, filtered, and the filtrate was concentrated under reduced pressure to give compound **280-2** (43 mg, 93.98%).

LCMS: (ESI, m/z): 151.00 [M+H]$^+$.

**Step 3: Synthesis of 1-cycloproyl-N-[3-(7-{[(3S,4R)-3-fluoro-1-methylpiperidin-4-yl]amino}-3-(2,2,2-ethyl trifluoroacetate)pyrazolo[1,5-a]pyridin-2-yl)-prop-2-yn-1-yl]-1*H*-pyrrole-3-carboxamide (compound 280)**

A solution of 1-cyclopropylpyrrole-3-carboxylic acid (compound **280-2,** 10 mg, 0.066 mmol, 1.5 eq), 2-(3-aminoprop-1-yn-1-yl)-*N*-((3S,4R)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.044 mmol, 1 eq), and *N,N*-diisopropylethylamine (28.5 mg, 0.220 mmol, 5 eq) in *N,N*-dimethylformamide (1 mg, 0.014 mmol, 0.21 eq) was stirred to react at room temperature for 2 h. After the reaction was completed, the reaction mixture was extracted with ethyl acetate (3 × 10 mL). The organic phases were combined, dried over anhydrous sodium sulfate, filtered, and the filtrate was concentrated under reduced pressure. The crude product was purified by HPLC with the following conditions (column specifications: YMC Triart C18 ExRs 5 μm, 30 mm * 150 mm; mobile phase A: water (10 mmol/L ammonium bicarbonate), mobile phase B: acetonitrile; flow rate: 60 mL/min; elution gradient: 40% B to 59% B in 8 min; detection wavelength: UV 254 nm/220 nm; retention time (min): 7.98), to give compound **1-cycloproyl-N-[3-(7-{[(3S,4R)-3-fluoro-1-methylpiperidin-4-yl]amino}-3-(2,2,2-ethyl trifluoroacetate)pyrazolo[1,5-a]pyridin-2-yl)-prop-2-yn-1-yl]-1*H*-pyrrole-3-carboxamide 280** (19.36 mg, 47.66%).

LCMS: (ESI, m/z): 517.00 [M+H]$^+$.

$^1$H (400 MHz, DMSO-d6, ppm) δ 8.32 (t, J = 5.6 Hz, 1H), 7.39 (t, J = 2.0 Hz, 1H), 7.28 (dd, J = 8.8, 7.6 Hz, 1H), 7.02 (d, J = 8.7 Hz, 1H), 6.81 (t, J = 2.5 Hz, 1H), 6.45 (dd, J = 2.9, 1.8 Hz, 1H), 6.31 (d, J = 7.4 Hz, 1H), 6.23 (d, J = 9.0 Hz, 1H), 4.87 (d, J = 49.9 Hz, 1H), 4.29 (d, J = 5.6 Hz, 2H), 3.91 - 3.81 (m, 1H), 3.75 (q, J = 11.2 Hz, 2H), 3.50 - 3.43 (m, 1H), 3.05 (t, J = 11.5 Hz, 1H), 2.78 (d, J = 11.3 Hz, 1H), 2.36 - 2.24 (m, 1H), 2.20 (s, 3H), 2.13 (t, J = 10.5 Hz, 1H), 2.00 - 1.84 (m, 2H), 0.94-0.85 (m,4H).

**Example 41**

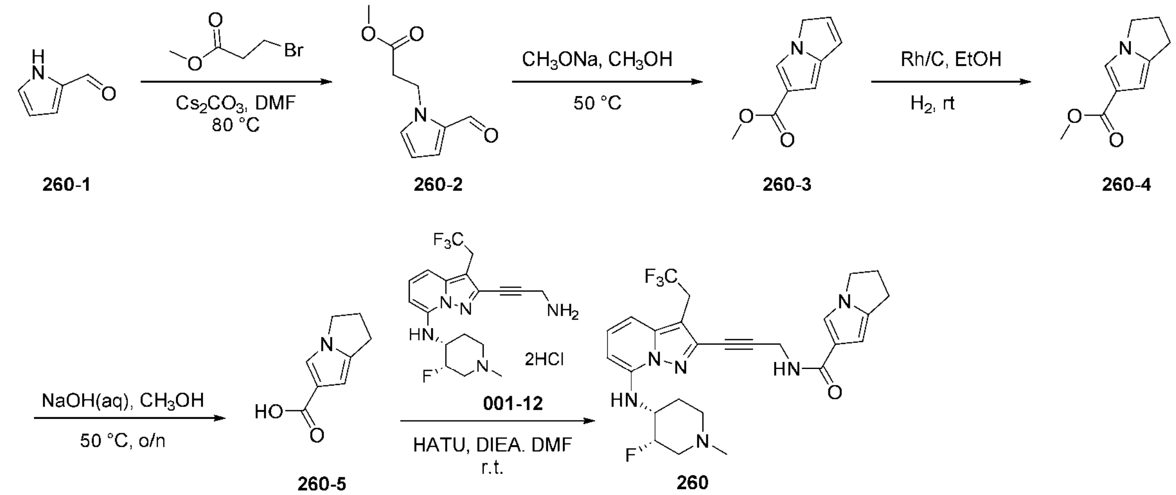


260-1 260-2 260-3 260-4 260-5 001-12 260

**Step** 1: **Synthesis of compound 260-2**

At room temperature, pyrrole-2-carboxaldehyde (compound **260-1,** 2 g, 21.030 mmol, 1 eq), methyl 3-bromopropionate (7.02 g, 42.060 mmol, 2 eq), and cesium carbonate (20.56 g, 63.090 mmol, 3 eq) were added to a reaction flask, and dissolved in N,N-dimethylformamide (20.0 mL, 258.459 mmol, 12.29 eq). The mixture was allowed to react at 80°C for 1 h, then quenched with water and extracted with ethyl acetate (3 × 20 mL). The organic phases were combined,

backwashed with saturated sodium chloride solution (1 × 10 mL), dried over anhydrous sodium sulfate, and filtered. The filtrate was concentrated under reduced pressure. The resulting residue was purified by silica gel column chromatography (petroleum ether/ethyl acetate = 20:1) to give compound **260-2** (2.4 g, 59.83%).

LCMS: (ESI, m/z): 182.05 $[M+H]^+$.

**Step 2: Synthesis of compound 260-3**

At room temperature, methyl 3-(2-formylpyrrol-1-yl)propionate (compound **260-2,** 1 g, 5.519 mmol, 1 eq), and sodium ethoxide (0.33 g, 6.071 mmol, 1.1 eq) were added to a reaction flask, dissolved in ethanol (10.0 mL), and the reaction was carried out at 50°C overnight. After the reaction was completed, the reaction mixture was quenched with water, and extracted with dichloromethane (3 × 10 mL). The organic phases were combined, backwashed with saturated sodium chloride solution (1 × 10 mL), dried over anhydrous sodium sulfate, and filtered. The filtrate was concentrated under reduced pressure. The resulting residue was purified by silica gel column chromatography (petroleum ether/ethyl acetate = 5:1) to give compound **260-3** (210 mg, 22.15%).

LCMS: (ESI, m/z): 164.10 $[M+H]^+$.

**Step 3: Synthesis of compound 260-4**

At room temperature, methyl 3H-pyrrolizine-6-carboxylate (compound **260-3,** 100 mg, 0.613 mmol, 1 eq) and rhodium on carbon (25.23 mg, 0.245 mmol, 0.4 eq) were added to a reaction flask, dissolved in ethanol (5 mL), placed in a hydrogen environment, and stirred at room temperature for 4 h. After the reaction was completed, the reaction solution was filtered through diatomaceous earth, the filter cake was washed with ethyl acetate, and the filtrate was concentrated under reduced pressure to give compound **260-4** (98.7 mg, 97.50%).

LCMS: (ESI, m/z): 166.05 $[M+H]^+$.

**Step 4: Synthesis of compound 260-5**

At room temperature, methyl 2,3-dihydro-1*H*-pyrrolizine-6-carboxylate (compound **260-4,** 50 mg, 0.303 mmol, 1 eq) was added to a reaction flask, dissolved in methanol (1 mL, 24.699 mmol, 81.60 eq), and then a solution of sodium hydroxide (48.42 mg, 1.212 mmol, 4 eq) in water (2 mL) was added. The reaction was allowed to proceed overnight at 50°C. After the reaction was completed, the mixture was adjusted to neutral with 3 M hydrochloric acid solution. The resulting residue was concentrated under reduced pressure, dissolved in tetrahydrofuran (5 mL) solution, filtered, dried over anhydrous sodium sulfate, filtered, and the filtrate was concentrated under reduced pressure to give compound **260-5** (35 mg, 72.59%).

LCMS: (ESI, m/z): 152.00 $[M+H]^+$.

**Step 5: Synthesis of N-[3-(7-{[(3S,4R)-3-fluoro-1-methylpiperidin-4-yl]amino}-3-(2,2,2-trifluoroethyl)pyrazolo [1,5-a]pyridin-2-yl)prop-2-yn-1-yl]-2,3-dihydro-1*H*-pyrrolizine-6-carboxamide (compound 260)**

At room temperature, 2,3-dihydro-1*H*-pyrrolizine-6-carboxylic acid (compound **260-5,** 13.25 mg, 0.088 mmol, 2 eq), *N,N*-diisopropylethylamine (22.66 mg, 0.176 mmol, 4 eq), and O-(7-azabenzotriazol-1-yl)-*N,N,N',N'*-tetramethyluronium hexafluorophosphate (16.67 mg, 0.044 mmol, 1 eq) were added to a reaction flask, dissolved in N,N-dimethylformamide (1 mL), and stirred for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.044 mmol, 1.00 eq) was added, and the system was further stirred for 1 h. After the reaction was completed, the reaction mixture was diluted with ethyl acetate (5 mL), washed with saturated sodium chloride solution (10 mL × 3), dried over anhydrous sodium sulfate, filtered, and the filtrate was concentrated under reduced pressure. The crude product was purified by HPLC with the following conditions (column specifications: Xselect CSHTM Prep C18 5 μm 30*150mm OBD; mobile phase A: water (0.1% formic acid), mobile phase B: acetonitrile; flow rate: 60 mL/min; elution gradient: 12% B to 34% B in 7 min; detection wavelength: UV 254 nm/220 nm; retention time (min): 6.48), to give compound **N-[3-(7-{[(3S,4R)-3-fluoro-1-methylpiperidin-4-yl] amino}-3-(2,2,2-trifluoroethyl)pyrazolo [1,5-a] pyridin-2-yl)prop-2-yn-1-yl]-2,3-dihydro-1*H*-pyrrolizine-6-carboxamide 260** (9.86 mg, 39.71%).

LCMS: (ESI, m/z): 517.15 $[M+H]^+$.

**Example 42**

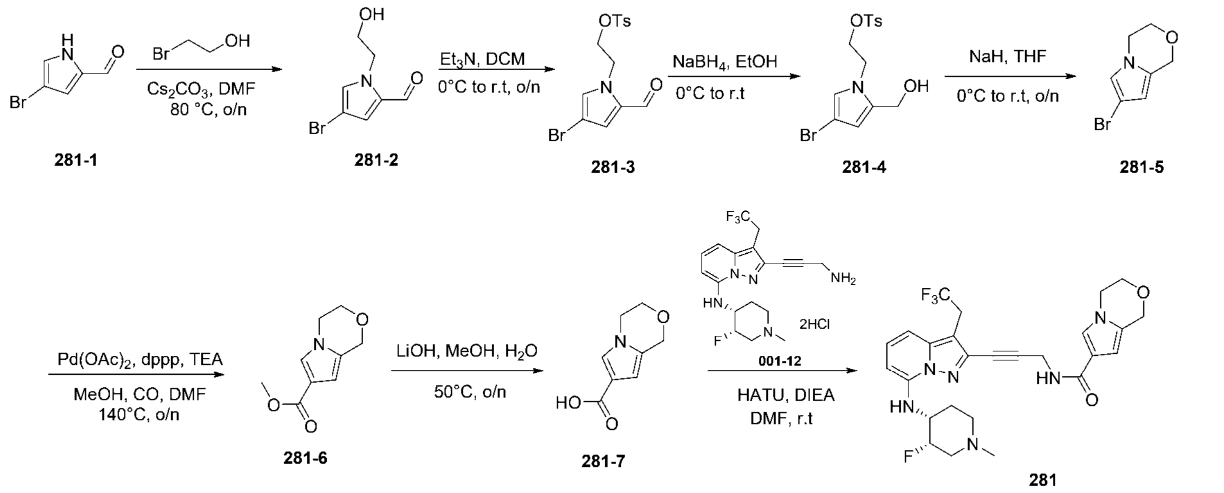


281-1 281-2 281-3 281-4 281-5 281-6 281-7 281

**Step** 1: **Synthesis of compound 281-2**

Under nitrogen protection, 4-bromo-1H-pyrrole-2-carbaldehyde (compound **281-1,** 5 g, 28.736 mmol, 1 eq), 2-bromoethanol (5.39 g, 43.104 mmol, 1.5 eq), and cesium carbonate (18.73 g, 57.472 mmol, 2 eq) were added to a reaction flask. The mixture was dissolved in N,N-dimethylformamide (10 mL), and stirred to react at 80°C for 1 h. After the reaction was completed, the reaction mixture was extracted with ethyl acetate (50 mL × 2), the organic phases were combined, backwashed with saturated sodium chloride solution (50 mL × 3), dried over anhydrous sodium sulfate, and filtered. The filtrate was concentrated under reduced pressure, and the resulting residue was purified by silica gel column chromatography (petroleum ether/ethyl acetate = 1:1) to give compound **281-2** (4 g, 63.65%).

LCMS: (ESI, m/z): 219.95 $[M+H]^+$.

**Step 2: Synthesis of compound 281-3**

Under nitrogen protection, 4-bromo-1-(2-hydroxyethyl)-1H-pyrrole-2-carbaldehyde (compound **281-2,** 3.3 g, 15.134 mmol, 1 eq) and triethylamine (6.31 mL, 45.402 mmol, 3 eq) were added to a reaction flask, dissolved in dichloromethane (20 mL), and p-toluenesulfonyl chloride (3.46 g, 18.161 mmol, 1.2 eq) was added at 0°C. The mixture was stirred to react at room temperature overnight. After the reaction was completed, ice water (15 mL) was added to the reaction mixture at 0°C for quenching, and the mixture was extracted with dichloromethane (20 mL × 3). The organic phases were combined, backwashed with saturated sodium chloride solution (50 mL × 1), dried over anhydrous sodium sulfate, filtered, and the filtrate was concentrated under reduced pressure. Compound **281-3** (3 g, 42.44%) was obtained.

**Step 3: Synthesis of compound 281-4**

Under nitrogen protection, 2-(4-bromo-2-formyl-*1H*-pyrrol-1-yl)ethyl-4-methylbenzenesulfonate (compound **281-3,** 2 g, 5.373 mmol, 1 eq) was added to a reaction flask and dissolved in anhydrous ethanol (10 mL). Sodium borohydride (101.63 mg, 2.687 mmol, 0.5 eq) was added at 0°C and the mixture was stirred to react at room temperature for 1 h. After the reaction was completed, ice water (10 mL) was added to the reaction mixture at 0°C for quenching. The mixture was concentrated under reduced pressure, 20 mL of dichloromethane was added, the mixture was stirred for 5 min, and filtered to give compound **281-4** (1.5 g, 62.21%).

**Step 4: Synthesis of compound 281-5**

Under nitrogen protection, 2-(4-bromo-2-(hydroxymethyl)-1H-pyrrol-1-yl)ethyl-4-methylbenzenesulfonate (compound **281-4,** 1 g, 2.672 mmol, 1 eq) was added to a reaction flask and dissolved in tetrahydrofuran (10 mL, 123.428 mmol). Sodium hydride (76.95 mg, 3.206 mmol, 1.2 eq) was added at 0°C. The mixture was stirred to react at room temperature for 1 h. After the reaction was completed, the reaction mixture was quenched with ice water at 0°C. The mixture was purified by reverse phase column chromatography with the following conditions (column specifications: mobile phase: water and acetonitrile, 10% to 45% gradient in 20 min, detection wavelength: UV 254 nm) to give compound **281-5** (160 mg, 22.05%).

LCMS: (ESI, m/z): 201.95 $[M+H]^+$.

**Step 5: Synthesis of compound 281-6**

7-bromo-3,4-dihydro-1*H*-pyrrolo[2,1-c][1,4]oxazine (compound **281-5,** 160 mg, 0.792 mmol, 1 eq), palladium acetate (17.78 mg, 0.079 mmol, 0.1 eq), 1,3-bis(diphenylphosphino)propane (65.32 mg, 0.158 mmol, 0.2 eq), and triethylamine (560.93 mg, 5.544 mmol, 7 eq) were added to a 20-mL autoclave and dissolved in anhydrous methanol (3 mL, 74.096 mmol, 93.57 eq) and N,N-dimethylformamide (3 mL, 38.765 mmol). 4MPa carbon monoxide was introduced into the autoclave, the mixture was warmed to 140°C and stirred to react overnight. After the reaction was completed, the reaction mixture was purified by reverse phase column chromatography with the following conditions (column specifications: mobile phase: water and acetonitrile, 5% to 20% gradient in 15 min, detection wavelength: UV 254 nm) to give compound **281-6** (90 mg, 62.66%).
LCMS: (ESI, m/z): 182.00 $[M+H]^+$.

**Step 6: Synthesis of compound 281-7**

Under nitrogen protection, methyl 3,4-dihydro-1*H*-pyrrolo[2,1-c][1,4]oxazine-7-carboxylate (compound **281-6,** 80 mg, 0.442 mmol, 1 eq) and sodium hydroxide (35.32 mg, 0.884 mmol, 2 eq) were added to a reaction flask, dissolved in water (1 mL, 55.509 mmol) and anhydrous methanol (3 mL, 74.096 mmol), and stirred to react at 50°C overnight. After the reaction was completed, the reaction mixture was acidified to pH=3 with 1 mol/L hydrochloric acid solution, and the mixture was concentrated under reduced pressure. 10 mL of dichloromethane was added, the mixture was stirred for 5 min, filtered, and the filtrate was concentrated under reduced pressure to give compound **281-7** (70 mg, 90.38%).
LCMS: (ESI, m/z): 167.95 $[M+H]^+$.

**Step 7: Synthesis of N-[3-(7-{[(3S,4R)-3-fluoro-1-methylpiperidin-4-yl]amino}-3-(2,2,2-trifluoroethyl)pyrazolo [1,5-a]pyridin-2-yl)prop-2-yn-1-yl]-3,4-dihydro-1*H*-pyrrolo[2,1-c][1,4]oxazine-7-carboxamide (compound 281)**

Under nitrogen protection, 3,4-dihydro-1H-pyrrolo[2,1-c][1,4]oxazine-7-carboxylic acid (compound **281-7,** 13.19 mg, 0.079 mmol, 1.2 eq), 2-(7-azabenzotriazole)-*N,N,N',N'*-tetramethyluronium hexafluorophosphate (27.5 mg, 0.073 mmol, 1.1 eq), and N,N-diisopropylethylamine (42.49 mg, 0.330 mmol, 5 eq) were added to a reaction flask, dissolved in N,N-dimethylformamide (2 mL, 25.843 mmol) and stirred at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 30 mg, 0.066 mmol, 1.00 eq) was added, and the system was further stirred for 1 h. After the reaction was completed, the reaction mixture was extracted with ethyl acetate (20 mL × 2). The organic phases were combined, backwashed with saturated sodium chloride solution (30 mL × 1), dried over anhydrous sodium sulfate, filtered, and the filtrate was concentrated under reduced pressure. The crude product was purified by HPLC with the following conditions (column specifications: Xselect CSHTM Prep C18 5 μm 30*150mm OBD; mobile phase A: water (0.1% formic acid), mobile phase B: acetonitrile; flow rate: 60 mL/min; elution gradient: 10% B to 32% B in 7 min; detection wavelength: UV 254 nm/220 nm; retention time (min): 6.05), to give compound **N-[3-(7-{ [(3 S,4R)-3-fluoro-1-methylpiperidin-4-yl] amino}-3-(2,2,2-trifluoroethyl)pyrazolo [1,5-a]pyridin-2-yl)prop-2-yn-1-yl]-3,4-dihydro-1*H*-pyrrolo [2,1-c][1,4]oxazine-7-carboxamide 281** (13.62 mg, 38.08%).
LCMS: (ESI, m/z): 533.15 $[M+H]^+$.
$^1$H NMR (400 MHz, DMSO-$d_6$, *ppm)* δ 8.34 (t, *J* = 5.7 Hz, 1H), 7.31 - 7.25 (m, 2H), 7.02 (d, *J= 8.6* Hz, 1H), 6.31 (d, *J* = 7.6 Hz, 1H), 6.26 - 6.21 (m, 2H), 4.87 (d, *J* = 49.5 Hz, 1H), 4.70 (s, 2H), 4.29 (d, *J* = 5.6 Hz, 2H), 3.96 (p, *J* = 2.0 Hz, 4H), 3.91 - 3.81 (m, 1H), 3.87 - 3.71 (q, *J* = 11.2 Hz, 2H), 3.05 (t, *J* = 11.5 Hz, 1H), 2.78 (d, *J* = 11.2 Hz, 1H), 2.35 - 2.24 (m, 1H), 2.20 (s, 3H), 2.13 (t, J = 10.8 Hz, 1H, 1.97 - 1.79 (m, 2H).

**Example 43**

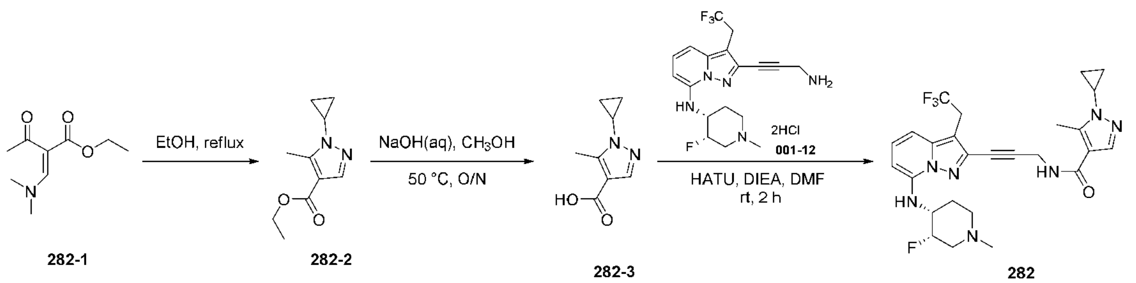


282-1 282-2 282-3 282

**Step 1: Synthesis of compound 282-2**

Ethyl 2-acetyl-3-(dimethylamino)acrylate (compound **282-1,** 1 g, 5.399 mmol, 1 eq) and cyclopropylhydrazine hydrochloride (586.16 mg, 5.399 mmol, 1 eq) were added to a reaction flask, dissolved in ethanol (8 mL), and allowed to react at 80°C for 2 h. After the reaction was completed, the mixture was diluted with 10 mL of dichloromethane, washed with saturated sodium chloride solution (1 × 20 mL), dried over anhydrous sodium sulfate, and the solvent was removed by spin drying. The resulting residue was purified by silica gel column chromatography (petroleum ether/ethyl acetate = 5:1) to give compound **282-2** (454 mg, 41.13%).
LCMS: (ESI, m/z): 195.10 $[M+H]^+$.

**Step 2: Synthesis of compound 282-3**

To a reaction flask, ethyl 1-cyclopropyl-5-methyl-1H-pyrazole-4-carboxylate (compound **282-2,** 100 mg, 0.515 mmol, 1 eq) was added and dissolved in methanol (3 mL). Then, sodium hydroxide (1M) (1 mL, 1.0 mmol, 2 eq) was added. The reaction was carried out at 50°C for 10 h. After the reaction was completed, the solvent was removed by spin drying, and hydrochloric acid (1M) was added to adjust the pH to acidic. The system was extracted with ethyl acetate (3 × 10 mL). The organic phases were combined, backwashed with saturated sodium chloride solution (1 × 10 mL), dried over anhydrous sodium sulfate, and filtered. The filtrate was concentrated under reduced pressure directly to give compound **282-3** (compound **282-3,** 90 mg, 94.67%).
LCMS: (ESI, m/z): 167.05 $[M+H]^+$.

**Step 3: Synthesis of 1-cyclopropyl-N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-5-methyl-1H-pyrazole-4-carboxamide (compound 282)**

To a reaction flask, 1-cyclopropyl-5-methyl-1*H*-pyrazole-4-carboxylic acid (compound **282-3,** 30 mg, 0.181 mmol, 1 eq), 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 69.21 mg, 0.181 mmol, 1 eq), *O*-(7-azabenzotriazol-1-yl)-*N,N,N',N'*-tetramethyluronium hexafluorophosphate (102.96 mg, 0.271 mmol, 1.5 eq), and *N,N*-diisopropylethylamine (116.66 mg, 0.905 mmol, 5 eq) were added in sequence, and dissolved in *N,N*-dimethylformamide (2 mL). The reaction was carried out at room temperature for 1 h. After the reaction was completed, the mixture was quenched with water and extracted with ethyl acetate (3 × 10 mL). The organic phases were combined, backwashed with saturated sodium chloride solution (1 × 10 mL), dried over anhydrous sodium sulfate, filtered, and the filtrate was concentrated under reduced pressure. The crude product was purified by HPLC with the following conditions (column specifications: Sunfire C18 5 μm, 30 mm * 150 mm; mobile phase A: water (0.1% formic acid), mobile phase B: acetonitrile; flow rate: 60 mL/min; elution gradient: 10% B to 33% B in 8 min; detection wavelength: UV 254 nm/220 nm; retention time (min): 7.8), to give compound **1-cyclopropyl-N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-5-methyl-1*H*-pyrazole-4-carboxamide 282** (34.25 mg, 35.58%).
LCMS: (ESI, m/z): 532.55 $[M+H]^+$.
$^1$H NMR (400 MHz, DMSO-$d_6$, *ppm*) $\delta$ 8.53 (t, *J* = 5.6 Hz, 1H), 7.83 (s, 1H), 7.28 (t, *J* = 8.2 Hz, 1H), 7.03 (d, *J* = 8.7 Hz, 1H), 6.32 (d, *J* = 7.6 Hz, 1H), 6.25 (d, *J* = 9.0 Hz, 1H), 4.89 (d, *J* = 49.4 Hz, 1H), 4.30 (d, *J* = 5.6 Hz, 2H), 3.95 - 3.81 (m, 1H), 3.75 (q, *J* = 11.1 Hz, 2H), 3.53 (td, *J* = 7.0, 3.7 Hz, 1H), 3.16 - 3.02 (m, 1H), 2.81 (d, *J* = 11.3 Hz, 1H), 2.58 (s, 3H), 2.42 - 2.28 (m, 1H), 2.23 (s, 3H), 2.18 (s, 1H), 2.01 - 1.76 (m, 2H),1.07 - 0.96 (m, 4H).

**Example 44**

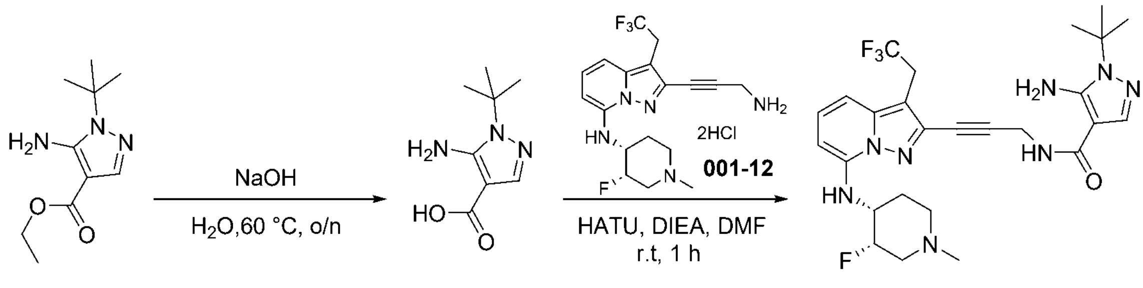


**047-1** **047-2** **047**

**Step 1: Synthesis of compound 047-2**

At room temperature, water (100 mL) was added to a reaction flask, sodium hydroxide (11.36 g, 0.284 mol, 3.0 eq) was added while stirring, and then ethyl 5-amino-1-tert-butyl-1*H*-pyrazole-4-carboxylate (compound **047-1,** 20 g, 94.67 mmol, 1 eq) was added. The system was warmed to 60°C and stirred to react overnight. After the reaction was completed, the system was cooled to room temperature, adjusted to PH 2-2.5 with 4 mol/L hydrochloric acid and extracted with ethyl acetate (3 × 150 mL). The organic phases were combined, backwashed with saturated sodium chloride (1 × 50 mL), dried over anhydrous sodium sulfate and filtered. The filtrate was concentrated under reduced pressure to give compound **047-2** (15.6 mg, 90.1%).

LCMS: (ESI, m/z): 183.95 $[M+H]^+$.

**Step 2: Synthesis of 5-amino-1-(tert-butyl)-N-[3-(7-{[(3S,4R)-3-fluoro-1-methylpiperidin-4-yl]amino}-3-(2,2,2-trifluoroethylpyrazolo[1,5-a]pyridin-2-yl)prop-2-yn-1-yl]-1*H*-pyrazole-4-carboxamide (compound 047)**

At room temperature, a solution of 5-amino-1-tert-butyl-1*H*-pyrazole-4-carboxylic acid (compound **047-2,** 20 mg, 0.109 mmol, 1 eq) and 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 41.85 mg, 0.109 mmol, 1.0 eq), *O*-(7-azabenzotriazol-1-yl)-*N,N,N',N'*-tetramethyluronium hexafluorophosphate (45.66 mg, 0.120 mmol, 1.1 eq), and *N,N*-diisopropylethylamine (70.25 mg, 0.545 mmol, 5 eq) in N,N-dimethylformamide (2 mL) was stirred to react for 1 h. After the reaction was completed, the resulting residue was concentrated under reduced pressure, and the crude product was purified by HPLC with the following conditions (Column: Xselect CSHTM Prep C18 5 μm 30*150mm OBD; mobile phase A: water (0.1% formic acid), mobile phase B: acetonitrile; flow rate: 60 mL/min; elution gradient: 15% B to 31% B in 7 min; detection wavelength: 254 nm/220 nm; retention time (min): 6.43), to give compound **5-amino-1-(tert-butyl)-N-[3-(7-{ [(3 S,4R)-3-fluoro-1-methylpiperidin-4-yl] amino}-3-(2,2,2-trifluoroethyl)pyrazolo[1,5-a]pyridin-2-yl)prop-2-yn-1-yl]-1H-pyrazole-4-carboxamide 047** (9.3 mg, 15.42%).

LCMS: (ESI, m/z): 549.15 $[M+H]^+$.

$^1$H NMR (400 MHz, DMSO-d6) δ 8.31 (t, J = 5.6 Hz, 1H), 7.65 (s, 1H), 7.28 (t, J = 8.3 Hz, 1H), 7.02 (d, J = 8.7 Hz, 1H), 6.31 (d, J = 7.6 Hz, 1H), 6.25 (d, J = 9.0 Hz, 1H), 6.18 (s, 2H), 4.87 (d, J = 49.5 Hz, 1H), 4.28 (d, J = 5.6 Hz, 2H), 3.94 - 3.81 (m, 1H), 3.75 (q, J = 11.2 Hz, 2H), 3.06 (t, J = 10.6 Hz, 1H), 2.79 (d, J = 10.6 Hz, 1H), 2.38 - 2.22 (m, 1H), 2.21 (s, 3H), 2.14 (t, J = 10.7 Hz, 1H), 1.98 - 2.85 (m, 1H), 1.87 - 1.79 (m, 1H), 1.52 (s, 9H).

**Example 45**

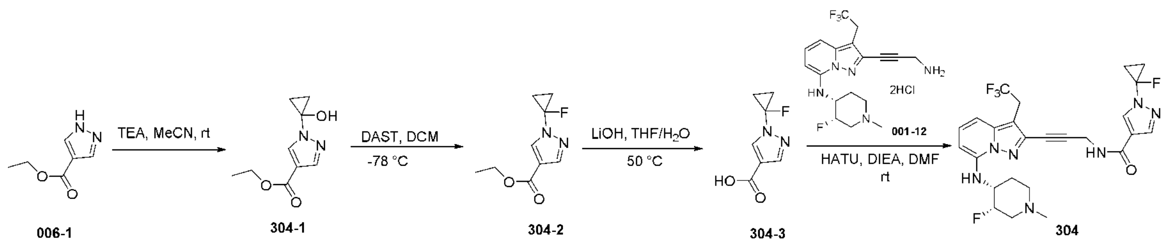


006-1 304-1 304-2 304-3 304

**Step 1: Synthesis of compound 304-1**

Under nitrogen protection, at room temperature, a solution of ethyl 1*H* pyrazole-4-carboxylate (compound **006-1,** 180 mg, 1.284 mmol, 1 eq), 1-(phenylsulfonyl)cyclopropan-1-ol (280.08 mg, 1.412 mmol, 1.1 eq) and triethylamine (129.97 mg, 1.284 mmol, 1 eq) in acetonitrile (18.00 mL) was stirred to react for 1 h. After the reaction was completed, the resulting residue was concentrated under reduced pressure and purified by silica gel column chromatography (petroleum ether/ethyl acetate = 1:1) to give compound **304-1** (150 mg, 59.52%).

LCMS: (ESI, m/z): 197.05 $[M+H]^+$.

**Step 2: Synthesis of compound 304-2**

Under nitrogen protection, at -78°C, a solution of ethyl 1-(1-hydroxycyclopropyl)-1*H*-pyrazole-4-carboxylate (compound **304-1,** 130 mg, 0.663 mmol, 1 eq) and diethylaminosulphur trifluoride (160.20 mg, 0.995 mmol, 1.5 eq) in dichloromethane (2 mL) was stirred to react for 1 h. After the reaction was completed, the reaction mixture was quenched

with saturated sodium bicarbonate aqueous solution (10 mL) at room temperature, and then extracted with ethyl acetate (3 × 10 mL). The organic phases were combined, backwashed with saturated sodium chloride (1 × 10 mL), dried over anhydrous sodium sulfate, and filtered. The filtrate was concentrated under reduced pressure, and the resulting residue was purified by silica gel column chromatography (petroleum ether/ethyl acetate = 10:1) to give compound **304-2** (110 mg, 83.77%).

LCMS: (ESI, m/z): 199.35 $[M+H]^+$.

**Step 3: Synthesis of compound 304-3**

At 50°C, a solution of ethyl 1-(1-fluorocyclopropyl)-1*H*-pyrazole-4-carboxylate (compound **304-2,** 130 mg, 0.656 mmol, 1 eq) and lithium hydroxide (78.55 mg, 3.280 mmol, 5 eq) in tetrahydrofuran (2 mL) and water (2 mL) was stirred to react for 2 h. After the reaction was completed, the reaction mixture was acidified to pH=5 with hydrochloric acid solution, and extracted with ethyl acetate (3 × 30 mL). The organic phases were combined, backwashed with saturated sodium chloride (1 × 30 mL), dried over anhydrous sodium sulfate, filtered, and the filtrate was concentrated under reduced pressure to give compound 304-3 (100 mg, 89.61%).

LCMS: (ESI, m/z): 171.35 $[M+H]^+$.

Step 4: Synthesis of **N-(3-(7-{[(3S,4R)-3-fluoro-1-methylpiperidin-4-yl]**amino}-3-(2,2,2-**trifluoroethylpyrazolo[1,5-a]pyridin-2-yl)prop-2-yn-1-yl]-1-(1-fluorocyclopropyl)-1*H*-**pyrazole-4-carboxamide (compound 304)

At room temperature, a solution of 1-(1-fluorocycloproyl)-1*H*-pyrazole-4-carboxylic acid (compound 304-3, 20 mg, 0.118 mmol, 1 eq) and 2-(3-aminoprop-1-yn-1-yl)-N-((3S,4R)-3-fluoro-1-methylpiperidin-4-yl)-3-(2,2,2-trifluoroethyl) pyrazolo[1,5-*a*]pyridin-7-amine dihydrochloride (compound **001-12,** 45 mg, 0.118 mmol, 1 eq), 2-(7-azabenzotriazole)-*N,N,N',N'*-tetramethyluronium hexafluorophosphate (44.7 mg, 0.118 mmol, 1 eq), and diisopropylethylamine (75.96 mg, 0.590 mmol, 5 eq) in N,N-dimethylformamide (1.66 mL) was stirred to react for 1 h. After the reaction was completed, the reaction mixture was concentrated under reduced pressure, and the crude product was purified by HPLC with the following conditions (Column: XBridge BEH Shield RP18 5 μm, 30 mm *150 mm; mobile phase A: water (0.1% formic acid), mobile phase B: acetonitrile; flow rate: 60 mL/min; elution gradient: 15% B to 31% B in 7 min; detection wavelength: 254 nm/220 nm; retention time (min): 6.43), to give compound **N-(3-(7-{[(3S,4R)-3-fluoro-1-methylpiperidin-4-yl]amino}-3-(2,2,2-trifluoroethylpyrazolo[1,5-a]pyridin-2-yl)prop-2-yn-1-yl]-1-(1-fluorocyclopropyl)-1*H*-pyrazole-4-carboxamide** 304 (10.06 mg, 15.28%).

LCMS: (ESI, m/z): 535.85 $[M+H]^+$.

$^1$H NMR (400 MHz, DMSO-d6) δ 8.86 (t, J = 5.4 Hz, 1H), 8.63 (s, 1H), 8.08 (s, 1H), 7.29 (t, J = 8.2 Hz, 1H), 7.03 (d, J = 8.7 Hz, 1H), 6.32 (d, J = 7.6 Hz, 1H), 6.22 (d, J = 9.0 Hz, 1H), 4.87 (d, J = 49.5 Hz, 1H), 4.36 (d, J = 5.4 Hz, 2H), 3.93 - 3.70 (m, 3H), 3.05 (t, J = 11.9 Hz, 1H), 2.78 (d, J = 11.2 Hz, 1H), 2.33 (d, J = 12.9 Hz, 1H), 2.20 (s, 3H), 2.13 (t, J = 11.8 Hz, 1H), 2.01 - 1.88 (m, 2H), 1.76 - 1.62 (m, 2H), 1.50 (s, 2H).

**Example 46**

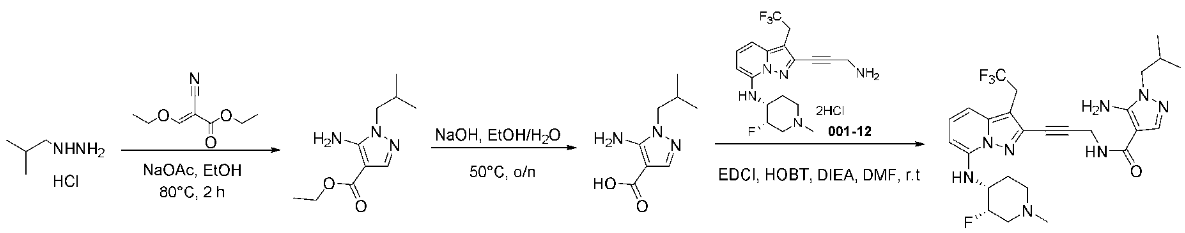


286-1 286-2 286-3 286

**Step** 1: **Synthesis of compound 286-2**

Under nitrogen protection, to a solution of (2-methylpropyl)hydrazine hydrochloride (compound **286-1,** 300 mg, 2.408 mmol, 1 eq) and ethyl (2*E*)-2-cyano-3-ethoxyacrylate (448.03 mg, 2.649 mmol, 1.1 eq) in anhydrous ethanol (6.0 mL) was added sodium acetate (197.5 mg, 2.408 mmol, 1 eq) at room temperature, and the mixture was stirred at 80°C for 2 h. After the reaction was completed, the mixture was filtered, the filter cake was washed with anhydrous ethanol (3 × 4 mL), the filtrate was concentrated under reduced pressure, and the resulting residue was purified by silica gel column chromatography (petroleum ether/ethyl acetate = 5:1) to give compound **286-2** (250 mg, 49.15%).

LCMS: (ESI, m/z): 211.90 [M+H]+.

**Step 2: Synthesis of compound 286-3**

Under nitrogen protection, at room temperature, to a solution of ethyl 5-amino-1-(2-methylpropyl)-1H-pyrazole-4-carboxylate (compound **286-2,** 100 mg, 0.473 mmol, 1 eq) in anhydrous ethanol (1.0 mL) was added a solution of sodium hydroxide (37.86 mg, 0.946 mmol, 2 eq) in water (0.5 mL) dropwise. The mixture was stirred at 50°C overnight. After the reaction was completed, the reaction mixture was acidified to pH=5 with hydrochloric acid solution (3M). The resulting residue was concentrated under reduced pressure. The reaction mixture was dissolved in tetrahydrofuran (3 mL) and filtered. The filtrate was concentrated under reduced pressure to give compound **286-3** (100 mg). The resulting crude product was not further purified and was directly used in the next step.
LCMS: (ESI, m/z): 184.05 [M+H]+.

**Step 3: Synthesis of 5-amino-N-[3-(7-{[(3S,4R)-3-fluoro-1-methylpiperidin-4-yl]amino}-3-(2,2,2-trifluoroethyl) pyrazolo[1,5-a]pyridin-2-yl)prop-2-yn-1-yl]-1-isobutyl-1*H*-pyrazole-4-carboxamide (compound 286)**

Under nitrogen protection, at room temperature, to a solution of 5-amino-1-(2-methylpropyl)-1*H*-pyrazole-4-carboxylic acid (compound **286-3,** 51.39 mg, 0.280 mmol, 1.6 eq) and 1-(3-dimethylaminopropyl)-3-ethyl carbodiimide hydrochloride (50.41 mg, 0.262 mmol, 1.5 eq) in *N,N*-dimethylformamide (1.5 mL) were added N-hydroxybenzotriazole (35.54 mg, 0.262 mmol, 1.5 eq) and N,N-diisopropylethylamine (113.3 mg, 0.875 mmol, 5 eq) in portions. After the reaction was carried out for 1 h, 2-(3-aminoprop-1-yn-1-yl)-*N*-((3*S*,4*R*)-3-fluoro-1-methylpiperidin-4-yl)-3-(2,2,2-trifluoroethyl) pyrazolo[1,5-*a*]pyridin-7-amine dihydrochloride (compound **001-12,** 80 mg, 0.175 mmol, 1 eq) was added at room temperature and the reaction was continued for 1 h. After the reaction was completed, methanol (1.0 mL) was added to the reaction mixture and the reaction mixture was concentrated under reduced pressure. The crude product was purified by HPLC with the following conditions (column specifications: Xselect CSHTM Prep C18 5 μm 30x150mm OBD; mobile phase A: water (0.1% formic acid), mobile phase B: acetonitrile; flow rate: 60 mL/min; elution gradient: 17% B to 30% B in 7 min; detection wavelength: UV 254 nm/220 nm; retention time (min): 5.67), to give the carboxylate of **5-amino-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl) prop-2-yn-1-yl]-1-isobutyl-1H-pyrazole-4-carboxamide 286** (26.9 mg, 25.75%).
LCMS: (ESI, m/z): 549.15 [M+H]+.
$^1$H NMR (400 MHz, DMSO-$d_6$, *ppm*) δ 8.27 (*t, J* = 5.7 Hz, 1H), 7.68 (s, 1H), 7.28 (dd, *J* = 8.8, 7.6 Hz, 1H), 7.02 (d, *J* = 8.6 Hz, 1H), 6.31 (dd, *J* = 7.9, 1.1 Hz, 1H), 6.25 (m, 3H), 4.87 (d, *J* = 49.6 Hz, 1H), 4.29 (d, *J* = 5.6 Hz, 2H), 3.93 - 3.81 (m, 1H), 3.75 (q, *J* = 11.2 Hz, 2H), 3.68 (d, *J* = 7.4 Hz, 2H), 3.06 *(t, J* = 11.5 Hz, 1H), 2.78 (d, *J* = 11.4 Hz, 1H), 2.36 - 2.21 (m, 1H), 2.20 (s, 3H), 2.18 - 2.03 (m, 2H), 2.00 - 1.80 (m, 2H), 0.83 (d, *J* = 6.7 Hz, 6H).

**Example 47**

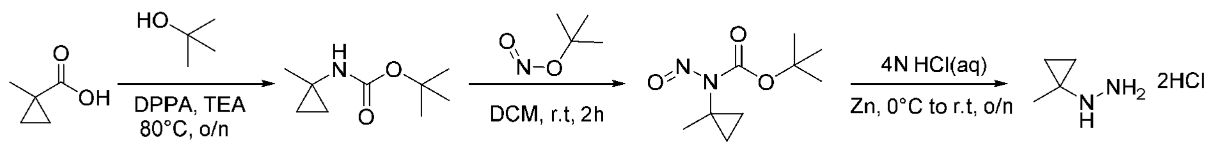


263-1 263-2 263-3 263-4

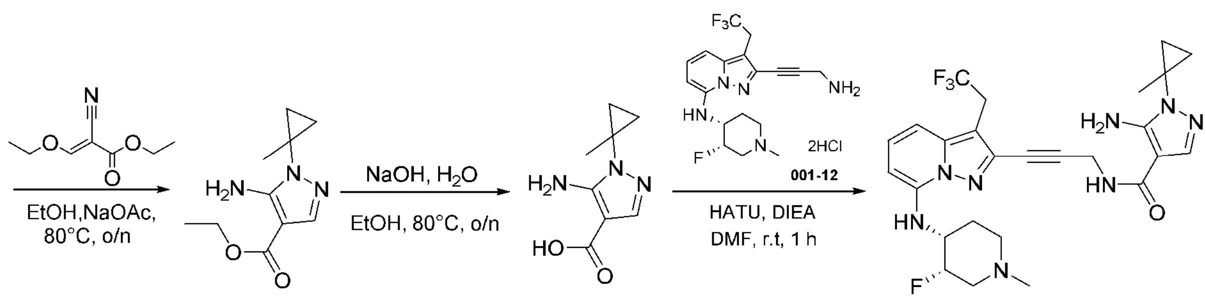


263-5 263-6 263

**Step 1: Synthesis of compound 263-2**

Under nitrogen protection, 1-methylcyclopropane-1-carboxylic acid (compound **263-1,** 3 g, 29.965 mmol, 1 eq) was added to a reaction flask, dissolved in tert-butanol (10 mL), and then diphenylphosphoryl azide (6.5 mL, 30.067 mmol, 1.00 eq) and triethylamine (4 mL) were added. The mixture was warmed to 80°C and stirred to react overnight. After the reaction was completed, the reaction mixture was concentrated under reduced pressure, and the resulting residue was purified by silica gel column chromatography (petroleum ether/ethyl acetate = 9:1) to give compound **263-2** (2 g, 35.12%).
$^1$H NMR (400 MHz, DMSO-$d_6$, *ppm)* $\delta$ 7.04 (s, 1H), 1.36 (s, 9H), 1.22 (s, 3H), 0.57 (q, *J* = 4.4 Hz, 2H), 0.46 - 0.42 (m, 2H).

**Step 2: Synthesis of compound 263-3**

Under nitrogen protection, tert-butyl (1-methylcyclopropyl)carbamate (compound **263-2,** 1.9 g, 11.096 mmol, 1 eq) was added to a reaction flask and dissolved in dichloromethane (20 mL). The reaction flask was purged with nitrogen three times, then tert-butyl nitrite (2.29 g, 22.192 mmol, 2 eq) was added and the mixture was stirred to react at room temperature for 2 h. After the reaction was completed, the reaction mixture was concentrated under reduced pressure and the resulting residue was purified by silica gel column chromatography (petroleum ether/ethyl acetate = 12:1) to give compound **263-3** (1.2 g, 53.96%).
$^1$H NMR (400 MHz, DMSO-$d_6$, *ppm)* $\delta$ 1.59 (s, 9H), 1.09 (s, 3H), 0.87 - 0.83 (m, 2H), 0.64 (s, 2H).

**Step 3: Synthesis of compound 263-4**

Under nitrogen protection, tert-butyl (1-methylcyclopropyl)(nitroso)carbamate (compound **263-3,** 1.1 g, 5.493 mmol, 1 eq) and zinc powder (0.72 g, 10.986 mmol, 2 eq) were added to a reaction flask, and the nitrogen was replaced 3 times. The mixture was cooled to 0°C, and 4 mol/L hydrochloric acid aqueous solution (8 mL) was added. The mixture was warmed to room temperature and stirred to react overnight. After the reaction was completed, the mixture was filtered, the filter cake was washed with water (5 mL $\times$ 2), and the filtrate was concentrated under reduced pressure to give compound **263-4** (1.2 g, 89.09%).

**Step 4: Synthesis of compound 263-5**

Under nitrogen protection, (1-methylcyclopropyl)hydrazine hydrochloride (compound **263-4,** 1 g, 8.157 mmol, 1 eq), ethyl (*E*)-2-cyano-3-ethoxyacrylate (1.38 g, 8.157 mmol, 1 eq), and sodium acetate (669.12 mg, 8.157 mmol, 1 eq) were added to a reaction flask, dissolved in anhydrous ethanol (10 mL) and stirred to react at 80°C overnight. After the reaction was completed, the mixture was filtered, the filter cake was washed with ethyl acetate (20 mL), the filtrate was concentrated under reduced pressure, and the resulting residue was purified by reverse phase column chromatography with the following conditions (column specifications: C18; mobile phase: water (0.1% trifluoroacetic acid) and acetonitrile, 10% to 50% gradient in 10 min, detection wavelength: UV 254 nm) to give compound **263-5** (70 mg, 4.10%).
LCMS: (ESI, m/z): 210.05 $[M+H]^+$.

**Step 5: Synthesis of compound 263-6**

Under nitrogen protection, ethyl 5-amino-1-(1-methylcyclopropyl)-1H-pyrazole-4-carboxylate (compound 263-5, 60 mg, 0.287 mmol, 1 eq) and sodium hydroxide (22.94 mg, 0.574 mmol, 2 eq) were added to a reaction flask, dissolved in anhydrous ethanol (5 mL) and water (2 mL), and stirred to react at 80°C overnight. After the reaction was completed, the reaction mixture was acidified to a pH of about 3 with 1 mol/L hydrochloric acid solution. The mixture was then concentrated under reduced pressure to give a crude product. To the crude product was added 10 mL of tetrahydrofuran, and the mixture was stirred for 5 min, filtered, and the filtrate was concentrated under reduced pressure to give compound **263-6** (30 mg, 56.18%).
LCMS: (ESI, m/z): 181.95 $[M+H]^+$.

**Step 6: Synthesis of 5-amino-*N*-[3-(7-{[(3*S*,4*R*)-3-fluoro-1-methylpiperidin-4-yl]amino}-3-(2,2,2-trifluoroethyl) pyrazolo[1,5-*a*]pyridin-2-yl)prop-2-yn-1-yl]-1-(1-methylcyclopropyl)-1H-pyrazole-4-carboxamide (compound 263)**

Under nitrogen protection, 5-amino-1-(1-methylcyclopropyl)-1H-pyrazole-4-carboxylic acid (compound **263-6,** 14.3 mg, 0.079 mmol, 1.2 eq), 2-(7-azabenzotriazole)-*N,N,N',N'*-tetramethyluronium hexafluorophosphate (25.0 mg, 0.066 mmol, 1 eq), and *N,N*-diisopropylethylamine (42.49 mg, 0.330 mmol, 5 eq) were added to a reaction flask, dissolved

in *N,N*-dimethylformamide (2 mL, 25.843 mmol) and stirred at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 30 mg, 0.066 mmol, 1.00 eq) was added, and the system was stirred to react at room temperature for 1 h. After the reaction was completed, water was added and the mixture was extracted with ethyl acetate (20 mL × 2). The organic phases were combined, backwashed with saturated sodium chloride solution (30 mL × 1), dried over anhydrous sodium sulfate, filtered, and the filtrate was concentrated under reduced pressure. The crude product was purified by HPLC with the following conditions (column specifications: Xselect CSHTM Prep C18 5 μm 30*150mm OBD; mobile phase A: water (0.1% formic acid), mobile phase B: acetonitrile; flow rate: 60 mL/min; elution gradient: 10% B to 34% B in 7 min; detection wavelength: UV 254 nm/220 nm; retention time (min): 5.88), to give the carboxylate of compound **5-amino-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1-(1-methylcyclopropyl)-1*H*-pyrazole-4-carboxamide 263** (13.4 mg, 36.47%).

LCMS: (ESI, m/z): 547.05 $[M+H]^+$.

1H NMR (400 MHz, DMSO) δ 8.25 (t, J = 5.6 Hz, 1H), 7.63 (s, 1H), 7.29 (dd, J = 14.0, 5.5 Hz, 1H), 7.02 (d, J = 8.6 Hz, 1H), 6.31 (d, J = 7.4 Hz, 1H), 6.23 (d, J = 9.1 Hz, 1H), 6.18 (s, 2H), 4.87 (d, J = 49.8 Hz, 1H), 4.28 (d, J = 5.6 Hz, 2H), 3.92 - 3.82 (m, 1H), 3.75 (q, *J* = 11.2 Hz, 2H), 3.05 (t, J = 10.7 Hz, 1H), 2.78 (d, J = 11.2 Hz, 1H), 2.37 - 2.23 (m, 1H), 2.20 (s, 3H), 2.13 (t, J = 10.4 Hz, 1H), 2.00 - 1.80 (m, 2H), 1.36 (s, 3H), 1.07 - 1.01 (m, 2H), 0.89 (m, 2H).

**Example 48**

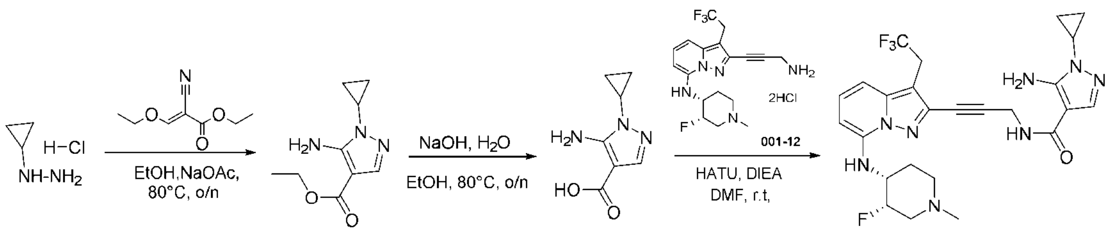


**305-1** **305-2** **305-3** **305**

**Step** 1: **Synthesis of compound 305-2**

Under nitrogen protection, cyclopropyl hydrazine hydrochloride (compound **305-1,** 1 g, 9.211 mmol, 1 eq), ethyl (*E*)-2-cyano-3-ethoxyacrylate (1.56 g, 9.211 mmol, 1 eq), and sodium acetate (0.76 g, 9.211 mmol, 1 eq) were added to a reaction flask, dissolved in anhydrous ethanol (10 mL), warmed to 80°C and stirred to react overnight. After the reaction was completed, the reaction mixture was concentrated under reduced pressure. The resulting residue was purified by silica gel column chromatography (petroleum ether/ethyl acetate = 4:1) to give compound **305-2** (900 mg, 49.50%).

LCMS: (ESI, m/z): 196.05 $[M+H]^+$.

**Step 2: Synthesis of compound 305-3**

Under nitrogen protection, ethyl 5-amino-1-cyclopropyl-1H-pyrazole-4-carboxylate (compound **305-2,** 300 mg, 1.537 mmol, 1 eq) and sodium hydroxide (122.93 mg, 3.074 mmol, 2 eq) were added to a reaction flask, dissolved in anhydrous ethanol (5 mL) and water (2 mL), and warmed to 80°C and stirred to react overnight. After the reaction was completed, the reaction mixture was acidified to a pH of about 3 with 1 mol/L hydrochloric acid solution. The mixture was then concentrated under reduced pressure, to the crude product was added 10 mL of anhydrous ethanol, and the mixture was stirred for 5 min, filtered, and the filtrate was concentrated under reduced pressure to give compound **305-3** (150 mg, 57.22%).

LCMS: (ESI, m/z): 167.95 $[M+H]^+$.

**Step 3: Synthesis of 5-amino-*N*-[3-(7-{[(3*S*,4*R*)-3-fluoro-1-methylpiperidin-4-yl]amino}-3-(2,2,2-trifluoroethyl) pyrazolo[1,5-*a*]pyridin-2-yl)prop-2-yn-1-yl]-1-(cyclopropyl)-1*H*-pyrazole-4-carboxamide (compound 305)**

Under nitrogen protection, 5-amino-1-cyclopropyl-1*H*-pyrazole-4-carboxylic acid (compound **305-3,** 73.27 mg, 0.438 mmol, 2 eq), 2-(7-azabenzotriazole)-*N,N,N',N'*-tetramethyluronium hexafluorophosphate (83.33 mg, 0.219 mmol, 1 eq), and *N,N*-diisopropylethylamine (141.62 mg, 1.095 mmol, 5 eq) were added to a reaction flask, dissolved in *N,N*-dimethylformamide (5 mL, 64.608 mmol) and stirred at room temperature for 10 min. Then, 2-(3-aminoprop-1-yn-1-yl)-N-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 100 mg, 0.219 mmol, 1.00 eq) was added, and the system was stirred to react at room temperature for 1 h. After the reaction was completed, the reaction mixture was extracted with ethyl acetate (20 mL × 2). The organic phases were combined, backwashed with saturated sodium chloride solution (30 mL × 1), dried over anhydrous sodium sulfate, filtered, and the filtrate was concentrated under reduced pressure. The crude product was purified by HPLC with the following conditions (column specifications: YMC Triart C18 ExRs 5 μm, 30 mm * 150 mm; mobile phase A: water (10 mmol/L ammonium bicarbonate), mobile phase B: acetonitrile; flow rate: 60 mL/min; elution gradient: 35% B to 55% B in 9 min; detection wavelength: UV 254 nm/220 nm; retention time (min): 7.05), to give a crude product of compound 305. The crude product was subjected to secondary preparation and purified by HPLC with the following conditions (column specifications: Xselect CSHTM Prep C18 5μm 30*150mm OBD; mobile phase A: water (0.1% formic acid), mobile phase B: acetonitrile; flow rate: 60 mL/min; elution gradient: 12% B to 30% B in 7 min; detection wavelength: UV 254 nm/220 nm; retention time (min): 5.75), to give the carboxylate of compound **5-amino-*N*-[3-(7-{[(3S,4R)-3-fluoro-1-methylpiperidin-4-yl]amino}-3-(2,2,2-trifluoroethyl)pyrazolo[1,5-*a*]pyridin-2-yl)prop-2-yn-1-yl]-1-(cyclopropyl)-1H-pyrazole-4-carboxamide 305** (52.16 mg, 44.47%).

LCMS: (ESI, m/z): 533.55 $[M+H]^+$.

$^1H$ NMR (400 MHz, DMSO-$d_6$, *ppm)* δ 8.24 *(t, J* = 5.7 Hz, 1H), 7.62 (s, 1H), 7.28 (dd, *J* = 8.7, 7.6 Hz, 1H), 7.02 (d, *J* = 8.6 Hz, 1H), 6.31 (d, *J* = 7.6 Hz, 1H), 6.22 (m, 3H), 4.87 (d, *J* = 49.4 Hz, 1H), 4.28 (d, *J* = 5.6 Hz, 2H), 3.93 - 3.80 (m, 1H), 3.74 (q, *J* = 11.2 Hz, 2H), 3.28 - 3.19 (m, 1H), 3.06 *(t, J= 11.2* Hz, 1H), 2.78 (d, *J* = 11.1 Hz, 1H), 2.37 - 2.24 (m, 1H), 2.21 (s, 3H), 2.14 (t, *J* = 10.8 Hz, 1H), 1.99 - 1.80 (m, 2H), 0.95 - 0.91 (m, 4H).

**Example 49**

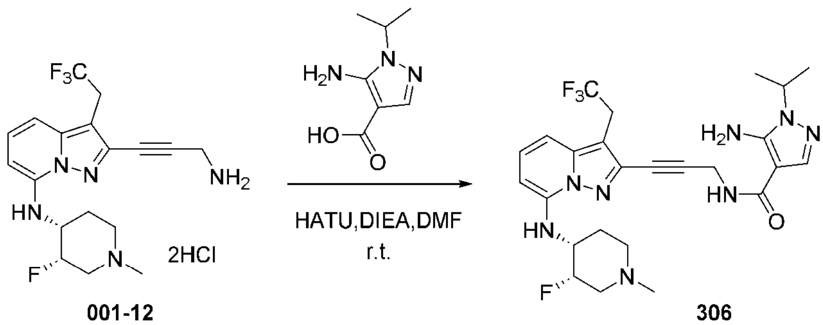


001-12 306

**Step 1: Synthesis of 5-amino-*N*-[3-(7-{[(3*S*,4*R*)-3-fluoro-1-methylpiperidin-4-yl]amino}-3-(2,2,2-trifluoroethyl) pyrazolo[1,5-*a*]pyridin-2-yl)prop-2-yn-1-yl]-1-isopropyl-1*H*-pyrazole-4-carboxamide (compound 306)**

At room temperature, 5-amino-1-isopropyl-1*H*-pyrazole-4-carboxylic acid (444.92 mg, 2.629 mmol, 1.2 eq), N,N-diisopropylethylamine (1132.98 mg, 8.764 mmol, 4 eq), and O-(7-azabenzotriazol-1-yl)-*N,N,N',N'*-tetramethyluronium hexafluorophosphate (833.28 mg, 2.191 mmol, 1 eq) were added to a reaction flask, dissolved in *N,N-dimethylformamide* (20 mL) and stirred at room temperature for 10 min. Then, 2-(3-aminoprop-1-yn-1-yl)-N-((3S,4R)-3-fluoro-1-methylpiperidin-4-yl)-3-(2,2,2-trifluoroethyl)pyrazolo[1,5-*a*]pyridin-7-amine dihydrochloride (compound **001-12,** 1 g, 2.191 mmol, 1 eq) was added and the mixture was stirred at room temperature for 1 h. After the reaction was completed, the reaction mixture was diluted with ethyl acetate (20 mL), washed with saturated sodium chloride solution (20 mL × 3), dried over anhydrous sodium sulfate, filtered, and the filtrate was concentrated under reduced pressure. The resulting residue was purified by silica gel column chromatography (ethyl acetate), to give crude compound **306.** The crude product was purified by HPLC with the following conditions (column specifications: Xselect CSHTM Prep C18 5 μm 30*150mm OBD; mobile phase A: water (0.1% formic acid), mobile phase B: acetonitrile; flow rate: 60 mL/min; elution gradient: 14% B to 26% B in 8 min; detection wavelength: UV 254 nm/220 nm; retention time (min): 6.3), to give the carboxylate of compound **5-amino-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1-isopropyl-1*H*-pyrazole-4-carboxamide 306** (31.21 mg, 2.64%).

LCMS: (ESI, m/z): 534.90 $[M+H]^+$.

$^1H$ NMR (400 MHz, DMSO-$d_6$, *ppm)* δ 8.27 (t, J = 5.6 Hz, 1H), 7.70 (s, 1H), 7.28 (dd, *J* = 8.7, 7.6 Hz, 1H), 7.02 (d, *J* = 8.7 Hz, 1H), 6.31 (d, *J* = 7.6 Hz, 1H), 6.25 (d, *J* = 9.2 Hz, 1H), 6.22 (s, 2H), 4.87 (d, *J* = 49.4 Hz, 1H), 4.40 (p, *J* = 6.5 Hz, 1H), 4.28 (d, *J* = 5.6 Hz, 2H), 3.95 - 3.82 (m, 1H), 3.75 (q, *J* = 11.2 Hz, 2H), 3.05 (t, *J* = 11.4 Hz, 1H), 2.78 (d, *J* = 11.6 Hz, 1H), 2.36 - 2.24 (m, 1H), 2.20 (s, 3H), 2.13 (t, *J* = 11.3 Hz, 1H), 2.00 - 1.79 (m, 2H), 1.28 (d, *J* = 6.5 Hz, 6H).

**Example 50**

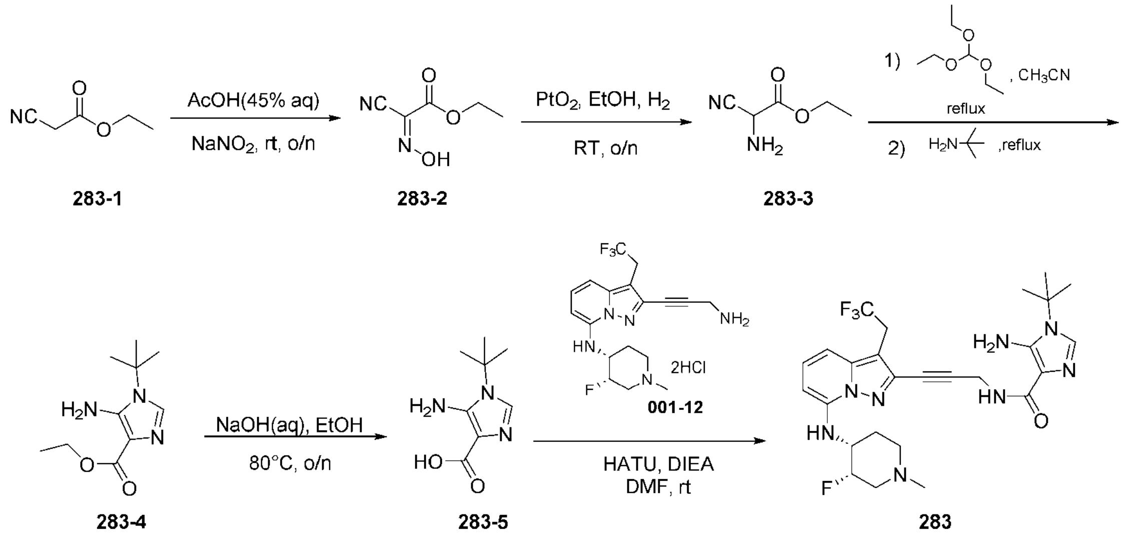


**Step 1: Synthesis of compound 283-2**

Ethyl 2-cyanoacetate (compound **283-1,** 5 g, 44.202 mmol, 1 eq) was added to a reaction flask, and dissolved in acetic acid aqueous solution (45 w/w%, 20 mL). The mixture was allowed to react at 0°C for 15 min, and then sodium nitrite (9.15 g, 132.606 mmol, 3 eq) was added. The mixture was warmed to room temperature and stirred to react overnight. After the reaction was completed, diethyl ether and water were added to the reaction mixture until the reaction mixture became clear. The mixture was extracted with diethyl ether (3 × 30 mL), the organic phases were combined, backwashed with saturated sodium chloride solution (1 × 30 mL), dried over anhydrous sodium sulfate, filtered, and the filtrate was concentrated under reduced pressure to give compound **283-2** (5.5 g, 83.18%).
$^1$H NMR (400 MHz, DMSO-$d_6$, *ppm) δ* 15.07 (s, 1H), 4.31 (q, *J* = 7.1 Hz, 2H), 1.28 (t, *J* = 7.1 Hz, 3H).

**Step 2: Synthesis of compound 283-3**

Ethyl 2-cyano-2-(hydroxyimino)acetate (compound **283-2,** 3 g, 21.110 mmol, 1 eq) and platinum oxide (239.68 mg, 1.056 mmol, 0.05 eq) were added to a reaction flask, dissolved in ethanol (15.0 mL), and then placed in a 0.4 MPa hydrogen environment, stirred to react at room temperature overnight. After the reaction was completed, the system was filtered with diatomaceous earth, the filter cake was washed with dichloromethane, and the filtrate was spin dried to directly give compound **283-3** (2.2 g, 77.27%).
$^1$H NMR (400 MHz, DMSO-$d_6$, *ppm) δ* 4.80 (s, 1H), 4.20 (q, *J* = 7.1, 2H), 1.24 *(t, J* = 7.1 Hz, 3H).

**Step 3: Synthesis of compound 283-4**

Ethyl 2-amino-2-cyanoacetate (compound **283-3,** 1 g, 7.805 mmol, 1 eq), and triethyl orthoformate (2.08 g, 14.049 mmol, 1.8 eq) were added to a reaction flask, dissolved in acetonitrile (20.0 mL), allowed to react at 90°C for 35 min, and then the solvent was removed by spin drying so that the system became oily. Tert-butylamine (970.38 mg, 13.268 mmol, 1.7 eq) was added, the mixture was dissolved in acetonitrile (20.0 mL), and further refluxed to react for 30. After the reaction was completed, the solvent was removed by spin drying, and the residue was dissolved in chloroform (30 mL), and then washed with 2 mol/L sodium hydroxide solution (10 mL) and water (10 mL) in sequence, and the organic phase was spin dried to give compound 283-4 (300 mg, 17.29%).
LCMS: (ESI, m/z): 212.05 [M+H]$^+$.

**Step 4: Synthesis of compound 283-5**

Ethyl 5-amino-1-tert-butyl-1*H*-imidazole-4-carboxylate (compound **283-4,** 100 mg, 0.473 mmol, 1 eq) and 1 mol/L sodium hydroxide aqueous solution (0.94 mL, 0.946 mmol, 2 eq) were added to a reaction flask, dissolved in ethanol (2 mL), and allowed to react at 80°C overnight. After the reaction was completed, the reaction mixture was acidified to pH=3

with 1 mol/L hydrochloric acid solution, and the solvent was removed by spin drying to directly give compound **283-5** (90 mg, 98.59%).

LCMS: (ESI, m/z): 184.05 [M+H]+.

**Step 5: Synthesis of 5-amino-1-tert-butyl-N [3-(7-{[(3S,4R)-3-fluoro-1-methylpiperidin-4-yl]amino}-3-(2,2,2-trifluoroethyl)pyrazolo[1,5-*a*]pyridin-2-yl)prop-2-yn-1-yl]-1*H*-pyrazole-4-carboxamide (compound 283)**

To a reaction flask, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 84 mg, 0.219 mmol, 1 eq), 5-amino-1-tert-butyl-1H-imidazole-4-carboxylic acid compound **283-5** (40.14 mg, 0.219 mmol, 1.00 eq) and *O*-(7-azabenzotriazol-1-yl)-*N,N,N',N'*-tetramethyluronium hexafluorophosphate (99.97 mg, 0.263 mmol, 1.2 eq), and N,N-diisopropylethylamine (141.59 mg, 1.095 mmol, 5 eq) were added, dissolved in *N,N*-dimethylformamide (3 mL) and allowed to react at room temperature for 2 h. After the reaction was completed, the mixture was quenched with water and extracted with ethyl acetate (3 × 10 mL). The organic phases were combined, backwashed with saturated sodium chloride solution (1 × 10 mL), dried over anhydrous sodium sulfate, filtered, and the filtrate was concentrated under reduced pressure. The crude product was purified by HPLC with the following conditions (column specifications: Xselect CSHTM Prep C18 5μm 30*150mm OBD; mobile phase A: water (0.1% formic acid), mobile phase B: acetonitrile; flow rate: 60 mL/min; elution gradient: 13% B to 29% B in 7 min; detection wavelength: UV 254 nm/220 nm; retention time (min): 6.4), to give compound **5-amino-1-tert-butyl-N-[3-(7-{[(3S,4R)-3-fluoro-1-methylpiperidin-4-yl]amino}-3-(2,2,2-trifluoroethyl)pyrazolo [1,5-a]pyridin-2-yl)prop-2-yn-1-yl]-1H-pyrazole-4-carboxamide 283** (35.0 mg, 28.94%).

LCMS: (ESI, m/z): 549.20 [M+H]+.

$^1$H NMR (400 MHz, DMSO-$d_6$)δ 7.97 (t, *J* = 6.0 Hz, 1H), 7.30 - 7.25 (m, 1H), 7.23 (s, 1H), 7.01 (d, *J* = 8.7 Hz, 1H), 6.30 (d, *J* = 7.4 Hz, 1H), 6.26 (d, *J* = 9.0 Hz, 1H), 5.82 (s, 2H), 4.87 (d, *J* = 49.6 Hz, 1H), 4.26 (d, *J* = 6.0 Hz, 2H), 3.91 - 3.82 (m, 1H), 3.75 (q, *J* = 11.2 Hz, 2H), 3.05 (t, *J* = 11.5 Hz, 1H), 2.78 (d, *J* = 11.4 Hz, 1H), 2.36 - 2.23 (m, 1H), 2.20 (s, 3H), 2.13 (t, *J* = 11.3 Hz, 1H), 2.01 - 1.88 (m, 1H), 1.87 - 1.79 (m, 1H), 1.56 (s, 9H).

**Example 51**

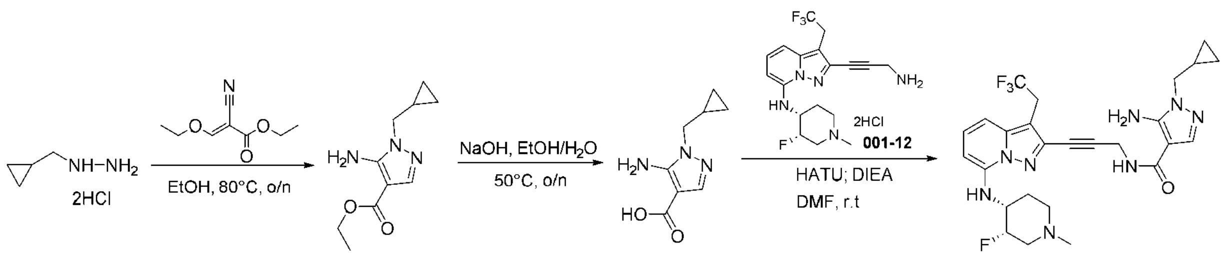


**292-1** **292-2** **292-3** **292**

**Step** 1: **Synthesis of compound 292-2**

Under nitrogen protection, (cyclopropylmethyl)hydrazine dihydrochloride (compound **292-1,** 500 mg, 3.144 mmol, 1 eq), ethyl (2*E*)-2-cyano-3-ethoxyacrylate (531.85 mg, 3.144 mmol, 1 eq), and sodium acetate (257.89 mg, 3.144 mmol, 1 eq) were added to a reaction flask, dissolved in anhydrous ethanol (5 mL), warmed to 80°C and stirred to react overnight. After the reaction was completed, the reaction mixture was concentrated under reduced pressure. The resulting residue was purified by silica gel column chromatography (petroleum ether/ethyl acetate = 8:1) to give compound **292-2** (250 mg, 37.97%).

LCMS: (ESI, m/z): 210.10 [M+H]+.

**Step 2: Synthesis of compound 292-3**

Under nitrogen protection, ethyl 5-amino-1-(cyclopropylmethyl)-1*H*-pyrazole-4-carboxylate (compound **292-2**, 200 mg, 0.956 mmol, 1 eq), and sodium hydroxide (76.46 mg, 1.912 mmol, 2 eq) were added in a reaction flask, dissolved in anhydrous ethanol (5 mL, 86.067 mmol) and water (2 mL, 111.019 mmol), warmed to 50°C and stirred to react for 2 h. After the reaction was completed, the reaction mixture was concentrated under reduced pressure. To the crude product was added 20 mL of water, the mixture was adjusted to pH 3 with 1 mol/L hydrochloric acid solution, and then extracted with ethyl acetate (20 mL × 2). The organic phases were combined, dried over anhydrous sodium sulfate, filtered, and the

filtrate was concentrated under reduced pressure to give compound **292-3** (100 mg, 45.33%).

LCMS: (ESI, m/z): 182.05 [M+H]+.

**Step 3: Synthesis of 5-amino-4-(cyclopropylmethyl)-N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-1H-pyrazole-4-carboxamide (compound 292)**

Under nitrogen protection, 5-amino-1-(cyclopropylmethyl)-1H-pyrazole-4-carboxylic acid (compound **292-3,** 15.49 mg, 0.086 mmol, 1.3 eq), 2-(7-azabenzotriazole)-N,N,N',N'-tetramethyluronium hexafluorophosphate (25.0 mg, 0.066 mmol, 1 eq), and *N,N*-diisopropylethylamine (42.49 mg, 0.330 mmol, 5 eq) were added to a reaction flask, dissolved in *N,N-dimethylformamide* (2 mL, 25.843 mmol) and stirred at room temperature for 10 min. Then, 2-(3-aminoprop-1-yn-1-yl)-N-((3S,4R)-3-fluoro-1-methylpiperidin-4-yl)-3-(2,2,2-trifluoroethyl)pyrazolo[1,5-*a*]pyridin-7-amine dihydrochloride (compound **001-12,** 30 mg, 0.066 mmol, 1.00 eq) was added, and the system was stirred to react at room temperature for 1 h. After the reaction was completed, 20 mL of water was added and the mixture was extracted with ethyl acetate (20 mL × 2). The organic phases were combined, backwashed with saturated sodium chloride solution (30 mL × 1), dried over anhydrous sodium sulfate, filtered, and the filtrate was concentrated under reduced pressure. The crude product was purified by HPLC with the following conditions (column specifications: Sunfire C18 5 μm, 30 mm * 150 mm; mobile phase A: water (0.1% formic acid), mobile phase B: acetonitrile; flow rate: 60 mL/min; elution gradient: 7% B to 33% B in 7 min; detection wavelength: UV 254 nm/220 nm; retention time (min): 7.1), to give the carboxylate of compound **5-amino-4-(cyclopropylmethyl)-N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-1*H*-pyrazole-4-carboxamide 292** (15.92 mg, 44.17%).

LCMS: (ESI, m/z): 547.10 [M+H]+.

$^1$H NMR (400 MHz, DMSO-$d_6$, *ppm) δ* 8.26 (t, *J* = 5.6 Hz, 1H), 7.68 (s, 1H), 7.28 (t, *J* = 8.2 Hz, 1H), 7.02 (d, *J* = 8.7 Hz, 1H), 6.31 (d, *J* = 7.7 Hz, 1H), 6.26 - 6.21 (m, 3H), 4.87 (d, *J* = 49.4 Hz, 1H), 4.29 (d, *J* = 5.6 Hz, 2H), 3.92 - 3.82 (m, 1H), 3.81 - 3.68 (m, 4H), 3.05 (t, *J* = 10.9 Hz, 1H), 2.78 (d, *J* = 11.3 Hz, 1H), 2.30 (dd, *J* = 38.3, 13.0 Hz, 1H), 2.20 (s, 3H), 2.15 (t, *J* = 10.6 Hz, 1H), 1.99 - 1.79 (m, 2H), 1.22 - 1.10 (m, 1H), 0.47 - 0.40 (m, 2H), 0.35 - 0.28 (m, 2H).

**Example 52**

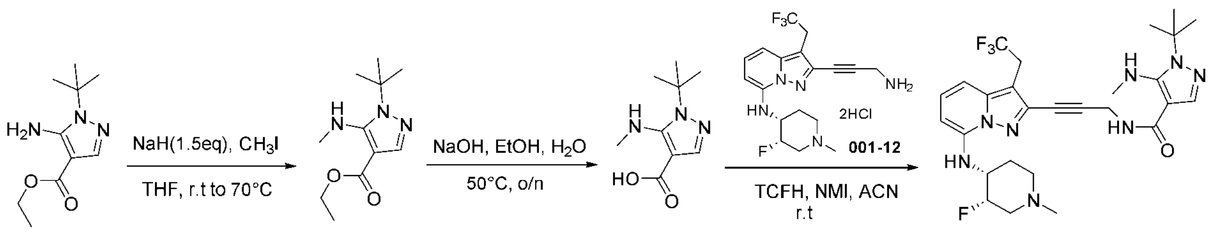


047-1 307-1 307-2 307

**Step** 1: **Synthesis of compound 307-1**

Under nitrogen protection, ethyl 5-amino-1-(tert-butyl)-1H-pyrazole-4-carboxylate (compound **047-1,** 300 mg, 1.420 mmol, 1 eq) was added to a reaction flask and dissolved in tetrahydrofuran (3 mL, 37.028 mmol). Sodium hydride (51.12 mg, 2.130 mmol, 1.5 eq) was added at 0°C and the mixture was stirred at room temperature for 30 min. Methyl iodide (0.11 mL, 1.704 mmol, 1.2 eq) was then added and the mixture was warmed to 70°C and stirred to react for 2 h. After the reaction was completed, 2 mL of water was added and the mixture was extracted with ethyl acetate (3 mL × 2). The organic phases were combined, dried over anhydrous sodium sulfate, filtered, and the filtrate was concentrated under reduced pressure to give crude compound **307-1,** which was directly used in the next step without further purification.

LCMS: (ESI, m/z): 226.00 [M+H]+.

**Step 2: Synthesis of compound 307-2**

Under nitrogen protection, a crude mixture of ethyl 1-(tert-butyl)-5-(methylamino)-1H-pyrazole-4-carboxylate (compound **307-1,** 300 mg, 1.332 mmol, 1 eq) and sodium hydroxide (106.52 mg, 2.664 mmol, 2 eq) was added to a reaction flask. The mixture was dissolved in anhydrous ethanol (2 mL, 34.427 mmol) and water (0.5 mL, 27.755 mmol), warmed to 50°C and stirred to react overnight. After the reaction was completed, the reaction mixture was concentrated

under reduced pressure to give a crude mixture of compound (220 mg, 53.02%) **307-2.**
LCMS: (ESI, m/z): 198.05 [M+H]+.

**Step 3: Synthesis of 1-(tert-butyl)-N-[3-(7-{[(3S,4R)-3-fluoro-1-methylpiperidin-4-yl] amino}-3-(2,2,2-trifluoroethyl)pyrazolo [1,5-a] pyridin-2-yl)prop-2-yn-1-yl]-5-(methylamino)-1H-pyrazole-4-carboxamide (compound 307)**

Under nitrogen protection, 1-(tert-butyl)-5-(methylamino)-1H-pyrazole-4-carboxylic acid (compound **307-2,** 64.84 mg, 0.329 mmol, 1.50 eq) and tetramethylchlorouronium hexafluorophosphate (245.96 mg, 0.876 mmol, 4 eq) were added to a reaction flask and dissolved in acetonitrile (5 mL). Then, 2-(3-aminoprop-1-yn-1-yl)-N-((3S,4R)-3-fluoro-1-methylpiperidin-4-yl)-3-(2,2,2-trifluoroethyl)pyrazolo[1,5-*a*]pyridin-7-amine dihydrochloride (compound **001-12,** 100 mg, 0.219 mmol, 1.00 eq) and N-methylimidazole (179.93 mg, 2.190 mmol, 10 eq) were added, and the system was stirred to react at room temperature for 4 h. After the reaction was completed, 10 mL of water was added and the mixture was extracted with dichloromethane (20 mL × 2). The organic phases were combined, backwashed with saturated sodium chloride solution (30 mL × 1), dried over anhydrous sodium sulfate, filtered, and the filtrate was concentrated under reduced pressure. The resulting residue was purified by silica gel column chromatography (dichloromethane/methanol = 10:1), to give a crude product of compound **307.** The crude product was purified by HPLC with the following conditions (column specifications: Sunfire C18 5 μm, 30 mm * 150 mm; mobile phase A: water (0.1% formic acid), mobile phase B: acetonitrile; flow rate: 60 mL/min; elution gradient: 17% B to 33% B in 8 min; detection wavelength: UV 254 nm/220 nm; retention time (min): 7.52), to give the carboxylate of compound **1-(tert-butyl)-N-[3-(7-{ [(3S,4R)-3-fluoro-1-methylpiperidin-4-yl] amino}-3-(2,2,2-trifluoroethyl)pyrazolo [1,5-*a*]pyridin-2-yl)prop-2-yn-1-yl]-5-(methylamino)-1*H*-pyrazole-4-carboxamide 307** (11.34 mg, 9.16%).
LCMS: (ESI, m/z): 563.10 [M+H]+.
$^1$H NMR (400 MHz, DMSO-$d_6$, *ppm*) δ 8.38 (t, *J*= 5.7 Hz, 1H), 7.68 (s, 1H), 7.28 (dd, *J* = 8.8, 7.6 Hz, 1H), 7.02 (d, J = 8.6 Hz, 1H), 6.32 (d, J = 7.3 Hz, 1H), 6.24 (d, *J* = 9.0 Hz, 1H), 4.98 (q, *J* = 5.5 Hz, 1H), 4.88 (d, *J* = 49.5 Hz, 1H), 4.31 (d, *J* = 5.6 Hz, 2H), 3.94 - 3.80 (m, 1H), 3.75 (q, *J* = 11.1 Hz, 2H), 3.07 (t, *J* = 11.5 Hz, 1H), 2.79 (d, *J* = 11.4 Hz, 1H), 2.74 (d, *J* = 5.6 Hz, 3H), 2.40 - 2.24 (m, 1H), 2.22 (s, 3H), 2.16 (t, J = 11.0 Hz, 1H), 1.99 - 1.81 (m, 2H), 1.57 (s, 9H).

**Example 53**

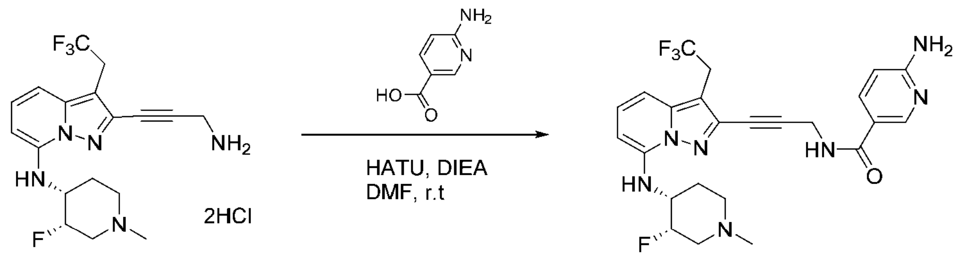


**001-12** **296**

**Step 1: Synthesis of 6-amino-N-[3-(7-{[(3S,4R)-3-fluoro-1-methylpiperidin-4-yl]amino}-3-(2,2,2-trifluoroethyl) pyrazolo[1,5-a]pyridin-2-yl)prop-2-yn-1-yl]nicotinamide (compound 296)**

At room temperature, a solution of 6-aminonicotinic acid (1.66 mg, 0.012 mmol, 1.1 eq), O-(7-azabenzotriazol-1-yl)-*N,N,N',N'*-tetramethyluronium hexafluorophosphate (4.58 mg, 0.012 mmol, 1.1 eq) and N,N-diisopropylethylamine (7.08 mg, 0.055 mmol, 5 eq) in *N,N*-dimethylformamide (1 mL) was stirred to react for 30 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 5 mg, 0.011 mmol, 1 eq) was added and the system was further stirred to react for 2 h. After the reaction was completed, the system was quenched with water and extracted with ethyl acetate (3 × 10 mL). The organic phases were combined, backwashed with saturated sodium chloride solution (1 × 10 mL), dried over anhydrous sodium sulfate, filtered, and the filtrate was concentrated under reduced pressure. The crude product was purified by HPLC with the following conditions (column specifications: Sunfire C18 5 μm, 30 mm * 150 mm; mobile phase A: water (0.1% formic acid), mobile phase B: acetonitrile; flow rate: 60 mL/min; elution gradient: 15% B to 35% B in 8 min; detection wavelength: UV 254 nm/220 nm; retention time (min): 7.22), to give the carboxylate of compound **6-amino-N-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]nicotina-**

**mide 296** (19.37 mg, 63.82%).

LCMS: (ESI, m/z): 504.00 [M+H]+.

1H NMR (400 MHz, DMSO-$d_6$, *ppm)* $\delta$ 8.75 (t, *J* = 5.6 Hz, 1H), 8.48 (d, *J* = 2.4 Hz, 1H), 7.84 (dd, *J* = 8.7, 2.5 Hz, 1H), 7.28 (t, *J* = 8.2 Hz, 1H), 7.03 (d, *J* = 8.7 Hz, 1H), 6.53 (s, 2H), 6.43 (d, *J* = 8.7 Hz, 1H), 6.32 *(d, J* = 7.6 Hz, 1H), 6.26 (d, *J* = 9.0 Hz, 1H), 4.89 (d, *J* = 49.4 Hz, 1H), 4.34 (d, *J* = 5.5 Hz, 2H), 3.94 - 3.82 (m, 1H), 3.76 (q, *J* = 11.1 Hz, 2H), 3.09 (t, *J* = 11.6 Hz, 1H), 2.81 (d, *J* = 11.5 Hz, 1H), 2.42 - 2.26 (m, 1H), 2.23 (s, 3H), 2.16 *(t, J* = 11.7 Hz, 1H), 2.01 - 1.90 (m, 1H), 1.88 - 1.80 (m, 1H).

**Example 54**

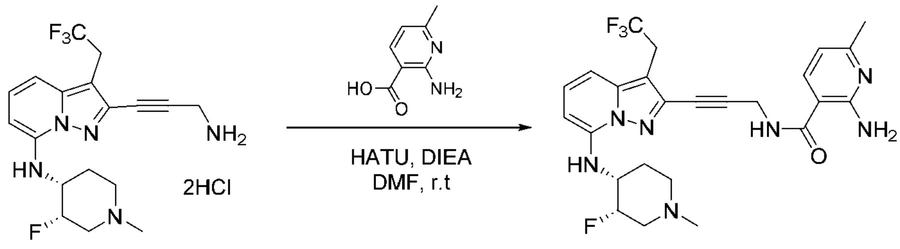


**001-12**

**295**

**Step** 1: **Synthesis of 2-amino-N-[3-(7-{[(3S,4R)-3-fluoro-1-methylpiperidin-4-yl]amino}-3-(2,2,2-trifluoroethyl) pyrazolo[1,5-a]pyridin-2-yl)prop-2-yn-1-yl]-6-methylnicotinamide (compound 295)**

At room temperature, 2-amino-6-methylnicotinic acid (6.67 mg, 0.044 mmol, 1 eq), O-(7-azabenzotriazol-1-yl)-*N,N,N',N'*-tetramethyluronium hexafluorophosphate (16.67 mg, 0.044 mmol, 1 eq) and *N,N*-diisopropylethylamine (28.32 mg, 0.220 mmol, 5 eq) were added to a reaction flask, dissolved in *N,N-dimethylformamide* (1 mL), and stirred to react for 30 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.044 mmol, 1 eq) was added and the system was stirred to react for 2 h. After the reaction was completed, the reaction mixture was quenched with water at room temperature and extracted with ethyl acetate (3 × 10 mL). The organic phases were combined, backwashed with saturated sodium chloride solution (1 × 10 mL), dried over anhydrous sodium sulfate, filtered, and the filtrate was concentrated under reduced pressure. The crude product was purified by HPLC with the following conditions (column specifications: Sunfire C18 5 μm, 30 mm * 150 mm; mobile phase A: water (0.1% formic acid), mobile phase B: acetonitrile; flow rate: 60 mL/min; elution gradient: 15% B to 35% B in 8 min; detection wavelength: UV 254 nm/220 nm; retention time (min): 7.22), to give the carboxylate of compound **2-amino-*N*-[3-(7-{[(3S,4R)-3-fluoro-1-methylpiperidin-4-yl]amino}-3-(2,2,2-trifluoroethyl)pyrazolo[1,5-*a*]pyridin-2-yl)prop-2-yn-1-yl]-6-methylnicotinamide 295** (18.89 mg, 83.03%).

LCMS: (ESI, m/z): 518.10 [M+H]+.

1H NMR (400 MHz, DMSO-d6, ppm) $\delta$ 8.91 (t, J = 5.5 Hz, 1H), 7.85 (d, J = 7.9 Hz, 1H), 7.29 (dd, J = 8.7, 7.6 Hz, 1H), 7.13 (s, 2H), 7.03 (d, J = 8.6 Hz, 1H), 6.47 (d, J = 7.9 Hz, 1H), 6.32 (d, J = 7.6 Hz, 1H), 6.26 (d, J = 9.0 Hz, 1H), 4.89 (d, J = 49.4 Hz, 1H), 4.33 (d, J = 5.4 Hz, 2H), 3.94 - 3.81 (m, 1H), 3.76 (q, J = 11.4 Hz, 2H), 3.09 (t, *J* = 11.6 Hz, 1H), 2.81 (d, J = 11.1 Hz, 1H), 2.42 - 2.34 (m, 1H), 2.28 (s,3H), 2.23 (s, 3H), 2.20 - 2.12 (m, 1H), 2.01 - 1.88 (m, 1H), 1.87 - 1.80 (m, 1H).

**Example 55**

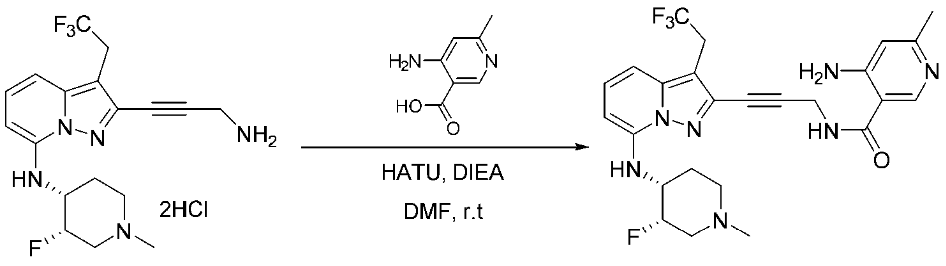


001-12 294

**Step 1: Synthesis of 4-amino-N-[3-(7-{[(3S,4R)-3-fluoro-1-methylpiperidin-4-yl]amino}-3-(2,2,2-trifluoroethyl) pyrazolo[1,5-a]pyridin-2-yl)prop-2-yn-1-yl]-6-methylnicotinamide (compound 294)**

Under nitrogen protection, 4-amino-6-methylnicotinic acid (12.0 mg, 0.079 mmol, 1.2 eq), 2-(7-azabenzotriazole)-*N,N,N',N'*-tetramethyluronium hexafluorophosphate (25.0 mg, 0.066 mmol, 1 eq), and *N,N*-diisopropylethylamine (42.49 mg, 0.330 mmol, 5 eq) were added to a reaction flask, dissolved in *N,N*-dimethylformamide (2 mL, 25.843 mmol) and stirred at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 30 mg, 0.066 mmol, 1 eq) was added, and the system was stirred to react for 1 h. After the reaction was completed, the reaction mixture was extracted with ethyl acetate (20 mL × 2). The organic phases were combined, backwashed with saturated sodium chloride solution (30 mL × 1), dried over anhydrous sodium sulfate, filtered, and the filtrate was concentrated under reduced pressure. The crude product was purified by HPLC with the following conditions (column specifications: Xselect CSHTM Prep C18 5 μm 30*150mm OBD; mobile phase A: water (0.1% formic acid), mobile phase B: acetonitrile; flow rate: 100 mL/min; elution gradient: to 26% B in 7 min; detection wavelength: UV 254 nm/220 nm; retention time (min): 6.27), to give the carboxylate of compound **4-amino-*N*-[3-(7-{ [(3*S*,4R)-3-fluoro-1-methylpiperidin-4-yl] amino}-3-(2,2,2-trifluoroethyl)pyrazolo[1,5-*a*]pyridin-2-yl)prop-2-yn-1-yl]-6-methylnicotinamide 294** (20.3 mg, 59.36%).
LCMS: (ESI, m/z): 518.05 $[M+H]^+$.
$^1H$ NMR (400 MHz, DMSO-$d_6$, *ppm)* δ 8.95 (t, *J* = 5.5 Hz, 1H), 8.46 (s, 1H), 7.29 (t, *J* = 8.2 Hz, 1H), 7.18 (s, 2H), 7.03 (d, *J* = 8.7 Hz, 1H), 6.48 (s, 1H), 6.32 (d, J = 7.6 Hz, 1H), 6.24 (d, J = 9.0 Hz, 1H), 4.88 (d, *J* = 49.2 Hz, 1H), 4.33 (d, *J* = 5.4 Hz, 2H), 3.94 - 3.83 (m, 1H), 3.78 (q, J = 11.4 Hz, 2H), 3.07 (t, *J* = 11.0 Hz, 1H), 2.79 (d, *J* = 11.3 Hz, 1H), 2.35 (d, J = 13.2 Hz, 1H), 2.26 (s, 3H), 2.21 (s, 3H), 2.15 (t, J = 10.6 Hz, 1H), 1.97 - 1.81 (m, 2H).

**Example 56**

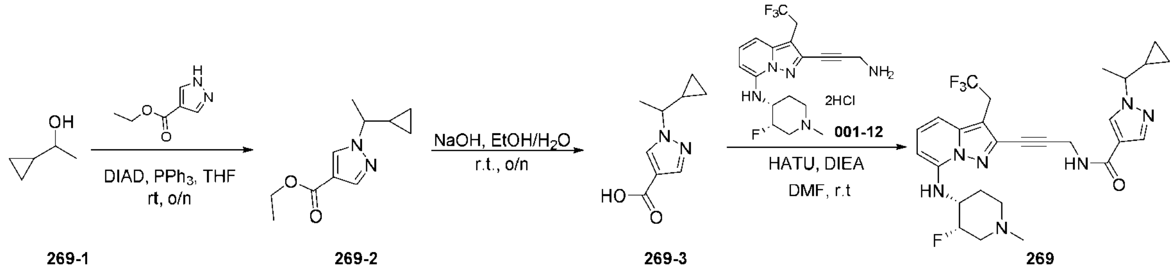


269-1 269-2 269-3 269

**Step 1: Synthesis of compound 269-2**

Under nitrogen protection, at 0°C, to a solution of 1-cyclopropylethanol (compound **269-1,** 614.62 mg, 7.136 mmol, 2.00 eq), ethyl *1H*-pyrazole-4-carboxylate (500 mg, 3.568 mmol, 1.00 eq) and triphenylphosphine (1.87 g, 7.136 mmol, 2.00 eq) in tetrahydrofuran (40 mL) was added dropwise diisopropyl azodicarboxylate (1.44 g, 7.136 mmol, 2.00 eq). After the addition was completed, the mixture was stirred at room temperature overnight. The resulting residue was concentrated under reduced pressure and then diethyl ether was added. The mixture was cooled to 0°C, filtered, and the filtrate was spin dried. The resulting residue was purified by silica gel column chromatography (petroleum ether/ethyl acetate = 5:1) to give compound **269-2** (700 mg, 94.21%).
LCMS: (ESI, m/z): 208.90 $[M+H]^+$.

**Step 2: Synthesis of compound 269-3**

At room temperature, a solution of ethyl (1-cyclopropylethyl)-1*H*-pyrazole-4-carboxylate (compound **269-2,** 160 mg, 0.768 mmol, 1 eq) and sodium hydroxide (5M) (0.312 mL, 1.536 mmol, 2 eq) in ethanol (3.2 mL) was stirred to react overnight. The resulting residue was concentrated under reduced pressure, acidified to a pH of about 5 with hydrochloric acid solution (2M), and the resulting residue was concentrated under reduced pressure. The mixture was washed with tetrahydrofuran/methanol (3/1 mL), filtered, and the filter cake was washed with tetrahydrofuran (1 × 4 mL). The filtrate was concentrated under reduced pressure to give compound **269-3** (100 mg, 72.23%).

LCMS: (ESI, m/z): 181.05 $[M+H]^+$.

**Step 3: Synthesis of 1-(1-cyclopropylethyl)-N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-1*H*-pyrazole-4-carboxamide (compound 269)**

At room temperature, a solution of 1-(1-cyclopropylethyl)-1*H*-pyrazole-4-carboxylic acid (compound **269-3**, 37.91 mg, 0.210 mmol, 1.2 eq), *N,N*-diisopropylethylamine (90.64 mg, 0.700 mmol, 4 eq) and O-(7-azabenzotriazol-1-yl)-*N,N,N',N'*-tetramethyluronium hexafluorophosphate (80.0 mg, 0.210 mmol, 1.2 eq) *in N,N-dimethylformamide* (4.0 mL) was stirred to react for 20 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 80 mg, 0.175 mmol, 1 eq) was added, and the system was further stirred at room temperature for 1 h. After the reaction was completed, the reaction mixture was quenched with water (20 mL), and extracted with ethyl acetate (2 × 20 mL). The organic phases were combined, backwashed with saturated sodium chloride solution (2 × 20 mL), dried over anhydrous sodium sulfate, filtered, and the filtrate was concentrated under reduced pressure. The crude product was purified by HPLC with the following conditions (column specifications: Xselect CSHTM Prep C18 5 μm 30x150mm OBD; mobile phase A: water (0.1% formic acid), mobile phase B: acetonitrile; flow rate: 60 mL/min; elution gradient: 16% B to 32% B in 7 min; detection wavelength: UV 254 nm/225 nm; retention time (min): 6.42), to give the carboxylate of compound **1-(1-cyclopropylethyl)-N-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1*H*-pyrazole-4-carboxamide 269** (2.07 mg, 1.98%).

LCMS: (ESI, m/z): 545.95 $[M+H]^+$.

$^1$H NMR (400 MHz, DMSO-$d_6$, *ppm)* δ 8.67 (t, *J* = 5.6 Hz, 1H), 8.30 (s, 1H), 7.90 (s, 1H), 7.29 (dd, *J* = 8.7, 7.7 Hz, 1H), 7.03 (d, *J* = 8.7 Hz, 1H), 6.32 (d, *J* = 7.6 Hz, 1H), 6.24 (d, *J* = 9.1 Hz, 1H), 4.87 (d, *J* = 48.0 Hz, 1H), 4.34 (d, *J* = 5.6 Hz, 2H), 3.92 - 3.82 (m, 1H), 3.81 - 3.63 (m, 3H), 3.05 (t, J = 10.6 Hz, 1H), 2.78 (d, J = 11.7 Hz, 1H), 2.37 - 2.23 (m, 1H), 2.20 (s, 3H), 2.13 (d, J = 9.5 Hz, 1H), 2.00 - 1.78 (m, 2H), 1.50 (d, *J* = 6.8 Hz, 3H), 1.28 - 1.15 (m, 1H), 0.65 - 0.55 (m, 1H), 0.50 - 0.40 (m, 1H), 0.40 - 0.26 (m, 2H).

**Example 57**

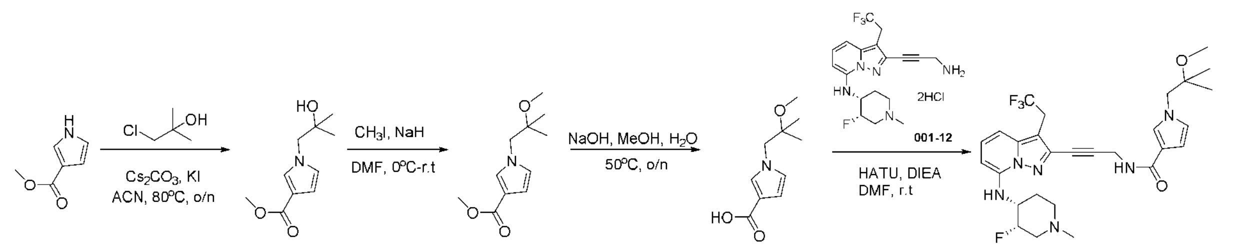


014-1 300-1 300-2 300-3 300

**Step** 1: **Synthesis of compound 300-1**

Under nitrogen protection at room temperature, to a solution of methyl 1*H*-pyrrole-3-carboxylate (compound **014-1,** 500 mg, 3.996 mmol, 1 eq) and 1-chloro-2-methyl-2-propanol (650.76 mg, 5.994 mmol, 1.5 eq) in acetonitrile (10.0 mL) were added cesium carbonate (3.91 g, 11.988 mmol, 3 eq) and potassium iodide (331.67 mg, 1.998 mmol, 0.5 eq) in portions. The mixture was stirred at 80°C overnight. After the reaction was completed, the mixture was filtered, and the filter cake was washed with acetonitrile (3 × 10 mL). The filtrate was concentrated under reduced pressure. The resulting residue was purified by silica gel column chromatography (petroleum ether/ethyl acetate = 3:1) to give compound **300-1** (320 mg, 40.60%).

LCMS: (ESI, m/z): 197.95 $[M+H]^+$.

**Step 2: Synthesis of compound 300-2**

Under nitrogen protection and at 0°C, to a solution of methyl 1-(2-hydroxy-2-methylpropyl)-1*H*-pyrrole-3-carboxylate (compound **300-1,** 270 mg, 1.369 mmol, 1 eq) in *N,N*-dimethylformamide (4.0 mL) was added sodium hydride (65.7 mg, 2.738 mmol, 2 eq), and the mixture was stirred to react for 30 min. Iodomethane (388.61 mg, 2.738 mmol, 2 eq) was added dropwise at 0°C. The mixture was allowed to recover to room temperature naturally, and further stirred for 1 h. After the reaction was completed, water (20 mL) was added to the reaction mixture for quenching. The mixture was extracted with ethyl acetate (3 × 20 mL). The organic phases were combined, backwashed with saturated brine (3 × 15 mL), dried over anhydrous sodium sulfate, and filtered. The filtrate was concentrated under reduced pressure. The resulting residue was purified by silica gel column chromatography (petroleum ether/ethyl acetate = 3:1) to give compound 300-**2** (250 mg, 86.44%).

LCMS: (ESI, m/z): 212.00 $[M+H]^+$.

**Step 3: Synthesis of compound 300-3**

Under nitrogen protection, at room temperature, to a solution of methyl 1-(2-methoxy-2-methylpropyl)-1H-pyrrole-3-carboxylate (compound **300-2,** 100 mg, 0.473 mmol, 1 eq) in methanol (1.0 mL), a solution of sodium hydroxide (37.87 mg, 0.946 mmol, 2 eq) in water (0.5 mL) was dropwise added. The mixture was stirred at 50°C overnight. After the reaction was completed, the reaction mixture was acidified to pH=5 with hydrochloric acid solution. The resulting residue was concentrated under reduced pressure, then dissolved in tetrahydrofuran (3 mL). The resulting residue was filtered through a filter membrane and concentrated under reduced pressure again to give compound **300-3** (60 mg, 64.27%). The crude product was not further purified and was directly used in the next step.

LCMS: (ESI, m/z): 198.00 $[M+H]^+$.

**Step 4: Synthesis of N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-1-(2-methoxy-2-methylpropyl)-1H-pyrrole-3-carboxamide (compound 300)**

Under nitrogen protection at room temperature, to a solution of 2-(3-aminoprop-1-yn-1-yl)-N-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 30 mg, 0.066 mmol, 1 eq) and 1-(2-methoxy-2-methylpropyl)-1*H*-pyrrole-3-carboxylic acid (compound **300-3,** 16.86 mg, 0.086 mmol, 1.3 eq) in *N,N-dimethylformamide* (0.8 mL) was added *N,N*-diisopropylethylamine (42.49 mg, 0.330 mmol, 5 eq) dropwise, and the mixture was stirred to react for 1 h. At room temperature, O-(7-azabenzotriazol-1-yl)-*N,N,N',N'*-tetramethyluronium hexafluorophosphate (27.5 mg, 0.073 mmol, 1.1 eq) was added. After the addition was completed, the system was further stirred at room temperature for 1 h. After the reaction was completed, methanol (1.0 mL) was added to the reaction mixture at room temperature for quenching. The crude product was purified by HPLC with the following conditions (column specifications: XBridge BEH C18 OBD Prep Column 130, 5 μm, 30 mm × 150 mm; mobile phase A: water (17 mmol/L ammonium bicarbonate), mobile phase B: acetonitrile; flow rate: 60 mL/min; elution gradient: 38% B to 56% B in 8 min; detection wavelength: UV 254 nm/220 nm; retention time (min): 6.6) to give compound **N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-1-(2-methoxy-2-methylpropyl)-1H-pyrrole-3-carboxamide 300** (5.81 mg, **15.64%).**

LCMS: (ESI, m/z): 563.15 $[M+H]^+$.

$^1$H NMR (400 MHz, DMSO-$d_6$, *ppm)* $\delta$ 8.35 (t, *J* = 5.7 Hz, 1H), 7.32 - 7.24 (m, 2H), 7.02 (d, *J* = 8.7 Hz, 1H), 6.69 (t, *J* = 2.5 Hz, 1H), 6.46 (dd, *J* = 2.9, 1.7 Hz, 1H), 6.31 (d, *J* = 7.6 Hz, 1H), 6.26 (*d, J* = 9.0 Hz, 1H), 4.87 (d, *J* = 49.3 Hz, 1H), 4.29 (d, *J* = 5.7 Hz, 2H), 3.89 (s, 2H), 3.87 - 3.68 (m, 3H), 3.16 (s, 3H), 3.05 (t, *J* = 11.4 Hz, 1H), 2.78 (d, *J* = 11.5 Hz, 1H), 2.36 - 2.24 (m, 1H), 2.20 (s, 3H), 2.13 (d, *J* = 10.3 Hz, 1H), 2.00 - 1.88 (m, 1H), 1.87 - 1.79 (m, 1H), 1.03 (s, 6H).

**Example 58**

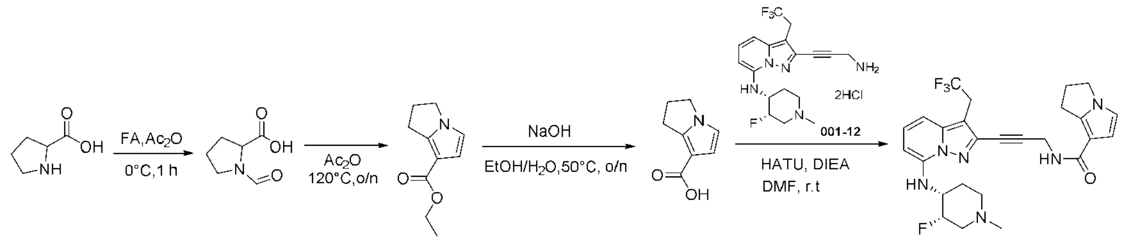


302-1 302-2 302-3 302-4 302

**Step** 1: **Synthesis of compound 302-2**

Under nitrogen protection, proline (compound **302-1,** 1 g, 8.686 mmol, 1 eq) was added to a reaction flask, dissolved in formic acid (8.5 mL), and acetic anhydride (5.75 mL, 60.802 mmol, 7 eq) was added at 0°C. The mixture was stirred to react at room temperature for 1 h. After the reaction was completed, the reaction mixture was quenched with ice water at 0°C, and the resulting residue was concentrated under reduced pressure to give compound **302-2** (1.2 g, 88.60%).
LCMS: (ESI, m/z): 144.00 $[M+H]^+$.

**Step 2: Synthesis of compound 302-3**

Under nitrogen protection, formylproline (compound **302-2,** 500 mg, 3.493 mmol, 1 eq) and ethyl acrylate (2.40 g, 24.451 mmol, 7 eq) were added to a reaction flask. The mixture was dissolved in acetic anhydride (5 mL, 52.895 mmol), warmed to 120°C and stirred to react overnight. After the reaction was completed, the reaction mixture was quenched at room temperature with water, and then extracted with ethyl acetate (50 mL × 2). The organic phases were combined, backwashed with saturated sodium chloride solution (50 mL × 1), dried over anhydrous sodium sulfate, and filtered. The resulting residue was purified by silica gel column chromatography (petroleum ether/ethyl acetate = 8:1) to give compound 302-3 (200 mg, 23.96%).
LCMS: (ESI, m/z): 180.05 $[M+H]^+$.

**Step 3: Synthesis of compound 302-4**

Under nitrogen protection, ethyl 2,3-dihydro-1*H*-pyrrozine-7-carboxylate (compound **302-3,** 200 mg, 1.116 mmol, 1 eq) and sodium hydroxide (89.27 mg, 2.232 mmol, 2 eq) were added to a reaction flask, dissolved in anhydrous ethanol (5 mL, 86.067 mmol) and water (2 mL, 111.019 mmol), and the mixture was warmed to 50°C and stirred to react overnight. After the reaction was completed, the reaction mixture was concentrated under reduced pressure. To the crude product were added 20 mL of ethyl acetate and 20 mL of water, and the product was in the aqueous phase. The aqueous phase was adjusted to pH 3 with 1 mol/L hydrochloric acid solution, and then extracted with ethyl acetate (20 mL × 2). The organic phases were combined, dried over anhydrous sodium sulfate, filtered, and the filtrate was concentrated under reduced pressure to give compound **302-4.**
LCMS: (ESI, m/z): 152.05 $[M+H]^+$.

**Step 4: Synthesis of N-[3-(7-{[(3S,4R)-3-fluoro-1-methylpiperidin-4-yl]amino}-3-(2,2,2-trifluoroethyl)pyrazolo [1,5-a]pyridin-2-yl)prop-2-yn-1-yl]-2,3-dihydro-1H-pyrrolizine-7-carboxamide (compound 302)**

**Under** nitrogen protection, 2,3-dihydro-1*H*-pyrrozine-7-carboxylic acid (compound **302-4**, 12.92 mg, 0.086 mmol, 1.3 eq), 2-(7-azabenzotriazole)-*N,N,N',N'*-tetramethyluronium hexafluorophosphate (25.0 mg, 0.066 mmol, 1 eq), and *N,N*-diisopropylethylamine (42.49 mg, 0.330 mmol, 5 eq) were added to a reaction flask, dissolved in *N,N-dimethylformamide* (2 mL, 25.843 mmol) and stirred at room temperature for 10 min. Then, 2-(3-aminoprop-1-yn-1-yl)-N-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 30 mg, 0.066 mmol, 1.00 eq) was added, and the system was stirred to react at room temperature for 1 h. After the reaction was completed, the reaction mixture was extracted with ethyl acetate (20 mL × 2). The organic phases were combined, backwashed with saturated sodium chloride solution (30 mL × 1), dried over anhydrous sodium sulfate, filtered, and the filtrate was concentrated under reduced pressure. The crude product was purified by HPLC with the following conditions (column specifications: Xselect CSHTM Prep C18 5μm 30*150mm OBD; mobile phase A: water (0.1% formic acid), mobile phase B: acetonitrile; flow rate: 60 mL/min; elution gradient: 16% B to 33% B in 8 min; detection wavelength: UV 254 nm/220 nm; retention time (min): 6.28), to give a crude product of compound 302. The mixture was subjected to chiral resolution with the following conditions (column specifications: CHIRALPAK-IA 2*25cm, 5 μm; mobile phase A: n-hexane (0.1% diethylamine), mobile phase B: ethanol: DCM = 1:1; flow rate: 20 mL/min; elution gradient:

isocratic 45; detection wavelength: UV 254/220 nm; retention time (min): 6.097; rear peak retention time (min): 8.463; sample dissolving solvent: methanol; single injection volume: 2.0 mL; number of injections: 1), to give compound **N-[3-(7-{[(3*S*,4*R*)-3-fluoro-1-methylpiperidin-4-yl]amino}-3-(2,2,2-trifluoroethyl)pyrazolo[1,5-*a*]pyridin-2-yl) prop-2-yn-1-yl]-2,3-dihydro-1*H*-pyrrolizine-7-carboxamide** 302 (4.7 mg, 13.76%).

LCMS: (ESI, m/z): 517.15 $[M+H]^+$.

$^1$H NMR (400 MHz, DMSO-$d_6$, *ppm)* $\delta$ 8.10 (s, 1H), 7.28 *(t, J* = 8.2 Hz, 1H), 7.02 (d, *J* = 8.7 Hz, 1H), 6.66 (d, *J* = 2.9 Hz, 1H), 6.56 (s, 1H), 6.31 (d, J = 7.4 Hz, 1H), 6.26 (d, J = 9.1 Hz, 1H), 4.87 *(d, J* = 49.5 Hz, 1H), 4.28 (d, *J* = 5.6 Hz, 2H), 3.91 (t, *J* = 7.2 Hz, 3H), 3.86 - 3.69 (m, 3H), 3.05 (t, *J* = 11.6 Hz, 1H), 2.94 (t, *J* = 7.3 Hz, 2H), 2.78 (d, *J* = 11.1 Hz, 1H), 2.46 - 2.40 (m, 1H), 2.36 - 2.25 (m, 1H), 2.20 (s, 3H), 2.12 (t, *J* = 11.2 Hz, 1H), 1.97 - 1.80 (m, 2H).

### Example 59

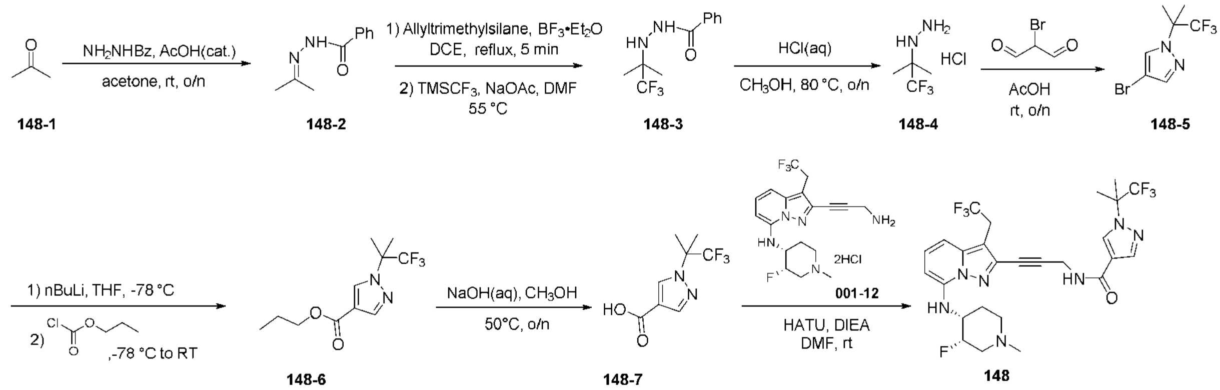


148-1 148-2 148-3 148-4 148-5 148-6 148-7 148

### Step 1: Synthesis of compound 148-2

Benzoylhydrazine (5 g, 36.723 mmol, 1 eq), and acetic acid (44.11 mg, 0.734 mmol, 0.02 eq) were added to a reaction flask, dissolved in acetone (22 mL) and allowed to react at room temperature overnight. After the reaction was completed, the reaction mixture was extracted with ethyl acetate (3 × 50 mL). The organic phases were combined, backwashed with saturated sodium chloride solution (1 × 50 mL), dried over anhydrous sodium sulfate, filtered, and concentrated under reduced pressure to directly give compound **148-2** (5.1 g, 74.87%).

LCMS: (ESI, m/z): 176.70 $[M+H]^+$.

### Step 2: Synthesis of compound 148-3

N-(prop-2-ylidene)benzoyl hydrazide (compound **148-2,** 1 g, 5.675 mmol, 1 eq) was added to a reaction flask, and dissolved in 1,2-dichloroethane (10 mL). Allyltrimethylsilane (972.6 mg, 8.512 mmol, 1.5 eq) and boron trifluoride diethyl etherate (1.21 g, 8.512 mmol, 1.5 eq) were added, and the reaction was carried out at 85°C for 5 min. The solvent was removed by spin drying, and the residue was dissolved in *N,N-dimethylformamide* (10 mL). (Trifluoromethyl) trimethylsilane (1.61 g, 11.350 mmol, 2 eq) and sodium acetate (1.86 g, 22.700 mmol, 4 eq) were added. The mixture was allowed to react at 55°C for 3 h. After the reaction was completed, the reaction mixture was cooled to room temperature, and saturated sodium carbonate was added to quench the reaction. The mixture was stirred for 5 min, diluted with water, and extracted with ethyl acetate (3 × 20 mL). The organic phases were combined, backwashed with saturated sodium chloride solution (1 × 20 mL), dried over anhydrous sodium sulfate, filtered, and the filtrate was concentrated under reduced pressure. The resulting residue was purified by silica gel column chromatography (petroleum ether/ethyl acetate = 1:1) to give compound **148-3** (1.1 g, 74.79%).

LCMS: (ESI, m/z): 246.85 $[M+H]^+$.

### Step 3: Synthesis of compound 148-4

N-(1,1,1-trifluoro-2-methylpropan-2-yl)benzoyl hydrazine (compound **148-3,** 1.1 g, 4.467 mmol, 1 eq) was added to a reaction flask, dissolved in methanol (6.6 mL), and then hydrochloric acid (6 mL) was added. The reaction was allowed to proceed at 80°C overnight. After the reaction was completed, the reaction mixture was cooled to room temperature, the solvent was removed by spin drying, and diethyl ether was added to precipitate the product. The product

was filtered and the filter cake was washed with diethyl ether to give the hydrochloride of compound **148-4** (570 mg, 85.29%).

$^1$H NMR (400 MHz, DMSO-$d_6$, *ppm)* $\delta$ 9.44 (s, 2H), 6.02 (s, 1H), 1.33 (s, 6H).

**Step 4: Synthesis of compound 148-5**

To a reaction flask, (1,1,1-trifluoro-2-methylprop-2-yl)hydrazine hydrochloride (compound 148-4, 2.2 g, 15.479 mmol, 1 eq), and 2-bromomalonaldehyde (2.80 g, 18.575 mmol, 1.2 eq) were added, dissolved in acetic acid (33 mL), and stirred at room temperature overnight. After the reaction was completed, 1 mol/L sodium hydroxide aqueous solution was added to adjust the pH to 7. The reaction mixture was extracted with ethyl acetate (3 × 50 mL), the organic phases were combined, backwashed with saturated sodium chloride solution (1 × 50 mL), dried over anhydrous sodium sulfate, filtered, and the filtrate was concentrated under reduced pressure. The residue was purified by reverse phase column chromatography with the following conditions (C18 column 120 g, mobile phase: water and acetonitrile, 10% to 80% gradient in 10 min, UV220 nm detection) to give compound **148-5** (200 mg, 4.27%).

LCMS: (ESI, m/z): 256.85 $[M+H]^+$.

**Step 5: Synthesis of compound 148-6**

Under nitrogen protection, 4-bromo-1-(1,1,1-trifluoro-2-methylprop-2-yl)-1*H*-pyrazole (compound **148-5,** 120 mg, 0.467 mmol, 1 eq) was added to a reaction flask, dissolved in tetrahydrofuran (5 mL), and a solution of 2.5 mol/L n-butyl lithium in hexane (0.373 mL, 0.934 mmol, 2 eq) was added dropwise at -78°C. The reaction was carried out at this temperature for 30 min. Then, propyl chlorocarbonate (62.93 mg, 0.514 mmol, 1.1 eq) was added, and the reaction was continued for 30 min. The mixture was then warmed to room temperature to further react for 72 h. After the reaction was completed, saturated ammonium chloride solution was added for quenching and the mixture was extracted with ethyl acetate (3 × 10 mL). The organic phases were combined, backwashed with saturated sodium chloride solution (1 × 10 mL), dried over anhydrous sodium sulfate, and filtered. The filtrate was concentrated under reduced pressure. The resulting residue was purified by reverse phase column chromatography with the following conditions (column specification: C18; mobile phase: water and acetonitrile, 10% to 90% gradient in 10 min, UV 220 nm), to give compound **148-6** (25 mg, 19.25%).

LCMS: (ESI, m/z): 265.00 $[M+H]^+$.

**Step 6: Synthesis of compound 148-7**

Propyl 1-(1,1,1-trifluoro-2-methylprop-2-yl)-1*H*-pyrazole-4-carboxylate (compound **148-6,** 25 mg, 0.094 mmol, 1 eq) and 1 mol/L sodium hydroxide aqueous solution (0.188 mL, 0.188 mmol, 2 eq) were added to a reaction flask, dissolved in methanol (1 mL), and allowed to react at 50°C overnight. After the reaction was completed, the reaction mixture was acidified to pH=3 with 1 mol/L hydrochloric acid solution, and the solvent was removed by spin drying to give compound **148-7** (20 mg, 95.15%).

LCMS: (ESI, m/z): 222.95 $[M+H]^+$.

**Step 7: Synthesis of N-[3-(7-{[(3S,4R)-3-fluoro-1-methylpiperidin-4-yl]amino}-3-(2,2,2-trifluoroethyl)pyrazolo [1,5-a]pyridin-2-yl)prop-2-yn-1-yl]-1-(1,1,1-trifluoro-2-methylprop-2-yl)-1*H*-pyrazole-4-carboxamide (compound 148)**

To a reaction flask, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 35 mg, 0.091 mmol, 1 eq), 1-(1,1,1-trifluoro-2-methylprop-2-yl)-1*H*-pyrazole-4-carboxylic acid (compound **148-7,** 20.28 mg, 0.091 mmol, 1 eq), *O*-(7-azabenzotriazol-1-yl)-*N,N,N',N'*-tetramethyluronium hexafluorophosphate (41.65 mg, 0.109 mmol, 1.2 eq), and *N,N*-diisopropylethylamine (58.99 mg, 0.455 mmol, 5 eq) were added, dissolved in *N,N*-dimethylformamide (1 mL) and allowed to react at room temperature for 2 h. After the reaction was completed, water was added and the reaction mixture was extracted with ethyl acetate (3 × 10 mL). The organic phases were combined, backwashed with saturated sodium chloride solution (1 × 10 mL), dried over anhydrous sodium sulfate, filtered, and the filtrate was concentrated under reduced pressure. The crude product was purified by HPLC with the following conditions (column specifications: Xselect CSHTM Prep C18 5μm 30*150mm OBD; mobile phase A: water (0.1% formic acid), mobile phase B: acetonitrile; flow rate: 60 mL/min; elution gradient: 18% B to 36% B in 7 min; detection wavelength: UV 254 nm/220 nm; retention time (min): 6.05), to give the carboxylate of compound ***N*-[3-(7-{[(3*S*,4*R*)-3-fluoro-1-methylpiperidin-4-yl]amino}-3-(2,2,2-trifluoroethyl)pyrazolo [1,5-*a*]pyridin-2-yl)prop-2-yn-1-yl]-1-(1,1,1-trifluoro-2-methylprop-2-yl)-1H-pyrazole-4-carboxamide 148** (4.51 mg, 8.34%).

LCMS: (ESI, m/z): 588.10 $[M+H]^+$.

$^1H$ NMR (400 MHz, DMSO-$d_6$, *ppm)* $\delta$ 8.77 (t, *J* = 5.6 Hz, 1H), 8.60 (s, 1H), 8.02 (s, 1H), 7.29 (dd, *J* = 8.7, 7.6 Hz, 1H), 7.03 (d, *J* = 8.7 Hz, 1H), 6.32 (d, *J* = 7.6 Hz, 1H), 6.23 (d, *J* = 9.0 Hz, 1H), 4.87 (d, *J* = 49.5 Hz, 1H), 4.36 (d, *J* = 5.5 Hz, 2H), 3.90 - 3.82 (m, 1H), 3.76 (q, J = 11.4 Hz, 2H), 3.05 (t, *J* = 11.6 Hz, 1H), 2.78 (d, *J* = 11.5 Hz, 1H), 2.36 - 2.21 (m, 1H), 2.20 (s, 3H), 2.17 - 2.09 (m, 1H), 1.99 - 1.86(m, 2H), 1.84 (s, 6H).

**Example 60**

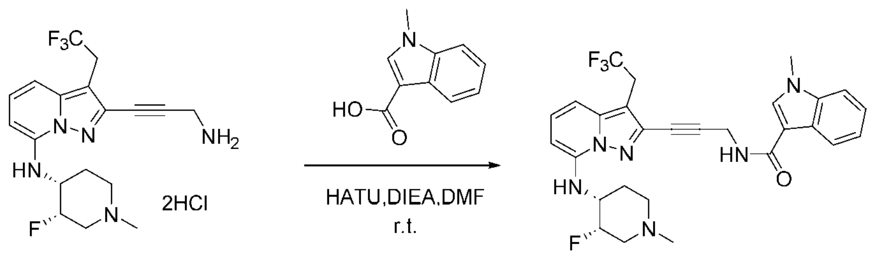


**001-12** **308**

**Step 1: Synthesis of N-[3-(7-{[(3S,4R)-3-fluoro-1-methylpiperidin-4-yl]amino}-3-(2,2,2-trifluoroethyl)pyrazolo [1,5-a]pyridin-2-yl)prop-2-yn-1-yl]-1-methylindole-3-carboxamide (compound 308)**

At room temperature, 1-methylindole-3-carboxylic acid (13.82 mg, 0.079 mmol, 1.2 eq), *N,N*-diisopropylethylamine (33.99 mg, 0.264 mmol, 4 eq), and *O*-(7-azabenzotriazol-1-yl)-*N,N,N',N'*-tetramethyluronium hexafluorophosphate (30 mg, 0.066 mmol, 1 eq) were added to a reaction flask, dissolved in *N,N*-dimethylformamide (2 mL) and stirred at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12**, 30 mg, 0.066 mmol, 1 eq) was added and the mixture was stirred for 1 h. After the reaction was completed, the reaction mixture was diluted with ethyl acetate (5 mL), washed with saturated sodium chloride solution (10 mL × 3), dried over anhydrous sodium sulfate, filtered, and the filtrate was concentrated under reduced pressure. The crude product was purified by HPLC with the following conditions (column specifications: Sunfire C18 5 μm, 30 mm * 150 mm; mobile phase A: water (0.1% formic acid), mobile phase B: acetonitrile; flow rate: 60 mL/min; elution gradient: 19% B to 33% B in 1 min; detection wavelength: UV 254 nm/220 nm), to give compound N-[3-(7-**{[(3S,4R)-3-fluoro-1-methylpiperidin-4-yl]amino}-3-(2,2,2-trifluoroethyl)pyrazolo[1,5-*a*]pyridin-2-yl)prop-2-yn-1-yl]-1-methylindole-3-carboxamide** 308 (13.82 mg, 38.65%).

LCMS: (ESI, m/z): 541.20 $[M+H]^+$.

$^1H$ NMR (400 MHz, DMSO-$d_6$, *ppm)* $\delta$ 8.46 *(t, J* = 5.6 Hz, 1H), 8.17 (d, *J* = 7.9 Hz, 1H), 8.03 (s, 1H), 7.50 (d, *J* = 8.1 Hz, 1H), 7.32 - 7.20 (m, 2H), 7.17 (t, J = 7.2 Hz, 1H), 7.02 (d, *J* = 8.7 Hz, 1H), 6.31 (d, J = 7.5 Hz, 1H), 6.23 (d, J = 9.1 Hz, 1H), 4.87 (d, *J* = 49.5 Hz, 1H), 4.39 *(d, J* = 5.6 Hz, 2H), 3.84 (s, 4H), 3.77 (q, J = 11.4 Hz, 2H), 3.05 (t, *J* = 11.8 Hz, 1H), 2.77 (d, *J* = 11.4 Hz, 1H), 2.36 - 2.22 (m, 1H), 2.20 (s, 3H), 2.11 (t, *J* = 10.9 Hz, 1H), 1.98 - 1.80 (m, 2H).

**Example 61**

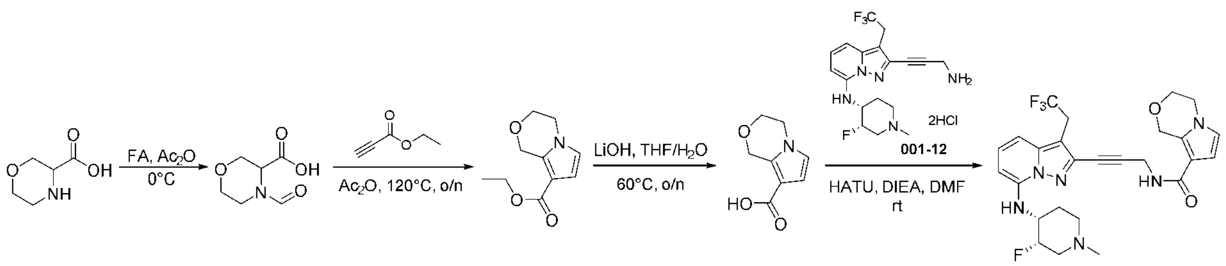


**301-1** **301-2** **301-3** **301-4** **301**

**Step 1: Synthesis of compound 301-2**

Under nitrogen protection, at 0°C, a solution of 3-morpholine carboxylic acid (compound **301-**1, 1 g, 7.626 mmol, 1 eq) and acetic anhydride (7 mL) in formic acid (2 mL) was stirred to react for 1 h. After the reaction was completed, the reaction mixture was quenched with water at room temperature, and the resulting residue was concentrated under reduced pressure to give compound **301-2** (1.5 g).
LCMS: (ESI, m/z): 159.95 $[M+H]^+$.

**Step 2: Synthesis of compound 301-3**

Under nitrogen protection, at 120°C, a solution of 4-formylmorpholine-3-carboxylic acid (compound **301-2,** 1.5 g, 9.426 mmol, 1 eq) and ethyl propiolate (1.29 g, 13.196 mmol, 1.40 eq) in acetic anhydride (3 mL) was stirred to react overnight. After the reaction was completed, the resulting residue was concentrated under reduced pressure. The resulting residue was purified by silica gel column chromatography (petroleum ether/ethyl acetate = 20:1) to give compound **301-3** (600 mg, 32.61%).
LCMS: (ESI, m/z): 196.05 $[M+H]^+$.

**Step 3: Synthesis of compound 301-4**

Under air protection, at 60°C, a solution of ethyl 3,4-dihydro-1H-pyrrolo[2,1-c][1,4]oxazine-8-carboxylate (compound **301-3,** 230 mg, 1.178 mmol, 1 eq) and lithium hydroxide (141.09 mg, 5.890 mmol, 5 eq) in tetrahydrofuran (1 mL) and water (1 mL) was stirred to react overnight. After the reaction was completed, the reaction mixture was concentrated under reduced pressure to give compound **301-4** (182 mg, 92.41%).
LCMS: (ESI, m/z): 168.00 $[M+H]^+$.

**Step 4: Synthesis of N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-3,4-dihydro-1H-pyrrolo[2,1-c][1,4]oxazine-8-carboxamide (compound 301)**

At room temperature, compound 3,4-dihydro-1*H*-pyrrolo[2,1-*c*][1,4]oxazine-8-carboxylic acid (compound **301-4,** 10 mg, 0.060 mmol, 1 eq), *N,N*-diisopropylethylamine (30.93 mg, 0.240 mmol, 4 eq) and O-(7-azabenzotriazol-1-yl)-*N,N,N',N'*-tetramethyluronium hexafluorophosphate (22.75 mg, 0.060 mmol, 1 eq) were dissolved in *N,N-dimethylformamide* (1 mL), and the mixture was stirred to react for 20 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 27.3 mg, 0.060 mmol, 1 eq) was added, and the system was stirred overnight. After the reaction was completed, the reaction mixture was quenched with water (5 mL), and extracted with ethyl acetate (2 × 5 mL). The organic phases were combined, backwashed with saturated sodium chloride (2 × 5 mL), dried over anhydrous sodium sulfate, filtered, and the filtrate was concentrated under reduced pressure. The resulting residue was purified by silica gel column chromatography (dichloromethane/methanol = 10:1) to give crude compound **301.The** crude product was purified by HPLC with the following conditions (column specifications: Xselect CSHTM Prep C18 5 μm 30x150mm OBD; mobile phase A: water (0.1% formic acid), mobile phase B: acetonitrile; flow rate: 60 mL/min; elution gradient: 15% B to 29% B in 8 min; detection wavelength: UV 254 nm/220 nm; retention time (min): 6.77), to give the carboxylate of compound ***N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-3,4-dihydro-1*H*-pyrrolo[2,1-*c*][1,4]oxazine-8-carboxamide 301** (2.81 mg, 8.05%).
LCMS: (ESI, m/z): 533.15 $[M+H]^+$.

**Example 62**

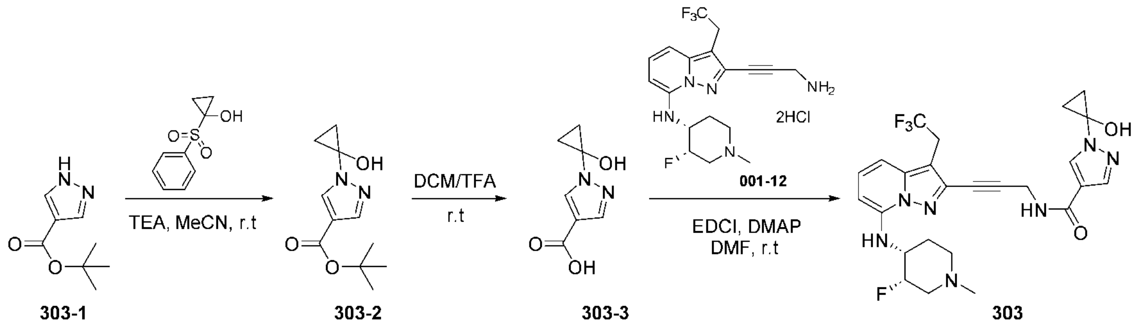

303-1 303-2 303-3 001-12 303

**Step 1: Synthesis of compound 303-2**

Under nitrogen protection, at room temperature, to a solution of tert-butyl 1*H*-pyrazole-4-carboxylate (compound **303-1,** 200 mg, 1.189 mmol, 1 eq) and 1-(benzenesulfonyl)cyclopropan-1-ol (259.3 mg, 1.308 mmol, 1.1 eq) in acetonitrile (3.0 mL) was added triethylamine (132.36 mg, 1.308 mmol, 1.1 eq) dropwise. After the addition was completed, the reaction mixture was stirred at room temperature for 1 h. After the reaction was completed, the reaction mixture was quenched with methanol (2.0 mL) at room temperature. The resulting residue was concentrated under reduced pressure and purified by silica gel column chromatography (petroleum ether/ethyl acetate = 3:1) to give compound **303-2** (200 mg, 75.00%).

LCMS: (ESI, m/z): 225.00 $[M+H]^+$.

**Step 2: Synthesis of compound 303-3**

Under nitrogen protection, at room temperature, to a solution of tert-butyl 1-(1-hydroxycyclopropyl)-1*H*-pyrazole-4-carboxylate (compound **303-2,** 100 mg, 0.446 mmol, 1 eq) in dichloromethane (1.0 mL) was added trifluoroacetic acid (0.5 mL) dropwise. After the addition was completed, the mixture was stirred at room temperature for 1 h. After the reaction was completed, the reaction mixture was concentrated under reduced temperature to give compound **303-3** (100 mg). The resulting mixture was not further purified and was directly used in the next step.

LCMS: (ESI, m/z): 168.95 $[M+H]^+$.

**Step 3: Synthesis of N-[3-(7-{[(3S,4R)-3-fluoro-1-methylpiperidin-4-yl]amino}-3-(2,2,2-trifluoroethyl)pyrazolo [1,5-a]pyridin-2-yl)prop-2-yn-1-yl]-1-(1-hydroxycyclopropyl)-1*H*-pyrazole-4-carboxamide (compound 303)**

Under nitrogen protection, at room temperature, to a solution of 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.044 mmol, 1 eq) and 1-(1-hydroxycyclopropyl)-1*H*-pyrazole-4-carboxylic acid (compound **303-3,** 9.58 mg, 0.057 mmol, 1.3 eq) in *N,N*-dimethylformamide (0.5 mL) were added 1-(3-dimethylaminopropyl)-3-ethyl carbodiimide hydrochloride (10.08 mg, 0.053 mmol, 1.2 eq) and N,N-dimethylaminopyridine (0.54 mg, 0.004 mmol, 0.1 eq). The mixture was stirred at room temperature for 1 h. After the reaction was completed, the reaction mixture was quenched with methanol (1.0 mL). The crude product was purified by HPLC with the following conditions (column specifications: YMC Triart C18 ExRs 5 μm, 30 mm × 150 mm; mobile phase A: water (10 mmol/L ammonium bicarbonate), mobile phase B: acetonitrile; flow rate: 60 mL/min; elution gradient: 28% B to 52% B in 10 min; detection wavelength: UV 254 nm/220 nm; retention time (min): 9.5), to give compound ***N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1-(1-hydroxycyclopropyl)-1*H*-pyrazole-4-carboxamide 303** (2.14 mg, 8.51%).

LCMS: (ESI, m/z): 534.05 $[M+H]^+$.

$^1$H NMR-PH-CCJS-P-477-0: (400 MHz, DMSO-d6, ppm) δ 8.73 (t, J = 5.6 Hz, 1H), 8.39 (d, J = 0.8 Hz, 1H), 7.89 (d, J = 0.8 Hz, 1H), 7.62 (s, 1H), 7.29 (dd, J = 8.8, 7.6 Hz, 1H), 7.03 (d, J = 8.7 Hz, 1H), 6.32 (d, J = 7.5 Hz, 1H), 6.23 (d, J = 9.1 Hz, 1H), 4.87 (d, J = 49.4 Hz, 1H), 4.34 (d, J = 5.5 Hz, 2H), 3.92 - 3.82 (m, 1H), 3.76 (q, J = 11.1 Hz, 2H), 3.05 (t, J = 11.5 Hz, 1H), 2.78 (d, J = 11.4 Hz, 1H), 2.37 - 2.22 (m, 1H), 2.20 (s, 3H), 2.13 (t, J = 11.1 Hz, 1H), 2.01 - 1.90 (m, 1H), 1.89 - 1.80 (m, 1H), 1.34 - 1.26 (m, 2H), 1.22 - 1.15 (m, 2H).

**Example 63**

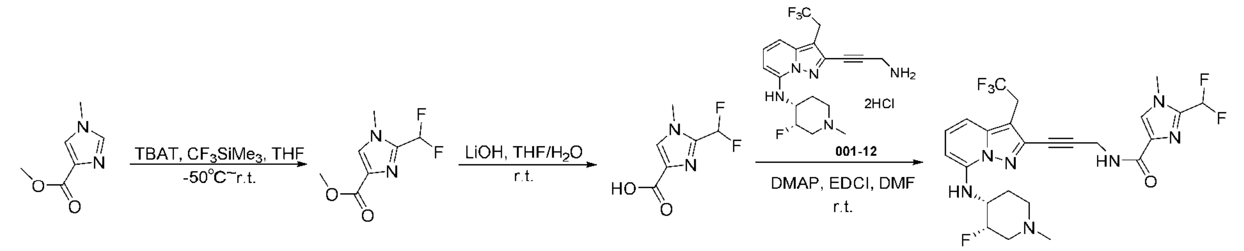


309-1 309-2 309-3 309

### Step 1: Synthesis of compound 309-2

Under argon protection, to a solution of methyl 1-methyl-1*H*-imidazole-4-carboxylate (compound **309-1,** 1 g, 7.136 mmol, 1 eq) and tetra-n-butylammonium difluorotriphenylsilicate (0.77 g, 1.427 mmol, 0.2 eq) in tetrahydrofuran (69.4 mL) was slowly added dropwise trifluoromethyltrimethylsilane (2.03 g, 14.272 mmol, 2 eq) at -50°C. After the addition was completed, the mixture was warmed to room temperature and stirred overnight under argon protection. The reaction mixture was concentrated under reduced pressure. The resulting residue was purified by silica gel column chromatography (dichloromethane:petroleum ether = 10:01) to give compound **309-2** (300 mg, 20.12%).

LCMS: (ESI, m/z): 191.05 $[M+H]^+$.

### Step 2: Synthesis of compound 309-3

At room temperature, methyl 2-(difluoromethyl)-1-methyl-1*H*-imidazole-4-carboxylate (compound **309-2**, 100 mg, 0.526 mmol, 1 eq) and lithium hydroxide (25.19 mg, 1.052 mmol, 2 eq) were added to a reaction flask, dissolved in tetrahydrofuran (5 mL) and water (1 mL), and stirred at room temperature for 2 h. After the reaction was completed, the mixture was adjusted to neutral with 3 mol/L hydrochloric acid solution. The resulting residue was concentrated under reduced pressure, dissolved in tetrahydrofuran (5 mL), dried over anhydrous sodium sulfate, filtered, and the filtrate was further concentrated under reduced pressure to give compound **309-3** (90 mg, 97.17%).

LCMS: (ESI, m/z): 176.95 $[M+H]^+$.

### Step 3: Synthesis of 2-difluoromethyl-N-[3-(7-{[(3S,4R)-3-fluoro-1-methylpiperidin-4-yl] amino}-3-(2,2,2-trifluoroethyl)pyrazolo [1,5-a] pyridin-2-yl)prop-2-yn-1-yl]-1-methyl-1*H* imidazole-4-carboxamide (compound 309)

At room temperature, 2-difluoromethyl-1-methyl-1*H*-imidazole-4-carboxylic acid (compound **309-3**, 23.16 mg, 0.132 mmol, 2 eq), 4-dimethylaminopyridine (0.8 mg, 0.007 mmol, 0.1 eq), 1-(3-dimethylaminopropyl)-3-ethyl-carbodiimide (15.12 mg, 0.079 mmol, 1.2 eq), and 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12**, 30 mg, 0.066 mmol, 1 eq) were added to a reaction flask, dissolved in *N,N*-dimethylformamide (2 mL) and allowed to react at room temperature for 2 h. After the reaction was completed, the reaction mixture was diluted with ethyl acetate (10 mL), washed with saturated sodium chloride solution (3 × 10 mL), dried over anhydrous sodium sulfate, filtered, and the filtrate was concentrated under reduced pressure. The crude product was purified by HPLC with the following conditions (column specifications: Xselect CSHTM Prep C18 5μm 30*150mm OBD; mobile phase A: water (0.1% formic acid), mobile phase B: acetonitrile; flow rate: 60 mL/min; elution gradient: 12% B to 33% B in 7 min; detection wavelength: UV 254 nm/220 nm; retention time (min): 6.2), to give compound **2-difluoromethyl-*N*-[3-(7-{[(3*S*,4*R*)-3-fluoro-1-methylpiperidin-4-yl] amino}-3-(2,2,2-trifluoroethyl)pyrazolo[1,5-*a*]pyridin-2-yl)prop-2-yn-1-yl]-1-methyl-1*H*-imidazole-4-carboxamide 309** (5.94 mg, 15.22%).

LCMS: (ESI, m/z): 542.05 $[M+H]^+$.

$^1$H NMR (400 MHz, DMSO-$d_6$, *ppm*) $\delta$ 8.61 (t, *J* = 5.9 Hz, 1H), 7.93 (s, 1H), 7.31 - 7.23 (m, 1H), 7.17 (s, 1H), 7.07 - 6.96 (m, 1H), 6.31 (d, J = 7.4 Hz, 1H), 6.25 (d, J = 8.9 Hz, 1H), 4.87 (d, *J* = 49.7 Hz, 1H), 4.30 (d, *J* = 5.9 Hz, 2H), 3.91 - 3.85 (m, 1H), 3.81 (s, 3H), 3.75 (q, *J* = 11.2 Hz, 2H), 3.05 (t, *J* = 11.5 Hz, 1H), 2.78 (d, *J* = 11.4 Hz, 1H), 2.37 - 2.21 (m, 1H), 2.20 (s, 3H), 2.13 (t, *J* = 11.2 Hz, 1H), 2.00 - 1.87 (m, 1H), 1.83 (d, *J* = 12.4 Hz, 1H).

### Example 64

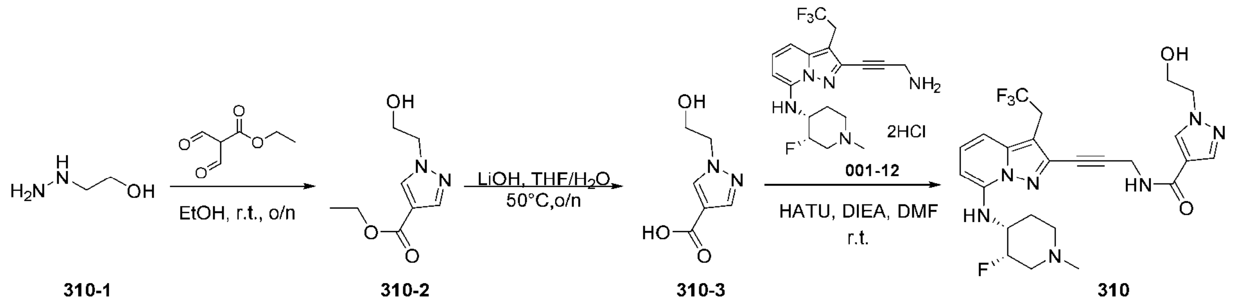


310-1 310-2 310-3 310

**Step 1: Synthesis of compound 310-2**

To a solution of 2-hydrazinoethanol (compound **310-1,** 76.1 mg, 1.000 mmol, 1 eq) in ethanol (100 mL) was added dropwise a solution of ethyl 2-formyl-3-oxopropanoate (144.13 mg, 1.000 mmol, 1 eq) in ethanol (100 mL) at 0°C, and the mixture was returned to room temperature and stirred overnight. After the reaction was completed, the mixture was concentrated in vacuum to give compound **310-2** (180 mg, 97.8%).

LCMS: (ESI, m/z): 184.95 $[M+H]^+$.

**Step 2: Synthesis of compound 310-3**

At room temperature, ethyl 1-(2-hydroxyethyl)-1H-pyrazole-4-carboxylate (compound **310-2,** 100 mg, 0.543 mmol, 1 eq) and lithium hydroxide (26.01 mg, 1.086 mmol, 2 eq) were added to a reaction flask, then dissolved in tetrahydrofuran (5 mL, 61.714 mmol, 113.67 eq) and water (1 mL, 55.509 mmol, 102.25 eq), and stirred at 50°C overnight. After the reaction was completed, the reaction mixture was adjusted to neutral with 3 mol/L hydrochloric acid solution. The resulting residue was concentrated under reduced pressure, dissolved in tetrahydrofuran (10 mL), filtered, and the filtrate was concentrated under reduced pressure to give compound **310-3** (80 mg, 94.37%).

LCMS: (ESI, m/z): 157.00 $[M+H]^+$.

**Step 2: Synthesis of N-[3-(7-{[(3S,4R)-3-fluoro-1-methylpiperidin-4-yl]amino}-3-(2,2,2-trifluoroethyl)pyrazolo [1,5-a]pyridin-2-yl)prop-2-yn-1-yl]-1-(2-hydroxyethyl)-1*H*-pyrazole-4-carboxamide (compound 310**)

At room temperature, 1-(2-hydroxyethyl)-1*H*-pyrazole-4-carboxylic acid (compound **310-3,** 20.53 mg, 0.132 mmol, 2 eq), *N,N*-diisopropylethylamine (33.99 mg, 0.264 mmol, 4 eq), and O-(7-azabenzotriazol-1-yl)-*N,N,N',N'*-tetramethyluronium hexafluorophosphate (30 mg, 0.066 mmol, 1 eq) were added to a reaction flask, dissolved in *N,N*-dimethylformamide (2 mL) and stirred at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12**, 30 mg, 0.066 mmol, 1 eq) was added, and the system was stirred for 1 h. After the reaction was completed, the reaction mixture was diluted with ethyl acetate (5 mL), washed with saturated sodium chloride solution (10 mL × 3), dried over anhydrous sodium sulfate, filtered, and the filtrate was concentrated under reduced pressure. The crude product was purified by HPLC with the following conditions (column specifications: XBridge BEH C18 OBD Prep Column 130, 5 μm, 30 mm * 150 mm; mobile phase A: water (17 mmol/L ammonium bicarbonate), mobile phase B: acetonitrile; flow rate: 60 mL/min; elution gradient: 25% B to 44% B in 8 min; detection wavelength: UV 254 nm/220 nm; retention time (min): 6.95), to give **compound N-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1-(2-hydroxyethyl)-1*H*-pyrazole-4-carboxamide 310** (9.4 mg, 26.89%).

LCMS: (ESI, m/z): 522.00 $[M+H]^+$.

$^1$H NMR (400 MHz, DMSO-$d_6$, *ppm)* δ 8.67 (t, *J* = 5.6 Hz, 1H), 8.19 (s, 1H), 7.89 (d, *J* = 0.8 Hz, 1H), 7.28 (dd, *J* = 8.8, 7.6 Hz, 1H), 7.02 (d, *J* = 8.7 Hz, 1H), 6.32 (d, J = 7.4 Hz, 1H), 6.23 (d, *J* = 9.0 Hz, 1H), 4.96 - 4.79 (m, 2H), 4.33 (d, *J* = 5.6 Hz, 2H), 4.16 (t, *J* = 5.4 Hz, 2H), 3.92 - 3.82 (m, 1H), 3.80 - 3.66 (m, 4H), 3.05 (t, *J* = 11.5 Hz, 1H), 2.78 (d, *J* = 11.5 Hz, 1H), 2.36 - 2.24 (m, 1H), 2.20 (s, 3H), 2.13 (t, J = 10.5 Hz, 1H), 1.98 - 1.89 (m, 1H), 1.88 - 1.80 (m, 1H).

**Example 65**

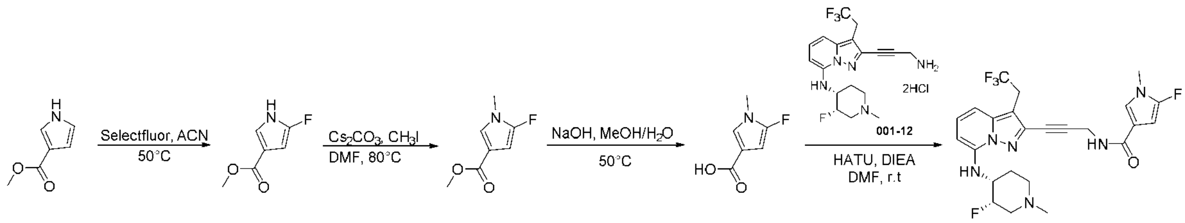


014-1 312-1 312-2 312-3 312

**Step 1: Synthesis of compound 312-1**

Under nitrogen protection, at 50°C, a solution of methyl 1H-pyrrole-3-carboxylate (compound **014-1**, 1 g, 7.992 mmol, 1 eq) and 1-chloromethyl-4-fluoro-1,4-diazabicyclo[2.2.2]octane di(tetrafluoroborate) (4.25 g, 11.988 mmol, 1.5 eq) in acetonitrile (20.0 mL) was stirred to react for 1 h. After the reaction was completed, the mixture was concentrated in vacuum and the resulting residue was purified by silica gel column chromatography (petroleum ether/ethyl acetate = 5:1) to give compound **312-1** (58 mg, 5.07%).

LCMS: (ESI, m/z): 144.00 $[M+H]^+$.

**Step 2: Synthesis of compound 312-2**

At 80°C, a solution of 5-fluoro-1*H*-pyrrole-3-carboxylate (compound **312-1,** 48 mg, 0.335 mmol, 1 eq), cesium carbonate (327.83 mg, 1.005 mmol, 3 eq) and methyl iodide (95.21 mg, 0.670 mmol, 2 eq) in *N,N-dimethylformamide* (3 mL) was stirred to react for 1 h. After the reaction was completed, the reaction mixture was quenched with water at room temperature, and then extracted with ethyl acetate (3 × 10 mL). The organic phases were combined, backwashed with saturated sodium chloride solution (3 × 10 mL), dried over anhydrous sodium sulfate, filtered, and the filtrate was concentrated under reduced pressure to give crude compound **312-2** (40 mg, 75.89%), which was not further purified.

LCMS: (ESI, m/z): 158.00 $[M+H]^+$.

**Step 3: Synthesis of compound 312-3**

At 50°C, a solution of methyl 5-fluoro-1-methyl-1*H*-pyrrole-3-carboxylate (compound **312-2,** 80 mg, 0.509 mmol, 1 eq) and sodium hydroxide (40.72 mg, 1.018 mmol, 2 eq) in methanol (2 mL) and water (2 mL) was stirred to react for 1 h. After the reaction was completed, the reaction mixture was acidified with 1 mol/L hydrochloric acid solution to pH=5, then concentrated in vacuum to give crude compound **312-3** (35 mg, 48.04%), which was not further purified.

LCMS: (ESI, m/z): 144.00 $[M+H]^+$.

**Step 4: Synthesis of 5-fluoro-N-[3-(7-{[(3S,4R)-3-fluoro-1-methylpiperidin-4-yl]amino}-3-(2,2,2-trifluoroethyl) pyrazolo[1,5-a]pyridin-2-yl)prop-2-yn-1-yl]-1-methyl-1*H*-pyrrole-3-carboxamide (compound 312)**

At room temperature, a solution of 5-fluoro-1-methyl-1H-pyrrole-3-carboxylic acid (compound **312-3,** 6.27 mg, 0.044 mmol, 1 eq), O-(7-azabenzotriazol-1-yl)-*N,N,N',N'*-tetramethyluronium hexafluorophosphate (16.67 mg, 0.044 mmol, 1 eq) and *N,N*-diisopropylethylamine (28.32 mg, 0.220 mmol, 5 eq) in *N,N*-dimethylformamide (1 mL) was stirred to react for 30 min. Then, 2-(3-aminoprop-1-yn-1-yl)-N-((3S,4R)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.044 mmol, 1 eq) was added and the system was further stirred to react for 2 h. After the reaction was completed, the reaction mixture was quenched with water at room temperature and extracted with ethyl acetate (3 × 10 mL). The organic phases were combined, backwashed with saturated sodium chloride solution (3 × 10 mL), dried over anhydrous sodium sulfate, filtered, and the filtrate was concentrated under reduced pressure. The crude product was purified by HPLC with the following conditions (column specifications: Sunfire C18 5 μm, 30 mm × 150 mm; mobile phase A: water (0.1% formic acid), mobile phase B: acetonitrile; flow rate: 60 mL/min; elution gradient: 15% to 30% in 10 min; detection wavelength: UV 254 nm/220 nm; retention time (min): 9.08), to give compound **5-fluoro-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1-methyl-1*H*-pyrrole-3-carboxamide 312** (12.59 mg, 56.15%).

LCMS: (ESI, m/z): 509.05 $[M+H]^+$.

$^1$H NMR (400 MHz, DMSO-$d_6$, *ppm*) $\delta$ 8.48 (s, 1H), 7.32 (t, *J* = 8.1 Hz, 1H), 7.06 (d, *J* = 8.7 Hz, 1H), 7.01 (d, *J* = 2.5 Hz, 1H), 6.33 (d, *J* = 7.7 Hz, 1H), 6.00 (s, 1H), 5.13 (d, *J* = 47.6 Hz, 1H), 4.29 (d, *J* = 4.8 Hz, 2H), 4.06 (dd, J = 28.8, 9.0 Hz, 1H), 3.73 - 3.59 (m, 4H), 3.53 (s, 3H), 3.33 (d, J = 10.6 Hz, 1H), 3.21 (d, J = 39.8 Hz, 1H), 3.14 (d, *J* = 13.7 Hz, 1H), 2.69 (s, 3H), 2.25 - 1.95 (m, 2H).

**Example 66**

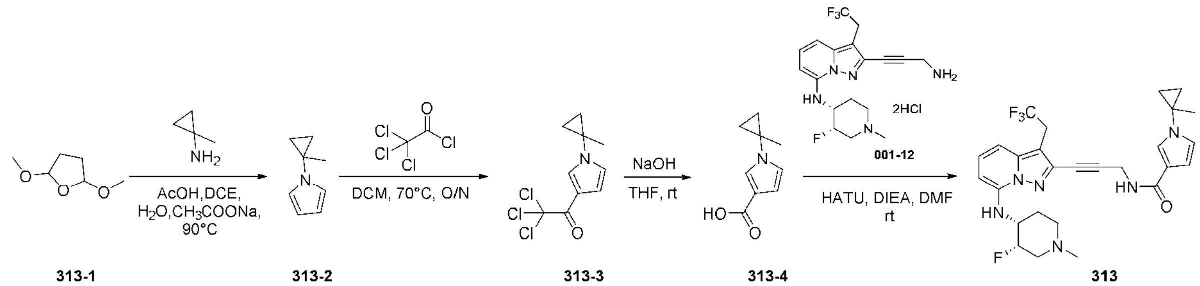


313-1 313-2 313-3 313-4 313

### Step 1: Synthesis of compound 313-2

Under argon protection, at 90°C, a solution of 2,5-dimethoxytetrahydrofuran (compound **313-1,** 500 mg, 3.783 mmol, 1 eq), 1-methylcycpropanamine (269.08 mg, 3.783 mmol, 1 eq) and sodium acetate anhydrous (772.25 mg, 5.675 mmol, 1.50 eq) in acetic acid (2 mL)/dichloroethane (10 mL)/water (6 mL) was stirred to react for 4 h. After the reaction was completed, the reaction mixture was quenched with 2 mol/L sodium hydroxide solution at room temperature, and extracted with dichloromethane (3 × 30 mL). The organic phases were combined, backwashed with saturated sodium chloride (1 × 10 mL), dried over anhydrous sodium sulfate, and filtered. The filtrate was concentrated under reduced pressure to give compound 313-2 (480 mg). The resulting mixture was not further purified and was directly used in the next step.
$^1$H NMR (400 MHz, DMSO-d6, ppm) δ 6.79 (t, J = 2.2 Hz, 2H), 5.94 (t, J = 2.2 Hz, 2H), 1.46 (s, 3H), 1.03 - 0.99 (m,2H), 0.82 - 0.78 (m,2H).

### Step 2: Synthesis of compound 313-3

At 70°C, a solution of 1-(1-methylcyclopropyl)-1*H*-pyrazole (compound **313-2,** 480 mg, 3.961 mmol, 1 eq) and trichloroacetyl chloride (1.08 g, 5.940 mmol, 1.50 eq) in dichloromethane (5 mL) was stirred to react overnight. After the reaction was completed, the reaction mixture was quenched with saturated sodium carbonate aqueous solution at 0°C, and extracted with ethyl acetate (3 × 50 mL). The organic phases were combined, backwashed with saturated sodium chloride (1 × 30 mL), dried over anhydrous sodium sulfate, and filtered. The filtrate was concentrated under reduced pressure. The resulting residue was purified by reverse phase column chromatography with the following conditions (column specifications: C18; mobile phase: water and acetonitrile (0.1% formic acid), 20% to 60% gradient in 15 min; detection wavelength: UV 254 nm), to give compound **313-3** (450 mg, 42.62%).
LCMS: (ESI, m/z): 267.85 $[M+2+H]^+$.

### Step 3: Synthesis of compound 313-4

Under nitrogen protection, at room temperature, a solution of 2,2,2-trichloro-1-(1-(1-methylcyclopropyl)-1*H*-pyrrol-3-yl)ethan-1-one (compound **313-3,** 200 mg, 0.750 mmol, 1 eq) and 10 mol/L sodium hydroxide (0.1 mL, 5 eq) in tetrahydrofuran (10.0 mL) was stirred to react for 2 h. After the reaction was completed, the reaction mixture was acidified to a pH of about 5 with hydrochloric acid solution, and then extracted with ethyl acetate (3 × 30 mL). The organic phases were combined, backwashed with saturated sodium chloride solution (1 × 10 mL), dried over anhydrous sodium sulfate, and filtered. The filtrate was concentrated under reduced pressure to give compound **313-4** (120 mg, 96.81%). The resulting mixture was not further purified and was directly used in the next step.
$^1$H NMR (400 MHz, DMSO-$d_6$, *ppm)* δ 11.70 (s, 1H), 7.39 (t, *J* = 2.0 Hz, 1H), 6.88 (t, *J* = 2.6 Hz, 1H), 6.32 (dd, *J* = 2.9, 1.7 Hz, 1H), 1.49 (s, 3H), 1.09 - 1.04 (m, 2H), 0.87 - 0.83 (m, 2H).

### Step 4: Synthesis of N-[3-(7-{[(3S,4R)-3-fluoro-1-methylpiperidin-4-yl]amino}-3-(2,2,2-trifluoroethyl)pyrazolo [1,5-a]pyridin-2-yl)prop-2-yn-1-yl]-1-(1-methylcyclopropyl)-1H-pyrrole-3-carboxamide (compound 313)

At room temperature, a solution of 1-(1-methylcyclopropyl)-1*H*-pyrrole-3-carboxylic acid (compound **313-4,** 20 mg, 0.121 mmol, 1 eq) and 2-(3-aminoprop-1-yn-1-yl)-*N*-((3*S*,4*R*)-3-fluoro-1-methylpiperidin-4-yl)-3-(2,2,2-trifluoroethyl) pyrazolo[1,5-*a*]pyridin-7-amine dihydrochloride (compound **001-12,** 46.42 mg, 0.121 mmol, 1 eq), *N,N*-diisopropylethylamine (78.24 mg, 0.605 mmol, 5 eq) and 2-(7-azobenzotriazole)-*N,N,N',N'*-tetramethyluronium hexafluorophosphate

(46.04 mg, 0.121 mmol, 1 eq) in *N,N*-dimethylformamide (2 mL) was stirred to react for 1 h. After the reaction was completed, the resulting residue was concentrated under reduced pressure, and the crude product was purified by HPLC with the following conditions (column specifications: Xselect CSHTM Prep C18 5μm 30*150mm OBD; mobile phase A: water (0.1% formic acid), mobile phase B: acetonitrile; flow rate: 60 mL/min; elution gradient: 18% B to 34% B in 8 min; detection wavelength: 254 nm/220 nm; retention time (min): 6.9), to give compound **N-[3-(7-{[(3*S*,4*R*)-3-fluoro-1-methylpiperidin-4-yl]amino}-3-(2,2,2-trifluoroethyl)pyrazolo[1,5-a]pyridin-2-yl)prop-2-yn-1-yl]-1-(1-methylcyclopropyl)-1*H*-pyrrole-3-carboxamide 313** (6.12 mg, 9.44%).

LCMS: (ESI, m/z): 531.20 [M+H]$^+$.

$^1$H NMR (400 MHz, DMSO-$d_6$, *ppm*) δ 8.32 (t, *J* = 5.8 Hz, 1H), 7.44 (t, *J* = 2.0 Hz, 1H), 7.28 (t, *J* = 8.2 Hz, 1H), 7.02 (d, *J* = 8.7 Hz, 1H), 6.86 (t, *J* = 2.6 Hz, 1H), 6.44 (t, *J* = 2.2 Hz, 1H), 6.31 (d, *J* = 7.6 Hz, 1H), 6.23 (d, *J* = 9.0 Hz, 1H), 4.87 (d, *J* = 49.5 Hz, 1H), 4.29 (d, *J* = 5.7 Hz, 2H), 3.93 - 3.83 (m, 1H), 3.75 (q, *J* = 11.1 Hz, 2H), 3.05 (t, *J* = 11.6 Hz, 1H), 2.78 (d, *J* = 11.5 Hz, 1H), 2.40 - 2.22 (m, 1H), 2.20 (s, 3H), 2.14 (t, *J* = 11.3 Hz, 1H), 2.00 - 1.88 (m, 1H), 1.87 - 1.79 (d, *J* = 13.1 Hz, 1H), 1.49 (s, 3H), 1.05 (q, *J* = 4.9 Hz, 2H), 0.85 (q, J = 5.0 Hz, 2H).

**Example 67**

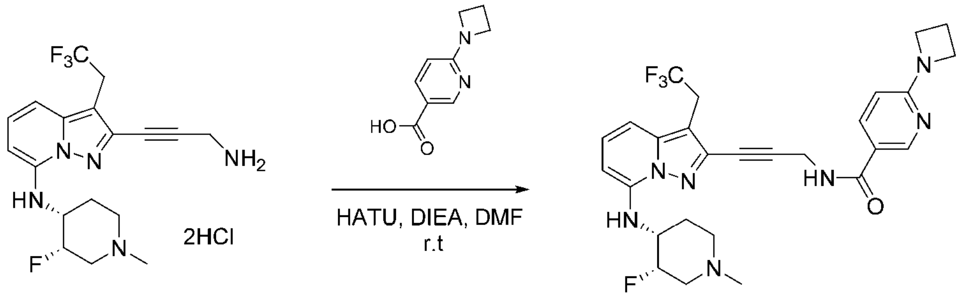


**001-12** **314**

**Step 1: Synthesis of 6-(azetidin-1-yl)-N-[3-(7-{[(3S,4R)-3-fluoro-1-methylpiperidin-4-yl]amino}-3-(2,2,2-trifluoroethyl)pyrazolo[1,5-*a*]pyridin-2-yl)prop-2-yn-1-yl]-3-nicotinamide (compound 314)**

At room temperature, a solution of 6-(azetidin-1-yl)pyridine-3-carboxylic acid (9.76 mg, 0.055 mmol, 1 eq), O-(7-azabenzotriazol-1-yl)-*N,N,N',N'*-tetramethyluronium hexafluorophosphate (20.83 mg, 0.055 mmol, 1 eq) and *N,N*-diisopropylethylamine (35.41 mg, 0.275 mmol, 5 eq) in *N,N*-dimethylformamide (1 mL) was stirred to react for 30 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 25 mg, 0.055 mmol, 1 eq) was added and the system was stirred to react for 2 h. After the reaction was completed, the reaction mixture was quenched with water at room temperature and extracted with ethyl acetate (3 × 10 mL). The organic phases were combined, backwashed with saturated sodium chloride solution (3 × 10 mL), dried over anhydrous sodium sulfate, filtered, and the filtrate was concentrated under reduced pressure. The crude product was purified by HPLC with the following conditions (column specifications: Sunfire C18 5 μm, 30 mm × 150 mm; mobile phase A: water (0.1% formic acid), mobile phase B: acetonitrile; flow rate: 60 mL/min; elution gradient: 3 to 27% in 10 min; detection wavelength: UV 254 nm/220 nm; retention time (min): 9.33), to give the carboxylate of compound **6-(azetidin-1-yl)-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-3-nicotinamide 314** (13.12 mg, 40.29%).

LCMS: (ESI, m/z): 543.85 [M+H]$^+$.

$^1$H NMR (400 MHz, DMSO-$d_6$, *ppm)* δ 8.83 (t, *J* = 5.5 Hz, 1H), 8.59 (d, *J* = 2.3 Hz, 1H), 7.96 (dd, *J* = 8.8, 2.4 Hz, 1H), 7.28 (t, J = 7.9 Hz, 1H), 7.03 (d, *J* = 8.6 Hz, 1H), 6.36 (d, J = 8.8 Hz, 1H), 6.32 (d, J = 7.6 Hz, 1H), 6.25 (d, *J* = 9.0 Hz, 1H), 4.88 (d, *J* = 49.4 Hz, 1H), 4.35 *(d, J* = 5.5 Hz, 2H), 4.02 (t, *J* = 7.4 Hz, 4H), 3.92 - 3.82 (m, 1H), 3.76 (q, *J* = 11.2 Hz, 2H), 3.07 (s, 1H), 2.79 (s, 1H), 2.40 - 2.28 (m, 3H), 2.27 - 2.08 (s, 4H), 2.05 - 1.91 (m, 1H), 1.88 - 1.79 (d, *J* = 12.8 Hz, 1H).

**Example 68**

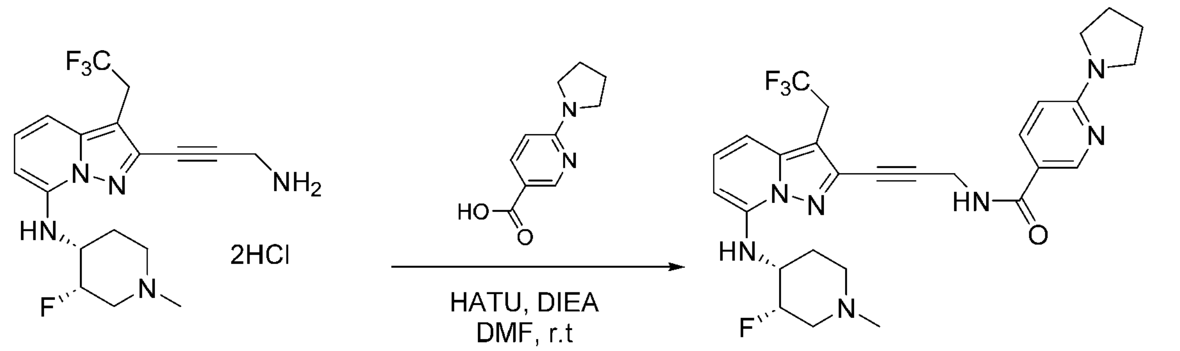


**001-12** **315**

**Step 1: Synthesis of N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-6-(pyrrolidin-1-yl)nicotinamide (compound 315)**

At room temperature, a solution of 6-(pyrrolidin-1-yl)nicotinic acid (8.42 mg, 0.044 mmol, 1 eq), O-(7-azabenzotriazol-1-yl)-*N,N,N',N'*-tetramethyluronium hexafluorophosphate (16.67 mg, 0.044 mmol, 1 eq) and *N,N*-diisopropylethylamine (28.32 mg, 0.220 mmol, 5 eq) in *N,N*-dimethylformamide (1 mL) was stirred to react for 30 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.044 mmol, 1 eq) was added and the system was further stirred to react for 2 h. After the reaction was completed, the reaction mixture was quenched with water at room temperature, and extracted with ethyl acetate (3 × 10 mL). The organic phases were combined, backwashed with saturated sodium chloride solution (3 × 10 mL), dried over anhydrous sodium sulfate, filtered, and the filtrate was concentrated under reduced pressure. The crude product was purified by HPLC with the following conditions (column specifications: Sunfire C18 5 μm, 30 mm × 150 mm; mobile phase A: water (0.1% formic acid), mobile phase B: acetonitrile; flow rate: 60 mL/min; elution gradient: 3 to 27% in 10 min; detection wavelength: UV 254 nm/220 nm; retention time (min): 9.47), to give the carboxylate of compound ***N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-6-(pyrrolidin-1-yl)nicotinamide 315** (14.01 mg, 52.74%).

LCMS: (ESI, m/z): 558.10 $[M+H]^+$.

$^1H$ NMR (400 MHz, DMSO-$d_6$, *ppm*) δ 8.79 (t, *J* = 5.6 Hz, 1H), 8.62 (d, *J* = 2.1 Hz, 1H), 7.95 (dd, *J* = 8.9, 2.5 Hz, 1H), 7.28 (d, J = 8.0 Hz, 1H), 7.02 (d, *J* = 8.7 Hz, 1H), 6.48 (d, *J* = 9.0 Hz, 1H), 6.32 (d, *J* = 7.6 Hz, 1H), 6.24 (d, *J* = 9.0 Hz, 1H), 4.87 *(d, J* = 49.5 Hz, 1H), 4.36 *(d, J* = 5.5 Hz, 2H), 3.92 - 3.82 (m, 1H), 3.76 (q, *J* = 11.1 Hz, 2H), 3.43 (s, 4H), 3.06 (t, *J* = 11.6 Hz, 1H), *2.79* (d, *J* = 11.4 Hz, 1H), 2.38 - 2.25 (m, 1H), 2.21 (s, 3H), 2.15 (t, *J* = 12.0 Hz, 1H), 2.00 - 1.88 (d, *J* = 6.9 Hz, 5H), 1.87 (d, *J* = 16.0 Hz, 1H).

**Example 69**

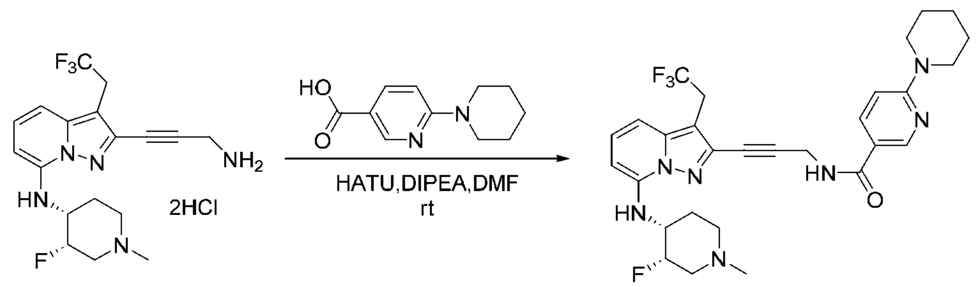


**001-12** **316**

**Step 1: Synthesis of N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-6-(piperidin-1-yl)nicotinamide (compound 316)**

Under air protection, at room temperature, a solution of 6-(piperidin-1-acyl)pyridine-3-carboxylic acid (19.37 mg, 0.094 mmol, 1.2 eq), 2-(7-azobenzothiazole)-*N,N,N',N'*-tetramethyluronium hexafluorophosphate (59.51 mg, 0.156 mmol, 2 eq) and *N,N*-diisopropylethylamine (50.57 mg, 0.390 mmol, 5 eq) in *N,N*-dimethylformamide (mL) was stirred

to react for 20 min. Then, 2-(3-aminoprop-1-yn-1-yl)-N-((3S,4R)-3-fluoro-1-methylpiperidin-4-yl)-3-(2,2,2-trifluoroethyl) pyrazolo[1,5-*a*]pyridin-7-amine dihydrochloride (compound **001-12,** 35.50 mg, 0.078 mmol, 1 eq) was added and the system was further stirred to react for 2 h. After the reaction was completed, the reaction mixture was quenched with water at room temperature and extracted with ethyl acetate (3 × 15 mL). The organic phases were combined, filtered, and the filtrate was concentrated under reduced pressure. The crude product was purified by HPLC with the following conditions (column specifications: Xselect CSHTM Prep C18 5μm 30*150mm OBD; mobile phase A: water (0.1% formic acid), mobile phase B: acetonitrile; flow rate: 60 mL/min; elution gradient: 7% B to 35% B in 8 min; detection wavelength: UV 254 nm/220 nm; retention time (min): 7.32), to give the carboxylate of compound **N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-6-(piperidin-1-yl)nicotinamide 316.**

LCMS: (ESI, m/z): 572.30 [M+H]+.

$^1$H NMR (400 MHz, DMSO-$d_6$, *ppm*) δ 8.81 (t, *J* = 5.5 Hz, 1H), 8.61 (d, *J* = 2.4 Hz, 1H), 7.95 (dd, *J* = 9.1, 2.5 Hz, 1H), 7.28 (t, *J* = 7.8 Hz, 1H), 7.02 (d, *J* = 8.7 Hz, 1H), 6.85 (d, *J* = 9.0 Hz, 1H), 6.32 (d, *J* = 7.6 Hz, 1H), 6.24 (d, *J* = 9.1 Hz, 1H), 4.87 (d, J = 50.0 Hz, 1H), 4.36 (d, *J* = 5.5 Hz, 2H), 3.91 - 3.81 (m, 1H), 3.76 (q, *J* = 11.1 Hz, 2H), 3.62 (t, *J* = 5.5 Hz, 4H), 3.06 (t, *J* = 11.6 Hz, 1H), 2.79 (d, *J* = 11.4 Hz, 1H), 2.38 - 2.24 (m, 1H), 2.21 (s, 3H), 2.14 (t, *J* = 11.5 Hz, 1H), 2.00 - 1.83 (m, 2H), 1.67 - 1.58 (m, 2H), 1.56 - 1.47 (m, 4H).

**Example 70**

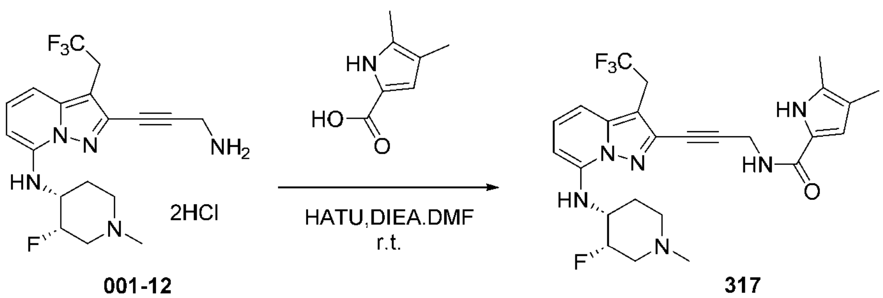


001-12

317

**Step 1: Synthesis of N-[3-(7-{[(3S,4R)-3-fluoro-1-methylpiperidin-4-yl]**amino}-3-(2,2,2-**trifluoroethyl)pyrazolo [1,5-a]pyridin-2-yl)prop-2-yn-1-yl]-4,5-dimethyl-1*H*-pyrrole-2-carboxamide (compound 317)**

At room temperature, a solution of 4,5-dimethyl-1*H*-pyrrole-2-carboxylic acid (10.98 mg, 0.079 mmol, 1.2 eq), *N,N*-diisopropylethylamine (33.99 mg, 0.264 mmol, 4 eq), and O-(7-azabenzotriazol-1-yl)-*N,N,N',N'*-tetramethyluronium hexafluorophosphate (30 mg, 0.066 mmol, 1 eq) in *N,N-dimethylformamide* (2 mL) was stirred for 10 min. Then, 2-(3-aminoprop-1-yn-1-yl)-*N*-((3*S*,4*R*)-3- fluoro-1-methylpiperidin-4-yl)-3-(2,2,2-trifluoroethyl)pyrazolo[1,5-*a*]pyridin-7-amine dihydrochloride (compound **001-12,** 30 mg, 0.066 mmol, 1 eq) was added and the mixture was further stirred for 1 h. After the reaction was completed, the reaction mixture was diluted with ethyl acetate (5 mL), washed with saturated sodium chloride solution (10 mL × 3), dried over anhydrous sodium sulfate, filtered, and the filtrate was concentrated under reduced pressure. The crude product was purified by HPLC with the following conditions (column specifications: Xselect CSHTM Prep C18 5μm 30*150mm OBD; mobile phase A: water (0.1% formic acid), mobile phase B: acetonitrile; flow rate: 60 mL/min; elution gradient: 11% B to 39% B in 8 min; detection wavelength: UV 254 nm/220 nm; retention time (min): 7.08), to give the carboxylate of compound ***N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-4,5-dimethyl-1*H*-pyrrole-2-carboxamide 317** (2.23 mg, 6.13%).

LCMS: (ESI, m/z): 505.10 [M+H]+.

$^1$H NMR (400 MHz, DMSO-$d_6$, *ppm)* δ 11.05 (s, 1H), 8.30 (t, *J* = 5.7 Hz, 1H), 7.28 (dd, *J* = 8.8, 7.6 Hz, 1H), 7.02 (d, *J* = 8.7 Hz, 1H), 6.56 (d, *J* = 2.6 Hz, 1H), 6.31 (d, *J* = 7.6 Hz, 1H), 6.24 (d, *J* = 9.0 Hz, 1H), 4.87 (d, *J* = 49.6 Hz, 1H), 4.30 (d, *J* = 5.7 Hz, 2H), 3.91 - 3.81 (m, 1H), 3.76 (q, *J* = 11.1 Hz, 2H), 3.06 (t, *J* = 11.8 Hz, 1H), 2.78 (d, *J* = 11.6 Hz, 1H), 2.36 - 2.22 (m, 1H), 2.20 (s, 3H), 2.17 - 2.12 (m, 1H), 2.10 (s, 3H), 1.99 - 1.88 (m, 4H), 1.87 - 1.79 (m, 1H).

**Example 71**

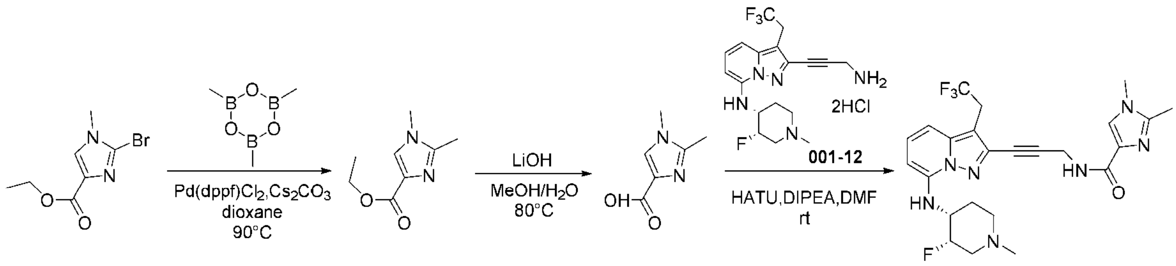


318-1 318-2 318-3 318

**Step 1: Synthesis of compound 318-2**

Under nitrogen protection, to a solution of ethyl 2-bromo-1-methyl-1H-imidazole-4-carboxylate (compound **318-1,** 500 mg, 2.145 mmol, 1 eq) in 1,4-dioxane (2 mL) were added trimethyl-1,3,5,2,4,6-cycloboroxane (134.65 mg, 1.075 mmol, 5 eq), cesium carbonate (2096.96 mg, 6.435 mmol, 3 eq) and [1,1-bis(diphenylphosphino)ferrocene] dichloropalladium (156.98 mg, 0.215 mmol, 0.1 eq). The mixture was stirred to react at 90°C for 2 h. After the reaction was completed, the reaction mixture was extracted with ethyl acetate (3 × 50 mL), the organic phases were combined, dried over anhydrous sodium sulfate, filtered, and the filtrate was concentrated under reduced pressure to give crude compound **318-2** (30 mg, 83.14%).

LCMS: (ESI, m/z): 169.00 $[M+H]^+$.

**Step 2: Synthesis of compound 318-3**

Under air protection, a solution of ethyl 1,2-dimethyl-1*H*-imidazole-4-carboxylate (compound **318-2,** 100 mg, 0.595 mmol, 1 eq) and lithium hydroxide (42.72 mg, 1.785 mmol, 3 eq) in methanol (1 mL)/water (1 mL) was stirred to react at 80°C for 1 h. After the reaction was completed, the resulting residue was concentrated under reduced pressure to give crude compound **318-3** (126 mg).

LCMS: (ESI, m/z): 141.00 $[M+H]^+$.

**Step 3: Synthesis of *N*-[3-(7-{[(3*S*,4*R*)-3-fluoro-1-methylpiperidin-4-yl]amino}-3-(2,2,2-trifluoroethyl)pyrazolo [1,5-*a*]pyridin-2-yl)prop-2-yn-1-yl]-1,2-dimethyl-1*H*-imidazole-4-carboxamide (compound 318)**

Under air protection, at room temperature, a solution of 1,2-dimethyl-1H-imidazole-4-carboxylic acid (compound **318-3**, 14.26 mg, 0.101 mmol, 1.3 eq), 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 30 mg, 0.78 mmol, 1.00 eq), 2-(7-azobenzothiazole)-*N,N,N',N'*-tetramethyluronium hexafluorophosphate (59.51 mg, 0.156 mmol, 2 eq) and *N,N*-diisopropylethylamine (50.57 mg, 0.390 mmol, 5 eq) in *N,N-dimethylformamide* (2 mL) was stirred to react for 1 h. After the reaction was completed, the reaction mixture was extracted with ethyl acetate (3 × 10 mL). The organic phases were combined, dried over anhydrous sodium sulfate, filtered, and the filtrate was concentrated under reduced pressure. The crude product was purified by HPLC with the following conditions (column specifications: Sunfire C18 5 μm, 30 mm * 150 mm; mobile phase A: water (0.1% formic acid), mobile phase B: acetonitrile; flow rate: 60 mL/min; elution gradient: 8% B to 27% B in 7 min; detection wavelength: UV 254 nm/220 nm; retention time (min): 6.85), to give the carboxylate of compound ***N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl) prop-2-yn-1-yl]-1,2-dimethyl-1*H*-imidazole-4-carboxamide 318** (9.45 mg, 21.83%).

LCMS: (ESI, m/z): 506.00 $[M+H]^+$.

$^1$H NMR (400 MHz, DMSO-d6, ppm) δ 8.32 (t, J = 6.0 Hz, 1H), 7.61 (s, 1H), 7.28 (dd, J = 8.8, 7.6 Hz, 1H), 7.02 (d, J = 8.7 Hz, 1H), 6.31 (d, J = 7.5 Hz, 2H), 4.95 (s, 1H), 4.28 (d, J = 6.0 Hz, 3H), 3.91 - 3.81 (m, 1H), 3.75 (q, J = 11.1 Hz, 2H), 3.56 (s, 3H), 3.10 (s, 1H), 2.83 (s, 1H), 2.47 (s, 3H), 2.35 - 2.10 (m, 4H), 2.04 - 1.80 (m, 2H).

**Example 72**

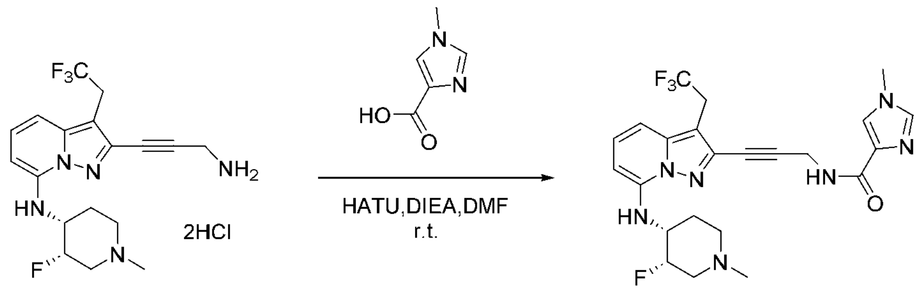


001-12 319

**Step 1: Synthesis of *N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1-methyl-1*H*-imidazole-4-carboxamide (compound 319)**

At room temperature, a solution of 1-methyl-1H-imidazole-4-carboxylic acid (9.87 mg, 0.079 mmol, 1.2 eq), *N,N*-diisopropylethylamine (33.99 mg, 0.264 mmol, 4 eq), and O-(7-azabenzotriazol-1-yl)-*N,N,N',N'*-tetramethyluronium hexafluorophosphate (30 mg, 0.066 mmol, 1 eq) in *N,N*-dimethylformamide (2 mL) was stirred for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 30 mg, 0.066 mmol, 1 eq) was added and the mixture was further stirred for 1 h. After the reaction was completed, the reaction mixture was diluted with ethyl acetate (5 mL), washed with saturated sodium chloride solution (10 mL × 3), dried over anhydrous sodium sulfate, filtered, and the filtrate was concentrated under reduced pressure. The crude product was purified by HPLC with the following conditions (column specifications: Sunfire C18 5 μm, 30 mm * 150 mm; mobile phase A: water (0.1% formic acid), mobile phase B: acetonitrile; flow rate: 60 mL/min; elution gradient: 10-10% B to 35% B in 7 min; detection wavelength: UV 254 nm/220 nm; retention time (min): 6.7), to give the carboxylate of compound ***N*-[3-(7-{[(3*S*,4*R*)-3-fluoro-1-methylpiperidin-4-yl]amino}-3-(2,2,2-trifluoroethyl)pyrazolo[1,5-*a*pyridin-2-yl)prop-2-yn-1-yl]-1-methyl-1*H*-imidazole-4-carboxamide 319** (3.2 mg, 8.92%).

LCMS: (ESI, m/z): 492.05 $[M+H]^+$.

$^1$H NMR (400 MHz, DMSO-$d_6$, *ppm*) δ 8.46 (t, *J* = 6.0 Hz, 1H), 7.69 (q, *J* = 1.3 Hz, 2H), 7.28 (dd, *J* = 8.7, 7.7 Hz, 1H), 7.01 (d, *J* = 8.5 Hz, 1H), 6.31 (d, *J* = 7.6 Hz, 1H), 6.25 (d, *J* = 9.0 Hz, 1H), 4.87 (d, *J* = 49.4 Hz, 1H), 4.34 - 4.24 (m, 2H), 3.91 - 3.81 (m, 1H), 3.75 (q, J = 11.1 Hz, 2H), 3.70 (s, 3H), 3.06 (t, J = 10.8 Hz, 1H), 2.78 (d, *J* = 11.2 Hz, 1H), 2.39 - 2.22 (m, 1H), 2.21 (s, 3H), 2.14 (t, J = 10.8 Hz, 1H), 2.01 - 1.88 (m, 1H), 1.88 - 1.80 (m, 1H).

**Example 73**

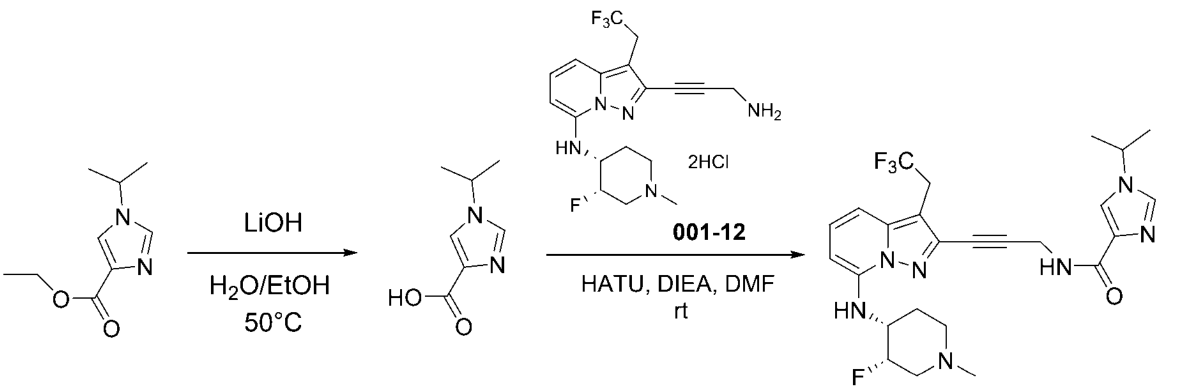


320-1 320-2 320

**Step 1: Synthesis of compound 320-2**

Under air protection, at 60°C, a solution of ethyl 1-isopropyl-1H-imidazole-4-carboxylate (compound **320-1,** 60 mg, 0.329 mmol, 1 eq) and lithium hydroxide (23.66 mg, 0.987 mmol, 3 eq) in ethanol (0.5 mL)/water (0.5 mL) was stirred to react for 1 h. After the reaction was completed, the reaction mixture was acidified to a pH of about 3 with hydrochloric acid solution. The reaction mixture was extracted with dichloromethane/methanol (3:1, 3 × 10 mL), the organic phase was combined, dried over anhydrous sodium sulfate, filtered, and the filtrate was concentrated under reduced pressure to give

compound **320-2** (30 mg, 59.10%).

LCMS: (ESI, m/z): 154.00 [M+H]+.

**Step 2: Synthesis of *N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1-isopropyl-1*H*-imidazole-4-carboxamide (compound 320)**

At room temperature, a solution of 1-isopropyl-1H-imidazole-4-carboxylic acid (compound **320-2,** 14.48 mg, 0.094 mmol, 1.2 eq) and 2-(3-aminoprop-1-yn-1-yl)-N-((3S,4R)-3-fluoro-1-methylpiperidin-4-yl)-3-(2,2,2-trifluoroethyl) pyrazolo[1,5-*a*]pyridin-7-amine dihydrochloride (compound **001-12,** 30 mg, 0.078 mmol, 1.00 eq), N,N-diisopropylethylamine (50.57 mg, 0.390 mmol, 5 eq) in *N,N*-dimethylformamide (2 mL) was stirred to react for 1 h. The reaction mixture was extracted with ethyl acetate (3 × 10 mL). The organic phases were combined, and dried over sodium sulfate. The resulting mixture was filtered, and then the filtrate was concentrated under reduced pressure. The crude product was purified by HPLC with the following conditions (column specifications: Xselect CSHTM Prep C18 5μm 30*150mm OBD; mobile phase A: water (0.1% formic acid), mobile phase B: acetonitrile; flow rate: 60 mL/min; elution gradient: 5% B to 28% B in 10 min; detection wavelength: UV 254 nm/220 nm; retention time (min): 9.17) to give compound ***N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1-isopropyl-1*H*-imidazole-4-carboxamide 320** (1.75 mg, 4.21%).

LCMS: (ESI, m/z): 520.55 [M+H]+.

$^1$H NMR (400 MHz, DMSO-$d_6$, ppm) δ 8.45 (t, J = 5.9 Hz, 1H), 7.83 (d, J = 11.8 Hz, 2H), 7.28 (t, J = 8.1 Hz, 1H), 7.02 (d, J = 8.7 Hz, 1H), 6.31 (d, J = 7.6 Hz, 2H), 4.91 (d, J = 49.4 Hz, 1H), 4.52 - 4.40 (m, 1H), 4.30 (d, J = 6.0 Hz, 2H), 3.96 - 3.81 (m, 1H), 3.75 (q, J = 11.2 Hz, 2H), 3.14 (s, 2H), 2.86 (s, 1H), 2.40 - 2.14 (m, 4H), 2.06 - 1.92 (m, 1H), 1.90 - 1.82 (m, 1H), 1.42 (d, J = 6.7 Hz, 6H).

**Example 74**

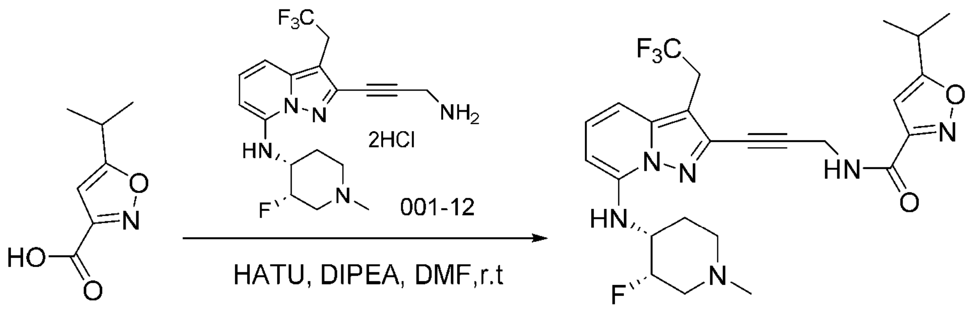


**321-1** **321**

**Step 1: Synthesis of *N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-5-isopropylisoxazole-3-carboxamide (compound 319)**

At room temperature, to a solution of 5-methylisoxazole-3-carboxylic acid (compound **321-1,** 12.24 mg, 0.079 mmol, 1.2 eq) in *N,N-dimethylformamide* (1 mL) were added 2-(7-azobenzothiazole)-*N,N,N',N'*-tetramethyluronium hexafluorophosphate (50.0 mg, 0.131 mmol, 2.00 eq) and *N,N*-diisopropylethylamine (42.49 mg, 0.329 mmol, 5.00 eq), and the mixture was stirred to react for 5 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound 001-12, 30 mg, 0.066 mmol, 1.00 eq) was added, and the system was allowed to react for 1 h. After the reaction was completed, water (10 mL) was added and the reaction mixture was extracted with ethyl acetate (3 × 15 mL). The organic phases were combined, dried over anhydrous sodium sulfate, filtered, and the filtrate was concentrated under reduced pressure. The resulting residue was purified by silica gel column chromatography (dichloromethane/methanol = 9:1). The crude product was purified by HPLC with the following conditions (column specifications: Xselect CSHTM Prep C18 5μm 30x150mm OBD; mobile phase A: water (0.1% formic acid), mobile phase B: acetonitrile; flow rate: 60 mL/min; elution gradient: 15% B to 40% B in 8 min; detection wavelength: UV 254 nm/220 nm; retention time (min): 7), to give compound ***N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-5-isopropylisoxazole-3-carboxamide 321** (5.92 mg, 17.26%).

LCMS: (ESI, m/z): 521.15 [M+H]+.

$^1$H NMR (400 MHz, DMSO-$d_6$, *ppm)* $\delta$ 9.31 (t, *J* = 5.7 Hz,1H), 7.29 (t, *J* = 8.2 Hz, 1H), 7.04 (d, *J* = 8.7 Hz, 1H), 6.61 (s, 1H), 6.35 (s, 1H), 6.32 (d, J = 7.5 Hz, 1H), 4.94 (d, J = 48.8 Hz, 1H), 4.35 (d, *J* = 5.7 Hz, 2H), 4.00 - 3.82 (m, 1H), 3.76 (q, *J* = 11.1 Hz, 2H), 3.24 - 3.06 (m,2H), 2.91 (s, 1H), 2.32 (s, 5H), 2.07 - 1.94 (m, 1H), 1.93 - 1.85 (m,1H), 1.27 (d, *J* = 6.9 Hz, 6H).

**Example 75**

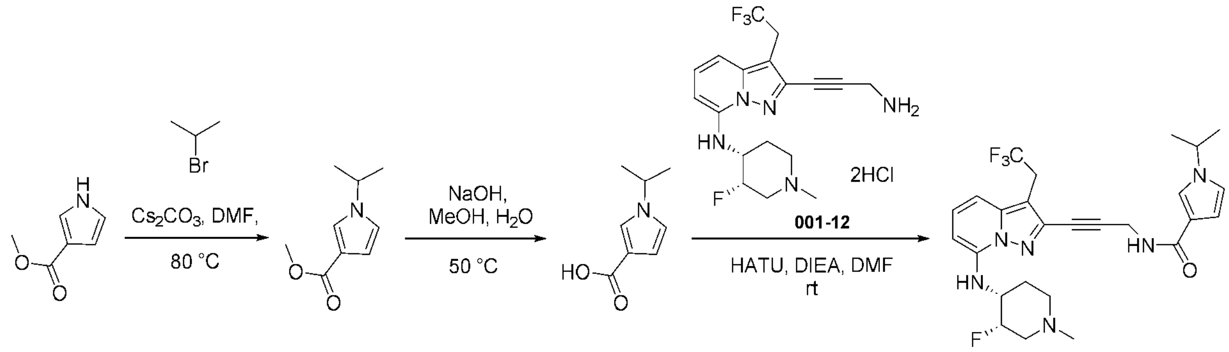


**014-1** **322-1** **322-2** **322**

**Step 1: Synthesis of compound 322-1**

At room temperature, to a solution of methyl 1*H*-pyrrole-3-carboxylate (compound **014-1,** 200 mg, 1.598 mmol, 1 eq) in *N,N-dimethylformamide* (3 mL) were added 2-bromopropane (393.18 mg, 3.196 mmol, 2 eq) and cesium carbonate (1562.34 mg, 4.794 mmol, 3 eq). After the addition was completed, the system was warmed to 80°C and further stirred for 1 h. After the reaction was completed, the reaction mixture was diluted with water (50 mL), and the aqueous phase was extracted with ethyl acetate (3 × 50 mL). The organic phases were combined, backwashed with saturated brine (3 × 50 mL), dried over anhydrous sodium sulfate, filtered, and the filtrate was concentrated under reduced pressure to give compound **322-1** (260 mg, 96.80%) which was not further purified and was directly used in the next step.
LCMS: (ESI, m/z): 168.05 [M+H]$^+$.

**Step 2: Synthesis of compound 322-2**

At room temperature, to a solution of methyl 1-isopropyl-1*H*-pyrrole-3-carboxylate (compound **322-1,** 20 mg, 0.120 mmol, 1 eq) in water (0.6 mL) and anhydrous methanol (0.2 mL) was added sodium hydroxide (9.57 mg, 0.240 mmol, 2 eq). After the addition was completed, the system was warmed to 50°C and further stirred for 2 h. After the reaction was completed, the reaction mixture was diluted with water (20 mL), and extracted with ethyl acetate (3 × 20 mL). The aqueous phase was acidified with 4 mol/L hydrochloric acid to pH=1, and then backwashed with ethyl acetate (3 × 20 mL). The organic phase was dried over anhydrous sodium sulfate, filtered, and the filtrate was concentrated under reduced pressure to give compound **322-2** (16 mg, 83.66%). The crude product was directly used in the next step.
LCMS: (ESI, m/z): 154.10 [M+H]$^+$.

**Step 3: Synthesis of N-[3-(7-{[(3S,4R)-3-fluoro-1-methylpiperidin-4-yl]**amino}-3-(2,2,2-**trifluoroethyl)pyrazolo [1,5-a]pyridin-2-yl)prop-2-yn-1-yl]-1-isopropyl-1*H*-pyrrole-3-carboxamide (compound 300)**

At room temperature, to a solution of 1-isopropyl-1*H*-pyrrole-3-carboxylic acid (compound **322-2,** 6.71 mg, 0.044 mmol, 1 eq) in *N,N-dimethylformamide* (0.5 mL) were added O-(7-azabenzotriazol-1-yl)-*N,N,N',N'*-tetramethyluronium hexafluorophosphate (16.67 mg, 0.044 mmol, 1 eq) and diisopropylethylamine (22.66 mg, 0.176 mmol, 4 eq), and the mixture was stirred to react for 30 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.044 mmol, 1 eq) was added and the mixture was further stirred for 1 h. After the reaction was completed, the reaction mixture was diluted with water (20 mL) and the aqueous phase was extracted with ethyl acetate (3 × 20 mL). The organic phases were combined, backwashed with saturated brine (3 × 20 mL), dried over anhydrous sodium sulfate. The crude product was purified by HPLC with the following conditions (column specifications: Kinetex 5 μm EVO C18, 30mm * 150 mm; mobile phase A: water (10 mmol/L ammonium bicarbonate), mobile phase B: acetonitrile; flow rate: 60 mL/min; elution gradient: 37% B to 53% B in 7 min; detection wavelength: UV 254 nm/220 nm; retention time (min): 6.6), to give compound **N-[3-(7-{[(3S,4R)-3-fluoro-1-methylpiperidin-4-yl]amino}-3-(2,2,2-trifluoroethyl)pyrazolo[1,5-a]pyridin-2-yl)**

**prop-2-yn-1-yl]-1-isopropyl-1*H*-pyrrole-3-carboxamide 300** (4.37 mg, 19.02%).

LCMS: (ESI, m/z): 519.15 $[M+H]^+$.

$^1H$ NMR (400 MHz, DMSO-$d_6$, ppm) δ 8.30 (t, J = 5.7, 5.7 Hz, 1H), 7.44 (t, J = 2.0, 2.0 Hz, 1H), 7.28 (m, J = 8.7, 7.6 Hz, 1H), 7.02 (d, J = 8.7 Hz, 1H), 6.85 (t, J = 2.6, 1H), 6.47 (dd, J = 2.9, 1.8 Hz, 1H), 6.30 (t, J = 9.0 Hz, 1H), 6.25 (d, J = 9.0 Hz, 1H), 4.87 (d, J = 49.4 Hz, 1H), 4.36 - 4.21 (m, 3H), 3.93 - 3.81 (m, 1H), 3.75 (q, J = 11.1 Hz, 2H), 3.06 (t, J = 11.3, 11.3 Hz, 1H), 2.78 (d, J = 11.3 Hz, 1H), 2.39 - 2.24 (m, 1H), 2.21 (s, 3H), 2.14 (t, J = 11.5, 11.5 Hz, 1H), 1.90 - 2.01 (m, 1H), 1.87 - 1.79 (m, 1H), 1.37 (d, J = 6.7 Hz, 6H).

**Example 76**

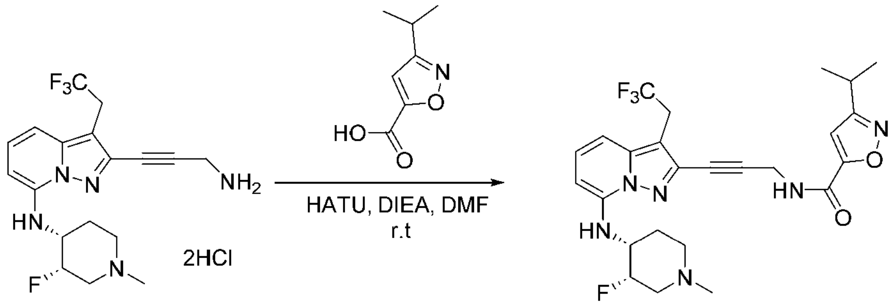


**001-12** **323**

**Step 1: Synthesis of *N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-3-isopropylisoxazole-5-carboxamide (compound 323)**

At room temperature, to a solution of 3-isopropyl-1,2-oxazole-5-carboxylic acid (13.6 mg, 0.088 mmol, 2 eq) in *N,N-dimethylformamide* (0.5 mL) were added O-(7-azabenzotriazol-1-yl)-*N,N,N',N'*-tetramethyluronium hexafluorophosphate (33.33 mg, 0.088 mmol, 2 eq) and diisopropylethylamine (22.66 mg, 0.176 mmol, 4 eq), and the mixture was stirred to react for 30 min. Then, 2-(3-aminoprop-1-yn-1-yl)-*N*-((3*S*,4*R*)-3-fluoro-1-methylpiperidin-4-yl)-3-(2,2,2-trifluoroethyl) pyrazolo[1,5-*a*]pyridin-7-amine dihydrochloride (compound **001-12**, 20 mg, 0.044 mmol, 1 eq) was added and the mixture was stirred to react at room temperature for 1 h. After the reaction was completed, the reaction mixture was diluted with water (20 mL) and the aqueous phase was extracted with ethyl acetate (3 × 20 mL). The organic phases were combined, backwashed with saturated brine (3 × 20 mL), dried over anhydrous sodium sulfate, filtered, and the filtrate was concentrated under reduced pressure. The crude product was purified by HPLC with the following conditions (column specifications: XBridge BEH C18 OBD Prep Column 130, 5 μm, 30 mm * 150 mm; mobile phase A: water (17 mmol/L ammonium bicarbonate), mobile phase B: acetonitrile; flow rate: 60 mL/min; elution gradient: 36% B to 56% B in 8 min; detection wavelength: UV 254 nm/220 nm; retention time (min): 7.87), to give compound ***N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-3-isopropylisoxazole-5-carboxamide 323** (3.44 mg, 15.03%).

LCMS: (ESI, m/z): 521.05 $[M+H]^+$.

$^1H$ NMR (400 MHz, DMSO-$d_6$) δ 9.52 (t, J = 5.6, 5.6 Hz, 1H), 7.29 (m, J = 8.8, 7.6 Hz, 1H), 7.13 (s, 1H), 7.03 (d, J = 8.7 Hz, 1H), 6.33 (d, J = 7.4 Hz, 1H), 6.24 (d, J = 9.1 Hz, 1H), 4.87 (d, J = 49.5 Hz, 1H), 4.37 (d, J = 5.6 Hz, 2H), 3.95 - 3.81 (m, 1H), 3.75 (q, J = 11.1 Hz, 2H), 3.13 - 2.98 (m, 2H), 2.78 (d, J = 11.4 Hz, 1H), 2.37 - 2.22 (m, 1H), 2.20 (s, 3H), 2.13 (t, J = 11.0, 1H), 2.00 - 1.78 (m, 2H), 1.25 (d, J = 6.9 Hz, 6H).

**Example 77**

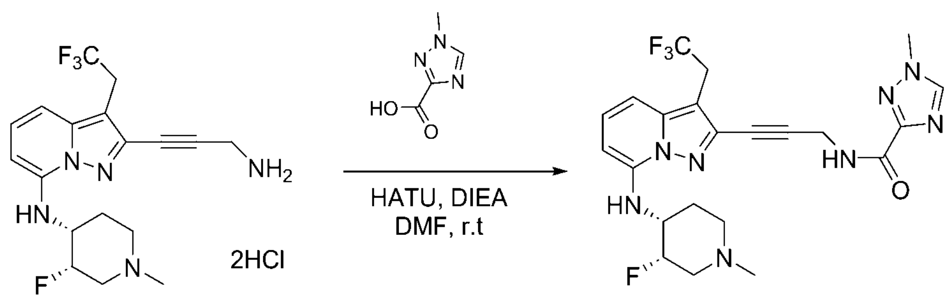


001-12

324

**Step 1: Synthesis of *N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1-methyl-1*H*-1,2,4-triazole-3-carboxamide (compound 324)**

At room temperature, a solution of 1-methyl-1*H*-1,2,4-triazole-3-carboxylic acid (5.57 mg, 0.044 mmol, 1 eq), O-(7-azabenzotriazol-1-yl)-*N,N,N',N'*-tetramethyluronium hexafluorophosphate (16.67 mg, 0.044 mmol, 1 eq) and N,N-diisopropylethylamine (28.32 mg, 0.220 mmol, 5 eq) in *N,N*-dimethylformamide (1 mL) was stirred to react for 30 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.044 mmol, 1 eq) was added and the system was further stirred to react for 2 h. After the reaction was completed, the reaction mixture was quenched with water at room temperature, and extracted with ethyl acetate (3 × 30 mL). The organic phases were combined, backwashed with saturated sodium chloride solution (3 × 30 mL), dried over anhydrous sodium sulfate, filtered, and the filtrate was concentrated under reduced pressure. The crude product was purified by HPLC with the following conditions (column specifications: Sunfire C18 5 μm, 30 mm × 150 mm; mobile phase A: water (0.1% ammonium bicarbonate), mobile phase B: acetonitrile; flow rate: 60 mL/min; elution gradient: 30% to 52% in 8 min; detection wavelength: UV 254 nm/220 nm; retention time (min): 7.12), to give compound ***N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1-methyl-1*H*-1,2,4-triazole-3-carboxamide 324** (8.98 mg, 41.44%).

LCMS: (ESI, m/z): 493.05 $[M+H]^+$.

$^1H$ NMR (400 MHz, DMSO-$d_6$, *ppm*) δ 9.05 (t, *J* = 5.9 Hz, 1H), 8.61 (s, 1H), 7.28 (dd, *J* = 8.8, 7.6 Hz, 1H), 7.02 (d, *J* = 8.7 Hz, 1H), 6.31 (dd, *J* = 7.9, 1.0 Hz, 1H), 6.27 (d, *J* = 9.0 Hz, 1H), 4.87 (d, *J* = 49.4 Hz, 1H), 4.32 (d, *J* = 5.9 Hz, 2H), 3.94 (s, 3H), 3.91 - 3.82 (m, 1H), 3.75 (q, *J* = 11.1 Hz, 2H), 3.06 (t, *J* = 11.6 Hz, 1H), 2.79 (d, *J* = 11.3 Hz, 1H), 2.39 - 2.20 (m, 1H), 2.21 (s, 3H), 2.14 (t, *J* = 11.3 Hz, 1H), 2.01 - 1.89 (m, 1H), 1.83 (d, *J* = 12.6 Hz, 1H).

**Example 78**

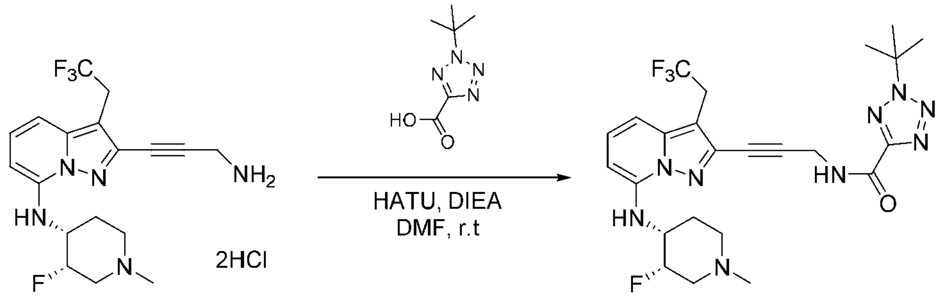


001-12

145

**Step 1: Synthesis of 2-tert-butyl-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-2*H*-tetrazole-5-carboxamide (compound 145)**

At room temperature, a solution of 2-tert-butyl-2*H*-tetrazole-5-carboxylic acid (18.65 mg, 0.044 mmol, 1 eq), O-(7-azabenzotriazol-1-yl)-*N,N,N',N'*-tetramethyluronium hexafluorophosphate (16.67 mg, 0.044 mmol, 1 eq) and *N,N*-diisopropylethylamine (28.32 mg, 0.220 mmol, 5 eq) in *N,N*-dimethylformamide (1 mL) was stirred to react for 30 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.044 mmol, 1 eq) was added and the system was stirred to react at room temperature for 2 h. After the reaction was completed, the reaction mixture was quenched with water at room temperature, and extracted with ethyl acetate (3 × 10 mL). The organic phases were combined, backwashed with saturated sodium chloride solution (3 × 10 mL), dried over anhydrous sodium sulfate, filtered, and the filtrate was concentrated under reduced pressure. The crude product was purified by HPLC with the following conditions (column specifications: Sunfire C18 5 μm, 30 mm × 150 mm; mobile phase A: water (0.1% ammonium bicarbonate), mobile phase B: acetonitrile; flow rate: 60 mL/min; elution gradient: 42% to 60% in 7 min; detection wavelength: UV 254 nm/220 nm; retention time (min): 6.07), to give compound **2-tert-butyl-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-2*H*-tetrazole-5-carboxamide 145** (6.74 mg, 28.63%).

LCMS: (ESI, m/z): 536.15 $[M+H]^+$.

$^1H$ NMR (400 MHz, DMSO-$d_6$, *ppm*) δ 9.60 (t, *J* = 5.7 Hz, 1H), 7.29 (dd, *J* = 8.8, 7.6 Hz, 1H), 7.03 (d, *J* = 8.7 Hz, 1H), 6.32 (dd, *J* = 7.9, 1.1 Hz, 1H), 6.24 (d, *J* = 9.0 Hz, 1H), 4.87 (d, J = 49.8 Hz, 1H), 4.40 (d, *J* = 5.8 Hz, 2H), 3.93 - 3.81 (m, 1H), 3.76 (q, *J* = 11.0 Hz, 2H), 3.05 (t, *J* = 11.4 Hz, 1H), 2.78 (d, *J* = 11.4 Hz, 1H), 2.37 - 2.23 (m, 1H), 2.20 (s, 3H), 2.13 (t, *J* = 11.1 Hz, 1H), 1.99 - 1.87 (m, 1H), 1.88 - 1.81 (m, 1H), 1.74 (s, 9H).

**Example 79**

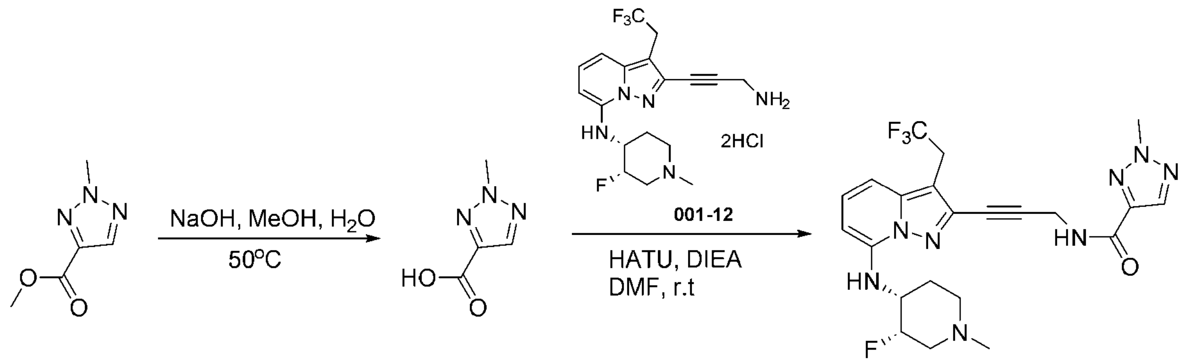


325-1 325-2 325

**Step 1: Synthesis of compound 325-2**

Under nitrogen protection, at room temperature, to a solution of methyl 2-methyl-1,2,3-triazole-4-carboxylate (compound **325-1,** 50 mg, 0.354 mmol, 1 eq) in anhydrous methanol (1.0 mL) and water (0.5 mL) was added sodium hydroxide (28.34 mg, 0.708 mmol, 2 eq), and the mixture was further stirred at 50°C for 1 h. After the reaction was completed, the reaction mixture was acidified to pH=6 with hydrochloric acid solution. The resulting residue was concentrated under reduced pressure and then dissolved in tetrahydrofuran (2.0 mL), filtered, and the filter cake was washed with tetrahydrofuran (3 × 5 mL). The filtrate was concentrated under reduced pressure to give compound **325-2** (35 mg, 77.73%).

LCMS: (ESI, m/z): 536.15 $[M+H]^+$.

$^1H$ NMR (400 MHz, DMSO-$d_6$, *ppm*) δ 13.30 (s, 1H), 8.17 (s, 1H), 4.22 (s, 3H).

**Step 2: Synthesis of *N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-2-methyl-2*H*-1,2,3-triazole-4-carboxamide (compound 325)**

Under nitrogen protection at room temperature, to a solution of O-(7-azabenzotriazol-1-yl)-*N,N,N',N'*-tetramethyluronium hexafluorophosphate (16.67 mg, 0.044 mmol, 1 eq) and 2-methyl-1,2,3-triazole-4-carboxylic acid (compound **325-2,** 8.36 mg, 0.066 mmol, 1.5 eq) in *N,N*-dimethylformamide (0.8 mL) was added *N,N*-diisopropylethylamine (28.32 mg, 0.220 mmol, 5 eq) dropwise. The mixture was stirred to react for 1 h, and then 2-(3-aminoprop-1-yn-1-yl)-*N*-((3S,4R)-3-fluoro-1-methylpiperidin-4-yl)-3-(2,2,2-trifluoroethyl)pyrazolo[1,5-*a*]pyridin-7-amine dihydrochloride (compound **001-12**, 20 mg, 0.044 mmol, 1 eq) was added and the mixture was further stirred for 1 h. After the reaction was completed, the reaction mixture was quenched at room temperature with methanol (1.0 mL). The crude product was purified by HPLC with the following conditions (column specifications: Xselect CSHTM Prep C18 5μm 30x150mm OBD; mobile phase A: water (0.1% formic acid), mobile phase B: acetonitrile; flow rate: 100 mL/min; elution gradient: 13% B to

27% B in 8 min; detection wavelength: UV 254 nm/223 nm; retention time (min): 6.35), to give the carboxylate of compound ***N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl) prop-2-yn-1-yl]-2-methyl-2*H*-1,2,3-triazole-4-carboxamide 325** (7.45 mg, 31.15%).

LCMS: (ESI, m/z): 493.20 $[M+H]^+$.

$^1H$ NMR (400 MHz, DMSO-$d_6$, *ppm)* $\delta$ 9.09 (t, *J* = 5.7 Hz, 1H), 8.16 (s, 1H), 7.28 (t, *J* = 8.2 Hz, 1H), 7.02 (d, *J* = 8.6 Hz, 1H), 6.32 (d, *J* = 7.6 Hz, 1H), 6.24 (d, *J* = 9.0 Hz, 1H), 4.87 (d, *J* = 49.5 Hz, 1H), 4.34 (d, *J* = 5.7 Hz, 2H), 4.23 (s, 3H), 3.95 - 3.81 (m, 1H), 3.76 (q, *J* = 11.0 Hz, 2H), 3.05 (t, *J* = 11.6 Hz, 1H), 2.78 (d, *J* = 11.0 Hz, 1H), 2.36 - 2.23 (m, 1H), 2.20 (s, 3H), 2.13 (t, J = 10.6 Hz, 1H), 1.99 - 1.81 (m, 2H).

**Example 80**

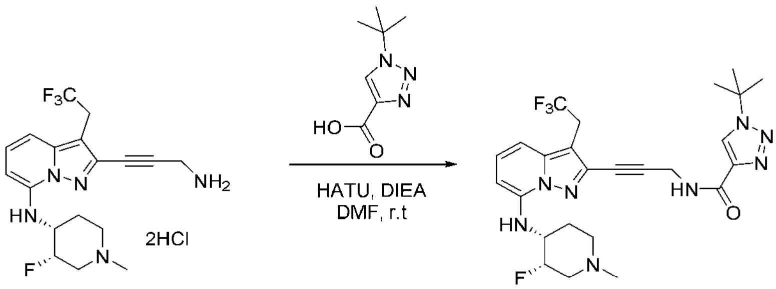


**001-12** **142**

**Step 1: Synthesis of 1-tert-butyl-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1*H*-1,2,3-triazole-4-carboxamide (compound 142)**

Under nitrogen protection, at room temperature, to a solution of O-(7-azabenzotriazol-1-yl)-*N,N,N',N'*-tetramethyluronium hexafluorophosphate (19.84 mg, 0.052 mmol, 1 eq) and 1-tert-butyl-1*H*-1,2,3-triazole-4-carboxylic acid (13.24 mg, 0.078 mmol, 1.5 eq) in *N,N*-dimethylformamide (0.8 mL) was added *N,N*-diisopropylethylamine (33.71 mg, 0.260 mmol, 5 eq) dropwise. The mixture was stirred to react for 1 h, and then 2-(3-aminoprop-1-yn-1-yl)-N-((3S,4R)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.052 mmol, 1 eq) was added and the mixture was further stirred for 1 h. After the reaction was completed, the reaction mixture was quenched at room temperature with methanol (1.0 mL). The crude product was purified by HPLC with the following conditions (column specifications: Kinetex 5 μm EVO C18, 30mm × 150 mm; mobile phase A: water (10 mmol/L ammonium bicarbonate), mobile phase B: acetonitrile; flow rate: 60 mL/min; elution gradient: 36% B to 54% B in 8 min; detection wavelength: UV 254 nm/220 nm; retention time (min): 7.13), to give compound **1-tert-butyl-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl) prop-2-yn-1-yl]-1*H*-1,2,3-triazole-4-carboxamide 142** (7.45 mg, 26.66%).

LCMS: (ESI, m/z): 535.20 $[M+H]^+$.

$^1H$ NMR (400 MHz, DMSO-$d_6$, *ppm)* $\delta$ 9.08 (t, *J* = 5.8 Hz, 1H), 8.75 (s, 1H), 7.28 (dd, *J* = 8.8, 7.6 Hz, 1H), 7.02 (d, *J* = 8.6 Hz, 1H), 6.32 (d, J = 7.5 Hz, 1H), 6.24 (d, *J* = 9.1 Hz, 1H), 4.87 (d, *J = 49.3* Hz, 1H), 4.35 (d, *J* = 5.8 Hz, 2H), 3.92 - 3.81 (m, 1H), 3.76 (q, *J* = 11.1 Hz, 2H), 3.05 (t, *J= 11.6* Hz, 1H), 2.78 (d, *J* = 11.4 Hz, 1H), 2.38 - 2.23 (m, 1H), 2.20 (s, 3H), 2.13 *(t, J* = 9.9 Hz, 1H), 1.99 - 1.79 (m, 2H), 1.64 (s, 9H).

**Example 81**

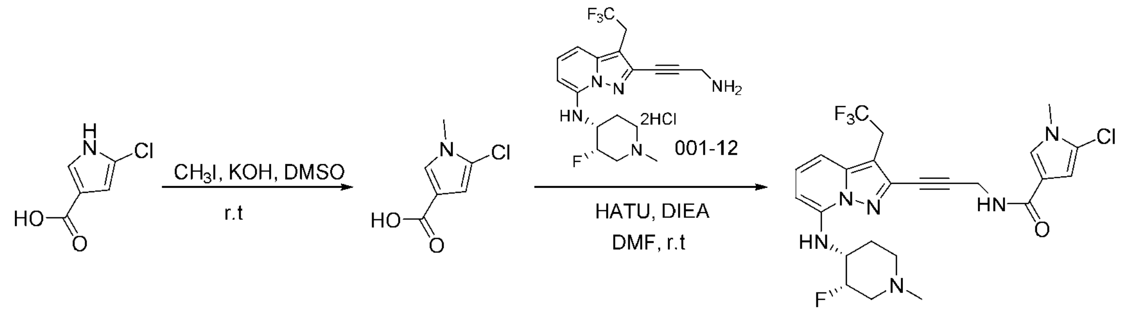


**326-1** **326-2** **326**

**Step 1: Synthesis of compound 326-2**

Under nitrogen protection, 5-chloro-1*H*-pyrrole-3-carboxylic acid (compound **326-1,** 50 mg, 0.344 mmol, 1 eq) and potassium hydroxide (154.2 mg, 2.752 mmol, 8 eq) were added to a reaction flask, dissolved in dimethyl sulfoxide (1 mL, 14.079 mmol), and stirred at room temperature for 1 h. Then iodomethane (48.76 mg, 0.344 mmol, 1 eq) was added and the mixture was further continued for 1 h. After the reaction was completed, the reaction mixture was acidified to pH=6 with 1 mol/L hydrochloric acid aqueous solution. The mixture was extracted with ethyl acetate (20 mL × 2), the organic phases were combined, backwashed with saturated sodium chloride solution (20 mL × 3), dried over anhydrous sodium sulfate and filtered. The filtrate was concentrated under reduced pressure to give compound **326-2** (40 mg, 61.66%).

LCMS: (ESI, m/z): 159.95 $[M+H]^+$.

**Step 2: Synthesis of 5-chloro-*N*-(3-(7-{[(3*S*,4*R*)-3-fluoro-1-methylpiperidin-4-yl]amino}-3-(2,2,2-trifluoroethyl) pyrazolo[1,5-*a*]pyridin-2-yl)prop-2-yn-1-yl)-1-methyl-1*H*-pyrrole-3-carboxamide (compound 326)**

Under nitrogen protection, 5-chloro-1-methyl-1*H*-pyrrole-3-carboxylic acid (compound **326-2,** 15.74 mg, 0.099 mmol, 1.5 eq), 2-(7-azabenzotriazole)-*N,N,N',N'*-tetramethyluronium hexafluorophosphate (25.0 mg, 0.066 mmol, 1 eq), and *N,N*-diisopropylethylamine (42.49 mg, 0.330 mmol, 5 eq) were added to a reaction flask, dissolved in *N,N*-dimethylformamide (2 mL, 25.843 mmol), and stirred at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 30 mg, 0.066 mmol, 1.00 eq) was added and the mixture was stirred to react at room temperature for 1 h. After the reaction was completed, water (10 mL) was added, and the system was extracted with ethyl acetate (20 mL × 2). The organic phases were combined, backwashed with saturated sodium chloride solution (10 mL × 3), dried over anhydrous sodium sulfate, filtered, and the filtrate was concentrated under reduced pressure. The crude product was purified by HPLC with the following conditions (column specifications: XBridge BEH C18 OBD Prep Column 130, 5 μm, 30 mm * 150 mm; mobile phase A: water (17 mmol/L ammonium bicarbonate), mobile phase B: acetonitrile; flow rate: 60 mL/min; elution gradient: 40% B to 56% B in 7 min; detection wavelength: UV 254 nm/220 nm; retention time (min): 6.13), to give compound **5-chloro-*N*-(3-(7-{[(3*S*,4*R*)-3-fluoro-1-methylpiperidin-4-yl]amino}-3-(2,2,2-trifluoroethyl)pyrazolo [1,5-*a*]pyridin-2-yl)prop-2-yn-1-yl)-1-methyl-1*H*-pyrrole-3-carboxamide 326.**

LCMS: (ESI, m/z): 525.05 $[M+H]^+$.

$^1$H NMR (400 MHz, DMSO-$d_6$, *ppm*) $\delta$ 8.43 (t, *J* = 5.7 Hz, 1H), 7.42 (d, *J* = 2.1 Hz, 1H), 7.28 (dd, *J* = 8.7, 7.6 Hz, 1H), 7.02 (d, *J* = 8.6 Hz, 1H), 6.56 (d, *J* = 2.2 Hz, 1H), 6.32 (d, *J* = 7.8, 1H), 6.23 (d, *J* = 9.1 Hz, 1H), 4.87 (d, *J* = 49.5 Hz, 1H), 4.30 (d, *J* = 5.6 Hz, 2H), 3.94 - 3.81 (m, 1H), 3.75 (q, *J* = 11.2 Hz, 2H), 3.59 (s, 3H), 3.05 (t, *J* = 11.4 Hz, 1H), 2.78 (d, *J* = 11.6 Hz, 1H), 2.39 - 2.24 (m, 1H), 2.20 (s, 3H), 2.12 (t, J = 10.7 Hz, 1H), 1.97 - 1.81 (m, 2H).

**Example 82**

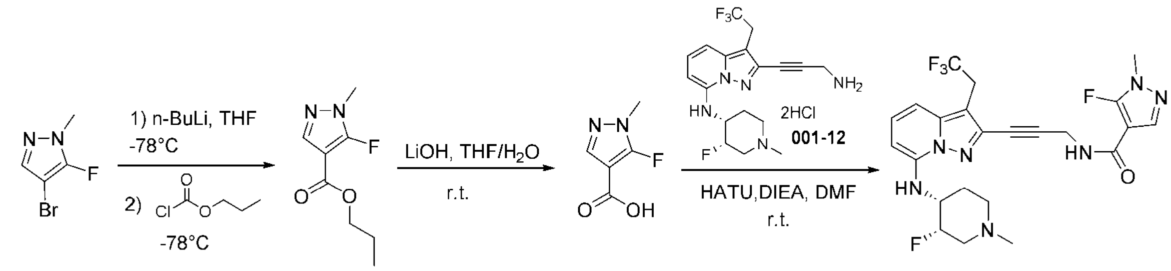


327-1 327-2 327-3 327

**Step 1: Synthesis of compound 327-2**

Under Nitrogen protection, at -78°C, to a solution of 4-bromo-5-fluoro-1-methyl-1*H*-pyrazole (compound **327-1,** 200 mg, 1.117 mmol, 1 eq) in tetrahydrofuran (5 mL) was added dropwise a solution of 1 mol/L n-butyllithium in n-hexane (0.9 mL), the mixture was stirred to react for 30 min, then a solution of propyl chlorocarbonate (123.24 mg, 1.005 mmol, 0.9 eq) in tetrahydrofuran (1 mL) was added dropwise at -78°C, and the mixture was stirred to react for 1 h at -78°C. After the reaction was completed, the reaction mixture was quenched with ammonium chloride solution, and extracted with ethyl acetate (3 × 10 mL). The organic phases were combined, backwashed with saturated sodium chloride solution (3 × 10 mL), dried over anhydrous sodium sulfate, filtered, and the filtrate was concentrated under reduced pressure to give compound **327-2** (60 mg, 28.84%), which was not further purified and was directly used in the next step.

LCMS: (ESI, m/z): 187.05 $[M+H]^+$.

**Step 2: Synthesis of compound 327-3**

At room temperature, a solution of propyl 5-fluoro-1-methyl-1*H*-pyrazole-4-carboxylate (compound **327-2,** 40 mg, 0.215 mmol, 1 eq) and lithium hydroxide (5.15 mg, 0.215 mmol, 1 eq) in tetrahydrofuran (1 mL) and water (1 mL) was stirred to react for 6 h. After the reaction was completed, the reaction mixture was acidified with 1 mol/L hydrochloric acid to pH=5, then concentrated in vacuum to give compound **327-3** (25 mg, 80.56%), which was not further purified and was directly used in the next step.

**Step 3: Synthesis of 5-fluoro-*N*-[3-(7-{[(3*S*,4*R*)-3-fluoro-1-methylpiperidin-4-yl]amino}-3-(2,2,2-trifluoroethyl) pyrazolo[1,5-*a*]pyridin-2-yl)prop-2-yn-1-yl]-1-methyl-1*H*-pyrazole-4-carboxamide (compound 327)**

At room temperature, a solution of 5-fluoro-1-methyl-1*H*-pyrazole-4-carboxylic acid (compound **327-3,** 6.32 mg, 0.044 mmol, 1 eq), O-(7-azabenzotriazol-1-yl)-*N,N,N',N'*-tetramethyluronium hexafluorophosphate (16.67 mg, 0.044 mmol, 1 eq) and *N,N*-diisopropylethylamine (28.32 mg, 0.220 mmol, 5 eq) in *N,N*-dimethylformamide (1 mL) was stirred to react for 30 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.044 mmol, 1 eq) was added and the system was further stirred to react for 2 h. After the reaction was completed, the reaction mixture was quenched with water at room temperature, and extracted with ethyl acetate (3 × 30 mL). The organic phases were combined, backwashed with saturated sodium chloride solution (3 × 30 mL), dried over anhydrous sodium sulfate, filtered, and the filtrate was concentrated under reduced pressure. The crude product was purified by HPLC with the following conditions (column specifications: Sunfire C18 5 μm, 30 mm × 150 mm; mobile phase A: water (0.1% ammonium bicarbonate), mobile phase B: acetonitrile; flow rate: 60 mL/min; elution gradient: 30% to 44% in 8 min; detection wavelength: UV 254 nm/220 nm; retention time (min): 6.95), to give compound **5-fluoro-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1-methyl-1*H*-pyrazole-4-carboxamide 327** (10.61 mg, 46.90%).

LCMS: (ESI, m/z): 510.00 $[M+H]^+$.

$^1$H NMR (400 MHz, DMSO-$d_6$, *ppm*) δ 8.63 (t, *J* = 5.5 Hz, 1H), 7.86 (d, *J* = 2.5 Hz, 1H), 7.29 (t, *J* = 8.1 Hz, 1H), 7.03 (d, *J* = 8.7 Hz, 1H), 6.32 (d, *J* = 7.6 Hz, 1H), 6.24 (d, *J* = 9.0 Hz, 1H), 4.88 (d, *J* = 49.4 Hz, 1H), 4.31 (d, *J* = 5.5 Hz, 2H), 3.94 - 3.81 (m, 1H), 3.81 - 3.68 (m, 5H), 3.06 (t, *J* = 11.6 Hz, 1H), 2.79 (d, *J* = 11.3 Hz, 1H), 2.36 - 2.24 (m, 1H), 2.21 (s, 3H), 2.14 (t, *J* = 11.4 Hz, 1H), 2.01 - 1.88 (m, 1H), 1.87 - 1.78 (m, 1H).

**Example 83**

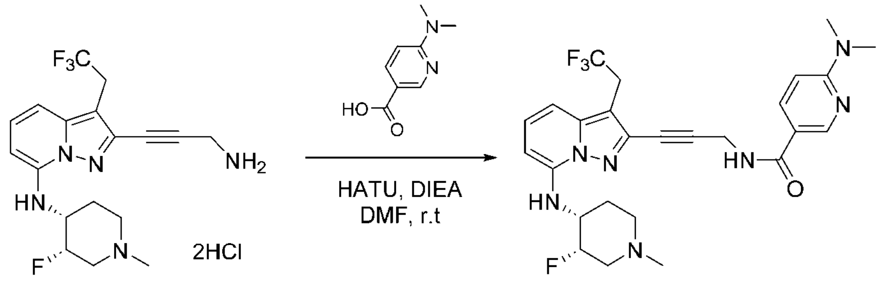


**001-12** **328**

**Step 1: Synthesis of 6-dimethylamino-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]pyridine-3-carboxamide (compound 328)**

Under nitrogen protection, to a solution of O-(7-azabenzotriazol-1-yl)-*N,N,N',N'*-tetramethyluronium hexafluorophosphate (16.67 mg, 0.044 mmol, 1 eq) and 6-(dimethylamino)pyridine-3-carboxylic acid (10.93 mg, 0.066 mmol, 1.5 eq) in *N,N*-dimethylformamide (0.8 mL) was added dropwise *N,N*-diisopropylethylamine (28.32 mg, 0.220 mmol, 5 eq), and the mixture was stirred to react for 1 h. Then, 2-(3-aminoprop-1-yn-1-yl)-N-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.044 mmol, 1 eq) was added and the system was further stirred for 1 h. After the reaction was completed, methanol (1.0 mL) was added to quench the reaction at room temperature. The crude product was purified by HPLC with the following conditions (column specifications: XBridge BEH C18 OBD Prep Column 130, 5 μm, 30 mm × 150 mm; mobile phase A: water (17 mmol/L ammonium bicarbonate), mobile phase B: acetonitrile; flow rate: 60 mL/min; elution gradient: 35% B to 55% B in 7 min; detection wavelength: UV 254 nm/220 nm; retention time (min): 6.12), to give compound **6-dimethylamino-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]pyridine-3-carboxamide 328** (12.04 mg, 51.42%).

LCMS: (ESI, m/z): 532.10 $[M+H]^+$.

$^1$H NMR (400 MHz, DMSO-$d_6$, *ppm*) $\delta$ 8.81 (t, *J* = 5.5 Hz, 1H), 8.63 (d, J = 2.3 Hz, 1H), 7.97 (dd, *J* = 9.0, 2.5 Hz, 1H), 7.28 (dd, *J* = 8.8, 7.6 Hz, 1H), 7.02 (d, *J* = 8.7 Hz, 1H), 6.67 (dd, *J* = 9.0, 0.7 Hz, 1H), 6.32 (d, J = 7.4 Hz, 1H), 6.24 (d, *J* = 9.1 Hz, 1H), 4.87 (d, *J* = 49.4 Hz, 1H), 4.36 (d, *J* = 5.5 Hz, 2H), 3.93 - 3.82 (m, 1H), 3.76 (q, *J* = 11.1 Hz, 2H), 3.09 (s, 6H), 3.05 (t, J = 10.8 Hz, 1H), 2.78 (d, *J* = 11.4 Hz, 1H), 2.37 - 2.21 (m, 1H), 2.20 (s, 3H), 2.13 (t, *J* = 10.9 Hz, 1H), 1.99 - 1.80 (m, 2H).

**Example 84**

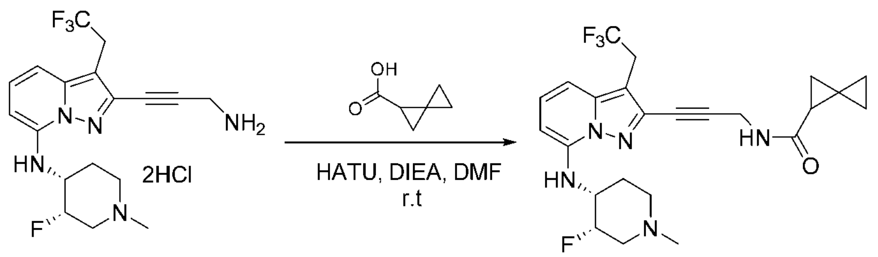


**001-12** **241**

**Step 1: Synthesis of *N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]spiro[2.2]pentane-1-carboxamide (compound 241)**

At room temperature, to a solution of spiro[2.2]pentane-1-carboxylic acid (14.62 mg, 0.130 mmol, 1 eq) in *N,N*-dimethylformamide (1 mL) were added O-(7-azabenzotriazol-1-yl)-*N,N,N',N'*-tetramethyluronium hexafluorophosphate (49.59 mg, 0.130 mmol, 1 eq) and diisopropylethylamine (0.09 mL, 0.520 mmol, 4 eq), and the mixture was stirred to react for 30 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 50 mg, 0.130 mmol, 1 eq) was added and the mixture was stirred to react at room temperature for 1 h. After the reaction was completed, the reaction mixture was diluted with water (20 mL) and the aqueous phase was extracted with ethyl acetate (3 × 20 mL). The organic phases were combined, backwashed with saturated brine (3 × 20 mL), dried over anhydrous sodium sulfate, filtered, and the filtrate was

concentrated under reduced pressure. The crude product was purified by HPLC with the following conditions (column specifications: XBridge BEH C18 OBD Prep Column 130, 5 μm, 30 mm * 150 mm; mobile phase A: water (17 mmol/L ammonium bicarbonate), mobile phase B: acetonitrile; flow rate: 60 mL/min; elution gradient: 36% B to 56% B in 8 min; detection wavelength: UV 254 nm/220 nm; retention time (min): 6.68), to give compound ***N*-[3-(7-{[(3*S*,4*R*)-3-fluoro-1-methylpiperidin-4-yl] amino}-3-(2,2,2-trifluoroethyl)pyrazolo [1,5-*a*]pyridin-2-yl)prop-2-yn-1-yl]spiro[2.2]pentane-1-carboxamide 241** (1.7 mg, 2.71%).

LCMS: (ESI, m/z): 478.10 $[M+H]^+$.

$^1$H NMR (400 MHz, DMSO-$d_6$, *ppm)* δ 8.46 (t, *J* = 5.5, 5.5 Hz, 1H), 7.29 (t, *J* = 8.2, 8.2 Hz, 1H), 7.03 (d, *J* = 8.7 Hz, 1H), 6.33 (d, *J* = 7.6 Hz, 1H), 6.23 (d, *J* = 9.1 Hz, 1H), 4.88 (d, *J* = 49.5 Hz, 1H), 4.11 - 4.27 (m, 2H), 3.94 - 3.80 (m, 1H), 3.76 (q, J = 11.1 Hz, 2H), 3.06 (t, *J* = 11.3, 11.3 Hz, 1H), 2.79 (d, J = 11.7 Hz, 1H), 2.37 - 2.24 (m, 1H), 2.21 (s, 3H), 2.14 (t, *J* = 10.9, 1H), 1.98 - 1.90 (m, 2H), 1.81 - 1.88 (m, 1H), 1.31 (t, *J* = 3.9, 1H), 1.22 (dd, J = 7.5, 3.3 Hz, 1H), 0.90 - 0.76 (m, 3H), 0.75 - 0.66 (m, 1H).

**Example 85**

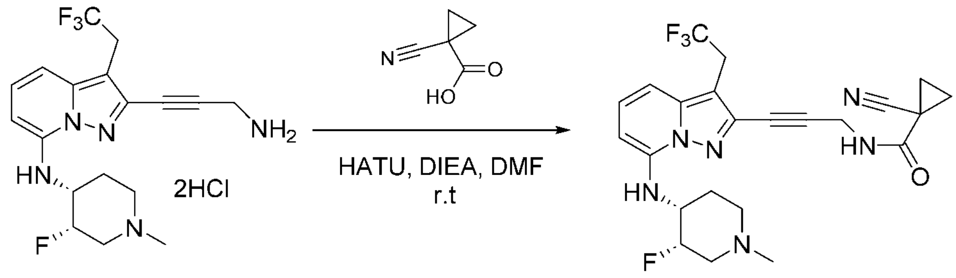


**001-12** **238**

**Step 1: Synthesis of 1-cyano-*N*-[3-(7-{[(3*S*,4*R*)-3-fluoro-1-methylpiperidin-4-yl]amino}-3-(2,2,2-trifluoroethyl) pyrazolo[1,5-*a*]pyridin-2-yl)prop-2-yn-1-yl]cyclopropane-1-carboxamide (compound 238)**

At room temperature, to a solution of 1-cyanocyclopropane-1-carboxylic acid (38.96 mg, 0.352 mmol, 8 eq) in *N,N*-dimethylformamide (0.5 mL) were added O-(7-azabenzotriazol-1-yl)-*N,N,N',N'*-tetramethyluronium hexafluorophosphate (41.66 mg, 0.110 mmol, 2.5 eq) and diisopropylethylamine (45.32 mg, 0.352 mmol, 8 eq), and the mixture was stirred to react for 30 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12**, 20 mg, 0.044 mmol, 1 eq) was added and the mixture was further stirred to react for 1 h. After the reaction was completed, the reaction mixture was diluted with water (20 mL) and the aqueous phase was extracted with ethyl acetate (3 × 20 mL). The organic phases were combined, backwashed with saturated brine (3 × 20 mL), dried over anhydrous sodium sulfate, filtered, and the filtrate was concentrated under reduced pressure. The crude product was purified by HPLC with the following conditions (column specifications: YMC Triart C18 ExRs 5 μm, 30 mm * 150 mm; mobile phase A: water (10 mmol/L ammonium bicarbonate), mobile phase B: acetonitrile; flow rate: 60 mL/min; elution gradient: 40% B to 60% B in 10 min; detection wavelength: UV 254 nm/220 nm; retention time (min): 7.83), to give compound **1-cyano-N-[3-(7-{[(3S,4R)-3-fluoro-1-methylpiperidin-4-yl]amino}-3-(2,2,2-trifluoroethyl)pyrazolo[1,5-*a*]pyridin-2-yl)prop-2-yn-1-yl]cyclopropane-1-carboxamide 238** (8.1 mg, 38.67%).

LCMS: (ESI, m/z): 477.00 $[M+H]^+$.

$^1$H NMR (400 MHz, DMSO) δ 8.84 (t, J = 5.2 Hz, 1H), 7.29 (t, J = 8.1 Hz, 1H), 7.03 (d, J = 8.7 Hz, 1H), 6.33 (d, J = 7.6 Hz, 1H), 6.24 (d, J = 9.0 Hz, 1H), 4.88 (d, J = 49.8 Hz, 1H), 4.20 (d, J = 5.4 Hz, 2H), 3.95 - 3.80 (m, 1H), 3.74 (q, J = 11.1 Hz, 2H), 3.06 (t, J = 11.0 Hz, 1H), 2.79 (d, J = 10.9 Hz, 1H), 2.29 (dd, J = 38.4, 12.9 Hz, 1H), 2.19 (s, J = 15.6 Hz, 3H), 2.14 (t, J = 10.9 Hz, 1H), 2.00 - 1.80 (m, 2H), 1.67 - 1.58 (m, 2H), 1.57 - 1.50 (m, 2H).

**Example 86**

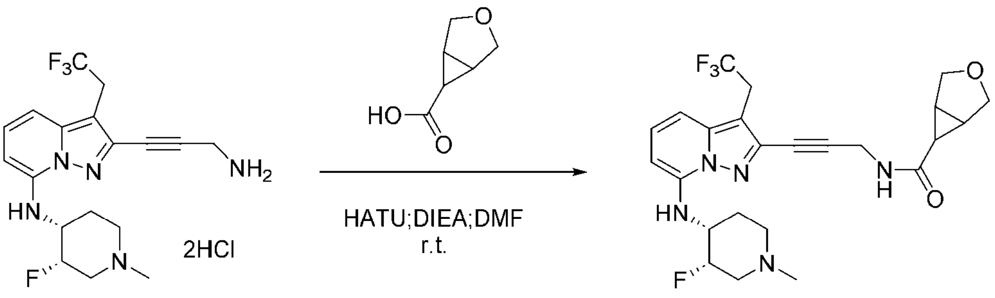


001-12 240

**Step 1: Synthesis of N-[3-(7-{[(3*S*,4*R*)-3-fluoro-1-methylpiperidin-4-yl]amino}-3-(2,2,2-trifluoroethyl)pyrazolo[1,5-a]pyridin-2-yl)prop-2-yn-1-yl]-3-oxabicyclo[3.1.0]hexane -6-carboxamide (compound 240)**

At room temperature, a solution of 3-oxabicyclo[3.1.0]hexane-6-carboxylic acid (6.74 mg, 0.053 mmol, 1.2 eq), *N,N*-diisopropylethylamine (28.32 mg, 0.220 mmol, 5 eq), and O-(7-azabenzotriazol-1-yl)-*N,N,N',N'*-tetramethyluronium hexafluorophosphate (16.67 mg, 0.044 mmol, 1 eq) in N,N-dimethylformamide (2 mL) was added to a reaction flask and stirred at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.044 mmol, 1 eq) was added, and the system was stirred at room temperature for 1 h. After the reaction was completed, the reaction mixture was diluted with ethyl acetate (5 mL), washed with saturated sodium chloride solution (10 mL × 3), dried over anhydrous sodium sulfate, filtered, and the filtrate was concentrated under reduced pressure. The crude product was purified by HPLC with the following conditions (column specifications: Kinetex 5 μm EVO C18, 30mm * 150 mm; mobile phase A: water (10 mmol/L ammonium bicarbonate), mobile phase B: acetonitrile; flow rate: 60 mL/min; elution gradient: 45% B to 60% B in 7 min; detection wavelength: UV 254 nm/220 nm; retention time (min): 6.53), to give compound ***N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl) prop-2-yn-1-yl]-3-oxabicyclo[3.1.0]hexane -6-carboxamide 240** (3.7 mg, 16.87%).

LCMS: (ESI, m/z): 494.10 $[M+H]^+$.

$^1H$ NMR (400 MHz, DMSO-$d_6$, *ppm*) δ 8.64 (t, *J* = 5.4 Hz, 1H), 7.29 (t, *J* =8.2 Hz, 1H), 7.03 (d, *J* = 8.7 Hz, 1H), 6.33 (d, *J* = 7.6 Hz, 1H), 6.23 (d, *J* = 9.0 Hz, 1H), 4.88 (d, *J* = 49.5 Hz, 1H), 4.20 (d, *J* = 5.6 Hz, 2H), 4.00 - 3.82 (m, 1H), 3.81 - 3.68 (m, 4H), 3.63 (d, *J* = 8.5 Hz, 2H), 3.06 (t, *J* = 11.6 Hz, 1H), 2.79 (d, *J* = 11.4 Hz, 1H), 2.37 - 2.24 (m, 1H), 2.21 (s, 3H), 2.18 - 2.09 (m, 1H), 2.05 - 1.81 (m, 4H), 1.50 (t, *J* = 3.3 Hz, 1H).

**Example 87**

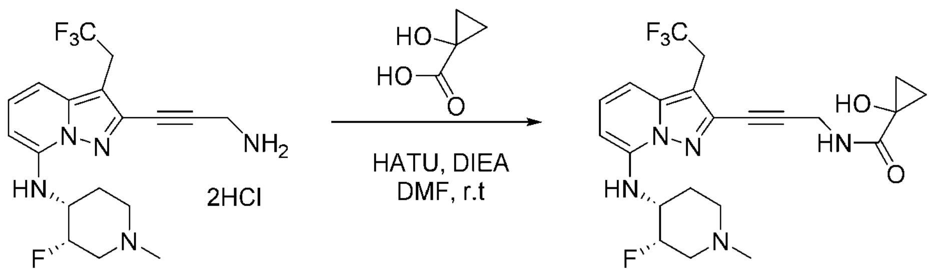


001-12 239

**Step 1: Synthesis of N-[3-(7-{[(3*S*,4*R*)-3-fluoro-1-methylpiperidin-4-yl]amino}-3-(2,2,2-trifluoroethyl)pyrazolo[1,5-a]pyridin-2-yl)prop-2-yn-1-yl]-1-hydroxycyclopropane-1-carboxamide (compound 239)**

Under nitrogen protection, at room temperature, to a solution of O-(7-azabenzotriazol-1-yl)-*N,N,N',N'*-tetramethyluronium hexafluorophosphate (19.84 mg, 0.052 mmol, 1 eq) and 1-hydroxycyclopropane-1-carboxylic acid (6.71 mg, 0.066 mmol, 1.5 eq) in*N,N*-dimethylformamide (0.8 mL) was added *N,N*-diisopropylethylamine (33.71 mg, 0.260 mmol, 5 eq). The mixture was stirred to react for 10 min, and then 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.052 mmol, 1 eq) was added and the mixture was allowed to further react for 1 h. After the reaction was completed, the

reaction mixture was quenched at room temperature with methanol (1.0 mL). The crude product was purified by HPLC with the following conditions (column specifications: Xselect CSHTM Prep C18 5μm 30 × 150mm OBD; mobile phase A: water (0.1% formic acid), mobile phase B: acetonitrile; flow rate: 100 mL/min; elution gradient: 5% B to 25% B in 9 min; detection wavelength: UV 254 nm/220 nm; retention time (min): 9.43), to give compound ***N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1-hydroxycyclopropane-1-carboxamide 239** (2.98 mg, 9.31%).

LCMS: (ESI, m/z): 468.05 $[M+H]^+$.

$^1H$ NMR (400 MHz, DMSO-$d_6$, *ppm)* $\delta$ 8.49 (t, *J* = 5.9 Hz, 1H), 7.33 - 7.26 (m, 1H), 7.03 (d, *J* = 8.7 Hz, 1H), 6.32 (d, *J* = 7.6 Hz, 1H), 6.27 (d, *J* = 9.0 Hz, 1H), 4.90 (d, *J* = 49.4 Hz, 1H), 4.21 (d, *J* = 5.9 Hz, 2H), 3.96 - 3.81 (m, 1H), 3.75 (q, *J* = 11.2 Hz, 2H), 3.12 (t, *J* = 11.9 Hz, 1H), 2.88 - 2.79 (m, 1H), 2.46 - 2.29 (m, 1H), 2.25 (s, 3H), 2.21 (t, J = 11.3 Hz, 1H), 2.02 - 1.82 (m, 2H), 1.13 - 0.99 (m, 2H), 0.93 - 0.81 (m, 2H).

**Example 88**

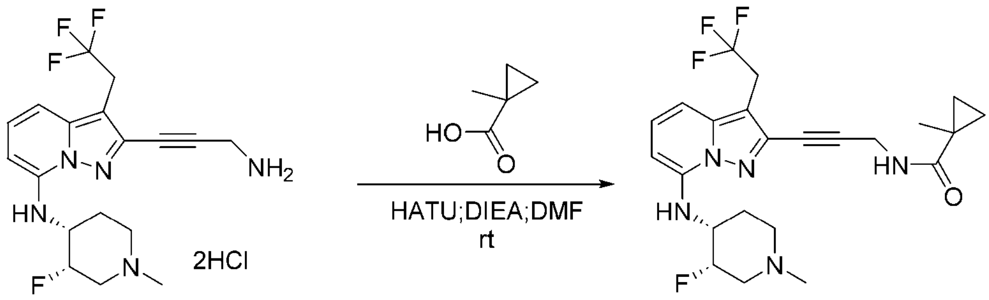


**001-12** **237**

**Step 1: Synthesis of *N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1-methylcyclopropane-1-carboxamide (compound 237)**

At room temperature, a solution of 1-methylcyclopropane-1-carboxylic acid (5.27 mg, 0.053 mmol, 1.2 eq), *N,N*-diisopropylethylamine (28.32 mg, 0.220 mmol, 5 eq), and O-(7-azabenzotriazol-1-yl)-*N,N,N',N'*-tetramethyluronium hexafluorophosphate (16.67 mg, 0.044 mmol, 1 eq) in *N,N*-dimethylformamide (2 mL) was stirred for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12**, 20 mg, 0.044 mmol, 1 eq) was added, and the system was further stirred to react for 1 h. After the reaction was completed, the reaction mixture was diluted with ethyl acetate (5 mL), washed with saturated sodium chloride solution (10 mL × 3), dried over anhydrous sodium sulfate, filtered, and the filtrate was concentrated under reduced pressure. The crude product was purified by HPLC with the following conditions (column specifications: XBridge BEH Shield RP18 5 μm, 30 mm × 151 mm; mobile phase A: water (10 mmol/L ammonium bicarbonate), mobile phase B: acetonitrile; flow rate: 61 mL/min; elution gradient: 35% B to 55% B in 7 min; detection wavelength: UV 254 nm/220 nm; retention time (min): 7.53), to give compound ***N*-[3-(7-{[(3*S*,4*R*)-3-fluoro-1-methylpiperidin-4-yl]amino)-3-(2,2,2-trifluoroethyl)pyrazolo[1,5-*a*]pyridin-2-yl)prop-2-yn-1-yl]-1-methylcyclopropane-1-carboxamide 237** (3.98 mg, 18.58%).

LCMS: (ESI, m/z): 466.10 $[M+H]^+$.

$^1H$ NMR (400 MHz, DMSO-$d_6$, ppm) $\delta$ 8.13 (d, *J* = 7.3 Hz, 1H), 7.29 (t, *J* = 8.1 Hz, 1H), 7.02 (d, *J* = 8.7 Hz, 1H), 6.32 (d, *J* = 7.6 Hz, 1H), 6.24 (d, *J* = 9.0 Hz, 1H), 4.88 (d, *J* = 49.6 Hz, 1H), 4.16 (d, *J* = 5.1 Hz, 2H), 3.96 - 3.81 (m, 1H), 3.75 (q, J = 10.7 Hz, 2H), 3.08 (t, *J* = 12.1 Hz, 1H), 2.79 (d, *J* = 10.8 Hz, 1H), 2.38 - 2.24 (m, 1H), 2.21 (s, 3H), 2.12 (t, *J* = 11.3 Hz, 1H), 2.05 - 1.77 (m, 2H), 1.27 (s, 3H), 1.05 - 0.93 (m, 2H), 0.61 - 0.48 (s, 2H).

**Example 89**

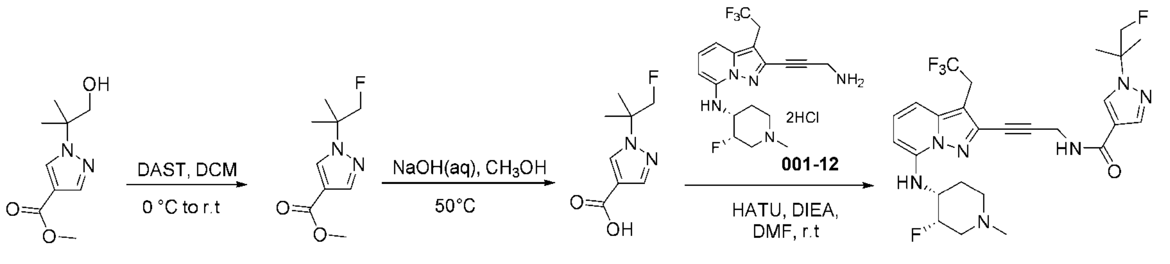


234-1 234-2 234-3 234

**Step 1: Synthesis of compound 234-2**

Methyl 1-(1-hydroxy-2-methyl-2-yl)-1*H*-pyrazole-4-carboxylate (compound **234-1**, 100 mg, 0.504 mmol, 1 eq) was added to a reaction flask and dissolved in dichloromethane (2 mL). Then, diethylaminosulphur trifluoride (162.64 mg, 1.008 mmol, 2 eq) was added at 0°C, and the mixture was warmed room temperature to react for 1 h. After the reaction was completed, saturated ammonium chloride solution was added for quenching. The reaction mixture was extracted with ethyl acetate (3 × 10 mL). The organic phases were combined, backwashed with saturated sodium chloride solution (1 × 10 mL), dried over anhydrous sodium sulfate, and filtered. The filtrate was concentrated under reduced pressure. The resulting residue was purified by reverse phase column chromatography with the following conditions (mobile phase: water (1% formic acid) and acetonitrile, 20% to 60% gradient in 10 min, UV 220 nm) to give compound **234-2** (37 mg, 32.97%).

LCMS: (ESI, m/z): 201.40 $[M+H]^+$.

**Step 2: Synthesis of compound 234-3**

Methyl 1-(1-fluoro-2-methyl-2-yl)-1*H*-pyrazole-4-carboxylate (compound **234-2,** 27 mg, 0.135 mmol, 1 eq) was added to a reaction flask, dissolved in methanol (2.7 mL), and then sodium hydroxide (10.79 mg, 0.270 mmol, 2 eq) and water (1 mL) were added, and the reaction was carried out at 50°C for 5 h. After the reaction was completed, the reaction mixture was adjusted to pH 2 with hydrochloric acid (1M), and the solvent was then removed by spin drying to directly give compound **234-3** (30 mg, 95.59%).

LCMS: (ESI, m/z): 187.05 $[M+H]^+$.

**Step 3: Synthesis of *N*-[3-(7-{[(3*S*,4*R*)-3-fluoro-1-methylpiperidin-4-yl]amino}-3-(2,2,2-trifluoroethyl)pyrazolo [1,5-*a*]pyridin-2-yl)prop-2-yn-1-yl]-1-(1-fluoro-2-methylpropan-2-yl)-1*H*-pyrazole-4-carboxamide (compound 234)**

1-(1-fluoro-2-methyl-2-yl)-1*H*-pyrazole-4-carboxylic acid (compound **234-3,** 30 mg, 0.161 mmol, 1 eq), 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 61.78 mg, 0.161 mmol, 1 eq), O-(7-azabenzotriazol-1-yl)-*N,N,N',N'*-tetramethyluronium hexafluorophosphate (91.9 mg, 0.241 mmol, 1.5 eq) and *N,N*-diisopropylethylamine (104.13 mg, 0.805 mmol, 5 eq) were added to a reaction flask, dissolved in *N,N*-dimethylformamide (3 mL) and allowed to react at room temperature for 1 h. After the reaction was completed, water was added for quenching, and the reaction mixture was extracted with ethyl acetate (3 × 10 mL). The organic phases were combined, backwashed with saturated sodium chloride solution (1 × 10 mL), dried over anhydrous sodium sulfate, filtered, and the filtrate was concentrated under reduced pressure. The crude product was purified by HPLC with the following conditions (column specifications: YMC Triart C18 ExRs 5 μm, 20 mm *250 mm; mobile phase A: water (10 mmol/L ammonium bicarbonate), mobile phase B: acetonitrile; flow rate: 25 mL/min; gradient: 45% B to 61% B in 8 min; wavelength: UV 254 nm/220 nm; retention time (min): 7.32), to give compound ***N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl) prop-2-yn-1-yl]-1-(1-fluoro-2-methylpropan-2-yl)-1*H*-pyrazole-4-carboxamide 234** (11.05 mg, 12.21%).

LCMS: (ESI, m/z): 552.15 $[M+H]^+$.

$^1H$ NMR (400 MHz, DMSO-$d_6$, *ppm)* δ 8.74 (t, *J* = 5.6 Hz, 1H), 8.17 (s, 1H), 7.92 (s, 1H), 7.32 - 7.24 (m, 1H), 7.02 (d, *J* = 8.7 Hz, 1H), 6.32 (d, *J* = 7.2 Hz, 1H), 6.24 (d, *J* = 9.0 Hz, 1H), 4.87 (d, *J* = 49.5 Hz, 1H), 4.42 - 4.28 (m, 4H), 3.92 - 3.81 (m, 1H), 3.75 (q, *J* = 11.2 Hz, 2H), 3.05 (t, *J* = 11.6 Hz, 1H), 2.78 (d, *J* = 11.2 Hz, 1H), 2.36 - 2.24 (m, 1H), 2.20 (s, 3H), 2.13 (t, *J* = 11.0 Hz, 1H), 1.99 - 1.80 (m, 2H), 1.28 (d, *J* = 21.4 Hz, 6H).

**Example 90**

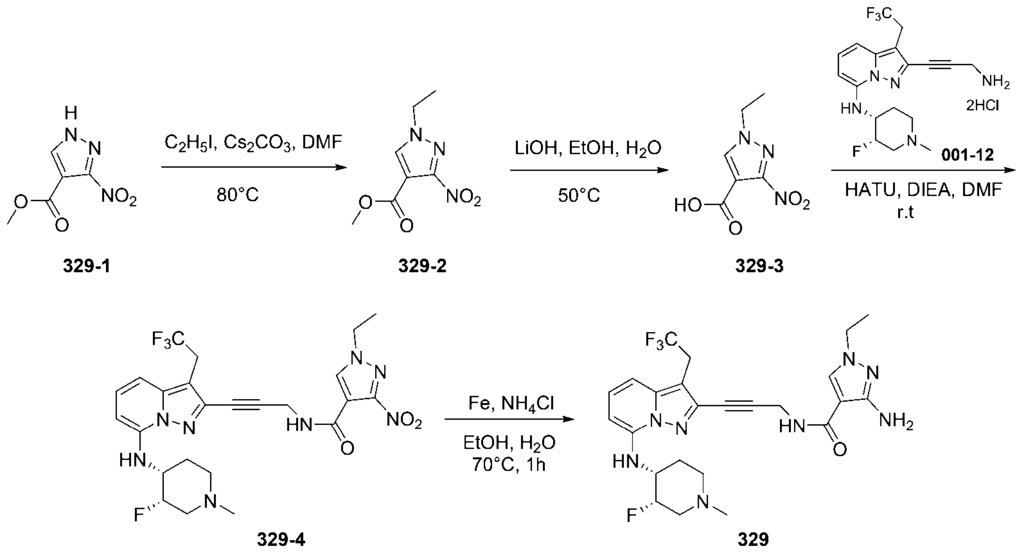


329-1 329-2 329-3

329-4 329

**Step 1: Synthesis of compound 329-2**

Under nitrogen protection, methyl 3-nitro-1*H*-pyrazole-4-carboxylate (compound **329-1**, 500 mg, 2.922 mmol, 1 eq), and cesium carbonate (1.90 g, 5.844 mmol, 2 eq) were added to a reaction flask and dissolved in *N,N*-dimethylformamide (5 mL). Then, iodoethane (683.61 mg, 4.383 mmol, 1.5 eq) was added and the mixture was warmed to 75°C and stirred to react for 1 h. After the reaction was completed, water (15 mL) was added and the reaction mixture was extracted with ethyl acetate (30 mL × 2). The organic phases were combined, backwashed with saturated sodium chloride solution (30 mL × 1), dried over anhydrous sodium sulfate and filtered. The filtrate was concentrated under reduced pressure. The resulting residue was purified by silica gel column chromatography (petroleum ether/ethyl acetate = 4:1) to give compound **329-2** (300 mg, 47.17%).

LCMS: (ESI, m/z): 200.05 $[M+H]^+$.

**Step 2: Synthesis of compound 329-3**

Under nitrogen protection, methyl 1-ethyl-3-nitro-1H-pyrazole-4-carboxylate (compound 329-2, 300 mg, 1.506 mmol, 1 eq), and lithium hydroxide (72.15 mg, 3.012 mmol, 2 eq) were added to a reaction flask, dissolved in anhydrous ethanol (3 mL) and water (3 mL), and warmed to 50°C and stirred to react for 1 h. After the reaction was completed, the reaction mixture was concentrated under reduced pressure. To the crude product was added 5 mL of tetrahydrofuran, the system was stirred for 10 min, and then filtered, and the filtrate was concentrated under reduced pressure to give compound **329-3** (210 mg, 75.15%).

LCMS: (ESI, m/z): 185.95 $[M+H]^+$.

**Step 3: Synthesis of compound 329-4**

Under nitrogen protection, 1-ethyl-3-nitro-1H-pyrazole-4-carboxylic acid (compound **329-3,** 18.26 mg, 0.099 mmol, 1.5 eq), 2-(7-azabenzotriazole)-*N,N,N',N'*-tetramethyluronium hexafluorophosphate (25.0 mg, 0.066 mmol, 1.0 eq), and *N,N*-diisopropylethylamine (42.49 mg, 0.330 mmol, 5 eq) were added to a reaction flask, dissolved in *N,N*-dimethylformamide (5 mL) and stirred at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 30 mg, 0.066 mmol, 1.00 eq) was added and the mixture was stirred to react at room temperature for 1 h. After the reaction was completed, water (10 mL) was added and the reaction mixture was extracted with ethyl acetate (20 mL × 2). The organic phases were combined, backwashed with saturated sodium chloride solution (10 mL × 3), dried over anhydrous sodium sulfate, filtered, and the filtrate was concentrated under reduced pressure. The resulting residue was purified by silica gel column chromatography (dichloromethane/anhydrous methanol = 12:1) to give compound **329-4** (30 mg, 61.25%).

LCMS: (ESI, m/z): 550.95 $[M+H]^+$.

**Step 3: Synthesis of 3-amino-1-ethyl-*N*-(3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1*H*-pyrazole-4-carboxamide (compound 329)**

Under nitrogen protection, 1-ethyl-*N*-(3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl)-3-nitro-1*H*-pyrazole-4-carboxamide (compound **329-4,** 25 mg, 0.045 mmol, 1 eq), reduced iron powder (11.41 mg, 0.202 mmol, 4.5 eq), and ammonium chloride (21.86 mg, 0.405 mmol, 9 eq) were added to a reaction flask, and dissolved in anhydrous ethanol (1 mL) and water (1 mL). The mixture was warmed to 70°C and stirred to react for 1 h. After the reaction was completed, the mixture was filtered, and the filtrate was concentrated under reduced pressure. The crude product was purified by HPLC with the following conditions (column specifications: Kinetex 5 μm EVO C18, 30mm * 150 mm; mobile phase A: water (10 mmol/L ammonium bicarbonate), mobile phase B: acetonitrile; flow rate: 60 mL/min; elution gradient: 28% B to 44% B in 7 min; detection wavelength: UV 254 nm/220 nm; retention time (min): 6.53), to give compound **3-amino-1-ethyl-*N*-(3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1*H*-pyrazole-4-carboxamide 329** (4.78 mg, 19.84%).

LCMS: (ESI, m/z): 520.85 $[M+H]^+$.

$^1H$ NMR (400 MHz, DMSO-$d_6$, *ppm)* $\delta$ 8.31 (t, *J* = 5.6 Hz, 1H), 7.95 (s, 1H), 7.28 (dd, *J* = 8.7, 7.6 Hz, 1H), 7.03 (d, *J* = 8.7 Hz, 1H), 6.32 (d, *J* = 7.6 Hz, 1H), 6.24 (d, *J* = 9.1 Hz, 1H), 5.39 (s, 2H), 4.87 (d, *J* = 49.6 Hz, 1H), 4.29 (d, *J* = 5.6 Hz, 2H), 3.95 - 3.83 (m, 3H), 3.75 (q, *J* = 11.2 Hz, 2H), 3.06 (t, *J* = 11.6 Hz, 1H), 2.78 (d, *J* = 11.4 Hz, 1H), 2.38 - 2.23 (m, 1H), 2.20 (s, 3H), 2.15 (t, J = 10.7 Hz, 1H), 1.98 - 1.80 (m, 2H), 1.31 (t, *J* = 7.2 Hz, 3H).

**Example 91**

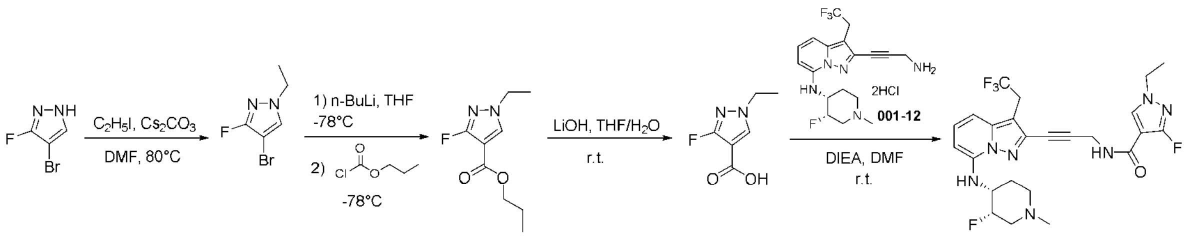


330-1 330-2 330-3 330-4 330

**Step 1: Synthesis of compound 330-2**

Under nitrogen protection, at 80°C, a solution of 4-bromo-3-fluor-1*H*-pyrazole (compound **330-1,** 300 mg, 1.819 mmol, 1 eq), iodoethane (340 mg, 2.183 mmol, 1.2 eq) and cesium carbonate (1.48 g, 4.547 mmol, 2.50 eq) in *N,N*-dimethylformamide (3 mL) was stirred to react for 1 h. After the reaction was completed, the reaction mixture was poured into water (30 mL), and extracted with ethyl acetate (2 × 30 mL). The organic phases were combined, backwashed with saturated sodium chloride (2 × 20 mL), dried over anhydrous sodium sulfate, filtered, and the filtrate was concentrated under reduced pressure. The resulting residue was purified by silica gel column chromatography (dichloromethane/petroleum ether = 5:1), to give compound **330-2** (220 mg, 62.67%).

LCMS: (ESI, m/z): 192.90 $[M+H]^+$.

**Step 2: Synthesis of compound 330-3**

Under nitrogen protection, at -78°C, to a solution of 4-bromo-1-ethyl-3-fluoro-1*H*-pyrazole (compound **330-2,** 30 mg, 0.155 mmol, 1 eq) in tetrahydrofuran (3 mL) was added n-butyl lithium (0.12 mL, 1.274 mmol, 8.20 eq). The mixture was stirred to react for 30 min, then propyl chlorocarbonate (17.14 mg, 0.140 mmol, 0.90 eq) was added dropwise at -78°C, and the reaction was allowed to proceed for 1 h. After the reaction was completed, at room temperature, the reaction mixture was quenched with saturated ammonium chloride aqueous solution and extracted with ethyl acetate (2 × 10 mL). The organic phases were combined, backwashed with saturated sodium chloride solution (2 × 10 mL), dried over anhydrous sodium sulfate, and filtered. The filtrate was concentrated under reduced pressure. The resulting residue was purified by silica gel column chromatography (petroleum ether/ethyl acetate = 10: 1) to give compound **330-3** (30 mg, 16.07%).

LCMS: (ESI, m/z): 201.05 $[M+H]^+$.

**Step 3: Synthesis of compound 330-4**

At room temperature, a solution of propyl 1-ethyl-3-fluoro-1H-pyrazole-4-carboxylate (compound **330-3,** 30 mg, 0.150 mmol, 1 eq) and lithium hydroxide (3.59 mg, 0.150 mmol, 1.00 eq) in tetrahydrofuran (0.5 mL) and water (0.25 mL) was stirred to react overnight. The resulting residue was concentrated under reduced pressure to give compound **330-4** (16 mg, 67.53%).
LCMS: (ESI, m/z): 159.05 $[M+H]^+$.

**Step 4: Synthesis of 1-ethyl-3-fluoro-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1*H*-pyrazole-4-carboxamide (compound 330)**

At room temperature, a solution of 1-ethyl-3-fluoro-1*H*-pyrazole-4-carboxylic acid (compound **330-4,** 8.75 mg, 0.055 mmol, 2.50 eq), O-(7-azabenzotriazol-1-yl)-*N,N,N',N'*-tetramethyluronium hexafluorophosphate (8.42 mg, 0.022 mmol, 1.00 eq) and *N,N*-diisopropylethylamine (11.44 mg, 0.088 mmol, 4.00 eq) in *N,N*-dimethylformamide (0.5 mL) was stirred to react for 20 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 10.1 mg, 0.022 mmol, 1.00 eq) was added and the system was further stirred to react for 1 h. After the reaction was completed, the reaction mixture was quenched with water (5 mL) at room temperature, and extracted with ethyl acetate (2 × 10 mL). The organic phases were combined, backwashed with sodium chloride aqueous solution (2 × 10 mL), dried over anhydrous sodium sulfate, filtered, and the filtrate was concentrated under reduced pressure. The crude product was purified by HPLC with the following conditions (column specifications: XBridge BEH Shield RP18 5 μm, 30 mm × 151 mm; mobile phase A: water (10 mmol/L ammonium bicarbonate), mobile phase B: acetonitrile; flow rate: 61 mL/min; elution gradient: 4% B to 61% B in 7 min; detection wavelength: UV 254 nm/220 nm; retention time (min): 7.55), to give compound **1-ethyl-3-fluoro-N-[3-(7-{[(3S,4R)-3-fluoro-1-methylpiperidin-4-yl]amino}-3-(2,2,2-trifluoroethyl)pyrazolo[1,5-*a*]pyridin-2-yl)prop-2-yn-1-yl]-1*H*-pyrazole-4-carboxamide 330** (1.72 mg, 14.58%).
LCMS: (ESI, m/z): 523.85 $[M+H]^+$.
$^1$H NMR (400 MHz, DMSO-$d_6$, *ppm)* $\delta$ 9.21 *(t, J* = 5.6 Hz, 1H), 7.29 (dd, *J* = 8.7, 7.6 Hz, 1H), 7.03 (d, *J* = 8.6 Hz, 1H), 6.57 (d, *J* = 5.9 Hz, 1H), 6.33 (d, *J* = 7.6 Hz, 1H), 6.22 (d, *J* = 9.0 Hz, 1H), 4.87 (d, *J* = 52.0 Hz, 1H), 4.42 (q, J = 7.1 Hz, 2H), 4.37 (d, J = 5.5 Hz, 2H), 3.93 - 3.81 (m, 1H), 3.76 (q, J = 11.1 Hz, 2H), 3.06 (t, J = 11.6 Hz, 1H), 2.78 (d, *J* = 11.3 Hz, 1H), 2.38 - 2.23 (m, 1H), 2.20 (s, 3H), 2.12 (t, *J* = 10.8 Hz, 1H), 1.98 - 1.84 (m, 2H), 1.30 (t, *J* = 7.1 Hz, 3H).

**Example 92**

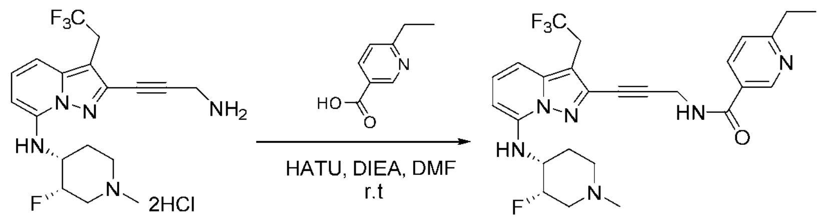


**001-12** **331**

**Step 1: Synthesis of 6-ethyl-*N*-[3-(7-{[(3*S*,4*R*)-3-fluoro-1-methylpiperidin-4-yl]amino}-3-(2,2,2-trifluoroethyl) pyrazolo[1,5-*a*]pyridin-2-yl)prop-2-yn-1-yl]pyridine-3-carboxamide (compound 331)**

At room temperature, to a solution of 6-ethylpyridine-3-carboxylic acid (9.94 mg, 0.066 mmol, 1 eq) in *N,N*-dimethylformamide (1 mL) were added O-(7-azabenzotriazol-1-yl)-*N,N,N',N'*-tetramethyluronium hexafluorophosphate (25.0 mg, 0.066 mmol, 1 eq) and diisopropylethylamine (0.05 mL, 0.264 mmol, 4 eq), and the mixture was stirred to react for 30 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo [1,5-*a*]pyridin-7-amine dihydrochloride (compound **001-12**, 30 mg, 0.066 mmol, 1 eq) was added and the mixture was further stirred to react for 1 h. After the reaction was completed, the reaction mixture was diluted with water (20 mL) and extracted with ethyl acetate (3 × 20 mL). The organic phases were combined, backwashed with saturated brine (3 × 20 mL), dried over anhydrous sodium sulfate, filtered, and the filtrate was concentrated under reduced pressure. The crude product was purified by HPLC with the following conditions (column specifications: Kinetex 5 μm EVO C18, 30mm * 150 mm; mobile phase A: water (10 mmol/L ammonium bicarbonate), mobile phase B: acetonitrile; flow rate: 60 mL/min; elution gradient: 33% B to 49% B in 8 min; detection wavelength: UV 254 nm/220 nm; retention time (min): 7.17), to give

compound **6-ethyl-*N*-[3-(7-{[(3*S*,4*R*)-3-fluoro-1-methylpiperidin-4-yl] amino}-3-(2,2,2-trifluoroethyl)pyrazolo[1,5-*a*]]pyridin-2-yl)prop-2-yn-1-yl]pyridine-3-carboxamide 331** (12.29 mg, 36.08%).

LCMS: (ESI, m/z): 516.85 $[M+H]^+$.

$^1$H NMR (400 MHz, DMSO-$d_6$, *ppm)* δ 9.21 (t, *J* = 5.5, 5.5 Hz, 1H), 8.95 (d, *J* = 1.8 Hz, 1H), 8.14 (dd, *J* = 8.1, 2.4 Hz, 1H), 7.39 (d, *J* = 8.1 Hz, 1H), 7.29 (dd, *J* = 8.8, 7.6 Hz, 1H), 7.03 (d, *J* = 8.7 Hz, 1H), 6.32 (d, J = 7.5 Hz, 1H), 6.21 (d, *J* = 9.0 Hz, 1H), 4.87 (d, *J* = 49.5 Hz, 1H), 4.41 (d, *J* = 5.5 Hz, 2H), 3.93 - 3.82 (m, 1H), 3.76 (q, *J* = 11.1 Hz, 2H), 3.06 (t, *J* = 11.5, 11.5 Hz, 1H), 2.86 - 2.72 (m, 3H), 2.37 - 2.22 (m, 1H), 2.20 (s, 3H), 2.14 (t, *J* = 11.3, 1H), 1.82 - 1.98 (m, 2H), 1.24 (t, *J* = 7.6, 7.6 Hz, 3H).

**Example 93**

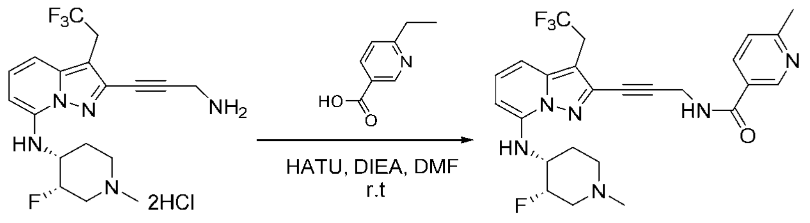


**001-12** **332**

**Step 1: Synthesis of *N*-[3-(7-{[(3*S*,4*R*)-3-fluoro-1-methylpiperidin-4-yl]amino}-3-(2,2,2-trifluoroethyl)pyrazolo [1,5-*a*]pyridin-2-yl)prop-2-yn-1-yl]-6-methylnicotinamide (compound 332)**

At room temperature, a solution of 6-methylnicotinic acid (6.01 mg, 0.044 mmol, 1 eq), *N,N*-diisopropylethylamine (28.32 mg, 0.220 mmol, 5 eq) and 2-(7-azobenzotriazole)-*N,N,N',N'*-tetramethyluronium hexafluorophosphate (16.67 mg, 0.044 mmol, 1 eq) in *N,N*-dimethylformamide (1 mL) was stirred to react for 30 min. Then, 2-(3-aminoprop-1-yn-1-yl)-N-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.044 mmol, 1 eq) was added and the system was further stirred to react for 1 h. After the reaction was completed, the reaction mixture was quenched with water at room temperature, and extracted with ethyl acetate (3 × 10 mL). The organic phases were combined, backwashed with saturated sodium chloride solution (3 × 10 mL), dried over anhydrous sodium sulfate, filtered, and the filtrate was concentrated under reduced pressure. The crude product was purified by HPLC with the following conditions (column specifications: Sunfire C18 5 μm, 30 mm × 150 mm; mobile phase A: water (0.1% ammonium bicarbonate), mobile phase B: acetonitrile; flow rate: 60 mL/min; elution gradient: 33% to 60% in 8 min; detection wavelength: UV 254 nm/220 nm; retention time (min): 6.82), to give compound ***N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl) prop-2-yn-1-yl]-6-methylnicotinamide 332** (6.29 mg, 28.44%).

LCMS: (ESI, m/z): 503.10 $[M+H]^+$.

$^1$H NMR (400 MHz, DMSO-$d_6$, *ppm)* δ 9.23 (t, *J* = 5.4 Hz, 1H), 8.93 (d, *J* = 2.4 Hz, 1H), 8.13 (dd, *J* = 8.2, 2.4 Hz, 1H), 7.38 (d, *J* = 8.1 Hz, 1H), 7.29 (t, *J* = 8.1 Hz, 1H), 7.03 (d, *J* = 8.6 Hz, 1H), 6.32 *(d, J* = 7.7 Hz, 1H), 6.23 (d, *J* = 9.0 Hz, 1H), 4.88 *(d, J* = 49.5 Hz, 1H), 4.41 (d, *J* = 5.4 Hz, 2H), 3.93 - 3.82 (m, 1H), 3.77 (q, *J* = 11.1 Hz, 2H), 3.07 (t, *J* = 11.3 Hz, 1H), 2.79 (d, *J* = 11.3 Hz, 1H), 2.53 (s, 3H), 2.38 - 2.23 (m, 1H), 2.21 (s, 3H), 2.15 (t, *J* = 11.5 Hz, 1H), 2.01 - 1.92 (m, 1H), 1.90 - 1.80 (m, 1H).

**Example 94**

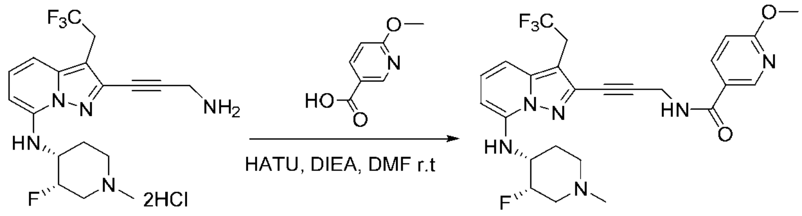


001-12 266

**Step 1: Synthesis of *N*-(3-(7-{[(3*S*,4*R*)-3-fluoro-1-methylpiperidin-4-yl]amino}-3-(2,2,2-trifluoroethyl)pyrazolo [1,5-*a*]pyridin-2-yl)prop-2-yn-1-yl)-6-methoxynicotinamide (compound 266)**

Under nitrogen protection, 6-methoxynicotinic acid (10.07 mg, 0.066 mmol, 1.5 eq), 2-(7-azabenzotriazole)-*N,N,N',N'*-tetramethyluronium hexafluorophosphate (16.67 mg, 0.044 mmol, 1 eq), and *N,N*-diisopropylethylamine (33.71 mg, 0.260 mmol, 5 eq) were added to a reaction flask, dissolved in *N,N*-dimethylformamide (2 mL), and stirred at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.052 mmol, 1 eq) was added and the mixture was further stirred to react for 1 h. After the reaction was completed, water (10 mL) was added, the reaction mixture was extracted with ethyl acetate (20 mL × 2). The organic phases were combined, backwashed with saturated sodium chloride solution (10 mL × 3), dried over anhydrous sodium sulfate, filtered, and the filtrate was concentrated under reduced pressure. The crude product was purified by HPLC with the following conditions (column specifications: XBridge BEH C18 OBD Prep Column 130, 5 μm, 30 mm * 150 mm; mobile phase A: water (17 mmol/L ammonium bicarbonate), mobile phase B: acetonitrile; flow rate: 60 mL/min; elution gradient: 30% B to 60% B in 8 min; detection wavelength: UV 254 nm/220 nm; retention time (min): 7.15), to give compound ***N*-(3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl)-6-methoxynicotinamide 266** (4.91 mg, 21.45%).

LCMS: (ESI, m/z): 518.85 $[M+H]^+$.

$^1$H NMR (400 MHz, DMSO-$d_6$, *ppm)* δ 9.12 *(t, J* = 5.5 Hz, 1H), 8.71 (d, *J* = 2.4 Hz, 1H), 8.16 (dd, *J* = 8.7, 2.5 Hz, 1H), 7.29 (dd, *J* = 8.7, 7.6 Hz, 1H), 7.03 (d, *J* = 8.7 Hz, 1H), 6.92 (d, *J* = 8.7 Hz, 1H), 6.32 (d, *J* = 7.6 Hz, 1H), 6.23 (d, *J* = 9.0 Hz, 1H), 4.87 (d, *J* = 49.3 Hz, 1H), 4.40 (d, *J* = 5.5 Hz, 2H), 3.92 (s, 3H), 3.89 - 3.82 (m, 1H), 3.76 (q, *J* = 11.1 Hz, 2H), 3.06 *(t, J* = 11.5 Hz, 1H), 2.78 (d, *J* = 11.4 Hz, 1H), 2.36 - 2.25 (m, 1H), 2.20 (s, 3H), 2.13 (t, J = 10.6 Hz, 1H), 1.99 - 1.79 (m, 2H).

**Example 95**

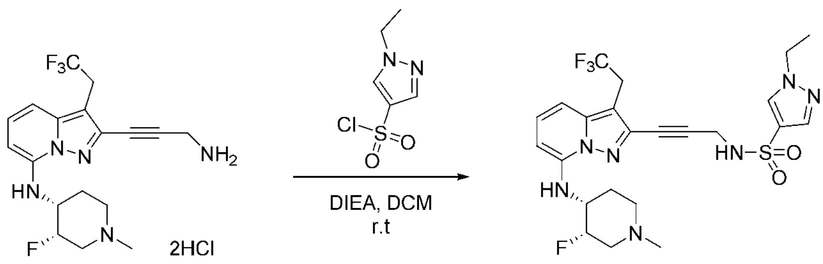


001-12 333

**Step 1: Synthesis of 1-ethyl-*N*-[3-(7-{[(3*S*,4*R*)-3-fluoro-1-methylpiperidin-4-yl]amino}-3-(2,2,2-trifluoroethyl) pyrazolo[1,5-*a*]pyridin-2-yl)prop-2-yn-1-yl]-1*H*-pyrazole-4-carboxamide (compound 333)**

At room temperature, 2-(3-aminoprop-1-yn-1-yl)-N-((3S,4R)-3-fluoro-1-methylpiperidin-4-yl)-3-(2,2,2-trifluoroethyl)pyrazolo[1,5-*a*]pyridin-7-amine dihydrochloride (compound **001-12,** 25 mg, 0.065 mmol, 1 eq) waw added to a reaction flask, and dissolved in dichloromethane (1 mL). The system was cooled to 0°C, 1-ethylpyrazole-4-sulfonyl chloride (10.66 mg, 0.055 mmol, 1 eq) was added dropwise to the reaction mixture. After the addition was completed, the system was warmed to room temperature, and further stirred at room temperature for 1 h. After the reaction was completed, the reaction mixture was quenched at room temperature with water, and the aqueous phase was extracted with ethyl acetate (3 × 20 mL). The organic phases were combined, dried over anhydrous sodium sulfate, filtered, and the filtrate was concentrated under reduced pressure. The crude product was purified by HPLC with the following conditions (column specifications: YMC Triart C18 ExRs 5 μm, 30 mm * 150 mm; mobile phase A: water (10 mmol/L ammonium bicarbonate), mobile phase B: acetonitrile; flow rate: 60 mL/min; elution gradient: 34% B to 64% B in 10 min; detection wavelength: UV 254 nm/220 nm; retention time (min): 3), to give compound **1-ethyl-*N*-[3-(7-{[(3*S*,4*R*)-3-fluoro-1-methylpiperidin-4-yl] amino}-3-(2,2,2-trifluoroethyl)pyrazolo[1,5-*a*]pyridin-2-yl)prop-2-yn-1-yl]-1*H*-pyrazole-4-sulfonamide 333** (4.99 mg, 16.82%).

LCMS: (ESI, m/z): 541.80 $[M+H]^+$.

$^1$H NMR (400 MHz, DMSO-$d_6$, *ppm)* δ 8.31 (s, 1H), 8.03 (s, 1H), 7.77 (s, 1H), 7.29 (t, *J* = 8.8 Hz, 1H), 7.02 (d, *J* = 8.7 Hz, 1H), 6.33 (d, *J* = 7.6 Hz, 1H), 6.19 (d, *J* = 9.1 Hz, 1H), 4.88 (d, *J* = 49.5 Hz, 1H), 4.15 - 3.98 (m, 4H), 3.95 - 3.76 (m, 1H), 3.70 (q, *J* = 11.1 Hz, 2H), 3.06 (t, *J* = 11.7 Hz, 1H), 2.79 (d, *J* = 11.3 Hz, 1H), 2.29 (dd, J = 38.3, 12.6 Hz, 1H), 2.21 (s, 3H),

2.18 - 2.10 (m, 1H), 1.98 - 1.81 (m, 2H), 1.25 (t, $J$ = 7.3 Hz, 3H).

Example 96

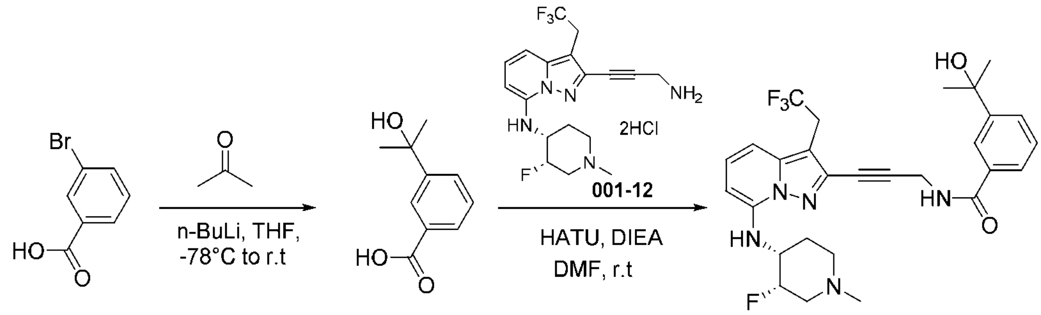


334-1 334-2 334

**Step 1: Synthesis of compound 334-2**

Under nitrogen protection, 3-bromobenzoic acid (compound **334-1**, 500 mg, 2.487 mmol, 1 eq) was added to a reaction flask, dissolved in tetrahydrofuran (5 mL), cooled to -78°C, and 2.5 mol/L n-butyl lithium n-hexane solution (0.47 mL, 4.974 mmol, 2 eq) was added dropwise. After the addition was completed, the mixture was kept at that temperature and stirred for 30 min. Acetone (288.93 mg, 4.974 mmol, 2 eq) was added, and the mixture was stirred to react at -78°C for 1 h, then warmed to room temperature and stirred for 1 h. The desired product was found by LC-MS. At 0°C, the reaction mixture was quenched with saturated ammonium chloride (10 mL). The mixture was purified by reverse phase column chromatography with the following conditions: C18 column, mobile phase: water and acetonitrile, 0% to 20% gradient in 10 min, detection wavelength: UV 254 nm. Compound **334-2**(160 mg, 22.42%) was obtained.
LCMS: (ESI, m/z): 179.25 $[M-H]^-$.

**Step 2: Synthesis of N-[3-(7-{[(3*S*,4*R*)-3-fluoro-1-methylpiperidin-4-y]amino}-3-(2,2,2-trifluoroethyl)pyrazolo[1,5-*a*]pyridin-2-yl)prop-2-yn-1-yl)-3-(2-hydroxyprop-2-yl)benzamide (compound 334)**

Under nitrogen protection, 3-(2-hydroxyprop-2-yl)benzoic acid (compound **334-2**, 11.85 mg, 0.066 mmol, 1.5 eq), 2-(7-azabenzotriazole)-*N,N,N',N'*-tetramethyluronium hexafluorophosphate (16.67 mg, 0.044 mmol, 1 eq), and *N,N*-diisopropylethylamine (28.32 mg, 0.220 mmol, 5 eq) were added to a reaction flask, dissolved in *N,N*-dimethylformamide (1.33 mL), and stirred at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12**, 20 mg, 0.044 mmol, 1.00 eq) was added and the mixture was stirred to react at room temperature for 1 h. After the reaction was completed, water was added (10 mL), and the reaction mixture was extracted with ethyl acetate (20 mL × 2). The organic phases were combined, backwashed with saturated sodium chloride solution (10 mL × 3), dried over anhydrous sodium sulfate, filtered, and the filtrate was concentrated under reduced pressure. The crude product was purified by HPLC with the following conditions (column specifications: Kinetex 5 μm EVO C18, 30mm * 150 mm; mobile phase A: water (10 mmol/L ammonium bicarbonate), mobile phase B: acetonitrile; flow rate: 60 mL/min; elution gradient: 35% B to 50% B in 8 min; detection wavelength: UV 254 nm/220 nm; retention time (min): 6.38), to give compound **N-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl)-3-(2-hydroxyprop-2-yl)benzamide 334** (8.12 mg, 33.82%).
LCMS: (ESI, m/z): 545.90 $[M+H]^+$.
$^1$H NMR (400 MHz, DMSO-$d_6$, *ppm*) $\delta$ 9.07 (t, $J$ = 5.5 Hz, 1H), 7.99 (s, 1H), 7.71 (d, J = 7.7 Hz, 1H), 7.65 (d, J = 8.0 Hz, 1H), 7.41 (t, $J$ = 7.8 Hz, 1H), 7.28 (dd, $J$ = 8.8, 7.6 Hz, 1H), 7.03 (d, $J$ = 8.7 Hz, 1H), 6.32 (d, J = 7.4 Hz, 1H), 6.24 (d, $J$ = 9.0 Hz, 1H), 5.12 (s, 1H), 4.87 (d, $J$ = 49.4 Hz, 1H), 4.39 (d, $J$ = 5.5 Hz, 2H), 3.93 - 3.82 (m, 1H), 3.78 (p, $J$ = 11.7, 2H), 3.05 (t, $J$ = 11.4 Hz, 1H), 2.78 (d, $J$ = 11.3 Hz, 1H), 2.38 - 2.23 (m, 1H), 2.20 (s, 3H), 2.13 (t, $J$ = 11.2 Hz, 1H), 1.97 - 1.81 (m, 2H), 1.45 (s, 6H).

**Example 97**

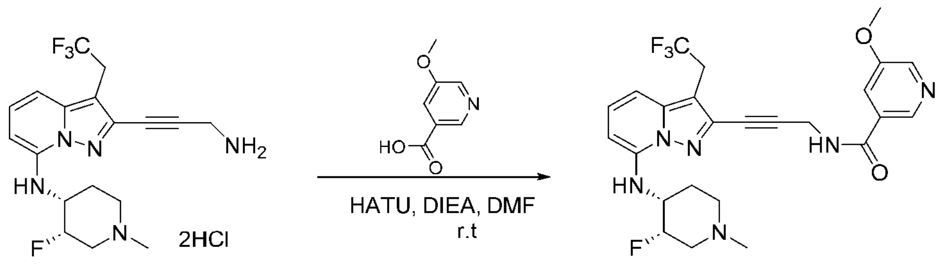


001-12　　335

**Step 1: Synthesis of *N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-5-methoxynicotinamide (compound 335)**

At room temperature, a solution of 5-methoxynicotinic acid (6.71 mg, 0.044 mmol, 1 eq), *N,N*-diisopropylethylamine (28.32 mg, 0.220 mmol, 5 eq) and 2-(7-azobenzotriazole)-*N,N,N',N'*-tetramethyluronium hexafluorophosphate (16.67 mg, 0.044 mmol, 1 eq) in *N,N*-dimethylformamide (1 mL) was stirred to react for 30 min. At room temperature, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.044 mmol, 1 eq) was added. The mixture was stirred to react for 1 h, and the reaction system was monitored by LC-MS. After the reaction was completed, the reaction mixture was quenched with water at room temperature. The reaction mixture was extracted with ethyl acetate (3 × 10 mL). The organic phases were combined, backwashed with saturated sodium chloride solution (3 × 10 mL), and dried over anhydrous sodium sulfate. The resulting mixture was filtered, and then the filtrate was concentrated under reduced pressure. The crude product was purified by HPLC with the following conditions (column specifications:Sunfire C18 5 m, 30 mm × 150 mm; mobile phase A: water (0.1% ammonium bicarbonate), mobile phase B: acetonitrile; flow rate: 60 mL/min; elution gradient: 38% to 58% in 8 min; detection wavelength: UV 254 nm/220 nm; retention time (min): 6.6) to give compound ***N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-5-methoxynicotinamide 335** (16.52 mg, 72.40%).

LCMS: (ESI, m/z): 519.20 $[M+H]^+$.

$^1H$ NMR (400 MHz, DMSO-$d_6$, *ppm*) $\delta$ 9.31 (t, *J* = 5.5 Hz, 1H), 8.65 (d, *J* = 1.8 Hz, 1H), 8.45 (d, *J* = 2.9 Hz, 1H), 7.79 (dd, *J* = 2.9, 1.8 Hz, 1H), 7.29 (dd, *J* = 8.8, 7.6 Hz, 1H), 7.03 (d, J = 8.6 Hz, 1H), 6.33 (d, J = 7.3 Hz, 1H), 6.22 *(d, J* = 9.0 Hz, 1H), 4.87 (d, *J* = 49.4 Hz, 1H), 4.43 (d, *J* = 5.5 Hz, 2H), 3.89 (s, 3H), 3.86 - 3.68 (m, 3H), 3.06 (t, *J* = 11.4 Hz, 1H), 2.78 (d, *J* = 11.3 Hz, 1H), 2.36 - 2.24 (m, 1H), 2.20 (s, 3H), 2.13 (td, J = 11.4, 9.4 Hz, 1H), 2.00 - 1.88 (m, 1H), 1.88 - 1.75 (m, 1H).

Example 98

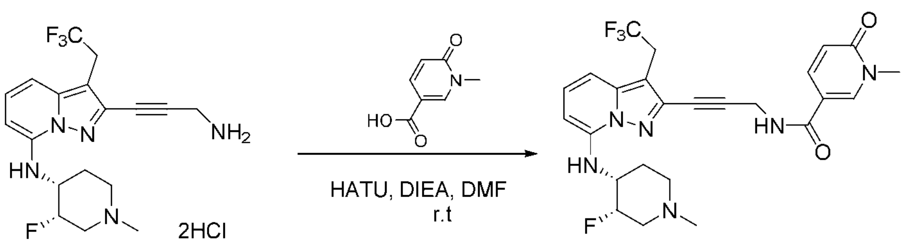


001-12　　265

**Step 1: Synthesis of *N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1-methyl-6-oxo-1,6-dihydropyridine-3-carboxamide (compound 265)**

At room temperature, 1-methyl-6-oxo-1,6-dihydropyridine-3-carboxylic acid (8.05 mg, 0.053 mmol, 1.2 eq), *N,N*-diisopropylethylamine (0.04 mL, 0.220 mmol, 5 eq), and O-(7-azabenzotriazol-1-yl)-*N,N,N',N'*-tetramethyluronium hexafluorophosphate (16.67 mg, 0.044 mmol, 1 eq) were added to a reaction flask, dissolved in *N,N*-dimethylformamide (2 mL) and stirred at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-a]pyridin-7-amine dihydrochloride (compound **001-12**, 20 mg, 0.044 mmol, 1 eq) was added, the mixture was stirred for 1 h and diluted with ethyl acetate (5 mL). The reaction mixture was washed with saturated sodium chloride solution (5 mL × 3), dried over anhydrous sodium sulfate, filtered, and the filtrate was

concentrated under reduced pressure. The crude product was purified by HPLC with the following conditions (column specifications: YMC Triart C18 ExRs 5 m, 30 mm × 150 mm; mobile phase A: water (10 mmol/L ammonium bicarbonate), mobile phase B: acetonitrile; flow rate: 60 mL/min; elution gradient: 27% B to 57% B in 10 min; detection wavelength: UV 254 nm/220 nm; retention time (min): 7.58), to give compound ***N*-[3-(7-{[(3*S*,4*R*)-3-fluoro-1-methylpiperidin-4-yl] amino}-3-(2,2,2-trifluoroethyl)pyrazolo[1,5-*a*]pyridin-2-yl)prop-2-yn-1-yl]-1-methyl-6-oxo-1,6-dihydropyridine-3-carboxamide 265** (4.5 mg, 19.72 %).

LCMS: (ESI, m/z): 518.85 $[M+H]^+$.

$^1$H NMR (400 MHz, DMSO-$d_6$, *ppm)* δ 8.84 (t, *J* = 5.5 Hz, 1H), 8.41 (d, *J* = 2.6 Hz, 1H), 7.88 (dd, *J* = 9.5, 2.6 Hz, 1H), 7.29 (dd, *J* = 8.7, 7.6 Hz, 1H), 7.03 (d, *J* = 8.7 Hz, 1H), 6.43 (d, *J* = 9.5 Hz, 1H), 6.33 (d, *J* = 7.6 Hz, 1H), 6.21 (d, *J* = 9.1 Hz, 1H), 4.87 (d, *J* = 49.5 Hz, 1H), 4.36 (d, *J* = 5.4 Hz, 2H), 3.93 - 3.82 (m, 1H), 3.76 (q, *J* = 11.2 Hz, 2H), 3.49 (s, 3H), 3.06 (t, *J* = 11.5 Hz, 1H), 2.78 (d, *J* = 11.4 Hz, 1H), 2.38 - 2.23 (m, 1H), 2.20 (s, 3H), 2.13 (t, *J* = 11.1 Hz, 1H), 1.98 - 1.80 (m, 2H).

**Example 99**

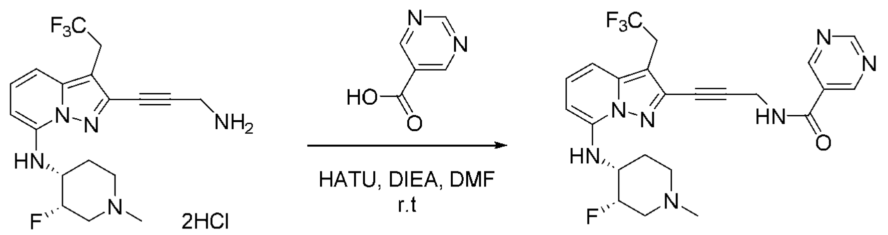


**001-12**

**264**

**Step 1: Synthesis of *N*-[3-(7-{[(3*S*,4*R*)-3-fluoro-1-methylpiperidin-4-yl]amino}-3-(2,2,2-trifluoroethyl)pyridino [1,5-*a*]pyridin-2-yl)prop-2-yn-1-yl]pyrimidine-5-carboxamide (compound 264)**

At room temperature, a solution of pyrimidine-5-carboxylic acid (6.53 mg, 0.053 mmol, 1.2 eq), *N,N*-diisopropylethylamine (0.04 mL, 0.220 mmol, 5 eq), and O-(7-azabenzotriazol-1-yl)-*N,N,N',N'*-tetramethyluronium hexafluorophosphate (16.67 mg, 0.044 mmol, 1 eq) in *N,N*-dimethylformamide (2 mL) was stirred at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-a]pyridin-7-amine dihydrochloride (compound **001-12**, 20 mg, 0.044 mmol, 1 eq) was added and the mixture was stirred at room temperature for 1 h. After the reaction was completed, the reaction mixture was diluted with ethyl acetate (5 mL), washed with saturated sodium chloride solution (3 x5 mL). The organic phases were combined, dried over anhydrous sodium sulfate, filtered, and the filtrate was concentrated under reduced pressure. The crude product was purified by HPLC with the following conditions (column specifications: :Xselect CSHTM Prep C18 5μm 30 × 150mm OBD; mobile phase A: water (0.1% formic acid), mobile phase B: acetonitrile; flow rate: 60 mL/min; elution gradient: 5% B to 30% B in 10 min; detection wavelength: UV 254 nm/220 nm; retention time (min): 8.2), to give the carboxylate of compound ***N*-[3-(7-{[(3*S*,4*R*)-3-fluoro-1-methylpiperidin-4-yl]amino}-3-(2,2,2-trifluoroethyl)pyridino[1,5-*a*]pyridin-2-yl) prop-2-yn-1-yl]pyrimidine-5-carboxamide 264** (1.9 mg, 8.00 %).

LCMS: (ESI, m/z): 490.10 $[M+H]^+$.

$^1$H NMR (400 MHz, DMSO-$d_6$, *ppm)* δ 9.50 (s, 1H), 9.35 (d, *J* = 3.1 Hz, 1H), 9.21 (d, *J* = 3.2 Hz, 2H), 7.33 - 7.23 (m, 1H), 7.04 (d, *J* = 8.7 Hz, 1H), 6.34 (d, J = 7.4 Hz, 1H), 6.21 (d, *J* = 8.7 Hz, 1H), 4.88 (d, *J* = 49.3 Hz, 1H), 4.46 (dd, *J* = 5.5, 3.1 Hz, 2H), 3.85 - 3.70 (m, 3H), 3.06 (s, 1H), 2.78 (s, 1H), 2.38 - 2.25 (m, 1H), 2.21 (d, *J* = 3.1 Hz, 3H), 2.15 (t, *J* = 11.4 Hz, 1H), 1.99 - 1.81 (m, 2H).

**Example 100**

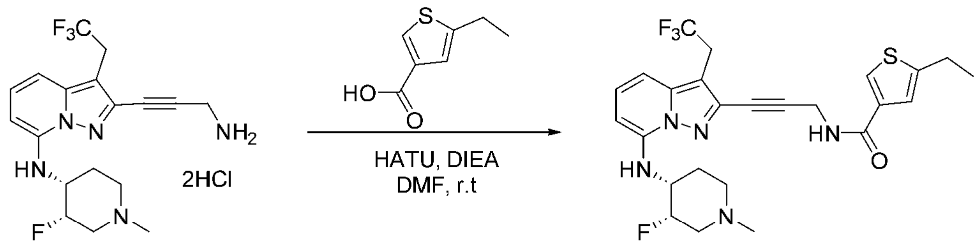


001-12

336

**Step 1: Synthesis of 5-ethyl-*N*-[3-(7-{[(3*S*,4*R*)-3-fluoro-1-methylpiperidin-4-yl]amino}-3-(2,2,2-trifluoroethyl) pyrazolo[1,5-*a*]pyridin-2-yl)prop-2-yn-1-yl]thiophene-3-carboxamide (compound 336)**

Under nitrogen protection, at room temperature, to a solution of O-(7-azabenzotriazol-1-yl)-*N,N,N',N'*-tetramethyluronium hexafluorophosphate (16.67 mg, 0.044 mmol, 1.0 eq) and 5-ethylthiophene-3-carboxylic acid (10.27 mg, 0.066 mmol, 1.5 eq) in *N,N*-dimethylformamide (0.7 mL) was added *N,N*-diisopropylethylamine (28.32 mg, 0.220 mmol, 5 eq), and the mixture was stirred to react for 1 h. Then, at room temperature, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12**, 20 mg, 0.044 mmol, 1 eq) was added and the system was further stirred for 1 h. After the reaction was completed, the reaction mixture was quenched with methanol (1.0 mL) at room temperature. The crude product was purified by HPLC with the following conditions (column specifications: XBridge BEH C18 OBD Prep Column 130, 5 μm, 30 mm × 150 mm; mobile phase A: water (17 mmol/L ammonium bicarbonate), mobile phase B: acetonitrile; flow rate: 60 mL/min; elution gradient: 37% B to 67% B in 8 min; detection wavelength: UV 254 nm/220 nm; retention time (min): 7.42), to give compound **5-ethyl-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl) prop-2-yn-1-yl]thiophene-3-carboxamide 336** (4.13 mg, 17.99%).

LCMS: (ESI, m/z): 522.10 $[M+H]^+$.

$^1$H NMR (400 MHz, DMSO-$d_6$, *ppm*) δ 8.82 (t, *J* = 5.6 Hz, 1H), 7.95 (d, *J* = 1.4 Hz, 1H), 7.32 - 7.25 (m, 2H), 7.02 (d, *J* = 8.7 Hz, 1H), 6.32 (d, *J* = 7.6 Hz, 1H), 6.23 (d, *J* = 9.0 Hz, 1H), 4.87 (d, *J* = 49.5 Hz, 1H), 4.34 (d, *J* = 5.6 Hz, 2H), 3.91 - 3.82 (m, 1H), 3.75 (q, *J* = 11.1 Hz, 2H), 3.05 (t, *J* = 11.7 Hz, 1H), 2.86 - 2.75 (m, 3H), 2.37 - 2.23 (m, 1H), 2.20 (s, 3H), 2.13 (t, *J* = 11.2 Hz, 1H), 1.98 - 1.82 (m, 2H), 1.25 (t, *J* = 7.5 Hz, 3H).

Example 101

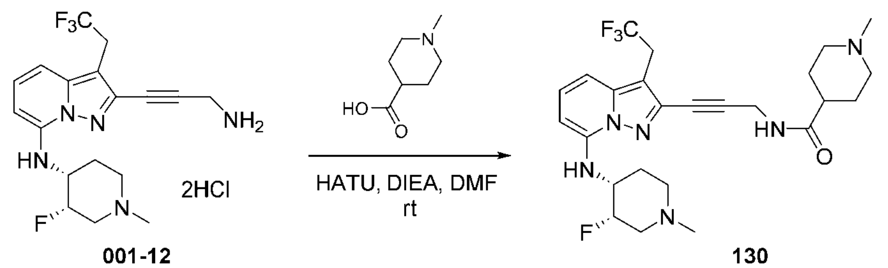


001-12

130

**Step 1: Synthesis of *N*-[3-(7-{[(3*S*,4*R*)-3-fluoro-1-methylpiperidin-4-yl]amino}-3-(2,2,2-trifluoroethyl)pyrazolo [1,5-a]pyridin-2-yl)prop-2-yn-1-yl]-1-methylpiperidin-4-carboxamide (compound 130)**

At room temperature, a solution of 1-methylpiperidin-4-carboxylic acid (7.53 mg, 0.053 mmol, 1.2 eq), *N,N*-diisopropylethylamine (28.32 mg, 0.220 mmol, 5 eq) and O-(7-azabenzotriazol-1-yl)-*N,N,N',N'*-tetramethyluronium hexafluorophosphate (20.0 mg, 0.053 mmol, 1.2 eq) in *N,N*-dimethylformamide (1 mL) was stirred to react for 30 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12**, 20 mg, 0.044 mmol, 1 eq) was added and the system was stirred to react for 2 h. After the reaction was completed, the reaction mixture was quenched with water at room temperature, and extracted with ethyl acetate (3 × 30 mL). The organic phases were combined, backwashed with saturated sodium chloride solution (3 × 30 mL), dried over anhydrous sodium sulfate, filtered, and the filtrate was concentrated under reduced pressure. The crude product was purified by HPLC with the following conditions (column specifications: Sunfire C18 5 μm, 30 mm × 150 mm; mobile phase A: water (0.1% ammonium bicarbonate), mobile phase B: acetonitrile; flow rate: 60

mL/min; elution gradient: 20% to 60% in 10 min; detection wavelength: UV 254 nm/220 nm; retention time (min): 7.38), to give compound ***N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1-methylpiperidin-4-carboxamide 130** (14.73 mg, 65.88%).

LCMS: (ESI, m/z): 509.15 [M+H]$^+$.

$^1$H NMR (400 MHz, DMSO-$d_6$, *ppm)* $\delta$ 8.37 (t, *J* = 5.6 Hz, 1H), 7.29 (t, *J* = 8.2 Hz, 1H), 7.02 (d, *J* = 8.7 Hz, 1H), 6.32 (d, *J* = 7.6 Hz, 1H), 6.22 (d, *J* = 9.0 Hz, 1H), 4.88 (d, *J* = 49.5 Hz, 1H), 4.16 (d, *J* = 5.5 Hz, 2H), 3.98 - 3.79 (m, 1H), 3.75 (q, J = 11.1 Hz, 2H), 3.06 (t, *J* = 11.1 Hz, 1H), 2.85 - 2.69 (m, 3H), 2.39 - 2.23 (m, 1H), 2.21 (s, 3H), 2.17 - 1.99 (m, 5H), 1.99 - 1.75 (m, 4H), 1.71 - 1.50 (m, 4H).

Example 102

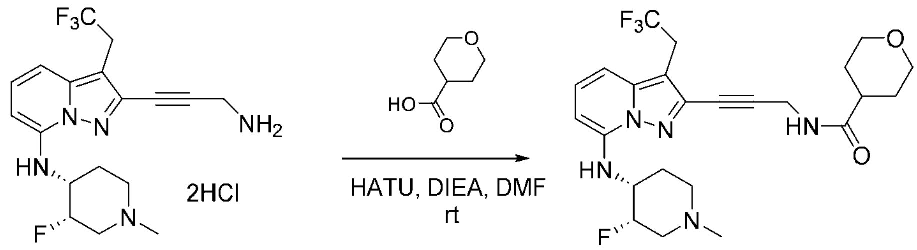


**001-12** **129**

**Step 1: Synthesis of *N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]tetrahydropyran-4-carboxamide (compound 129)**

At room temperature, a solution of tetrahydropyran-4-carboxylic acid (6.84 mg, 0.053 mmol, 1.2 eq), *N,N*-diisopropylethylamine (28.32 mg, 0.220 mmol, 5 eq) and O-(7-azabenzotriazol-1-yl)-*N,N,N',N'*-tetramethyluronium hexafluorophosphate (20.0 mg, 0.053 mmol, 1.2 eq) in *N,N*-dimethylformamide (1 mL) was stirred to react for 30 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12**, 20 mg, 0.044 mmol, 1 eq) was added and the system was further stirred for 2 h. After the reaction was completed, the reaction mixture was quenched with water at room temperature, and extracted with ethyl acetate (3 × 10 mL). The organic phases were combined, backwashed with saturated sodium chloride solution (3 × 10 mL), dried over anhydrous sodium sulfate, filtered, and the filtrate was concentrated under reduced pressure. The crude product was purified by HPLC with the following conditions (column specifications: Sunfire C18 5 μm, 30 mm × 150 mm; mobile phase A: water (0.1% formic acid), mobile phase B: acetonitrile; flow rate: 60 mL/min; elution gradient: 5% to 30% in 10 min; detection wavelength: UV 254 nm/220 nm; retention time (min): 8.38), to give compound ***N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]tetrahydropyran-4-carboxamide 129** (15.55 mg, 64.86%).

LCMS: (ESI, m/z): : 496.05 [M+H]$^+$.

$^1$H NMR (400 MHz, DMSO-$d_6$, *ppm)* $\delta$ 8.39 *(t, J* = 5.5 Hz, 1H), 7.29 (dd, *J* = 8.7, 7.6 Hz, 1H), 7.02 (d, *J* = 8.6 Hz, 1H), 6.32 (d, *J* = 7.6 Hz, 1H), 6.22 (d, *J* = 9.0 Hz, 1H), 4.89 (d, *J* = 49.3 Hz, 1H), 4.18 (d, *J* = 5.4 Hz, 2H), 3.94 - 3.81 (m, 3H), 3.74 (q, *J* = 11.2 Hz, 2H), 3.28 - 3.21 (m, 2H), 3.08 (t, J = 10.4 Hz, 1H), 2.80 (d, *J* = 11.5 Hz, 1H), 2.46 - 2.25 (m, 2H), 2.22 (s, 3H), 2.16 (t, J = 9.5 Hz, 1H), 2.01 - 1.80 (m, 2H), 1.72 - 1.48 (m, 4H).

**Example 103**

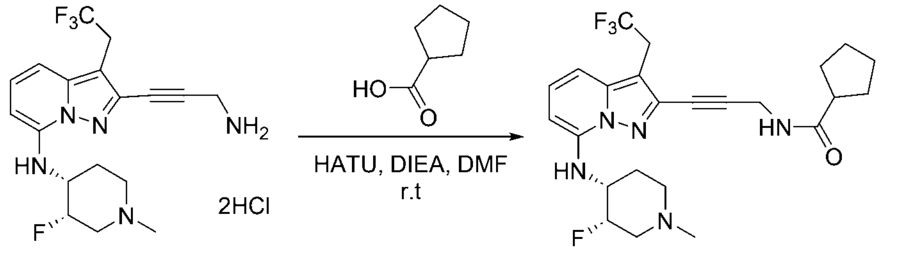


001-12 137

**Step 1: Synthesis of *N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]cyclopentanecarboxamide (compound 137)**

Under nitrogen protection, to a solution of O-(7-azabenzotriazol-1-yl)-*N,N,N',N'*-tetramethyluronium hexafluorophosphate (16.67 mg, 0.044 mmol, 1.0 eq) and cyclopentyl carboxylic acid (7.5 mg, 0.066 mmol, 1.5 eq) in N,N-dimethylformamide (0.5 mL) was added *N,N*-diisopropylethylamine (28.32 mg, 0.220 mmol, 5 eq), and the mixture was stirred to react for 1 h. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12**, 20 mg, 0.044 mmol, 1 eq) was added and the system was further stirred for 1 h. After the reaction was completed, the reaction mixture was quenched with methanol (1.0 mL) at room temperature. The crude product was purified by HPLC with the following conditions (column specifications: YMC Triart C18 ExRs 5 μm, 30 mm × 150 mm; mobile phase A: water (10 mmol/L ammonium bicarbonate), mobile phase B: acetonitrile; flow rate: 60 mL/min; elution gradient: 37% B to 70% B in 8 min; detection wavelength: UV 254 nm/220 nm; retention time (min): 7.33), to give compound ***N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]cyclopentanecarboxamide 137** (16.96 mg, 80.21%).

LCMS: (ESI, m/z): 480.10 $[M+H]^+$.

$^1$H NMR (400 MHz, DMSO-$d_6$, *ppm) δ* 8.35 (t, *J* = 5.6 Hz, 1H), 7.29 (dd, *J* = 8.8, 7.6 Hz, 1H), 7.02 (d, *J* = 8.7 Hz, 1H), 6.32 (d, *J* = 7.5 Hz, 1H), 6.22 (d, *J* = 9.1 Hz, 1H), 4.88 (d, *J* = 49.4 Hz, 1H), 4.17 (d, *J* = 5.5 Hz, 2H), 3.97 - 3.79 (m, 1H), 3.75 (p, *J* = 11.2 Hz, 2H), 3.07 (t, J = 11.1 Hz, 1H), 2.79 (d, *J* = 11.2 Hz, 1H), 2.64 - 2.55 (m, 1H), 2.30 - 2.09 (m, 5H), 2.01 - 1.81 (m, 2H), 1.81 - 1.70 (m, 2H), 1.69 - 1.56 (m, 4H), 1.54 - 1.44 (m, 2H).

**Example 104**

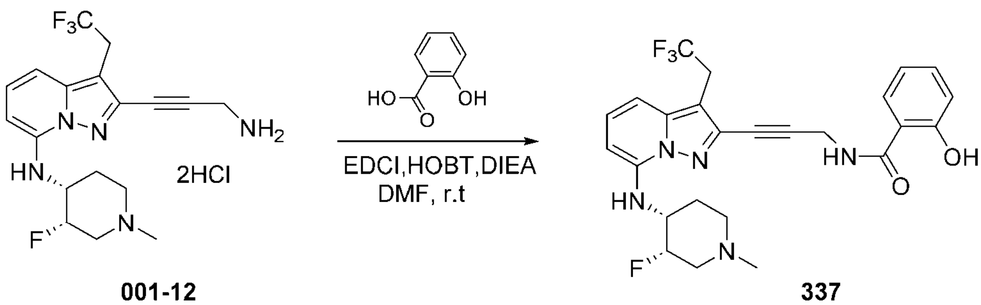


001-12 337

**Step 1: Synthesis of *N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl)-2-hydroxybenzamide (compound 337)**

Under nitrogen protection, salicylic acid (15.13 mg, 0.109 mmol, 1.2 eq), 1-ethyl-(3-dimethylaminopropyl) carbodiimide hydrochloride (26.25 mg, 0.137 mmol, 1.5 eq), 1-hydroxy-benzotriazole (18.5 mg, 0.137 mmol, 1.5 eq), and *N,N*-diisopropylethylamine (58.99 mg, 0.455 mmol, 5 eq) were added to a reaction flask, and dissolved in *N,* N-dimethylformamide (2 mL). The mixture was stirred at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12**, 35 mg, 0.091 mmol, 1 eq) was added and the mixture was stirred to react at room temperature for 1 h. After the reaction was completed, the reaction mixture was extracted with ethyl acetate (20 mL × 2). The organic phases were combined, backwashed with saturated sodium chloride solution (10 mL × 3), and dried over anhydrous sodium

sulfate. The resulting mixture was filtered, and the filtrate was concentrated under reduced pressure. The crude product was purified by HPLC with the following conditions (column specifications: Kinetex 5μm EVO C18, 30mm * 150 mm; mobile phase A: water (10 mmol/L ammonium bicarbonate), mobile phase B: acetonitrile; flow rate: 60 mL/min; elution gradient: 33% B to 61% B in 10 min; detection wavelength: UV 254 nm/220 nm; retention time (min): 8.58) to give compound ***N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl)-2-hydroxybenzamide 337** (5.6 mg, 12.12%).

LCMS: (ESI, m/z): 503.85 $[M+H]^+$.

$^1H$ NMR (400 MHz, DMSO-$d_6$, *ppm)* $\delta$ 10.17 (s, 1H), 7.82 (dd, J = 7.9, 1.5 Hz, 1H), 7.28 (t, *J* = 8.4 Hz, 2H), 7.02 (d, *J* = 8.7 Hz, 1H), 6.81 (d, *J* = 8.3 Hz, 1H), 6.71 (t, *J* = 7.5 Hz, 1H), 6.31 (d, J = 7.4 Hz, 1H), 6.26 (d, J = 9.0 Hz, 1H), 4.87 (d, *J* = 49.5 Hz, 1H), 4.42 (s, 2H), 3.79 - 3.71 (m, 3H), 3.11 - 2.97 (m, 1H), 2.78 (d, *J* = 11.4 Hz, 1H), 2.36 - 2.23 (m, 1H), 2.20 (s, 3H), 2.13 (t, J = 10.7 Hz, 1H), 2.01 - 1.79 (m, 2H).

**Example 105**

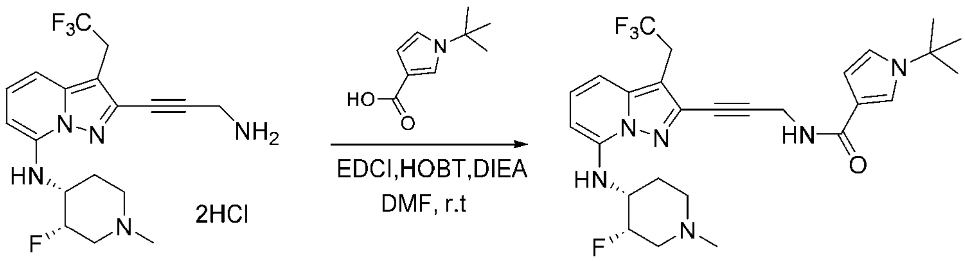


**001-12** **087**

**Step 1: Synthesis of 1-(tert-butyl)-*N*-(3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl)-1*H*-pyrrole-3-carboxamide (compound 087)**

Under nitrogen protection, 1-(tert-butyl)-1*H*-pyrrole-3-carboxylic acid (32.71 mg, 0.195 mmol, 1.5 eq), 1-hydroxy-benzotriazole (26.43 mg, 0.195 mmol, 1.5 eq), 1-ethyl-(3-dimethylaminopropyl)carbodiimide hydrochloride (37.5 mg, 0.195 mmol, 1.5 eq), and *N, N*-diisopropylethylamine (84.28 mg, 0.650 mmol, 5 eq) were added to a reaction flask, dissolved in *N,N*-dimethylformamide (3 mL) and stirred at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 50 mg, 0.130 mmol, 1 eq) was added, and the system was further stirred to react for 1 h. After the reaction was completed, water (8 mL) was added, and the reaction mixture was extracted with ethyl acetate (10 mL × 3). The organic phases were combined, backwashed with saturated sodium chloride solution (20 mL × 2), dried over anhydrous sodium sulfate, and filtered, and the filtrate was concentrated under reduced pressure. The crude product was purified by HPLC with the following conditions (column specifications: YMC Triart C18 ExRs 5 μm, 30 mm × 150 mm; mobile phase A: water (10 mmol/L ammonium bicarbonate), mobile phase B: acetonitrile; flow rate: 60 mL/min; elution gradient: 45% B to 67% B in 8 min; detection wavelength: UV 254 nm/220 nm; retention time (min): 6.92) to give compound **1-(tert-butyl)-*N*-(3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl)-1*H*-pyrrole-3-carboxamide 087** (18.75 mg, 26.86%).

LCMS: (ESI, m/z): 533.10 $[M+H]^+$.

$^1H$ NMR (400 MHz, DMSO-$d_6$, *ppm)* $\delta$ 8.32 (t, *J* = 5.5 Hz, 1H), 7.53 (t, *J* = 2.1 Hz, 1H), 7.28 (dd, *J* = 8.8, 7.6 Hz, 1H), 7.02 (d, *J* = 8.7 Hz, 1H), 6.95 (t, *J* = 2.7 Hz, 1H), 6.48 (d, *J* = 1.8 Hz, 1H), 6.31 (d, *J* = 7.7 Hz, 1H), 6.26 (d, *J* = 9.0 Hz, 1H), 4.87 *(d, J* = 49.5 Hz, 1H), 4.30 (d, *J* = 5.6 Hz, 2H), 3.92 - 3.82 (m, 1H), 3.75 (q, *J* = 11.2 Hz, 2H), 3.05 (t, *J* = 11.4 Hz, 1H), 2.78 (d, *J* = 11.5 Hz, 1H), 2.30 (dd, J = 38.7, 13.4 Hz, 1H), 2.20 (s, 3H), 2.14 (t, *J* = 11.0 Hz, 1H), 1.95 (td, *J* = 12.1, 3.7 Hz, 1H), 1.89 - 1.80 (m, 1H), 1.48 (s, 9H).

**Example 106**

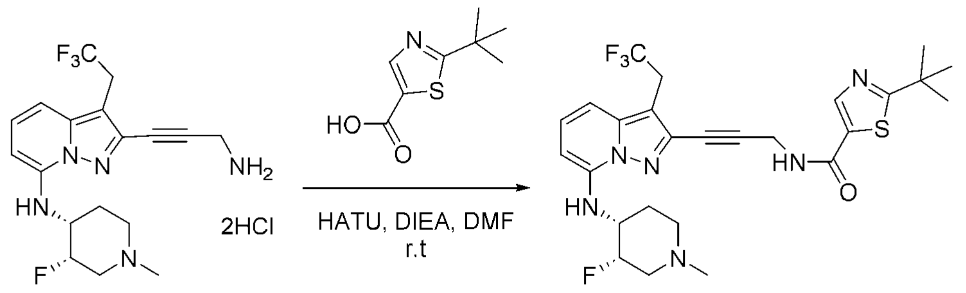


**001-12** **089**

**Step 1: Synthesis of 2-tert-butyl-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1,3-thiazole-5-carboxamide (compound 089)**

Under nitrogen protection, at room temperature, to a solution of O-(7-azabenzotriazol-1-yl)-*N,N,N',N'*-tetramethyluronium hexafluorophosphate (2-(7-azobenzotriazole)-*N,N,N',N'*-tetramethyluronium hexafluorophosphate) (50.00 mg, 0.132 mmol, 1.5 eq), *N,N*-diisopropylethylamine (56.65 mg, 0.440 mmol, 5 eq) and 2-tert-butyl-1,3-thiazole-5-carboxylic acid (24.36 mg, 0.132 mmol, 1.5 eq) in *N,N*-dimethylformamide (0.8 mL) was added 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12**, 40 mg, 0.088 mmol, 1 eq), and the mixture was further stirred at room temperature for 1 h. After the reaction was completed, methanol (1.0 mL) was added to the reaction mixture for quenching. The crude product was purified by HPLC with the following conditions (column specifications: Kinetex 5 μm EVO C18, 30mm × 150 mm; mobile phase A: water (10 mmol/L ammonium bicarbonate), mobile phase B: acetonitrile; flow rate: 60 mL/min; elution gradient: 45% B to 65% B in 10 min; detection wavelength: UV 254 nm/220 nm; retention time (min): 7.7), to give compound **2-tert-butyl-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1,3-thiazole-5-carboxamide 089** (16.73 mg, 33.73%).

LCMS: (ESI, m/z): 550.90 $[M+H]^+$.

$^1$H NMR (400 MHz, DMSO-$d_6$, *ppm*) δ 8.77 (t, *J* = 5.9 Hz, 1H), 8.19 (s, 1H), 7.32 - 7.24 (m, 1H), 7.02 (d, *J* = 8.7 Hz, 1H), 6.31 (d, *J* = 7.5 Hz, 1H), 6.25 (d, *J* = 9.0 Hz, 1H), 4.87 (d, *J* = 49.4 Hz, 1H), 4.37 (d, *J* = 6.0 Hz, 2H), 3.93 - 3.80 (m, 1H), 3.75 (q, *J* = 11.1 Hz, 2H), 3.05 (t, *J* = 11.5 Hz, 1H), 2.78 (d, *J* = 11.4 Hz, 1H), 2.38 - 2.24 (m, 1H), 2.20 (s, 3H), 2.14 (t, *J* = 11.2 Hz, 1H), 1.93 (qd, *J* = 12.0, 3.8 Hz, 1H), 1,87 - 1,79 (m, 1H), 1.43 (s, 9H).

**Example 107**

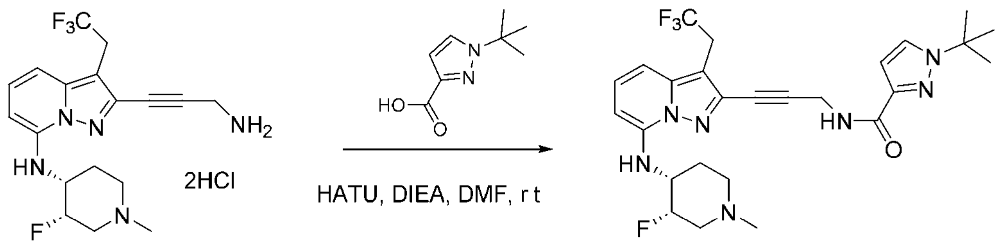


**001-12** **067**

**Step 1: Synthesis of 1-tert-butyl-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1*H*-pyrazole-3-carboxamide (compound 067)**

At room temperature, a solution of 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-a]pyridin-7-amine dihydrochloride (compound **001-12,** 50 mg, 0.130 mmol, 1 eq), 1-tert-butyl-1*H*-pyrazole-3-carboxylic acid (32.9 mg, 0.195 mmol, 1.5 eq), O-(7-azabenzotriazol-1-yl)-*N,N,N',N'*-tetramethyluronium hexafluorophosphate (74.38 mg, 0.195 mmol, 1.5 eq) and *N,N*-diisopropylethylamine (33.71 mg, 0.260 mmol, 2 eq) in *N,N*-dimethylformamide (1 mL) was stirred to react for 2 h. After the reaction was completed, the reaction mixture was quenched with water at room temperature, and extracted with ethyl acetate (3 × 10 mL). The organic phases were combined, backwashed with saturated sodium chloride solution (3 × 10 mL), dried over anhydrous sodium sulfate, filtered, and the filtrate was concentrated under reduced pressure. The crude product was purified by HPLC with

the following conditions (column specifications: Sunfire C18 5 μm, 30 mm × 150 mm; mobile phase A: water (0.1% ammonium bicarbonate), mobile phase B: acetonitrile; flow rate: 60 mL/min; elution gradient: 42% to 66% in 10 min; detection wavelength: UV 254 nm/220 nm; retention time (min): 9.08), to give compound **1-tert-butyl-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1*H*-pyrazole-3-carboxamide 067** (28.89 mg, 41.43%).

LCMS: (ESI, m/z): 534.20 $[M+H]^+$.

$^1$H NMR (400 MHz, DMSO-$d_6$, *ppm*) $\delta$ 8.53 (t, *J* = 5.9 Hz, 1H), 7.92 (d, *J* = 2.4 Hz, 1H), 7.28 (dd, *J* = 8.8, 7.6 Hz, 1H), 7.02 (d, *J* = 8.7 Hz, 1H), 6.66 (d, *J* = 2.4 Hz, 1H), 6.31 (d, J = 7.5 Hz, 1H), 6.25 (d, J = 9.0 Hz, 1H), 4.87 (d, *J* = 49.5 Hz, 1H), 4.33 (d, *J* = 5.9 Hz, 2H), 3.92 - 3.81 (m, 1H), 3.75 (q, *J* = 11.1 Hz, 2H), 3.05 (t, *J* = 11.5 Hz, 1H), 2.78 (d, *J* = 11.2 Hz, 1H), 2.39 - 2.22 (m, 1H), 2.20 (s, 3H), 2.14 (t, J = 10.7 Hz, 1H), 2.00 - 1.88 (m, 1H), 1.86 - 1.78 (m, 1H), 1.56 (s, 9H).

**Example 108**

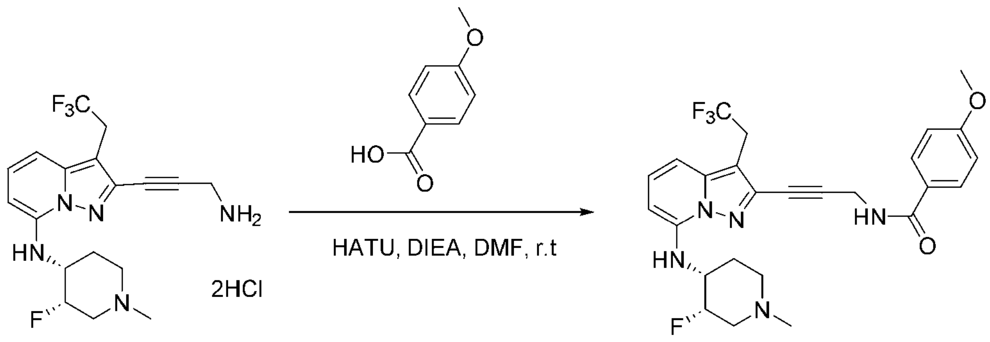


**001-12** **070**

**Step 1: Synthesis of *N*-[3-(7-{[(3*S*,4*R*)-3-fluoro-1-methylpiperidin-4-yl]*amino*}-3-(2,2,2-trifluoroethyl)pyrazolo[1,5-*a*]pyridin-2-yl)prop-2-yn-1-yl]-4-methoxybenzamide (compound 070)**

Under nitrogen protection, at room temperature, to a solution of 4-methoxybenzoic acid (20.01 mg, 0.132 mmol, 1.5 eq), O-(7-azabenzotriazol-1-yl)-*N,N,N',N'*-tetramethyluronium hexafluorophosphate (50.00 mg, 0.132 mmol, 1.5 eq), and *N,N*-diisopropylethylamine (56.65 mg, 0.440 mmol, 5 eq) in N,N-dimethylformamide (0.8 mL) was added 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12**, 40 mg, 0.088 mmol, 1 eq) in portions and the mixture was stirred at room temperature for 1 h. After the reaction was completed, methanol (1.0 mL) was added to the reaction mixture for quenching. The crude product was purified by HPLC with the following conditions (column specifications: YMC Triart C18 ExRs 5 μm, 30 mm × 150 mm; mobile phase A: water (10 mmol/L ammonium bicarbonate), mobile phase B: acetonitrile; flow rate: 60 mL/min; elution gradient: 38% B to 64% B in 10 min; detection wavelength: UV 254 nm/220 nm; retention time (min): 9.07), to give compound ***N*-[3-(7-{[(3*S*,4*R*)-3-fluoro-1-methylpiperidin-4-yl]amino}-3-(2,2,2-trifluoroethyl)pyrazolo[1,5-a]pyridin-2-yl)prop-2-yn-1-yl]-4-methoxybenzamide 070** (14.71 mg, 32.26%).

LCMS: (ESI, m/z): 517.90 $[M+H]^+$.

$^1$H NMR (400 MHz, DMSO-$d_6$) $\delta$ 8.93 (t, *J* = 5.6 Hz, 1H), 7.99 - 7.71 (m, 2H), 7.28 (dd, *J* = 8.8, 7.6 Hz, 1H), 7.11 - 6.90 (m, 3H), 6.31 (d, J = 7.4 Hz, 1H), 6.23 (d, *J*= 9.1 Hz, 1H), 4.87 (d, *J* = 49.4 Hz, 1H), 4.37 (d, *J* = 5.5 Hz, 2H), 3.92 - 3.83 (m, 1H), 3.82 (s, 3H), 3.76 (q, *J* = 11.2 Hz, 2H), 3.05 (t, *J* = 11.3 Hz, 1H), 2.78 (d, *J* = 11.5 Hz, 1H), 2.37 - 2.21 (m, 1H), 2.20 (s, 3H), 2.13 (t, J = 10.7 Hz, 1H), 1.99 - 1.88 (m, 1H), 1.88 - 1.80 (m, 1H).

**Example 109**

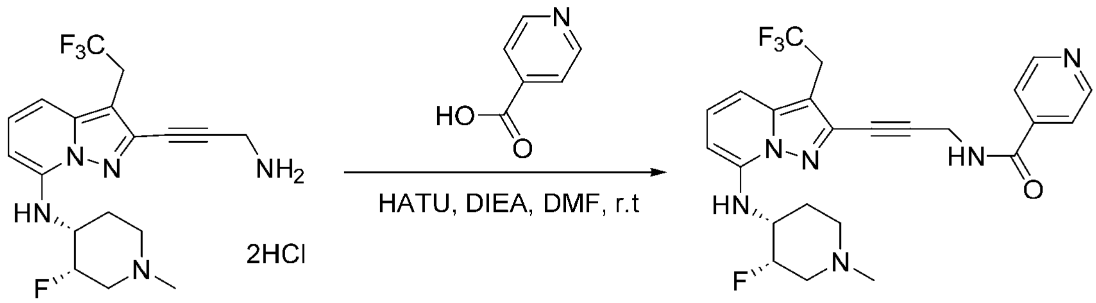


**001-12** **073**

**Step 1: Synthesis of *N*-[3-(7-{[(3*S*,4*R*)-3-fluoro-1-methylpiperidin-4-yl]amino}-3-(2,2,2-trifluoroethyl)pyrazolo [1,5-*a*]pyridin-2-yl)prop-2-yn-1-yl]pyridine-4-carboxamide (compound 073)**

Under nitrogen protection, at room temperature, to a solution of isonicotinic acid (16.19 mg, 0.132 mmol, 1.5 eq), O-(7-azabenzotriazol-1-yl)-*N,N,N',N'*-tetramethyluronium hexafluorophosphate (50.00 mg, 0.132 mmol, 1.5 eq), and N,N-diisopropylethylamine (56.65 mg, 0.440 mmol, 5 eq) in N,N-dimethylformamide (0.8 mL) was added 2-(3-aminoprop-1-yn-1-yl)-N-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12**, 40 mg, 0.088 mmol, 1 eq), and the mixture was further stirred at room temperature for 1 h. After the reaction was completed, methanol (1.0 mL) was added to the reaction mixture for quenching. The crude product was purified by HPLC with the following conditions (column specifications: YMC Triart C18 ExRs 5 μm, 30 mm × 150 mm; mobile phase A: water (10 mmol/L ammonium bicarbonate), mobile phase B: acetonitrile; flow rate: 60 mL/min; gradient: 35% B to 55% B in 10 min; wavelength: UV 254 nm/220 nm; retention time (min): 6.58), to give compound ***N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl) prop-2-yn-1-yl]pyridine-4-carboxamide 073** (18.32 mg, 42.61%).

LCMS: (ESI, m/z): 488.90 $[M+H]^+$.

$^1H$ NMR (400 MHz, DMSO-$d_6$) $\delta$ 9.41 (t, *J* = 5.5 Hz, 1H), 8.76 (dd, J = 4.4, 1.6 Hz, 2H), 7.79 (dd, J = 4.4, 1.6 Hz, 2H), 7.29 (dd, *J* = 8.8, 7.6 Hz, 1H), 7.03 (d, *J* = 8.7 Hz, 1H), 6.32 (d, J = 7.4 Hz, 1H), 6.22 (d, *J* = 9.1 Hz, 1H), 4.87 (d, *J* = 49.4 Hz, 1H), 4.42 (d, *J* = 5.5 Hz, 2H), 3.95 - 3.82 (m, 1H), 3.76 (q, *J* = 11.1 Hz, 2H), 3.06 (t, J = 11.1 Hz, 1H), 2.78 (d, *J* = 11.3 Hz, 1H), 2.38 - 2.22 (m, 1H), 2.20 (s, 3H), 2.14 (t, J = 10.3 Hz, 1H), 1.98 - 1.81 (m, 2H).

**Example 110**

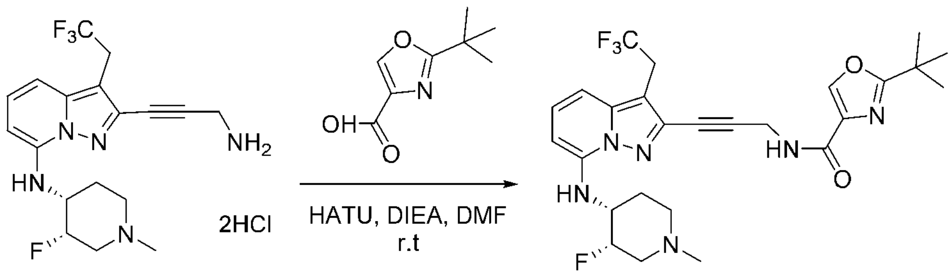


**001-12** **090**

**Step 1: Synthesis of 2-tert-butyl-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1,3-oxazole-4-carboxamide (compound 090)**

Under nitrogen protection, at room temperature, to a solution of O-(7-azabenzotriazol-1-yl)-*N,N,N',N'*-tetramethyluronium hexafluorophosphate (2-(7-azobenzotriazole)-*N,N,N',N'*-tetramethyluronium hexafluorophosphate) (50.00 mg, 0.132 mmol, 1.5 eq), 2-tert-butyl-1,3-oxazole-4-carboxylic acid (22.25 mg, 0.132 mmol, 1.5 eq) and *N,N*-diisopropylethylamine (56.65 mg, 0.440 mmol, 5 eq) in *N,N*-dimethylformamide (0.8 mL) was added 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 40 mg, 0.088 mmol, 1 eq), and the mixture was stirred at room temperature for 2 h. After the reaction was completed, methanol (1.0 mL) was added to the reaction mixture for quenching. The crude product was purified by HPLC with the following conditions (column specifications: YMC Triart C18 ExRs 5 μm, 30 mm × 150 mm;

mobile phase A: water (10 mmol/L ammonium bicarbonate), mobile phase B: acetonitrile; flow rate: 60 mL/min; elution gradient: 47% B to 67% B in 10 min; detection wavelength: UV 254 nm/220 nm; retention time (min): 9.17), to give compound **2-tert-butyl-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1,3-oxazole-4-carboxamide 090** (5.07 mg, 10.78%).

LCMS: (ESI, m/z): 534.90 $[M+H]^+$.

$^1H$ NMR (400 MHz, DMSO-$d_6$, *ppm*) $\delta$ 8.66 (t, *J* = 5.9 Hz, 1H), 8.56 (s, 1H), 7.28 (dd, *J* = 8.8, 7.6 Hz, 1H), 7.02 (d, *J* = 8.7 Hz, 1H), 6.32 (d, *J* = 7.4, 1H), 6.24 (d, *J* = 9.0 Hz, 1H), 4.87 (d, *J* = 49.3 Hz, 1H), 4.32 (d, *J* = 5.8 Hz, 2H), 3.93 - 3.82 (m, 1H), 3.75 (q, *J* = 11.2 Hz, 2H), 3.05 (t, *J* = 11.6 Hz, 1H), 2.78 (d, *J* = 11.3 Hz, 1H), 2.37 - 2.22 (m, 1H), 2.20 (s, 3H), 2.13 (t, *J* = 9.8 Hz, 1H), 1.99 - 1.87 (m, 1H), 1.85 - 1.78 (m, 1H), 1.36 (s, 9H).

**Example 111**

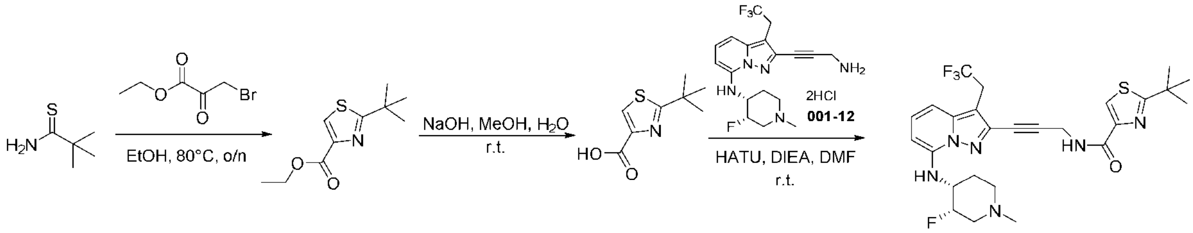


088-1 088-2 088-3 088

**Step 1: Synthesis of compound 088-2**

At 80°C, a solution of 2,2,2-trimethylthiacetamide (compound **088-1**, 1 g, 8.532 mmol, 1 eq) and ethyl bromopropionate (1.66 g, 8.532 mmol, 1 eq) in ethanol (10 mL) was stirred to react overnight. After the reaction was completed, the reaction mixture was quenched with water (100 mL) at room temperature, and extracted with ethyl acetate (3 × 100 mL). The organic phases were combined, backwashed with saturated sodium chloride solution (1 × 100 mL), dried over anhydrous sodium sulfate, filtered, and the filtrate was concentrated under reduced pressure to give compound **088-2** (1.2 g, 65.94%).

LCMS: (ESI, m/z): 213.85 $[M+H]^+$.

**Step 2: Synthesis of compound 088-3**

At room temperature, a solution of ethyl 2-tert-butyl-1,3-thiazole-4-carboxylate (compound **088-2**, 500 mg, 2.344 mmol, 1 eq) and sodium hydroxide (93.89 mg, 2.344 mmol, 1 eq) in methanol (2 mL) and water (1 mL) was stirred to react for 1 h. After the reaction was completed, the resulting residue was concentrated under reduced pressure to give compound **088-3** (400 mg, 92.12%). The resulting mixture was used directly in the next step without further purification.

LCMS:(ESI, m/z):185.85 $[M+H]^+$.

**Step 3: Synthesis of 2-tert-butyl-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1,3-thiazole-4-carboxamide (compound 088)**

At room temperature, a solution of 2-tert-butyl-1,3-thiazole-4-carboxylic acid (compound **088-3**, 20 mg, 0.108 mmol, 1 eq), *N,N*-diisopropylethylamine (55.72 mg, 0.432 mmol, 4 eq) and O-(7-azabenzotriazol-1-yl)-*N,N,N',N'*-tetramethyluronium hexafluorophosphate (41.05 mg, 0.108 mmol, 1 eq) in *N,N*-dimethylformamide (1 mL) was stirred to react for 20 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 49.27 mg, 0.108 mmol, 1.00 eq) was added and the system was further stirred for 1 h. After the reaction was completed, the reaction mixture was poured into water (10 mL) for quenching, and extracted with ethyl acetate (2 × 10 mL). The organic phases were combined, backwashed with sodium chloride aqueous solution (2 × 10 mL), dried over anhydrous sodium sulfate, filtered, and the filtrate was concentrated under reduced pressure. The crude product was purified by HPLC with the following conditions (column specifications: XBridge BEH C18 OBD Prep Column 130, 5 μm, 30 mm × 150 mm; mobile phase A: water (10 mmol/L ammonium bicarbonate), mobile phase B: acetonitrile; flow rate: 60 mL/min; gradient: 43% B to 73% B in 8 min; wavelength: UV 254 nm/220 nm; retention time (min): 7.33), to give compound **2-tert-butyl-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1,3-thiazole-4-carboxamide 088**

(10.4 mg, 17.18%).

LCMS: (ESI, m/z): 550.85 [M+H]⁺.

$^1$H NMR (400 MHz, DMSO-$d_6$, *ppm*) $\delta$ 8.78 (t, *J* = 6.0 Hz, 1H), 8.19 (s, 1H), 7.28 (dd, *J* = 8.8, 7.6 Hz, 1H), 7.02 (d, *J* = 8.7 Hz, 1H), 6.31 (dd, *J* = 7.4 Hz, 1H), 6.25 (d, *J* = 9.1 Hz, 1H), 4.87 (d, *J* = 48 Hz, 1H), 4.37 (d, *J* = 5.9 Hz, 2H), 3.93 - 3.81 (m, 1H), 3.75 (q, *J* = 11.2 Hz, 2H),3.05 (t, *J* = 11.6 Hz, 1H), 2.78 (d, *J* = 11.5 Hz, 1H), 2.34-2.23(m, 1H), 2.20 (s, 3H), 2.13 (t, *J* = 11.3 Hz, 1H), 1.94 (qd, *J* = 11.8, 3.7 Hz, 1H), 1.87 - 1.80 (m, 1H), 1.43 (s, 9H).

**Example 112**

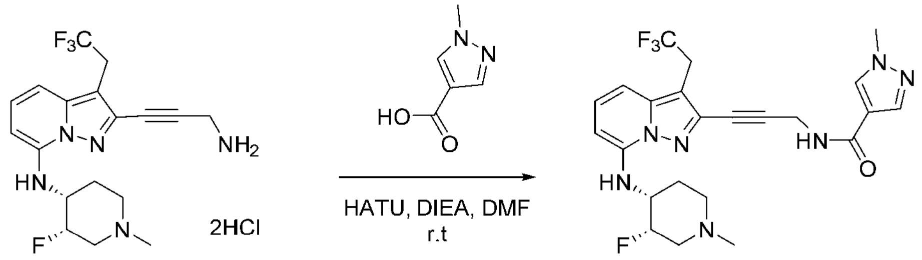


**001-12** **002**

**Step 1: Synthesis of *N*-[3-(7-{[(3*S*,4*R*)-3-fluoro-1-methylpiperidin-4-yl]amino}-3-(2,2,2-trifluoroethyl)pyrazolo [1,5-*a*]pyridin-2-yl)prop-2-yn-1-yl]-1-methyl-1*H*-pyrazole-4-carboxamide (compound 002)**

Under nitrogen protection, at room temperature, to a solution of O-(7-azabenzotriazol-1-yl)-*N,N,N',N'*-tetramethyluronium hexafluorophosphate (33.33 mg, 0.088 mmol, 1.0 eq) and 1-methyl-1*H*-pyrazole-4-carboxylic acid (16.58 mg, 0.132 mmol, 1.5 eq) in*N,N*-dimethylformamide (0.8 mL) was added *N,N*-diisopropylethylamine (56.65 mg, 0.440 mmol, 5 eq). The mixture was stirred to react for 1 h, and then at room temperature, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12**, 40 mg, 0.088 mmol, 1 eq) was added and the mixture was further stirred for 1 h. After the reaction was completed, the reaction mixture was quenched at room temperature with methanol. The crude product was purified by HPLC with the following conditions (column specifications: XBridge BEH Shield RP18 5 μm, 30 mm × 150 mm; mobile phase A: water (10 mmol/L ammonium bicarbonate), mobile phase B: acetonitrile; flow rate: 60 mL/min; elution gradient: 25% B to 50% B in 10 min; detection wavelength: UV 254 nm/220 nm; retention time (min): 8.92), to give compound ***N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl) prop-2-yn-1-yl]-1-methyl-1*H* pyrazole-4-carboxamide 002** (28.22 mg, 65.11%).

LCMS: (ESI, m/z): 492.00 [M+H]⁺.

$^1$H NMR (400 MHz, DMSO-$d_6$, *ppm*) $\delta$ 8.66 (t, *J* = 5.6 Hz, 1H), 8.16 (s, 1H), 7.87 (d, *J* = 0.7 Hz, 1H), 7.28 (dd, *J* = 8.8, 7.6 Hz, 1H), 7.03 (d, *J* = 8.7 Hz, 1H), 6.32 (d, *J* = 7.6 Hz, 1H), 6.24 (d, *J* = 9.0 Hz, 1H), 4.88 (d, *J* = 49.4 Hz, 1H), 4.33 (d, *J* = 5.6 Hz, 2H), 3.86 (s, 4H), 3.75 (q, *J* = 11.1 Hz, 2H), 3.07 (t, J = 11.6 Hz, 1H), 2.79 (d, *J* = 11.2 Hz, 1H), 2.40 - 2.25 (m, 1H), 2.21 (s, 3H), 2.14 (t, *J* = 11.5 Hz, 1H), 1.99 - 1.81 (m, 2H).

**Example 113**

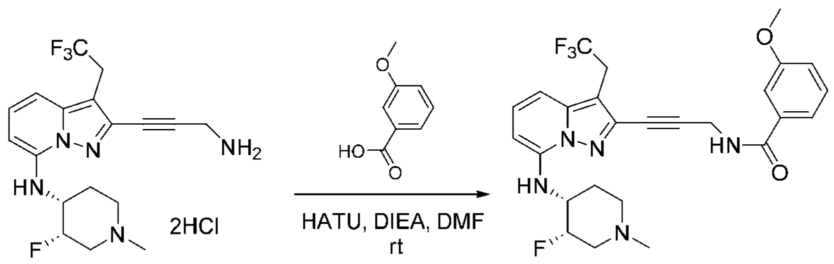


**001-12** **072**

**Step 1: Synthesis of *N*-[3-(7-{[(3*S*,4*R*)-3-fluoro-1-methylpiperidin-4-yl]amino}-3-(2,2,2-trifluoroethyl)pyrazolo [1,5-*a*]pyridin-2-yl)prop-2-yn-1-yl]-3-methoxybenzamide (compound 072)**

At room temperature, a solution of 3-methoxybenzoic acid (12.00 mg, 0.079 mmol, 1.2 eq), *N,N*-diisopropylethylamine (42.49 mg, 0.330 mmol, 5 eq) and O-(7-azabenzothiazol-1-yl)-*N,N,N',N'*-tetramethyluronium hexafluorophosphate (2-(7-azobenzothiazole)-*N,N,N',N'*-tetramethyluronium hexafluorophosphate) (30.00 mg, 0.079 mmol, 1.2 eq) in *N,N*-dimethylformamide (1 mL) was stirred to react for 30 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12**, 30 mg, 0.078 mmol, 1 eq) was added and the system was further stirred for 2 h. After the reaction was completed, the reaction mixture was quenched with water at room temperature, and extracted with ethyl acetate (3 × 10 mL). The organic phases were combined, backwashed with saturated sodium chloride solution (3 × 10 mL), dried over anhydrous sodium sulfate, filtered, and the filtrate was concentrated under reduced pressure. The crude product was purified by HPLC with the following conditions (column specifications: Sunfire C18 5 μm, 30 mm × 150 mm; mobile phase A: water (0.1% ammonium bicarbonate), mobile phase B: acetonitrile; flow rate: 60 mL/min; elution gradient: 42% to 66% in 10 min; detection wavelength: UV 254 nm/220 nm; retention time (min): 9.08), to give compound ***N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-3-methoxybenzamide 072** (22.3 mg, 54.85%).

LCMS: (ESI, m/z): 518.05 $[M+H]^+$.

$^1$H NMR (400 MHz, DMSO-$d_6$, *ppm*) δ 9.08 (t, *J* = 5.5 Hz, 1H), 7.51 - 7.36 (m, 3H), 7.28 (dd, *J* = 8.8, 7.6 Hz, 1H), 7.12 (dd, *J* = 8.1, 2.6, 1.1 Hz, 1H), 7.03 (d, *J* = 8.7 Hz, 1H), 6.32 (d, *J* = 7.6 Hz, 1H), 6.23 (d, *J* = 9.0 Hz, 1H), 4.87 (d, *J* = 49.4 Hz, 1H), 4.39 (d, *J* = 5.5 Hz, 2H), 3.94 - 3.83 (m, 1H), 3.81 (s, 3H), 3.75 (q, J = 11.1 Hz, 2H), 3.06 (t, J = 10.8 Hz, 1H), 2.78 (d, *J* = 11.3 Hz, 1H), 2.29 (dd, J = 38.7, 13.0 Hz, 1H), 2.21 (s, 3H), 2.14 (t, *J* = 11.2 Hz, 1H), 1.98 - 1.80 (m, 2H).

**Example 114**

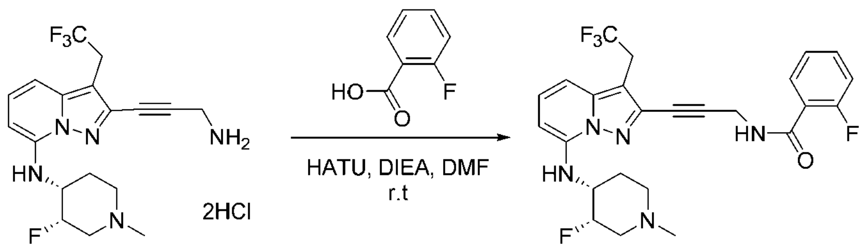


001-12 338

**Step 1: Synthesis of 2-fluoro-*N*-[3-(7-{[(3*S*,4*R*)-3-fluoro-1-methylpiperidin-4-yl]amino}-3-(2,2,2-trifluoroethyl) pyrazolo[1,5-*a*]pyridin-2-yl)prop-2-yn-1-yl]benzamide (compound 338)**

Under nitrogen protection, at room temperature, to a solution of O-(7-azabenzotriazol-1-yl)-*N,N,N',N'*-tetramethyluronium hexafluorophosphate (25.0 mg, 0.066 mmol, 1.0 eq) and 2-fluorobenzoic acid (9.21 mg, 0.066 mmol, 1.5 eq) in *N,N*-dimethylformamide (0.8 mL) were added *N,N*-diisopropylethylamine (42.49 mg, 0.330 mmol, 5 eq) and 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12**, 30 mg, 0.066 mmol, 1 eq), and the mixture was stirred at room temperature for 1 h. After the reaction was completed, methanol (1.0 mL) was added to the reaction mixture for quenching at room temperature. The crude product was purified by HPLC with the following conditions (column specifications: Kinetex 5 μm EVO C18, 30mm × 150 mm; mobile phase A: water (10 mmol/L ammonium bicarbonate), mobile phase B: acetonitrile; flow rate: 60 mL/min; elution gradient: 35% B to 62% B in 8 min; detection wavelength: UV 254 nm/220 nm; retention time (min): 6.52), to give compound **2-fluoro-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]benzamide 338** (11.26 mg, 50.57%).

LCMS: (ESI, m/z): 506.05 $[M+H]^+$.

$^1$H NMR (400 MHz, DMSO-$d_6$, *ppm*) δ 8.92 (t, J = 4.4 Hz, 1H), 7.66 (td, *J* = 7.5, 1.8 Hz, 1H), 7.60 - 7.51 (m, 1H), 7.40 - 7.22 (m, 3H), 7.03 (d, *J* = 8.7 Hz, 1H), 6.32 (d, J = 7.5 Hz, 1H), 6.24 (d, *J* = 9.0 Hz, 1H), 4.88 (d, *J* = 49.5 Hz, 1H), 4.38 (d, *J* = 5.6 Hz, 2H), 3.95 - 3.82 (m, 1H), 3.77 (q, *J* = 11.1 Hz, 2H), 3.06 (t, *J* = 11.0 Hz, 1H), 2.78 (d, *J* = 11.5 Hz, 1H), 2.38 - 2.22 (m, 1H), 2.20 (s, 3H), 2.14 (t, *J* = 11.1 Hz, 1H), 2.00 - 1.79 (m, 2H).

**Example 115**

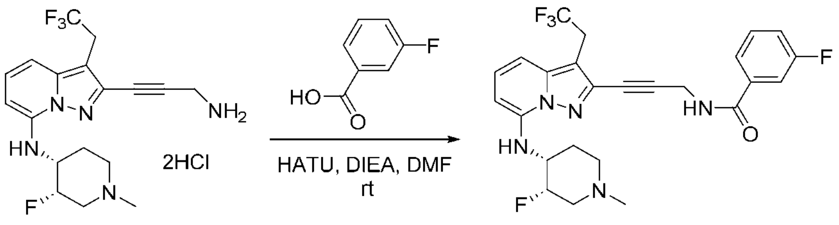


**001-12** **339**

**Step 1: Synthesis of 3-fluoro-*N*-[3-(7-{[(3*S*,4*R*)-3-fluoro-1-methylpiperidin-4-yl]amino}-3-(2,2,2-trifluoroethyl) pyrazolo[1,5-*a*]pyridin-2-yl)prop-2-yn-1-yl]benzamide (compound 339)**

At room temperature, a solution of 3-fluorobenzoic acid (14.74 mg, 0.106 mmol, 1.2 eq)), *N,N*-diisopropylethylamine (56.65 mg, 0.440 mmol, 5 eq) and O-(7-azabenzothiazol-1-yl)-*N,N,N',N'*-tetramethyluronium hexafluorophosphate (2-(7-azobenzothiazole)-*N,N,N',N'*-tetramethyluronium hexafluorophosphate) (40.00 mg, 0.106 mmol, 1.2 eq) in *N,N*-dimethylformamide (1 mL) was stirred to react for 30 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-a]pyridin-7-amine dihydrochloride (compound **001-12, 40** mg, 0.104 mmol, 1 eq) was added and the system was further stirred for 2 h. After the reaction was completed, the reaction mixture was quenched with water at room temperature, and extracted with ethyl acetate (3 × 10 mL). The organic phases were combined, backwashed with saturated sodium chloride solution (3 × 10 mL), dried over anhydrous sodium sulfate, filtered, and the filtrate was concentrated under reduced pressure. The crude product was purified by HPLC with the following conditions (column specifications: Sunfire C18 5 μm, 30 mm × 150 mm; mobile phase A: water (0.1% ammonium bicarbonate), mobile phase B: acetonitrile; flow rate: 60 mL/min; elution gradient: 41% to 71% in 8 min; detection wavelength: UV 254 nm/220 nm; retention time (min): 7.2), to give compound **3-fluoro*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]benzamide 339** (10.53 mg, 23.55%).

LCMS: (ESI, m/z): 506.05 $[M+H]^+$.

$^1$H NMR (400 MHz, DMSO-$d_6$, *ppm*) $\delta$ 9.20 (t, J = 4.9 Hz, 1H), 7.75 (d, *J* = 7.7 Hz, 1H), 7.68 (d, J = 10.0 Hz, 1H), 7.56 (qd, *J* = 8.0, 5.8 Hz, 1H), 7.42 (td, *J* = 8.4, 2.7 Hz, 1H), 7.33 - 7.23 (m, 1H), 7.03 (d, *J* = 8.7 Hz, 1H), 6.32 (d, *J* = 7.6 Hz, 1H), 6.22 (d, *J* = 9.0 Hz, 1H), 4.87 (d, *J* = 49.6 Hz, 1H), 4.40 (d, *J* = 5.4 Hz, 2H), 3.94 - 3.82 (m, 1H), 3.76 (q, *J* = 11.0 Hz, 2H), 3.05 (t, *J* = 11.2 Hz, 1H), 2.78 (d, *J* = 11.3 Hz, 1H), 2.38 - 2.24 (m, 1H), 2.20 (s, 3H), 2.14 (t, J = 10.4 Hz, 1H), 1.96 - 1.79 (m, 2H).

**Example 116**

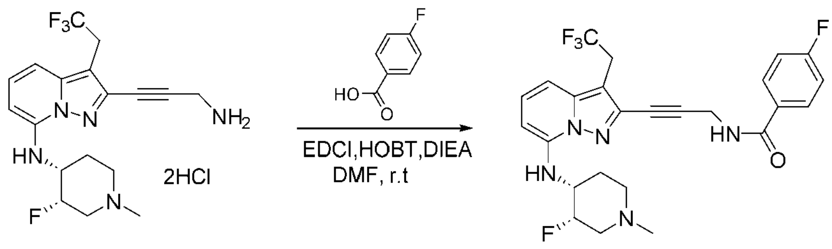


**001-12** **076**

**Step 1: Synthesis of 4-fluoro-*N*-[3-(7-{[(3*S*,4*R*)-3-fluoro-1-methylpiperidin-4-yl]amino}-3-(2,2,2-trifluoroethyl) pyrazolo[1,5-*a*]pyridin-2-yl)prop-2-yn-1-yl]benzamide (compound 076)**

Under nitrogen protection, 4-fluorobenzoic acid (15.35 mg, 0.109 mmol, 1.2 eq), 1-ethyl-(3-dimethylaminopropyl)carbodiimide hydrochloride (26.25 mg, 0.137 mmol, 1.5 eq), 1-hydroxy-benzotriazole (18.5 mg, 0.137 mmol, 1.5 eq), and *N, N*-diisopropylethylamine (58.99 mg, 0.455 mmol, 5 eq) were added to a reaction flask, dissolved in *N, N*-dimethylformamide (2 mL) and stirred at room temperature for 10 min. Then, 2-(3-aminoprop-1-yn-1-yl)-*N*-((3*S*,4*R*)-3-

fluoro-1-methylpiperidin-4-yl)-3-(2,2,2-trifluoroethyl)pyrazolo[1,5-a]pyridin-7-amine dihydrochloride **(001-12,** 35 mg, 0.091 mmol, 1 eq) was added, and the system was stirred to react at room temperature for 1 h. After the reaction was completed, water (10 mL) was added to the reaction mixture and the system was extracted with ethyl acetate (20 mL × 2). The organic phases were combined, backwashed with saturated sodium chloride solution (10 mL × 3), dried over anhydrous sodium sulfate, and filtered, and the filtrate was concentrated under reduced pressure. The crude product was purified by HPLC with the following conditions (column specifications: XBridge BEH Shield RP18 5 μm, 30 mm *150 mm; mobile phase A: water (10 mmol/L ammonium bicarbonate), mobile phase B: acetonitrile; flow rate: 60 mL/min; elution gradient: 33% B to 63% B in 10 min; detection wavelength: UV 254 nm/220 nm; retention time (min): 9.38) to give compound **4-fluoro-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]benzamide 076** (16.3 mg, 34.19%).

LCMS: (ESI, m/z): 505.85 $[M+H]^+$.

$^1$H NMR (400 MHz, DMSO-$d_6$, *ppm*) $\delta$ 9.13 (t, *J* = 5.5 Hz, 1H), 7.99 - 7.94 (m, 2H), 7.37 - 7.25 (m, 3H), 7.03 (d, *J* = 8.6 Hz, 1H), 6.32 (d, *J* = 7.4 Hz, 1H), 6.23 (d, *J* = 9.1 Hz, 1H), 4.87 (d, *J* = 49.1 Hz, 1H), 4.39 (d, *J* = 5.5 Hz, 2H), 3.93 - 3.83 (m, 1H), 3.76 (q, *J* = 11.1 Hz, 2H), 3.05 (t, *J* = 10.9 Hz, 1H), 2.78 (d, *J* = 10.9 Hz, 1H), 2.37 - 2.22 (m, 1H), 2.20 (s, 3H), 2.13 (t, J = 10.4 Hz, 1H), 1.99 - 1.79 (m, 2H).

**Example 117**

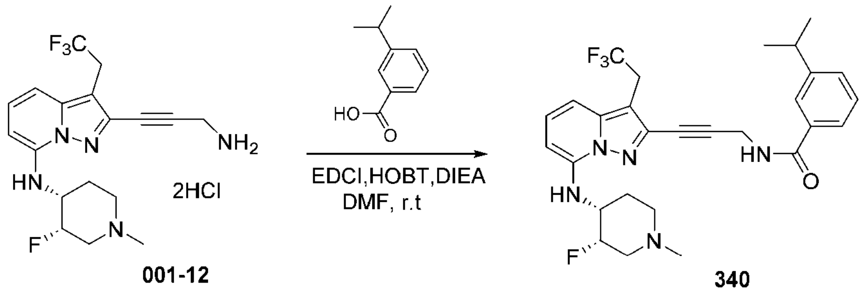


**001-12** **340**

**Step 1: Synthesis of *N*-(3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl)-3-isopropylbenzamide (compound 340)**

Under nitrogen protection, 3-isopropylbenzoic acid (12.85 mg, 0.078 mmol, 1.5 eq), 1-ethyl-(3-dimethylaminopropyl)carbodiimide hydrochloride (15.00 mg, 0.078 mmol, 1.5 eq), 1-hydroxy-benzotriazole (10.57 mg, 0.078 mmol, 1.5 eq), and *N, N*-diisopropylethylamine (33.71 mg, 0.260 mmol, 5 eq) were added to a reaction flask, dissolved in *N, N*-dimethylformamide (2 mL) and stirred at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (**001-12**, 20 mg, 0.052 mmol, 1 eq) was added, and the system was further stirred to react for 1 h. After the reaction was completed, the reaction mixture was quenched with water (10 mL) and extracted with ethyl acetate (20 mL × 2). The organic phases were combined, backwashed with saturated sodium chloride solution (10 mL × 3), dried over anhydrous sodium sulfate, and filtered, and the filtrate was concentrated under reduced pressure. The crude product was purified by HPLC with the following conditions (column specifications: XBridge BEH Shield RP18 5 μm, 30 mm *150 mm; mobile phase A: water (10 mmol/L ammonium bicarbonate), mobile phase B: acetonitrile; flow rate: 60 mL/min; elution gradient: 45% B to 68% B in 10 min; detection wavelength: UV 254 nm/220 nm; retention time (min): 8.8) to give compound ***N*-(3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl)-3-isopropylbenzamide 340** (8.14 mg, 29.32%).

LCMS: (ESI, m/z): 529.90 $[M+H]^+$.

$^1$H NMR (400 MHz, DMSO-$d_6$, *ppm*) $\delta$ 9.05 (t, *J* = 5.6 Hz, 1H), 7.78 (s, 1H), 7.70 (dt, *J* = 7.1, 1.8 Hz, 1H), 7.45 - 7.36 (m, 2H), 7.28 (dd, *J* = 8.8, 7.6 Hz, 1H), 7.02 (d, *J* = 8.6 Hz, 1H), 6.32 (d, J = 7.5 Hz, 1H), 6.23 (d, *J* = 9.1 Hz, 1H), 4.87 (d, *J* = 49.5 Hz, 1H), 4.39 (d, *J* = 5.5 Hz, 2H), 3.93 - 3.83 (m, 1H), 3.76 (q, *J* = 11.0 Hz, 2H), 3.05 (t, *J* = 11.2 Hz, 1H), 2.95 (p, *J* = 6.9 Hz, 1H), 2.78 (d, J = 11.0 Hz, 1H), 2.36 - 2.22 (m, 1H), 2.20 (s, 3H), 2.13 (t, J = 10.6 Hz, 1H), 1.99 - 1.80 (m, 2H), 1.24 (d, *J* = 6.9 Hz, 6H).

**Example 118**

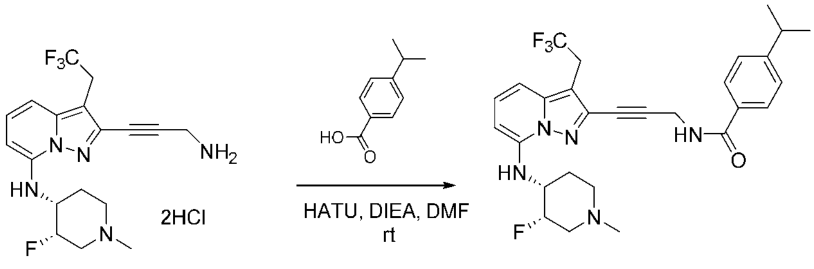


**001-12** **341**

**Step 1: Synthesis of *N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-4-isopropylbenzamide (compound 341)**

At room temperature, a solution of 4-isopropylbenzoic acid (10.8 mg, 0.066 mmol, 1.2 eq), O-(7-azabenzotriazol-1-yl)-*N,N,N',N'*-tetramethyluronium hexafluorophosphate (25.0 mg, 0.066 mmol, 1.2 eq) and *N,N*-diisopropylethylamine (14.16 mg, 0.110 mmol, 2 eq) and in *N,N*-dimethylformamide (1 mL) was stirred to react for 30 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12**, 25 mg, 0.055 mmol, 1 eq) was added and the system was further stirred for 2 h. After the reaction was completed, the reaction mixture was quenched with water at room temperature, and extracted with ethyl acetate (3 × 10 mL). The organic phases were combined, backwashed with saturated sodium chloride solution (3 × 10 mL), dried over anhydrous sodium sulfate, filtered, and the filtrate was concentrated under reduced pressure. The crude product was purified by HPLC with the following conditions (column specifications: Sunfire C18 5 μm, 30 mm × 150 mm; mobile phase A: water (0.1% ammonium bicarbonate), mobile phase B: acetonitrile; flow rate: 60 mL/min; elution gradient: 42% to 68% in 10 min; detection wavelength: UV 254 nm/220 nm; retention time (min): 8.08), to give compound ***N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-4-isopropylbenzamide 341** (11.17 mg, 38.23%).

LCMS: (ESI, m/z): 530.25 $[M+H]^+$.

$^1$H NMR (400 MHz, DMSO-$d_6$, *ppm*) δ 8.99 (t, *J* = 5.6 Hz, 1H), 7.82 (d, *J* = 8.2 Hz, 2H), 7.36 (d, *J* = 8.2 Hz, 2H), 7.28 (t, *J* = 8.2 Hz, 1H), 7.02 (d, *J* = 8.7 Hz, 1H), 6.31 (d, *J* = 7.6 Hz, 1H), 6.23 (d, *J* = 9.0 Hz, 1H), 4.87 (d, *J* = 49.4 Hz, 1H), 4.38 (d, *J* = 5.5 Hz, 2H), 3.95 - 3.81 (m, 1H), 3.76 (q, *J* = 11.1 Hz, 2H), 3.05 (t, *J* = 10.7 Hz, 1H), 2.94 (h, *J* = 6.9 Hz, 1H), 2.78 (d, J = 10.7 Hz, 1H), 2.28 (dd, J = 39.8, 14.3 Hz, 1H), 2.20 (s, 3H), 2.14 (t, J = 10.4 Hz, 1H), 1.99 - 1.78 (m, 2H), 1.22 (d, *J* = 6.9 Hz, 6H).

**Example 119**

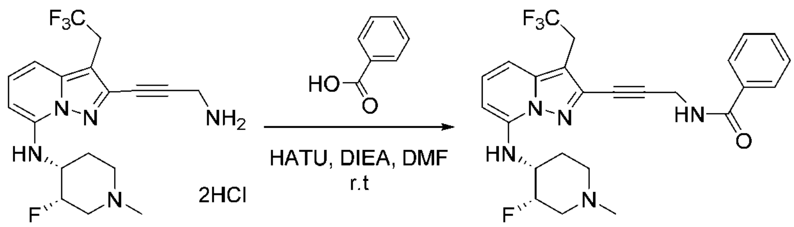


**001-12** **069**

**Step 1: Synthesis of *N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]benzamide (compound 069)**

Under nitrogen protection, at room temperature, a solution of O-(7-azabenzotriazol-1-yl)-*N,N,N',N'*-tetramethyluronium hexafluorophosphate (2-(7-azobenzotriazole)-*N,N,N',N'*-tetramethyluronium hexafluorophosphate) (37.5 mg, 0.099 mmol, 1.5 eq), benzoic acid (12.04 mg, 0.099 mmol, 1.5 eq), and *N,N*-diisopropylethylamine (42.49 mg, 0.330 mmol, 5 eq) in *N,N*-dimethylformamide (0.7 mL) was stirred to react for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12**, 30 mg, 0.066 mmol, 1 eq) was added and the mixture was further stirred for 1 h. After the reaction was completed, methanol (1.0 mL) was added to the reaction mixture for quenching. The crude product was purified by HPLC with the following conditions (column specifications: XBridge BEH C18 OBD Prep Column 130, 5 μm, 30 mm × 150 mm; mobile phase A: water (10 mmol/L ammonium bicarbonate), mobile phase B: acetonitrile; flow rate: 60 mL/min; elution gradient: 35% B to

59% B in 8 min; detection wavelength: UV 254 nm/220 nm; retention time (min): 7.2), to give compound ***N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl) prop-2-yn-1-yl]benzamide 069** (9.02 mg, 28.03%).

LCMS: (ESI, m/z): 487.85 $[M+H]^+$.

$^1$H NMR (400 MHz, DMSO-$d_6$, *ppm)* $\delta$ 9.09 (t, *J* = 5.6 Hz, 1H), 7.93 - 7.86 (m, 2H), 7.56 (t, J = 7.3 Hz, 1H), 7.49 (t, J = 7.4 Hz, 2H), 7.28 (d, J = 8.1 Hz, 1H), 7.02 (d, *J* = 8.6 Hz, 1H), 6.32 (d, *J* = 7.6 Hz, 1H), 6.23 *(d, J* = 9.1 Hz, 1H), 4.87 (d, *J* = 49.3 Hz, 1H), 4.39 (d, *J* = 5.5 Hz, 2H), 3.94 - 3.81 (m, 1H), 3.76 (q, *J* = 11.1 Hz, 2H), 3.05 (t, *J* = 11.4 Hz, 1H), 2.78 (d, *J* = 11.4 Hz, 1H), 2.37 - 2.21 (m, 1H), 2.20 (s, 3H), 2.13 (t, J = 10.6 Hz, 1H), 2.00 - 1.78 (m, 2H).

**Example 120**

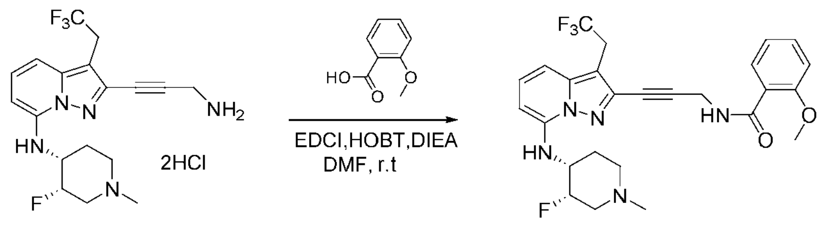


**001-12** **071**

**Step 1: Synthesis of *N*-[3-(7-{[(3*S*,4*R*)-3-fluoro-1-methylpiperidin-4-yl]amino}-3-(2,2,2-trifluoroethyl)pyrazolo [1,5-*a*]pyridin-2-yl)prop-2-yn-1-yl]-2-methoxybenzamide (compound 071)**

Under nitrogen protection, 2-methoxybenzoic acid (14.29 mg, 0.094 mmol, 1.2 eq), 1-ethyl-(3-dimethylaminopropyl)carbodiimide hydrochloride (22.5 mg, 0.117 mmol, 1.5 eq), 1-hydroxy-benzotriazole (15.86 mg, 0.117 mmol, 1.5 eq), and *N, N*-diisopropylethylamine (50.57 mg, 0.390 mmol, 5 eq) were added to a reaction flask, dissolved in *N, N*-dimethylformamide (2 mL) and stirred at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-a]pyridin-7-amine dihydrochloride (**001-12**, 30 mg, 0.078 mmol, 1 eq) was added, and the system was further stirred to react for 1 h. After the reaction was completed, water (10 mL) was added for quenching, and the reaction mixture was extracted with ethyl acetate (20 mL × 2). The organic phases were combined, backwashed with saturated sodium chloride solution (10 mL × 3), dried over anhydrous sodium sulfate, and filtered, and the filtrate was concentrated under reduced pressure. The crude product was purified by HPLC with the following conditions (column specifications: XBridge BEH Shield RP18 5 μm, 30 mm *150 mm; mobile phase A: water (10 mmol/L ammonium bicarbonate), mobile phase B: acetonitrile; flow rate: 60 mL/min; elution gradient: 35% B to 61% B in 10 min; detection wavelength: UV 254 nm/220 nm; retention time (min): 9.52) to give compound ***N*-[3-(7-{[(3*S*,4*R*)-*3*-fluoro-1-methylpiperidin-4-yl]amino}-3-(2,2,2-trifluoroethyl)pyrazolo[1,5-*a*]pyridin-2-yl) prop-2-yn-1-yl]-2-methoxybenzamide 071** (18.36 mg, 45.25%).

LCMS: (ESI, m/z): 517.85 $[M+H]^+$.

$^1$H NMR (400 MHz, *DMSO-$d_6$, ppm*) $\delta$ 8.70 (t, *J* = 5.6 Hz, 1H), 7.80 (dd, *J* = 7.7, 1.9 Hz, 1H), 7.54 - 7.43 (m, 1H), 7.28 (dd, *J* = 8.8, 7.6 Hz, 1H), 7.16 (dd, *J* = 8.5, 1.0 Hz, 1H), 7.07 - 7.01 (m, 2H), 6.31 (d, *J* = 7.5 Hz, 1H), 6.26 (d, *J* = 9.0 Hz, 1H), 4.88 (d, *J* = 49.3 Hz, 1H), 4.39 (d, *J* = 5.6 Hz, 2H), 3.91 (s, 3H), 3.89 - 3.82 (m, 1H), 3.76 (q, *J* = 11.1 Hz, 2H), 3.07 (t, J = 10.2 Hz, 1H), 2.80 (d, *J* = 11.4 Hz, 1H), 2.40 - 2.25 (m, 1H), 2.22 (s, 3H), 2.15 (t, *J* = 11.7 Hz, 1H), 2.00 - 1.82 (m, 2H).

**Example 121**

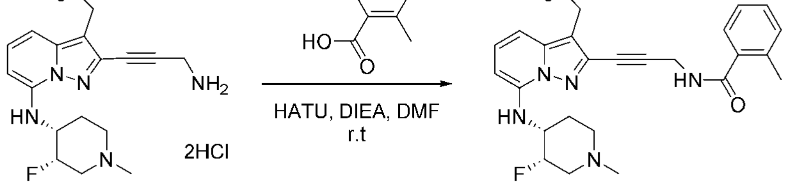


**001-12** **342**

**Step 1: Synthesis of *N*-[3-(7-{[(3*S*,4*R*)-3-fluoro-1-methylpiperidin-4-yl]amino}-3-(2,2,2-trifluoroethyl)pyrazolo [1,5-*a*]pyridin-2-yl)prop-2-yn-1-yl]-2-methylbenzamide (compound 342)**

Under nitrogen protection, at room temperature, to a solution of O-(7-azabenzotriazol-1-yl)-*N,N,N',N'*-tetramethyluronium hexafluorophosphate (25.0 mg, 0.066 mmol, 1.0 eq) and o-toluic acid (13.43 mg, 0.099 mmol, 1.5 eq) in *N,N*-dimethylformamide (0.8 mL) were added *N,N*-diisopropylethylamine (42.49 mg, 0.330 mmol, 5 eq) and then 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12**, 30 mg, 0.066 mmol, 1 eq) was added, and the mixture was further stirred at room temperature for 1 h. After the reaction was completed, methanol (1.0 mL) was added to the reaction mixture for quenching. The crude product was purified by HPLC with the following conditions (column specifications: XBridge BEH Shield RP18 5 μm, 30 mm × 150 mm; mobile phase A: water (10 mmol/L ammonium bicarbonate), mobile phase B: acetonitrile; flow rate: 60 mL/min; elution gradient: 33% B to 63% B in 10 min; detection wavelength: UV 254 nm/220 nm; retention time (min): 9.27), to give compound ***N*-[3-(7-{[(3*S*,4*R*)-3-fluoro-1-methylpiperidin-4-yl]amino}-3-(2,2,2-trifluoroethyl)pyrazolo [1,5-*a*]pyridin-2-yl)prop-2-yn-1-yl]-2-methylbenzamide 342** (19.52 mg, 58.90%).
LCMS: (ESI, m/z): 502.10 $[M+H]^+$.
$^1H$ NMR (400 MHz, DMSO-$d_6$, *ppm*) $\delta$ 8.84 (t, *J* = 5.6 Hz, 1H), 7.43 - 7.15 (m, 4H), 7.03 (d, *J* = 8.7 Hz, 1H), 6.33 (d, *J* = 7.7 Hz, 1H), 6.23 (d, *J* = 9.0 Hz, 1H), 4.88 (d, *J* = 49.4 Hz, 1H), 4.35 (d, *J* = 5.6 Hz, 2H), 3.92 - 3.84 (m, 1H), 3.77 (q, *J* = 11.1 Hz, 2H), 3.07 (t, J = 10.2 Hz, 1H), 2.79 (d, *J* = 11.3 Hz, 1H), 2.36 (s, 3H), 2.33 (p, *J* = 1.8 Hz, 1H), 2.30 - 2.22 (m, 1H), 2.21 (s, 3H), 2.18 - 2.10 (m, 1H), 2.00 - 1.80 (m, 2H).

**Example 122**

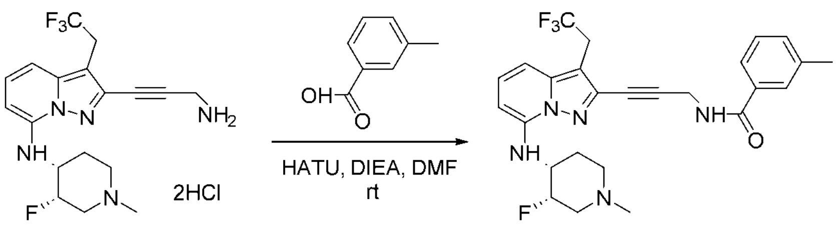


001-12 161

**Step 1: Synthesis of *N*-[3-(7-{[(3*S*,4*R*)-3-fluoro-1-methylpiperidin-4-yl]amino}-3-(2,2,2-trifluoroethyl)pyrazolo [1,5-*a*]pyridin-2-yl)prop-2-yn-1-yl]-3-methylbenzamide (compound 161)**

At room temperature, a solution of 3-methylbenzoic acid (10.74 mg, 0.079 mmol, 1.2 eq), *N,N*-diisopropylethylamine (42.49 mg, 0.330 mmol, 5 eq) and O-(7-azabenzothiazol-1-yl)-*N,N,N',N'*-tetramethyluronium hexafluorophosphate (2-(7-azobenzothiazole)-*N,N,N',N'*-tetramethyluronium hexafluorophosphate) (30.00 mg, 0.079 mmol, 1.2 eq) in *N,N*-dimethylformamide (1 mL) was stirred to react for 30 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12**, 30 mg, 0.078 mmol, 1 eq) was added and the system was further stirred to react for 2 h. After the reaction was completed, the reaction mixture was quenched with water at room temperature, and extracted with ethyl acetate (3 × 10 mL). The organic phases were combined, backwashed with saturated sodium chloride solution (3 × 10 mL), dried over anhydrous sodium sulfate, filtered, and the filtrate was concentrated under reduced pressure. The crude product was purified by HPLC with the following conditions (column specifications: Sunfire C18 5 μm, 30 mm × 150 mm; mobile phase A: water (0.1% ammonium bicarbonate), mobile phase B: acetonitrile; flow rate: 60 mL/min; elution gradient: 42% to 72% in 8 min; detection wavelength: UV 254 nm/220 nm; retention time (min): 7.6), to give compound ***N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-3-methylbenzamide 161** (11.15 mg, 33.71%).
LCMS: (ESI, m/z): 502.25 $[M+H]^+$.
$^1H$ NMR (400 MHz, DMSO-$d_6$, *ppm*) $\delta$ 9.03 (t, *J* = 5.6 Hz, 1H), 7.71 (s, 1H), 7.69 - 7.64 (m, 1H), 7.37 (d, J = 5.2 Hz, 2H), 7.28 (dd, *J* = 8.8, 7.6 Hz, 1H), 7.02 (d, *J* = 8.7 Hz, 1H), 6.32 (d, *J* = 7.7 Hz, 1H), 6.23 (d, *J* = 9.0 Hz, 1H), 4.87 (d, *J* = 49.5 Hz, 1H), 4.38 (d, *J* = 5.5 Hz, 2H), 3.93 - 3.82 (m, 1H), 3.76 (q, *J* = 11.0 Hz, 2H), 3.05 (t, *J* = 11.7 Hz, 1H), 2.78 (d, *J* = 11.4 Hz, 1H), 2.37 (s, 3H), 2.35 - 2.22 (m, 1H), 2.20 (s, 3H), 2.13 (t, *J* = 11.2 Hz, 1H), 1.98 - 1.79 (m, 2H).

**Example 123**

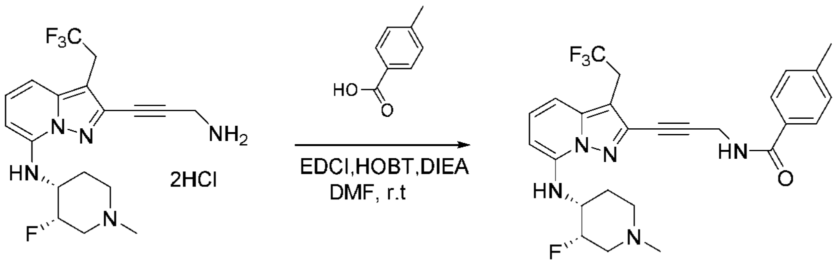


**001-12** **160**

**Step 1: Synthesis of *N*-[3-(7-{[(3*S*,4*R*)-3-fluoro-1-methylpiperidin-4-yl]amino}-3-(2,2,2-trifluoroethyl)pyrazolo [1,5-*a*]pyridin-2-yl)prop-2-yn-1-yl]-4-methylbenzamide (compound** 160)

Under nitrogen protection, 4-methylbenzoic acid (14.91 mg, 0.109 mmol, 1.2 eq), 1-ethyl-(3-dimethylaminopropyl)carbodiimide hydrochloride (26.25 mg, 0.137 mmol, 1.5 eq), 1-hydroxy-benzotriazole (18.5 mg, 0.137 mmol, 1.5 eq), and *N, N*-diisopropylethylamine (58.99 mg, 0.455 mmol, 5 eq) were added to a reaction flask, dissolved in *N, N*-dimethylformamide (2 mL) and stirred at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (**001-12**, 35 mg, 0.091 mmol, 1 eq) was added, and the system was further stirred to react for 1 h. After the reaction was completed, water (10 mL) was added for quenching, and the reaction mixture was extracted with ethyl acetate (20 mL × 2). The organic phases were combined, backwashed with saturated sodium chloride solution (10 mL × 3), dried over anhydrous sodium sulfate, and filtered, and the filtrate was concentrated under reduced pressure. The crude product was purified by HPLC with the following conditions (column specifications: Xselect CSHTM Prep C18 5μm 30*150mm OBD; mobile phase A: water (0.1 formic acid), mobile phase B: acetonitrile; flow rate: 60 mL/min; elution gradient: 12% B to 42% B in 8 min; detection wavelength: UV 254 nm/220 nm; retention time (min): 6.62) to give compound ***N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-4-methylbenzamide 160** (21.9 mg, 46.35%).

LCMS: (ESI, m/z): 501.90 $[M+H]^+$.

$^1H$ NMR (400 MHz, DMSO-$d_6$, *ppm)* $\delta$ 8.98 (t, *J* = 5.5 Hz, 1H), 7.79 (d, J = 8.2 Hz, 2H), 7.31 - 7.26 (m, 3H), 7.01 (d, *J* = 8.7 Hz, 1H), 6.31 (d, J = 7.5 Hz, 1H), 6.22 (d, *J* = 9.0 Hz, 1H), 4.87 (d, *J* = 49.3 Hz, 1H), 4.37 (d, *J* = 5.6 Hz, 2H), 3.93 - 3.81 (m, 1H), 3.75 (q, *J* = 11.1 Hz, 2H), 3.05 (t, *J* = 11.2 Hz, 1H), 2.78 (d, *J* = 11.4 Hz, 1H), 2.35 (s, 3H), 2.28 - 2.21 (m, 1H), 2.20 (s, 3H), 2.14 (t, J = 10.5 Hz, 1H), 1.98 - 1.90 (m, 1H), 1.89 - 1.78 (m, 1H).

**Example 124**

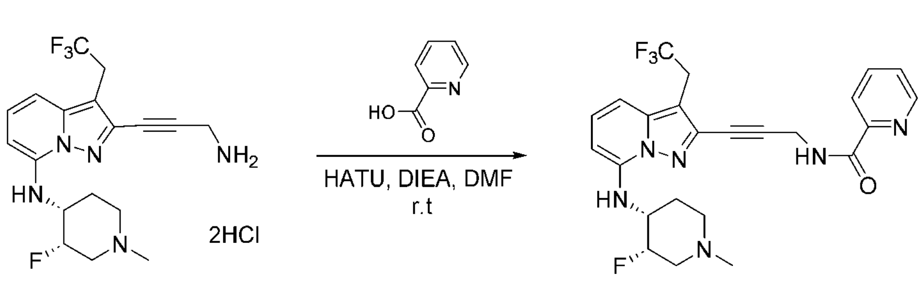


**001-12** **075**

**Step 1: Synthesis of *N*-[3-(7-{[(3*S*,4*R*)-3-fluoro-1-methylpiperidin-4-yl]amino}-3-(2,2,2-trifluoroethyl)pyrazolo [1,5-*a*]pyridin-2-yl)prop-2-yn-1-yl]pyridine-2-carboxamide (compound 075)**

Under nitrogen protection, to a solution of O-(7-azabenzotriazol-1-yl)-*N,N,N',N'*-tetramethyluronium hexafluorophosphate (16.67 mg, 0.044 mmol, 1.0 eq) and 2-pyridinecarboxylic acid (8.09 mg, 0.066 mmol, 1.5 eq) in *N,N*-dimethylformamide (0.5 mL) was added *N,N*-diisopropylethylamine (28.32 mg, 0.220 mmol, 5 eq), and the mixture was

stirred to react for 30 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12**, 20 mg, 0.044 mmol, 1 eq) was added and the system was further stirred for 1 h. After the reaction was completed, the reaction mixture was quenched with methanol (1.0 mL) at room temperature. The crude product was purified by HPLC with the following conditions (column specifications: XBridge BEH C18 OBD Prep Column 130, 5 μm, 30 mm × 150 mm; mobile phase A: water (10 mmol/L ammonium bicarbonate), mobile phase B: acetonitrile; flow rate: 60 mL/min; elution gradient: 30% B to 60% B in 8 min; detection wavelength: UV 254 nm/220 nm; retention time (min): 7.53), to give compound ***N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]pyridine-2-carboxamide 075** (7.84 mg, 36.43%).

LCMS: (ESI, m/z): 489.05 $[M+H]^+$.

$^1$H NMR (400 MHz, DMSO-$d_6$, *ppm*) δ 9.33 (t, *J* = 6.0 Hz, 1H), 8.72 - 8.64 (m, 1H), 8.08 (dt, *J* = 7.8, 1.2 Hz, 1H), 8.02 (td, *J* = 7.6, 1.7 Hz, 1H), 7.63 (ddd, *J* = 7.4, 4.8, 1.4 Hz, 1H), 7.28 (dd, *J* = 8.8, 7.6 Hz, 1H), 7.01 (d, *J* = 8.7 Hz, 1H), 6.31 (d, J = 7.3 Hz, 1H), 6.23 (d, *J* = 9.0 Hz, 1H), 4.86 (d, *J* = 49.4 Hz, 1H), 4.40 (d, *J* = 6.0 Hz, 2H), 3.91 - 3.81 (m, 1H), 3.75 (q, *J* = 11.1 Hz, 2H), 3.05 (t, *J* = 11.3 Hz, 1H), 2.77 (d, *J* = 11.5 Hz, 1H), 2.36 - 2.21 (m, 1H), 2.20 (s, 3H), 2.13 (t, *J* = 11.2 Hz, 1H), 1.99 - 1.80 (m, 2H).

Example 125

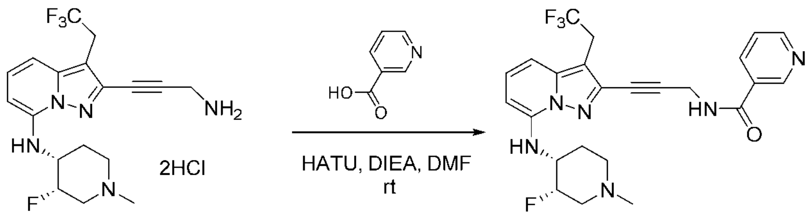


**001-12** **074**

**Step 1: Synthesis of *N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]nicotinamide (compound 074)**

At room temperature, a solution of nicotinic acid (9.71 mg, 0.079 mmol, 1.2 eq), *N,N*-diisopropylethylamine (42.49 mg, 0.330 mmol, 5 eq) and O-(7-azabenzotriazol-1-yl)-*N,N,N',N'*-tetramethyluronium hexafluorophosphate (30.00 mg, 0.079 mmol, 1.2 eq) in *N,N*-dimethylformamide (1 mL) was stirred to react for 30 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12**, 30 mg, 0.066 mmol, 1 eq) was added and the system was allowed to react at room temperature for 2 h. After the reaction was completed, the reaction mixture was quenched with water at room temperature, and extracted with ethyl acetate (3 × 10 mL). The organic phases were combined, backwashed with saturated sodium chloride solution (3 × 10 mL), dried over anhydrous sodium sulfate, filtered, and the filtrate was concentrated under reduced pressure. The crude product was purified by HPLC with the following conditions (column specifications: Sunfire C18 5 μm, 30 mm × 150 mm; mobile phase A: water (0.1% ammonium bicarbonate), mobile phase B: acetonitrile; flow rate: 60 mL/min; elution gradient: 31% to 53% in 10 min; detection wavelength: UV 254 nm/220 nm; retention time (min): 8.57), to give compound ***N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]nicotinamide 074** (18.84 mg, 58.43%).

LCMS: (ESI, m/z): 489.10 $[M+H]^+$.

$^1$H NMR (400 MHz, DMSO-$d_6$, *ppm*) δ 9.30 (t, *J* = 5.4 Hz, 1H), 9.04 (d, *J* = 1.6 Hz, 1H), 8.73 (dd, *J* = 4.8, 1.7 Hz, 1H), 8.26 - 8.17 (m, 1H), 7.54 (ddd, *J* = 7.9, 4.8, 0.9 Hz, 1H), 7.29 (dd, *J* = 8.7, 7.6 Hz, 1H), 7.03 (d, *J* = 8.7 Hz, 1H), 6.32 (d, J = 7.4 Hz, 1H), 6.22 (d, *J* = 9.1 Hz, 1H), 4.87 (d, *J* = 49.5 Hz, 1H), 4.42 (d, *J* = 5.5 Hz, 2H), 3.93 - 3.82 (m, 1H), 3.76 (q, *J* = 11.1 Hz, 2H), 3.05 (t, *J* = 11.5 Hz, 1H), 2.78 (d, *J* = 11.5 Hz, 1H), 2.38 - 2.21 (m, 1H), 2.20 (s, 3H), 2.14 (t, J = 10.3 Hz, 1H), 1.98 - 1.80 (m, 2H).

**Example 126**

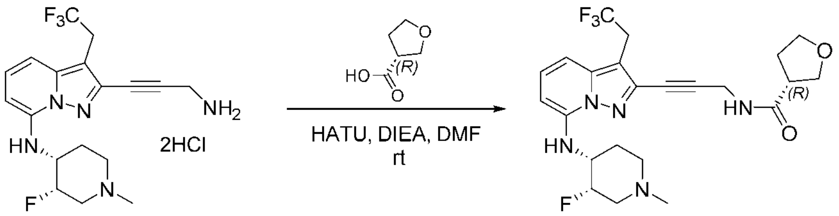


001-12 139-A

**Step 1: Synthesis of (3*R*)-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]tetrahydrofuran-3-carboxamide (compound 139-A)**

At room temperature, a solution of (3*R*)-tetrahydrofuran-3-carboxylic acid (6.11 mg, 0.053 mmol, 1.2 eq), *N,N*-diisopropylethylamine (28.32 mg, 0.220 mmol, 5 eq) and O-(7-azabenzotriazol-1-yl)-*N,N,N',N'*-tetramethyluronium hexafluorophosphate (20.0 mg, 0.053 mmol, 1.2 eq) in *N,N*-dimethylformamide (1 mL) was stirred to react for 30 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12**, 20 mg, 0.044 mmol, 1 eq) was added and the system was further stirred to react for 2 h. After the reaction was completed, the reaction mixture was quenched with water at room temperature, and extracted with ethyl acetate (3 × 30 mL). The organic phases were combined, backwashed with saturated sodium chloride solution (3 × 30 mL), dried over anhydrous sodium sulfate, filtered, and the filtrate was concentrated under reduced pressure. The crude product was purified by HPLC with the following conditions (column specifications: Sunfire C18 5 μm, 30 mm × 150 mm; mobile phase A: water (0.1% ammonium bicarbonate), mobile phase B: acetonitrile; flow rate: 60 mL/min; elution gradient: 31% to 46% in 10 min; detection wavelength: UV 254 nm/220 nm; retention time (min): 9.6), to give compound **(3*R*)-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]tetrahydrofuran-3-carboxamide 139-A** (11.7 mg, 55.16%).

LCMS: (ESI, m/z): 482.10 $[M+H]^+$.

$^1$H NMR (400 MHz, DMSO-$d_6$, *ppm*) δ 8.57 (t, *J* = 5.5 Hz, 1H), 7.29 (dd, *J* = 8.8, 7.6 Hz, 1H), 7.03 (d, *J* = 8.7 Hz, 1H), 6.33 (d, *J* = 7.6 Hz, 1H), 6.22 (d, *J* = 9.1 Hz, 1H), 4.88 (d, *J* = 49.4 Hz, 1H), 4.20 (d, *J* = 5.4 Hz, 2H), 3.93 - 3.59 (m, 7H), 3.06 (t, *J* = 11.6 Hz, 1H), 2.96 (q, *J* = 7.7 Hz, 1H), 2.78 (d, *J* = 11.2 Hz, 1H), 2.34 (dd, J = 39.6, 14.0 Hz, 1H), 2.21 (s, 3H), 2.14 (td, *J* = 11.5, 3.0 Hz, 1H), 2.06 - 1.95 (m, 2H), 1.96 - 1.80 (m, 2H).

**Example 127**

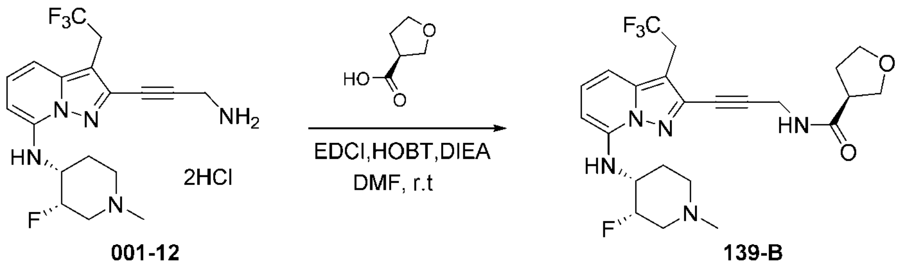


001-12 139-B

**Step 1: Synthesis of (3*S*)-*N*-[3-(7-1[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]tetrahydrofuran-3-carboxamide (compound 139-B)**

Under nitrogen protection, (3*S*)-tetrahydrofuran-3-carboxylic acid (7.63 mg, 0.066 mmol, 1.5 eq), 1-ethyl-(3-dimethylaminopropyl)carbodiimide hydrochloride (12.6 mg, 0.066 mmol, 1.5 eq), 1-hydroxy-benzotriazole (8.88 mg, 0.066 mmol, 1.5 eq), and *N, N*-diisopropylethylamine (28.32 mg, 0.220 mmol, 5 eq) were added to a reaction flask, dissolved in *N, N*-dimethylformamide (2 mL) and stirred at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (**001-12**, 20 mg, 0.044 mmol, 1 eq) was added, and the system was further stirred to react for 1 h. After the reaction was completed, water (10 mL) was added for quenching, and the reaction mixture was extracted with ethyl acetate (20 mL × 2). The organic phases were combined, backwashed with saturated sodium chloride solution (10 mL × 3), dried over anhydrous sodium sulfate, and filtered, and the filtrate was concentrated under reduced pressure. The crude product was

purified by HPLC with the following conditions (column specifications: YMC Triart C18 ExRs 5 μm, 30 mm × 150 mm; mobile phase A: water (10 mmol/L ammonium bicarbonate), mobile phase B: acetonitrile; flow rate: 60 mL/min; elution gradient: 29% B to 55% B in 8 min; detection wavelength: UV 254 nm/220 nm; retention time (min): 7.42) to give compound **(3*S*)-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]tetrahydrofuran-3-carboxamide 139-B** (9.25 mg, 43.61%).

LCMS: (ESI, m/z): 482.50 $[M+H]^+$.

$^1H$ NMR (400 MHz, DMSO-$d_6$, *ppm*) δ 8.54 (t, *J* = 5.5 Hz, 1H), 7.31 - 7.26 (m, 1H), 7.02 (d, *J* = 8.7 Hz, 1H), 6.32 (d, *J* = 7.6 Hz, 1H), 6.20 (d, *J* = 9.1 Hz, 1H), 4.88 (d, *J* = 49.4 Hz, 1H), 4.20 (d, *J* = 5.4 Hz, 2H), 3.96 - 3.52 (m, 7H), 3.06 (t, *J* = 11.5 Hz, 1H), 2.96 (q, *J* = 7.6 Hz, 1H), 2.78 (d, *J* = 11.5 Hz, 1H), 2.37 - 2.22 (m, 1H), 2.21 (s, 3H), 2.16 - 2.10 (m, 1H), 2.04 - 1.95 (m, 2H), 1.95 - 1.85 (m, 2H).

### Example 128

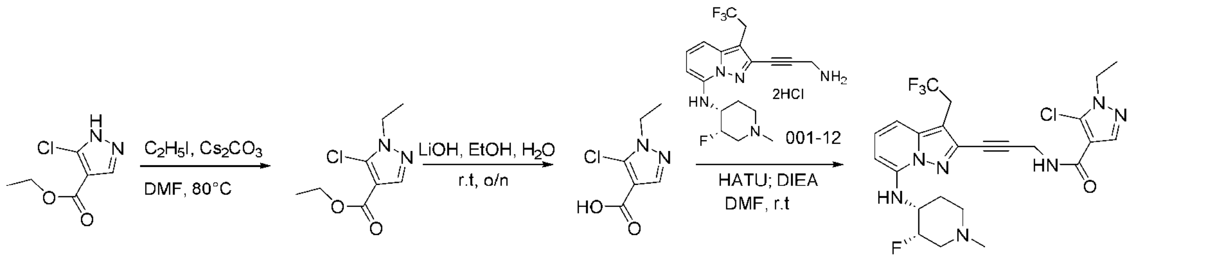


343-1 343-2A 343-3 343

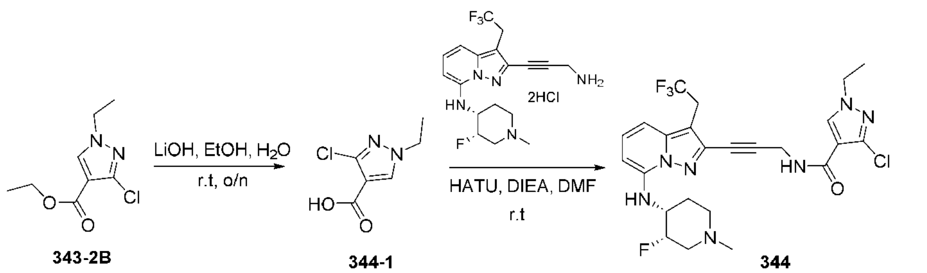


343-2B 344-1 344

### Step 1: Synthesis of compounds 343-2A and 343-2B

Under nitrogen protection, ethyl 5-chloro-1*H*-pyrazole-4-carboxylate (compound **343-1**, 500 mg, 2.864 mmol, 1 eq) and cesium carbonate (1866.3 mg, 5.728 mmol, 2 eq) were added to a reaction flask, and dissolved in N,N-dimethylformamide (5 mL). Then, iodoethane (893.4 mg, 5.728 mmol, 2 eq) was added and the mixture was warmed to 80°C and stirred to react for 1 h. After the reaction was completed, the reaction mixture was purified by reversed-phase column chromatography with the following conditions: C18 column, mobile phase: water (0.1% trifluoroacetic acid) and acetonitrile, 5% to 40% gradient in 15 min, detection wavelength: UV 254 nm. Compounds **343-2A** (100 mg, 16.25%) and **343-2B** (350 mg, 59.64%) were obtained.

**343- 2A:** LCMS:(ESI, m/z): 202.90 $[M+H]^+$.

**343-2B:** LCMS: (ESI, m/z): 202.95 $[M+H]^+$.

### Step 2: Synthesis of compound 343-3

Under nitrogen protection, ethyl 5-chloro-1-ethyl-1*H*-pyrazole-4-carboxylate (compound **343-2A**, 100 mg, 0.493 mmol, 1 eq) and lithium hydroxide (23.64 mg, 0.986 mmol, 2 eq) were added to a reaction flask, dissolved in ethanol (2 mL) and water (2 mL), and stirred to react at room temperature overnight. After the reaction was completed, the reaction mixture was concentrated under reduced pressure to give compound **343-3** (65 mg, 70.24%).

LCMS: (ESI, m/z): 174.95 $[M+H]^+$.

**Step 3: Synthesis of 5-chloro-1-ethyl-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1-(1-methylpiperidin-4-yl)-1*H*-pyrazole-4-carboxamide (compound 343)**

Under nitrogen protection, 5-chloro-1-ethyl-1*H*-pyrazole-4-carboxylic acid (compound **343-3**, 11.48 mg, 0.066 mmol, 1.50 eq), 2-(7-azabenzotriazole)-*N,N,N',N'*-tetramethyluronium hexafluorophosphate (16.67 mg, 0.044 mmol, 1 eq), and N, *N*-diisopropylethylamine (33.71 mg, 0.260 mmol, 5 eq) were added to a reaction flask, dissolved in *N,N*-dimethylformamide (2 mL), and stirred at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12**, 20 mg, 0.052 mmol, 1 eq) was added and the mixture was further stirred to react for 1 h. After the reaction was completed, water (10 mL) was added for quenching, the reaction mixture was extracted with ethyl acetate (20 mL × 2). The organic phases were combined, backwashed with saturated sodium chloride solution (10 mL × 3), dried over anhydrous sodium sulfate, filtered, and the filtrate was concentrated under reduced pressure. The crude product was purified by HPLC with the following conditions (column specifications: XBridge BEH C18 OBD Prep Column 130, 5 μm, 30 mm × 150 mm; mobile phase A: water (17 mmol/L ammonium bicarbonate), mobile phase B: acetonitrile; flow rate: 60 mL/min; elution gradient: 30% B to 60% B in 8 min; detection wavelength: UV 254 nm/220 nm; retention time (min): 7.07), to give compound **5-chloro-1-ethyl-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1-(1-methylpiperidin-4-yl)-1*H*-pyrazole-4-carboxamide 343(4.7** mg, 19.80%).

LCMS: (ESI, m/z): 539.85 $[M+H]^+$.

$^1$H NMR (400 MHz, DMSO-$d_6$, *ppm*) δ 8.71 (t, *J* = 5.6 Hz, 1H), 8.06 (s, 1H), 7.29 (dd, *J* = 8.8, 7.6 Hz, 1H), 7.03 (d, *J* = 8.7 Hz, 1H), 6.32 (d, *J* = 7.6 Hz, 1H), 6.24 (d, *J* = 9.1 Hz, 1H), 4.87 (d, *J* = 49.5 Hz, 1H), 4.33 (d, *J* = 5.6 Hz, 2H), 4.18 (q, *J* = 7.2 Hz, 2H), 3.93 - 3.81 (m, 1H), 3.76 (q, *J* = 11.1 Hz, 2H), 3.05 (t, *J* = 11.6 Hz, 1H), 2.78 (d, *J* = 11.6 Hz, 1H), 2.39 - 2.23 (m, 1H), 2.20 (s, 3H), 2.13 (t, *J* = 11.2 Hz, 1H), 1.98 - 1.83 (m, 2H), 1.33 (t, *J* = 7.2 Hz, 3H).

**Step 4: Synthesis of compound 344-1**

Under nitrogen protection, ethyl 3-chloro-1-ethyl-1*H*-pyrazole-4-carboxylate (compound **343-2B,** 350 mg, 1.727 mmol, 1 eq) and lithium hydroxide (82.73 mg, 3.454 mmol, 2 eq) were added to a reaction flask, dissolved in anhydrous ethanol (5 mL) and water (5 mL), and stirred to react at room temperature overnight. After the reaction was completed, the reaction mixture was concentrated under reduced pressure to give compound **344-1** (220 mg, 72.01%).

LCMS: (ESI, m/z): 174.95 $[M+H]^+$.

**Step 5: Synthesis of 3-chloro-1-ethyl-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1-(1-methylpiperidin-4-yl)-1*H*-pyrazole-4-carboxamide (compound 344)**

Under nitrogen protection, 3-chloro-1-ethyl-1*H*-pyrazole-4-carboxylic acid (compound **344-1,** 11.48 mg, 0.066 mmol, 1.50 eq), 2-(7-azabenzotriazole)-*N,N,N',N'*-tetramethyluronium hexafluorophosphate (16.67 mg, 0.044 mmol, 1 eq), and *N,N*-diisopropylethylamine (33.71 mg, 0.260 mmol, 5 eq) were added to a reaction flask, dissolved in -*N,N*-dimethylformamide (2 mL), and stirred at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 20 mg, 0.052 mmol, 1 eq) was added and the mixture was further stirred to react for 1 h. After the reaction was completed, water (10 mL) was added for quenching, the reaction mixture was extracted with ethyl acetate (20 mL × 2). The organic phases were combined, backwashed with saturated sodium chloride solution (10 mL × 3), dried over anhydrous sodium sulfate, filtered, and the filtrate was concentrated under reduced pressure. The crude product was purified by HPLC with the following conditions (column specifications: Sunfire C18 5 μm, 30 mm × 150 mm; mobile phase A: water (0.1% formic acid), mobile phase B: acetonitrile; flow rate: 60 mL/min; elution gradient: 11% B to 36% B in 10 min; detection wavelength: UV 254 nm/220 nm; retention time (min): 8.15), to give compound **3-chloro-1-ethyl-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1-(1-methylpiperidin-4-yl)-1*H*-pyrazole-4-carboxamide 344** (12.16 mg, 51.07%).

LCMS: (ESI, m/z): 540.05 $[M+H]^+$.

$^1$H NMR (400 MHz, DMSO-$d_6$, *ppm*) δ 8.53 (t, *J* = 5.6 Hz, 1H), 8.31 (s, 1H), 7.29 (dd, *J* = 8.7, 7.6 Hz, 1H), 7.03 (d, *J* = 8.7 Hz, 1H), 6.32 (d, J = 7.6 Hz, 1H), 6.26 (d, J = 8.9 Hz, 1H), 4.89 (d, *J* = 49.4 Hz, 1H), 4.32 (d, *J* = 5.5 Hz, 2H), 4.13 (q, *J* = 7.3 Hz, 2H), 3.94 - 3.81 (m, 1H), 3.76 (q, *J* = 11.2 Hz, 2H), 3.10 (t, *J* = 11.6 Hz, 1H), 2.82 (d, *J* = 11.3 Hz, 1H), 2.42 - 2.27 (m, 1H), 2.23 (s, 3H), 2.18 (t, *J* = 12.0 Hz, 1H), 1.97 - 1.85 (m, 2H), 1.37 (t, *J* = 7.3 Hz, 3H).

**Example 129**

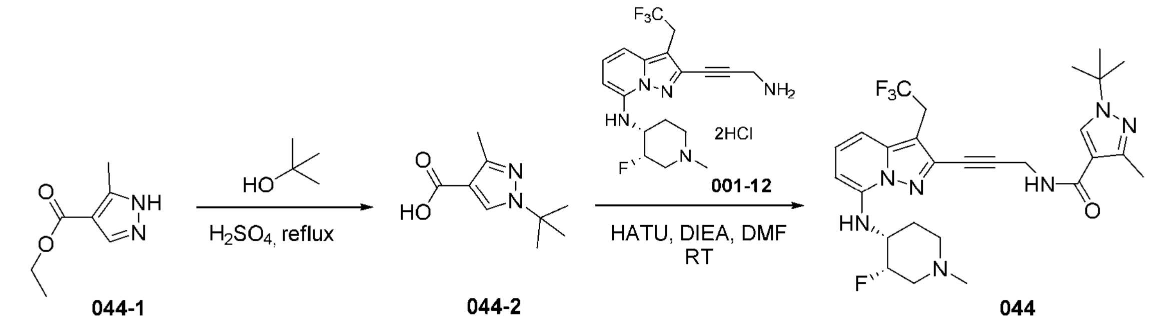


044-1 044-2 044

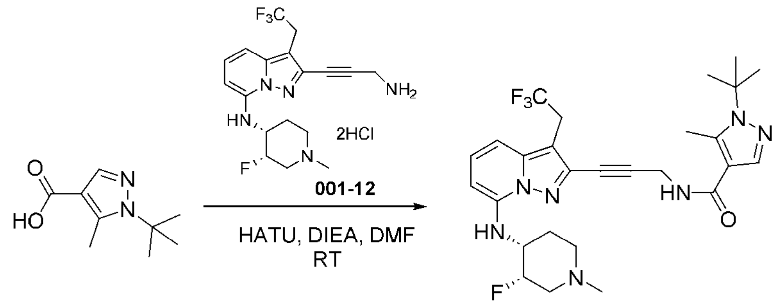


045-1 045

**Step 1: Synthesis of compounds 044-2 and 045-1**

To a reaction flask, ethyl 3-methyl-1*H*-pyrazole-4-carboxylate (compound **044-1**, 200 mg, 1.297 mmol, 1 eq) was added and dissolved in tert-butanol (1 mL, 0.013 mmol, 0.01 eq). Then, sulfuric acid (0.2 mL, 3.752 mmol, 2.89 eq) was added and the mixture was warmed to 90°C to react for 3 h. After the reaction was completed, the mixture was diluted with ethyl acetate, then water was added. The reaction mixture was extracted with ethyl acetate (3 × 10 mL), the organic phases were combined, backwashed with saturated sodium chloride solution (1 × 10 mL), dried over anhydrous sodium sulfate, and filtered. The filtrate was concentrated under reduced pressure to directly give compound **044-2** (250 mg, 95.18%) and compound **045-1** (60 mg).

LCMS: (ESI, m/z): 183.05 $[M+H]^+$.

**Step 2: Synthesis of 1-tert-butyl-*N*-[3-(7-{[(3*S*,4*R*)-3-fluoro-1-methylpiperidin-4-yl]amino}-3-(2,2,2-trifluoroethylpyrazolo[1,5-*a*]pyridin-2-yl)prop-2-yn-1-yl]-3-methyl-1*H*-pyrazole-4-carboxamide (compound 044)**

1-tert-butyl-3-methyl-1*H*-pyrazole-4-carboxylic acid (compound **044-2**, 60 mg, 0.329 mmol, 1 eq), 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12**, 126.24 mg, 0.329 mmol, 1 eq), O-(7-azabenzotriazol-1-yl)-*N,N,N',N'*-tetramethyluronium hexafluorophosphate (187.7 mg, 0.494 mmol, 1.5 eq) and N,N-diisopropylethylamine (212.8 mg, 1.645 mmol, 5 eq) were added to a reaction flask in sequence, dissolved in *N,N*-dimethylformamide (5 mL) and allowed to react at room temperature for 1 h. After the reaction was completed, water was added for quenching, and the reaction mixture was extracted with ethyl acetate (3 × 10 mL). The organic phases were combined, backwashed with saturated sodium chloride solution (1 × 10 mL), dried over anhydrous sodium sulfate, filtered, and the filtrate was concentrated under reduced pressure. The crude product was purified by HPLC with the following conditions (column specifications: YMC Triart C18 ExRs 5 μm, 30 mm × 150 mm; mobile phase A: water (10 mmol/L ammonium bicarbonate), mobile phase B: acetonitrile; flow rate: 60 mL/min; elution gradient: 39% B to 62% B in 10 min; detection wavelength: UV 254 nm/220 nm; retention time (min): 8.87), to give compound **1-tert-butyl-*N*-[3-(7-{[(3*S*,4*R*)-3-fluoro-1-methylpiperidin-4-yl]amino}-3-(2,2,2-trifluoroethylpyrazolo[1,5-*a*]pyridin-2-yl)prop-2-yn-1-yl]-3-methyl-1*H*-pyrazole-4-carboxamide 044** (8.79 mg, 4.86%).

LCMS: (ESI, m/z): 547.90 $[M+H]^+$.

$^1$H NMR (400 MHz, DMSO-$d_6$, ppm) $\delta$ 8.37 (t, *J* = 5.5 Hz, 1H), 8.31 (s, 1H), 7.28 (dd, *J* = 8.7, 7.6 Hz, 1H), 7.02 (d, *J* = 8.7 Hz, 1H), 6.32 (d, *J* = 7.6 Hz, 1H), 6.21 (d, *J* = 9.0 Hz, 1H), 4.87 (d, *J* = 49.5 Hz, 1H), 4.31 (d, *J* = 5.5 Hz, 2H), 3.94 - 3.83 (m, 1H), 3.75 (q, *J* = 11.2 Hz, 2H), 3.05 (t, *J* = 11.7 Hz, 1H), 2.78 (d, *J* = 11.4 Hz, 1H), 2.35 (s, 3H), 2.24 (d, J = 12.9 Hz, 1H),

2.20 (s, 3H), 2.18 - 2.09 (m, 1H), 1.98 - 1.80 (m, 2H), 1.50 (s, 9H).

**Step 3: Synthesis of 1-tert-butyl-*N*-[3-(7-{[(3*S*,4*R*)-3-fluoro-1-methylpiperidin-4-yl]amino}-3-(2,2,2-trifluoroethylpyrazolo[1,5-*a*]pyridin-2-yl)prop-2-yn-1-yl]-5-methyl-1*H*-pyrazole-4-carboxamide (compound 045)**

1-tert-butyl-5-methyl-1*H*-pyrazole-4-carboxylic acid (compound **045-1**, 60 mg, 0.329 mmol, 1 eq), 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12**, 126.24 mg, 0.329 mmol, 1 eq), O-(7-azabenzotriazol-1-yl)-*N,N,N',N'*-tetramethyluronium hexafluorophosphate (187.72 mg, 0.494 mmol, 1.5 eq) and *N,N*-diisopropylethylamine (212.78 mg, 1.645 mmol, 5 eq) were added to a reaction flask in sequence, dissolved in *N,N*-dimethylformamide (5 mL) and allowed to react at room temperature for 1 h. Then, water was added for quenching, and the reaction mixture was extracted with ethyl acetate (3 × 10 mL). The organic phases were combined, backwashed with saturated sodium chloride solution (1 × 10 mL), dried over anhydrous sodium sulfate, filtered, and the filtrate was concentrated under reduced pressure. The crude product was purified by HPLC with the following conditions (column specifications: YMC Triart C18 ExRs 5 μm, 30 mm * 150 mm; mobile phase A: water (10 mmol/L ammonium bicarbonate), mobile phase B: acetonitrile; flow rate: 60 mL/min; elution gradient: 39% B to 62% B in 10 min; detection wavelength: UV 254 nm/220 nm; retention time (min): 8.87), to give compound 1-tert-**butyl-*N*-[3-(7-{[(3*S*,4*R*)-3-fluoro-1-methylpiperidin-4-yl]amino}-3-(2,2,2-trifluoroethylpyrazolo[1,5-*a*]pyridin-2-yl)prop-2-yn-1-yl]-5-methyl-1*H*-pyrazole-4-carboxamide 045** (0.56 mg, 0.30%).

LCMS: (ESI, m/z): 547.95 [M+H]$^+$.

$^1$H NMR (400 MHz, DMSO-$d_6$, *ppm*) $\delta$ 8.51 (t, *J* = 5.6 Hz, 1H), 7.81 (s, 1H), 7.32 - 7.25 (m, 1H), 7.02 (d, *J* = 8.7 Hz, 1H), 6.31 (d, *J* = 7.6 Hz, 1H), 6.22 (d, *J* = 9.1 Hz, 1H), 4.87 (d, *J* = 49.5 Hz, 1H), 4.30 (d, *J* = 5.6 Hz, 2H), 3.94 - 3.83 (m, 1H), 3.75 (q, *J* = 11.3 Hz, 2H), 3.05 (t, *J* = 11.7 Hz, 1H), 2.78 (d, *J* = 11.2 Hz, 1H), 2.71 (s, 3H), 2.34 - 2.22 (m, 1H), 2.20 (s, 3H), 2.18 - 2.09 (m, 1H), 1.99 - 1.79 (m, 2H), 1.58 (s, 9H).

Example 130

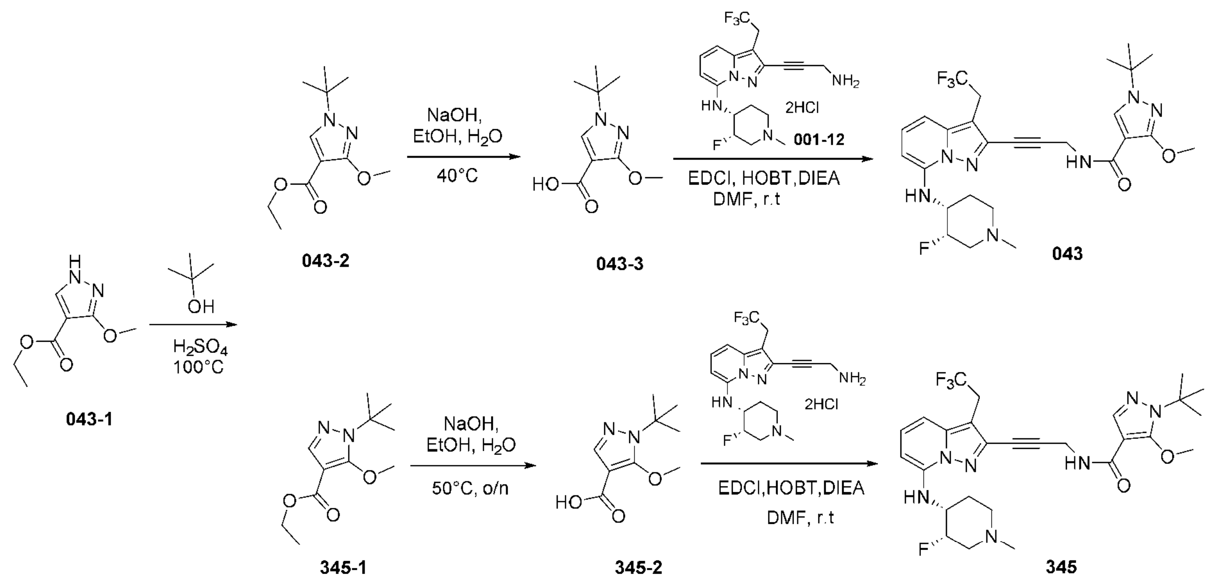


**Step 1: Synthesis of compound 043-2**

Under nitrogen protection, ethyl 3-methoxy-1*H*-pyrazole-4-carboxylate (compound **043-1**, 1.5 g, 8.815 mmol, 1 eq) was added to a reaction flask and dissolved in tert-butanol (9 mL). The mixture was warmed to 30°C and stirred for 10 min. Then, concentrated sulfuric acid (0.5 mL, 8.991 mmol, 1.02 eq) was added, and the mixture was warmed to 100°C and stirred to react for 2 h. After the reaction was completed, water (15 mL) was added for quenching, and the reaction mixture was extracted with ethyl acetate (30 mL × 2). The organic phases were combined, washed with water (20 mL × 2), and then backwashed with saturated sodium chloride solution (50 mL × 1), dried over anhydrous sodium sulfate and filtered, and the filtrate was concentrated under reduced pressure. The resulting residue was purified by silica gel column chromatography (petroleum ether/ethyl acetate = 10:1) to give compound **043-2** (200 mg, 10.03%) and compound **345-1**

(700 mg, 35.10%).

LCMS: (ESI, m/z): 227.05 [M+H]+.

**Step 2: Synthesis of compound 043-3**

Under nitrogen protection, ethyl 1-(tert-butyl)-3-methoxy-1*H*-pyrazole-4-carboxylate (compound **043-2**, 100 mg, 0.442 mmol, 1 eq), and sodium hydroxide (176.76 mg, 4.420 mmol, 10 eq) were added to a reaction flask, dissolved in anhydrous ethanol (3 mL) and water (0.9 mL), warmed to 40°C and stirred to react for 3 h. After the reaction was completed, the reaction mixture was acidified to pH=3 with 1 mol/L hydrochloric acid aqueous solution. The reaction mixture was then concentrated under reduced pressure. To the resulting residue was added 10 mL of tetrahydrofuran, the mixture was stirred for 10 min, filtered, and the filtrate was concentrated under reduced pressure to give compound **043-3** (90 mg, 93.49%).

LCMS: (ESI, m/z): 198.85 [M+H]+.

**Step 3: Synthesis of 1-(tert-butyl)-*N*-[3-(7-{[(3*S*,4*R*)-3-fluoro-1-methylpiperidin-4-yl]amino}-3-(2,2,2-trifluoroethylpyrazolo[1,5-*a*]pyridin-2-yl)prop-2-yn-1-yl]-3-methoxy-1*H*-pyrazole-4-carboxamide (compound 043)**

Under nitrogen protection, 1-(tert-butyl)-3-methoxy-1*H*-pyrazole-4-carboxylic acid (compound **043-3**, 62.04 mg, 0.314 mmol, 1.50 eq), 1-ethyl-(3-dimethylaminopropyl)carbodiimide hydrochloride (60.00 mg, 0.314 mmol, 1.5 eq), 1-hydroxy-benzotriazole (42.29 mg, 0.314 mmol, 1.5 eq), and *N, N*-diisopropylethylamine (134.84 mg, 1.045 mmol, 5 eq) were added to a reaction flask, dissolved in *N,N*-dimethylformamide (2.5 mL)) and stirred to react at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo [1,5-*a*]pyridin-7-amine dihydrochloride (compound **001-12**, 80 mg, 0.209 mmol, 1.00 eq) was added, and the system was further stirred to react for 1 h. After the reaction was completed, water (10 mL) was added for quenching, and the reaction mixture was extracted with ethyl acetate (20 mL × 2). The organic phases were combined, backwashed with saturated sodium chloride solution (10 mL × 5), dried over anhydrous sodium sulfate, and filtered, and the filtrate was concentrated under reduced pressure. The crude product was purified by HPLC with the following conditions (column specifications: Kinetex 5 μm EVO C18, 30mm × 150 mm; mobile phase A: water (10 mmol/L ammonium bicarbonate), mobile phase B: acetonitrile; flow rate: 60 mL/min; elution gradient: 40% B to 58% B in 10 min; detection wavelength: UV 254 nm/220 nm; retention time (min): 8.63) to give compound **1-(tert-butyl)-*N*-[3-(7-{ [(3*S*,4*R*)-3-fluoro-1-methylpiperidin-4-yl]amino}-3-(2,2,2-trifluoroethylpyrazolo[1,5-*a*]pyridin-2-yl)prop-2-yn-1-yl)-3-methoxy-1*H*-pyrazole-4-carboxamide 043** (22.06 mg, 18.57%).

LCMS: (ESI, m/z): 563.90 [M+H]+.

$^1$H NMR (400 MHz, DMSO-$d_6$, *ppm*) δ 8.04 (s, 1H), 7.61 (t, *J* = 5.7 Hz, 1H), 7.28 (dd, *J* = 8.8, 7.6 Hz, 1H), 7.02 (d, J = 8.6 Hz, 1H), 6.31 (d, J = 7.3 Hz, 1H), 6.23 (d, *J* = 9.0 Hz, 1H), 4.87 (d, *J* = 49.4 Hz, 1H), 4.32 (d, *J* = 5.7 Hz, 2H), 3.92 (s, 3H), 3.88 - 3.80 (m, 1H), 3.75 (q, *J* = 11.2 Hz, 2H), 3.05 (t, *J* = 11.4 Hz, 1H), 2.78 (d, *J* = 11.1 Hz, 1H), 2.37 - 2.22 (m, 1H), 2.20 (s, 3H), 2.13 (t, J = 10.5 Hz, 1H), 2.00 - 1.80 (m, 2H), 1.48 (s, 9H).

**Step 4: Synthesis of compound 345-2**

Under nitrogen protection, ethyl 1-(tert-butyl)-5-methoxy-1*H*-pyrazole-4-carboxylate (compound **345-1**, 100 mg, 0.442 mmol, 1 eq), and sodium hydroxide (176.76 mg, 4.420 mmol, 10 eq) were added to a reaction flask, dissolved in anhydrous ethanol (3 mL) and water (0.9 mL), warmed to 50°C and stirred to react overnight. After the reaction was completed, the reaction mixture was concentrated under reduced pressure. To the resulting residue was added 10 mL of tetrahydrofuran, the mixture was stirred for 10 min, filtered, and the filtrate was concentrated under reduced pressure to give compound **345-2** (90 mg, 93.49%).

LCMS: (ESI, m/z): 198.85 [M+H]+.

**Step 5: Synthesis of 1-(tert-butyl)-*N*-[3-(7-{[(3*S*,4*R*)-3-fluoro-1-methylpiperidin-4-yl]amino}-3-(2,2,2-trifluoroethylpyrazolo[1,5-*a*]pyridin-2-yl)prop-2-yn-1-yl]-5-methoxy-1*H*-pyrazole-4-carboxamide (compound 345)**

Under nitrogen protection, 1-(tert-butyl)-5-methoxy-1*H*-pyrrole-4-carboxylic acid (compound **345-2**, 30 mg, 0.151 mmol, 1 eq), 1-ethyl-(3-dimethylaminopropyl)carbodiimide hydrochloride (43.52 mg, 0.226 mmol, 1.5 eq), 1-hydroxy-benzotriazole (30.68 mg, 0.226 mmol, 1.5 eq), and *N, N*-diisopropylethylamine (97.8 mg, 0.755 mmol, 5 eq) were added to a reaction flask, dissolved in *N,N*-dimethylformamide (2 mL)) and stirred to react at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12**, 58.03 mg, 0.151 mmol, 1 eq) was added, and the system was further stirred to react at room temperature for 1 h. After the reaction was completed, water (10 mL) was added for quenching, and

the reaction mixture was extracted with ethyl acetate (20 mL × 2). The organic phases were combined, backwashed with saturated sodium chloride solution (10 mL × 3), dried over anhydrous sodium sulfate, and filtered, and the filtrate was concentrated under reduced pressure. The crude product was purified by HPLC with the following conditions (column specifications: XBridge BEH C18 OBD Prep Column 130, 5 μm, 30 mm × 150 mm; mobile phase A: water (10 mmol/L ammonium bicarbonate), mobile phase B: acetonitrile; flow rate: 60 mL/min; elution gradient: 37% B to 57% B in 10 min; detection wavelength: UV 254 nm/220 nm; retention time (min): 8.83) to give compound **1-(tert-butyl)-*N*-[3-(7-{[(3*S*,4*R*)-3-fluoro-1-methylpiperidin-4-yl]amino}-3-(2,2,2-trifluoroethylpyrazolo[1,5-*a*]pyridin-2-yl) prop-2-yn-1-yl]-5-methoxy-1*H*-pyrazole-4-carboxamide 345** (4.48 mg, 5.22%).

LCMS: (ESI, m/z): 563.90 $[M+H]^+$.

$^1$H NMR (400 MHz, DMSO-$d_6$, *ppm*) $\delta$ 8.49 (t, *J* = 5.6 Hz, 1H), 7.75 (s, 1H), 7.28 (dd, *J* = 8.7, 7.6 Hz, 1H), 7.02 (d, *J* = 8.6 Hz, 1H), 6.32 (d, *J* = 7.5 Hz, 1H), 6.22 (d, *J* = 9.0 Hz, 1H), 4.87 (d, *J* = 49.5 Hz, 1H), 4.30 (d, *J* = 5.6 Hz, 2H), 4.04 (s, 3H), 3.92 - 3.80 (m, 1H), 3.75 (q, *J* = 11.1 Hz, 2H), 3.05 (t, *J* = 11.6 Hz, 1H), 2.78 (d, *J* = 11.5 Hz, 1H), 2.36 - 2.21 (m, 1H), 2.20 (s, 3H), 2.14 (t, J = 10.5 Hz, 1H), 1.96 - 1.83 (m, 2H), 1.52 (s, 9H).

**Example 131**

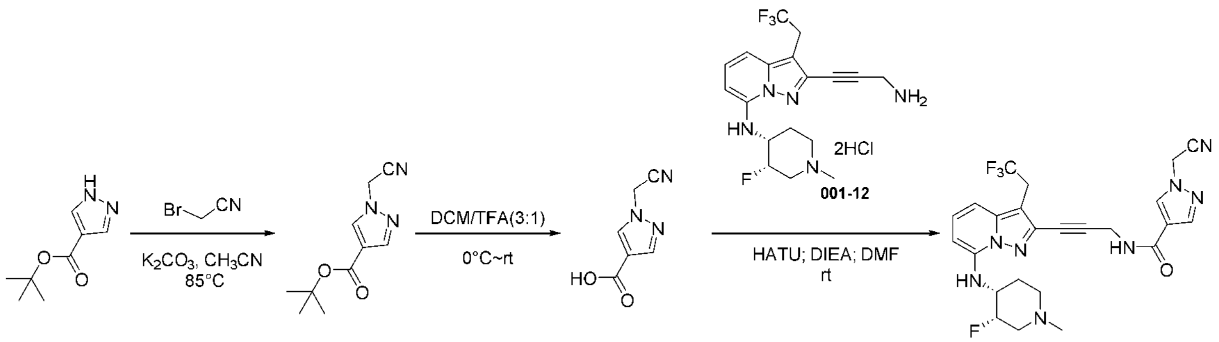


249-1 249-2 249-3 249

**Step 1: Synthesis of compound 249-2**

To a solution of tert-butyl 1*H*-pyrazole-4-carboxylate (compound **249-1**, 200 mg, 1.189 mmol, 1 eq) in acetonitrile (2 mL) were added bromoacetonitrile (171.16 mg, 1.427 mmol, 1.2 eq) and potassium carbonate (493.01 mg, 3.567 mmol, 3 eq) at room temperature. Thereafter, the system was warmed to 85°C and further stirred for 2 h. After the reaction was completed, the mixture was filtered, the filter cake was washed with acetonitrile (3 × 50 mL), the filtrate was concentrated under reduced pressure, the crude product was diluted with water (50 mL), the aqueous phase was extracted with ethyl acetate (3 × 50 mL), the organic phases were combined, dried over anhydrous sodium sulfate, filtered, and the filtrate was concentrated under reduced pressure to give crude compound **249-2** (265 mg, 107.54%), which was directly used in the next step without further purification.

LCMS: (ESI, m/z): 208.00 $[M+H]^+$.

**Step 2: Synthesis of compound 249-3**

At 0°C, to a solution of tert-butyl 1-(cyanomethyl)-1*H*-pyrazole-4-carboxylate (compound **249-2,** 10 mg, 0.048 mmol, 1 eq) in dichloromethane (0.5 mL) solution was added trifluoroacetic acid (0.15 mL), and the mixture was stirred to react for 2 h. After the reaction was completed, the resulting residue was concentrated under reduced pressure to give a crude product of compound **249-3** (10 mg, 137.13%). The crude product was not further purified and was directly used in the next step.

LCMS: (ESI, m/z): 152.00 $[M+H]^+$.

**Step 3: Synthesis of 1-cyanomethyl-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1*H*-pyrazole-4-carboxamide (compound 249)**

At room temperature, to a solution of 1-(cyanomethyl)-1*H*-pyrrole-4-carboxylic acid (compound **249-3,** 9.94 mg, 0.066 mmol, 1 eq) in *N*,*N*-dimethylformamide (1.5 mL) were added O-(7-azabenzotriazol-1-yl)-*N,N,N',N'*-tetramethyluronium hexafluorophosphate (25.0 mg, 0.066 mmol, 1 eq) and diisopropylethylamine (0.05 mL, 0.264 mmol, 4 eq), and the

mixture was stirred to react for 30 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 30 mg, 0.066 mmol, 1 eq) was added and the mixture was further stirred for 1 h. After the reaction was completed, the reaction mixture was diluted with water (20 mL) and extracted with ethyl acetate (3 × 20 mL). The organic phases were combined, backwashed with saturated brine (3 × 20 mL), dried over anhydrous sodium sulfate, filtered, and the filtrate was concentrated under reduced pressure. The resulting residue was purified by a preparative chromatography plate (dichloromethane/anhydrous methanol = 15:1), to give a crude compound. The crude product was purified by HPLC with the following conditions (column specifications: Xselect CSHTM Prep C18 5μm 30*150mm OBD; mobile phase A: water (0.1% formic acid), mobile phase B: acetonitrile; flow rate: 60 mL/min; elution gradient: 5% B to 30% B in 10 min; detection wavelength: UV 254 nm/220 nm; retention time (min): 8.43), to give compound **1-cyanomethyl-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1*H*-pyrazole-4-carboxamide 249** (3.68 mg, 10.76%).

LCMS: (ESI, m/z): 517.05 $[M+H]^+$.

$^1H$ NMR (400 MHz, DMSO) δ 8.85 (t, J = 5.4 Hz, 1H), 8.34 (s, 1H), 8.03 (s, 1H), 7.29 (t, J = 8.2 Hz, 1H), 7.03 (d, J = 8.7 Hz, 1H), 6.32 (d, J = 7.7 Hz, 1H), 6.24 (d, J = 9.0 Hz, 1H), 5.55 (s, 2H), 4.88 (d, J = 49.7 Hz, 1H), 4.34 (d, J = 5.4 Hz, 2H), 3.96 - 3.82 (m, 1H), 3.81 - 3.69 (m, 2H), 3.07 (t, J = 10.3 Hz, 1H), 2.79 (d, J = 11.4 Hz, 1H), 2.30 (dd, J = 38.4, 13.3 Hz, 1H), 2.20 (s, 3H), 2.14 (t, J = 10.9 Hz, 1H), 2.07 - 1.80 (m, 2H).

**Example 132**

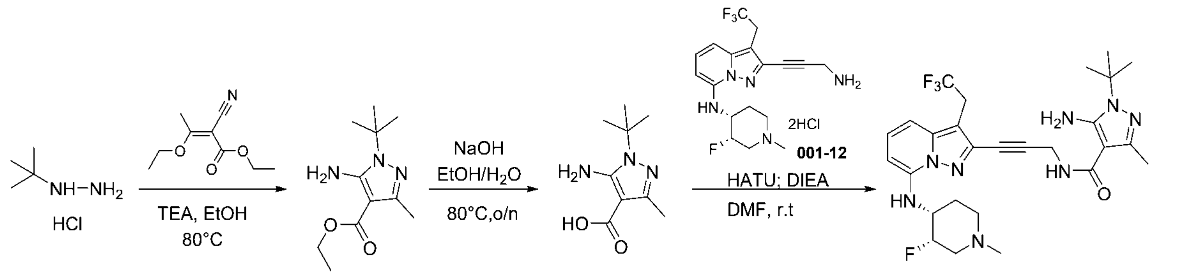


**288-1** **288-2** **288-3** **288**

**Step 1: Synthesis of compound 288-2**

To a solution of ethyl (2*Z*)-2-cyano-3-ethoxybut-2-enoate (3 g, 16.375 mmol, 1.00 eq) in ethanol (60.0 mL) were added tert-butylhydrazine hydrochloride (compound **288-1,** 2.45 g, 19.650 mmol, 1.2 eq) and triethylamine (3.31 g, 32.750 mmol, 2 eq) at room temperature, and the mixture was stirred at 80°C for 4 h. After the reaction was completed, the reaction mixture was concentrated under reduced pressure, and the resulting residue was washed with saturated brine (3×50 mL), extracted with ethyl acetate (3×50 mL), dried over anhydrous sodium sulfate, filtered, and the filtrate was concentrated under reduced pressure to give compound **288-2** (3.5 g, 94.87%).

LCMS: (ESI, m/z): 226.05 $[M+H]^+$.

**Step 2: Synthesis of compound 288-3**

At room temperature, ethyl 5-amino-1-tert-butyl-3-methyl-1*H*-pyrazole-4-carboxylate (compound **288-2,** 3.5 g, 15.535 mmol, 1 eq) was added to a reaction flask, dissolved in ethanol (90 mL), and then a solution of sodium hydroxide (1.24 g, 31.070 mmol, 2 eq) in water (28 mL) was added. The mixture was stirred at 80°C overnight, concentrated under reduced pressure, and the resulting residue was slurried with petroleum ether and filtered to give crude compound **288-3** (2.3 g, 75.06%).

LCMS: (ESI, m/z): 197.90 $[M+H]^+$.

**Step 3: Synthesis of 5-amino-tert-butyl-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-3-methyl-1*H*-pyrazole-4-carboxamide (compound 288)**

At room temperature, a solution of 5-amino-1-tert-butyl-3-methyl-1*H*-pyrazole-4-carboxylic acid (compound **288-3,** 43.22 mg, 0.220 mmol, 2 eq), *N*,*N*-diisopropylethylamine (56.65 mg, 0.440 mmol, 4 eq), and O-(7-azabenzotriazol-1-yl)-*N*,*N*,*N'*,*N'*-tetramethyluronium hexafluorophosphate (45.83 mg, 0.121 mmol, 1.1 eq) in *N*,*N*-dimethylformamide (2 mL) was stirred at room temperature for 10 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 50 mg, 0.110 mmol, 1.00 eq) was added and the mixture was further stirred for 1 h. After the reaction was completed, the reaction mixture was diluted with ethyl acetate (5 mL), washed with saturated sodium chloride solution (5 mL × 3), dried over anhydrous sodium sulfate, filtered, and the filtrate was concentrated under reduced pressure. The crude product was purified by HPLC with the following conditions (column specifications: Xselect CSHTM Prep C18 5μm 30*150mm OBD; mobile phase A: water (0.1% formic acid), mobile phase B: acetonitrile; flow rate: 60 mL/min; elution gradient: 14% B to 38% B in 8 min; detection wavelength: UV 254 nm/220 nm; retention time (min): 6.15), to give compound **5-amino-1-tert-butyl-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl) prop-2-yn-1-yl]-3-methyl-1*H*-pyrazole-4-carboxamide 288** (16.02 mg, 25.67%).

$^1$H NMR (400 MHz, DMSO-$d_6$, *ppm*) δ 7.29 (t, *J* = 8.2 Hz, 1H), 7.19 (t, *J* = 5.6 Hz, 1H), 7.03 (d, *J* = 8.7 Hz, 1H), 6.32 (d, *J* = 7.6 Hz, 1H), 6.24 (d, *J* = 9.1 Hz, 1H), 6.14 (s, 2H), 4.88 (d, *J* = 49.6 Hz, 1H), 4.28 (d, *J* = 5.7 Hz, 2H), 3.94 - 3.82 (m, 1H), 3.76 (q, J = 11.1 Hz, 2H), 3.06 (t, *J* = 11.5 Hz, 1H), 2.79 (d, *J* = 11.4 Hz, 1H), 2.37 - 2.30 (m, 1H), 2.25 (s, 3H), 2.21 (s, 3H), 2.14 (t, *J* = 11.3 Hz, 1H), 2.01 - 1.88 (m, 1H), 1.87 - 1.79 (s, 1H), 1.51 (s, 9H).

**Example 133**

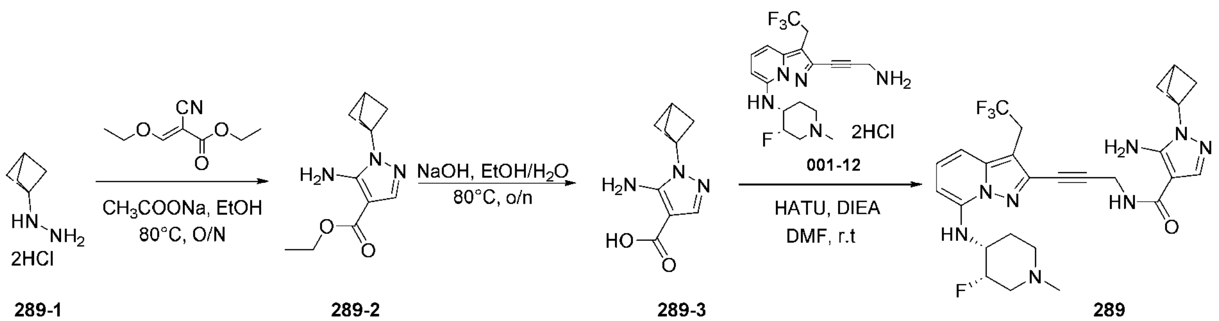


**289-1** **289-2** **289-3** **289**

**Step 1: Synthesis of compound 289-2**

Under nitrogen protection, at 80°C, a solution of {dicyclo[1.1.1]pent-1-yl}hydrazine dihydrochloride (compound **289-1,** 400 mg, 2.338 mmol, 1 eq), ethyl (E)-2-cyano-3-ethoxyacrylate (395.58 mg, 2.338 mmol, 1.00 eq) and sodium acetate (191.81 mg, 2.338 mmol, 1.00 eq) in ethanol (8.0 mL) was stirred to react overnight. After the reaction was completed, the resulting residue was concentrated under reduced pressure. The resulting residue was purified by silica gel column chromatography (petroleum ether/ethyl acetate = 5:1) to give compound **289-2** (100 mg).

LCMS: (ESI, m/z): 221.90 [M+H]$^+$.

**Step 2: Synthesis of compound 289-3**

At room temperature, a solution of ethyl 5-amino-1-(dicyclo[1.1.1]pent-1-yl)-1*H*-pyrazole-4-carboxylate (compound **289-2,** 100 mg, 0.452 mmol, 1 eq) and sodium hydroxide (0.180 mL, 0.904 mmol, 2.00 eq) in ethanol (2.0 mL) and water (2.0 mL) was stirred to react overnight. After the reaction was completed, the reaction mixture was acidified to a pH of about 5 with hydrochloric acid solution (2M). The resulting residue was concentrated under reduced pressure, the mixture was dissolved in tetrahydrofuran (4 mL), filtered, the filter cake was washed with tetrahydrofuran (2 × 5 mL), and the filtrate was concentrated under reduced pressure to give compound **289-3** (80 mg, 91.62%).

LCMS: (ESI, m/z): 194.05 [M+H]$^+$.

**Step 3: Synthesis of 5-amino-1-(dicyclo[1.1.1]pent-1-yl-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1*H*-pyrazole-4-carboxamide (compound 289)**

Under nitrogen protection, at room temperature, a solution of 5-amino-1-(dicyclo[1.1.1]pent-1-yl)-1*H*-pyrazole-4-carboxylic acid (compound **289-3,** 25 mg, 0.129 mmol, 1 eq), *N,N*-diisopropylethylamine (66.9 mg, 0.516 mmol, 4.00 eq) and O-(7-azabenzotriazol-1-yl)-*N,N,N',N'*-tetramethyluronium hexafluorophosphate (49.2 mg, 0.129 mmol, 1.00 eq) in *N,N*-dimethylformamide (2.5 mL) was stirred to react for 20 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (53.14 mg, 0.116

mmol, 0.90 eq) was added and the system was further stirred for 1 h. After the reaction was completed, the reaction mixture was quenched with water (10 mL), and extracted with ethyl acetate (2 × 10 mL). The organic phases were combined, backwashed with saturated sodium chloride solution (2 × 10 mL), dried over anhydrous sodium sulfate, filtered, and the filtrate was concentrated under reduced pressure. The resulting residue was purified by silica gel column chromatography (dichloromethane/methanol = 10:01) to give a crude compound. The crude product was purified by HPLC with the following conditions (column specifications: Sunfire C18 5 μm, 30 mm × 150 mm; mobile phase A: water (0.1% formic acid), mobile phase B: acetonitrile; flow rate: 60 mL/min; gradient: 17% B to 32% B in 7 min; wavelength: UV 254 nm/220 nm; retention time (min): 5.87), to give compound **5-amino-1-(dicyclo[1.1.1]pent-1-yl)-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1*H*-pyrazole-4-carboxamide 289** (14.14 mg, 17.97%).

LCMS: (ESI, m/z): 558.95 $[M+H]^+$.

$^1H$ NMR (400 MHz, DMSO-$d_6$, *ppm*) $\delta$ 8.35 (q, *J* = 6.0 Hz, 1H), 7.68 (s, 1H), 7.28 (dd, *J* = 8.8, 7.6 Hz, 1H), 7.02 (d, *J* = 8.7 Hz, 1H), 6.32 (d, *J* = 7.9 Hz, 1H), 6.23 (d, *J* = 9.1 Hz, 1H), 6.06 (s, 2H), 4.87 (d, *J* = 48 Hz, 1H), 4.29 (d, *J* = 5.6 Hz, 2H), 3.87 - 3.81 (m, 1H), 3.78 (q, *J* = 11.2 Hz, 2H), 3.05 (t, *J* = 11.4 Hz, 1H), 2.78 (d, *J* = 11.2 Hz, 1H), 2.57 (s, 1H), 2.36 - 2.22 (m, 7H), 2.20 (s, 3H), 2.18 - 2.08 (m, 1H), 1.94-1.81 (m, 2H).

**Example 134**

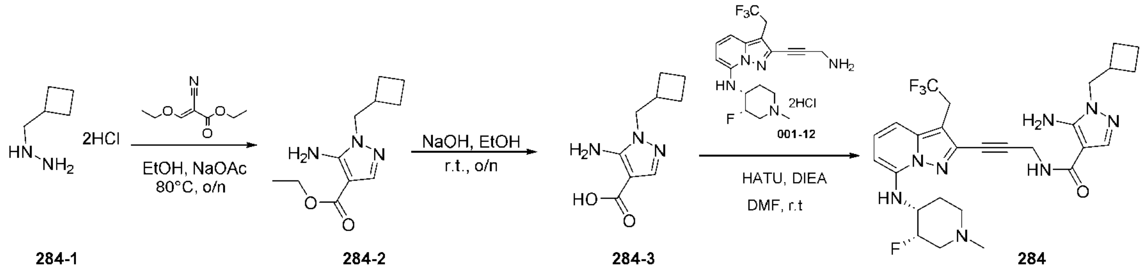


284-1 284-2 284-3 284

**Step 1: Synthesis of compound 284-2**

Under nitrogen protection, at 80°C, a solution of (cyclobutylmethyl)hydrazine dihydrochloride (compound **284-1,** 400 mg, 2.311 mmol, 1 eq), ethyl (*E*)-2-cyano-3-ethoxyacrylate (391 mg, 2.311 mmol, 1.00 eq) and sodium acetate (379.17 mg, 4.622 mmol, 2 eq) in ethanol (8.0 mL) was stirred to react overnight. After the reaction was completed, the resulting residue was concentrated under reduced pressure. The resulting residue was purified by silica gel column chromatography (petroleum ether/ethyl acetate = 5:01) to give compound **284-2** (500 mg, 96.90%).

LCMS: (ESI, m/z): 224.05 $[M+H]^+$.

**Step 2: Synthesis of compound 284-3**

At room temperature, a solution of ethyl 5-amino-1-(cyclobutylmethyl)-1*H*-pyrazole-4-carboxylate (compound **284-2,** 200 mg, 0.896 mmol, 1 eq) and sodium hydroxide (71.65 mg, 1.792 mmol, 2 eq) in ethanol (2 mL) was stirred to react overnight. After the reaction was completed, the resulting residue was concentrated under reduced pressure, the reaction mixture was diluted with tetrahydrofuran (2 mL), filtered, the filter cake was washed with tetrahydrofuran (2 × 5 mL), and the filtrate was concentrated under reduced pressure to give compound **284-3** (120 mg, 68.62%).

LCMS: (ESI, m/z): 196.35 $[M+H]^+$.

**Step 3: Synthesis of 5-amino-1-(cyclobutylmethyl)-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1*H*-pyrazole-4-carboxamide (compound 284)**

Under nitrogen protection, at room temperature, to a solution of 5-amino-1-(cyclobutylmethyl)-1*H*-pyrazole-4-carboxylic acid (compound **284-3,** 25 mg, 0.109 mmol, 1 eq, 85%) in *N*,*N*-dimethylformamide (2.0 mL) were added *N*,*N*-diisopropylethylamine (56.27 mg, 0.435 mmol, 4.00 eq) and O-(7-azabenzotriazol-1-yl)-*N,N,N',N'*-tetramethyluronium hexafluorophosphate (41.39 mg, 0.109 mmol, 1.00 eq), and the mixture was stirred to react for 20 min. Then, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*pyridin-7-amine dihydrochloride (compound **001-12,** 49.67 mg, 0.109 mmol, 1.00 eq) was added and the system was further stirred for 1 h.

The reaction mixture was quenched with water (10 mL), and extracted with ethyl acetate (2 × 10 mL). The organic phases were combined, backwashed with saturated sodium chloride solution (2 × 10 mL), dried over anhydrous sodium sulfate, filtered, and the filtrate was concentrated under reduced pressure. The resulting residue was purified by silica gel column chromatography (dichloromethane/methanol = 10:01) to give a pale yellow solid. The crude product was purified by HPLC with the following conditions (column specifications: Xselect CSHTM Prep C18 5μm 30×150mm OBD; mobile phase A: water (0.1% formic acid), mobile phase B: acetonitrile; flow rate: 60 mL/min; gradient: 14% B to 30% B in 10 min; wavelength: UV 254 nm/220 nm; retention time (min): 8.58), to give **compound 5-amino-1-(cyclobutylmethyl)-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1*H*-pyrazole-4-carboxamide 284** (19.18 mg, 28.73%).

LCMS: (ESI, m/z): 561.40 $[M+H]^+$.

$^1$H NMR (400 MHz, DMSO-$d_6$, *ppm*) δ 8.25 (t, *J* = 5.6 Hz, 1H), 7.66 (s, 1H), 7.28 (t, *J* = 8.2 Hz, 1H), 7.02 (d, *J* = 8.7 Hz, 1H), 6.31 (d, *J* = 7.6 Hz, 1H), 6.26 - 6.17 (m, 3H), 4.87 (d, *J* = 48 Hz, 1H), 4.28 (d, *J* = 5.6 Hz, 2H), 3.95 - 3.81 (m, 3H), 3.75 (q, *J* = 11.1 Hz, 2H), 3.05 (t, *J* = 11.7 Hz, 1H), 2.78 (d, *J* = 11.6 Hz, 1H), 2.75 - 2.67 (m, 1H), 2.37 - 2.21 (m, 1H), 2.20 (s, 3H), 2.13 (t, *J*= 11.3 Hz, 1H), 1.99 - 1.67 (m, 8H).

**Example 135**

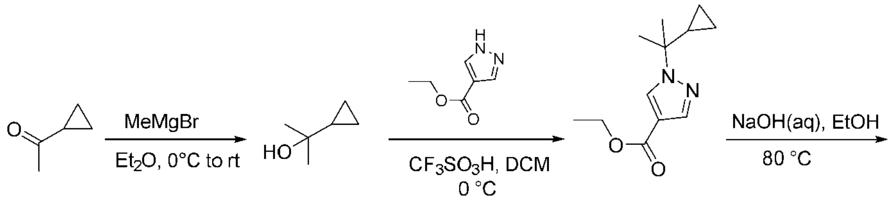


**346-1** **346-2** **346-3**

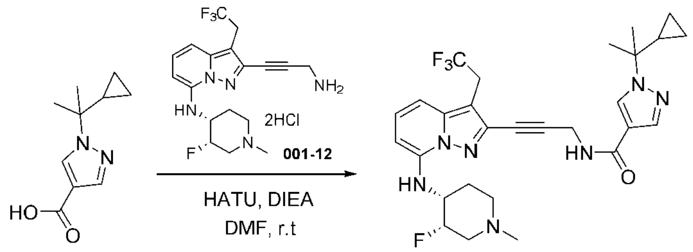


**346-4** **346**

**Step 1: Synthesis of compound 346-2**

Under nitrogen protection, cyclopropylmethyl ketone (compound **346-1,** 1 g, 11.888 mmol, 1 eq) was added to a reaction flask and dissolved in diethyl ether (10 mL). Then, methyl magnesium bromide(5.6 mL, 2.8M, 15.454 mmol, 1.3 eq) was added dropwise at 0°C. The reaction was carried out at 0°C for 2 h, and then the mixture was warmed to room temperature to react overnight. After the reaction was completed, saturated ammonium chloride solution (10 mL) was added to quench the reaction. The reaction mixture was extracted with diethyl ether (3 × 10 mL), the organic phases were combined, backwashed with saturated sodium chloride solution (1 × 10 mL), dried over anhydrous sodium sulfate, and filtered. The filtrate was concentrated under reduced pressure to directly give crude product compound **346-2** (1.86 g, 93.72%).

$^1$H NMR (400 MHz, Chloroform-*d*, *ppm*) δ 1.19 (s, 6H), 0.96 (tt, *J* = 8.4, 5.5 Hz, 1H), 0.41 - 0.29 (m, 4H).

**Step 2: Synthesis of compound 346-3**

2-cyclopropylprop-2-ol (compound **346-2,** 1.00 g, 9.984 mmol, 0.50 eq), and ethyl 4-pyrazole carboxylate (2.8 g, 19.980 mmol, 2 eq) were added to a reaction flask, dissolved in dichloromethane (70 mL), and then trifluoromethanesulfonic acid (1.50 g, 9.990 mmol, 1 eq) was slowly added at 0°C. The reaction was carried out at 0°C for 60 min. Saturated sodium bicarbonate was added to quench the reaction. The reaction mixture was extracted with ethyl acetate (3 × 50 mL). The organic phases were combined, backwashed with saturated sodium chloride solution (1 × 50 mL), dried over anhydrous sodium sulfate, and filtered. The filtrate was concentrated under reduced pressure. The resulting residue was

purified by silica gel column chromatography (petroleum ether/ethyl acetate = 10:01) to give compound **346-3** (800 mg, 34.22%).

LCMS: (ESI, m/z): 222.90 [M+H]$^+$.

### Step 3: Synthesis of compound 346-4

To a reaction flask, ethyl 1-(2-cyclopropylprop-2-yl)-1*H*-pyrazole-4-carboxylate (compound **346-3,** 110 mg, 0.495 mmol, 1 eq) was added and dissolved in ethanol (2 mL). Then, sodium hydroxide aqueous solution (1.0 mL, 1.0M, 1.0 mmL, 2 eq) was added and the mixture was allowed to react at 80°C for 1 h. After the reaction was completed, the reaction mixture was acidified to pH=3 with hydrochloric acid solution and the solvent was directly removed by spin drying to give compound **346-4** (90 mg, 88.95%).

LCMS: (ESI, m/z): 195.05 [M+H]$^+$.

### Step 4: Synthesis of 1-(2-cyclopropylprop-2-yl)-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1*H*-pyrazole-4-carboxamide (compound 346)

To a reaction flask, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 40 mg, 0.104 mmol, 1 eq), 1-(2-cyclopropylprop-2-yl)-1*H*-pyrazole-4-carboxylic acid (compound **346-4,** 20.26 mg, 0.104 mmol, 1 eq), O-(7-azabenzotriazol-1-yl)-*N,N,N',N'*-tetramethyluronium hexafluorophosphate (47.6 mg, 0.125 mmol, 1.2 eq), and *N*,*N*-diisopropylethylamine (67.42 mg, 0.520 mmol, 5 eq) were added, dissolved in *N*,*N*-dimethylformamide (4 mL) and allowed to react at room temperature for 1 h. After the reaction was completed, water was added for quenching and the reaction mixture was extracted with ethyl acetate (3 × 10 mL). The organic phases were combined, backwashed with saturated sodium chloride solution (1 × 10 mL), dried over anhydrous sodium sulfate, filtered, and the filtrate was concentrated under reduced pressure. The crude product was purified by HPLC with the following conditions (column specifications: Xselect CSHTM Prep C18 5μm 30 × 150mm OBD; mobile phase A: water (0.1% formic acid), mobile phase B: acetonitrile; flow rate: 60 mL/min; elution gradient: 15% B to 33% B in 8 min; detection wavelength: UV 254 nm/220 nm; retention time (min): 7.78), to give compound **1-(2-cyclopropylprop-2-yl)-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1*H*-pyrazole-4-carboxamide 346** (37.66 mg, 64.18%).

LCMS: (ESI, m/z): 560.65 [M+H]$^+$.

$^1$H NMR (400 MHz, DMSO-$d_6$, *ppm*) $\delta$ 8.65 (t, *J* = 5.6 Hz, 1H), 8.39 (s, 1H), 7.89 (s, 1H), 7.32 - 7.25 (m, 1H), 7.03 (d, *J* = 8.7 Hz, 1H), 6.32 (d, *J* = 7.6 Hz, 1H), 6.23 (d, *J* = 9.0 Hz, 1H), 4.87 (d, *J* = 49.4 Hz, 1H), 4.34 (d, *J* = 5.6 Hz, 2H), 3.92 - 3.81 (m, 1H), 3.75 (q, J = 11.1 Hz, 2H), 3.05 (t, *J* = 11.1 Hz, 1H), 2.78 (d, *J* = 11.4 Hz, 1H), 2.36 - 2.21 (m, 1H), 2.20 (s, 3H), 2.13 (t, J = 10.5 Hz, 1H), 2.00 - 1.77 (m, 2H), 1.42 (s, 6H), 1.36 - 1.24 (m, 1H), 0.51 - 0.45 (m, 2H), 0.39 (dd, *J* = 6.0, 4.4 Hz, 2H).

### Example 136

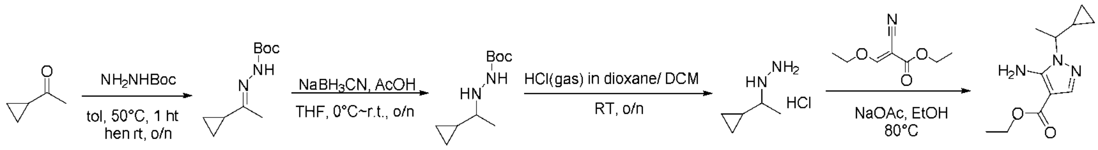


346-1 291-1 291-2 291-3 291-4

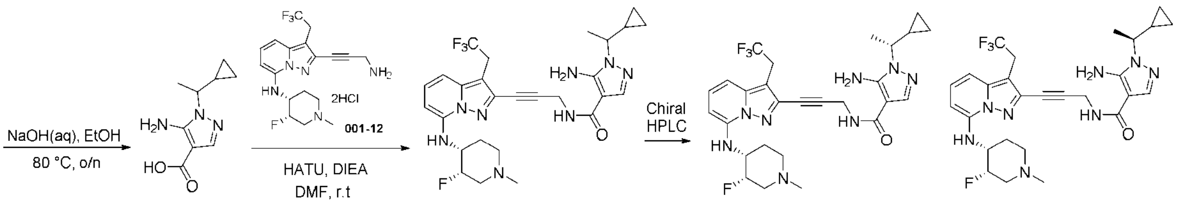


291-5 291

**Step 1: Synthesis of compound 291-1**

Tert-butyl hydrazine carboxylate (5 g, 37.832 mmol, 1 eq) and cyclopropylmethyl ketone (compound **346-1,** 3.50 g, 41.615 mmol, 1.1 eq) were added to a reaction flask, dissolved in toluene (75 mL), allowed to react at 50°C for 1 h, then cooled to room temperature and further stirred overnight. After the reaction was completed, the solvent was directly removed by spin drying to give compound **291-1** (7.33 g, 92.84%).

$^1$H NMR (400 MHz, DMSO-$d_6$, *ppm*) $\delta$ 9.28 (s, 1H), 1.59 (s, 3H), 1.58 - 1.53 (m, 1H), 1.43 (s, 9H), 0.65 (tq, *J* = 6.6, 2.1 Hz, 4H).

**Step 2: Synthesis of compound 291-2**

Tert-butyl 2-(1-cyclopropylethylidene)hydrazine-1-carboxylate (compound **291-1,** 2 g, 10.087 mmol, 1 eq) was added to a reaction flask, dissolved in tetrahydrofuran (25.0 mL), and then acetic acid (1.0 mL, 17.451 mmol, 1.73 eq) was added. The mixture was cooled to 0°C, sodium cyanoborohydride (1.58 g, 25.218 mmol, 2.5 eq) was added at 0°C, and then the mixture was warmed to room temperature to react overnight. After the reaction was completed, the solvent was removed by spin drying, the residue was dissolved in ethyl acetate (150 mL), and saturated sodium chloride solution (50 mL) was added. The organic phase was collected, washed with saturated sodium bicarbonate solution (50 mL) and saturated sodium chloride solution (50 mL), dried over anhydrous sodium sulfate, filtered, and the filtrate was concentrated under reduced pressure. The resulting residue was purified by silica gel column chromatography (petroleum ether/ethyl acetate = 5:1) to give compound **291-2** (1.4 g, 34.65%).

$^1$H NMR (400 MHz, DMSO-$d_6$, *ppm*) $\delta$ 9.59 (dd, *J* = 34.8, 8.1 Hz, 1H), 8.66 (s, 1H), 1.45 (s, 9H), 1.38 (s, 3H), 0.87 (ddq, *J* = 16.9, 8.4, 4.7, 4.0 Hz, 1H), 0.62 - 0.29 (m, 4H), 0.17 (ddq, *J* = 21.7, 10.9, 5.5 Hz, 1H).

**Step 3: Synthesis of compound 291-3**

Tert-butyl 2-(1-cyclopropylethyl)hydrazine-1-carboxylate (compound **291-2,** 1.4 g, 6.990 mmol, 1 eq) and 4 mol/L hydrogen chloride solution in 1,4-dioxane (15 mL) were added to a reaction flask, dissolved in dichloromethane (15 mL), and allowed to react at room temperature overnight. After the reaction was completed, the solvent was directly removed by spin drying to give **291-3** (1.65 g, 94.26%).

**Step 4: Synthesis of compound 291-4**

To a reaction flask, (1-cyclopropylethyl)hydrazine hydrochloride (compound **291-3,** 450 mg, 1.314 mmol, 1 eq, 40%), ethyl (*E*)-2-cyano-3-ethoxyacrylate (222.28 mg, 1.314 mmol, 1.00 eq), and sodium acetate (0.32 g, 3.901 mmol, 2.97 eq) were added, dissolved in ethanol (5 mL) and warmed to 80°C to react for 2 h. After the reaction was completed, water was added to quench the reaction. The reaction mixture was extracted with ethyl acetate (3 $\times$ 10 mL), the organic phases were combined, backwashed with saturated sodium chloride solution (1 $\times$ 10 mL), dried over anhydrous sodium sulfate, and filtered. The filtrate was concentrated under reduced pressure. The resulting residue was purified by silica gel column chromatography (petroleum ether/ethyl acetate = 5:1), to give compound **291-4** (200 mg, 64.77%).

LCMS: (ESI, m/z): 223.90 $[M+H]^+$.

**Step 5: Synthesis of compound 291-5**

Ethyl 5-amino-1-(1-cyclopropylethyl)-1*H*-pyrazole-4-carboxylate (compound **291-4,** 110 mg, 0.493 mmol, 1 eq), and sodium hydroxide aqueous solution (1 mL, 1M, 1.0 mmol, 2 eq) were added to a reaction flask, and dissolved in ethanol (2 mL). The reaction was carried out at 80°C overnight. After the reaction was completed, the solvent was directly removed by spin drying to give compound **291-5** (100 mg, 98.77%).

**Step 6: Synthesis of 5-amino-1-(1-cyclopropylethyl)-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1*H*-pyrazole-4-carboxamide (compound 291)**

To a reaction flask, 2-(3-aminoprop-1-yn-1-yl)-*N*-((3*S*,4*R*)-3-fluoro-1-methylpiperidin-4-yl)-3-(2,2,2-trifluoroethyl)pyrazolo[1,5-*a*]pyridin-7-amine dihydrochloride (compound **001-12,** 40 mg, 0.104 mmol, 1 eq), 5-amino-1-(1-cyclopropylethyl)-1*H*-pyrazole-4-carboxylic acid (compound **291-5,** 20.37 mg, 0.104 mmol, 1 eq), *O*-(7-azabenzotriazol-1-yl)-*N,N,N',N'*-tetramethyluronium hexafluorophosphate (47.6 mg, 0.125 mmol, 1.2 eq), and *N,N*-diisopropylethylamine (67.42 mg, 0.520 mmol, 5 eq) were added, dissolved in *N,N*-dimethylformamide (4 mL) and allowed to react at room temperature for 1 h. After the reaction was completed, water was added for quenching and the reaction mixture was

extracted with ethyl acetate (3 × 10 mL). The organic phases were combined, backwashed with saturated sodium chloride solution (1 × 10 mL), dried over anhydrous sodium sulfate, filtered, and the filtrate was concentrated under reduced pressure. The crude product was purified by HPLC with the following conditions (column specifications: Sunfire C18 5 μm, 30 mm × 150 mm; mobile phase A: water (0.1% formic acid), mobile phase B: acetonitrile; flow rate: 60 mL/min; elution gradient: 18% B to 35% B in 8 min; detection wavelength: UV 254 nm/220 nm; retention time (min): 5.87), to give compound **5-amino-1-(1-cyclopropylethyl)-*N*-[3-(7-{[(3*S*,4*R*)-3-fluoro-1-methylpiperidin-4-yl]amino}-3-(2,2,2-trifluoroethyl) pyrazolo[1,5-*a*]pyridin-2-yl)prop-2-yn-1-yl]-1*H*-pyrazole-4-carboxamide 291** (30.94 mg, 52.37%).

LCMS: (ESI, m/z): 561.60 [M+H]+.

$^1$H NMR (400 MHz, DMSO-$d_6$, *ppm*) $\delta$ 8.26 (t, *J* = 5.7 Hz, 1H), 7.72 (s, 1H), 7.28 (dd, *J* = 8.7, 7.6 Hz, 1H), 7.02 (d, *J* = 8.7 Hz, 1H), 6.31 (d, *J* = 7.6 Hz, 1H), 6.25 (d, *J* = 9.0 Hz, 1H), 6.16 (s, 2H), 4.88 (d, *J* = 49.4 Hz, 1H), 4.29 (d, *J* = 5.7 Hz, 2H), 3.92 - 3.81 (m, 1H), 3.75 (q, J = 11.1 Hz, 2H), 3.63 (td, *J* = 8.7, 6.6 Hz, 1H), 3.06 (t, *J* = 11.5 Hz, 1H), 2.79 (d, *J* = 11.1 Hz, 1H), 2.38 - 2.23 (m, 1H), 2.21 (s, 3H), 2.19 - 2.11 (m, 1H), 2.00 - 1.81 (m, 2H), 1.38 (d, *J* = 6.6 Hz, 3H), 1.29 - 1.17 (m, 1H), 0.57 - 0.49 (m, 1H), 0.36 - 0.26 (m, 2H), 0.25 - 0.16 (m, 1H).

**Step 7: Synthesis of compounds 291A and 291B**

The carboxylate of 5-amino-1-(1-cyclopropylethyl)-*N*-[3-(7-([(3*S*,4*R*)-3-fluoro-1-methylpiperidin-4-yl]amino-3-(2,2,2-trifluoroethyl)pyrazolo[1,5-*a*]pyridin-2-yl)prop-2-yn-1-yl]-1*H*-pyrazole-4-carboxamide **291** (28 mg, 0.0499 mmol, 1 eq) was subjected to chiral resolution with the following conditions (column specifications: CHIRALPAK-IA 3*25cm, 5 μm; mobile phase A: methyl tert-butyl ether (0.1% diethylamine), mobile phase B: methanol : dichloromethane = 1:1; flow rate: 20 mL/min; elution gradient: isocratic 7; detection wavelength: UV 220/254 nm; front peak retention time (min): 16.174; rear peak retention time (min): 22.827; sample dissolving solvent: ethanol; single injection volume: 0.4 mL; number of injections: 3), to give compounds **291A** (9.0 mg, 32.14%, rear peak) and **291B** (7.3 mg, 16.07%, front peak).

**291B:** LCMS:(ESI, m/z): 561.50 [M+H]+.

$^1$H NMR (400 MHz, DMSO-$d_6$) 8.26 (t, *J* = 5.7 Hz, 1H), 7.72 (s, 1H), 7.28 (dd, *J* = 8.7, 7.6 Hz, 1H), 7.02 (d, *J* = 8.7 Hz, 1H), 6.31 (d, J = 7.3 Hz, 1H), 6.25 (d, *J* = 9.0 Hz, 1H), 6.17 (s, 2H), 4.87 (d, *J* = 49.5 Hz, 1H), 4.28 (d, *J* = 5.7 Hz, 2H), 3.91 - 3.70 (m, 3H), 3.67 - 3.58 (m, 1H), 3.04 (t, *J* = 11.9 Hz, 1H), 2.78 (d, *J* = 11.4 Hz, 1H), 2.37 - 2.22 (m, 1H), 2.21 (s, 3H), 2.14 (t, *J* = 11.4 Hz, 1H), 2.00 - 1.88 (m, 1H), 1.87 - 1.77 (m, 1H), 1.38 (d, *J* = 6.6 Hz, 3H), 1.28 - 1.16 (m, 1H), 0.58 - 0.47 (m, 1H), 0.36 - 0.27 (m, 2H), 0.26 - 0.18 (m, 1H).

**291A:** LCMS:(ESI, m/z): 561.50 [M+H]+.

$^1$H NMR (400 MHz, DMSO-$d_6$, *ppm*) $\delta$ 8.26 (t, *J* = 5.7 Hz, 1H), 7.72 (s, 1H), 7.28 (dd, *J* = 8.8, 7.6 Hz, 1H), 7.02 (d, *J* = 8.7 Hz, 1H), 6.31 (d, J = 7.7 Hz, 1H), 6.25 (d, *J* = 9.1 Hz, 1H), 6.17 (s, 2H), 4.87 (d, J = 50.4 Hz, 1H), 4.28 (d, *J* = 5.6 Hz, 2H), 3.93 - 3.80 (m, 1H), 3.76 (q, J = 11.1 Hz, 2H), 3.63 (dq, J = 13.3, 6.6 Hz, 1H), 3.06 (t, *J* = 11.7 Hz, 1H), 2.78 (d, *J* = 11.2 Hz, 1H), 2.37 - 2.30 (m, 1H), 2.20 (s, 3H), 2.14 (t, *J* = 11.4 Hz, 1H), 2.00 - 1.88 (m, 1H), 1.87 - 1.78 (m, 1H), 1.38 (d, *J* = 6.6 Hz, 3H), 1.28 - 1.18 (m, 1H), 0.58 - 0.48 (m, 1H), 0.37 - 0.27 (m, 2H), 0.25 - 0.17 (td, *J* = 8.7, 4.2 Hz, 1H).

**Example 137**

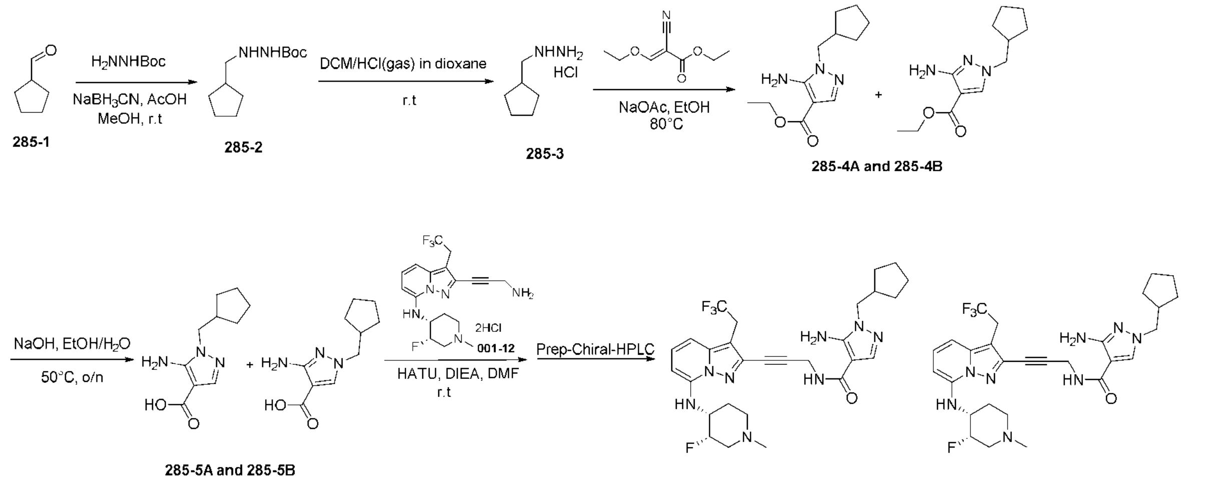

**Step 1: Synthesis of compound 285-2**

At room temperature, cyclopentanecarboxaldehyde (compound **285-1,** 5 g, 50.945 mmol, 1 eq) and tert-butoxyformyl hydrazide (6.73 g, 50.945 mmol, 1 eq) were added to a reaction flask, dissolved in methanol (100 mL), stirred at room temperature for 20 min, then acetic acid (2 mL) was added, and sodium cyanoborohydride (6.40 g, 101.890 mmol, 2 eq) was added in portions at 0°C over about 20 min. After the addition was completed, the system was allowed to react at room temperature for 2 h. After the reaction was completed, the resulting residue was concentrated under reduced pressure, the reaction mixture was diluted with ethyl acetate (50 mL), washed with saturated brine (3 × 20 mL), washed with sodium bicarbonate solution (2 × 20 mL), dried over anhydrous sodium sulfate, filtered, and the filtrate was concentrated under reduced pressure. The resulting residue was purified by silica gel column chromatography (petroleum ether/ethyl acetate = 20:1) to give compound *N*'-(cyclopentylmethyl)tert-butoxycarbohydrazide **285-2** (4.2 g, 38.47%).
LCMS: (ESI, m/z): 159.45 $[M+H]^+$.

**Step 2: Synthesis of compound 285-3**

At room temperature, N'-(cyclopentylmethyl)tert-butoxycarbohydrazide (compound **285-2,** 4 g, 18.665 mmol, 1 eq) was added to a reaction flask, dissolved in dichloromethane (24.0 mL), and then 4 mol/L hydrogen chloride in 1,4-dioxane (4M, 24.0 mL) was added and the mixture was stirred at room temperature for 2 h. After the reaction was completed, the resulting residue was concentrated under reduced pressure to give the hydrochloride of cyclopentylmethylhydrazine **285-3**(2 g, 93.84%). The crude product was not further purified and directly used in the next step.

**Step 3: Synthesis of compounds 285-4A and 285-4B**

At room temperature, cyclopentyl methylhydrazine (compound **285-3,** 3.50 g, 30.650 mmol, 1.00 eq), ethyl (2*E*)-2-cyano-3-ethoxyacrylate (5.19 g, 30.650 mmol, 1.00 eq), and sodium acetate (2.51 g, 30.650 mmol, 1 eq) were added to a 250 mL three-necked flask, dissolved in ethanol (50 mL)and stirred at 80°C for 2 h. After the reaction was completed, the mixture was filtered, the filter cake was washed with ethanol (3 × 10 mL) and the filtrate was concentrated under reduced pressure. The resulting residue was purified by silica gel column chromatography (petroleum ether/ethyl acetate = 3:1) to give a mixture of compounds ethyl 5-amino-1-(cyclopentyl methyl)-1*H*-pyrazole-4-carboxylate and ethyl 3-amino-1-(cyclopentyl methyl)-1*H*-pyrazole-4-carboxylate (compound **285-4A** and **compound 285-4B,** 800 mg, 11.00%).
LCMS: (ESI, m/z): 238.15 $[M+H]^+$.

**Step 3: Synthesis of compounds 285-5A and 285-5B**

At room temperature, a solution of a mixture of ethyl 5-amino-1-(cyclopentylmethyl)-1*H*-pyrazole-4-carboxylate and ethyl 3-amino-1-(cyclopentylmethyl)-1*H*-pyrazole-4-carboxylate (compound **285-4A** and **compound 285-4B,** 800 mg, 3.371 mmol, 1 eq) in ethanol (10 mL) was added to a reaction flask, and then a solution of sodium hydroxide (269.68 mg, 6.742 mmol, 2 eq) in water (5 mL) was added. The mixture was warmed to 80°C and stirred overnight. After the reaction was completed, the pH was adjusted to neutral with 3 mol/L hydrochloric acid solution, and ethyl acetate (3 × 10 mL) was added for extraction. The mixture was washed with saturated brine (3 × 20 mL), dried over anhydrous sodium sulfate, filtered, and the filtrate was concentrated under reduced pressure. The resulting residue was concentrated under reduced pressure to give a mixture of compounds 5-amino-1-cyclopentylmethyl-1*H*-pyrazole-4-carboxylic acid and 3-amino-1-cyclopentylmethyl-1*H*-pyrazole-4-carboxylic acid (compound **285-5A** and compound **285-5B,** 600 mg, 85.06%).
LCMS: (ESI, m/z): 210.40 $[M+H]^+$.

**Step 5: Synthesis of compounds 285 and 349**

Under nitrogen protection, a mixture of 5-amino-1-cyclopentylmethyl-1*H*-pyrazole-4-carboxylic acid and 3-amino-1-cyclopentylmethyl-1*H*-pyrazole-4-carboxylic acid (compound **285-5A** and **compound 285-5B,** 55.03 mg, 0.263 mmol, 1.2 eq), and 2-(7-azabenzotriazole)-*N*,*N*,*N*',*N*'-tetramethyluronium hexafluorophosphate (83.33 mg, 0.219 mmol, 1 eq), *N,N*-diisopropylethylamine (141.62 mg, 1.095 mmol, 5 eq) were added to a reaction flask, dissolved in *N*,*N*-dimethylformamide (3 mL), and stirred at room temperature for 10 min. Then, 2-(3-aminoprop-1-yn-1-yl)-N-((3S,4R)-3-fluoro-1-methylpiperidin-4-yl)-3-(2,2,2-trifluoroethyl)pyrazolo[1,5-a]pyridine-7-amine dihydrochloride (compound 001-12, 100 mg, 0.219 mmol, 1.00 eq) was added. The mixture was further stirred to react for 1 h. After the reaction was completed, water (10 mL) was added, and the reaction mixture was extracted with ethyl acetate (20 mL × 2). The organic phases were combined, backwashed with saturated sodium chloride solution (30 mL × 1) and dried over anhydrous sodium sulfate, filtered, and the filtrate was concentrated under reduced pressure. The crude product was

purified by HPLC with the following conditions (column specifications: Xselect CSHTM Prep C18 5 μm 30*150mm OBD; mobile phase A: water (0.1% formic acid), mobile phase B: acetonitrile; flow rate: 60 mL/min; elution gradient: 12% B to 35% B in 10 min; detection wavelength: UV 254 nm/220 nm; retention time (min): 8.57), to give a mixture of 5-amino-1-(cyclopentylmethyl)-*N*-(3-(7-((3*S*,4*R*)-3-fluoro-1-methylpiperidin-4-yl)amino)-3-(2,2,2-trifluoroethyl)pyrazolo[1,5-*a*]pyridin-2-yl)prop-2-yn-1-yl)-1*H*-pyrazole-4-carboxamide and 3-amino-1-(cyclopentylmethyl)-*N*-(3-(7-((3*S*,4*R*)-3-fluoro-1-methylpiperidin-4-yl)amino)-3-(2,2,2-trifluoroethyl)pyrazolo[1,5-*a*]pyridin-2-yl)prop-2-yn-1-yl)-1*H*-pyrazole-4-carboxamide. The mixture was subjected to chiral resolution with the following conditions (column specifications: CHIRAL ART Cellulose-SB 3*25cm, 5 μm; mobile phase A: n-hexane (0.1% formic acid), mobile phase B: ethanol : dichloromethane = 1:1; flow rate: 40 mL/min; elution gradient: isocratic 35; detection wavelength: UV 254/220 nm; front peak retention time (min): 5.057; rear peak retention time (min): 8.424; sample dissolving solvent: methanol; single injection volume: 1.0 mL; injection times: 2), to give compound **285 (front peak)** (21.78 mg, 17.23%) and compound **349 (rear peak)** (2.74 mg, 9.08%).

Front peak (compound **285**): LCMS:(ESI, m/z): 575.10 $[M+H]^+$.

$^1H$ NMR (400 MHz, DMSO-$d_6$, *ppm*) $\delta$ 8.25 (t, *J* = 5.7 Hz, 1H), 7.67 (s, 1H), 7.28 (dd, *J* = 8.7, 7.6 Hz, 1H), 7.02 (d, *J* = 8.7 Hz, 1H), 6.31 (d, *J* = 7.6 Hz, 1H), 6.28 - 6.17 (m, 3H), 4.87 (d, *J* = 49.2 Hz, 1H), 4.29 (d, *J* = 5.6 Hz, 2H), 3.93 - 3.65 (m, 5H), 3.05 (t, *J* = 11.4 Hz, 1H), 2.78 (d, *J* = 11.4 Hz, 1H), 2.37 - 2.22 (m, 2H), 2.20 (s, 3H), 2.14 (t, J = 10.8 Hz, 1H), 1.98 - 1.81 (m, 2H), 1.65 - 1.52 (m, 4H), 1.51 - 1.40 (m, 2H), 1.33 - 1.19 (m, 2H).

Rear peak (compound **349**): LCMS: (ESI, m/z): 575.00 $[M+H]^+$.

$^1H$ NMR (400 MHz, DMSO-$d_6$, *ppm*) $\delta$ 8.31 (t, *J* = 5.5 Hz, 1H), 7.94 (s, 1H), 7.28 (dd, *J* = 8.8, 7.6 Hz, 1H), 7.03 (d, *J* = 8.7 Hz, 1H), 6.31 (d, J = 7.5 Hz, 1H), 6.28 (s, 1H), 5.38 (s, 2H), 4.89 (d, *J* = 49.5 Hz, 1H), 4.29 (d, *J* = 5.6 Hz, 2H), 3.85 - 3.71 (m, 5H), 3.18 - 2.98 (m, 1H), 2.91 - 2.73 (s, 1H), 2.34 - 2.14 (m, 6H), 2.01 - 1.82 (m, 2H), 1.65 - 1.49 (m, 6H), 1.33 - 1.10 (m, 2H).

**Example 138**

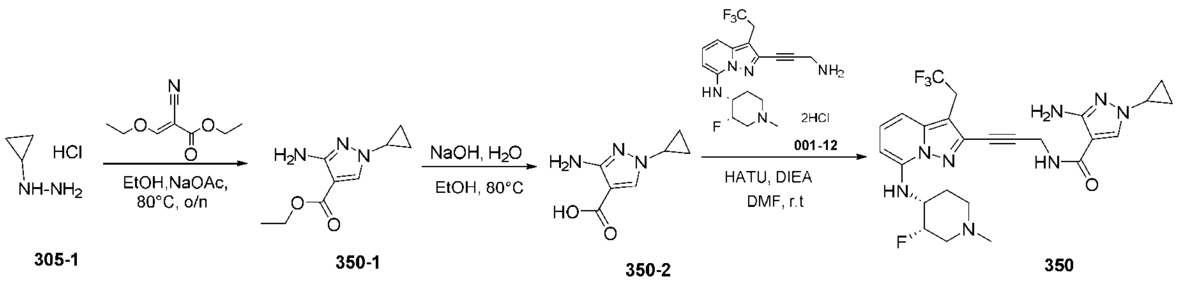


305-1 350-1 350-2 350

**Step 1: Synthesis of compound 350-1**

Under nitrogen protection, cyclopropyl hydrazine hydrochloride (10 g, 92.106 mmol, 1 eq), ethyl (*E*)-2-cyano-3-ethoxyacrylate (15.58 g, 92.106 mmol, 1 eq), and sodium acetate (22.67 g, 276.318 mmol, 3 eq) were added to a reaction flask, dissolved in anhydrous ethanol (100 mL), warmed to 80°C and stirred to react overnight. After the reaction was completed, the reaction mixture was concentrated under reduced pressure. The resulting residue was purified by silica gel column chromatography (petroleum ether/ethyl acetate = 3: 1) to give compound ethyl 3-amino-1-cyclopropyl-1*H*-pyrazole-4-carboxylate **350-1** (9 g, 48.60%).

LCMS: (ESI, m/z): 195.95 $[M+H]^+$.

**Step 2: Synthesis of compound 350-2**

Under nitrogen protection, a mixture of ethyl 3-amino-1-cyclopropyl-1*H*-pyrazole-4-carboxylate and **305-2** (9 g, 51.224 mmol, 1 eq), and sodium hydroxide (4.10 g, 102.448 mmol, 2 eq) were added to a reaction flask, dissolved in anhydrous ethanol (50 mL) and water (20 mL), and warmed to 80°C and stirred to react for 2 h. After the reaction was completed, the reaction mixture was concentrated under reduced pressure, and the crude product was acidified to a pH of about 3 with 1 mol/L hydrochloric acid solution. The aqueous phase was extracted with ethyl acetate (50 mL × 2), and the organic phases were combined and concentrated under reduced pressure to give crude compound 3-amino-1-cyclopropyl-1*H*-pyrazole-4-carboxylic acid (6 g, 70.00%). The crude product was subjected to chiral resolution with the following conditions (column specifications: CHIRALPAK-IG 3*25cm, 5μm; mobile phase A: n-hexane (0.1% formic acid), mobile phase B: ethanol:dichloromethane = 1:1; flow rate: 40 mL/min; elution gradient: isocratic 30; detection

wavelength: UV 254/220 nm; retention time (min): 10.054; rear peak retention time (min): 17.283; sample dissolving solvent: ethanol/dichloromethane; single injection volume: 2.0 mL; injection times: 15), to give compound 3-amino-1-cyclopropyl-1*H*-pyrazole-4-carboxylic acid **350-2** (1.9 g, 22.16%).

LCMS: (ESI, m/z): 168.00 [M+H]$^+$.

**Step 3: Synthesis of 3-amino-*N*-[3-(7-{[(3*S*,4*R*)-3-fluoro-1-methylpiperidin-4-yl]amino}-3-(2,2,2-trifluoroethyl) pyrazolo[1,5-*a*]pyridin-2-yl)prop-2-yn-1-yl]-1-(cyclopropyl)-1*H*-pyrazole-4-carboxamide (compound 350)**

Under nitrogen protection, 3-amino-1-cyclopropyl-1*H*-pyrazole-4-carboxylic acid (21.98 mg, 0.132 mmol, 1.2 eq), 2-(7-azabenzotriazole)-*N*,*N*,*N'*,*N'*-tetramethyluronium hexafluorophosphate (41.66 mg, 0.110 mmol, 1 eq), and *N*,*N*-diisopropylethylamine (70.81 mg, 0.550 mmol, 5 eq) were added to a reaction flask, dissolved in N,N-dimethylformamide (2 mL) and stirred at room temperature for 10 min. Then, 2-(3-aminoprop-1-yn-1-yl)-N-((3S,4R)-3-fluoro-1-methylpiperidin-4-yl)-3-(2,2,2-trifluoroethyl)pyrazolo[1,5-a]pyridin-7-amine dihydrochloride (compound **001-12,** 50 mg, 0.110 mmol, 1.00 eq) was added, and the system was stirred to react at room temperature for 1 h. After the reaction was completed, water (10 mL) was added and the reaction mixture was extracted with ethyl acetate (20 mL × 2). The organic phases were combined, backwashed with saturated sodium chloride solution (30 mL × 1), dried over anhydrous sodium sulfate, filtered, and the filtrate was concentrated under reduced pressure. The crude product was purified by HPLC with the following conditions (column specifications: XBridge BEH C18 OBD Prep Column 130, 5μm, 30 mm * 150 mm; mobile phase A: water (0.1% formic acid), mobile phase B: acetonitrile; flow rate: 60 mL/min; elution gradient: 20% B to 30% B in 12 min; detection wavelength: UV 254 nm/220 nm; retention time (min): 8.85), to give the carboxylate of compound **3-amino-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl) prop-2-yn-1-yl]-1-(cyclopropyl)-1*H*-pyrazole-4-carboxamide 350** (19.56 mg, 30.48%).

LCMS: (ESI, m/z): 533.50 [M+H]$^+$.

$^1$H NMR (400 MHz, DMSO-$d_6$, *ppm*) δ 8.29 (t, *J* = 5.6 Hz, 1H), 8.02 (s, 1H), 7.28 (dd, *J* = 8.7, 7.6 Hz, 1H), 7.03 (d, *J* = 8.7 Hz, 1H), 6.32 (d, *J* = 7.5 Hz, 1H), 6.23 (d, *J* = 9.0 Hz, 1H), 5.39 (s, 2H), 4.87 (d, *J* = 49.4 Hz, 1H), 4.29 (d, *J* = 5.6 Hz, 2H), 3.93 - 3.81 (m, 1H), 3.75 (q, *J* = 11.1 Hz, 2H), 3.53 - 3.47 (m, 1H), 3.06 (t, J = 10.6 Hz, 1H), 2.78 (d, *J* = 11.4 Hz, 1H), 2.36 - 2.22 (m, 1H), 2.21 (s, 3H), 2.14 (t, J = 10.5 Hz, 1H), 1.97 - 1.81 (m, 2H), 0.92 - 0.87 (m, 4H).

**Example 139**

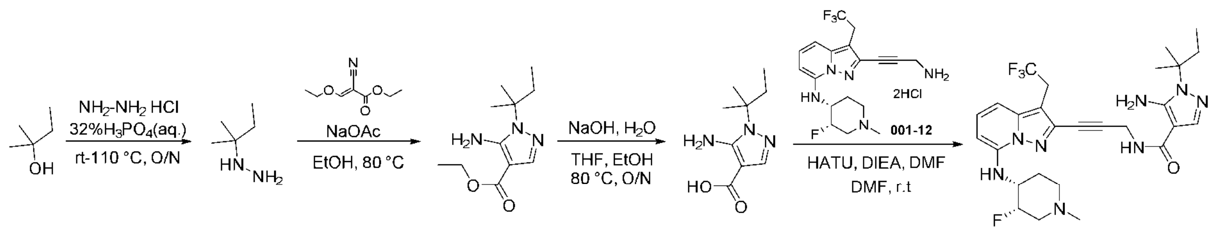


351-1 351-2 351-3 351-4 351

**Step 1: Synthesis of compound 351-2**

At room temperature, to a 32% saturated solution of crystalline phosphoric acid (15.01 g, 153.208 mmol, 4 eq) and hydrazine hydrochloride (6 g, 38.302 mmol, 1 eq) was added 2-methyl-2-butanol (compound **351-1,** 60 mL), the mixture was stirred, and after dissolution, heated to 110°C to react overnight, and the reaction was completed. The resulting residue was concentrated under reduced pressure to give compound 4-pentyl hydrazine **351-2** (4 g, 56.21%), which was not further purified and directly used in the next step.

**Step 2: Synthesis of compound 351-3**

At room temperature, 4-pentylhydrazine (compound **351-2,** 4 g, 39.146 mmol, 1 eq), sodium acetate (4816.98 mg, 58.719 mmol, 1.5 eq) and ethyl (2*E*)-2-cyano-3-carboxylate (6622.76 mg, 39.146 mmol, 1 eq) were added to a reaction flask, dissolved in ethanol (80 mL) and stirred to react at 80°C for 4 h. After the reaction was completed, the resulting residue was concentrated under reduced pressure. The resulting residue was purified by silica gel column chromatography (petroleum ether/ethyl acetate = 10:1) to give compound ethyl 5-amino-1-(2-methylbutyl-2-yl)-1*H*-pyrazole-4-carboxylate **351-3** (1 g, 10.20%).

LCMS: (ESI, m/z): 226.40 [M+H]$^+$.

**Step 3: Synthesis of compound 351-4**

At room temperature, a solution of ethyl 5-amino-1-(2-methylbutyl-2-yl)-1*H*-pyrazole-4-carboxylate (compound **351-3,** 100 mg, 0.444 mmol, 1 eq) and sodium hydroxide (88.77 mg, 2.220 mmol, 5 eq) in water (1 mL)/tetrahydrofuran (1 mL)/ethanol (1 mL) was added to a reaction flask, and stirred at 80°C overnight. After the reaction was completed, the reaction mixture was concentrated in vacuum, the residue was dissolved in tetrahydrofuran (10 mL), and filtered, and the filtrate was concentrated in vacuum, to give compound 5-amino-1-(2-methylbutyl-2-yl)-1*H*-pyrazole-4-formic acid **351-4** (50 mg, 57.11%).

LCMS: (ESI, m/z): 198.05 $[M+H]^+$.

**Step 4: Synthesis of 5-amino-*N*-[3-(7-{[(3*S*,4*R*)-3-fluoro-1-methylpiperidin-4-yl]amino}-3-(2,2,2-trifluoromethylethyl)pyrazolo[1,5-*a*]pyridin-2-yl)prop-2-yn-1-yl]-1-tert-pentyl-1*H*-pyrazole-4-carboxamide (compound 351)**

At room temperature, a solution of 2-(3-aminoprop-1-yn-1-yl)-N-((3S,4R)-3-fluoro-1-methylpiperidin-4-yl)-3-(2,2,2-trifluoroethyl)pyrazolo[1,5-a]pyridin-7-amine dihydrochloride (compound **001-12,** 50 mg, 0.130 mmol, 1 eq), 5-amino-1-(2-methylbutyl-2-yl)-1*H*-pyrazole-4-carboxylic acid (compound 351-4, 32 mg, 0.130 mmol, 1 eq), *O*-(7-azabenzotriazol-1-yl)-*N*,*N*,*N'*,*N'*-tetramethyluronium hexafluorophosphate (49.59 mg, 0.130 mmol, 1.00 eq) and *N,N*-diisopropylethylamine (50.57 mg, 0.391 mmol, 3.00 eq) in *N*,*N*-dimethylformamide (3 mL) was stirred to react for 2 h. After the reaction was completed, the crude product was purified by HPLC with the following conditions (column specifications: XBridge BEH Shield RP18 5 μm, 30 mm *150 mm; mobile phase A: water (10 mmol/L ammonium bicarbonate), mobile phase B: acetonitrile; flow rate: 60 mL/min; elution gradient: 40% B to 63% B in 9 min; detection wavelength: UV 254 nm/226 nm; retention time (min): 6.72), to give compound **5-amino-*N*-[3-(7-{[(3*S*,4*R*)-3-fluoro-1-methylpiperidin-4-yl] amino}-3-(2,2,2-trifluoromethylethyl)pyrazolo[1,5-*a*]pyridin-7-yl)prop-2-yn-1-yl]-1-tert-pentyl-1*H*-pyrazole-4-carboxamide 351** (7.51 mg, 10.19%).

LCMS: (ESI, m/z): 562.95 $[M+H]^+$.

$^1$H NMR (400 MHz, DMSO-$d_6$, *ppm*) $\delta$ 8.30 (t, *J* = 5.7 Hz, 1H), 7.66 (s, 1H), 7.32 - 7.24 (m, 1H), 7.02 (d, *J* = 8.7 Hz, 1H), 6.31 (d, *J* = 7.5 Hz, 1H), 6.24 (d, *J* = 9.0 Hz, 1H), 6.14 (s, 2H), 4.87 (d, J = 49.8 Hz, 1H), 4.28 (d, *J* = 5.6 Hz, 2H), 3.87-3.71 (m, 3H), 3.05 (t, *J* = 11.5 Hz, 1H), 2.78 (d, *J* = 11.2 Hz, 1H), 2.33-2.20 (m, 4H), 2.15 - 2.05 (m, 1H), 1.97 - 1.80 (m, 4H), 1.50 (s, 6H), 0.65 (t, *J* = 7.4 Hz, 3H).

**Example 140**

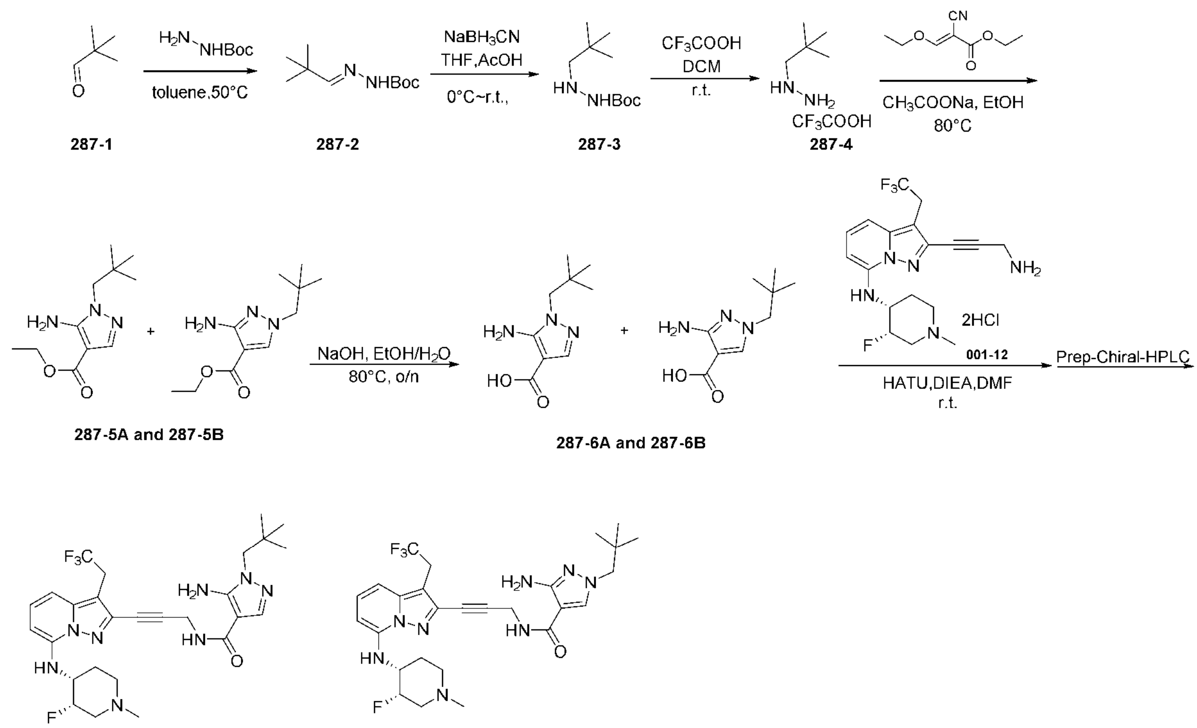

**Step** 1: **Synthesis of compound 287-2**

At room temperature, trimethylacetaldehyde (compound **287-1,** 5.08 g, 59.018 mmol, 1.2 eq) and tert-butoxycarbonyl hydrazide (6.5 g, 49.182 mmol, 1.00 eq) were added to a reaction flask, dissolved in toluene (100 mL) and stirred at 50°C for 1 h. After the reaction was completed, the resulting residue was concentrated under reduced pressure to give compound *N'*-[(1*E*)-2,2-dimethylpropylidene]tert-butoxycarbonyl hydrazide **287-2** (9.8 g, 99.49%).
LCMS: (ESI, m/z): 144.95 $[M+H]^+$.

**Step 2: Synthesis of compound 287-3**

At room temperature, a solution of *N'*-[(1*E*)-2,2-dimethylpropylene] tert-butoxycarbohydrazide (compound **287-2,** 10 g, 49.930 mmol, 1 eq) in tetrahydrofuran (121.4 mL) was added to a reaction flask, acetic acid (5 mL, 87.377 mmol, 1.75 eq) was added at 0°C, and then, sodium cyanoborohydride (7.84 g, 124.825 mmol, 2.5 eq) was added in portions over 20 min. The mixture was stirred at room temperature for 4 h. After the reaction was completed, the resulting residue was concentrated under reduced pressure, diluted with ethyl acetate (20 mL), washed with saturated sodium chloride solution ($3 \times 20$ mL), washed with saturated sodium carbonate solution ($2 \times 20$ mL), dried over anhydrous sodium sulfate solution, filtered, and the filtrate was concentrated under reduced pressure. The resulting residue was purified by silica gel column chromatography (petroleum ether/ethyl acetate =5:1), to give compound *N'*-(2,2-dimethylpropyl) tert-butoxycarbohydrazide **287-3** (3 g, 29.70%).

**Step 3: Synthesis of compound 287-4**

At room temperature, a solution of *N'*-(2,2-dimethylpropyl) tert-butoxycarbonylhydrazine (compound **287-3,** 3 g, 14.830 mmol, 1 eq) and trifluoroacetic acid (3 mL, 21.583 mmol, 1.46 eq) in dichloromethane (30 mL) was added to a reaction flask and stirred at room temperature for 2 h. The resulting residue was concentrated under reduced pressure to give compound 2,2-dimethylpropylhydrazine trifluoroacetate **287-4** (3 g, 93.57%). The crude product was not further purified and was directly used in the next step.

**Step 4: Synthesis of compounds 287-5A and** compound **287-5B**

At room temperature, 2,2-dimethylpropylhydrazine trifluoroacetate (compound **287-4,** 3 g, 13.876 mmol, 1 eq), ethyl (2*E*)-2-cyano-3-ethoxyacrylate (2.38 g, 13.876 mmol, 1 eq), and sodium acetate (2.28 g, 27.752 mmol, 2 eq) were added to a reaction flask, dissolved in ethanol (30 mL)and stirred at 80°C for 4 h. After the reaction was completed, the mixture was filtered, the filter cake was washed with ethyl acetate, and the filtrate was concentrated under reduced pressure. The resulting residue was purified by silica gel column chromatography (petroleum ether/ethyl acetate = 3:1) to give a mixture of compounds ethyl 5-amino-1-(2,2-dimethylpropyl)pyrazole-4-carboxylate and ethyl 3-amino-1-(2,2-dimethylpropyl)pyrazole-4-carboxylate (compound **287-5A** and compound **287-5B,** 1 g, 31.99%).
LCMS: (ESI, m/z): 226.05 $[M+H]^+$.

**Step 5: Synthesis of compounds 287-6A and 287-6B**

At room temperature, a solution of a mixture of ethyl 5-amino-1-(2,2-dimethylpropyl)pyrazole-4-carboxylate and ethyl 3-amino-1-(2,2-dimethylpropyl)pyrazole-4-carboxylate (compounds **287-5A** and **287-5B,** 1 g, 4.439 mmol, 1 eq) in ethanol (10 mL) was added to a reaction flask, then a solution of sodium hydroxide (355.07 mg, 8.878 mmol, 2 eq) in water (5 mL) was added. The mixture was stirred at 80°C overnight. After the reaction was completed, the resulting residue was concentrated under reduced pressure to give a mixture of 5-amino-1-(2,2-dimethylpropyl)-1*H*-pyrazole-4-carboxylic acid and 3-amino-1-(2,2-dimethylpropyl)-1*H*-pyrazole-4-carboxylic acid (compounds **287-6A** and **287-6B,** 800 mg, 91.38%) as a crude product. The resulting mixture was not further purified and was directly used in the next step.
LCMS: (ESI, m/z): 193.98 $[M+H]^+$.

**Step 6: Synthesis of compounds 287 and 352**

At room temperature, a solution of a mixture 5-amino-1-(2,2-dimethylpropyl)-1*H*-pyrazole-4-carboxylic acid and 3-amino-1-(2,2-dimethylpropyl)-1*H*-pyrazole-4-carboxylic acid (compounds **287-6A** and **287-6B,** 43.22 mg, 0.220 mmol, 2 eq), *N*,*N*-diisopropylethylamine (56.65 mg, 0.440 mmol, 4 e) and O-(7-azabenzoxazol-1-yl)-*N,N,N',N'*-tetramethyluronium hexafluorophosphate (45.83 mg, 0.121 mmol, 1.1 eq) in *N*,*N*-dimethylformamide (2 mL) was added to a reaction flask and stirred to react for 10 min. Then 2-(3-aminoprop-1-yn-1-yl)-N-((3S,4R)-3-fluoro-1-methylpiperidin-4-yl)-3-(2,2,2-trifluoroethyl)pyrazolo[1,5-a]pyridine-7-amine dihydrochloride (compound **001-12,** 50 mg, 0.110 mmol, 1 eq) was added

and the mixture was stirred at room temperature for 1 h. The desired product was found by LC-MS. The reaction mixture was quenched with water (5 mL) at room temperature. The aqueous phase was extracted with ethyl acetate (3×10 mL). The organic phases were combined, and washed with saturated brine (3 × 10 mL). The mixture was dried over anhydrous sodium sulfate, and the resulting residue was concentrated in vacuum.The crude product was purified by HPLC with the following conditions (column specifications: XBridge BEH C18 OBD Prep Column 130, 5 μm, 30 mm * 150 mm; mobile phase A: water (17 mmol/L ammonium bicarbonate), mobile phase B: acetonitrile; flow rate: 60 mL/min; elution gradient: 42% B to 48% B in 8 min; detection wavelength: UV 254 nm/220 nm; front peak retention time (min): 6.35, rear peak retention time (min): 7.02), to give compounds **352** (3.2 mg, 5.08%, front peak) and **287** (5.33 mg, 8.59%, **rear** peak).

Front peak **352:** LCMS:(ESI, m/z): 562.95 $[M+H]^+$.

$^1H$ NMR (400 MHz, Methanol-$d_4$, *ppm*) δ 7.84 (s, 1H), 7.25 (dd, *J* = 8.8, 7.6 Hz, 1H), 6.94 (d, *J* = 8.7 Hz, 1H), 6.19 (d, J = 7.3 Hz, 1H), 5.01 - 4.92 (m, 1H), 4.38 (s, 2H), 3.93 - 3.75 (m, 1H), 3.73 (s, 2H), 3.65 (q, *J* = 10.8 Hz, 2H), 3.22 (t, *J* = 11.5 Hz, 1H), 2.92 (d, *J* = 11.8 Hz, 1H), 2.53 - 2.35 (m, 1H), 2.32 (s, 3H), 2.30 - 2.24 (m, 1H), 2.08 - 1.97 (m, 2H), 0.96 (s, 9H).

Rear peak **287:** LCMS:(ESI, m/z): 562.95 $[M+H]^+$.

$^1H$ NMR (400 MHz, DMSO-$d_6$, *ppm*) δ 8.25 (t, *J* = 5.6 Hz, 1H), 7.69 (s, 1H), 7.28 (dd, *J* = 8.8, 7.6 Hz, 1H), 7.02 (d, *J* = 8.7 Hz, 1H), 6.31 (d, J = 7.4 Hz, 1H), 6.28 - 6.18 (m, 3H), 4.87 (d, *J* = 49.6 Hz, 1H), 4.29 (d, *J* = 5.7 Hz, 2H), 3.94 - 3.80 (m, 1H), 3.75 (q, *J* = 11.2 Hz, 2H), 3.68 (s, 2H), 3.05 (t, *J* = 11.5 Hz, 1H), 2.78 (d, *J* = 11.5 Hz, 1H), 2.36 - 2.22 (m, 1H), 2.20 (s, 3H), 2.13 (t, J = 10.6 Hz, 1H), 2.00 - 1.87 (m, 1H), 1.87 - 1.80 (m, 1H), 0.92 (s, 9H).

**Example 141**

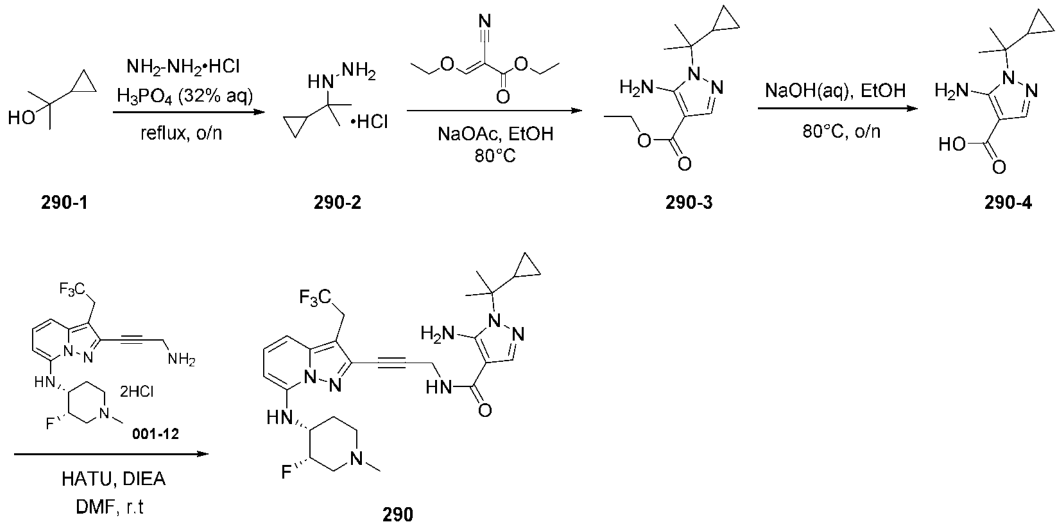


290-1 290-2 290-3 290-4

290

**Step 1: Synthesis of compound 290-2**

2-cyclopropylpropan-2-ol (compound **290-1,** 1 g, 9.984 mmol, 1 eq), hydrazine hydrochloride (683.96 mg, 9.984 mmol, 1 eq), and phosphoric acid (0.61 g, 1.992 mmol, 0.20 eq, 32% aqueous solution) were added to a reaction flask, and the mixture was allowed to react at 100°C overnight. After the reaction was completed, the mixture was directly dried to give crude product (2-cyclopropylpropan-2-yl)hydrazine hydrochloride **290-2** (770 mg, 67.54%), which was directly used in the next step of reaction.

**Step 2: Synthesis of compound 290-3**

To a reaction flask, (2-cyclopropylprop-2-yl)hydrazine hydrochloride (compound **290-2,** 570 mg, 4.992 mmol, 1 eq), ethyl 2-cyano-3-ethoxyacrylate (0.84 g, 4.992 mmol, 1 eq), and sodium acetate (1.23 g, 14.976 mmol, 3 eq) were added, dissolved in ethanol (10 mL) and heated to 80°C to react for 2 h. After the reaction was completed, water was added to quench the reaction. The reaction mixture was extracted with ethyl acetate (3 × 10 mL), the organic phases were combined, backwashed with saturated sodium chloride solution (1 × 10 mL), dried over anhydrous sodium sulfate, and filtered. The filtrate was concentrated under reduced pressure. The resulting residue was purified by silica gel column chromatography (petroleum ether/ethyl acetate = 5:1), to give compound ethyl 5-amino-1-(2-cyclopropylprop-2-yl)-1*H*-pyrazole-4-carboxylate **290-3** (200 mg, 16.04%).

LCMS: (ESI, m/z): 237.85 [M+H]+.

**Step 3: Synthesis of compound 290-4**

Ethyl 5-amino-1-(2-cyclopropylprop-2-yl)-1*H*-pyrazole-4-carboxylate (compound **290-3,** 95 mg, 0.400 mmol, 1 eq) and 1 mol/L sodium hydroxide aqueous solution (0.8 mL, 0.800 mmol, 2 eq) were added to a reaction flask, dissolved in ethanol (3 mL), and allowed to react at 80°C overnight. After the reaction was completed, the reaction mixture was acidified to pH=3 with hydrochloric acid solution, and the solvent was directly removed by spin drying to give compound 5-amino-1-(2-cyclopropylprop-2-yl)-1*H*-pyrazole-4-carboxylic acid **290-4** (90 mg, 96.69%).

LCMS: (ESI, m/z): 210.40 [M+H]+.

**Step 3: Synthesis of 5-amino-1-(2-cyclopropylprop-2-yl)-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1*H*-pyrazole-4-carboxamide (compound 290)**

To a reaction flask, 2-(3-aminoprop-1-yn-1-yl)-N-((3S,4R)-3-fluoro-1-methylpiperidin-4-yl)-3-(2,2,2-trifluoroethyl)pyrazolo[1,5-a]pyridin-7-amine dihydrochloride (compound **001-12,** 40 mg, 0.104 mmol, 1 eq), 5-amino-1-(2-cyclopropylprop-2-yl)-1*H*-pyrazole-4-carboxylic acid (compound **290-4,** 21.83 mg, 0.104 mmol, 1 eq), *O*-(7-azabenzotriazol-1-yl)-*N,N,N',N'*-tetramethyluronium hexafluorophosphate (47.6 mg, 0.125 mmol, 1.2 eq), and *N,N*-diisopropylethylamine (67.42 mg, 0.520 mmol, 5 eq) were added, dissolved in *N,N*-dimethylformamide (4 mL) and allowed to react at room temperature for 1 h. After the reaction was completed, water was added for quenching and the reaction mixture was extracted with ethyl acetate (3 × 10 mL). The organic phases were combined, backwashed with saturated sodium chloride solution (1 × 10 mL), dried over anhydrous sodium sulfate, filtered, and the filtrate was concentrated under reduced pressure. The crude product was purified by HPLC with the following conditions (column specifications: Xselect CSHTM Prep C18 5μm 30* 150mm OBD; mobile phase A: water (0.1% formic acid), mobile phase B: acetonitrile; flow rate: 60 mL/min; elution gradient: 15% B to 33% B in 10 min; detection wavelength: UV 254 nm/220 nm; retention time (min): 8.55), to give compound **5-amino-1-(2-cyclopropylprop-2-yl)-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-1*H*-pyrazole-4-carboxamide 290** (48.6 mg, 77.98%).

LCMS: (ESI, m/z): 575.15 [M+H]+.

$^1$H NMR(400 MHz, DMSO-$d_6$, *ppm*) δ 8.31 (t, *J* = 6.2 Hz, 1H), 7.67 (s, 1H), 7.36 - 7.28 (m, 1H), 7.07 (d, *J* = 8.7 Hz, 1H), 6.72 (s, 1H), 6.31 (d, *J* = 7.6 Hz, 1H), 6.11 (s, 2H), 5.19 (d, *J* = 47.3 Hz, 1H), 4.28 (d, *J* = 5.7 Hz, 2H), 4.19 - 3.95 (m, 1H), 3.77 (q, *J* = 11.1 Hz, 2H), 3.55 - 3.40 (m, 1H), 3.22 - 3.04 (m, 1H), 2.92 - 2.70 (m, 4H), 2.47 - 2.38 (m, 1H), 2.31 - 2.22 (m, 1H), 2.07 (d, J = 11.7 Hz, 1H), 1.44 - 1.32 (m, 7H), 0.52 - 0.40 (m, 4H).

**Example 142**

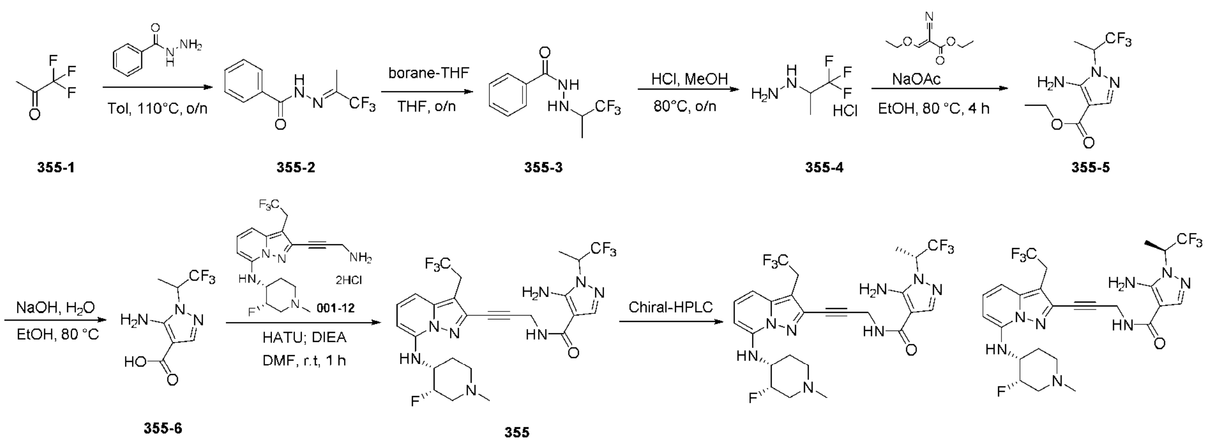


355-1 355-2 355-3 355-4 355-5

355-6 001-12 355

**Step 1: Synthesis of compound 355-2**

Under nitrogen protection, trifluoroacetone (compound **355-1,** 12.34 g, 110.169 mmol, 3 eq), and benzoylhydrazine (5 g, 36.723 mmol, 1.00 eq) were added to a reaction flask, dissolved in toluene (20 mL), warmed to 110°C and

stirred to react overnight. After the reaction was completed, the reaction mixture was concentrated under reduced pressure. The resulting residue was purified by silica gel column chromatography (petroleum ether/ethyl acetate = 5:1), to give compound N'-[(2*E*)-1,1,1-trifluoropropan-2-ylidene]benzoyl hydrazide **355-2** (1.1 g, 12.65%).

LCMS: (ESI, m/z): 230.95 $[M+H]^+$.

**Step 2: Synthesis of compound 355-3**

Under nitrogen protection, *N'*-[(2*E*)-1,1,1-trifluoropropan-2-ylidene]benzoyl hydrazine (compound **355-2,** 1 g, 4.344 mmol, 1 eq) was added to a reaction flask and dissolved in tetrahydrofuran (10.0 mL). A 1 mol/L solution of borane tetrahydrofuran complex in tetrahydrofuran (20 mL, 21.720 mmol, 5 eq) was added at 0°C, and the mixture warmed to room temperature and stirred to react for 2 h. After the reaction was completed, the reaction mixture was quenched with methanol at 0°C, and the resulting residue was concentrated under reduced pressure to give compound N-(1,1,1-trifluoroprop-2-yl)benzoyl hydrazine **355-3** (700 mg, 60.44%).

LCMS: (ESI, m/z): 232.95 $[M+H]^+$.

**Step 3: Synthesis of compound 355-4**

Under nitrogen protection, N-(1,1,1-trifluoroprop-2-yl)benzoyl hydrazide (compound **355-3,** 1 g, 4.307 mmol, 1 eq) was added to a reaction flask and dissolved in methanol (6 mL). Then, concentrated hydrochloric acid (2.51 g, 68.912 mmol, 16 eq) was added and the mixture was warmed to 80°C and stirred to react overnight. After the reaction was completed, the reaction mixture was concentrated under reduced pressure to give compound (1,1,1-trifluoroprop-2-yl) hydrazine hydrochloride **355-4** (500 mg, 49.39%).

$^1$H NMR (400 MHz, DMSO-$d_6$, *ppm*) $\delta$ 9.61 (s, 2H), 5.95 (d, *J* = 21.0 Hz, 1H), 3.85 - 3.77 (m, 1H), 1.27 (d, *J* = 6.8 Hz, 3H).

**Step 4: Synthesis of compound 355-5**

Under nitrogen protection, (1,1,1-trifluoroprop-2-yl)hydrazine hydrochloride (compound **355-4,** 450 mg, 2.735 mmol, 1 eq), sodium acetate (672.98 mg, 8.205 mmol, 3 eq), and ethyl (2*E*)-2-cyano-3-ethoxyacrylate (693.95 mg, 4.103 mmol, 1.5 eq) were added to a reaction flask, dissolved in anhydrous ethanol (5 mL), warmed to 80°C and stirred to react for 4 h. After the reaction was completed, the reaction mixture was concentrated under reduced pressure. The resulting residue was purified by silica gel column chromatography (petroleum ether/ethyl acetate = 1:1) to give compound ethyl 5-amino-1-(1,1,1-trifluoroprop-2-yl)-1*H*-pyrazole-4-carboxylate **355-5** (300 mg, 42.32%).

LCMS: (ESI, m/z): 249.95 $[M+H]^+$.

**Step 5: Synthesis of compound 355-6**

Under nitrogen protection, ethyl 5-amino-1-(1,1,1-trifluoroprop-2-yl)-1*H*-pyrazole-4-carboxylate (compound **355-5,** 300 mg, 1.194 mmol, 1 eq) and sodium hydroxide (95.53 mg, 2.388 mmol, 2 eq) were added to a reaction flask, dissolved in anhydrous ethanol (6.0 mL) and water (3.0 mL), and stirred to react at room temperature for 2 days. After the reaction was completed, the reaction mixture was acidified to a pH of about 6 with hydrochloric acid solution. The aqueous phase was extracted with ethyl acetate (20 mL × 2), the organic phases were combined, backwashed with saturated sodium chloride solution (20 mL × 1), dried over anhydrous sodium sulfate and filtered. The filtrate was concentrated under reduced pressure to give compound 5-amino-1-(1,1,1-trifluoroprop-2-yl)-1*H*-pyrazole-4-carboxylic acid **355-5** (100 mg, 37.41%).

LCMS: (ESI, m/z): 223.95$[M+H]^+$.

**Step 6: Synthesis of compound 355**

Under nitrogen protection, 5-amino-1-(1,1,1-trifluoroprop-2-yl)-1*H*-pyrazole-4-carboxylic acid (compound **355-6,** 58.69 mg, 0.263 mmol, 1.2 eq), 2-(7-azabenzotriazole)-*N*,*N*,*N'*,*N'*-tetramethyluronium hexafluorophosphate (83.33 mg, 0.219 mmol, 1 eq), and *N*,*N*-diisopropylethylamine (141.62 mg, 1.095 mmol, 5 eq) were added to a reaction flask, dissolved in *N*,*N*-dimethylformamide (3 mL) and stirred at room temperature for 10 min. Then, 2-(3-aminoprop-1-yn-1-yl)-N-((3S,4R)-3-fluoro-1-methylpiperidin-4-yl)-3-(2,2,2-trifluoroethyl)pyrazolo[1,5-a]pyridin-7-amine dihydrochloride (compound **001-12,** 100 mg, 0.219 mmol, 1.00 eq) was added, and the system was stirred to react at room temperature for 1 h. After the reaction was completed, water (10 mL) was added, and the reaction mixture was extracted with ethyl acetate (20 mL × 2). The organic phases were combined, backwashed with saturated sodium chloride solution (30 mL × 1), dried over anhydrous sodium sulfate, filtered, and the filtrate was concentrated under reduced pressure. The crude

product was purified by HPLC with the following conditions (column specifications: YMC Triart C18 ExRs 5 μm, 30 mm * 150 mm; mobile phase A: water (10 mmol/L ammonium bicarbonate), mobile phase B: acetonitrile; flow rate: 60 mL/min; elution gradient: 33% B to 67% B in 8 min; detection wavelength: UV 254 nm/220 nm; retention time (min): 7.65), to give compound 5-amino-*N*-[3-(7-{[(3S,4R)-3-fluoro-1-methylpiperidin-4-yl]amino}-3-(2,2,2-trifluoroethyl)pyrazolo[1,5-*a*]pyridin-2-yl)prop-2-yn-1-yl]-1-(1,1,1-trifluoroprop-2-yl)-1*H*-pyrazole-4-carboxamide **355** (30 mg, 23.12%), which was further subjected to chiral resolution with the following conditions (column specifications: CHIRALPAK-IA 2*25cm, 5m; mobile phase A: n-hexane (0.1% diethylamine), mobile phase B: ethanol: dichloromethane = 1:1; flow rate: 20 mL/min; elution gradient: isocratic 30; detection wavelength: UV 254/220 nm; front peak retention time (min): 7.745; rear peak retention time (min): 10.302; sample dissolving solvent: methanol; single injection volume: 1.0 mL; number of injections: 3), to give compounds **355A** (front peak) and **355B** (rear peak).

Front peak **355A:** LCMS:(ESI, m/z): 589.10 $[M+H]^+$.

$^1H$ NMR (400 MHz, DMSO-$d_6$, *ppm*) δ 8.38 (t, *J* = 5.6 Hz, 1H), 7.82 (s, 1H), 7.28 (dd, *J* = 8.8, 7.6 Hz, 1H), 7.02 (d, *J* = 8.7 Hz, 1H), 6.60 (s, 2H), 6.31 (d, J = 7.5 Hz, 1H), 6.25 (d, *J* = 9.0 Hz, 1H), 5.22 (p, *J* = 7.1 Hz, 1H), 4.88 (d, *J* = 49.4 Hz, 1H), 4.30 (d, *J* = 5.6 Hz, 2H), 3.94 - 3.80 (m, 1H), 3.75 (q, *J* = 11.1 Hz, 2H), 3.14 - 3.00 (m, 1H), 2.80 (d, *J* = 11.2 Hz, 1H), 2.40 - 2.30 (m, 1H), 2.22 (s, 3H), 2.18 - 2.10 (m, 1H), 1.99 - 1.83 (m, 2H), 1.58 (d, *J* = 6.8 Hz, 3H).

Rear peak **355B:** LCMS:(ESI, m/z): 589.05 $[M+H]^+$.

$^1H$ NMR (400 MHz, DMSO-$d_6$, *ppm*) δ 8.38 (t, *J* = 5.6 Hz, 1H), 7.82 (s, 1H), 7.28 (dd, *J* = 8.8, 7.6 Hz, 1H), 7.02 (d, *J* = 8.7 Hz, 1H), 6.60 (s, 2H), 6.31 (d, J = 7.4 Hz, 1H), 6.25 (d, *J* = 9.0 Hz, 1H), 5.22 (p, *J* = 7.0 Hz, 1H), 4.88 (d, *J* = 49.5 Hz, 1H), 4.30 (d, *J* = 5.6 Hz, 2H), 3.88 - 3.71 (m, 3H), 3.15 - 2.98 (m, 1H), 2.80 (d, *J* = 11.3 Hz, 1H), 2.40 - 2.32 (m, 1H), 2.22 (s, 3H), 2.18 - 2.10 (m, 1H), 1.98 - 1.83 (m, 2H), 1.58 (d, *J* = 6.8 Hz, 3H).

**Example 143**

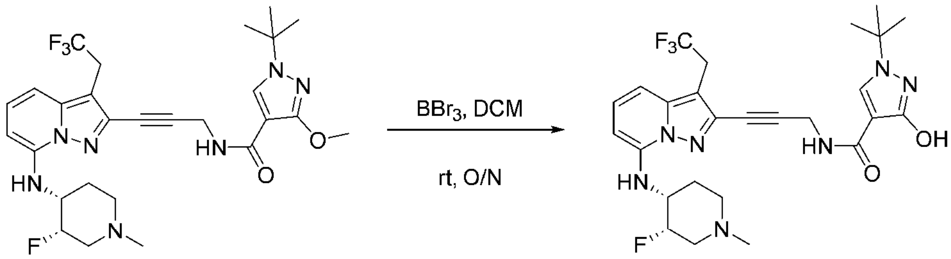


043 356

**Step 1: Synthesis of 1-(tert-butyl)-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl)-3-hydroxy-1*H*-pyrazole-4-carboxamide (compound 356)**

Under nitrogen protection, 1-(tert-butyl)-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl)-3-methoxy-1*H*-pyrazole-4-carboxamide (compound **043,** 80 mg, 0.142 mmol, 1 eq) was added to a react flask and dissolved in dichloromethane (5 mL). At 0°C, 1 mol/L boron tribromide in dichloromethane (0.5 mL, 5.289 mmol, 37.26 eq) was added dropwise, and the system was warmed to room temperature and stirred to react overnight. After the reaction was completed, the reaction mixture was quenched with methanol (20 mL) at 0°C. The resulting residue was concentrated under reduced pressure. The crude product was purified by HPLC with the following conditions (column specifications: Sunfire C18 5 m, 30 mm * 150 mm; mobile phase A: water (0.1% formic acid), mobile phase B: acetonitrile; flow rate: 60 mL/min; elution gradient: 12% B to 38% B in 8 min; detection wavelength: UV 254 nm/220 nm; retention time (min): 6.37), to give compound **1-(tert-butyl)-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-3-hydroxy-1*H*-pyrazole-4-carboxamide 356** (56.2 mg, 72.04%).

LCMS: (ESI, m/z): 550.00 $[M+H]^+$.

$^1H$ NMR (400 MHz, DMSO-$d_6$, *ppm*) δ 10.95 (s, 1H), 7.98 (s, 1H), 7.84 (s, 1H), 7.30 (dd, *J* = 8.7, 7.6 Hz, 1H), 7.05 (d, *J* = 8.7 Hz, 1H), 6.55 (s, 1H), 6.31 (d, J = 7.5 Hz, 1H), 5.07 (d, *J* = 48.1 Hz, 1H), 4.35 (d, *J* = 5.7 Hz, 2H), 4.08 - 3.85 (m, 1H), 3.75 (q, *J* = 11.1 Hz, 2H), 3.63 - 3.43 (m, 1H), 3.24 - 3.04 (m, 1H), 2.65 - 2.53(m, 5H), 2.23 - 2.07 (m, 1H), 2.06 - 1.91(m, 1H), 1.47 (s, 9H).

**Example 144**

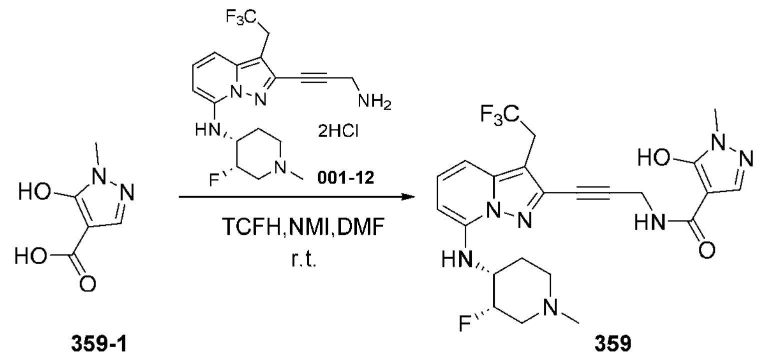


**359-1** **359**

**Step 1: Synthesis of *N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-5-hydroxy-1-methyl-1*H*-pyrazole-4-carboxamide (compound 359)**

Under nitrogen protection, at room temperature, a solution of 5-hydroxy-1-methyl-1*H*-pyrazole-4-carboxylic acid (compound **359-1,** 80.97 mg, 0.570 mmol, 2.00 eq), *N,N,N',N'*-tetramethylchloroformamidinium hexafluorophosphate (319.74 mg, 1.140 mmol, 4.00 eq), *N*-methylimidazole (233.92 mg, 2.850 mmol, 10.00 eq) and 2-(3-aminoprop-1-yn-1-yl)-N-((3S,4R)-3-fluoro-1-methylpiperidin-4-yl)-3-(2,2,2-trifluoroethyl)pyrazolo[1,5-a]pyridin-7-amine dihydrochloride (compound **001-12,** 130 mg, 0.285 mmol, 1.00 eq) in *N,N*-dimethylformamide (13.0 mL) was stirred to react for 2 h. After the reaction was completed, the reaction mixture was quenched with water, and extracted with ethyl acetate (2 × 10 mL). The organic phases were combined, backwashed with saturated sodium chloride solution (2 × 10 mL), dried over anhydrous sodium sulfate, filtered, and the filtrate was concentrated under reduced pressure. The resulting residue was purified by reverse phase column chromatography with the following conditions (C18 column, mobile phase: water (sodium bicarbonate 10 mmol/L) and acetonitrile, 15% to 50% gradient in 20 min, detection wavelength: UV 254 nm), to give the carboxylate of compound *N*-**[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-5-hydroxy-1-methyl-1*H*-pyrazole-4-carboxamide 359** (3.39 mg, 2.13%).

LCMS: (ESI, m/z): 508.05 $[M+H]^+$.

**Example 145**

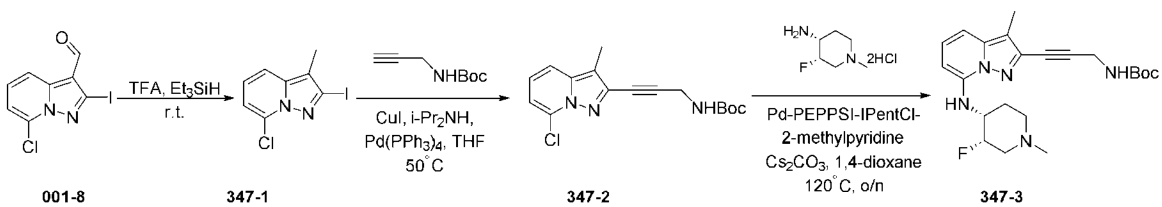


**001-8** **347-1** **347-2** **347-3**

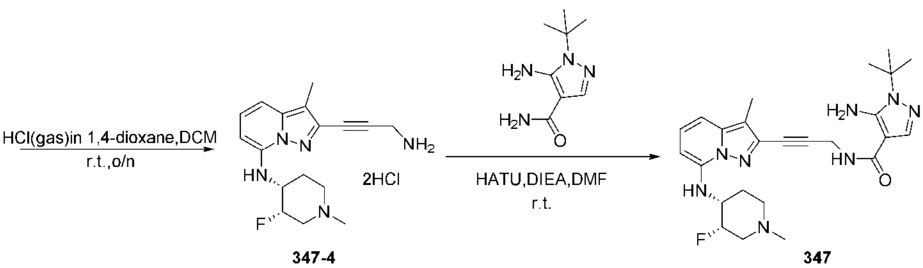


**347-4** **347**

**Step 1: Synthesis of compound 347-1**

At room temperature, 7-chloro-2-iodopyrazolo[1,5-*a*]pyridine-3-carboxaldehyde (compound **001-8,** 2 g, 6.525 mmol, 1 eq), triethylsilane (20.79 mL, 130.500 mmol, 20 eq) and trifluoroacetic acid (96.94 mL) were added to a reaction flask and stirred at room temperature for 4 h. After the reaction was completed, the reaction mixture was poured into 1 mol/L sodium hydroxide solution to adjust the pH to neutral. The aqueous phase was extracted with ethyl acetate (50 mL × 3), and the organic phases were combined, washed with sodium bicarbonate solution (50 mL × 3), washed with saturated brine (50 mL × 3), dried over anhydrous sodium sulfate, filtered, and the filtrate was concentrated under reduced pressure to give compound 7-chloro-2-iodo-3-methylpyrazolo[1,5-*a*]pyridine **347-1** (1 g, 52.39%).
LCMS: (ESI, m/z): 292.85 $[M+H]^+$.

**Step 2: Synthesis of compound 347-2**

Under nitrogen protection, at room temperature, 7-chloro-2-iodo-3-methylpyrazolo[1,5-*a*]pyridine (compound **347-1,** 900 mg, 3.077 mmol, 1 eq), tert-butyl *N*-(propyl-2-ynyl-1-yl)carbamate (573.04 mg, 3.692 mmol, 1.2 eq), tetrakis(triphenylphosphine)palladium (711.11 mg, 0.615 mmol, 0.2 eq), and cuprous iodide (58.6 mg, 0.308 mmol, 0.1 eq) were added to a reaction flask, dissolved in tetrahydrofuran (30 mL), and then *N*,*N*-diisopropylethylamine (4.01 g, 12.308 mmol, 4 eq) was added. The mixture was warmed to 50°C, and stirred for 2 h. The reaction mixture was concentrated under reduced pressure, and the resulting residue was purified by silica gel column chromatography (petroleum ether/ethyl acetate = 10:1) to give compound tert-butyl *N*-(3-{7-chloro-3-methylpyrazolo[1,5-*a*]pyridin-2-yl} prop-2-yn-1-yl)carbamate **347-2** (860 mg, 87.40%).
LCMS: (ESI, m/z): 319.95 $[M+H]^+$.

**Step 3: Synthesis of compound 347-3**

Under nitrogen protection, at room temperature, a solution of tert-butyl *N*-(3-{7-chloro-3-methylpyrazolo[1,5-*a*] pyridin-2-yl}prop-2-ynyl-1-yl)carbamate (compound **347-2,** 649.86 mg, 1.564 mmol, 1.0 eq), 3-fluoro-1-methylpiperidin-4-amine dihydrochloride (384.82 mg, 1.877 mmol, 1.2 eq), (SP-4-1)-[1,3-bis[2,6-bis(1-ethylpropyl)phenyl]-4,5-dichloro-1,3-dihydro-2*H*-imidazol-2-ylidene]dichloro(2-methylpyridine)palladium (131.52 mg, 0.156 mmol, 0.1 eq) and cesium carbonate (2037.71 mg, 6.256 mmol, 4 eq) in 1,4-dioxane (20 mL) was added to a reaction flask, warmed to 120°C, and stirred overnight. After the reaction was completed, the reaction mixture was filtered, the filter cake was washed with ethanol (3 × 10 mL), the filtrate was concentrated under reduced pressure, and the resulting residue was purified by silica gel column chromatography (ethyl acetate), to give compound tert-butyl *N*-[3-(7-[(3*S*,4*R*)-3-fluoro-1-methylpiperidin-4-yl]amino)-3-methylpyrazolo[1,5-*a*]pyridin-2-yl)prop-2-yn-1-yl]carbamate **347-3** (360 mg, 55.41%).
LCMS: (ESI, m/z): 415.90 $[M+H]^+$.

**Step 4: Synthesis of compound 347-4**

At room temperature, a solution of tert-butyl *N*-[3-(7-[(3*S*,4*R*)-3-fluoro-1-methylpiperidin-4-yl]amino}-3-methylpyrazolo[1,5-*a*]pyridin-2-yl)prop-2-yn-1-yl]carbamate (compound **347-3,** 200 mg, 0.481 mmol, 1 eq) in dichloromethane (4.0 mL) and a solution of hydrogen chloride in 1,4-dioxane (4M, 0.8 mL) were added to a reaction flask. The mixture was stirred overnight at room temperature. The reaction mixture was concentrated under reduced pressure to give compound 2-(3-aminoprop-1-yn-1-yl)-*N*-((3*S*,4*R*)-3-fluoro-1-methylpiperidin-4-yl)-3-methylpyrazolo[1,5-*a*]pyridin-7-amine dihydrochloride **347-4** (160 mg, 85.60%).
LCMS: (ESI, m/z): 316.00 $[M+H]^+$.

**Step 5: Synthesis of 5-amino-1-tert-butyl-*N*-[3-(7-{[(3*S*,4*R*)-3-fluoro-1-methylpiperidin-4-yl]amino}-3-methylpyrazolo[1,5-*a*]pyridin-2-yl)prop-2-yn-1-yl]-1*H*-pyrazole-4-carboxamide (compound 347)**

At room temperature, a solution of 5-amino-1-tert-butylpyrazole-4-carboxylic acid (compound **347-4,** 28.31 mg, 0.155 mmol, 1.2 eq), *N*,*N*-diisopropylethylamine (66.57 mg, 0.516 mmol, 4 eq) and O-(7-azabenzotriazol-1-yl)-*N,N,N',N'*-tetramethyluronium hexafluorophosphate (48.96 mg, 0.129 mmol, 1 eq) in *N*,*N*-dimethylformamide (2 mL) was added to a reaction flask. The mixture was stirred at room temperature for 10 min. At room temperature, 2-(3-aminoprop-1-yn-1-yl)-N-((3S,4R)-3-fluoro-1-methylpiperidin-4-yl)-3-(2,2,2-trifluoroethyl)pyrazolo[1,5-a]pyridin-7-amine dihydrochloride (compound **001-12,** 50 mg, 0.129 mmol, 1 eq) was added to the above system. The system was stirred at room temperature for 1 h. After the reaction was completed, the reaction mixture was diluted with ethyl acetate (5 mL). The mixture was washed with saturated sodium chloride solution (5 mL x 3), dried over anhydrous sodium sulfate, filtered, and the filtrate was concentrated under reduced pressure. The resulting residue was purified by HPLC with the following

conditions (column specifications: Xselect CSHTM Prep C18 5μm 30*150mm OBD; mobile phase A: water (0.1% formic acid), mobile phase B: acetonitrile; flow rate: 60 mL/min; elution gradient: 10% B to 27% B in 10 min; detection wavelength: UV 254 nm/220 nm; retention time (min): 9.27) to give the carboxylate of compound **5-amino-1-tert-butyl-*N*-[3-(7-{[(3*S*,4*R*)-3-fluoro-1-methylpiperidin-4-yl]amino}-3-methylpyrazolo[1,5-*a*]pyridin-2-yl)prop-2-yn-1-yl]-1*H*-pyrazole-4-carboxamide 347** (19.97 mg, 28.37%).

LCMS: (ESI, m/z): 481.55 $[M+H]^+$.

$^1H$ NMR (400 MHz, DMSO-$d_6$, *ppm*) $\delta$ 8.30 (t, *J* = 5.6 Hz, 1H), 7.66 (s, 1H), 7.16 (t, *J* = 8.1 Hz, 1H), 6.92 (d, *J* = 8.7 Hz, 1H), 6.25 - 6.05 (m, 4H), 4.90 (d, *J* = 49.6 Hz, 1H), 4.29 (d, *J* = 5.6 Hz, 2H), 3.95 - 3.74 (m, 1H), 3.30 (s, 3H), 3.11 (s, 1H), 2.83 (s, 1H), 2.31 - 2.13 (m, 5H), 2.01 - 1.79 (s, 2H), 1.52 (s, 9H).

**Example 146**

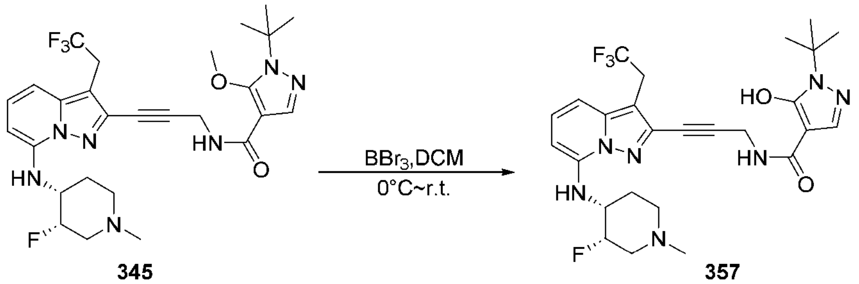


345 357

**Step 5: Synthesis of 1-(tert-butyl)-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-5-hydroxy-1*H*-pyrazole-4-carboxamide (compound 357)**

Under nitrogen protection, 1-(tert-butyl)-N-[3-(7-{[(3S,4R)-3-fluoro-1-methylpiperidin-4-yl]amino}-3-(2,2,2-trifluoroethyl)pyrazolo[1,5-a]pyridin-2-yl)prop-2-yn-1-yl]-5-methoxy-1*H*-pyrazole-4-carboxamide **345** (50 mg, 0.089 mmol, 1 eq) and dichloromethane (2.5 mL) were added to a react flask, boron tribromide (250 uL) was added dropwise at 0°C. After the addition was completed, the system was further stirred at room temperature for 2 h. After the reaction was completed, the reaction mixture was quenched with methanol at 0°C. The resulting residue was concentrated under reduced pressure. The crude product was purified by HPLC with the following conditions (column specifications: Sunfire C18 5 μm, 30 mm * 150 mm; mobile phase A: water (0.1% formic acid), mobile phase B: acetonitrile; flow rate: 60 mL/min; elution gradient: 15% B to 35% B in 8 min; detection wavelength: UV 254 nm/220 nm; retention time (min): 6.47), to give compound **1-(tert-butyl)-*N*-[3-(7-{[(3*S*,4*R*)-3-fluoro-1-methylpiperidin-4-yl]amino}-3-(2,2,2-trifluoroethyl)pyrazolo[1,5-*a*]pyridin-2-yl)prop-2-yn-1-yl]-5-hydroxy-1*H*-pyrazole-4-carboxamide** 357 **(13.7 mg, 27.99%).**

LCMS: (ESI, m/z): 550.10 $[M+H]^+$.

$^1H$ NMR (400 MHz, DMSO-$d_6$, *ppm*) $\delta$ 8.93 (t, J = 5.5 Hz, 1H), 7.62 (s, 1H), 7.29 (dd, *J* = 8.7, 7.6 Hz, 1H), 7.03 (d, *J* = 8.6 Hz, 1H), 6.41 (s, 1H), 6.31 (d, *J* = 7.6 Hz, 1H), 4.96 (d, *J* = 48.9 Hz, 1H), 4.34 (d, *J* = 5.6 Hz, 2H), 3.99 - 3.82 (m, 1H), 3.75 (q, *J* = 11.1 Hz, 2H), 3.25 - 3.11 (m, 1H) 3.05 - 2.89 (m, 1H), 2.45 - 2.25 (m, 5H), 2.17 - 1.98 (m, 1H), 1.94 - 1.84 (m, 1H), 1.50 (s, 9H).

**Example 147**

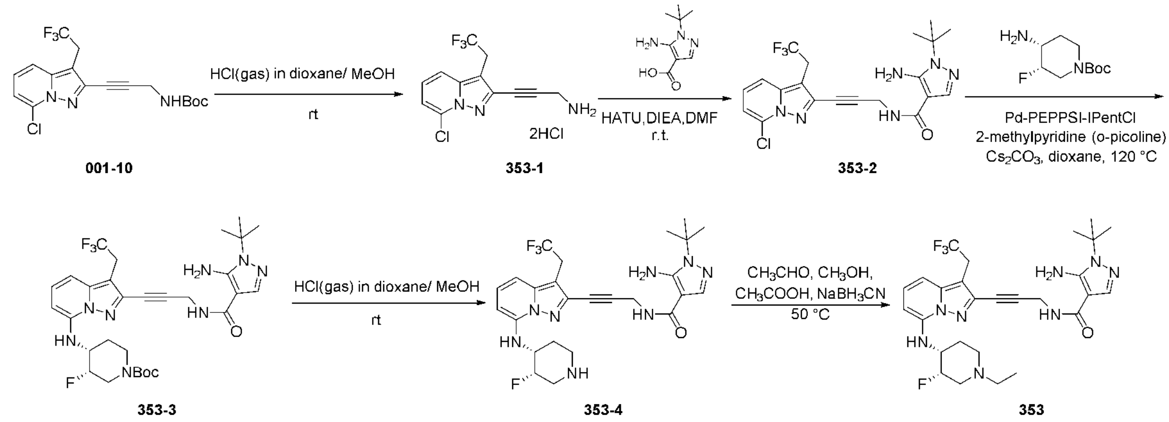


001-10 353-1 353-2

353-3 353-4 353

**Step 1: Synthesis of compound 353-1**

At room temperature, a solution of tert-butyl {3-[7-fluoro-3-(2,2,2-trifluoroethyl)pyrazolo[1,5-a]pyridin-2-yl] prop-2-yn-1-yl}carbamate (compound **001-10,** 1 g, 2.579 mmol, 1 eq) and 4 mol/L hydrogen chloride in 1,4-dioxane (3 mL) in dichloromethane (3 mL) was stirred to react for 2 h. After the reaction was completed, the mixture was concentrated in vacuum to give compound 3-[7-chloro-3-(2,2,2-trifluoroethyl)pyrazolo[1,5-*a*]pyridin-2-yl]prop-2-yn-1-amine dihydrochloride **353-1** (0.7 g, 94.36%).

LCMS: (ESI, m/z): 287.90 $[M+H]^+$.

**Step 2: Synthesis of compound 353-2**

At room temperature, a solution of 5-amino-1-tert-butyl-1*H*-pyrazole-4-carboxylic acid (477.66 mg, 2.607 mmol, 1 eq), O-(7-aza-benzotriazol-1-yl)-*N*,*N*,*N'*,*N'*-tetramethyluronium hexafluorophosphate (991.33 mg, 2.607 mmol, 1 eq), and *N*,*N*-diisopropylethylamine (1684.83 mg, 13.035 mmol, 5 eq) in *N*,*N*-dimethylformamide (7 mL) was stirred to react for 15 min. Then, 3-[7-chloro-3-(2,2,2-trifluoroethyl)pyrazolo[1,5-*a*]pyridin-2-yl]prop-2-yn-1-amine dihydrochloride (compound **353-1,** 750 mg, 2.607 mmol, 1 eq) was added and the mixture was further stirred to react for 1 h. After the reaction was completed, the reaction mixture was quenched with water at room temperature, and extracted with ethyl acetate (3 × 10 mL). The organic phases were combined, backwashed with saturated sodium chloride solution (3 × 10 mL), dried over anhydrous sodium sulfate, filtered, and the filtrate was concentrated under reduced pressure. The resulting residue was purified by silica gel column chromatography (dichloromethane/methanol = 10:1) to give compound 5-amino-1-tert-butyl-*N*-{3-[7-chloro-3-(2,2,2-trifluoroethyl)pyrazolo[1,5-*a*]pyridin-2-yl]prop-2-yn-1-yl}-1*H*-pyrazole-4-carboxamide **353-2** (700 mg, 59.29%).

LCMS: (ESI, m/z): 452.90 $[M+H]^+$.

**Step 3: Synthesis of compound 353-3**

Under nitrogen protection, at 120°C, a solution of 5-amino-1-tert-butyl-*N*-{3-[7-chloro-3-(2,2,2-trifluoroethyl) pyrazolo[1,5-*a*]pyridin-2-yl]prop-2-yn-1-yl}-1*H*-pyrazole-4-carboxamide (compound **353-2,** 100 mg, 0.221 mmol, 1 eq), tert-butyl (3S,4R)-4-amino-3-fluoropiperidine-1-carboxylate (48.2 mg, 0.221 mmol, 1 eq), (SP-4-1)-[1,3-bis[2,6-bis(1-ethylpropyl)phenyl]-4,5-dichloro-1,3-dihydro-2*H*-imidazol-2-ylidene]dichloro(2-methylpyridine)palladium (18.29 mg, 0.022 mmol, 0.1 eq) and cesium carbonate (287.94 mg, 0.884 mmol, 4 eq) in a 1,4-dioxane (3.0 mL) were stirred to react for 1.5 h. After the reaction was completed, the reaction mixture was filtered, and the filtrate was concentrated in vacuum. The resulting residue was purified by silica gel column chromatography (dichloromethane/methanol = 10:1), to give compound tert-butyl (3*S*,4*R*)-4-[(2-{3-[(5-amino-1-tert-butyl-1*H*-pyrazole-4-carboxamide)propyl-1-yn-1-yl]-3-(2,2,2-trifluoroethyl)pyrazolo[1,5-*a*]pyridin-7-amino]-3-fluoropiperidine-1-carboxylate **353-3** (100 mg, 71.35%).

LCMS: (ESI, m/z): 634.95 $[M+H]^+$.

**Step 4: Synthesis of compound 353-4**

At room temperature, compound tert-butyl (3*S*,4*R*)-4-[(2-{3-[(5-amino-1-tert-butyl-1*H*-pyrazole-4-carboxamide) propyl-1-yn-1-yl}-3-(2,2,2-trifluoroethyl)pyrazolo[1,5-*a*]pyridine-7-amino]-3-fluoropiperidine-1-carboxylate (compound **353-3,** 100 mg, 0.158 mmol, 1 eq) was dissolved in dichloromethane (4 mL), then 4 mol/L hydrogen chloride in 1,4-dioxane (2 mL) was added. The mixture was stirred to react for 2 h. After the reaction was completed, the resulting residue

was concentrated under reduced pressure to give compound 5-amino-1-tert-butyl-*N*-[3-(7-{[(3*S*,4*R*)-3-fluoropiperidin-4-yl]amino}-3-(2,2,2-trifluoroethyl)pyrazolo[1,5-*a*]pyridin-2-yl)prop-2-yn-1-yl]-1*H*-pyrazole-4-carboxamide **353-4** (80 mg, 94.98%), which was not further purified.

LCMS: (ESI, m/z): 535.10 $[M+H]^+$.

**Step 5: Synthesis of 5-amino-1-tert-butyl-*N*-[3-(7-{[(3*S*,4*R*)-1-ethyl-3-fluoropiperidin-4-yl]amino}-3-(2,2,2-trifluoroethyl)pyrazolo[1,5-*a*]pyridin-2-yl)prop-2-yn-1-yl]-1*H*-pyrazole-4-carboxamide (compound 353)**

At 50°C, a solution of 5-amino-1-tert-butyl-*N*-[3-(7-{[(3*S*,4*R*)-3-fluoropiperidin-4-yl]amino}-3-(2,2,2-trifluoroethyl)pyrazolo[1,5-*a*]pyridin-2-yl)prop-2-yn-1-yl]-1*H*-pyrazole-4-carboxamide (compound **353-4,** 50 mg, 0.094 mmol, 1 eq), acetaldehyde (4.12 mg, 0.094 mmol, 1 eq), acetic acid (0.56 mg, 0.009 mmol, 0.1 eq) and sodium cyanoborohydride (11.76 mg, 0.188 mmol, 2 eq) in methanol (3 mL) was stirred to react for 1 h. After the reaction was completed, the reaction mixture was quenched with water at room temperature, and extracted with ethyl acetate (3 × 10 mL). The organic phases were combined, backwashed with saturated sodium chloride solution (3 × 10 mL), dried over anhydrous sodium sulfate, filtered, and the filtrate was concentrated under reduced pressure. The crude product was purified by HPLC with the following conditions (column specifications: Sunfire C18 5 μm, 30 mm × 150 mm; mobile phase A: water (0.1% formic acid), mobile phase B: acetonitrile; flow rate: 60 mL/min; elution gradient: 15% to 41% in 9 min; detection wavelength: UV 254 nm/220 nm; retention time (min): 8.23), to give the carboxylate of compound **5-amino-1-tert-butyl-*N*-[3-(7-{[(3*S*,4*R*)-1-ethyl-3-fluoropiperidin-4-yl]amino}-3-(2,2,2-trifluoroethyl)pyrazolo[1,5-*a*]pyridin-2-yl)prop-2-yn-1-yl]-1*H*-pyrazole-4-carboxamide 353** (21.14 mg, 37.02%).

LCMS: (ESI, m/z): 563.10 $[M+H]^+$.

**Example 148**

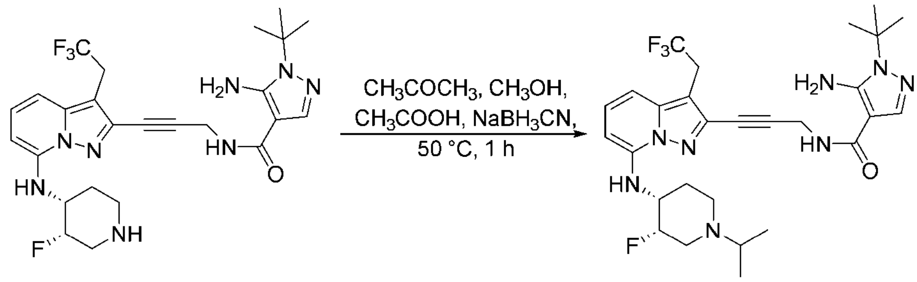


**353-4** **354**

**Step 5: Synthesis of 5-amino-1-tert-butyl-*N*-[3-(7-{[(3*S*,4*R*)-1-ethyl-3-fluoropiperidin-4-yllamino}-3-(2,2,2-trifluoroethyl)pyrazolo[1,5-*a*]pyridin-2-yl)prop-2-yn-1-yl]-1*H*-pyrazole-4-carboxamide (compound 354)**

At 50°C, a solution of 5-amino-1-tert-butyl-*N*-[3-(7-{[(3*S*,4*R*)-3-fluoropiperidin-4-yl]amino}-3-(2,2,2-trifluoroethyl)pyrazolo[1,5-*a*]pyridin-2-yl)prop-2-yn-1-yl]-1*H*-pyrazole-4-carboxamide (compound **353-4,** 50 mg, 0.094 mmol, 1 eq), acetone (5.43 mg, 0.094 mmol, 1 eq), acetic acid (0.56 mg, 0.009 mmol, 0.1 eq) and sodium cyanoborohydride (19.21 mg, 0.300 mmol, 2 eq) in methanol (5 mL) was stirred to react for 1 h. After the reaction was completed, the reaction mixture was quenched with water at room temperature, and extracted with ethyl acetate (3 × 10 mL). The organic phases were combined, backwashed with saturated sodium chloride solution (3 × 10 mL), dried over anhydrous sodium sulfate, filtered, and the filtrate was concentrated under reduced pressure. The crude product was purified by HPLC with the following conditions (column specifications: XBridge BEH C18 OBD Prep Column 130, 5 μm, 30 mm × 150 mm; mobile phase A: water (17mmol/L ammonium bicarbonate), mobile phase B: acetonitrile; flow rate: 60 mL/min; elution gradient: 43% to 56% in 10 min; detection wavelength: UV 254 nm/220 nm; retention time (min): 8.42), to give compound **5-amino-1-tert-butyl-*N*-[3-(7-{[(3*S*,4*R*)-1-ethyl-3-fluoropiperidin-4-yl]amino}-3-(2,2,2-trifluoroethyl)pyrazolo[1,5-*a*]pyridin-2-yl)prop-2-yn-1-yl]-1*H*-pyrazole-4-carboxamide 354** (25.18 mg, 46.40%).

LCMS: (ESI, m/z): 577.10 $[M+H]^+$.

$^1$H NMR (400 MHz, DMSO-$d_6$, *ppm*) δ 8.30 (t, J = 5.6 Hz, 1H), 7.65 (s, 1H), 7.28 (dd, J = 8.8, 7.6 Hz, 1H), 7.02 (d, J = 8.6 Hz, 1H), 6.31 (d, J = 7.5 Hz, 1H), 6.23 (d, J = 9.0 Hz, 1H), 6.18 (s, 2H), 4.88 (d, J = 49.6 Hz, 1H), 4.29 (d, J = 5.6 Hz, 2H), 3.94 - 3.81 (m, 1H), 3.75 (q, J = 10.8 Hz, 2H), 3.06 (t, J = 11.4 Hz, 1H), 2.85 - 2.68 (m, 2H), 2.59 - 2.42 (m, 1H), 2.40 - 2.26 (m, 1H), 1.92 - 1.79 (m, 2H), 1.52 (s, 9H), 0.99 (d, J = 2.9 Hz, 3H), 0.97 (d, J = 3.0 Hz, 3H).

**Example 149**

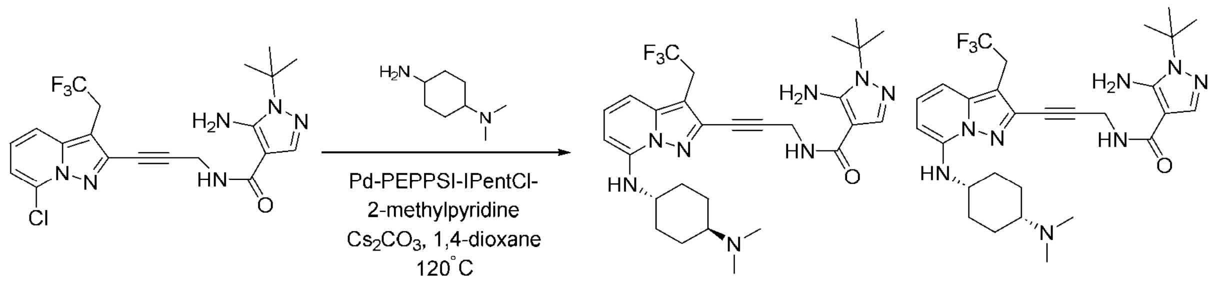


**353-2**

**Step 5: Synthesis of compounds 374A and 374B**

Under nitrogen protection, at room temperature, 5-amino-1-tert-butyl-*N*-{3-[7-chloro-3-(2,2,2-trifluoroethyl)pyrazolo[1,5-*a*]pyridin-2-yl]prop-2-ynyl-1-yl}-1*H*-pyrazole-4-carboxamide (compound **353-2,** 50 mg, 0.110 mmol, 1 eq), *N*$^1$, *N*$^1$-dimethylcyclohexane-1,4-diamine (18.85 mg, 0.132 mmol, 1.2 eq), (SP-4-1)-[1,3-bis[2,6-bis(1-ethylpropyl)phenyl]-4,5-dichloro-1,3-dihydro-2*H*-imidazol-2-ylidene]dichloro(2-methylpyridine)palladium (9.29 mg, 0.011 mmol, 0.1 eq) and cesium carbonate (143.89 mg, 0.440 mmol, 4 eq) were added to a reaction flask, dissolved in 1,4-dioxane solution (5 mL), warmed to 120°C and stirred for 2 h. After the reaction was completed, the reaction mixture was filtered, the filter cake was washed with ethanol (3 × 10 mL), the filtrate was concentrated under reduced pressure, and the crude product was purified by HPLC with the following conditions (column specifications: Xselect CSHTM Prep C18 5μm 30*150mm OBD; mobile phase A: water (0.1% formic acid), mobile phase B: acetonitrile; flow rate: 60 mL/min; elution gradient: 15% B to 33% B in 10 min; detection wavelength: UV 254 nm/220 nm; front peak retention time RT1 (min): 8.3, rear peak retention time RT2 (min): 8.78), to give the carboxylate of compound **374A** (front peak, 4.75 mg, 7.09%) and the carboxylate of compound **374B** (rear peak, 7.35 mg, 10.70%).

**Front peak compound 374A**

LCMS: (ESI, m/z): 559.60 $[M+H]^+$.
$^1H$ NMR (400 MHz, DMSO-$d_6$, *ppm*) $\delta$ 8.31 (t, *J* = 5.7 Hz, 1H), 7.65 (s, 1H), 7.25 (t, *J* = 8.2 Hz, 1H), 6.93 (d, *J* = 8.6 Hz, 1H), 6.63 (d, *J* = 8.7 Hz, 1H), 6.18 (s, 2H), 6.15 (d, J = 7.7 Hz, 1H), 4.27 (d, *J* = 5.7 Hz, 2H), 3.73 (q, *J* = 11.1 Hz, 2H), 3.55 - 3.40 (m, 2H), 2.43 (s, 6H), 2.05 (d, *J* = 10.9 Hz, 2H), 1.93 (d, *J* = 10.6 Hz, 2H), 1.59 - 1.38 (m, 13H).

**Rear peak compound 374B**

LCMS: (ESI, m/z): 559.60 $[M+H]^+$.
$^1H$ NMR (400 MHz, DMSO-$d_6$, *ppm*) $\delta$ 8.30 (t, *J* = 5.7 Hz, 1H), 7.65 (s, 1H), 7.37 - 7.17 (m, 1H), 6.96 (d, *J* = 8.7 Hz, 1H), 6.27 (d, *J* = 7.5 Hz, 1H), 6.17 (s, 2H), 6.14 (d, J = 7.5 Hz, 1H), 4.28 (d, *J* = 5.7 Hz, 2H), 3.81 - 3.65 (m, 3H), 2.20 (s, 6H), 2.14 (s, 1H), 1.94 - 1.81 (m, 2H), 1.73 - 1.55 (m, 6H), 1.52 (s, 9H).

**Example 150**

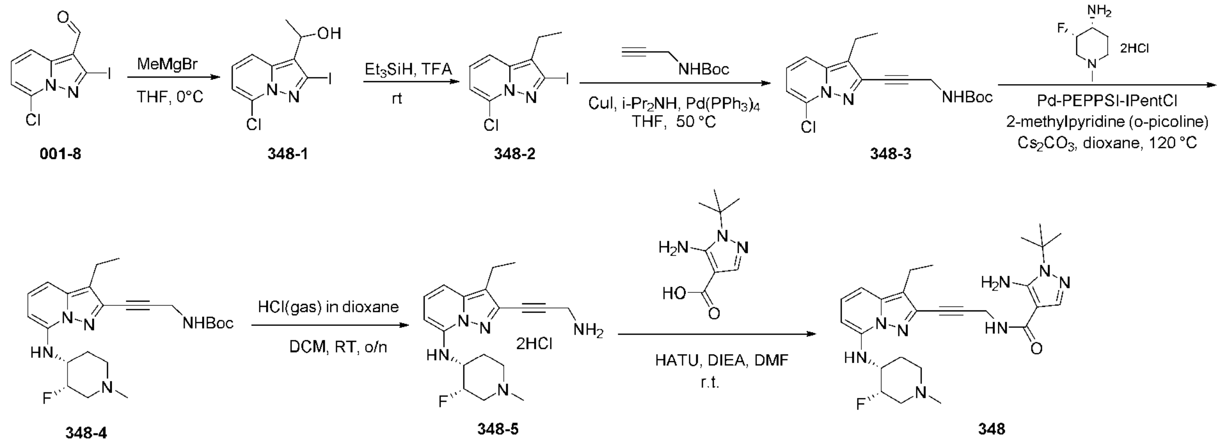


001-8 348-1 348-2 348-3 348-4 348-5 348

### Step 1: Synthesis of compound 348-1

7-chloro-2-iodopyrazolo[1,5-*a*]pyridine-3-carboxaldehyde (compound **001-8,** 3 g, 9.788 mmol, 1 eq) was added to a reaction flask and dissolved in tetrahydrofuran (15 mL). Methyl magnesium bromide (2.92 g, 24.470 mmol, 2.5 eq) was added dropwise at 0°C, and the reaction was carried out at 0°C for 1 h. After the reaction was completed, saturated ammonium chloride was added to quench the reaction, and the reaction mixture was extracted with ethyl acetate (3 × 20 mL). The organic phases were combined, backwashed with saturated sodium chloride solution (1 × 20 mL), dried over anhydrous sodium sulfate, and filtered. The filtrate was concentrated under reduced pressure, and the resulting residue was purified by silica gel column chromatography (petroleum ether/ethyl acetate = 5:1) to give compound 1-(7-chloro-2-iodopyrazolo[1,5-*a*]pyridin-3-yl)ethan-1-1-ol **348-1** (700 mg, 21.06%).

LCMS: (ESI, m/z): 322.75 $[M+H]^+$.

### Step 2: Synthesis of compound 348-2

1-(7-chloro-2-iodopyrazolo[1,5-*a*]pyridin-3-yl)ethan-1-1-ol (compound **348-1,** 735 mg, 2.279 mmol, 1 eq) and triethylsilane (1.59 g, 13.674 mmol, 6 eq) were added to a reaction flask, dissolved in trifluoroacetic acid (8.4 mL), and allowed to react at room temperature for 1 h. After the reaction was completed, the solvent was directly removed by spin drying, and the resulting residue was purified by silica gel column chromatography (petroleum ether/ethyl acetate = 10:1) to give compound 7-chloro-3-ethyl-2-iodopyrazolo[1,5-*a*]pyridine **348-2** (550 mg, 74.80%).

LCMS: (ESI, m/z): 306.75 $[M+H]^+$.

### Step 3: Synthesis of compound 348-3

Under nitrogen protection, 7-chloro-3-ethyl-2-iodopyrazolo[1,5-*a*]pyridine (compound **348-2,** 500 mg, 1.631 mmol, 1 eq), *N*-(tert-butoxycarbonyl)propargylamine (278.47 mg, 1.794 mmol, 1.1 eq), tetrakis(triphenylphosphine) palladium (188.5 mg, 0.163 mmol, 0.1 eq), cuprous iodide (31.07 mg, 0.163 mmol, 0.1 eq), and diisopropylamine (1.65 g, 16.310 mmol, 10 eq) were added to a reaction flask, dissolved in tetrahydrofuran (8 mL) and allowed to react at 50°C for 2 h. After the reaction was completed, the solvent was directly removed by spin drying, and the resulting residue was purified by silica gel column chromatography (petroleum ether/ethyl acetate = 5:1) to give compound tert-butyl (3-(7-chloro-3-ethyl-pyrazolo[1,5-*a*]pyridin-2-yl)prop-2-yn-1-yl)carbamate **348-3** (238 mg, 39.34%).

LCMS: (ESI, m/z): 333.85 $[M+H]^+$.

### Step 4: Synthesis of compound 348-4

Under nitrogen protection, tert-butyl (3-(7-chloro-3-ethylpyrazolo[1,5-*a*]pyridin-2-yl}prop-2-yn-1-yl)carbamate (compound **348-3,** 100 mg, 0.300 mmol, 1 eq), (3*S*,4*R*)-3-fluoro-1-methylpiperidin-4-amine dihydrochloride (79.19 mg, 0.600 mmol, 2 eq), (SP-4-1)-[1,3-bis[2,6-bis(1-ethylpropyl)phenyl]-4,5-dichloro-1,3-dihydro-2*H*-imidazol-2-ylidene]dichloro(2-methylpyridine)palladium (25.2 mg, 0.030 mmol, 0.1 eq) and cesium carbonate (390.41 mg, 1.200 mmol, 4 eq) were added to a reaction flask, dissolved in dioxane (5 mL), and warmed to 120°C to react for 4 h. After the reaction was completed, the mixture was filtered, the filtrate was spin dried, and the resulting residue was purified by silica gel column chromatography (petroleum ether/ethyl acetate = 1:1), to give compound tert-butyl [3-(3-ethyl-7-{[(3*S*,4*R*)-3-fluoro-1-methylpiperidin-4-yl]amino}-pyrazolo[1,5-*a*]pyridin-2-yl)prop-2-yn-1-yl]carbamate **348-4** (40 mg, 27.98%).

LCMS: (ESI, m/z): 430.10 $[M+H]^+$.

**Step 5: Synthesis of compound 348-5**

To a reaction flask, tert-butyl [3-(3-ethyl-7-{[(3*S*,4*R*)-3-fluoro-1-methylpiperidin-4-yl]amino}-pyrazolo[1,5-*a*]pyridin-2-yl)prop-2-yn-1-yl]carbamate (compound **348-4,** 30 mg, 0.070 mmol, 1 eq) was added and dissolved in dichloromethane (0.5 mL), and then 4 mol/L hydrogen chloride in 1,4-dioxane (0.28 mL, 16 eq) was added. The reaction was carried out at room temperature overnight. After the reaction was completed, the solvent was directly removed by spin drying to give compound 2-(3-aminopropyl-1-yn-1-yl)-3-ethyl-(3*S*,4*R*)-3-fluoro-1-methylpiperidin-4-yl)pyrazolo[1,5-*a*]pyridin-7-amine dihydrochloride **348-5** (30 mg, 97.79%).
LCMS: (ESI, m/z): 330.05 $[M+H]^+$.

**Step 6: Synthesis of 5-amino-1-tert-butyl-*N*-[3-(3-ethyl-7-{[(3*S*,4*R*)-3-fluoro-1-methylpiperidin-4-yl]amino}-pyrazolo[1,5-*a*]pyridin-2-yl)prop-2-yn-1-yl]-1*H*-pyrazole-4-carboxamide (compound 348)**

To a reaction flask, 2-(3-aminopropyl-1-yn-1-yl)-3-ethyl-(3*S*,4*R*)-3-fluoro-1-methylpiperidin-4-yl)pyrazolo[1,5-*a*]pyridin-7-amine dihydrochloride (compound **348-5,** 40 mg, 0.121 mmol, 1 eq), 5-amino-1-tert-butylpyrazole-4-carboxylic acid (22.25 mg, 0.121 mmol, 1 eq), O-(7-azabenzotriazol-1-yl)-*N,N,N',N'*-tetramethyluronium hexafluorophosphate (55.4 mg, 0.145 mmol, 1.2 eq), and *N*,*N*-diisopropylethylamine (78.47 mg, 0.605 mmol, 5 eq) were added, dissolved in *N*,*N*-dimethylformamide (4 mL) and allowed to react at room temperature for 2 h. After the reaction was completed, the mixture was quenched with water and extracted with ethyl acetate (3 × 10 mL). The organic phases were combined, backwashed with saturated sodium chloride solution (1 × 10 mL), dried over anhydrous sodium sulfate, filtered, and the filtrate was concentrated under reduced pressure. The crude product was purified by HPLC with the following conditions (column specifications: Xselect CSHTM Prep C18 5μm 30*150mm OBD; mobile phase A: water (0.1% formic acid), mobile phase B: acetonitrile; flow rate: 60 mL/min; elution gradient: 11% B to 35% B in 10 min; detection wavelength: UV 254 nm/220 nm; retention time (min): 7.89), to give the carboxylate of compound **5-amino-1-tert-butyl-*N*-[3-(3-ethyl-7-{[(3*S*,4*R*)-3-fluoro-1-methylpiperidin-4-yl]amino}-pyrazolo[1,5-*a*]pyridin-2-yl)prop-2-yn-1-yl]-1*H*-pyrazole-4-carboxamide 348** (1.9 mg, 3.07%).
LCMS: (ESI, m/z): 495.20 $[M+H]^+$.
$^1$H NMR (400 MHz, DMSO-$d_6$, *ppm*) δ 8.30 (t, *J* = 5.7 Hz, 1H), 7.66 (s, 1H), 7.16 (dd, *J* = 8.8, 7.5 Hz, 1H), 6.96 (dd, *J* = 8.8, 1.1 Hz, 1H), 6.21 (d, J = 7.4 Hz, 1H), 6.17 (s, 2H), 4.92 (d, *J* = 49.2 Hz, 1H), 4.28 (d, *J* = 5.6 Hz, 2H), 3.96 - 3.72 (m, 2H), 3.21 - 3.08 (m, 1H), 2.93 - 2.81 (m, 1H), 2.72 (q, *J* = 7.5 Hz, 2H), 2.41 - 2.15 (m, 5H), 2.01 - 1.86 (m, 2H), 1.52 (s, 9H), 1.18 (t, *J* = 7.5 Hz, 3H).

**Example 151**

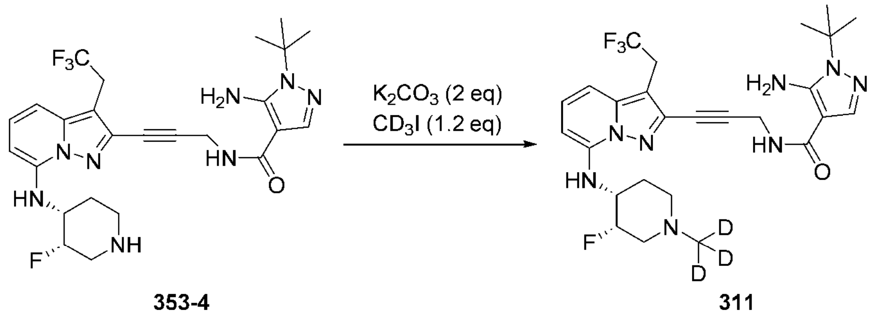


353-4 311

**Step 1: Synthesis of 5-amino-1-(tert-butyl)-N-[3-(7-{[(3S,4R)-3-fluoro-1-(methyl-d3)piperidin-4-yl]amino}-3-(2,2,2-trifluoroethyl)pyrazolo[1,5-a]pyridin-2-yl)prop-2-yn-1-yl]-1*H*-pyrazole-4-carboxamide (compound 311)**

To a solution of 5-amino-1-tert-butyl-*N*-[3-(7-{[(3*S*,4*R*)-3-fluoropiperidin-4-yl]amino}-3-(2,2,2-trifluoroethyl)pyrazolo[1,5-*a*]pyridin-2-yl)prop-2-yn-1-yl]-1*H*-pyrazole-4-carboxamide (compound 353-4, 100 mg, 0.19 mmol, 1 eq) in N,N-dimethylformamide (5 mL) were added potassium carbonate (52 mg, 0.38 mmol, 2 eq) and deuterated iodomethane (33 mg, 0.24 mmol, 1.3 eq) at room temperature. After the addition was completed, the system was stirred at 50°C for 2 h. After the reaction was completed, saturated aqueous ammonium chloride solution (20 mL) was added to the system to quench

the reaction, and the system was extracted with ethyl acetate (3 × 30 mL). The organic phases were combined, backwashed with saturated brine (30 mL), dried over anhydrous sodium sulfate, filtered, and the filtrate was concentrated under reduced pressure. The crude product was purified by HPLC with the following conditions (Waters 2767/Qda, column specifications: Atlantis TM T3 prep OBD TM, 19*250mm, 10μm; mobile phase A: water (0.1% formic acid), B: acetonitrile; flow rate: 20 mL/min; elution gradient: 31%-31% in 16 min; retention time: 5.4-8.0 min) to give the carboxylate of compound **5-amino-1-(tert-butyl)-N-[3-(7-{[(3S,4R)-3-fluoro-1-(methyl-d3)piperidin-4-yl]amino}-3-(2,2,2-trifluoroethyl)pyrazolo[1,5-a]pyridin-2-yl)prop-2-yn-1-yl]-1*H*-pyrazole-4-carboxamide 311** (19 mg, 17%).

LCMS: (ESI, m/z): 552.2 [M+H]+.

$^1$H NMR (400 MHz, DMSO-$d_6$) $\delta$ 8.30 (t, *J* = 5.6 Hz, 1H), 7.65 (s, 1H), 7.32 - 7.25 (m, 1H), 7.02 (d, *J* = 8.8 Hz, 1H), 6.31 (d, *J* = 7.2 Hz, 1H), 6.24 (d, *J* = 9.2 Hz, 1H), 6.18 (s, 2H), 4.87 (d, *J* = 49.6 Hz, 1H), 4.28 (d, *J* = 5.6 Hz, 2H), 3.91 - 3.69 (m, 3H), 3.05 (t, *J* = 10.6 Hz, 1H), 2.78 (d, *J* = 12.4 Hz, 1H), 2.29 (dd, J = 38.3, 12.5 Hz, 1H), 2.13 (t, J = 10.6 Hz, 1H), 2.04 - 1.90 (m, 1H), 1.92 - 1.82 (m, 1H), 1.52 (s, 9H).

## Example 152

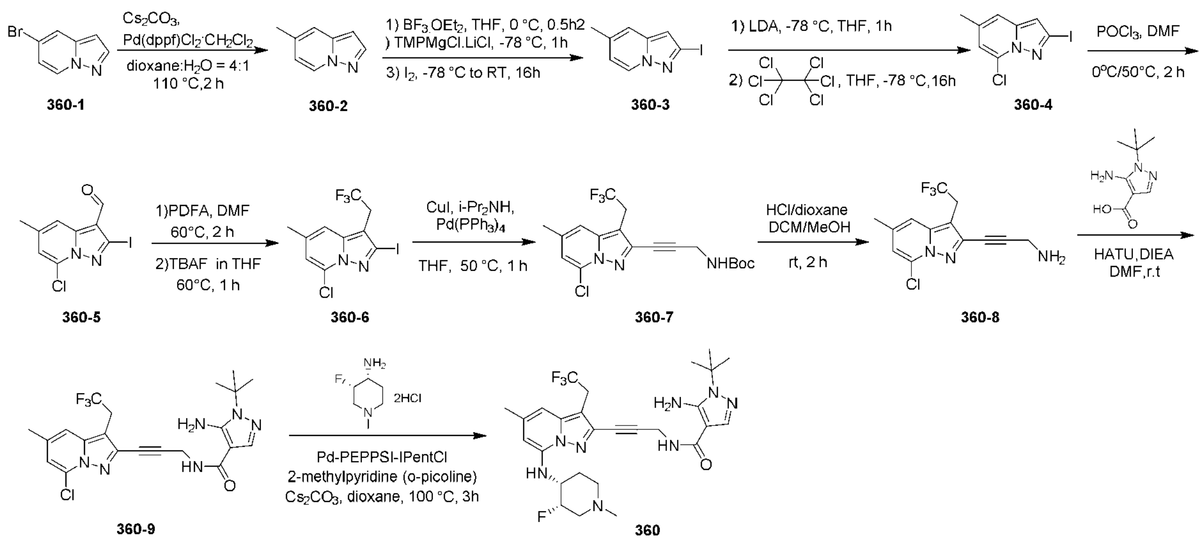


### Step 1: Synthesis of compound 360-2

Under nitrogen protection, to a solution of 5-bromopyrazolo[1,5-a]pyridine (compound 360-1, 10 g, 50.75 mmol, 1 eq), cesium carbonate (49.61 g, 152.25 mmol, 3 eq) and trimethyl-1,3,5,2,4,6-trioxatriborane (7.64 g, 60.9 mmol, 1.2 eq) in 1,4-dioxane (80 mL) and water (20 mL) was added [1,1'-bis(diphenylphosphino)ferrocene]dichloropalladium(II) dichloromethane complex (4.14 g, 5.08 mmol, 0.1 eq) at 25°C. The mixture was further stirred at 110°C for 2 h. After the reaction was completed, the reaction mixture was cooled to room temperature and quenched with water (200 mL). The reaction mixture was extracted with ethyl acetate (3 × 150 mL). The organic phases were combined, backwashed with saturated brine (2 × 150 mL), dried over anhydrous sodium sulfate, filtered, and the filtrate was concentrated under reduced pressure. The resulting residue was purified by silica gel column chromatography (ethyl acetate/petroleum ether = 0-10%) to give compound 5-methylpyrazolo[1,5-a]pyridine **360-2** (4.3 g, 64.11%).

LCMS: (ESI, m/z): 133.3 [M+H]+.

### Step 2: Synthesis of compound 360-3

Under nitrogen protection, to a solution of 5-methylpyrazolo[1,5-a]pyridine (compound 360-2, 4.3 g, 32.53 mmol, 1 eq) in tetrahydrofuran (60 mL) was added boron trifluoride ether (6.93 g, 48.80 mmol, 1.5 eq) at 0°C. The mixture was stirred to react for 30 min, then cooled to -78°C, and a solution of 2,2,6,6-tetramethylpiperidinyl magnesium chloride lithium chloride (65.06 mL, 1 M, 65.06 mmol, 2 eq) in tetrahydrofuran was added dropwise. The mixture was stirred to react for 1 h, and then a solution of iodine (12.39 g, 48.80 mmol, 1.5 eq) in tetrahydrofuran (60 mL) was added dropwise at -78°C. After the addition was completed, the system was stirred at -78°C for 1 h, then naturally returned to room temperature and further stirred overnight. After the reaction was completed, the reaction mixture was quenched with sodium thiosulfate aqueous solution (150 mL), and extracted with ethyl acetate (150 mL×3). The organic phases were combined, washed with

saturated brine (150 mL×2), dried over anhydrous sodium sulfate, filtered and concentrated to give a crude product. The crude product was purified by silica gel column chromatography (petroleum ether/ethyl acetate = 10:1) to give compound 2-iodo-5-methylpyrazolo[1,5-a]pyridine **360**-3 (4.3 g, 51.21%).

LCMS: (ESI, m/z): 259.1 $[M+H]^+$.

**Step 3: Synthesis of compound 360-4**

Under nitrogen protection, at -78°C, to a solution of 2-iodo-5-methylpyrazolo[1,5-a]pyridine (compound 360-3, 4.3 g, 16.66 mmol, 1 eq) in tetrahydrofuran (50 mL) was added dropwise lithium diisopropylamide (12.5 mL, 2M, 24.99 mmol, 1.5 eq) and stirred to react for 1 h at -78°C. Then, a solution of hexachloroethane (7.89 g, 33.33 mmol, 2 eq) in tetrahydrofuran (30 mL) was added dropwise. After the addition was completed, the mixture was stirred at -78°C for 30 min and then naturally returned to room temperature and further stirred overnight. After the reaction was completed, the reaction mixture was quenched with saturated aqueous ammonium chloride solution (100 mL) at room temperature, and extracted with ethyl acetate (3×100 mL). The organic phases were combined and backwashed with saturated brine (100 mL), dried over anhydrous sodium sulfate, filtered and concentrated to give a crude product. The crude product was purified by silica gel column chromatography (ethyl acetate/petroleum ether (0-10%)) to give compound 7-chloro-2-iodo-5-methylpyrazolo[1,5-a]pyridine **360-4** (3.5 g, 71.81%).

LCMS: (ESI, m/z): 292.9 $[M+H]^+$.

**Step 4: Synthesis of compound 360-5**

Under nitrogen protection and at 0°C, to a solution of 7-chloro-2-iodo-5-methylpyrazolo[1,5-a]pyridine (compound 360-4, 3 g, 10.26 mmol, 1 eq) in *N*,*N*-dimethylformamide (30 mL) was added phosphorus oxychloride (4.72 g, 30.77 mmol, 3 eq). The mixture was stirred to react at 0°C for 30, then the system was stirred at 50°C for 2 h. After the reaction was completed, the reaction mixture was slowly poured into ice water (50 mL) at room temperature to quench the reaction. The mixture was adjusted to a basic pH with saturated sodium bicarbonate solution, and then extracted with ethyl acetate (3×50 mL). The organic phases were combined, backwashed with saturated brine (100 mL), dried over anhydrous sodium sulfate, filtered, and concentrated to give a crude product. The crude product was purified by silica gel column chromatography (ethyl acetate/petroleum ether (0-10%)) to give compound 7-chloro-2-iodo-5-methylpyrazolo[1,5-a] pyridine-3-carboxaldehyde **360-5** (3 g, 91.30%).

LCMS: (ESI, m/z): 321.0 $[M+H]^+$.

**Step 5: Synthesis of compound 360-6**

Under nitrogen protection, to a solution of 7-chloro-2-iodo-5-methylpyrazolo[1,5-a]pyridine-3-carboxaldehyde (compound 360-5, 1 g, 3.12 mmol, 1 eq) in N,N-dimethylformamide (20 mL) was added (triphenylphosphono)difluoroacetate (2.22 g, 6.24 mmol, 2 eq), and the mixture was stirred to react at 60°C for 2 h. The mixture was cooled to room temperature, and then tetrabutylammonium fluoride (9.36 mL, 1 M in THF, 3 eq) was added. After the addition was completed, the system was stirred at 60°C for 1 h. After the reaction was completed, the reaction mixture was quenched with saturated aqueous ammonium chloride solution (100 mL) at room temperature, and then extracted with ethyl acetate (3 × 100 mL). The organic phases were combined, backwashed with saturated brine (100 mL), dried over anhydrous sodium sulfate, filtered and concentrated to give a crude product. The crude product was purified by silica gel column chromatography (ethyl acetate/petroleum ether (0-10%)) to give compound 7-chloro-2-iodo-5-methyl-3-(2,2,2-trifluoroethyl)pyrazolo[1,5-a]pyridine **360-6** (1 g, 85.58%).

LCMS: (ESI, m/z): 374.9 $[M+H]^+$.

**Step 6: Synthesis of compound 360-7**

Under nitrogen protection, at room temperature, to a solution of 7-chloro-2-iodo-5-methyl-3-(2,2,2-trifluoroethyl) pyrazolo[1,5-a]pyridine (compound 360-6, 800 mg, 2.14 mmol, 1 eq) and tert-butyl prop-2-yn-1-yl carbamate (50 mg, 3.21 mmol, 1.5 eq) in tetrahydrofuran (10 mL) were added diisopropylamine (3.01 mL, 10 eq), tetrakis(triphenylphosphine) palladium (0.25 g, 0.21 mmol, 0.1 eq) and cuprous iodide (41 mg, 0.21 mmol, 0.1 eq) in portions . After the addition was completed, the system was stirred to react at 50°C for 1 h. After the reaction was completed, the reaction mixture was diluted with water (50 mL) and then extracted with ethyl acetate (3×50 mL). The organic phases were combined, backwashed with saturated brine (50 mL), dried over anhydrous sodium sulfate, filtered and concentrated to give a crude product. The crude product was purified by silica gel column chromatography (ethyl acetate/petroleum ether (0-10%)) to give compound tert-butyl N-(3-(7-chloro-5-methyl-3-(2,2,2-trifluoroethyl)pyrazolo[1,5-a]pyridin-2-yl)prop-2-yn-1-yl)carbamate **360-7** (750 mg, 87.39%).

LCMS: (ESI, m/z): 402.1 $[M+H]^+$.

**Step 7: Synthesis of compound 360-8**

At room temperature, a solution of hydrochloric acid in ethyl acetate (4 M, 4 mL) was added to a solution of tert-butyl N-(3-(7-chloro-5-methyl-3-(2,2,2-trifluoroethyl)pyrazolo[1,5-a]pyridin-2-yl)prop-2-yn-1-yl) carbamate (compound 360-7, 150 mg, 0.37 mmol, 1 eq) in methanol (4 mL). The mixture was stirred to react at room temperature for 2 h. After the reaction was completed, the reaction mixture was concentrated and spin dried to give compound 3-(7-chloro-5-methyl-3-(2,2,2-trifluoroethyl)pyrazolo[1,5-a]pyridin-2-yl)prop-2-yn-1-amine **360-8** (150 mg, 133.19%). The crude product was directly used in the next step.
LCMS: (ESI, m/z): 302.1 $[M+H]^+$.

**Step 8: Synthesis of compound 360-9**

Under nitrogen protection, at 25°C, to a solution of 5-amino-1-tert-butyl-1*H*-pyrazole-4-carboxylic acid (73 mg, 0.40 mmol, 1.2 eq) in N,N-dimethylformamide (5 mL) were added N,N-diisopropylethylamine (260 mg, 1.98 mmol, 6 eq) and N,N,N',N'-tetramethyl-O-(7-azabenzothiadiazol-1-yl)hexafluorophosphorourea (140 mg, 0.36 mmol, 1.1 eq). The mixture was stirred at 25°C for 30 min. After 30 min, 3-(7-chloro-5-methyl-3-(2,2,2-trifluoroethyl)pyrazolo[1,5-a]pyridin-2-yl)prop-2-yn-1-amine (compound 360-8, 100 mg, 0.33 mmol, 1 eq) was added to the system. The mixture was further stirred at 25°C for 1 h. After the reaction was completed, the reaction mixture was diluted with water (50 mL) at room temperature, and then extracted with ethyl acetate (3×20 mL). The organic phases were combined, backwashed with saturated brine (3×50 mL), dried over anhydrous sodium sulfate, filtered, and concentrated to give a crude product. The crude product was purified by silica gel column chromatography (methanol/dichloromethane (0-5%)) to give compound 5-amino-1-tert-butyl-N-(3-(7-chloro-5-methyl-3-(2,2,2-trifluoroethyl)pyrazolo[1,5-a]pyridin-2-yl)prop-2-yn-1-yl)-1*H*-pyrazole-4-carboxamide **360-9** (150 mg, 96.93%).
LCMS: (ESI, m/z): 467.2 $[M+H]^+$.

**Step 9: Synthesis of 5-amino-1-tert-butyl-N-[3-(7-{[(3S,4R)-3-fluoro-1-methylpiperidin-4-yl]amino}-5-methyl-3-(2,2,2-trifluoroethyl)pyrazolo[1,5-a]pyridin-2-yl)prop-2-yn-1-yl]-1*H*-pyrazole-4-carboxamide (compound 360)**

Under nitrogen protection, at room temperature, to a solution of 5-amino-1-tert-butyl-N-(3-(7-chloro-5-methyl-3-(2,2,2-trifluoroethyl)pyrazolo[1,5-a]pyridin-2-yl)prop-2-yn-1-yl)-1H-pyrazole-4-carboxamide (compound 360-9, 100 mg, 0.21 mmol, 1 eq) and (3S,4R)-3-fluoro-1-methylpiperidin-4-amine (33 mg, 0.25 mmol, 1.2 eq) in 1,4-dioxane (5 mL) were added cesium carbonate (270 mg, 0.84 mmol, 4 eq) and (SP-4-1)-[1,3-bis[2,6-bis(1-propylbutyl) phenyl]-4,5-dichloro-1,3-dihydro-2*H*-imidazol-2-ylidene]dichloro(3-chloropyridine-KN)palladium (42 mg, 0.043 mmol, 0.1 eq) in portions. After the addition was completed, the system was further stirred at 100°C for 12 h. After the reaction was completed, the reaction mixture was diluted with water (50 mL) at room temperature, and extracted with ethyl acetate (3×20 mL). The organic phases were combined, backwashed with saturated brine (3×50 mL), dried over anhydrous sodium sulfate, filtered, and concentrated to give a crude product. The crude product was purified by silica gel column chromatography (methanol/dichloromethane (0-5%)) and further purified by HPLC with the following conditions: (Waters 2767/Qda) column: Sunfire C18 19*250*10μm; mobile phase A: 0.1% formic acid/water, B: acetonitrile; flow rate: 20 mL/min; gradient: 27-27%; retention time: 7.0-12.0 min. The resulting liquid phase was concentrated, then acetonitrile (2 mL) and pure water (10 mL) were added for freeze-drying, to give the carboxylate of **5-amino-1-tert-butyl-N-[3-(7-{ [(3 S,4R)-3-fluoro-1-methylpiperidin-4-yl] amino}-5-methyl-3-(2,2,2-trifluoroethyl)pyrazolo[1,5-a]pyridin-2-yl) prop-2-yn-1-yl]-1H-pyrazole-4-carboxamide 360** (39.95 mg, 33.15%).
LCMS: (ESI, m/z): 563.2 $[M+H]^+$.
1H NMR (400 MHz, DMSO-d6) δ 8.29 (t, J = 5.6 Hz, 1H), 7.65 (s, 1H), 6.81 (s, 1H), 6.18 (d, *J*= 3.1 Hz, 3H), 6.13 (d, *J*= 9.0 Hz, 1H), 4.86 (d, *J* = 49.8 Hz, 1H), 4.27 (d, J = 5.6 Hz, 2H), 3.90 - 3.75 (m, 1H), 3.69 (q, *J* = 11.0 Hz, 2H), 3.05 (t, J = 11.2 Hz, 1H), 2.78 (d, J = 12.4 Hz, 1H), 2.33 (s, 3H), 2.28 (d, *J* = 25.4 Hz, 1H), 2.20 (s, 3H), 2.13 (t, J = 10.4 Hz, 1H), 1.99 - 1.80 (m, 2H), 1.52 (s, 9H).

**Example 153**

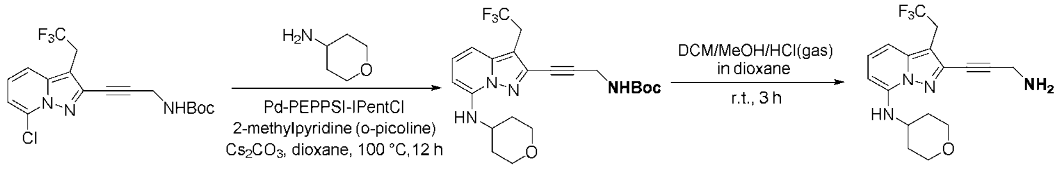


**001-10** **361-1** **361-2**

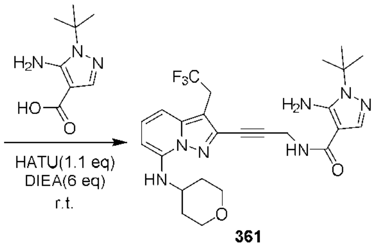


**361**

**Step 1: Synthesis of compound 361-1**

Under nitrogen protection, at room temperature, to a solution of tert-butyl {3-[7-chloro-3-(2,2,2-trifluoroethyl) pyrazolo[1,5-a]pyridin-2-yl]prop-2-yn-1-yl}carbamate (compound 001-10, 300 mg, 0.77 mmol, 1 eq) and tetrahydro-2*H*-pyran-4-amine (117.4 mg, 1.16 mmol, 1.5 eq) in 1,4-dioxane (5 mL) were added cesium carbonate (1.01 g, 3.09 mmol, 4 eq) and (SP-4-1)-[1,3-bis[2,6-bis(1-propylbutyl)phenyl]-4,5-dichloro-1,3-dihydro-2*H*-imidazol-2-ylidene]dichloro(3-chloropyridine-KN)palladium (74.9 mg, 0.08 mmol, 0.1 eq) in portions. The system was stirred to react at 100°C for 12 h. After the reaction was completed, the reaction mixture was quenched with water (30 mL) at room temperature, then extracted with ethyl acetate (3×20 mL), and the organic phases were combined, backwashed with saturated brine (3×20 mL), dried over anhydrous sodium sulfate, filtered and concentrated to give a crude product. The crude product was purified by silica gel column chromatography (dichloromethane/methanol (10:1)) to give compound tert-butyl N-(3-(7-[(tetrahydro-2*H*-pyran-4-yl)amino]-3-(2,2,2-trifluoroethyl)pyrazolo[1,5-a]pyridin-2-yl)prop-2-yn-1-yl)carbamate **361-1** (280 mg, 79.99%).

LCMS: (ESI, m/z): 453.3 $[M+H]^+$.

**Step 2: Synthesis of compound 361-2**

To a solution of tert-butyl N-(3-(7-[(tetrahydro-2*H*-pyran-4-yl)amino]-3-(2,2,2-trifluoroethyl)pyrazolo[1,5-a]pyridin-2-yl)prop-2-yn-1-yl)carbamate (compound 361-1, 230 mg, 0.51 mmol, 1 eq) in dichloromethane (2 mL) and methanol (4 mL) was added hydrochloric acid-1,4-dioxane solution (4 M, 4 mL) at 25°C, and the mixture was stirred to react at room temperature for 2 h. The reaction was detected by LC-MS. After the reaction was completed, the reaction mixture was directly spin-dried to give compound 2-(3-aminoprop-1-yn-1-yl)-N-(tetrahydro-2*H*-pyran-4-yl)-3-(2,2,2-trifluoroethyl)pyrazolo[1,5-a]pyridin-7-amine **361-2** (200 mg, crude product), and the crude product was directly used in the next step of reaction.

LCMS: (ESI, m/z): 353.3 $[M+H]^+$.

**Step 3: Synthesis of 5-amino-1-tert-butyl-N-(3-(7-[(tetrahydro-2H-pyran-4-yl)amino]-3-(2,2,2-trifluoroethyl)pyrazolo[1,5-a]pyridin-2-yl)prop-2-yn-1-yl)-1*H*-pyrazole-4-carboxamide (compound 361)**

Under nitrogen protection, at 25°C, to a solution of 5-amino-1-tert-butyl-1*H*-pyrazole-4-carboxylic acid (31.2 mg, 0.17 mmol, 1.2 eq) in N,N-dimethylformamide (5 mL) were added N,N-diisopropylethylamine (108.6 mg, 0.84 mmol, 6 eq) and N,N,N',N'-tetramethyl-O-(7-azabenzothiazol-1-yl)hexafluorophosphorourea (58.6 mg, 0.15 mmol, 1.1 eq), and the mixture was stirred at 25°C for 30 min. After 30 min, 2-(3-aminoprop-1-yn-1-yl)-N-(tetrahydro-2*H*-pyran-4-yl)-3-(2,2,2-trifluoroethyl)pyrazolo[1,5-a]pyridin-7-amine (compound 361-2, 50 mg, 0.14 mmol, 1 eq) was added to the solution and the system was further stirred at 25°C for 1 h. The reaction was detected by LC-MS. The reaction mixture was diluted with water (50 mL) and then extracted with ethyl acetate (3 × 20 mL). The organic phases were combined, backwashed with saturated brine (50 mL), dried over anhydrous sodium sulfate, filtered and concentrated to give a crude product. The crude product was pulped with methanol : dichloromethane (1:1), and then filtered to give compound **5-amino-1-tert-butyl-N-(3-(7-[(tetrahydro-2H-pyran-4-yl)amino]-3-(2,2,2-trifluoroethyl)pyrazolo[1,5-a]pyridin-2-yl)prop-2-yn-1-yl)-1*H*-pyrazole-4-carboxamide 361** (10.5 mg, 14.3%).

LCMS: (ESI, m/z): 518.3 $[M+H]^+$.

$^1$H NMR (400 MHz, DMSO-d6) δ 8.32 (t, J = 5.7 Hz, 1H), 7.65 (s, 1H), 7.25 (t, J = 8.4 Hz, 1H), 6.94 (d, J = 8.8 Hz,

1H), 6.75 (d, J = 8.4 Hz, 1H), 6.24 - 6.16 (m, 3H), 4.27 (d, J = 5.6 Hz, 2H), 3.88 (d, J = 11.6 Hz, 2H), 3.80 - 3.67 (m, 3H), 3.44 (d, J = 10.8 Hz, 2H), 1.88 (d, J = 10.4 Hz, 2H), 1.78 - 1.64 (m, 2H), 1.52 (s, 9H).

**Example 154**

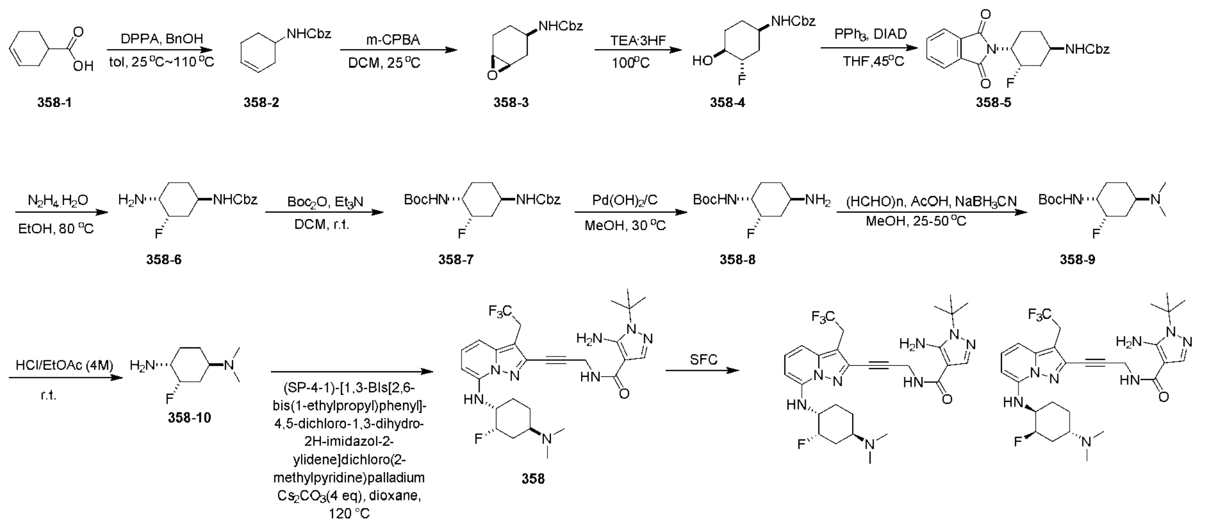


Note: "

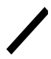

" indicates that there is relative chirality at a specific position in the molecule, for example,

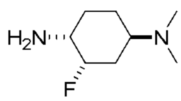

indicates a mixture of

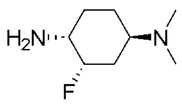

and

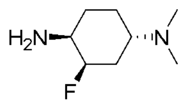

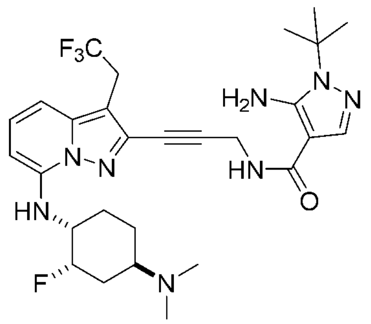

indicates a mixture of

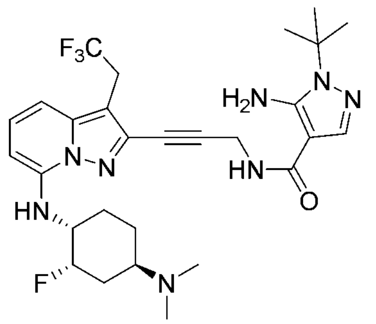

and

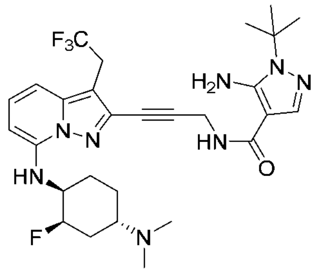

.

**Step 1: Synthesis of compound 358-2**

At 25°C, to a solution of cyclohex-3-ene-1-carboxylic acid (compound 358-1, 30 g, 237 mmol, 1 eq) in toluene (300 mL) were added DPPA (68.5 g, 249 mmol, 1.05 eq) and triethylamine (26.4 g, 261.6 mmol, 1.1 eq), nitrogen was replaced three times, the mixture was stirred at 25°C for 1.5 h, then warmed to 110°C and further stirred for 2.5 h. Then, benzyl alcohol (28.3 g, 261.6 mmol, 1.1 eq) was added, and the mixture was further stirred at 110°C for 12 h. After the reaction was completed, the reaction mixture was concentrated under reduced pressure, and the resulting residue was purified by silica gel column chromatography (petroleum ether/ethyl acetate = 5:1) to give compound benzyl cyclohex-3-ene-1-ylcarbamate **358-2** (51 g, 93.1%).

LCMS: (ESI, m/z): 232.2 $[M+H]^+$.

**Step 2: Synthesis of compound 358-3**

To a solution of benzyl cyclohex-3-en-1-ylcarbamate (compound 358-2, 56.5 g, 244 mmol, 1 eq) in dichloromethane (500 mL) was added m-CPBA (54.8 g, 317.5 mmol, 1.3 eq) at 25°C. The reaction mixture was allowed to react at room temperature for 3 h. After the reaction was completed, the reaction mixture was filtered, and the filtrate was washed with saturated sodium sulfite solution (500 mL×2) and saturated sodium bicarbonate solution (500 mL×2) in sequence. The organic phases were combined and concentrated under reduced pressure to give a crude product, which was purified by silica gel column chromatography (petroleum ether/ethyl acetate = 5:1) to give benzyl ((1R, 6S)-7-oxabicyclo[4.1.0] heptan-3-yl)carbamate **358-3** (52 g, 86.2%).

LCMS: (ESI, m/z): 248.2 $[M+H]^+$.

**Step 3: Synthesis of compound 358-4**

At room temperature, to benzyl ((1R,6S)-7-oxabicyclo[4.1.0]heptan-3-yl)carbamate (compound 358-3, 47 g, 190 mmol, 1 eq)) was added TEA.3HF (153 g, 161.2 mmol, 5 eq), and the mixture was stirred at 100°C for 2 h. After the reaction was completed, the reaction mixture was cooled to 20°C and slowly poured into an aqueous solution of potassium carbonate (137 g, 161.2 mmol, 5 eq). The mixed mixture was extracted with ethyl acetate (800 mL×3), and the organic phases were combined, backwashed with saturated brine (800 mL), dried over anhydrous sodium sulfate, filtered and concentrated. The resulting crude product was purified by silica gel column chromatography (petroleum ether/ethyl acetate = 5:1) to give compound benzyl ((1R,3S,4S)-3-fluoro-4-hydroxycyclohexyl)carbamate **358-4** (18 g, 35.4%).

LCMS: (ESI, m/z): 268.2 $[M+H]^+$.

**Step 4: Synthesis of compound 358-5**

Under nitrogen protection, to a solution of benzyl ((1R,3S,4S)-3-fluoro-4-hydroxycyclohexyl)carbamate (compound 358-4, 14 g, 52.4 mmol, 1 eq), isoindoline-1,3-dione (7.71 g, 52.4 mmol, 1 eq), and $PPh_3$ (17.88 g, 68.1 mmol, 1.3 eq) in tetrahydrofuran (150 mL) was added DIAD (13.78 g, 68.1 mmol, 1.3 eq). The mixture was stirred to react at 45°C for 12 h. After the reaction was completed, the mixture was filtered and the filter cake was rinsed with acetonitrile (20 mL×2) to give compound benzyl ((1R,3S,4R)-4-(1,3-dioxoisoindolin-2-yl)-3-fluorocyclohexyl)carbamate **358-5** (11 g, 53.0%).

$^1$H NMR (400 MHz, DMSO-d6) δ 7.92 - 7.81 (m, 4H), 7.42 - 7.28 (m, 6H), 5.03 (s, 2H), 4.93 - 4.74 (m, 1H), 4.23 - 4.02 (m, 1H), 3.80 - 3.54 (m, 1H), 3.02 - 2.84 (m, 1H), 2.18 (s, 1H), 1.99 (d, J = 12.4 Hz, 1H), 1.87 - 1.55 (m, 2H), 1.48 - 1.33 (m, 1H).

**Step 5: Synthesis of compound 358-6**

At room temperature, to a solution of benzyl ((1R,3S,4R)-4-(1,3-dioxoisoindolin-2-yl)-3-fluorocyclohexyl)carbamate (compound 358-5, 11 g, 27.7 mmol, 1 eq) in ethanol (110 mL) was added an ammonia water solution (35 mL, 98% purity), and the mixture was stirred to react at 80°C for 3 h. After the reaction was completed, the reaction mixture was concentrated under reduced pressure, dichloromethane (40 mL) was added and the mixture was filtered. The filter cake was rinsed with dichloromethane (20 mL×2), and the filtrate was concentrated to give compound benzyl ((1R,3S,4R)-4-amino-3-fluorocyclohexyl)carbamate **358-6** (14 g, crude product).

LCMS: (ESI, m/z): 267.1 $[M+H]^+$.

**Step 6: Synthesis of compound 358-7**

To a solution of benzyl ((1R,3S,4R)-4-amino-3-fluorocyclohexyl)carbamate (compound 358-6, 13 g, 48.8 mmol, 1 eq) in dichloromethane (130 mL) were added di-tert-butyl dicarbonate (21.3 g, 97.7 mmol, 2 eq) and triethylamine (14.8 g, 146.6 mmol, 3 eq) at room temperature. The system was stirred at 25°C for 3 h. After the reaction was completed, the mixture was concentrated under reduced pressure and the crude product was purified by silica gel column chromatography (petroleum ether/ethyl acetate = 2:1) to give compound benzyl N-[(1R,3S,4R)-4-{[(tert-butoxy)carbonyl] amino }-3-fluorocyclohexyl]carbamate **358-7** (13 g, 72.7%).

$^1$H NMR (400 MHz, DMSO-d6) δ 7.51 - 7.28 (m, 5H), 7.24 (d, J = 7.6 Hz, 1H), 6.92 (d, J = 7.6 Hz, 1H), 5.00 (s, 2H), 4.76 (d, J = 50.4 Hz, 1H), 3.59 - 3.39 (m, 2H), 2.08 (d, J = 8.4 Hz, 1H), 1.80 (d, J = 12.4 Hz, 1H), 1.64 - 1.46 (m, 3H), 1.45 - 1.34 (m, 9H), 1.33 - 1.19 (m, 1H).

**Step 7: Synthesis of compound 358-8**

At room temperature, to a solution of benzyl N-[(1R,3S,4R)-4-{[(tert-butoxy)carbonyl]amino}-3-fluorocyclohexyl] carbamate (compound 358-7, 400 mg, 1.09 mmol, 1 eq) in methanol (10 mL) was added palladium hydroxide on carbon (400 mg). The reaction was stirred at 30°C under hydrogen (15 Psi) for 4 h. After the reaction was completed, the mixture was filtered through diatomaceous earth and washed with methanol (50 mL × 2). The filtrate was concentrated to give tert-butyl N-[(1R,2S,4R)-4-amino-2-fluorocyclohexyl]carbamate **358-8** (320 mg, 126.2%), and the crude product was used directly in the next step of reaction.

**Step 8: Synthesis of compound 358-9**

Under nitrogen protection and room temperature, to a solution of tert-butyl N-[(1R, 2S, 4R)-4-amino-2-fluorocyclohexyl]carbamate (compound 358-8, 200 mg, 0.86 mmol, 1 eq) in methanol (4 mL) were added glacial acetic acid (5.2 mg, 0.086 mmol, 0.1 eq) and paraformaldehyde (218.8 mg, 3.44 mmol, 4 eq) in sequence. The reaction was stirred at 25°C for half an hour and then sodium cyanoborohydride (270 mg, 4.3 mmol, 5 eq) was added. After the addition was completed, the system was further stirred at 50°C for 16 h. After the reaction was completed, the reaction mixture was spin-dried and quenched with saturated sodium bicarbonate aqueous solution (50 mL), extracted with dichloromethane (50 mL×3), and the organic phases were combined and backwashed with saturated brine (50 mL), dried over anhydrous sodium sulfate, filtered and concentrated to give compound tert-butyl N-[(1R,2S,4R)-4-(dimethylamino)-2-fluorocyclohexyl]carbamate **358-9** (300 mg, 133.8%). The crude product was directly used in the next step.

$^1$H NMR (400 MHz, DMSO-d6) δ 6.86 (d, *J* = 7.6 Hz, 1H), 4.80 (d, *J* = 51.2 Hz, 1H), 3.47 - 3.37 (m, 1H), 2.47 - 2.37 (m, 1H), 2.14 (s, 6H), 2.07 - 1.96 (m, 1H), 1.80 - 1.71 (m, 1H), 1.64 - 1.44 (m, 3H), 1.38 (s, 9H), 1.29 - 1.22 (m, 1H).

**Step 9: Synthesis of compound 358-10**

To a mixed solution of tert-butyl N-[(1R,2S,4R)-4-(dimethylamino)-2-fluorocyclohexyl]carbamate (compound 358-9, 100 mg, 0.38 mmol, 1 eq) in methanol (2 mL) and dichloromethane (1 mL) was added a solution of hydrochloric acid in ethyl acetate (4 M, 2 mL), and the mixture was stirred at room temperature for 2 h. After the reaction was completed, the reaction mixture was spin-dried to give compound (1R,3S,4R)-3-fluoro-$N^1$,$N^1$-dimethylcyclohexane-1,4-diamine **358-10** (60 mg, 97.5%), and the crude product was used directly in the next step.

1H NMR (400 MHz, DMSO-d6) δ 11.20 (s, 1H), 8.59 (s, 3H), 5.20 (d, J = 49.2 Hz, 1H), 2.74 - 2.58 (m, 6H), 2.18 - 1.88 (m, 3H), 1.74 - 1.56 (m, 2H), 1.41 - 0.70 (m, 1H).

**Step 10: Synthesis of 5-amino-1-tert-butyl-N-(3-(7-{[(1R,2S)-4-(dimethylamino)-2-fluorocyclohexyl]amino}-3-(2,2,2-trifluoroethyl)pyrazolo[1,5-a]pyridin-2-yl)prop-2-yn-1-yl)-1*H*-pyrazole-4-carboxamide (compound 358)**

Under nitrogen protection, at room temperature, to a solution of 5-amino-1-tert-butyl-N-(3-(7-chloro-5-methyl-3-(2,2,2-trifluoroethyl)pyrazolo[1,5-a]pyridin-2-yl)prop-2-yn-1-yl)-1H-pyrazole-4-carboxamide (250 mg,0.55 mmol,1 eq) and (1R,3S,4R)-3-fluoro-$N^1$, $N^1$-dimethylcyclohexane-1,4-diamine (compound 358-10, 110 mg, 0.66 mmol, 1.2 eq) in 1,4-dioxane (5 mL) were added cesium carbonate (720 mg, 2.2 mmol, 4 eq) and (SP-4-1)-[1,3-bis[2,6-bis(1-ethylpropyl)phenyl]-4,5-dichloro-1,3-dihydro-2H-imidazol-2-ylidene]dichloro(2-methylpyridine)palladium (55 mg, 0.055 mmol, 0.1 eq) in sequence. After the addition was completed, the system was stirred at 120°C for 1.5 h. After the reaction was completed, the reaction mixture was diluted with water (50 mL) at room temperature, and extracted with ethyl acetate (50 mL×3). The organic phases were combined, backwashed with saturated brine (50 mL×2), dried over anhydrous sodium sulfate, filtered, and concentrated to give a crude product. The crude product was purified by silica gel column chromatography (methanol/dichloromethane (0-10%)), to give compound 5-amino-1-tert-butyl-N-(3-(7-{[(1R,2S)-4-(dimethylamino)-2-fluorocyclohexyl]amino}-3-(2,2,2-trifluoroethyl)pyrazolo[1,5-a]pyridin-2-yl)prop-2-yn-1-yl)-1*H*-pyrazole-4-carboxamide **358** (100 mg, 31.4%).

5-amino-1-tert-butyl-N-(3-(7-{[(1R,2S)-4-(dimethylamino)-2-fluorocyclohexyl]amino}-3-(2,2,2-trifluoroethyl) pyrazolo[1,5-a]pyridin-2-yl)prop-2-yn-1-yl)-1*H*-pyrazole-4-carboxamide (compound 358, 100 mg) was subjected to SFC resolution **(resolution conditions:** Waters SFC 150; column: DAICELCHIRALCEL®OD); specifications: 250*25 mm 10 μm; mobile phase: **A** supercritical $CO_2$, **B:** EtOH (+0.1% 7.0 mol/L ammonia methanol solution); **A:B:** 80:20; wavelength: 214 nm; **flow rate:** 140 mL/min; column temperature: RT; **back pressure:** 100 bar; **injection volume:** 1.0 mL; cycle time: 6.5 min; solvent: EtOH: distilled grade, supercritical $CO_2$: food grade) and freeze-dried to give compounds **358-A** (retention time: 3.109 min, 17.23 mg, 17.2%) and **358-B (retention time:** 4.055 min, 17.88 mg, 17.9%).

Compound **358-A**

LCMS: (ESI, m/z): 577.6 $[M+H]^+$.

$^1$H NMR (400 MHz, DMSO) δ 8.30 (t, J = 5.6 Hz, 1H), 7.65 (s, 1H), 7.28 (d, J = 8.0 Hz, 1H), 7.01 (d, J = 8.8 Hz, 1H), 6.29 (d, J = 7.6 Hz, 1H), 6.23 (d, J = 9.2 Hz, 1H), 6.18 (s, 2H), 5.05 (d, J = 51.2 Hz, 1H), 4.28 (d, J = 5.5 Hz, 2H), 3.93 - 3.81 (m, 1H), 3.78 - 3.70 (m, 2H), 2.59 - 2.54 (m, 1H), 2.19 (s, 6H), 2.16 - 2.08(m, 1H), 1.99 - 1.89 (m, 2H), 1.88 - 1.60 (m, 2H), 1.52 (s, 9H), 1.46 - 1.38 (m, 1H).

Compound **358-B**

LCMS: (ESI, m/z): 577.5 $[M+H]^+$.

$^1$H NMR (400 MHz, DMSO) δ 8.30 (t, J = 5.6 Hz, 1H), 7.65 (s, 1H), 7.28 (d, J = 8.0 Hz, 1H), 7.01 (d, J = 8.8 Hz, 1H), 6.29 (d, J = 7.6 Hz, 1H), 6.23 (d, J = 9.2 Hz, 1H), 6.18 (s, 2H), 5.05 (d, J = 51.2 Hz, 1H), 4.28 (d, J = 5.5 Hz, 2H), 3.93 - 3.81 (m, 1H), 3.78 - 3.70 (m, 2H), 2.59 - 2.54 (m, 1H), 2.19 (s, 6H), 2.16 - 2.08(m, 1H), 1.99 - 1.89 (m, 2H), 1.88 - 1.60 (m, 2H), 1.52 (s, 9H), 1.46 - 1.38 (m, 1H).

**Example 155**

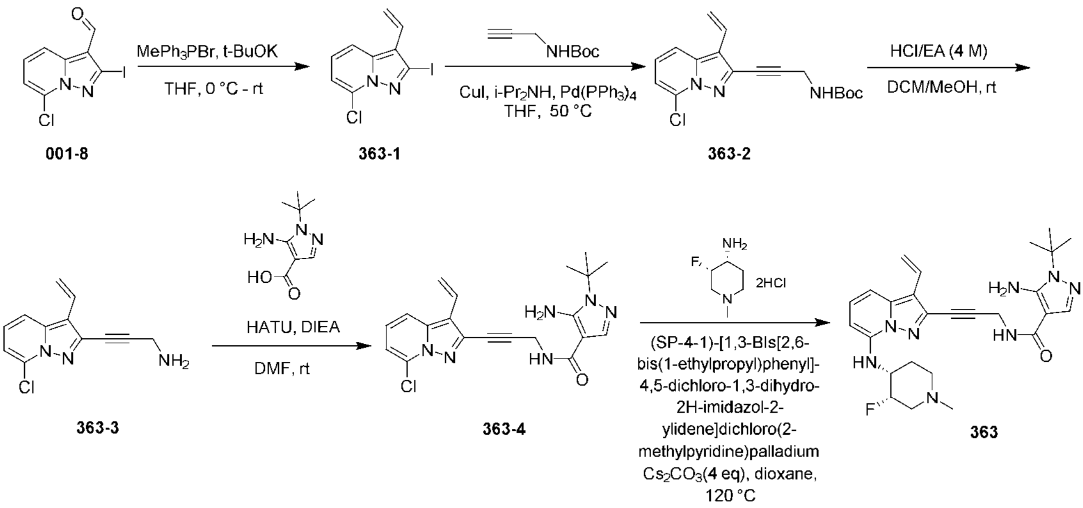


001-8 363-1 363-2

363-3 363-4 363

**Step 1: Synthesis of compound 363-1**

Under nitrogen protection, methyltriphenylphosphonium bromide (1.40 g, 3.91 mmol, 1.2 eq) was dissolved in tetrahydrofuran (15 mL) and cooled to 0°C. After cooling, potassium tert-butoxide (0.44 g, 3.91 mmol, 1.2 eq) was added to the mixture, and the mixture was further stirred at 0°C for 0.5 h. After 0.5 h, a solution of 7-chloro-2-iodopyrazolo[1,5-a]pyridine-3-carboxaldehyde (compound 001-8, 1 g, 3.26 mmol, 1 eq) in tetrahydrofuran (10 mL) was slowly added to the mixture. The reaction was gradually warmed to room temperature and stirred overnight. After the reaction was completed, the reaction mixture was slowly poured into ice water (100 mL) to quench the reaction, the pH of the aqueous phase was adjusted to neutral with 1M hydrochloric acid, the system was extracted with ethyl acetate (50 mL×3), the organic phases were combined, and backwashed with saturated brine (50 mL×2), dried over anhydrous sodium sulfate, filtered and concentrated, the crude product was purified by silica gel column chromatography (ethyl acetate/petroleum ether (0-4%)), and spin-dried to give compound 7-chloro-3-vinyl-2-iodopyrazolo[1,5-A]pyridine **363-1** (900 mg, 90.5% yield).

LCMS: (ESI, m/z): 304.8 $[M+H]^+$.

**Step 2: Synthesis of compound 363-2**

Under nitrogen protection, at room temperature, to a solution of 7-chloro-3-vinyl-2-iodopyrazolo[1,5-a]pyridine (363-1, 900 mg, 2.96 mmol, 1 eq) and tert-butyl N-(prop-2-yn-1-yl) carbamate (689.1 mg, 4.44 mmol, 1.5 eq) in tetrahydrofuran (15 mL) were added cuprous iodide (56.4 mg, 0.30 mmol, 0.1 eq), diisopropylamine (2.99 g, 29.6 mmol, 10 eq), and tetrakis(triphenylphosphine)palladium (303.9 mg, 0.30 mmol, 0.1 eq) in portions. After the addition was completed, the system was stirred to react at 50°C for 1 h. After the reaction was completed, the reaction mixture was cooled to room temperature, then diluted with water (50 mL), and then extracted with ethyl acetate (50 mL × 3). The organic phases were combined, backwashed with saturated brine (50 mL), dried over anhydrous sodium sulfate, filtered, and concentrated to give a crude product. The crude product was purified by silica gel column chromatography (ethyl acetate/petroleum ether (0-10%)) to give compound tert-butyl N-(3-(7-chloro-3-vinylpyrazolo[1,5-a]pyridin-2-yl)prop-2-yn-1-yl)carbamate **363-2** (1 g, 97.8% yield).

LCMS: (ESI, m/z): 276.0 $[M-56+H]^+$.

**Step 3: Synthesis of compound 363-3**

A solution of hydrochloric acid in ethyl acetate (4M, 4 mL) was added to a mixed solution of tert-butyl N-(3-(7-chloro-3-vinylpyrazolo[1,5-a]pyridin-2-yl)prop-2-yn-1-yl)carbamate (compound 363-2, 1 g, 3.01 mmol, leq) in methanol (10 mL) and dichloromethane (5 mL). The mixture was stirred to react at room temperature for 2 h. After the reaction was completed, the reaction mixture was concentrated and spin dried to give the hydrochloride of compound 3-(7-chloro-3-vinylpyrazolo[1,5-a]pyridin-2-yl)prop-2-yn-1-amine **363-3** (1 g, crude product). The crude product was directly used in the next step.

LCMS: (ESI, m/z): 232.1 $[M+H]^+$.

**Step 4: Synthesis of compound 363-4**

Under nitrogen protection, at room temperature, to a solution of 5-amino-1-tert-butyl-1H-pyrazole-4-carboxylic

acid (949.8 mg, 5.2 mmol,1.2 eq) in N,N-dimethylformamide (5 mL) were added N,N,N',N'-tetramethyl-O-(7-azabenzothiadiazol-1-yl)hexafluorophosphorourea (1.8 g, 4.75 mmol,1.1 eq) and N,N-diisopropylethylamine (3.3 g, 25.9 mmol,6 eq). The mixture was stirred to react at room temperature for 0.5 h. After 0.5 h, the hydrochloride of 3-(7-chloro-3-vinylpyrazolo[1,5-a]pyridin-2-yl)prop-2-yn-1-amine (compound 363-3) (1 g, 4.32 mmol,1 eq) was added to the solution. The mixture was further stirred at room temperature for 2 h. After the reaction was completed, the reaction mixture was diluted with water (60 mL) at room temperature, and then extracted with ethyl acetate (50 mL×3). The organic phases were combined, backwashed with saturated brine (50 mL×3), dried over anhydrous sodium sulfate, filtered, and concentrated to give a crude product. The crude product was purified by silica gel column chromatography (methanol/dichloromethane (0-3%)) to give compound 5-amino-1-tert-butyl-N-(3-(7-chloro-3-vinylpyrazolo[1,5-a]pyridin-2-yl)prop-2-yn-1-yl)-1*H*-pyrazole-4-carboxamide **363-4** (1 g, 44.4% purity).

LCMS: (ESI, m/z): 398.2 [M+H]+.

**Step 5: Synthesis of 5-amino-1-tert-butyl-N-(3-(3-vinyl-7-{[(3S,4R)-3-fluoro-1-methylpiperidin-4-yl]amino}-pyrazolo[1,5-a]pyridin-2-yl)prop-2-yn-1-yl)-1*H*-pyrazole-4-carboxamide (compound 363)**

Under nitrogen protection, at room temperature, to a solution of 5-amino-1-tert-butyl-N-(3-(7-chloro-3-vinylpyrazolo[1,5-a]pyridin-2-yl)prop-2-yn-1-yl)-1*H*-pyrazole-4-carboxamide (compound 363-4, 600 mg, 1.51 mmol, 1 eq) and (3S,4R)-3-fluoro-1-methylpiperidin-4-amine (371.6 mg, 1.8 mmol, 1.2 eq) in 1,4-dioxane (10 mL) were added cesium carbonate (1.9 g, 6.1 mmol, 4 eq) and (SP-4-1)-[1,3-bis[2,6-bis(1-ethylpropyl)phenyl]-4,5-dichloro-1,3-dihydro-2*H*-imidazol-2-ylidene]dichloro(2-methylpyridine)palladium (127.0 mg, 0.15 mmol, 0.1 eq) in portions. After the addition was completed, the system was further stirred at 120°C for 2 h. After the reaction was completed, the reaction mixture was diluted with water (50 mL) at room temperature, and extracted with ethyl acetate (50 mL×3). The organic phases were combined, backwashed with saturated brine (50 mL), dried over anhydrous sodium sulfate, filtered, and concentrated to give a crude product. The crude product was purified by silica gel column chromatography (methanol/dichloromethane (0-5%)) to give a crude product. The crude product was further purified by HPLC with the following conditions: (Waters 2767/Qda) Column: XBridge XBridge C18 19*250mm, 10 μm; mobile phase A: 0.03% ammonia water, B: acetonitrile; flow rate: 20 mL/min; elution gradient: 53%-53%; retention time: 7.1-8.3 min, to give compound **5-amino-1-tert-butyl-N-[3-(3-vinyl-7-{[(3S,4R)-3-fluoro-1-methylpiperidin-4-yl]amino}-pyrazolo[1,5-a]pyridin-2-yl)prop-2-yn-1-yl)-1*H*-pyrazole-4-carboxamide 363** (45.28 mg, 6.02%).

LCMS: (ESI, m/z): 493.4 [M+H]+.

**Example 156**

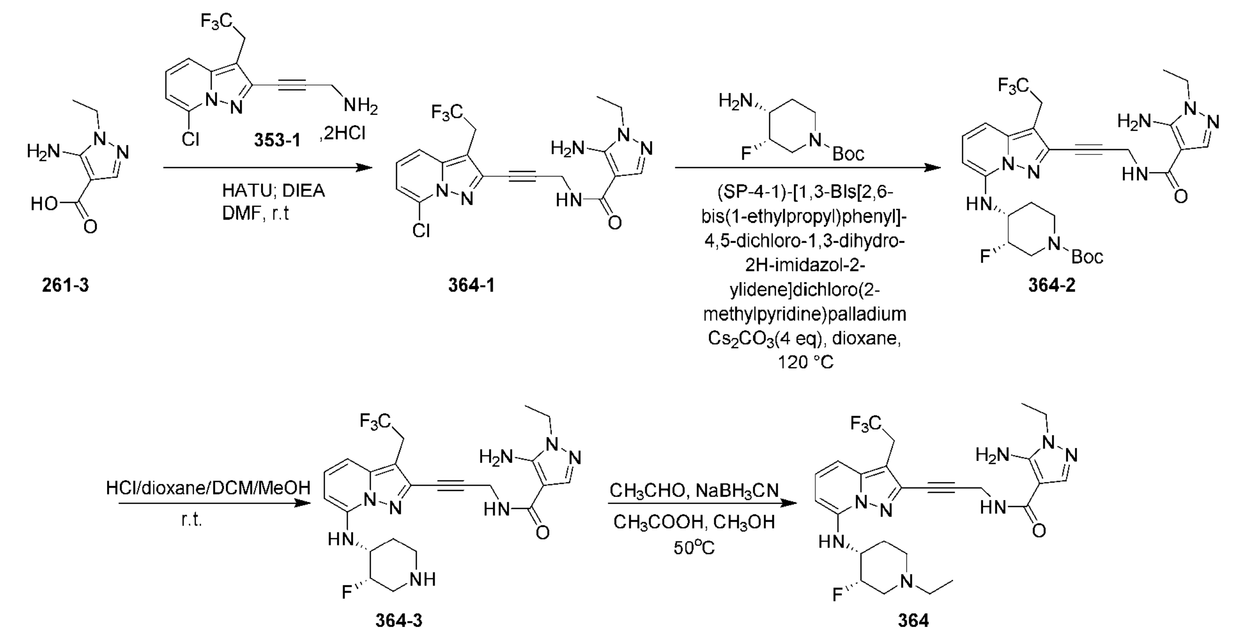

261-3
364-1
364-2
364-3
364

**Step 1: Synthesis of compound 364-1**

At room temperature, to a solution of 5-amino-1-(ethyl)-1*H*-pyrazole-4-carboxylic acid (compound 261-3, 200 mg, 1.3 mmol, 1 eq) in N,N-dimethylformamide (10 mL) were added N,N-diisopropylethylamine (1.4 mL, 7.7 mmol, 6 eq) and N,N,N',N'-tetramethyl-O-(7-azabenzothiadiazol-1-yl)hexafluorophosphorourea (539.3 mg, 1.4 mmol, 1.1 eq). After 30 min of reaction, a solution of 3-(7-chloro-3-(2,2,2-trifluoroethyl)pyrazolo[1,5-a]pyridin-2-yl)prop-2-yn-1-amine dihydrochloride (compound 353-1, 370 mg, 1.3 mmol, 1 eq) in N,N-dimethylformamide (4 mL) was added. After the addition was completed, the system was further stirred at room temperature for 1 h. After the reaction was completed, saturated ammonium chloride aqueous solution (50 mL) was added to quench the reaction. The system was extracted with ethyl acetate (50 mL × 3). The organic phases were combined, backwashed with saturated sodium chloride solution (30 mL × 2), dried over anhydrous sodium sulfate, filtered, and concentrated. The resulting crude product was purified by dry silica gel column chromatography (dichloromethane/methanol = 10 : 1) to give compound **364-1** (240 mg, 35.1%).
LCMS: (ESI, m/z): 425.1 $[M+H]^+$.

**Step 2: Synthesis of compound 364-2**

Under nitrogen protection, at room temperature, to a solution of 5-amino-N-(3-(7-chloro-3-(2,2,2-trifluoroethyl) pyrazolo[1,5-a]pyridin-2-yl)prop-2-yn-1-yl)-1-ethyl-1*H*-pyrazole-4-carboxamide (compound 364-1, 200 mg, 0.47 mmol, 1 eq) and tert-butyl (3S,4R)-4-amino-3-fluoropiperidine-1-carboxylate (133.7 mg, 0.61 mmol, 1.3 eq) in 1,4-dioxane (10 mL) were added cesium carbonate (614.6 mg, 1.89 mmol, 4 eq) and (SP-4-1)-[1,3-bis[2,6-bis(1-ethylpropyl)phenyl]-4,5-dichloro-1,3-dihydro-2H-imidazol-2-ylidene]dichloro(2-methylpyridine)palladium (39.6 mg, 0.05 mmol, 0.1 eq) in portions. After the addition was completed, the system was stirred to react at 120°C for 3 h. After the reaction was completed, the reaction mixture was quenched with water (50 mL) at room temperature, extracted with ethyl acetate (50 mL × 3), and the organic phases were combined, backwashed with saturated brine (50 mL × 2), dried over anhydrous sodium sulfate, filtered, and concentrated. The resulting crude product was purified by dry silica gel column chromatography (dichloromethane/methanol = 10:1) to give compound **364-2** (105 mg, 36.7%).
LCMS: (ESI, m/z): 607.2 $[M+H]^+$.

**Step 3: Synthesis of compound 364-3**

At room temperature, to a solution of tert-butyl (3S,4R)-4-((2-(3-(5-amino-1-ethyl-1*H*-pyrazole-4-carboxamido) prop-1-yn-1-yl)-3-(2,2,2-trifluoroethyl)pyrazolo[1,5-a]pyridin-7-yl)amino)-3-fluoropiperidine-1-carboxylate (compound 364-2, 105 mg, 0.17 mmol, 1 eq) in dichloromethane (3 mL) and methanol (3 mL) was added 4 mol/L hydrogen chloride in 1,4-dioxane (3 mL). After the addition was completed, the system was stirred to react at room temperature for 2 h. After the reaction was completed, the reaction mixture was concentrated under reduced pressure to give compound **364-3** (100 mg, crude product). The crude product was directly used in the next step of reaction.
LCMS: (ESI, m/z): 507.2 $[M+H]^+$.

**Step 4: Synthesis of 5-amino-1-ethyl-N-(3-(7-{[(3S,4R)-1-ethyl-3-fluoropiperidin-4-yl]amino}-3-(2,2,2-trifluoroethyl)pyrazolo[1,5-a]pyridin-2-yl)prop-2-yn-1-yl)-1*H*-pyrazole-4-carboxamide (compound 364)**

Under nitrogen protection, at room temperature, to a solution of 5-amino-1-ethyl-N-(3-(7-(((3S,4R)-3-fluoropiperidin-4-yl)amino)-3-(2,2,2-trifluoroethyl)pyrazolo[1,5-a]pyridin-2-yl)prop-2-yn-1-yl)-1*H*-pyrazole-4-carboxamide (compound 364-3, 75 mg, 0.15 mmol, 1 eq) in methanol (5 mL) were added a solution of acetaldehyde (0.44 mL, 5 M, 2.22 mmol, 15 eq) in tetrahydrofuran, acetic acid (0.89 mg, 0.02 mmol, 0.1 eq) and sodium cyanoborohydride (18.6 mg, 0.30 mmol, 2 eq). After the addition was completed, the system was stirred at 50°C overnight. After the reaction was completed, saturated aqueous ammonium chloride solution (30 mL) was added to the system to quench the reaction, and the system was extracted with ethyl acetate (30 mL × 3). The organic phases were combined, backwashed with saturated brine (30 mL × 2), dried over anhydrous sodium sulfate, filtered, and the filtrate was concentrated under reduced pressure. The system was purified by HPLC with the following conditions (Waters 2767/Qda, Column: Sunfire C18 19*250*10μm; mobile phase A: 0.1% formic acid/$H_2O$, B: acetonitrile; flow rate: 20 mL/min; elution gradient: 22-26%; retention time: 6.4-9.6 min) to give the carboxylate of compound **5-amino-1-ethyl-N-[3-(7-{[(3S,4R)-1-ethyl-3-fluoropiperidin-4-yl]amino}-3-(2,2,2-trifluoroethyl)pyrazolo[1,5-a]pyridin-2-yl)prop-2-yn-1-yl)-1*H*-pyrazole-4-carboxamide 364** (17.1 mg, 21.6%).
LCMS: (ESI, m/z): 535.3 $[M+H]^+$.

**Example 157**

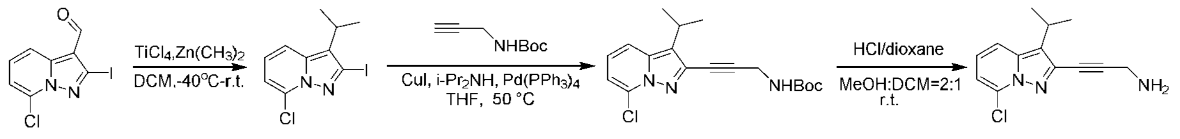


001-8 362-1 362-2 362-3

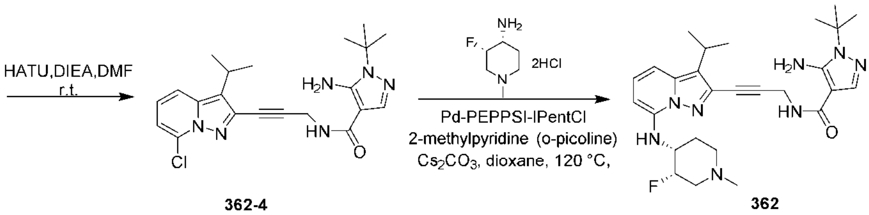


362-4 362

**Step 1: Synthesis of compound 362-1**

Under nitrogen protection, titanium tetrachloride (1.24 g, 6.52 mmol, 2 eq) was added to a dichloromethane (15 mL) solution at room temperature, and the reaction was cooled to -40°C. After the cooling was completed, dimethyl zinc (0.93 g, 9.78 mmol, 3 eq) was added to the mixture, and the mixture was further stirred at -40°C for 0.5 h. After 0.5 h, a solution of 7-chloro-2-iodopyrazolo[1,5-a]pyridine-3-carboxaldehyde (compound **001-8,** 1 g, 3.26 mmol, 1 eq) in dichloromethane (15 mL) was slowly added to the mixture. The reaction was gradually warmed to room temperature and stirred overnight. After the reaction was completed, the reaction mixture was slowly poured into ice water to quench the reaction, the pH of the aqueous phase was adjusted to acidic with concentrated hydrochloric acid, the system was extracted with ethyl acetate (3×50 mL), the organic phases were combined, backwashed with saturated brine (50 mL), dried over anhydrous sodium sulfate, filtered and concentrated, and the resulting crude product was purified by dry silica gel column chromatography (ethyl acetate/petroleum ether (0-10%)), to give compound **362-1** (0.8 g, 76.49%).
LCMS: (ESI, m/z): 321.0 $[M+H]^+$.

**Step 2: Synthesis of compound 362-2**

Under nitrogen protection, at room temperature, to a solution of 7-chloro-2-iodo-3-(prop-2-yl)pyrazolo[1,5-A] pyridine (compound 362-1, 800 mg, 2.50 mmol, 1 eq) and tert-butyl N-(prop-2-yn-1-yl)carbamate (580 mg, 3.75 mmol, 1.5 eq) in tetrahydrofuran (15 mL) were added cuprous iodide (48 mg, 0.25 mmol, 0.1 eq), diisopropylamine (2.53 g, 25 mmol, 10 eq), and tetrakis(triphenylphosphine)palladium (290 mg, 0.25 mmol, 0.1 eq) in portions. After the addition was completed, the system was stirred to react at 50°C for 12 h. After the reaction was completed, the reaction mixture was cooled to room temperature, then diluted with water (50 mL), and then extracted with ethyl acetate (3×50 mL). The organic phases were combined, backwashed with saturated brine (50 mL), dried over anhydrous sodium sulfate, filtered, and concentrated. The crude product was purified by dry silica gel column chromatography (ethyl acetate/petroleum ether (0-10%)) to give compound **362-2** (1 g).
LCMS: (ESI, m/z): 348.2 $[M+H]^+$.

**Step 3: Synthesis of compound 362-3**

A solution of hydrochloric acid in dioxane (4 M, 4 mL) was added to a mixed solution of tert-butyl N-(3-(7-chloro-3-(prop-2-yl)pyrazolo[1,5-a]pyridin-2-yl)prop-2-yn-1-yl)carbamate (362-2, 200 mg, 0.57 mmol, 1eq) in methanol (4 mL) and dichloromethane (2 mL). The mixture was stirred to react at room temperature for 3 h. After the reaction was completed, the reaction mixture was concentrated and spin dried to give the hydrochloride of compound **362-3** (190 mg, 133.40%). The crude product was directly used in the next step.
LCMS: (ESI, m/z): 248.2 $[M+H]^+$.

**Step 4: Synthesis of compound 362-4**

Under nitrogen protection, at 25°C, to a solution of 5-amino-1-tert-butyl-1*H*-pyrazole-4-carboxylic acid (130 mg,

0.68 mmol,1.2 eq) in N,N-dimethylformamide (S0,5mL) were added N,N,N',N'-tetramethyl-O-(7-azabenzothiadiazol-1-yl) hexafluorophosphorourea (240 mg, 0.63 mmol, 1.1 eq) and N,N-diisopropylethylamine (440 mg, 3.42 mmol, 6 eq). The mixture was further stirred at 25°C for 0.5 h. Then, the hydrochloride of 3-(7-chloro-3-(prop-2-yl)pyrazolo[1,5-a]pyridin-2-yl)prop-2-yn-1-amine (compound 362-3) (140 mg, 0.57mmol, 1 eq) was added, and the mixture was further stirred for 1 h. After the reaction was completed, the reaction mixture was diluted with water (50 mL) at room temperature, and then extracted with ethyl acetate (3×20 mL). The organic phases were combined, backwashed with saturated brine (3×50 mL), dried over anhydrous sodium sulfate, filtered, and concentrated. The crude product was purified by dry silica gel column chromatography (methanol/dichloromethane (0-5%)) to give compound **362-4** (200 mg, 85.70%).

LCMS: (ESI, m/z): 413.2 [M+H]+.

**Step 5: Synthesis of 5-amino-1-tert-butyl-N-(3-(7-{[(3S,4R)-3-fluoro-1-methylpiperidin-4-yl]amino}-3-(prop-2-yl) pyrazolo[1,5-a]pyridin-2-yl)prop-2-yn-1-yl)-1H-pyrazole-4-carboxamide (compound 362)**

Under nitrogen protection, at room temperature, to a solution of 5-amino-1-tert-butyl-N-(3-(7-chloro-3-(prop-2-yl) pyrazolo[1,5-a]pyridin-2-yl)prop-2-yn-1-yl)-1*H*-pyrazole-4-carboxamide (compound 362-4, 150 mg, 0.36 mmol, 1 eq) and (3S,4R)-3-fluoro-1-methylpiperidin-4-amine (57 mg, 0.43 mmol, 1.2 eq) in 1,4-dioxane (5 mL) were added cesium carbonate (470 mg, 1.44 mmol, 4 eq) and (SP-4-1)-[1,3-bis[2,6-bis(1-propylbutyl)phenyl]-4,5-dichloro-1,3-dihydro-2*H*-imidazol-2-ylidene]dichloro(3-chloropyridine-KN)palladium (36 mg, 0.036 mmol, 0.1 eq) in portions. The system was stirred at 120°C for 5 h. After the reaction was completed, the reaction mixture was diluted with water (50 mL) at room temperature, and extracted with ethyl acetate (3×20 mL). The organic phases were combined, backwashed with saturated brine (50 mL), dried over anhydrous sodium sulfate, filtered, and concentrated. The crude product was purified by dry silica gel column chromatography (methanol/dichloromethane (0-5%)) to give a crude product, which further purified by HPLC with the following conditions: (Waters 2767/Qda, Column: Sunfire C18 19*250*10μm; mobile phase A: 0.1% formic acid/water, B: acetonitrile; flow rate: 20 mL/min; gradient: 27-35%; retention time: 5.8-9.0, to give the carboxylate of compound **5-amino-1-tert-butyl-N-(3-(7-{[(3S,4R)-3-fluoro-1-methylpiperidin-4-yl]amino}-3-(prop-2-yl)pyrazolo[1,5-a]pyridin-2-yl)prop-2-yn-1-yl)-1*H*-pyrazole-4-carboxamide 362** (32.37 mg, 17.52%).

LCMS: (ESI, m/z): 509.3 [M+H]+.

$^1$H NMR (400 MHz, DMSO-d6) δ 8.30 (t, J = 5.6 Hz, 1H), 7.66 (s, 1H), 7.17 - 7.10 (m, 1H), 7.04 - 6.99 (m, 1H), 6.20 (d, J = 7.4 Hz, 1H), 6.18 (s, 2H), 6.11 (d, J = 9.2 Hz, 1H), 4.86 (d, J = 49.6 Hz, 1H), 4.28 (d, J = 5.6 Hz, 2H), 3.89 - 3.73 (m, 1H), 3.27 - 3.19 (m, 1H), 3.05 (t, J = 11.4 Hz, 1H), 2.77 (d, J = 11.2 Hz, 1H), 2.31 (dd, *J* = 22.8, 15.6 Hz, 1H), 2.20 (s, 3H), 2.17 - 2.09 (m, 1H), 1.96 - 1.81 (m, 2H), 1.52 (s, 9H), 1.36 - 1.29 (m, 6H).

**Example 158**

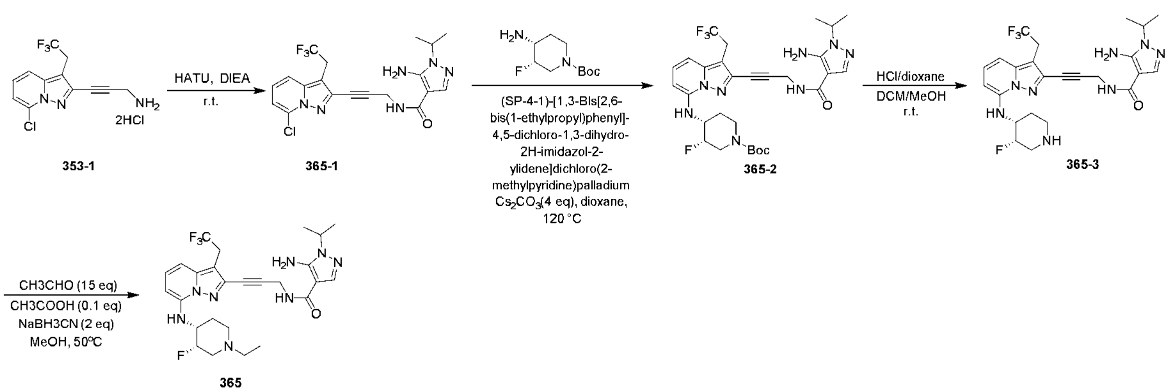


353-1 365-1 365-2 365-3 365

**Step 1: Synthesis of compound 365-1**

At room temperature, to a solution of 5-amino-1-isopropyl-1H-pyrazole-4-carboxylic acid (265.1 mg, 1.6 mmol, 1.5 eq) in N,N-dimethylformamide (10 mL) were added N,N-diisopropylethylamine (1.1 mL, 6.3 mmol, 6 eq) and N,N,N',N'-tetramethyl-O-(7-azabenzothiadiazol-1-yl)hexafluorophosphorourea (437 mg, 1.2 mmol, 1.1 eq). After 30 min of reaction, the dihydrochloride of 3-(7-chloro-3-(2,2,2-trifluoroethyl)pyrazolo[1,5-a]pyridin-2-yl)prop-2-yn-1-amine (compound 353-1, 300 mg, 1.1 mmol, 1 eq) was added. After the addition was completed, the system was further stirred at room temperature for 1.5 h. After the reaction was completed, the reaction mixture was quenched with saturated ammonium

chloride aqueous solution (30 mL), and extracted with ethyl acetate (30 mL × 3). The organic phases were combined, backwashed with saturated brine (30 mL × 2), dried over anhydrous sodium sulfate, filtered, and concentrated. The crude product was purified by dry silica gel column chromatography (dichloromethane/methanol = 10:1) to give compound **365-1** (330 mg, 72%).

LCMS: (ESI, m/z): 439.1 $[M+H]^+$.

**Step 2: Synthesis of compound 365-2**

Under nitrogen protection, at room temperature, to a solution of 5-amino-N-(3-(7-chloro-3-(2,2,2-trifluoroethyl) pyrazolo[1,5-a]pyridin-2-yl)prop-2-yn-1-yl)-1-isopropyl-1*H*-pyrazole-4-carboxamide (compound 365-1, 300 mg, 0.68 mmol, 1 eq) and tert-butyl (3S,4R)-4-amino-3-fluoropiperidine-1-carboxylate (224 mg, 1.03 mmol, 1.5 eq) in 1,4-dioxane (10 mL) wer added cesium carbonate (892 mg, 2.74 mmol, 4 eq) and (SP-4-1)-[1,3-bis[2,6-bis(1-ethylpropyl)phenyl]-4,5-dichloro-1,3-dihydro-2*H*-imidazol-2-ylidene]dichloro(2-methylpyridine)palladium (57.5 mg, 0.03 mmol, 0.1 eq) in portions. The mixture was stirred to react at 120°C for 3 h. After the reaction was completed, the reaction mixture was quenched with water (30 mL) at room temperature, and extracted with ethyl acetate (30 mL × 3). The organic phases were combined, backwashed with saturated brine (30 mL × 2), dried over anhydrous sodium sulfate, filtered, and concentrated. The crude product was purified by dry silica gel column chromatography (dichloromethane/methanol = 10:1) to give compound **365-2** (110 mg, 26%).

LCMS: (ESI, m/z): 621.2 $[M+H]^+$.

**Step 3: Synthesis of compound 365-3**

At room temperature, to a solution of tert-butyl (3S,4R)-4-((2-(3-(5-amino-1-isopropyl-1*H*-pyrazole-4-carboxamido)prop-1-yn-1-yl)-3-(2,2,2-trifluoroethyl)pyrazolo[1,5-a]pyridin-7-yl)amino)-3-fluoropiperidine-1-carboxylate (compound 365-2, 110 mg, 0.18 mmol, 1 eq) in dichloromethane (3 mL) and methanol (3 mL) was added 4 mol/L hydrogen chloride in 1,4-dioxane (3 mL). After the addition was completed, the system was stirred to react at room temperature for 2 h. After the reaction was completed, the mixture was concentrated under reduced pressure to give the hydrochloride of compound **365-3** (120 mg, crude product). The crude product was directly used in the next step.

LCMS: (ESI, m/z): 521.2 $[M+H]^+$.

**Step 4: Synthesis of 5-amino-N-(3-(7-{[(3S,4R)-1-ethyl-3-fluoropiperidin-4-yl]amino}-3-(2,2,2-trifluoroethyl)pyrazolo[1,5-a]pyridin-2-yl)prop-2-yn-1-yl)-1-isopropyl-1*H*-pyrazole-4-carboxamide (compound 365)**

Under nitrogen protection, at room temperature, to a solution of 5-amino-N-(3-(7-(((3S,4R)-3-fluoropiperidin-4-yl)amino)-3-(2,2,2-trifluoroethyl)pyrazolo[1,5-a]pyridin-2-yl)prop-2-yn-1-yl)-1-isopropyl-1*H*-pyrazole-4-carboxamide (compound 365-3, 90 mg, 0.17 mmol, 1 eq) in methanol (10 mL) were added a solution of acetaldehyde (0.52 mL, 5 M, 2.60 mmol, 15 eq) in tetrahydrofuran, acetic acid (1.04 mg, 0.02 mmol, 0.1 eq) and sodium cyanoborohydride (21.7 mg, 0.35 mmol, 2 eq). After the addition was completed, the system was stirred at 50°C overnight. After the reaction was completed, saturated aqueous ammonium chloride solution (30 mL) was added to quench the reaction, and the system was extracted with ethyl acetate (30 mL × 3). The organic phases were combined, backwashed with saturated brine (30 mL × 2), dried over anhydrous sodium sulfate, filtered, and concentrated. The system was purified by HPLC with the following conditions (Waters 2767/Qda, Column: Sunfire C18 19*250*10μm; mobile phase A: 0.1% formic acid/water, B: acetonitrile; flow rate: 20 mL/min; elution gradient: 25-29%; retention time: 5.3-7.2 min) to give the carboxylate of compound **5-amino-N-[3-(7-{[(3S,4R)-1-ethyl-3-fluoropiperidin-4-yl]amino}-3-(2,2,2-trifluoroethyl)pyrazolo[1,5-a]pyridin-2-yl) prop-2-yn-1-yl)-1-isopropyl-1*H*-pyrazole-4-carboxamide 365** (16.16 mg, 17%).

LCMS: (ESI, m/z): 549.2 $[M+H]^+$.

$^1$H NMR (400 MHz, DMSO-$d_6$) $\delta$ 8.27 (t, J = 5.7 Hz, 1H), 7.70 (s, 1H), 7.31 - 7.24 (m, 1H), 7.02 (d, *J* = 8.8 Hz, 1H), 6.31 (d, *J* = 7.6 Hz, 1H), 6.24 (d, J = 9.1 Hz, 1H), 6.21 (s, 2H), 4.88 (d, *J* = 50.0 Hz, 1H), 4.44 - 4.37 (m, 1H), 4.28 (d, *J* = 5.6 Hz, 2H), 3.92 - 3.81 (m, 1H), 3.75 (q, *J* = 11.2 Hz, 2H), 3.17 - 3.11 (m, 1H), 2.88 (d, *J* = 12.0 Hz, 1H), 2.41 - 2.36 (m, 2H), 2.34 - 2.22 (m, 1H), 2.16 - 2.11 (m, 1H), 1.97 - 1.82 (m, 2H), 1.29 (d, *J* = 6.4 Hz, 6H), 1.01 (t, *J* = 7.2 Hz, 3H).

**Example 159**

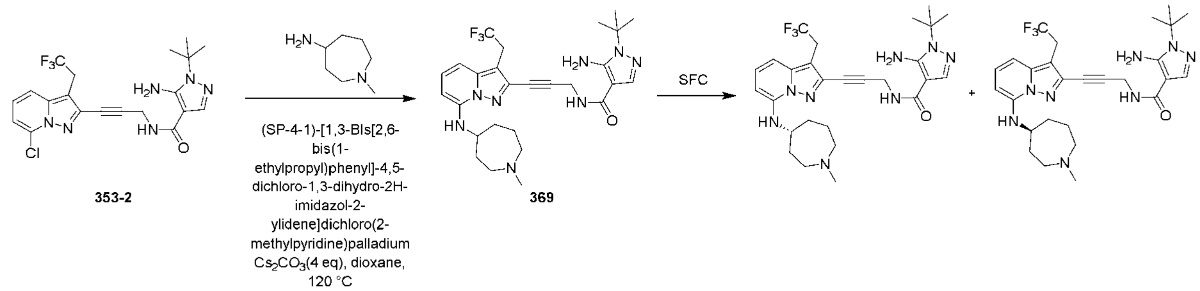

353-2 369

**Step 1: Synthesis of 5-amino-1-tert-butyl-N-(3-(7-[(1-methylazepan-4-yl)amino]-3-(2,2,2-trifluoroethyl)pyrazolo [1,5-a]pyridin-2-yl)prop-2-yn-1-yl)-1H-pyrazole-4-carboxamide (compound 369)**

Under nitrogen protection, at room temperature, to a solution of 5-amino-1-tert-butyl-N-(3-(7-chloro-3-(2,2,2-trifluoroethyl)pyrazolo[1,5-a]pyridin-2-yl)prop-2-yn-1-yl)-1*H*-pyrazole-4-carboxamide (compound 353-2, 150 mg, 0.33 mmol, 1 eq) and 1-methylazepan-4- amine (51 mg, 0.4 mmol, 1.5 eq) in 1,4-dioxane (5 mL) were added cesium carbonate (430 mg, 1.32 mmol, 4 eq) and (SP-4-1)-[1,3-bis[2,6-bis(1-ethylpropyl)phenyl]-4,5-dichloro-1,3-dihydro-2*H*-imidazol-2-ylidene]dichloro(2-methylpyridine)palladium (33 mg, 0.033 mmol, 0.1 eq) in portions. After the addition was completed, the system was further stirred at 120°C for 1.5 h. After the reaction was completed, the reaction mixture was quenched with water (50 mL) at room temperature, and extracted with ethyl acetate (3×60 mL). The organic phases were combined, backwashed with saturated brine (3×50 mL), dried over anhydrous sodium sulfate, filtered, and concentrated. The crude product was purified by silica gel column chromatography (methanol/dichloromethane (0-10%)), to give compound **5-amino-1-tert-butyl-N-(3-(7-[(1-methylazepan-4-yl)amino]-3-(2,2,2-trifluoroethyl)pyrazolo[1,5-a]pyridin-2-yl) prop-2-yn-1-yl)-1*H*-pyrazole-4-carboxamide 369** (35 mg, 19.4%).

The system was subjected to chiral resolution, SFC resolution **(resolution conditions:** Waters SFC 150; column : DAICELCHIRALCEL®OD; specifications: 250*30 mm 10 μm; mobile phase: **A** supercritical $CO_2$, **B**: MeOH(+0.1% 7.0 mol/L ammonia methanol solution); **A:B:** 80:20; wavelength: 214 nm; **flow rate:** 120 mL/min; column temperature: RT; **back pressure:** 100 bar; **injection volume:** 1.0 mL; cycle time: 11.69 min; solvent: MeOH: distilled grade, supercritical $CO_2$: food grade), and freeze-dried to give compounds **369-A** (retention time: 1.933 min, 13.65 mg, 17.2%) and **369-B** (retention time: 3.326 min, 13.71 mg).

Front peak **369-A:** LCMS:(ESI, m/z): 545.3 $[M+H]^+$.

$^1$H NMR (400 MHz, DMSO-$d_6$) $\delta$ 8.31 (t, *J* = 5.6 Hz, 1H), 7.65 (s, 1H), 7.29 - 7.21 (m, 1H), 6.93 (d, *J*= 8.8 Hz, 1H), 6.64 (d, *J*= 8.8 Hz, 1H), 6.18 (s, 2H), 6.05 (d, *J* = 7.6 Hz, 1H), 4.27 (d, *J* = 5.6 Hz, 2H), 3.90 - 3.78 (m, 1H), 3.73 (q, J = 11.2 Hz, 2H), 2.70 - 2.61 (m, 1H), 2.60 - 2.53 (m, 2H), 2.28 (s, 3H), 2.02 - 1.54 (m, 7H), 1.52 (s, 9H).

Rear peak **369-B:** LCMS:(ESI, m/z): 545.3 $[M+H]^+$.

$^1$H NMR (400 MHz, DMSO-$d_6$) $\delta$ 8.31 (t, *J* = 5.6 Hz, 1H), 7.65 (s, 1H), 7.29 - 7.21 (m, 1H), 6.93 (d, *J*= 8.8 Hz, 1H), 6.64 (d, *J*= 8.8 Hz, 1H), 6.18 (s, 2H), 6.05 (d, *J* = 7.6 Hz, 1H), 4.27 (d, *J* = 5.6 Hz, 2H), 3.90 - 3.78 (m, 1H), 3.73 (q, J = 11.2 Hz, 2H), 2.70 - 2.61 (m, 1H), 2.60 - 2.53 (m, 2H), 2.28 (s, 3H), 2.02 - 1.54 (m, 7H), 1.52 (s, 9H).

**Example 160**

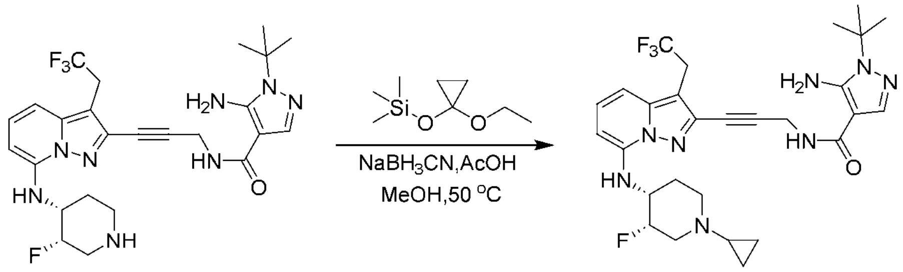


353-4 372

**Step 1: Synthesis of 5-amino-1-tert-butyl-N-(3-(7-{[(3S,4R)-1-cyclopropyl-3-fluoropiperidin-4-yl]amino}-3-(2,2,2-trifluoroethyl)pyrazolo[1,5-a]pyridin-2-yl)prop-2-yn-1-yl)-1*H*-pyrazole-4-carboxamide (compound 372)**

Under nitrogen protection, at room temperature, to a solution of 5-amino-1-tert-butyl-N-(3-(7-chloro-3-(2,2,2-trifluoroethyl)pyrazolo[1,5-a]pyridin-2-yl)prop-2-yn-1-yl)-1*H*-pyrazole-4-carboxamide (compound 353-4, 100 mg, 0.19 mmol, 1 eq) and (1-ethoxycyclopropoxy)trimethylsilane (130 mg, 0.76 mmol, 4 eq) in methanol (5 mL) were added glacial acetic acid (100 mg, 1.71 mmol, 9 eq) and sodium cyanoborohydride (160 mg, 2.47 mmol, 13 eq) in portions. After the addition was completed, the system was stirred to react at 50°C for 12 h. After the reaction was completed, water (50 mL) was added to the reaction mixture to quench the reaction, and the system was extracted with ethyl acetate (3×20 mL). The organic phases were combined, backwashed with saturated brine (50 mL), dried over anhydrous sodium sulfate, filtered, and concentrated. The crude product was purified by silica gel column chromatography (methanol/dichloromethane (0-10%)), to give a crude product, which was then purified by HPLC with the following conditions (Waters 2767/Qda, Column: Sunfire C18 19*250*10μm; mobile phase A: 0.1% formic acid/water, B: acetonitrile; flow rate: 20mL/min; elution gradient: 30-30%; retention time: 7.6-8.7 min) to give compound **5-amino-1-tert-butyl-N-(3-(7-{[(3S,4R)-1-cyclopropyl-3-fluoropiperidin-4-yl]amino}-3-(2,2,2-trifluoroethyl)pyrazolo[1,5-a]pyridin-2-yl)prop-2-yn-1-yl)-1*H*-pyrazole-4-carboxamide 372** (13.42 mg, 12.48%).

LCMS: (ESI, m/z): 575.3 $[M+H]^+$.

$^1$H NMR (400 MHz, DMSO-d6) δ 8.30 (t, *J* = 5.6 Hz, 1H), 7.65 (s, 1H), 7.28 (t, *J* = 8.0 Hz, 1H), 7.02 (d, *J* = 8.8 Hz, 1H), 6.32 (d, *J* = 7.6 Hz, 1H), 6.24 (d, *J* = 8.8 Hz, 1H), 6.17 (s, 2H), 4.87 (d, *J* = 49.6 Hz, 1H), 4.28 (d, *J* = 5.6 Hz, 2H), 3.99 - 3.80 (m, 1H), 3.75 (q, *J* = 10.8 Hz, 2H), 3.19 (t, J = 10.8 Hz, 1H), 2.95 (d, *J* = 11.2 Hz, 1H), 2.65 - 2.53 (m, 1H), 2.47 - 2.37 (m, 1H), 1.92 - 1.81 (m, 2H), 1.73 - 1.65 (m, 1H), 1.52 (s, 9H), 0.49 - 0.39 (m, 2H), 0.38 - 0.23 (m, 2H).

**Example 161**

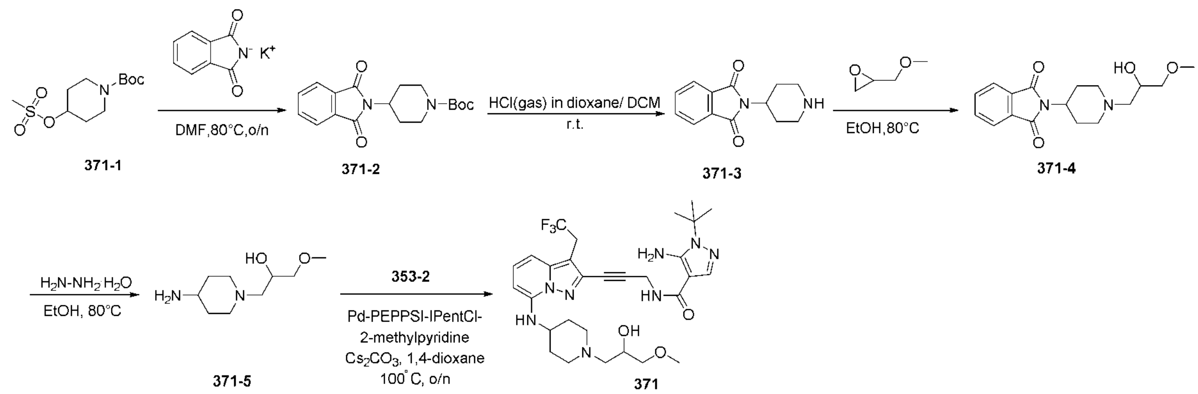


**Step 1: Synthesis of compound 371-2**

Under nitrogen protection, a solution of tert-butyl 4-(methylsulfonyloxy)piperidine-1-carboxylate (compound 371-1, 2.0 g, 7.168 mmol, 1 eq) in N,N-dimethylformamide (20 mL) was added to a reaction flask at room temperature, and potassium phthalimide (2.1 g, 11.278 mmol, 1.5 eq, 95%) was added. The mixture was stirred at 80°C overnight. The reaction mixture was quenched with water at room temperature, extracted with dichloromethane (3 × 20 mL), the organic phases were combined, backwashed with saturated sodium chloride (3 × 10 mL), dried over anhydrous sodium sulfate, filtered, and the filtrate was concentrated under reduced pressure. The resulting residue was purified by silica gel column chromatography (petroleum ether\ethyl acetate = 10:1) to give compound **371-2** (500 mg, 21.15%).

LCMS: (ESI, m/z): 275.05 $[M+H]^+$.

**Step 2: Synthesis of compound 371-3**

Under nitrogen protection, at room temperature, a solution of tert-butyl 4-(1,3-dioxoisoindol-2-yl)piperidine-1-carboxylate (compound 371-2, 500 mg, 1.513 mmol, 1 eq) in dichloromethane solution (2.5 mL) was added to a reaction flask. Then, a solution of hydrochloric acid in 1,4-dioxane (2.5 mL, 4M) was added and the mixture was stirred at room temperature for 2 h. After the reaction was completed, the reaction mixture was neutralized with 2M sodium hydroxide

solution to pH=7, and then extracted with ethyl acetate (3 × 10 mL). The organic phases were combined, backwashed with saturated sodium chloride solution (3 × 10 mL), dried over anhydrous sodium sulfate, filtered, and the filtrate was concentrated under reduced pressure to give compound **371-3** (300 mg, 86.09%).

LCMS: (ESI, m/z): 231.05 $[M+H]^+$.

**Step 3: Synthesis of compound 371-4**

Under nitrogen protection, to a solution of 2-(piperidin-4-yl)isoindole-1,3-dione (compound 371-3, 200 mg, 0.869 mmol, 1 eq) in anhydrous ethanol (5 mL) was added (methoxymethyl)-oxirane (459.15 mg, 5.214 mmol, 6 eq) at room temperature, and the mixture was warmed to 80°C and stirred for 2 h. After the reaction was completed, the resulting residue was concentrated under reduced pressure and the crude product was purified by silica gel column chromatography (dichloromethane\methanol=10:1) to give compound **371-4** (120 mg, 43.40%).

LCMS: (ESI, m/z): 319.05 $[M+H]^+$.

**Step 4: Synthesis of compound 371-5**

Under nitrogen protection, at room temperature, a solution of 2-[1-(2-hydroxy-3-methoxypropyl)piperidin-4-yl] isoindole-1,3-dione (compound 371-4, 100 mg, 0.314 mmol, 1.00 eq) in ethanol (1.5 mL) was added to a reaction flask. Hydrazine hydrate (196.12 μL, 3.429 mmol, 10.92 eq, 85%) was added dropwise to the above system at room temperature. After the addition was completed, the system was further stirred at 80°C for 30 min. After the reaction was completed, the resulting residue was concentrated under reduced pressure, the reaction mixture was washed with diethyl ether, filtered, the filter cake was washed with diethyl ether (3 × 10 mL), and the filtrate was concentrated under reduced pressure to give compound **371-5** (55 mg, 93.01%).

**Step 5: Synthesis of 5-amino-1-tert-butyl-*N*-[3-(7-{[1-(2-hydroxy-3-methoxypropy)piperidin-4-yl]amino}-3-(2,2,2-trifluoroethyl)pyrazolo[1,5-*a*]pyridin-2-yl)prop-2-yn-1-yl]-1*H*-pyrazole-4-carboxamide (compound 371)**

Under nitrogen protection, at room temperature, 5-amino-1-tert-butyl-*N*-{3-[7-chloro-3-(2,2,2-trifluoroethyl)pyrazolo[1,5-*a*]pyridin-2-yl]prop-2-ynyl-1-yl}pyrazole-4-carboxamide (compound 353-2, 40 mg, 0.088 mmol, 1 eq) and 1-(4-aminopiperidin-1-yl)-3-methoxy-2-propanol (compound 371-5, 33.26 mg, 0.176 mmol, 2 eq) in 1,4-Dioxane (1.0 mL) were added cesium carbonate (115.11 mg, 0.352 mmol, 4 eq) and (SP-4-1)-[1,3-bis[2,6-bis(1-ethylpropyl)phenyl]-4,5-dichloro-1,3-dihydro-2*H*-imidazol-2-ylidene]dichloro(2-methylpyridine)palladium (7.43 mg, 0.009 mmol, 0.1 eq) in portions, and the mixture was stirred at 100°C overnight. After the reaction was completed, the reaction mixture was quenched with methanol (1.0 mL) at room temperature. The crude product was purified by HPLC with the following conditions (column specifications: Sunfire C18 5 μm, 30 mm * 150 mm; mobile phase A: (0.1% formic acid), mobile phase B: acetonitrile; flow rate: 60 mL/min; elution gradient: 16% B to 35% B in 8 min; detection wavelength: UV 254 nm/220 nm; retention time (min): 6.75), to give the carboxylate of compound **371** (2.78 mg, 4.79%).

LCMS: (ESI, m/z): 605.10 $[M+H]^+$.

$^1H$ NMR (400 MHz, DMSO-$d_6$,*ppm*) δ 8.31 (t, *J* = 5.8 Hz, 1H), 7.65 (s, 1H), 7.24 (dd, *J* = 8.7, 7.7 Hz, 1H), 6.93 (d, *J* = 8.2 Hz, 1H), 6.61 (d, *J* = 8.4 Hz, 1H), 6.18 (s, 2H), 6.16 - 6.13 (m, 1H), 4.52 (s, 1H), 4.27 (d, *J* = 5.7 Hz, 2H), 3.72 (dd, *J* = 13.8, 8.5 Hz, 3H), 3.46 (t, *J* = 11.5 Hz, 1H), 3.29 (d, *J* = 4.4 Hz, 1H), 3.25 (s, 3H), 3.23 - 3.20 (m, 1H), 2.93 - 2.83 (m, 2H), 2.37 - 2.31 (m, 1H), 2.26 (dd, *J* = 12.7, 7.1 Hz, 1H), 2.15 (q, *J* = 10.3 Hz, 2H), 1.90 (d, *J* = 12.2 Hz, 2H), 1.68 (q, *J*= 11.1, 10.3 Hz, 2H), 1.52 (s, 9H).

**Example 162**

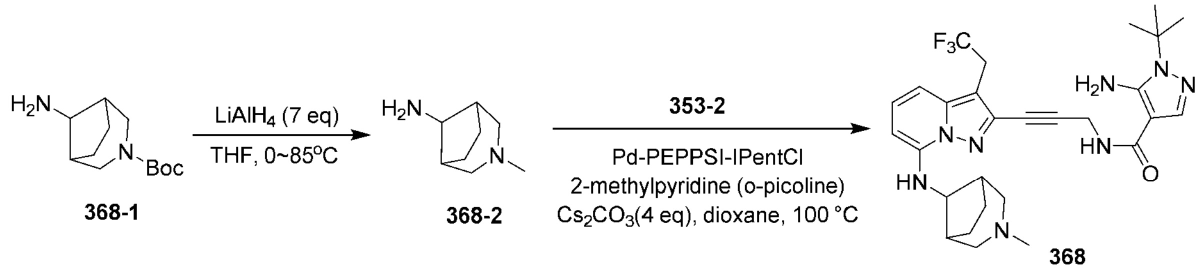


368-1 368-2 368

**Step 1: Synthesis of compound 368-2**

Under nitrogen protection, to a solution of tert-butyl 8-amino-3-azabicyclo[3.2.1]octane-3-carboxylate (compound 368-1, 500 mg, 2.2 mmol, 1 eq) in tetrahydrofuran (10 mL) was added lithium aluminum hydride (675 mg, 17.8 mmol, 7 eq) at 0°C. After the addition was completed, the system was allowed to react at 0°C for 30 min and then further stirred to react at 85°C for 3 h. After the reaction was completed, water (1 mL) and 8% sodium hydroxide solution (1 mL) were slowly added dropwise to the system at 0°C to quench the reaction, and the mixture was filtered. The filter cake was washed with ethyl acetate (30 mL), and the liquid phase was dried over anhydrous sodium sulfate, filtered, and concentrated to give crude product compound **368-2** (260 mg). The crude product was directly used in the next step.

**Step 2: Synthesis of 5-amino-1-(tert-butyl)-N-(3-(7-((3-methyl-3-azabicyclo[3.2.1]octan-8-yl)amino)-3-(2,2,2-trifluoroethyl)pyrazolo[1,5-a]pyridin-2-yl)prop-2-yn-1-yl)-1*H*-pyrazole-4-carboxamide (compound 368)**

Under nitrogen protection, at room temperature, to a solution of 5-amino-1-tert-butyl-N-(3-(7-chloro-3-(2,2,2-trifluoroethyl)pyrazolo[1,5-a]pyridin-2-yl)prop-2-yn-1-yl)-1*H*-pyrazole-4-carboxamide (353-2, 100 mg, 0.22 mmol, 1 eq) and 3-methyl-3-azabicyclo[3.2.1]octane-8-amine (120 mg, crude product) in 1,4-dioxane (10 mL) were added cesium carbonate (289 mg, 0.88 mmol, 4 eq) and (SP-4-1)-[1,3-bis[2,6-bis(1-propylbutyl)phenyl]-4,5-dichloro-1,3-dihydro-2*H*-imidazol-2-ylidene]dichloro(3-chloropyridine-KN)palladium (21 mg, 0.02 mmol, 0.1 eq) in portions. After the addition was completed, the system was stirred at 100°C overnight. After the reaction was completed, the reaction mixture was quenched with water (30 mL) at room temperature, and extracted with ethyl acetate (30 mL × 3). The organic phases were combined, backwashed with saturated brine (30 mL × 3), dried over anhydrous sodium sulfate, filtered, and concentrated. The system was purified by HPLC with the following conditions (Waters 2767/Qda, Column: Sunfire C18 19*250*10μm; mobile phase A: 0.1% formic acid/water, B: acetonitrile; flow rate: 20 mL/min; elution gradient: 21-23%; retention time: 5.4-8.2 min) to give compound **368** (24.3 mg, 13.2%).

LCMS: (ESI, m/z): 557.5 $[M+H]^+$.

$^1$H NMR (400 MHz, DMSO-$d_6$) δ 8.31 (t, *J* = 5.6 Hz, 1H), 7.65 (s, 1H), 7.30 - 7.24 (m, 1H), 6.99 (d, *J* = 8.8 Hz, 1H), 6.22 (d, *J* = 4.4 Hz, 1H), 6.18 (s, 2H), 6.12 (d, *J* = 7.2 Hz, 1H), 4.28 (d, *J* = 5.6 Hz, 2H), 3.74 (q, *J* = 11.2 Hz, 2H), 3.47 (s, 1H), 2.69 - 2.67 (m, 2H), 2.40 (s, 2H), 2.20 - 2.15 (m, 5H), 1.84 - 1.76 (m, 2H), 1.69 - 1.64 (m, 2H), 1.52 (s, 9H).

**Example 163**

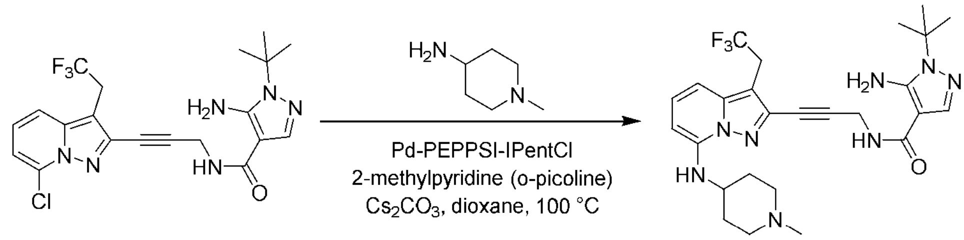


**353-2** **370**

**Step 1: Synthesis of 5-amino-1-tert-butyl-N-(3-(7-[(1-methylpiperidin-4-yl)amino]-3-(2,2,2-trifluoroethyl)pyrazolo[1,5-a]pyridin-2-yl)prop-2-yn-1-yl)-1*H*-pyrazole-4-carboxamide (compound 370)**

Under nitrogen protection, at room temperature, to a solution of 5-amino-1-tert-butyl-N-(3-(7-chloro-3-(2,2,2-trifluoroethyl)pyrazolo[1,5-a]pyridin-2-yl)prop-2-yn-1-yl)-1*H*-pyrazole-4-carboxamide (compound 353-2, 100 mg, 0.22 mmol, 1 eq) and 1-methylpiperidin-4-amine (37.8 mg, 0.33 mmol, 1.5 eq) in 1,4-dioxane (5 mL) were added cesium carbonate (290 mg, 0.88 mmol, 4 eq) and (SP-4-1)-[1,3-bis[2,6-bis(1-propylbutyl)phenyl]-4,5-dichloro-1,3-dihydro-2*H*-imidazol-2-ylidene]dichloro(3-chloropyridine-KN)palladium (20 mg, 0.02 mmol, 0.1 eq) in portions. After the addition was completed, the system was stirred at 100°C for 12 h. After the reaction was completed, the reaction mixture was diluted with water (20 mL), and then extracted with ethyl acetate (3×20 mL). The organic phases were combined, backwashed with saturated brine (20 mL), dried over anhydrous sodium sulfate, filtered, and concentrated to give a crude product. The crude product was purified by silica gel column chromatography (methanol/dichloromethane (0-10%)), to give compound **370** (22.77 mg, 17.7%).

LCMS: (ESI, m/z): 531.4 $[M+H]^+$.

$^1$H NMR (400 MHz, DMSO-d6) δ 8.31 (t, J = 5.6 Hz, 1H), 7.65 (s, 1H), 7.28 - 7.21 (m, 1H), 6.93 (d, J = 8.8 Hz, 1H),

6.60 (d, J = 8.8 Hz, 1H), 6.18 (s, 2H), 6.15 (d, J = 7.3 Hz, 1H), 4.27 (d, J = 6.0 Hz, 2H), 3.73 (q, J = 11.2 Hz, 2H), 3.54 - 3.41 (m, 1H), 2.76 (d, J = 11.2 Hz, 2H), 2.19 (s, 3H), 2.06 (t, J = 10.8 Hz, 2H), 1.89 (d, J = 10.8 Hz, 2H), 1.77 - 1.64 (m, 2H), 1.52 (s, 9H).

**Example 164**

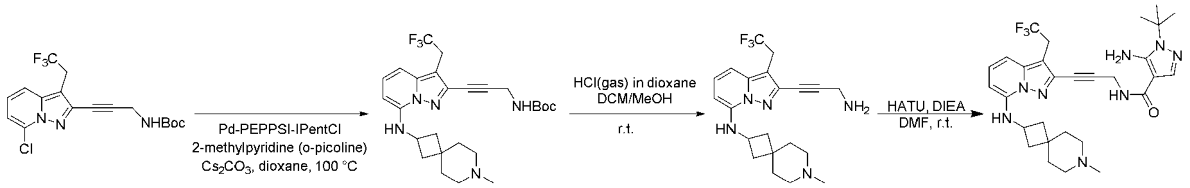


001-10 367-1 367-2 367

**Step 1: Synthesis of compound 367-1**

Under nitrogen protection, at room temperature, to a solution of tert-butyl {3-[7-chloro-3-(2,2,2-trifluoroethyl) pyrazolo[1,5-a]pyridin-2-yl]prop-2-yn-1-yl}carbamate (compound 001-10, 200 mg, 0.5 mmol, 1 eq) and 7-methyl-7-azaspiro[3.5]nonane-2-amine (120 mg, crude product) in 1,4-dioxane (10 mL) were added cesium carbonate (675 mg, 2.1 mmol, 4 eq) and (SP-4-1)-[1,3-bis[2,6-bis(1-propylbutyl)phenyl]-4,5-dichloro-1,3-dihydro-2*H*-imidazol-2-ylidene] dichloro(3-chloropyridine-KN)palladium (50.0 mg, 0.05 mmol, 0.1 eq). After the addition was completed, the system was stirred at 100°C overnight. After the reaction was completed, the reaction mixture was quenched with water (50 mL) at room temperature, extracted with ethyl acetate (50 mL × 3), and the organic phases were combined, backwashed with saturated brine (50 mL × 3), dried over anhydrous sodium sulfate, filtered, and concentrated. The resulting crude product was purified by silica gel column chromatography (dichloromethane/methanol = 10:1) to give compound **367-1** (90 mg, 34.5%).

LCMS: (ESI, m/z): 506.3 $[M+H]^+$.

**Step 2: Synthesis of compound 367-2**

At room temperature, to a solution of tert-butyl (3-(7-((7-methyl-7-azaspiro[3.5]nonan-2-yl)amino)-3-(2,2,2-trifluoroethyl)pyrazolo[1,5-a]pyridin-2-yl)prop-2-yn-1-yl)carbamate (compound 367-2, 90 mg, 0.2 mmol, 1 eq) in dichloromethane (3 mL) and methanol (3 mL) was added dropwise 4 mol/L hydrogen chloride in 1,4-dioxane (3 mL). After the addition was completed, the system was stirred to react at room temperature for 2 h. After the reaction was completed, the reaction mixture was concentrated under reduced pressure to give compound **367-2** (70 mg, 97.0%).

LCMS: (ESI, m/z): 406.3 $[M+H]^+$.

**Step 3: Synthesis of 5-amino-1-(tert-butyl)N-(3-(7-((7-azaspiro[3.5]nonan-2-yl)amino)-3-(2,2,2-trifluoroethyl) pyrazolo[1,5-a]pyridin-2-yl)prop-2-yn-1-yl)-1*H*-pyrazole-4-carboxamide (compound 367)**

Under nitrogen protection, at room temperature, to a solution of 5-amino-1-(tert-butyl)-1*H*-pyrazole-4-carboxylic acid (47.4 mg, 0.3 mmol, 1.5 eq) in N,N-dimethylformamide (10 mL) was added N,N-diisopropylethylamine (0.2 mL, 1.0 mmol, 6 eq) and 2-(7-azobenzotriazole)-N,N,N',N'-tetramethyluronium hexafluorophosphate (72.3 mg, 0.2 mmol, 1.1 eq). After 30 min of reaction, 2-(3-aminoprop-1-yn-1-yl)-N-(7-methyl-7-azaspiro[3.5]nonan-2-yl)-3-(2,2,2-trifluoroethyl)pyrazolo[1,5-a]pyridin-7-amine (compound 367-2, 70 mg, 0.2 mmol, 1 eq) was added. After the addition was completed, the system was further stirred at room temperature for 2 h. After the reaction was completed, the reaction mixture was quenched with saturated ammonium chloride aqueous solution (30 mL), extracted with ethyl acetate (30 mL × 3), and the organic phases were combined, backwashed with saturated brine (30 mL × 3), dried over anhydrous sodium sulfate, filtered, and concentrated. The resulting crude product was purified by silica gel column chromatography (dichloromethane/methanol = 10:1) to give compound **367** (31.2 mg, 31.68%).

LCMS: (ESI, m/z): 571.5 $[M+H]^+$.

$^1$H NMR (400 MHz, DMSO-$d_6$) $\delta$ 8.32 (t, *J* = 5.6 Hz, 1H), 7.65 (s, 1H), 7.23 (t, *J* = 8.4 Hz, 1H), 7.06 *(d, J*= 6.8 Hz, 1H), 6.94 (d, *J* = 8.4 Hz, 1H), 6.19 (s, 2H), 5.96 *(d, J*= 7.2 Hz, 1H), 4.28 (d, *J* = 5.6 Hz, 2H), 4.14 - 4.03 (m, 1H), 3,73 (q, *J* = 11.2 Hz, 2H), 2.29 - 2.24 (m, 4H), 2.16 - 2.06 (m, 5H), 1.90 - 1.85 (m, 2H), 1.62 - 1.58 (m, 2H), 1.56 - 1.50 (m, 11H).

**Example 165**

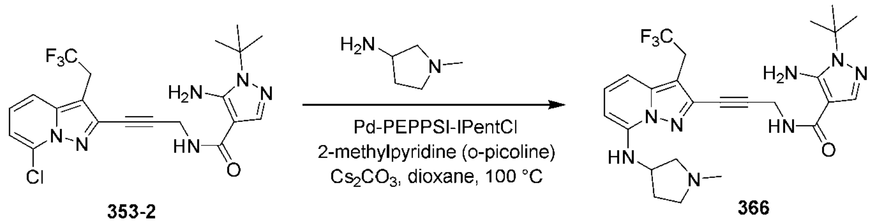


353-2 366

**Step 1: Synthesis of 5-amino-1-tert-butyl-N-(3-(7-[(1-methylpyrrolidin-3-yl)amino]-3-(2,2,2-trifluoroethyl)pyrazolo[1,5-a]pyridin-2-yl)prop-2-yn-1-yl)-1*H*-pyrazole-4-carboxamide (compound 366)**

Under nitrogen protection, at room temperature, to a solution of 5-amino-1-tert-butyl-N-(3-(7-chloro-3-(2,2,2-trifluoroethyl)pyrazolo[1,5-a]pyridin-2-yl)prop-2-yn-1-yl)-1*H*-pyrazole-4-carboxamide (compound 353-2, 150 mg, 0.33 mmol, 1 eq) and 1-methylpyrrolidine-3-amine (49.6 mg, 0.50 mmol, 1.5 eq) in 1,4-dioxane (10 mL) were added cesium carbonate (431.7 mg, 1.32 mmol, 4 eq) and (SP-4-1)-[1,3-bis[2,6-bis(1-propylbutyl)phenyl]-4,5-dichloro-1,3-dihydro-2*H*-imidazol-2-ylidene]dichloro(3-chloropyridine-KN)palladium (30 mg, 0.033 mmol, 0.1 eq) in portions. After the addition was completed, the system was stirred at 100°C for 12 h. After the reaction was completed, the reaction mixture was diluted with water (50 mL), and then extracted with ethyl acetate (3×60 mL). The organic phases were combined, backwashed with saturated brine (3×50 mL), dried over anhydrous sodium sulfate, filtered, and concentrated. The crude product was purified by silica gel column chromatography (dichloromethane/methanol = 10:1) to give compound **366** (65 mg, 37.99%).

LCMS: (ESI, m/z): 517.2 $[M+H]^+$.

$^1$H NMR (400 MHz, DMSO-d6) δ 8.31 (t, J = 5.6 Hz, 1H), 7.65 (s, 1H), 7.32 - 7.21 (m, 1H), 6.97 (d, J = 8.4 Hz, 1H), 6.63 (d, J = 7.6 Hz, 1H), 6.18 (s, 2H), 6.11 (d, J = 7.2 Hz, 1H), 4.28 (d, J = 5.6 Hz, 2H), 4.21 - 4.10 (m, 1H), 3.73 (q, J = 11.1 Hz, 2H), 2.83 - 2.72 (m, 1H), 2.71 - 2.62 (m, 1H), 2.61 - 2.55 (m, 1H), 2.45 - 2.35 (m, 1H), 2.31 - 2.20 (m, 4H), 1.89 - 1.77 (m, 1H), 1.53 (s, 9H).

**Biological Evaluation**

**Test Example 1. In vitro DNA binding activity assay**

The activity of the compound to modulate the binding of the p53-Y220C mutant protein to DNA was detected using TR-FREt (Time-Resolved Fluorescence Resonance Energy Transfer). The recombinant Y220C p53 DBD protein with his-tag and the DNA sequence with biotin label were prepared firstly. Donor Eu(Europium)-SA(Streptavidin) can be bound to the DNA sequence through biotin, receptor for his antibody attached to alphaphycocyanin(APC, allophycocyanin), and his antibody can be bound to the Y220C p53 DBD protein with his-tag. When exposed to an excitation light with a wavelength of 340 nm, the donor Eu(Europium)-SA(Streptavidin) can emit fluorescence with a wavelength of 615 nm. If the donor and the receptor are close to each other, the donor will transfer part of the energy to the receptor and produce fluorescence with a wavelength of 665 nm. That is to say, if the p53 mutant protein has its activity restored by the compound, and then gets close and binds to the DNA, the energy of the donor is transferred to the receptor to produce fluorescence with wavelengths of 615 nm and 665 nm. If there is no interaction, only 615 nm fluorescence is produced.

1) Experimental steps

a. Adding 4 μL of dilute compound solution to each well of a 384-well microplate;
a. Adding 4 μL of 4X P53 working solution to each well of the 384-well microplate;
c. Sealing the 384-well microplate and equilibrating the system at room temperature for 60 min;
d. Adding 4 μl of biotin DNA solution to the 384-well microplate;
e. Adding 4 μl of 4X test solution (MAb Anti-6His-Eu and streptavidin-d2) to each well of the 384-well microplate;
f. Incubating overnight in the dark; and
g. Reading data of 665 nm and 615 nm wavelengths on the BMG multifunctional microplate reader.

2) Data analysis

a. Calculating the ratio for each well ($Ratio_{665\ nm/615\ nm}$-$Ratio_{background}$);
b. Calculating the activity percentage ($\%_{activity}$) as follows:

$$C=(Ave_{_Ac}-Ave_{_Ba})/(Ave_{_Dc}-Ave_{_Bd})$$

$$R\ data=(A-Ave_{_Ba}-CD)/(D-Ave_{_Bd})*(Ave_{_Dc}-Ave_{_Bd})$$

$$\%\ Activity = R\ data/Ave_VC*100$$

A: Fluorescence intensity of the sample at 665 nm; D: Fluorescence intensity of the sample at 615 nm; Ba: Background fluorescence intensity of the culture plate at 665 nm; Bd: Background fluorescence intensity of the culture plate at 615 nm;
Dc: Background fluorescence intensity of HIS-E at 615 nm; Ac: Background fluorescence intensity of streptavidin-d2 at 665 nm); and

c. Calculating $SC_{150}$ and plotting dose-effect curve

Compared to the absence of the compound of the present invention, the activation percentage of binding of the p53 mutant protein to DNA in the presence of the compound of the present invention is expressed as $SC_{150}$, which indicates the concentration of the compound required to increase the DNA binding activity of the p53 mutant protein by 50%. The $SC_{150}$ is calculated using Prism™.

The activity of the representative compound for reactivation of the p53-Y220C mutant protein was determined by the above experiment, and the measured $SC_{150}$ is shown in Table 1.

Table 1 $SC_{150}$ of the representative compound for the activity of the p53-Y220C mutant protein

| Compound No. | $SC_{150}$ (nM) | Compound No. | $SC_{150}$ (nM) |
|---|---|---|---|
| 001 | 10 | 291B | 4 |
| 004 | 6 | 292 | 8 |
| 005 | 16 | 294 | 39 |
| 006 | 11 | 295 | 45 |
| 007 | 11 | 296 | 21 |
| 009 | 7 | 300 | 12 |
| 010 | 9 | 301 | 27 |
| 012 | 4 | 302 | 25 |
| 013 | 7 | 303 | 14 |
| 014 | 13 | 304 | 13 |
| 015 | 62 | 305 | 7 |
| 021 | 13 | 306 | 6 |
| 022 | 8 | 307 | 32 |
| 024 | 58 | 309 | 74 |
| 029 | 15 | 310 | 11 |
| 030 | 18 | 311 | 4 |
| 039 | 10 | 312 | 19 |
| 043 | 86 | 313 | 28 |
| 047 | 7 | 314 | 35 |
| 072 | 41 | 315 | 52 |
| 074 | 51 | 316 | 97 |

(continued)

| Compound No. | $SC_{150}$ (nM) | Compound No. | $SC_{150}$ (nM) |
|---|---|---|---|
| 075 | 95 | 317 | 44 |
| 076 | 75 | 318 | 59 |
| 082 | 20 | 319 | 41 |
| 087 | 19 | 320 | 28 |
| 135 | 22 | 321 | 99 |
| 142 | 29 | 322 | 16 |
| 145 | 50 | 323 | 57 |
| 146 | 13 | 324 | 29 |
| 148 | 27 | 325 | 35 |
| 160 | 84 | 326 | 28 |
| 230 | 29 | 327 | 27 |
| 231 | 19 | 328 | 30 |
| 232 | 8 | 329 | 9 |
| 234 | 21 | 331 | 36 |
| 236 | 22 | 332 | 29 |
| 240 | 75 | 334 | 33 |
| 241 | 82 | 335 | 41 |
| 248 | 14 | 336 | 68 |
| 249 | 17 | 339 | 92 |
| 260 | 18 | 343 | 25 |
| 261 | 6 | 344 | 56 |
| 263 | 4 | 346 | 18 |
| 264 | 78 | 348 | 28 |
| 265 | 40 | 349 | 12 |
| 266 | 46 | 350 | 13 |
| 268 | 10 | 351 | 7 |
| 269 | 8 | 352 | 13 |
| 270 | 13 | 353 | 4 |
| 271 | 23 | 353-4 | 9 |
| 272 | 13 | 354 | 3 |
| 273 | 11 | 355A | 7 |
| 274 | 12 | 355B | 16 |
| 275 | 27 | 356 | 87 |
| 276 | 18 | 358-A | 2 |
| 277 | 14 | 358-B | 32 |
| 278 | 24 | 360 | 160 |
| 279 | 9 | 361 | 49 |
| 280 | 16 | 362 | 98 |
| 281 | 13 | 365 | 5 |

(continued)

| Compound No. | $SC_{150}$ (nM) | Compound No. | $SC_{150}$ (nM) |
|---|---|---|---|
| 282 | 19 | 366 | 30 |
| 283 | 19 | 367 | 27 |
| 284 | 12 | 368 | 57 |
| 285 | 28 | 369-A | 8 |
| 286 | 8 | 369-B | 5 |
| 287 | 23 | 370 | 9 |
| 288 | 69 | 371 | 12 |
| 289 | 5 | 372 | 18 |
| 290 | 11 | 374A | 8 |
| 291 | 7 | 374B | 61 |
| 291A | 3 | Control compound | 14 |
| Note: $SC_{150}$ represents the concentration of compounds required to increase the binding activity of protein to DNA by 50% | | | |

Experimental conclusion: the above representative compounds can effectively restore the activity of p53-Y220C mutant protein.

**Test Example 2. NUGC-3 cell proliferation assay**

Experimental process

1. Cell culture

The cells were cultured in strict accordance with ATCC requirements.
Cell culture medium: 1640 culture medium, 10% serum, 1% penicillin + streptomycin.
Culture conditions: 37°C, 95% air, 5% carbon dioxide.

2. Compound treatment and cell suspension preparation

Compounds (20 mM) were subjected to gradient dilution in DMSO: 10 gradients, 3-fold dilution, duplicate wells.
Positive and negative controls (100% dimethyl sulfoxide) were prepared.
The compounds that were gradient diluted with DMSO and positive and negative controls (1st and 2nd step), each 75 nL, were transferred to 384-well cell culture plates by Echo550.
The cells were washed twice with PBS, digested with trypsin, and centrifuged for cell counting.
The experiment can only be carried out when the cell viability is above 90%.
The cells were plated into 384-well cell culture plates, with 200 (NUGC-3) cells and 30 μL of culture medium per well.
The 384-well microplate was placed in a cell culture incubator for 7 days.

3. Detection

30 μL of CTG detection reagent was added to the 384-well microplate.
The 384-well microplate was incubated for 30 min at 37°C, 5% carbon dioxide in the dark.
The reading was performed by a multi-functional microplate reader Envision.

4. Data analysis

% Inhibition is calculated as follows:

$$\% \text{ Inhibition} = 100 - (\text{Signal}_{cmpd} - \text{Signal}_{Ave_BL}) / (\text{Signal}_{Ave_VC} - \text{Signal}_{Ave_BL}) * 100$$

$\text{Signal}_{cmpd}$: Average signal of the compound in the cell plate
$\text{Signal}_{ave_vc}$: Average signal of the negative control in the cell plate
$\text{Signal}_{ave_BL}$: Average signal of the blank wells in the cell plate

Calculating $IC_{50}$ and plotting effect-dose curve

$IC_{50}$ was calculated by fitting % inhibition and compound concentration logarithmically to a nonlinear regression (dose response - variable slope) using Graphpad 8.0. The $IC_{50}$ of the representative compounds herein for inhibiting NUGC-3 cell proliferation are shown in Table 2.

Table 2 $IC_{50}$ of selected compounds in NUGC-3 cell proliferation assay

| Compound No. | $IC_{50}$ (nM) | Compound No. | $IC_{50}$ (nM) |
|---|---|---|---|
| 001 | 387 | 290 | 355 |
| 047 | 129 | 291 | 249 |
| 069 | 2852 | 291A | 166 |
| 070 | 3321 | 291B | 162 |
| 071 | 4564 | 292 | 214 |
| 074 | 1857 | 296 | 1226 |
| 082 | 1086 | 300 | 411 |
| 087 | 1368 | 301 | 1191 |
| 088 | 7106 | 305 | 193 |
| 089 | 8059 | 306 | 142 |
| 090 | 6874 | 311 | 173 |
| 135 | 1673 | 312 | 1461 |
| 146 | 955 | 313 | 1367 |
| 230 | 1059 | 314 | 1197 |
| 231 | 724 | 315 | 1240 |
| 232 | 419 | 316 | 2161 |
| 236 | 1283 | 317 | 1294 |
| 260 | 1189 | 318 | 1700 |
| 261 | 172 | 319 | 1373 |
| 263 | 268 | 320 | 1557 |
| 270 | 818 | 322 | 824 |
| 271 | 936 | 326 | 1178 |
| 272 | 1226 | 328 | 1206 |
| 274 | 611 | 331 | 1720 |
| 275 | 901 | 332 | 1714 |
| 276 | 856 | 353 | 152 |
| 277 | 726 | 353-4 | 181 |
| 278 | 1122 | 354 | 177 |
| 279 | 672 | 355A | 167 |
| 280 | 1064 | 355B | 154 |

(continued)

| Compound No. | $IC_{50}$ (nM) | Compound No. | $IC_{50}$ (nM) |
|---|---|---|---|
| 281 | 413 | 358-A | 123 |
| 283 | 1635 | 364 | 154 |
| 284 | 360 | 365 | 127 |
| 289 | 182 | Control compound | 384 |

Experimental conclusion: the above representative compounds can significantly inhibit the proliferation of NUGC-3 cells containing p53-Y220C mutation, and the activity of some compounds (such as those containing pyrazole structures, especially those containing 5-aminopyrazole structures) is significantly better than that of the control compound.

**Test Example 3. BXPC-3 3D cell proliferation assay**

Experimental process

1) Preparation of 1% methylcellulose solution

1 g of methylcellulose was weighed and placed into a glass ware and autoclaved. After cooling, 100 mL of the corresponding culture medium was added, the mixture was mixed vigorously, and stirred on a 4°C magnetic stirrer; the mixture was completely dissolved after 48 h and stored at 4°C.

2) Cell culture

The cells were resuscitated with the corresponding culture medium, and were maintained to grow to the logarithmic growth phase. The cell suspension was collected and centrifuged at 1000 rpm for 5 min. After centrifugation was completed, the supernatant was removed and the cells were re-suspended with an appropriate amount of culture medium.

3) Test of the inhibitory effect of the compound on tumor cells by 3D culture

Day 1: Cell plating

The above cell suspension was taken for counting, and a portion of the cells were frozen for later use; according to the density of 8000 cells per well, the concentration of the cell suspension was diluted and adjusted with culture medium. 3.5 mL of cell suspension and 6.5 mL of 1% methylcellulose were mixed evenly, trying to avoid bubbles. Cells were plated according to the required test number, 90 μL of cell suspension was added to each well of a 96-well plate, a T0 plate was provided, and cells were cultured at 37°C, 5% $CO_2$, and 95% air.

Day 2: T0 plate reading

After adding 10 μL of culture medium containing the vehicle to each well, the CTG plate was read. The CTG reagent was thawed and the cell plate was equilibrated to room temperature for 30 min; CTG solution equal to the volume of culture medium was added to each well (for example: 100 μL CTG/100 μL cell culture medium was added to a 96-well plate); the system was mixed thoroughly for 5 min to lyse the cells. The cell plate was placed at room temperature for 10 min to stabilize the luminescent signal, and the luminescent value was read using EnVision.

Day 2: Addition of drug to be tested

The test compound was diluted with culture medium to prepare a 10× solution of the positive drug and 10 μL of the drug solution was added to each well (three replicate wells for each cell concentration). The highest concentration of the test compound was 50 μM, with 9 concentrations, 3.5-fold dilution, and the highest concentration of the positive drug was 100 μM, with 9 concentrations, 3-fold dilution;

The cells were cultured at 37°C and 5% $CO_2$.

Day 9: Test plate reading (drug treatment for 7 days)

The CTG reagent was thawed and the cell plate was equilibrated to room temperature for 30 min. CTG solution equal to the volume of culture medium was added to each well (for example: 100 μL CTG/100 μL cell culture medium was added to a 96-well plate); the system was mixed thoroughly for 5 min to lyse the cells. The cell plate was placed at room temperature for 10 min to stabilize the luminescent signal, and the luminescent value was read using EnVision.

4) Data analysis

Cell viability was calculated using the following formula: $(V_{sample} - V_{Blank}) / (V_{vehicle\ control} - V_{Blank}) \times 100\%$. $V_{sample}$ is the reading of the drug treatment group, $V_{vehicle\ control}$ is the average value of the solvent control group, and $V_{Blank}$ is the average value of the culture medium wells (blank wells). The S-shaped dose-survival curve was plotted and $IC_{50}$ calculated by GraphPad Prism software using non-linear regression model.

The $IC_{50}$ values of the compounds for inhibiting BXPC-3 3D cell proliferation are shown in Table 3.

Table 3 $IC_{50}$ of selected compounds in BXPC-3 3D cell proliferation assay

| Compound No. | $IC_{50}$ (nM) |
|---|---|
| Control compound | 769 |
| 047 | 180 |

Experimental conclusion: Compound 047 can significantly inhibit the proliferation of BXPC-3 cells containing p53-Y220C mutation, and its activity is better than that of the control compound.

**Test Example 4: CYP3A4 inhibition experiment**

4.1 Experimental purpose

The inhibitory effects of the test compounds on CYP 3A4T (with testosterone as substrate) and 3A4M (with midazolam as substrate) in human liver microsomes were evaluated in vitro to assess the medication safety of the compounds.

4.2 Experimental method

This study used the human liver microsome system and the cytochrome P450 enzyme probe substrate recommended by FDA guidelines for in vitro drug interaction studies to investigate the inhibition of the CYP 3A4 enzyme in human liver microsomes by the test compounds. After human liver microsomes were co-incubated with different concentrations of the test compounds (0 - 30.0 μM) and the corresponding probe substrates, the changes of CYP enzyme activity were measured, and the $IC_{50}$ values were calculated to evaluate the inhibitory potential of the test compounds on CYP 3A4 enzyme.

4.2.1 Test concentration

The seven test concentrations were 30.00, 10.00, 3.33, 1.11, 0.370, 0.123, and 0.0412 μM, respectively.

4.2.2 Test group and control group

Test group (TG): Human liver microsomes were incubated with different concentrations of test compounds, NADPH (final concentration 2.00 mM) and probe substrates of different sub-enzymes (Table 4) for 5 min.

Positive control group (PC): Human liver microsomes were incubated with inhibitors (Table 4), NADPH (final concentration 2.00 mM) and probe substrates of different sub-enzymes (Table 4) for 5 min.

Table 4 Probe substrates of sub-enzymes, inhibitors and incubation systems

| CYP450 enzyme | Liver microsome concentration (Final concentration, mg/mL) | Probe substrate (Final concentration, μM) | Inhibitor (Final concentration, μM) | Incubation time (min) |
|---|---|---|---|---|
| CYP3A4 | 0.100<br>0.100 | Testosterone (80.0)<br>Midazolam (5.00) | Ketoconazole<br>(2.50) | 5<br>5 |
| At the end of incubation, the reaction was terminated with stop solution. | | | | |

4.2.3 Preparation of experimental solutions

4.2.3.1 Stop solution containing internal standard

18.26 μL of 4.379 mg/mL tolbutamide stock solution and 13.02 μL of 3.071 mg/mL verapamil stock solution were taken and added to 4 L of acetonitrile/methanol (volume 1:1) to give a stop solution with an internal standard concentration of 20.0 ng/mL tolbutamide and 10.0 ng/mL verapamil.

4.2.3.2 Preparation of human liver microsome (HLM) working solution

The 20.0 mg/mL HLM stock solution was diluted to 0.2 mg/mL HLM working solution using phosphate buffer.

4.2.3.3 Preparation of NADPH working solution

An appropriate amount of NADPH was weighed, prepared into 8.00 mM NADPH working solution with phosphate buffer, and preheated at 37°C.

4.2.3.4 Preparation of test sample stock solution

An appropriate amount of the test sample was weighed and dissolved in DMSO to give 30 mM stock solution.

4.2.3.5 Preparation of substrate working solution

The 80 mM testosterone stock solution was diluted to 320 μM working solution concentration with phosphate buffer, and the 5 mM midazolam stock solution was diluted to 20 μM working solution concentration with phosphate buffer.

4.2.4 Experimental process

4.2.4.1 Preheating of phosphate buffer (pH 7.40 ± 0.05)

4.2.4.2 Preparation of serial concentrations of test sample or positive control intermediate solutions

8 μL of 30 mM test sample was transferred to 12 μL of DMSO to give 12 mM test sample intermediate solution concentration. The system was further diluted with DMSO to get different concentrations of test sample working solutions (12 mM, 4 mM, 1.33 mM, 0.433 mM, 0.147 mM, 0.049 mM and 0.0164 mM).

8 μL of 2.5 mM stock solution of CYP3A4 inhibitor ketoconazole was measured and added to 12 μL DMSO to give 1 mM positive control inhibitor working solution.

4.2.4.3 Test group (TG)

199 μL of 0.2 mg/mL HLM and 1 μL of test sample working solution (12 mM, 4 mM, 1.33 mM, 0.433 mM, 0.147 mM, 0.049 mM and 0.0164 mM) were taken and mixed well. 30 μL of the drug-containing liver microsome solution was taken and added to a 96-well plate, then 10 μL of substrate working solution (320 μM testosterone or 20 μM midazolam) was added, in duplicate. The system was pre-incubated at 37°C for 5 min, and then 15 μL of 8 mM NADPH solution preheated at 37°C for 5-10 min to start the reaction. After incubation of the 96-well plate at 37°C for 5 min, 180 μL of acetonitrile:methanol (ACN:MeOH=1:1) solution containing internal standard (IS) was added to terminate the reaction. The 96-well plate was shaken at 600 rpm for 5-10 min and then centrifuged at 6000 rpm for 15 min. 80 μL of the supernatant was taken, added to 120 μL of ultrapure water, shaken evenly, and then detected by LC-MS/MS.

4.2.4.4 Positive control group (PC)

199 μL of 0.2 mg/mL HLM and 1 μL of 1 mM positive control inhibitor (ketoconazole) working solutions were taken and mixed evenly. 30 μL of the drug-containing liver microsome solution was taken and added to a 96-well plate, then 10 μL of substrate working solution (20 μM midazolam) was added, in duplicate. The system was pre-incubated at 37°C for 5 min, and then 15 μL of 8 mM NADPH solution preheated at 37°C for 5-10 min to start the reaction. After incubation of the 96-well plate at 37°C for 5 min, 180 μL of ACN:MeOH(1:1) solution containing IS was added to terminate the reaction. The 96-well plate was shaken at 600 rpm for 5-10 min and then centrifuged at 6000 rpm for 15 min. 80 μL of the supernatant was taken, added to 120 μL of ultrapure water, shaken evenly, and then detected by LC-MS/MS.

4.2.5. Data analysis

4.2.5.1 The relative activity of CYP450 sub-enzymes at different concentrations was calculated by dividing the peak area ratio (analyte peak area/internal standard peak area) of metabolites in the presence of the test sample or positive control by the peak area ratio of metabolites in the solvent control sample in the absence of the test article or positive control, according to the following formula:

Relative activity (% of NC) = metabolite peak area ratio in the test group or positive control group/metabolite peak area ratio in the negative control group × 100

PC group enzyme activity inhibition rate % = 100% - relative enzyme activity % at the test concentration

4.2.5.2 Half maximal inhibitory concentration ($IC_{50}$) was obtained by fitting according to the following model formula using Prism software:

$$Y = Bottom + (Top - Bottom)/(1 + 10\text{^} ((Log\ IC_{50} - X) \times HillSlope))$$

X is the test sample concentration converted to logarithm (Log μM), Y is the relative activity (% of NC), Bottom and Top represent the percentage of enzyme activity at the bottom platform and top platform of the curve, respectively, and HillSlope represents the slope; to optimize the fitting effect, the use of other (nonlinear) dose-response models for fitting is not considered as a deviation from the protocol.

When the relative activity (% of NC) of the PC group is ≤ 50.0%, it indicates that the known inhibitor exhibits an inhibitory effect, indicating that the test system is qualified.

4.3. Experimental Results

The experimental results of the P450 enzyme inhibition experiment of the test compounds are shown in Table 5.

Table 5 Experimental results of the P450 enzyme inhibition experiment

| Compound | CYP 3A4M $IC_{50}$ (μM) | CYP 3A4T $IC_{50}$ (μM) |
|---|---|---|
| Control compound | 9.4 | 4.3 |
| 001 | 26.3 | 27.2 |
| 004 | >30 | >30 |
| 039 | 20.2 | 27.8 |
| 047 | 20.7 | 17.3 |
| 087 | >30 | >30 |
| 146 | 9.7 | 10.5 |
| 261 | 15.6 | 7.4 |
| 263 | >30 | 16.4 |
| 276 | 17.4 | >30 |
| 274 | >30 | >30 |

(continued)

| Compound | CYP 3A4M $IC_{50}$ (μM) | CYP 3A4T $IC_{50}$ (μM) |
|---|---|---|
| 275 | >30 | >30 |
| 277 | 12.4 | 15.8 |
| 278 | 14.7 | 13.0 |
| 279 | 18.0 | 24.3 |
| 281 | >30 | >30 |
| 283 | >30 | 18.1 |
| 289 | 12.9 | 13.2 |
| 322 | 19.2 | 24.0 |
| 351 | 18.1 | 22 |
| 353 | 22.3 | 26.5 |
| 354 | 15.6 | 18.5 |
| 355A | >30 | 14.1 |
| 355B | 12.5 | 13.7 |

Experimental conclusion: the above representative compounds have weaker inhibition on CYP3A4 and lower risk of drug-drug interaction compared to the control compounds.

**Test Example 5: Caco-2 cell permeability test**

1) Cell culture: The resuscitated Caco-2 cells were cultured in DMEM culture solution containing 10% FBS, 1% Penicillin-Streptomycin Liquid (100X) and 1% NEAA in an incubator at 37 °C with 5% $CO_2$. The culture medium was replaced every 2-3 days and subculture was carried out when the cell confluence reached about 80%.
2) Preparation of transport buffer: Transport buffer (pH 7.4) was prepared by adding 5 mL of 1M HEPES to 500 mL of HBSS (with or without BSA) to a final concentration of 10 mM HEPES (with or without BSA).
3) Before the start of the transport experiment (day 14-28), the culture medium was taken out from the cell culture plate, the plate was washed twice with preheated HBSS buffer (10 mM HEPES, pH 7.4), and 100 μL of HBSS buffer was added into each well. After incubation at 37°C for 30 min, the TEER values were measured by a cell resistance meter (Millicell-ERS2) to confirm the integrity and compactness of the monolayer cells.
4) The HBSS buffer was discarded, the upper plate containing the cell monolayer membrane was transferred to the corresponding receiving plate, the test compound solution or buffer was added to the top (A side) and the basal side (B side), respectively, the cell plate was placed in the corresponding 96-well receiving plate, and grouping was carried out according to Table 6. The prepared positive control drug, test compound or HBSS (with or without BSA) buffer were added to the A side or B side of the Transwell cell plate filter membrane, and the cell plate was incubated at 37°C.

Table 6. Compound grouping scheme

| Test group | Transport direction | Side A | | Side B | | Number of duplicate wells |
|---|---|---|---|---|---|---|
| | | Volume | Compound or transport buffer | Volume | Compound or transport buffer | |
| G 1 | A→B | 0.1 mL | 10 μM Atenolol + 10 μM LY + TB | 0.3 mL | TB | 2 |
| | B→A | NA | NA | NA | NA | 2 |

(continued)

| Test group | Transport direction | Side A | | Side B | | Number of duplicate wells |
|---|---|---|---|---|---|---|
| | | Volume | Compound or transport buffer | Volume | Compound or transport buffer | |
| G 2 | A→B | 0.1 mL | 10 μM Propranolol + 10 μM LY + TB | 0.3 mL | TB | 2 |
| | B→A | NA | NA | NA | NA | 2 |
| G3 | A→B | 0.1 mL | 10 μM Digoxin + 10 μM LY + TB | 0.3 mL | TB | 2 |
| | B→A | 0.1 mL | 10 μM LY + TB | 0.3 mL | 10 μM Digoxin + TB | 2 |
| G 4 | A→B | 0.1 mL | 10 μM compound + 10 μM LY + TB | 0.3 mL | TB | 2 |
| | B→A | 0.1 mL | 10 μM LY + TB | 0.3 mL | 10 μM compound + TB | 2 |
| LY: Fluorescent Yellow<br>TB: Transport buffer (HBSS with 10 mM HEPES (with or without BSA)) | | | | | | |

1) After incubation for 5 min, a sample was taken from the A and B administration sides, 8 μL each side, and added into 72 μL of transport buffer (diluted 10 times) to serve as T0 of the initial administration solution.
2) After incubation for 120 min, the samples from both ends of the Transwell cell plate filter membrane were collected, and 80 μL of sample solution was taken from the receiving side to serve as the sample at the receiving side after transport, and 8 μL of sample was taken from the administration side and added into 72 μL of transport buffer to serve as the sample at the administration side after transport.
3) After collecting the samples from both sides, the plate was gently tapped to discard the remaining solution in the cell plate, the cell plate was rinsed with the transport buffer, 100 μL of acetonitrile solution containing internal standard working solution was added to each well to lyse the cells, the system was pipetted up and down to well mix it, and then 80 μL of cell lysate from each well was measured by pipetting to serve as the sample after cell lysis.
4) After collecting all samples in the transport system (T0 sample, receiving side sample, and donor side sample), 160 μL of IS/ACN solution was added to each well, 80 μL of IS/ACN solution was added to the collected lysate sample, and then the sample was sealed.
5) The sample plate was shaken for 10 min, and stored at -20°C before LC-MS/MS analysis.
6) A standard curve was plotted, and then 8 μL of standard curve solution was taken and mixed with 72 μL of transport buffer and 160 μL of IS/ACN to serve as the standard curve sample.
7) All the above samples were centrifuged at 6000 RPM for 10 min, 100 μL of supernatant was taken, 100 μL of $H_2O$ was added, and the mixture was fully mixed, and analyzed by LC-MS/MS.

The Caco-2 cell permeability test results of the test compounds are shown in Table 7.

Table 7 Caco-2 cell permeability results of the test compounds

| Compound | Papp($10^{-6}$cm/s) | | Efflux rate |
|---|---|---|---|
| | A→B | B→A | |
| Control compound | 4.68 | 19.43 | 4.16 |
| 001 | 4.39 | 6.50 | 1.48 |
| 047 | 5.18 | 7.80 | 1.51 |

Experimental conclusion: The above representative compounds have better cell permeability and lower efflux rate compared to the control compounds.

**Test Example 6. PXR activity test**

6.1 Cell seeding

1) DPX2 cells were cultured in growth medium containing 10% fetal bovine serum.
2) DPX2 cells were cultured in a T-75 flask in an incubator at 37°C, 5% $CO_2$, and 95% relative humidity. The cells were digested when they covered 80-90% of the bottom of the flask.
3) The surface of the T-75 cultured cells was washed with 10 mL of PBS, PBS was removed by pipetting, 3-5 mL of trypsin was added, the cells were digested at 37°C for 5 min or until the cells were completely digested and in a suspended state, and excessive culture medium containing fetal bovine serum was added to terminate trypsin digestion.
4) The cell suspension was transferred to a conical-bottom centrifuge tube and centrifuged at 150 g for 5 min at room temperature. The supernatant was carefully removed by pipetting and the cells were re-suspended in treatment medium to have a concentration of 3.2×105 cells/mL (incubation time 24 h, plate seeding density 4.0×105 cells/mL). In a 384-well cell culture plate, 25 μL of cell suspension was added to each well. The cell plate was placed in an incubator at 95% humidity, 37°C, and 5% $CO_2$ for 24 h.

6.2 Compound formulation

1) 1000× stock solutions of the test compound, positive control (rifampicin), and negative control (propranolol) were prepared using DMSO. The final concentrations of the positive control (rifampicin) were 1 μM and 10 μM, and the final concentration of the negative control (propranolol) was 10 μM. The final concentrations of the test compounds were 10, 1, 0.1 μM or EC50 (30, 10, 3, 1, 0.3, 0.1 μM). The final concentration of DMSO was 0.1%.
2) The cell culture plate in the incubator was taken out, 25 nL of positive control, negative control or test compound stock solution was directly added using Echo, and three parallels were arranged for each concentration. The cell plates were placed in an incubator and further incubated for 24 h.
3) Before starting experiments with the substrate, cell morphology and monolayer integrity were checked to ensure the monolayer is of acceptable quality for studies.

6.3 Quantitative detection of PXR activation

1) After 24 h of treatment with the drugs, the cultures can be quantified for PXR activation.
2) The CellTiter-Fluor™ cell viability assay kit and One-Glo Luciferase reagent were equilibrated to room temperature. GF-AFC substrate (10 μL) was added to Assay Buffer (10 ml) to form 2X reagent, which was then diluted into 1X reagent with 10 mL of PBS. ONE-Glo Luciferase substrate was added to ONE-Glo Luciferase Assay Buffer.
3) The culture plate was taken out of the incubator, the culture medium was discarded, 1X CellTiter-Fluor™ reagent was poured into the sample loading slot, 25 μL of reagent was added to each well of the culture plate using a pipetting workstation, and then the plate was placed in the incubator for incubation for 30 min.
4) The cell culture plate was taken out from the incubator, cooled slightly to room temperature, and the fluorescence value was measured by automatic quantitative microplate reader. The excitation light was 400 nm and the emission light was 505 nm.
5) ONE-Glo reagent was poured into the loading slot, 25μL was added into each well, the plate was lightly mixed, incubated for 5 min at room temperature, and the luminescent values were measured.

3.5 Data analysis

All data were calculated using Microsoft Excel.

1) The activity of luciferase is expressed as RFU/RLU, wherein RLU represents the average of three parallel relative luminescence units of each test compound at each dose, and RFU represents the average of three parallel relative fluorescence units of each test compound at each dose.

The activation fold of mRNA is calculated using the following formula:

$$\text{Fold activation} = (\text{RLU test}/\text{RFU test}) / (\text{RLU vehicle}/\text{RFU vehicle})$$

Fold activation: activation fold; RLU test: RLU sample; RFU test: RFU sample; RLU vehicle: RLU vehicle; RFU vehicle: RFU vehicle.

2) The percentage of cell viability of the compound is calculated as follows:

$$\text{Cell Viability \%} = (\text{RFU test} / \text{RFU vehicle}) \times 100$$

Cell Viability %: cell viability percentage; RFU test: RFU sample is the average fluorescence intensity value in the test wells; RFU vehicle: RFU vehicle is the average fluorescence intensity value of cells treated with 0.1% DMSO.

3) The percentage relative to the positive control is calculated according to the following formula:

$$\text{Percent of positive control (\%)} = (\text{Fold activation test} / \text{Fold activation Positive control})$$

Percent of positive control: Percentage of positive control; Fold activation test: activation fold of test sample; Fold activation Positive control: activation fold of positive control.

The PXR activity of the test compounds is shown in Table 8.

Table 8 PXR results for the test compounds

| Test compound | Concentration | Activation fold | Percentage relative to positive control |
|---|---|---|---|
| Control compound | 10 | 8.58 | 52.82 |
| | 1 | 1.40 | 8.61 |
| 001 | 10 | 3.05 | 17.50 |
| | 1 | 1.41 | 8.07 |
| 047 | 10 | 2.66 | 13.47 |
| | 1 | 1.12 | 5.67 |
| 261 | 10 | 1.43 | 7.20 |
| | 1 | 0.89 | 4.48 |

Experimental conclusion: The above representative compounds have lower PXR risk and lower risk of drug-drug interaction in clinical practice compared to the control compounds.

**Test example 7. Pharmacokinetic study of test compounds in SD rats**

Experimental method

On the day of administration, the test sample was prepared using a 2% HPC + 5% solutol aqueous solution vehicle formula, and the drug solution was prepared and kept ready for use.

The PO group was given the designated dose. The animals in the oral administration group were fasted overnight, allowed free access to water, and returned to food 3 hours after administration.

The animals were weighed before administration, and the dose was calculated based on the body weight. The drug was administered once via PO on the day of administration. After administration in the PO group, blood was collected from the jugular vein at 0.083 h, 0.25 h, 0.5 h, 1.0 h, 2.0 h, 4.0 h, 6.0 h, 8.0 h and 24 h, about 200 μL each time, placed in EDTA-K2 anticoagulation blood collection tubes, centrifuged at 4000 rpm for 10 min (4°C), and plasma was separated within 1 h. The plasma was drawn into labeled EP tubes (the label should at least indicate the subject code, animal number, collection time, collection date, etc.), and stored at -80°C for testing. The process from blood collection to centrifugation was carried out in ice bath. The concentration of the test sample in SD rat plasma was detected using validated LC-MS/MS.

The pharmacokinetics of the compounds of the present invention in SD rats were determined by the above experiments. The results are shown in Table 9.

Table 9. Experimental results of pharmacokinetics of test compounds in SD rats

| Compound | po 50 mpk | | po 300 mpk | |
|---|---|---|---|---|
| | $C_{max}$ (ng/mL) | $AUC_{0-24h}$ (ng*h/mL) | $C_{max}$ (ng/mL) | $AUC_{0-24h}$ (ng*h/mL) |
| Control compound | 11467 | 147646 | 25400 | 416215 |
| 047 | 13233 | 112905 | 32775 | 430924 |

Experimental conclusion: Compound 047 has good PK properties in SD rats.

**Test example 8. Pharmacokinetic study of test compounds in beagles**

Experimental method

On the day of administration, the test sample was prepared using a 5%DMSO+5%Solutol+90%Saline vehicle formula, and the drug solution was prepared and kept ready for use.

The IV group received a dose of 1 mg/kg, and the PO group received a dose of 2 mg/kg. The animals were fasted overnight before administration.

The animals were weighed before administration, and the dose was calculated based on the body weight. The drug was administered once via IV and PO on the day of administration. After administration, blood was collected from an appropriate vein at 0.083 h, 0.25 h, 0.5 h, 1.0 h, 2.0 h, 4.0 h, 6.0 h, 8.0 h and 24.0 h, about 1 mL each time, placed in EDTA-K2 anticoagulation blood collection tubes, centrifuged at 4000 rpm for 10 min (4°C), and plasma was separated within 1 h. The plasma was drawn into labeled EP tubes (the label should at least indicate the subject code, animal number, collection time, collection date, etc.), and stored at -80°C for testing. The process from blood collection to centrifugation was carried out in ice bath. The animals were allowed to eat and have free access to water 3 h after administration. The concentration of the test sample in beagle plasma was detected using validated LC-MS/MS.

Experimental results

The pharmacokinetics of the compounds of the present invention in beagles were determined by the above experiments. The results are shown in Table 10.

Table 10. Experimental results of pharmacokinetic study of test compounds in beagles (IV 1mpk/PO 2mpk)

| Compound | | Control compound | 047 |
|---|---|---|---|
| IV | $C_0$ (ng/ml) | 277 | 414 |
| | $T_{1/2}$ (h) | 4.8 | 5.6 |
| | Vd (L/kg) | 6.7 | 3.8 |
| | Cl (ml/Kg/min) | 20.4 | 12.8 |
| | $AUC_{0-last}$ (ng*h/ml) | 849 | 1402 |
| PO | $C_{max}$ (ng/ml) | 101 | 199 |
| | $T_{max}$ (h) | 4.7 | 2.0 |
| | $T_{1/2}$ (h) | 6.7 | 14.0 |
| | $AUC_{0-last}$ (ng*h/ml) | 1494 | 1635 |
| | F% | 82 | 42 |

Experimental conclusion: Compound 047 has good PK properties in beagles.

**Test Example 9 Efficacy experiment in BALB/c Nude mouse NUGC-3 human gastric cancer subcutaneous xenograft model**

NUGC-3 cells were cultured in RPMI-1640 medium containing 10% FBS. NUGC-3 cells in the exponential growth phase were collected, the total amount of cells required was calculated, the cells were re-suspended in PBS to a suitable concentration and then an equal volume of Matrigel was added for subcutaneous tumor seeding in BALB/c Nude mice. The

concentration of the cell suspension was $5x10^6$/0.1 mL.

0.1 mL ($5\times10^6$ NUGC-3 cells) of cell suspension (PBS : Matrigel = 1:1, V/V) was seeded subcutaneously on the right back of each mouse. When the tumor grew to have an average volume of 128.4 $mm^3$, group-based drug administration began (vehicle: 0.2% HPC + 0.5% Tween 80), and the average tumor volume of the enrolled animals was 146.1 $mm^3$. Tumor size calculation formula: tumor volume ($mm^3$) = 0.5 $\times$ (tumor long diameter $\times$ tumor short diameter$^2$), tumor volume inhibition rate $TGI_{TV}$(%): TGI% = (1-$\Delta T/\Delta C$) $\times$ 100%; wherein, $\Delta C = C_t - C_0$, $C_0$ is the average tumor volume of the control group at the time of grouping, Ct is the average tumor volume of the control group after treatment, $\Delta T = T_t - T_0$, $T_0$ is the average tumor volume of the treatment group at the time of grouping, Tt is the average tumor volume of the treatment group after treatment.

The administration information for each group is shown in Table 11.

Table 11 Route, dose and regimen of administration in the subcutaneous xenograft model

| Group | N | Compound | Dose (mg/kg) | Administrat ion volume (mL/kg) | Route of administr ation | Frequency of administration |
|---|---|---|---|---|---|---|
| G1 | 6 | Vehicle | / | 10 | PO | BID*19 |
| G2 | 6 | Control compound | 50 | 10 | PO | QD*19 |
| G3 | 6 | Control compound | 100 | 10 | PO | QD*19 |
| G4 | 6 | 047 | 50 | 10 | PO | BID*19 |
| G5 | 6 | 047 | 100 | 10 | PO | BID*19 |
| G6 | 6 | 047 | 150 | 10 | PO | BID*19 |
| G7 | 6 | 047 | 150 | 10 | PO | QD*19 |

The efficacy results of the compounds in the present invention in the BALB/c Nude mouse NUGC-3 human gastric cancer subcutaneous xenograft model are shown in Fig. 1.

Experimental conclusion: The results show that compound 047 has a significant anti-tumor effect in the NUGC-3 human gastric cancer subcutaneous xenograft model. The tumor volume inhibition rate TGI of the control compound is 52% and 77% at 50 mpk, QD and 100 mpk, QD, respectively. Compound 047 has tumor volume inhibition rates TGI of 68%, 93%, 103% and 91% at 50 mpk, BID, 100 mpk, BID, 150 mpk, BID and 150 mpk, QD, respectively.

**Test Example 10: Efficacy experiment in human-derived lung cancer LU5269 subcutaneous xenograft female NOD/SCID model**

Tumor tissues were collected from LU5269 lung cancer xenograft model bearing mice, cut into tumor masses with a diameter of 2-3 mm, and seeded subcutaneously in the right anterior scapula of NOD/SCID mice.

Before administration, all animals were weighed and tumor volume was measured using a vernier caliper. When the tumor grew to have an average volume of about 140 $mm^3$, group-based drug administration was performed based on tumor size (vehicle: 0.2% HPC + 0.5% Tween 80). Since tumor volume can affect the effectiveness of treatment, random grouping design was used to group the mice according to tumor volume to ensure that the tumor volumes in different groups were similar. Tumor volume calculation formula: Tumor volume ($mm^3$) = 1/2 $\times$ (a $\times b^2$) (wherein a denotes the long diameter and b denotes the short diameter). Tumor volume inhibition rate $TGI_{TV}$(%): TGI% = (1-$\Delta T/\Delta C$) $\times$ 100%; wherein, $\Delta C = C_t - C_0$, $C_0$ is the average tumor volume of the control group at the time of grouping, Ct is the average tumor volume of the control group after treatment, $\Delta T = T_t - T_0$, $T_0$ is the average tumor volume of the administration group at the time of grouping, $T_t$ is the average tumor volume of the administration group after treatment.

At the endpoint of the experiment, plasma samples from groups G2-G6 were collected: plasma was collected from the first 3 mice in each group at 0 h, 1 h, 2 h, and 4 h after the last administration; plasma was collected from the last 3 mice in each group at 0.5 h, 6 h, 9 h, and 24 h after the last administration.

The administration information for each group is shown in Table 12.

Table 12 Route, dose and regimen of administration in the subcutaneous xenograft model

| Group | N | Compound | Dose (mg/kg) | Administrat ion volume (mL/kg) | Route of administr ation | Frequency of administration |
|---|---|---|---|---|---|---|
| G1 | 6 | Vehicle | / | 10 | PO | QD*21 |
| G2 | 6 | Control compound | 100 | 10 | PO | QD*21 |
| G3 | 6 | 047 | 50 | 10 | PO | QD*21 |
| G4 | 6 | 047 | 75 | 10 | PO | QD*21 |
| G5 | 6 | 047 | 100 | 10 | PO | QD*21 |
| G6 | 6 | 047 | 150 | 10 | PO | QD*21 |

The efficacy results of the compounds in the present invention in the human-derived lung cancer LU5269 subcutaneous xenograft female NOD/SCID model are shown in Fig. 2, and the associated PK results are shown in Table 13.

Table 13 Associated PK results of tests compounds

| Compound | Control compound G2, 100 mpk | 047 | | | |
|---|---|---|---|---|---|
| | | G3, 50mpk | G4, 75mpk | G5, 100mpk | G6, 150mpk |
| TGI (%) | 119 | 82 | 111 | 119 | 119 |
| $C_{max}$ (ng/mL) | 24333 | 1693 | 2590 | 4440 | 5553 |
| $T_{1/2}$ (h) | \ | 2.2 | \ | 1.9 | 2.6 |
| **AUC**$_{0\text{-last}}$ (ng*h/mL) | 413142 | 11730 | 24432 | 39418 | 67952 |

Experimental conclusion: The results show that compound 047 has a significant anti-tumor effect in the human-derived lung cancer LU5269 subcutaneous xenograft tumor model.

Unless otherwise specified, the structure of the control compound described in the present invention is as follows:

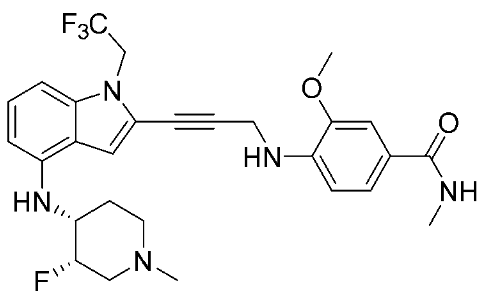

(WO2021061643A1\WO2023225477A2)

The embodiments of the technical solutions of the present invention are described in an illustrative manner above. It is to be understood that the scope of protection of the present invention is not limited to the above-mentioned embodiments. Any modifications, equivalent substitutions, improvements, etc. made by those skilled in the art within the spirit and principles of the present invention should be included in the scope of protection of the claims of the present application.

## Claims

1. A compound shown as formula (I), and a racemate, a stereoisomer, a tautomer, an isotope-labeled counterpart, a nitrogen oxide, a solvate, a polymorph, a metabolite, an ester, a prodrug or a pharmaceutically acceptable salt thereof:

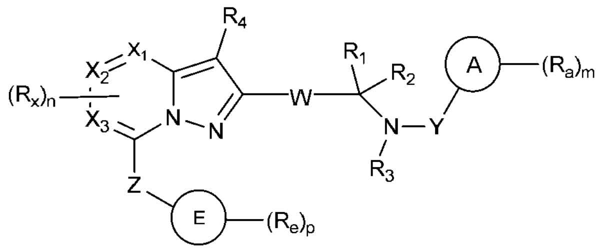

Formula (I)

wherein $R_1$ and $R_2$ are the same or different, and are independently selected from H, halogen, $C_{1-12}$ alkyl, or $C_{1-12}$ alkoxy;

$R_3$ is selected from H, or $C_{1-12}$ alkyl;

$R_4$ is selected from H, halogen, cyano, $C_{1-12}$ alkyl, $C_{2-12}$ alkoxy, $C_{2-12}$ alkenyl, $C_{1-12}$ alkynyl, $C_{3-12}$ cycloalkyl, halo-$C_{1-12}$ alkyl, halo-$C_{1-12}$ alkoxy, halo-$C_{3-12}$ cycloalkyl, cyano-$C_{1-12}$ alkyl, or cyano-$C_{1-12}$ alkoxy;

W is selected from $C_{2-6}$ alkenylene, $C_{2-6}$ alkynylene, $C_{3-14}$ cycloalkylene, $C_{6-10}$ arylene, or 5-10 membered heteroarylene;

ring A is selected from $C_{3-14}$ cycloalkyl, 3-14 membered heterocyclyl, $C_{6-10}$ aryl, or 5-10 membered heteroaryl;

$R_a$ is selected from H, CN, oxo (=O), halogen, OH, or the following groups unsubstituted or optionally substituted with one, two or more $R_{a1}$: $C_{1-12}$ alkyl, $C_{1-12}$ alkoxy, $C_{3-12}$ cycloalkyl, 3-14 membered heterocyclyl, $C_{6-10}$ aryl, 5-10 membered heteroaryl, $-C(O)N(R_{a11})(R_{a12})$, $-N(R_{a13})(R_{a14})$, $-S(O)_2-R_{a15}$, $-S(O)(=NR_{a16})(R_{a17})$, or $-P(O)(R_{a18})(R_{a19})$; each $R_{a1}$ is the same or different and is independently selected from H, OH, CN, halogen, or the following groups unsubstituted or optionally substituted with one, two or more $R_{a2}$: $C_{1-12}$ alkyl, $C_{1-12}$ alkoxy, $C_{3-12}$ cycloalkyl, 3-14 membered heterocyclyl, $C_{6-10}$ aryl, 5-10 membered heteroaryl, $-NH_2$, or $-S(O)_2-C_{1-12}$ alkyl; each $R_{a2}$ is the same or different and is independently selected from H, OH, $NH_2$, CN, halogen, $C_{1-6}$ alkyl, or $C_{1-6}$ alkoxy; $R_{a11}$, $R_{a12}$, $R_{a13}$, $R_{a14}$, $R_{a15}$, $R_{a16}$, $R_{a17}$, $R_{a18}$, and $R_{a19}$ are the same or different and are independently selected from H, $C_{1-12}$ alkyl, $C_{3-7}$ cycloalkyl, or 3-8 membered heterocyclyl; m is selected from 0, 1, 2, 3, 4, or 5;

Z is absent, or is selected from NH, S, O, $C_{1-6}$ alkylene, or $C_{1-6}$ alkylene-NH;

ring E is selected from 3-14 membered heterocyclyl, or $C_{3-12}$ cycloalkyl;

$R_e$ is selected from H, halogen, or the following groups unsubstituted or optionally substituted with one, two or more $R_{e1}$: $C_{1-12}$ alkyl, halo-$C_{1-12}$ alkyl, deuterated $C_{1-12}$ alkyl, $C_{1-12}$ alkoxy, $C_{3-12}$ cycloalkyl, 3-14 membered heterocyclyl, $C_{1-12}$ alkyl-NH-, or $(C_{1-12}$ alkyl$)_2$-N-; each $R_{e1}$ is the same or different and is independently selected from H, OH, $NH_2$, CN, halogen, $C_{1-6}$ alkyl, or $C_{1-6}$ alkoxy; or, two $R_e$ attached to the same carbon atom together with the atoms to which they are respectively attached form a $C_{3-12}$ cycloalkane ring, or a 3-14 membered heterocycle; or, two $R_e$ attached to different carbon atoms together with the atoms to which they are respectively attached form a 3-14 membered heterocycle; p is selected from 0, 1, 2, 3, 4, or 5;

$X_1$, $X_2$, and $X_3$ are the same or different, and are independently selected from CH or N, and at least two of $X_1$, $X_2$, and $X_3$ are CH;

$R_x$ is selected from H, CN, halogen, $C_{1-12}$ alkyl, or $C_{1-12}$ alkoxy; n is selected from 0, 1, 2 or 3;

Y is selected from C(O), C(O)NH, C(S), or $SO_2$.

2. The compound, and the racemate, stereoisomer, tautomer, isotope-labeled counterpart, nitrogen oxide, solvate, polymorph, metabolite, ester, prodrug or pharmaceutically acceptable salt thereof according to claim 1, wherein $R_1$, $R_2$, and $R_3$ are the same or different and are independently selected from H, or $C_{1-6}$ alkyl;

preferably, $R_1$, $R_2$, and $R_3$ are all H;

preferably, $R_4$ is selected from $C_{1-6}$ alkyl, $C_{2-6}$ alkenyl, halo-$C_{1-6}$ alkyl, or cyano-$C_{1-6}$ alkyl;

preferably, $R_4$ is selected from $C_{1-6}$ alkyl, halo-$C_{1-6}$ alkyl or cyano-$C_{1-6}$ alkyl;

preferably, $R_4$ is selected from methyl, ethyl, propyl, isopropyl, vinyl, cyanomethyl, 2-fluoroethyl, 2,2-difluoroethyl, or 2,2,2-trifluoroethyl;

preferably, $R_4$ is selected from methyl, ethyl, propyl, isopropyl, cyanomethyl, 2-fluoroethyl, 2,2-difluoroethyl, or 2,2,2-trifluoroethyl.

3. The compound, and the racemate, stereoisomer, tautomer, isotope-labeled counterpart, nitrogen oxide, solvate, polymorph, metabolite, ester, prodrug or pharmaceutically acceptable salt thereof according to claim 1 or 2, wherein W is selected from $C_{2-4}$ alkenylene, $C_{2-4}$ alkynylene, $C_{3-6}$ cycloalkylene, $C_{6-10}$ arylene, or 5-6 membered heteroarylene;

preferably, W is selected from:

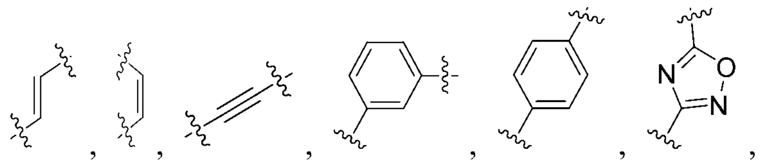

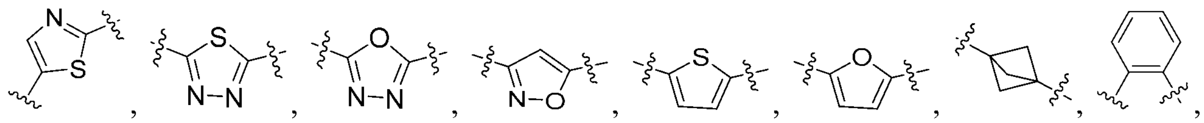

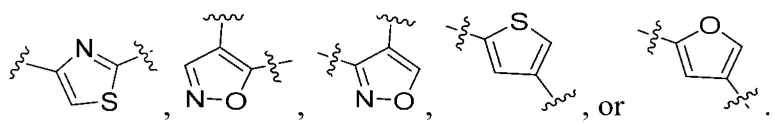

4. The compound, and the racemate, stereoisomer, tautomer, isotope-labeled counterpart, nitrogen oxide, solvate, polymorph, metabolite, ester, prodrug or pharmaceutically acceptable salt thereof according to any one of claims 1 to 3, wherein ring A is selected from phenyl, 5-6 membered heteroaryl, 8-9 membered heteroaryl, 6-10 membered heterocyclyl, or $C_{3-6}$ membered cycloalkyl;

preferably, ring A is selected from phenyl, 5-6 membered heteroaryl, 8-9 membered heteroaryl, 8-9 membered heterocyclyl, or $C_{3-6}$ membered cycloalkyl;

preferably, ring A is selected from:

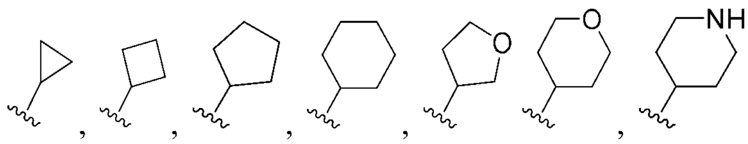

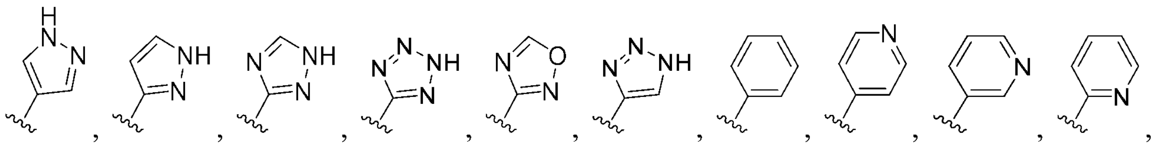

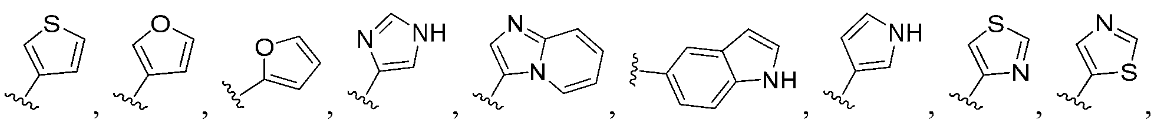

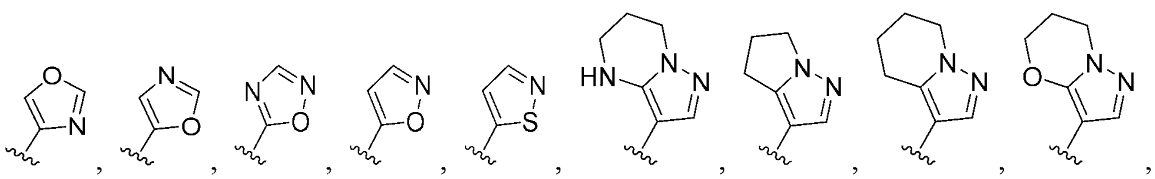

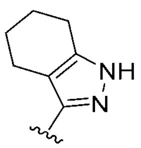, 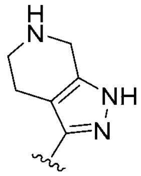, 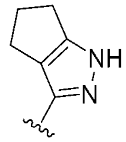, 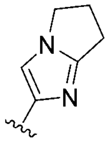, 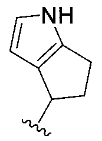, 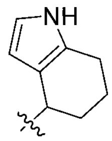, 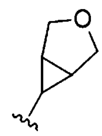, 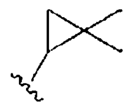,

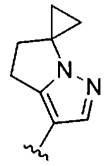, 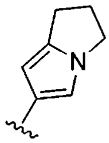, 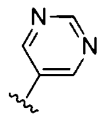, 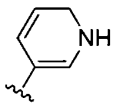, 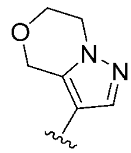, 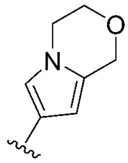, 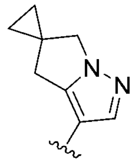, 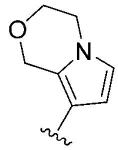,

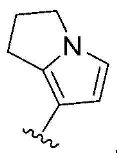, 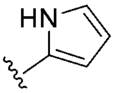, 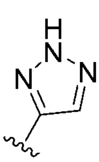, or 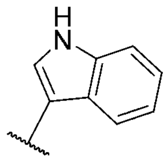;

preferably, $R_a$ is selected from H, CN, oxo (=O), halogen, OH, or the following groups unsubstituted or optionally substituted with one, two or more $R_{a1}$: $C_{1-6}$ alkyl, $C_{1-6}$ alkoxy, $C_{3-6}$ cycloalkyl, 4-7 membered heterocyclyl, phenyl, 5-6 membered heteroaryl, -C(O)(NH$C_{1-6}$ alkyl), - $S(O)_2$-$C_{1-6}$ alkyl, -S(O)(=NH)($C_{1-6}$ alkyl), -S(O)(N$C_{1-6}$ alkyl)($C_{1-6}$ alkyl), -P(O)($C_{1-6}$ alkyl)($C_{1-6}$ alkyl), or -$NH_2$; each $R_{a1}$ is the same or different and is independently selected from H, OH, CN, halogen, $C_{1-6}$ alkyl, halo-$C_{1-6}$ alkyl, $C_{1-6}$ alkoxy, $C_{3-6}$ cycloalkyl, 5-6 membered heterocyclyl, phenyl, 5-6 membered heteroaryl, cyano-$C_{1-6}$ alkyl, cyano-$C_{1-6}$ alkoxy, $C_{1-6}$ alkyl-NH-, ($C_{1-6}$ alkyl)$_2$-N-, -$S(O)_2$-$C_{1-6}$ alkyl, $C_{1-6}$ alkyl-O-$C_{1-6}$ alkyl, or hydroxy-$C_{1-6}$ alkyl;

preferably, $R_a$ is selected from H, F, Cl, CN, oxo (=O), OH, or the following groups unsubstituted or optionally substituted with one, two or more $R_{a1}$: methyl, ethyl, propyl, isopropyl, tert-butyl, cyclopropyl, cyclobutyl, cyclopentyl, cyclohexyl, methoxy, isopropoxy, propoxy, phenyl, pyridyl, benzyl, piperidyl,

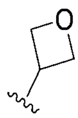, 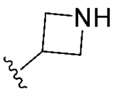, 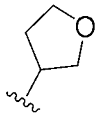, 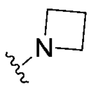, 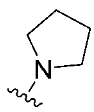, 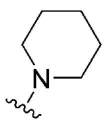, 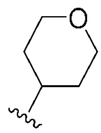,

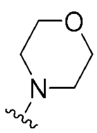, 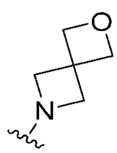,

-C(O)NH$CH_3$, -$S(O)_2$-$CH_3$, -S(O)(=NH)($CH_3$), -P(O)($CH_3$)($CH_3$), or -$NH_2$; each $R_{a1}$ is the same or different and is independently selected from H, CN, OH, F, Cl, methyl, ethyl, methoxy, trifluoromethyl, cyclopropyl, cyclobutyl, cyclopentyl, -$CH_2OCH_3$, -$CH_2CH_2OCH_3$, -$CH_2OH$, -$CH_2CN$, -$N(CH_3)_2$, -$S(O)_2$-$CH_3$,

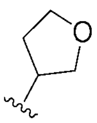, 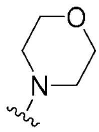, or 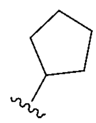;

preferably, $R_a$ is selected from H, F, Cl, CN, oxo (=O), OH, $NH_2$, methylamino, dimethylamino, methyl, ethyl, isopropyl, tert-butyl, -$C(CH_3)_2CH_2CH_3$, -$CH_2C(CH_3)_3$, cyclopropyl, cyclobutyl, cyclopentyl, cyclohexyl, phenyl, pyridyl, benzyl, 2,2-difluoroethyl, 2,2,2-trifluoroethyl, monofluoromethyl, difluoromethyl, trifluoromethyl, trifluoromethoxy, methoxy, isopropyloxy, 2-hydroxyethyl,

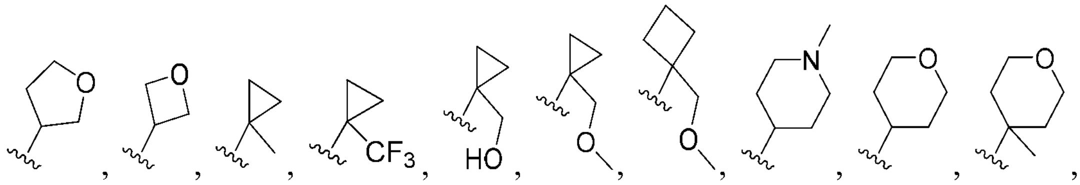,

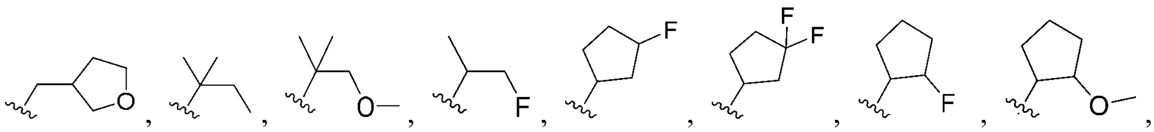,

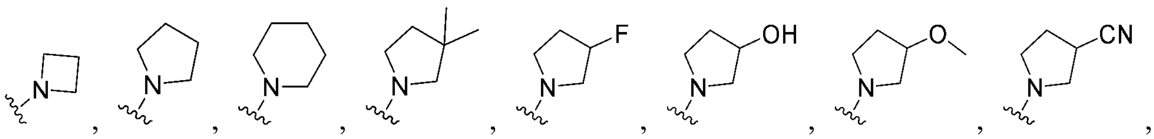,

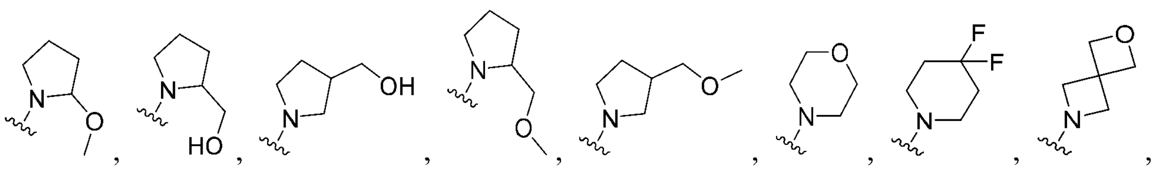,

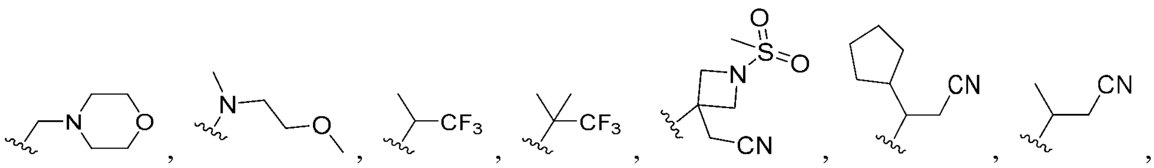,

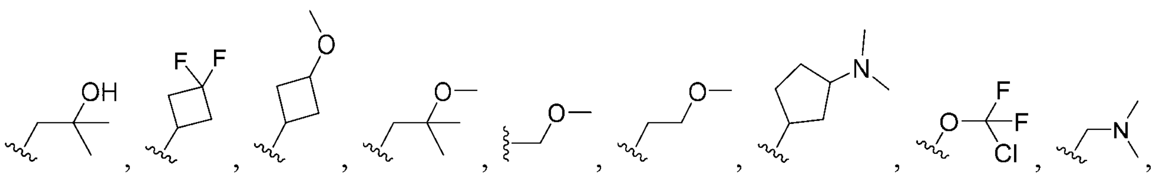,

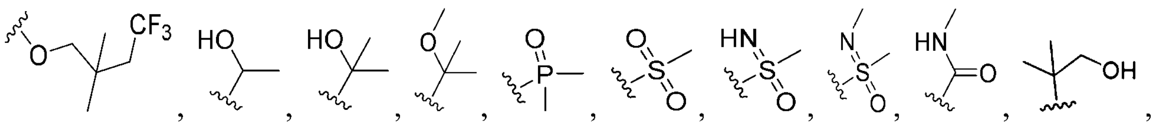,

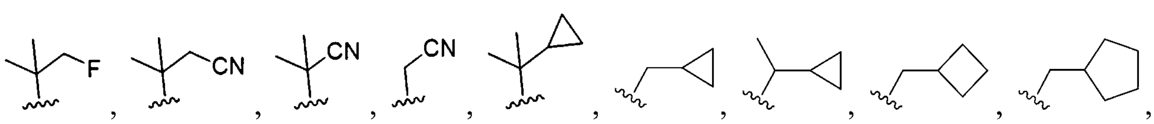,

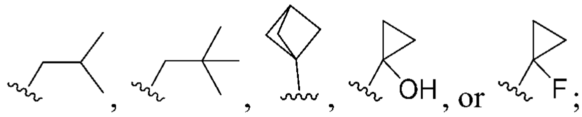, or;

preferably,

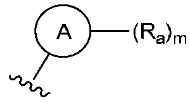

is selected from

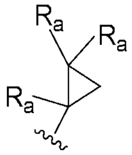, 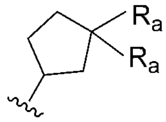, 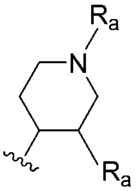, 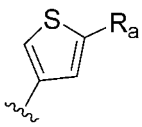,

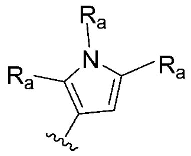, 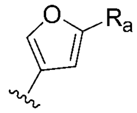, 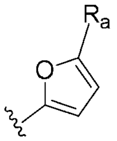, 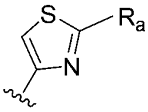, 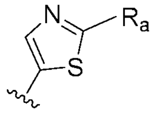, 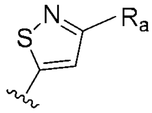, 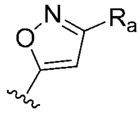,

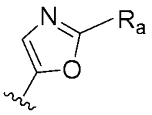, 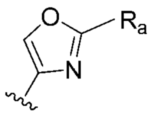, 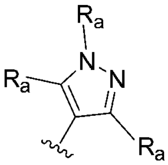, 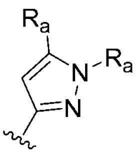, 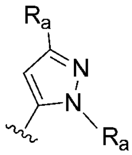, 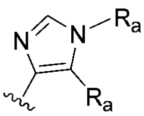, 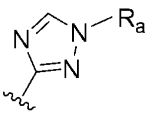,

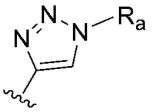, 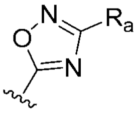, 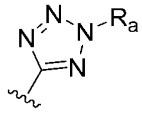, 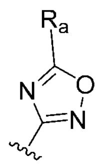, 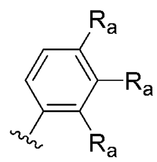, 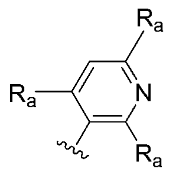, 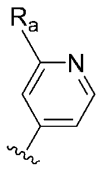,

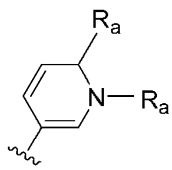, 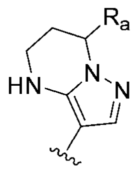, 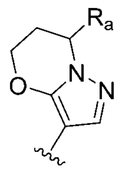, 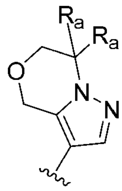, 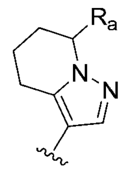, 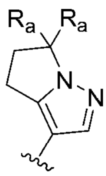, 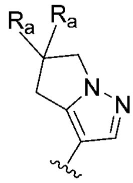, 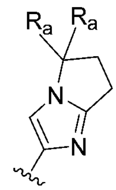,

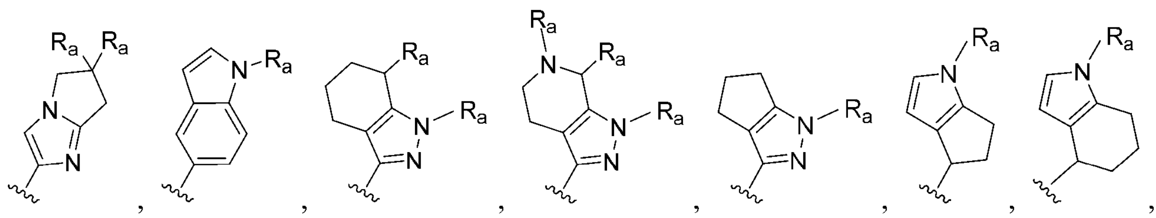, , , , , , ,

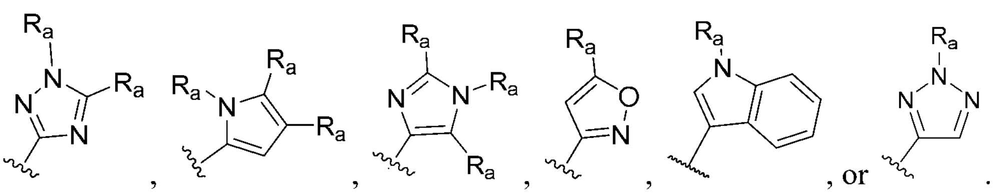, , , , , or .

**5.** The compound, and the racemate, stereoisomer, tautomer, isotope-labeled counterpart, nitrogen oxide, solvate, polymorph, metabolite, ester, prodrug or pharmaceutically acceptable salt thereof according to any one of claims 1 to 4, wherein Z is absent, or is selected from NH, S, O, $CH_2$, or $CH_2NH$;

preferably, ring E is selected from 5-9 membered heterocyclyl or $C_{5-6}$ cycloalkyl;
preferably, ring E is selected from 6-9 membered heterocyclyl or $C_{5-6}$ cycloalkyl;
preferably, ring E is selected from

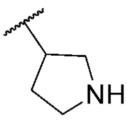, 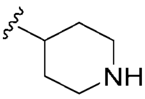, 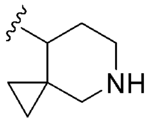, 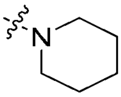, 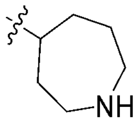,

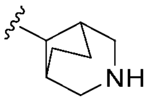, 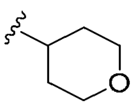, 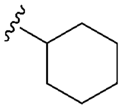, 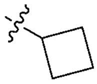, 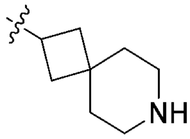, 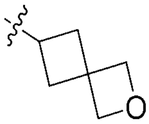, or 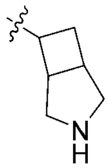;

preferably, ring E is selected from

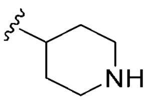, 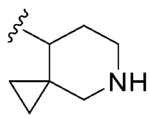, 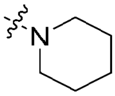, 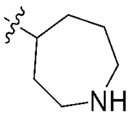,

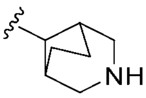, 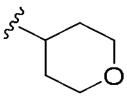, 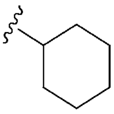, 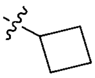, 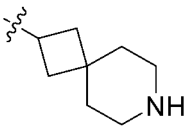, 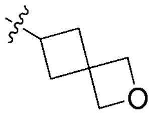, or 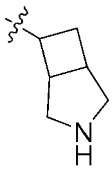;

preferably, $R_e$ is selected from H, halogen, or the following groups unsubstituted or optionally substituted with one, two or more $R_{e1}$: $C_{1-6}$ alkyl, halo-$C_{1-6}$ alkyl, deuterated $C_{1-6}$ alkyl, $C_{1-6}$ alkoxy, $C_{3-6}$ cycloalkyl, 4-9 membered heterocyclyl, or ($C_{1-6}$ alkyl)$_2$-N-; each $R_{e1}$ is the same or different and is independently selected from OH, halogen, $C_{1-3}$ alkyl, or $C_{1-3}$ alkoxy; or two $R_e$ together with the atoms to which they are respectively attached form a $C_{3-6}$ cycloalkane ring;
preferably, $R_e$ is selected from H, halogen, $C_{1-6}$ alkyl, halo-$C_{1-6}$ alkyl, deuterated $C_{1-6}$ alkyl, $C_{1-6}$ alkoxy, $C_{3-6}$ cycloalkyl, 5-6 membered heterocyclyl, or ($C_{1-6}$ alkyl)$_2$-N-; or, two $R_e$ together with the atoms to which they are respectively attached form a $C_{3-6}$ cycloalkane ring;
preferably, $R_e$ is selected from H, F, Cl, methyl, deuterated methyl ($CD_3$), trifluoromethyl, ethyl, isopropyl, cyclopropyl, methoxy, dimethylamino,

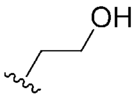, 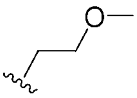, 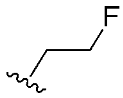, 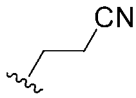,

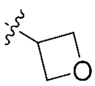, 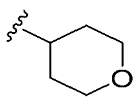, 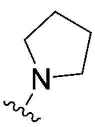, 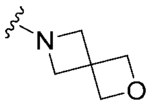, or 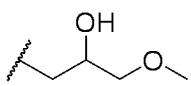;

or, two $R_e$ attached to the same carbon atom together with the atoms to which they are respectively attached form a cyclopropane ring,

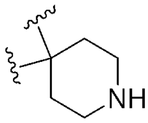 , or 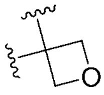 ;

or, two $R_e$ attached to different carbon atoms together with the atoms to which they are respectively attached form

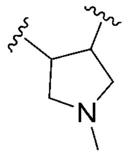 ;

preferably, $R_e$ is selected from H, F, Cl, methyl, deuterated methyl ($CD_3$), trifluoromethyl, ethyl, isopropyl, cyclopropyl, methoxy, dimethylamino,

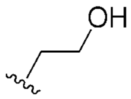 , 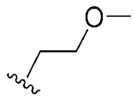 , 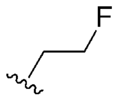 , 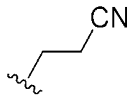 ,

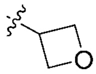 , 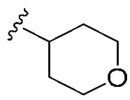 , 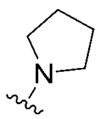 , or 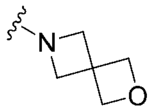 ;

or, two $R_e$ attached to the same carbon atom together with the atoms to which they are respectively attached form a cyclopropane ring,

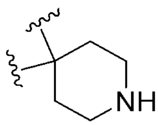 ,

or

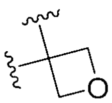 ;

or, two $R_e$ attached to different carbon atoms together with the atoms to which they are respectively attached form

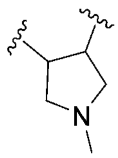 ;

preferably, $X_1$, $X_2$, and $X_3$ are all CH, or one of $X_1$, $X_2$, and $X_3$ is N;
preferably, $R_x$ is selected from H, CN, halogen, $C_{1-6}$ alkyl, or $C_{1-6}$ alkoxy;
preferably, $R_x$ is selected from H, F, Cl, CN, methoxy, or methyl.

**6.** The compound, and the racemate, stereoisomer, tautomer, isotope-labeled counterpart, nitrogen oxide, solvate, polymorph, metabolite, ester, prodrug or pharmaceutically acceptable salt thereof according to any one of claims 1 to 5, wherein the compound shown as formula (I) has a structure shown below:

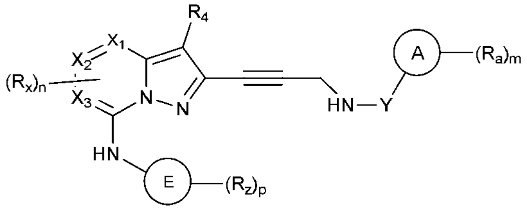

IA-1

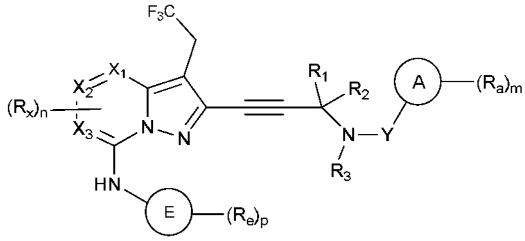

IA-2

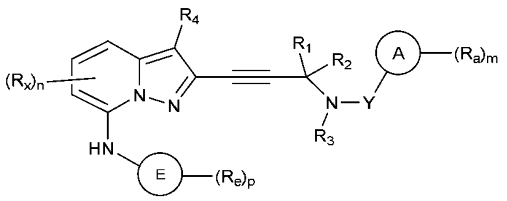

IA-3

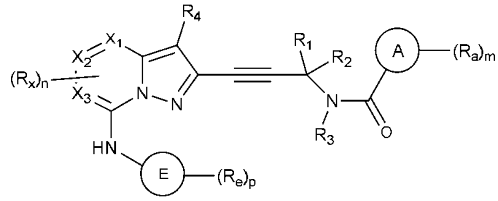

IA-4

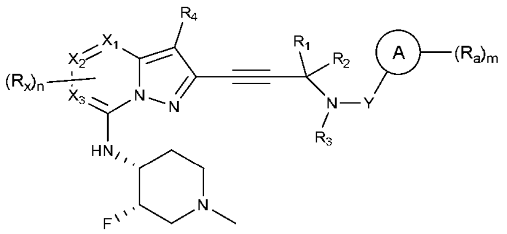

IA-5

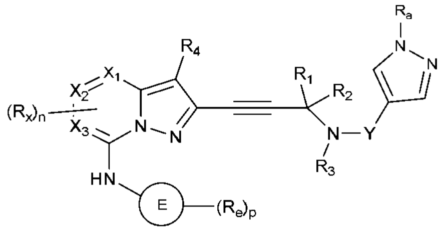

IA-6

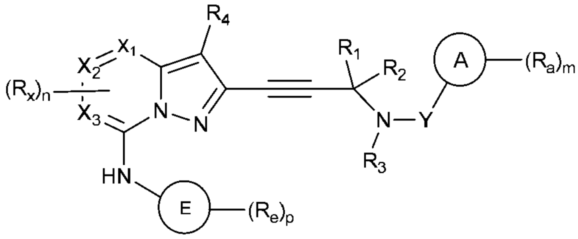

IA-7
,

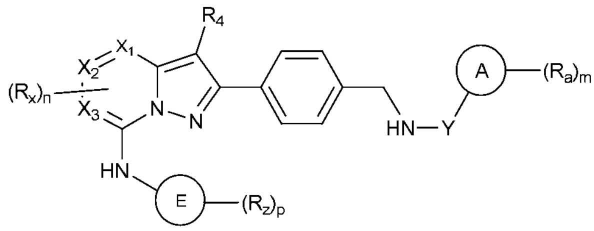

IA-8
,

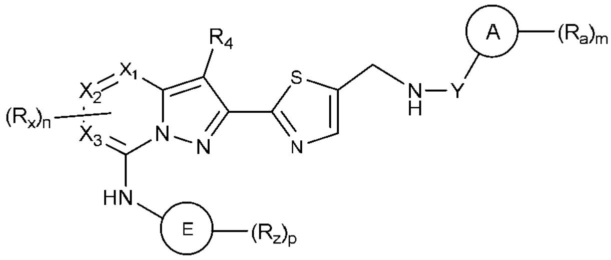

IA-9

or

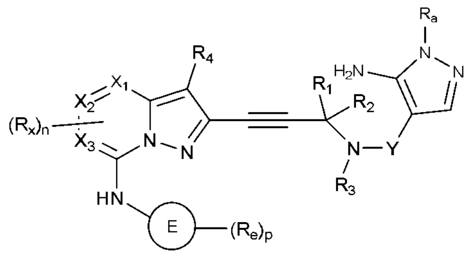

IA-10
;

wherein ring A, ring E, $X_1$, $X_2$, $X_3$, Y, $R_1$, $R_2$, $R_3$, $R_4$, $R_a$, $R_e$, $R_x$, m, n, and p are as defined in any one of claims 1 to 5; preferably, the compound shown as formula (I) has a structure shown below:

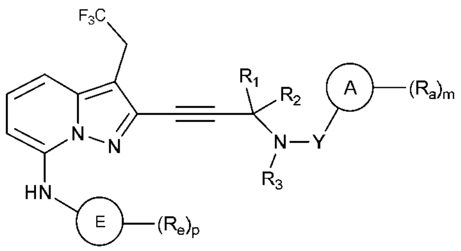

IB-1

,

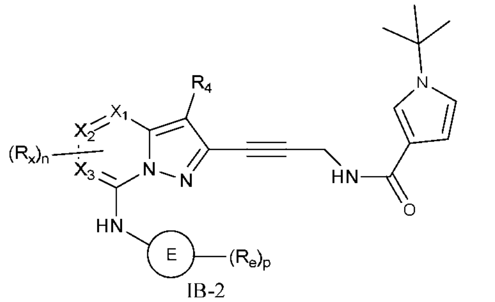

IB-2

or

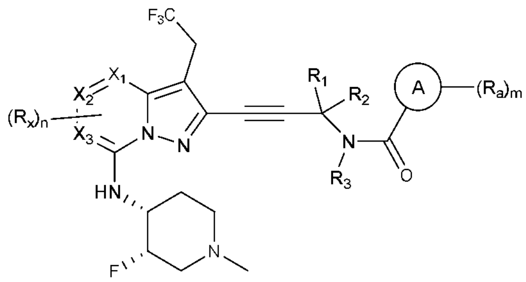

IB-3

;

wherein ring A, ring E, $X_1$, $X_2$, $X_3$, Y, $R_1$, $R_2$, $R_3$, $R_4$, $R_a$, $R_e$, $R_x$, m, n, and p are as defined in any one of claims 1 to 5; preferably, the compound shown as formula (I) has a structure shown below:

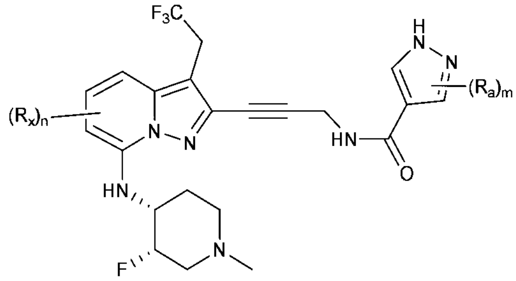

II

wherein $R_a$, $R_x$, m, and n are as defined in any one of claims 1 to 5;
preferably, the compound shown as formula (I) has a structure shown below:

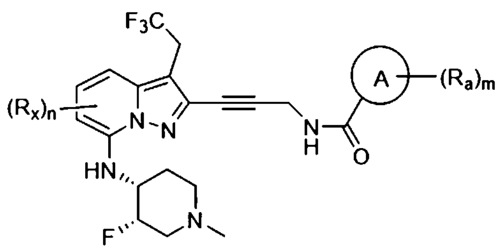

III

wherein ring A, $R_a$, $R_x$, m, and n are as defined in any one of claims 1 to 5;
preferably, the compound shown as formula (I) has a structure shown below:

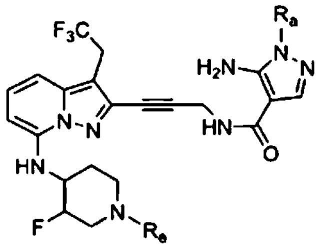

IV

or

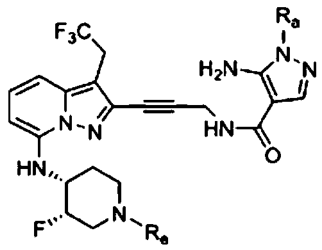

IV-1

wherein $R_a$ and $R_e$ are as defined in any one of claims 1 to 5.

7. The compound, and the racemate, stereoisomer, tautomer, isotope-labeled counterpart, nitrogen oxide, solvate, polymorph, metabolite, ester, prodrug or pharmaceutically acceptable salt thereof according to any one of claims 1 to 6, wherein the compound shown as formula (I) is selected from the following structures:

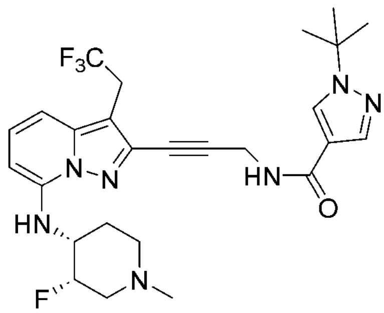

001 ,

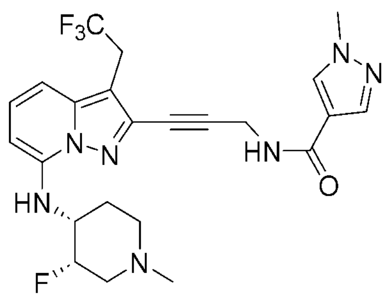

002 ,

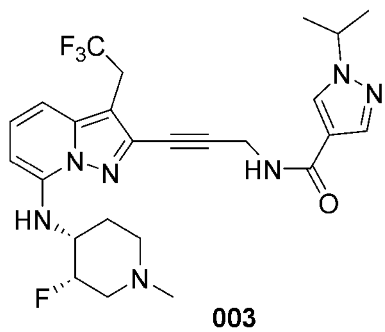

003 ,

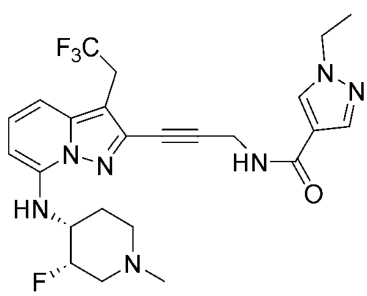

004 ,

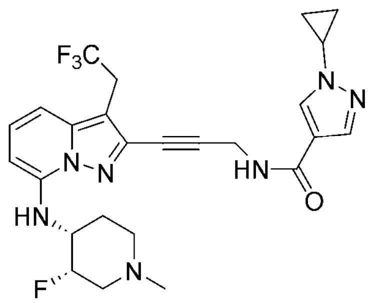

005 ,

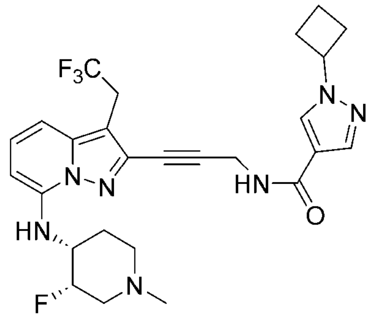

006 ,

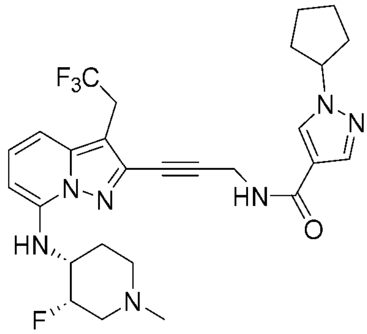

007 ,

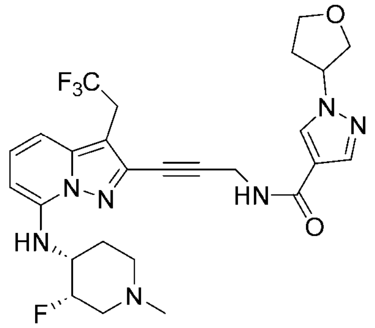

008 ,

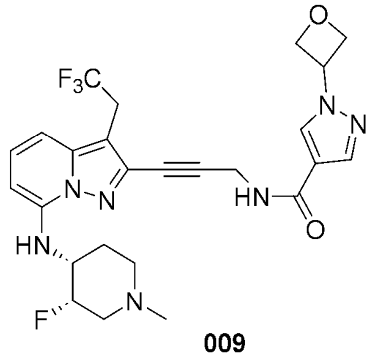

009 ,

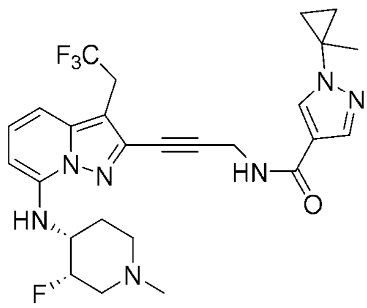

**010** ,

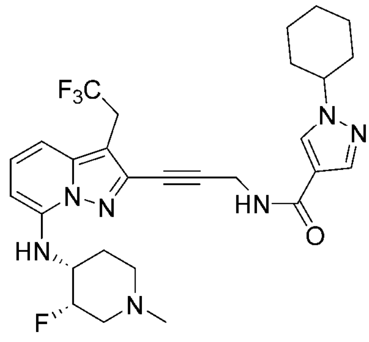

**011** ,

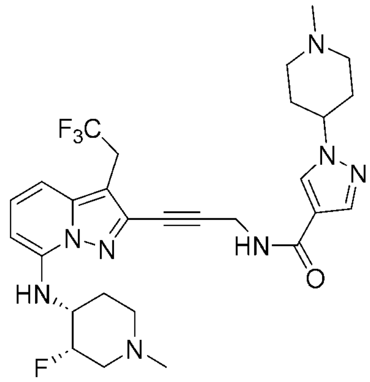

**012** ,

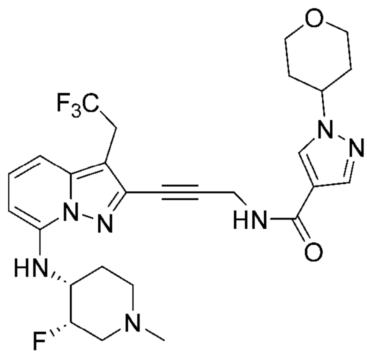

**013** ,

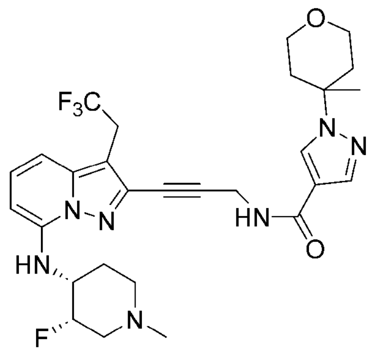

**014** ,

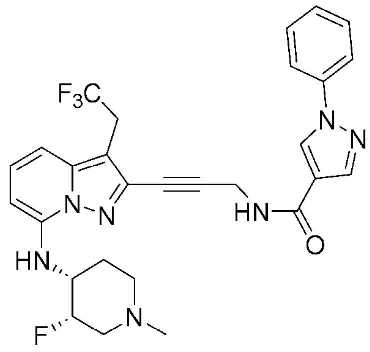

**015** ,

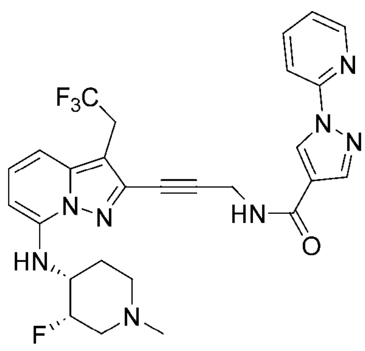

**016** ,

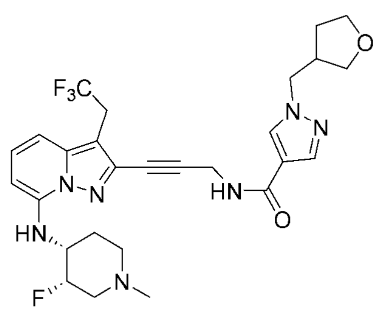

**017** ,

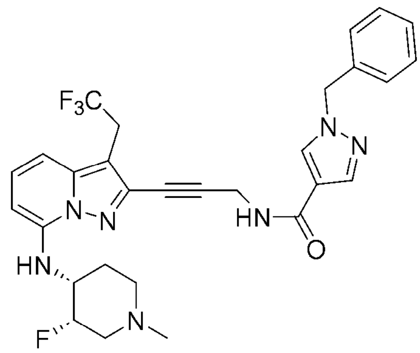

**018** ,

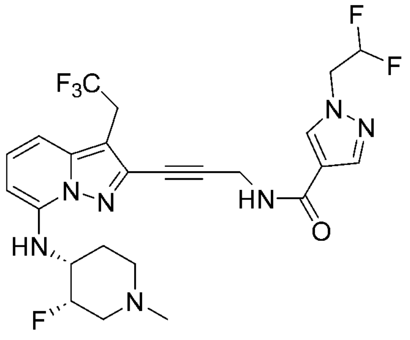

019 ,

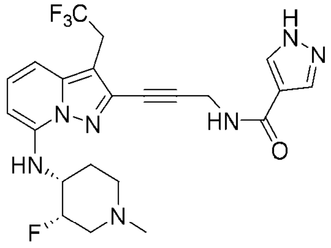

020 ,

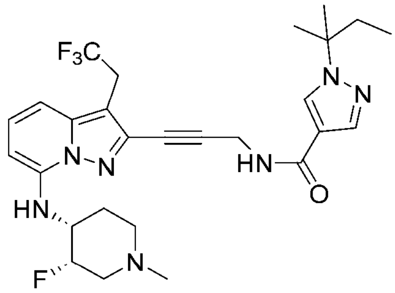

021 ,

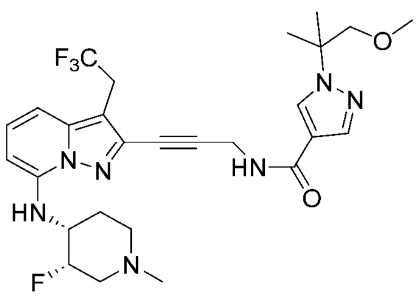

022 ,

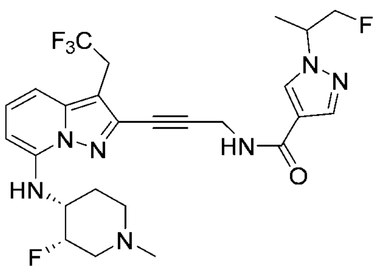

023 ,

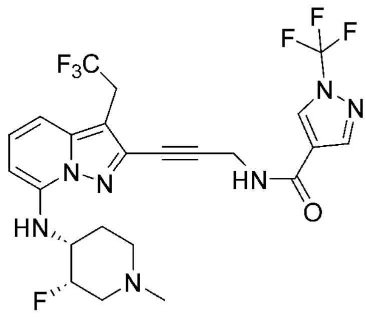

024 ,

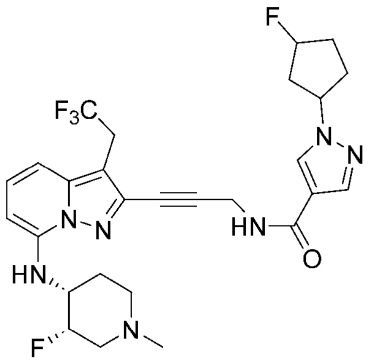

025 ,

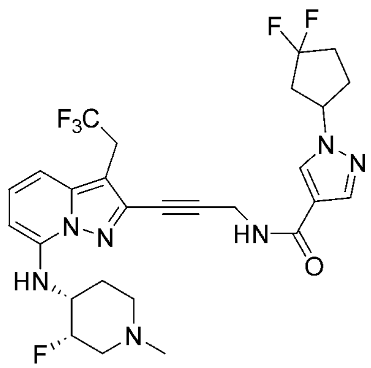

026 ,

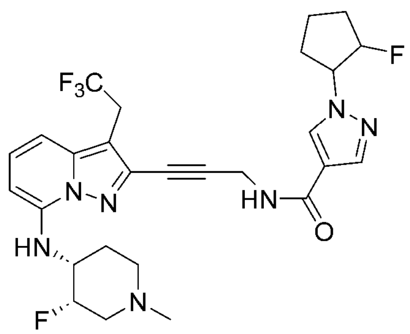

027 ,

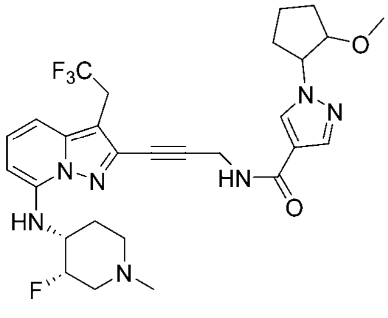

028 ,

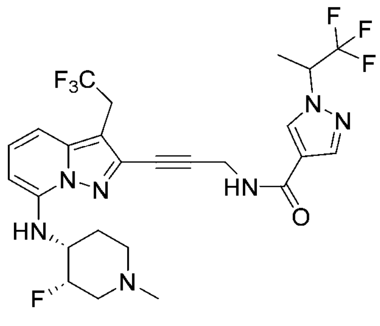

029 ,

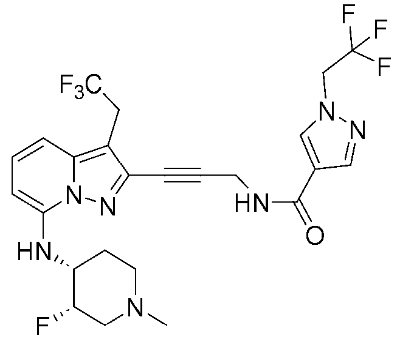

030 ,

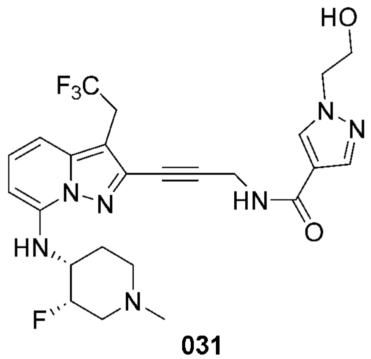

031 ,

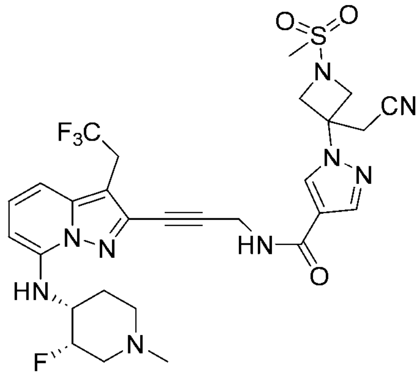

032 ,

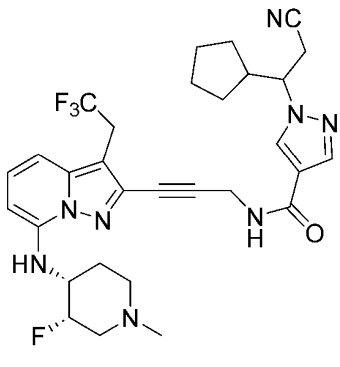

033 ,

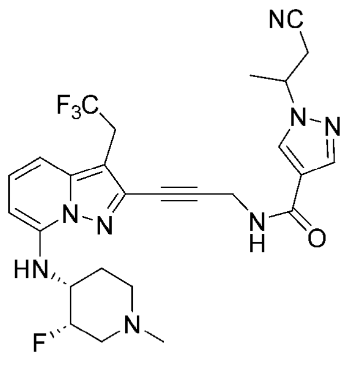

034 ,

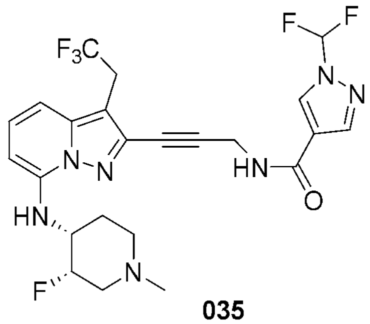

035 ,

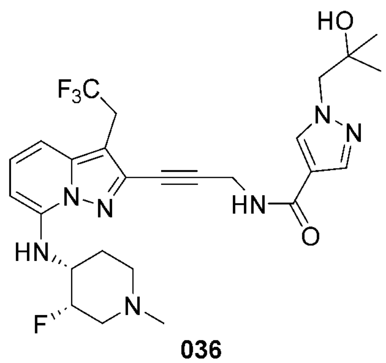

036 ,

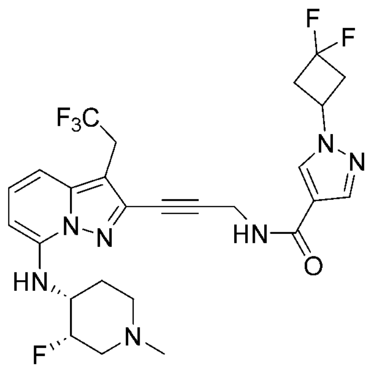

037 ,

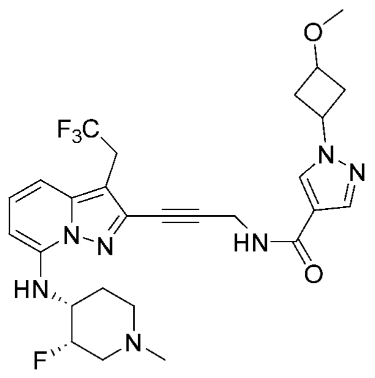

038 ,

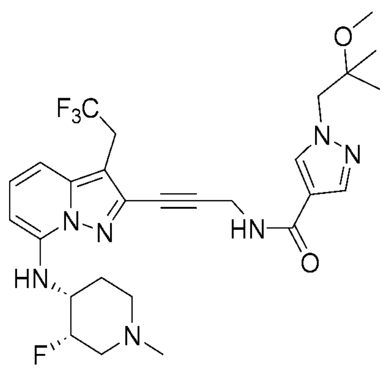

039 ,

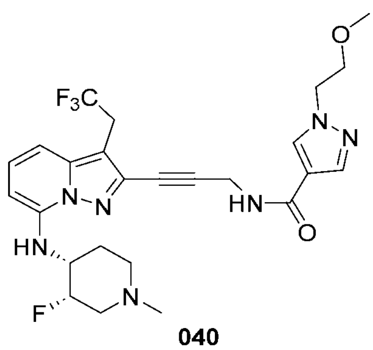

040 ,

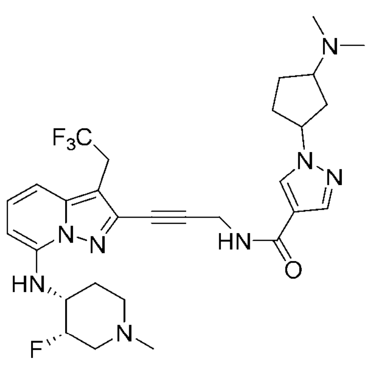

041 ,

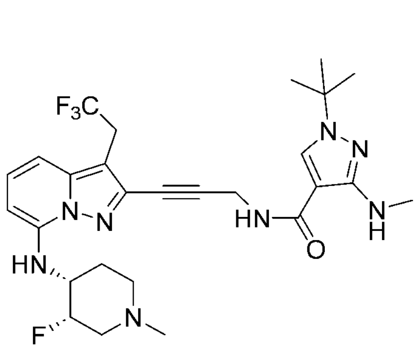

042 ,

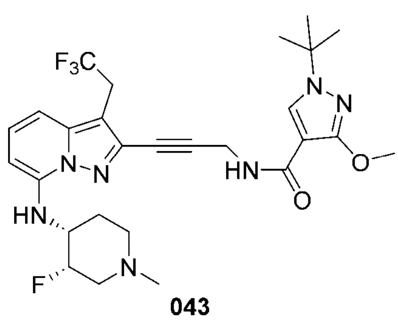
043 ,

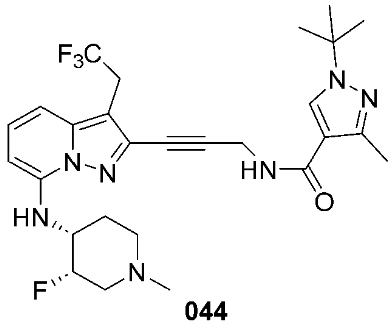
044 ,

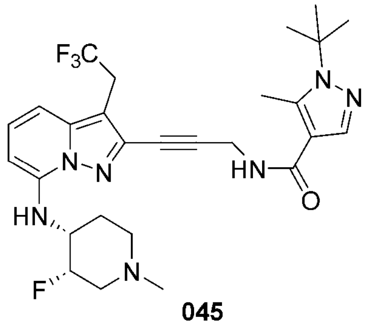
045 ,

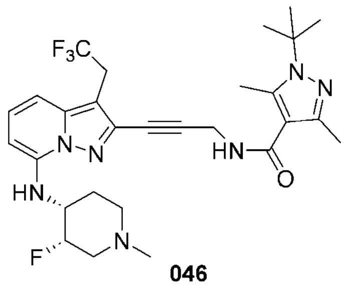
046 ,

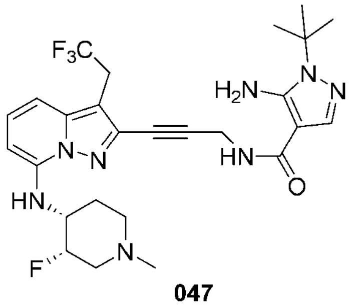
047 ,

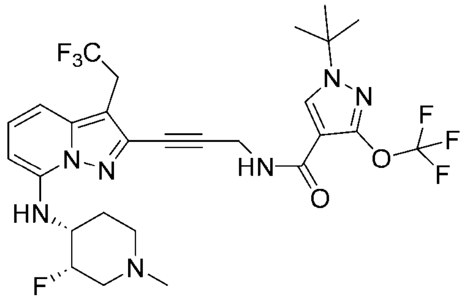
048 ,

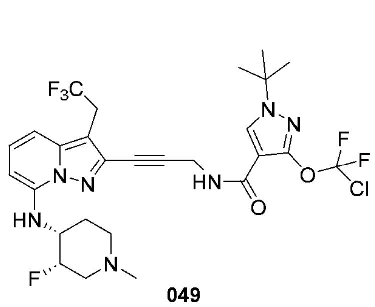
049 ,

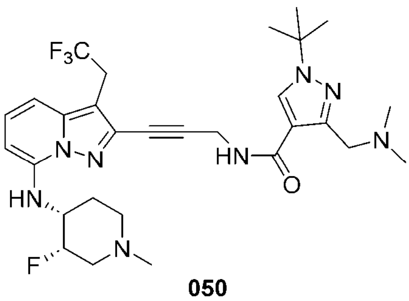
050 ,

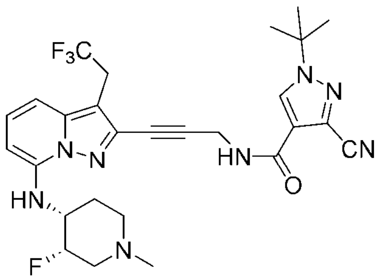
051 ,

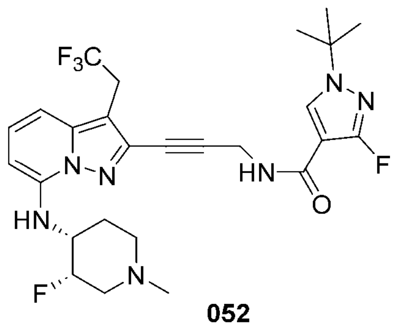
052 ,

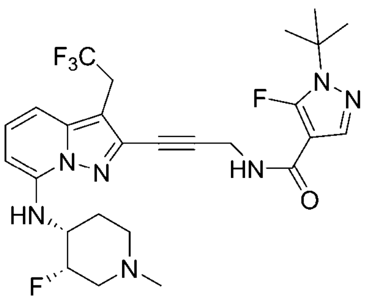
053 ,

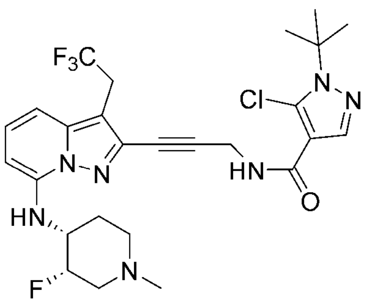
054 ,

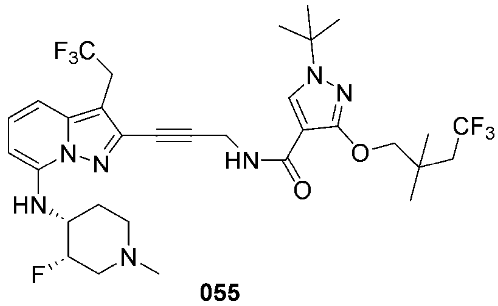
055 ,

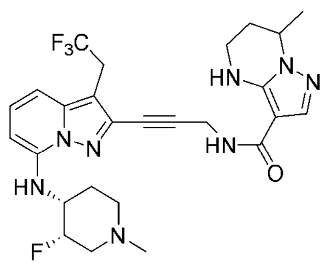
056 ,

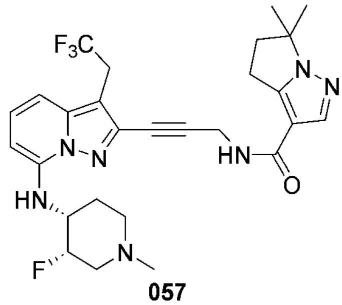
057 ,

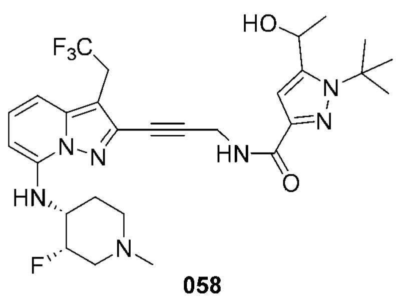
058 ,

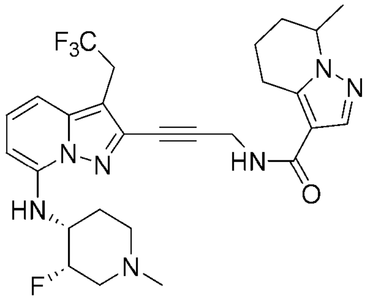
059 ,

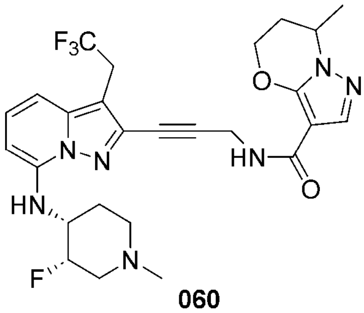
060 ,

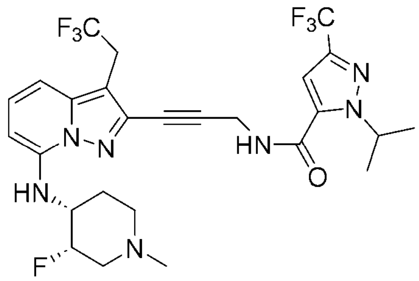
061 ,

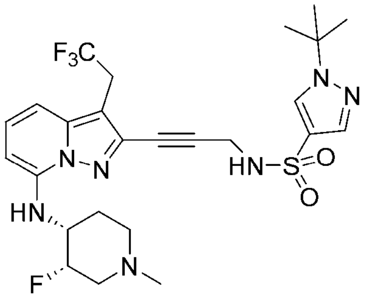
062 ,

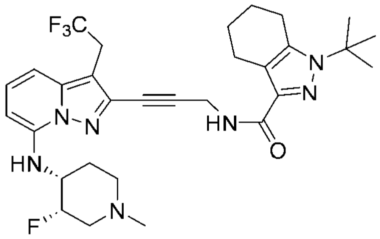
063 ,

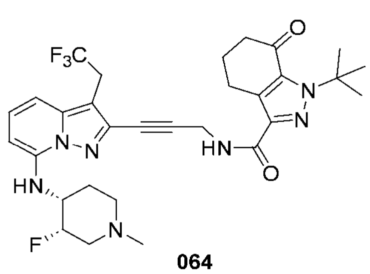
064 ,

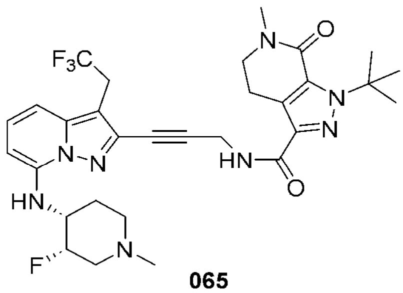
065 ,

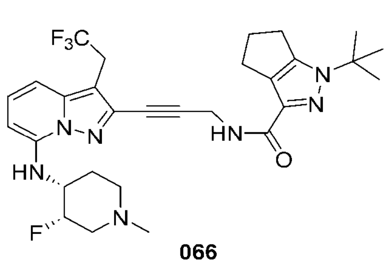
066 ,

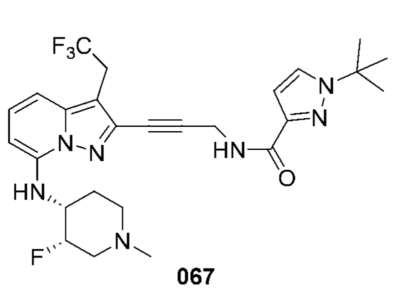
067 ,

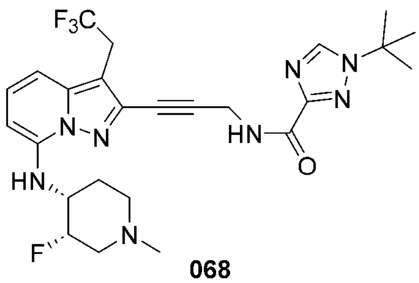
068 ,

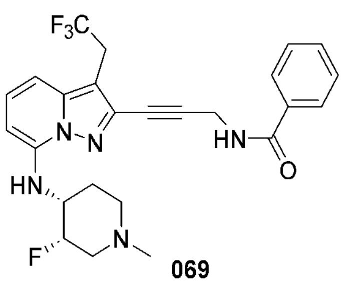
069 ,

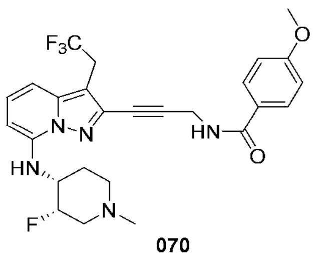
070
,
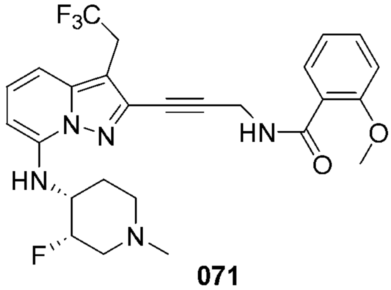
071
,
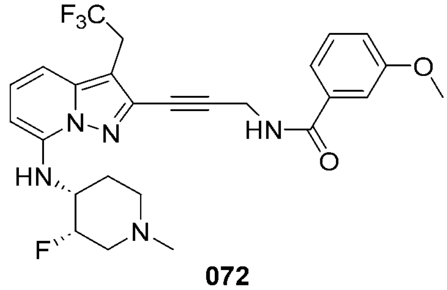
072
,

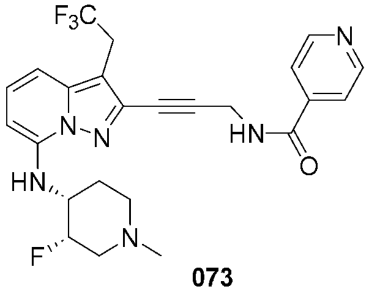
073
,
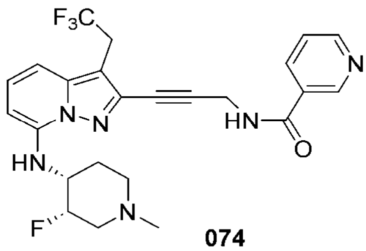
074
,
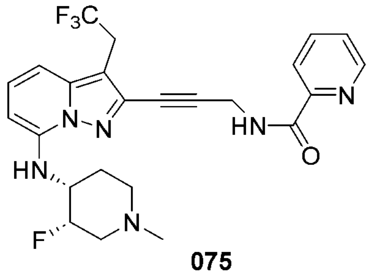
075
,

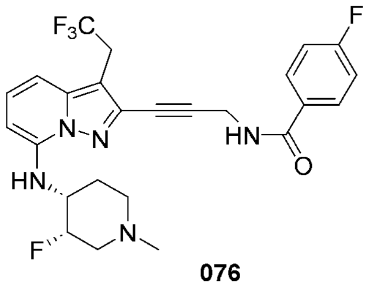
076
,
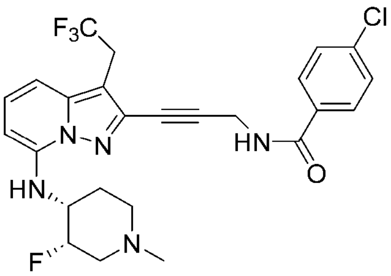
077
,
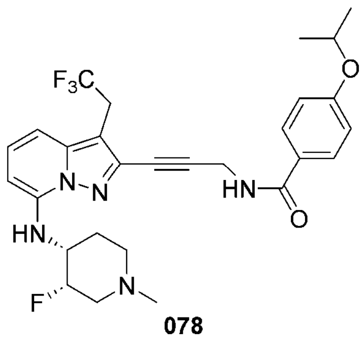
078
,

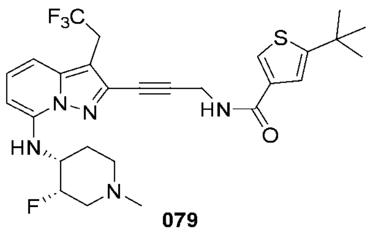
079
,
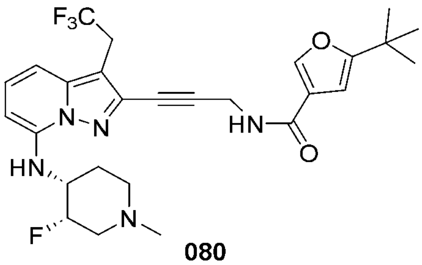
080
,
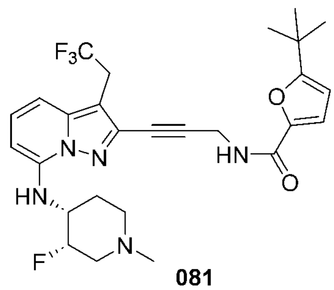
081
,

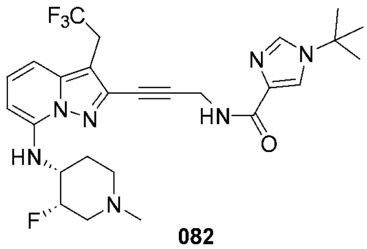
082
,
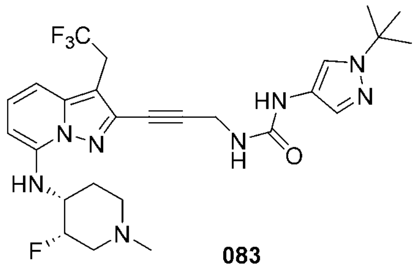
083
,
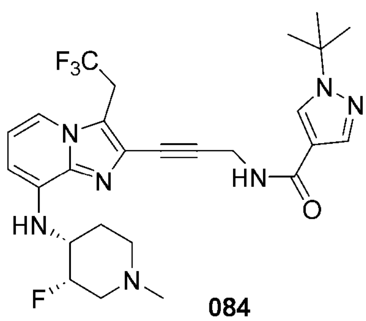
084
,

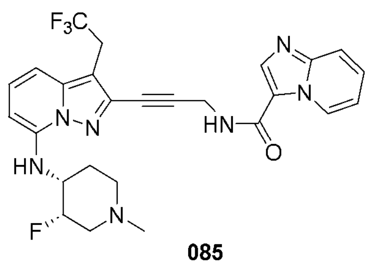
085,
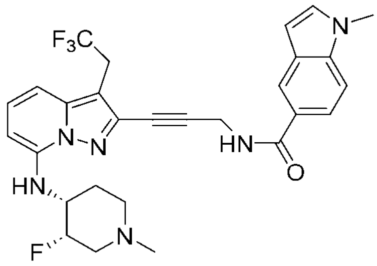
086,
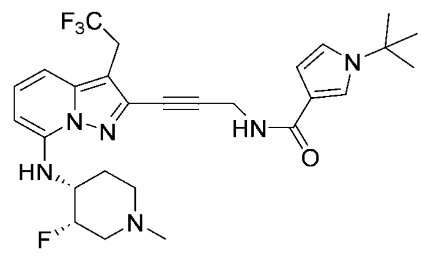
087,
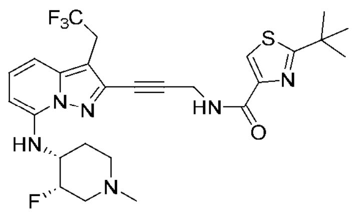
088,
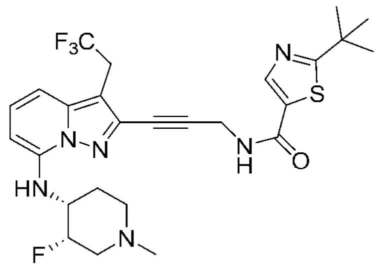
089,
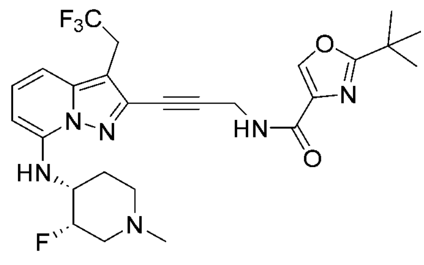
090,
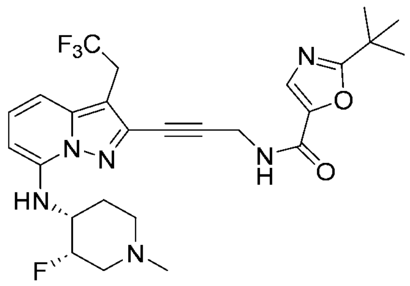
091,
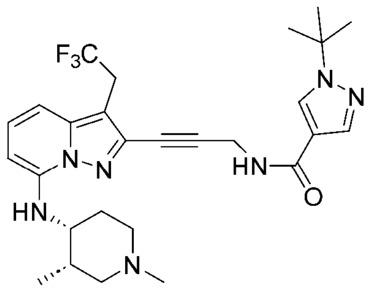
092,
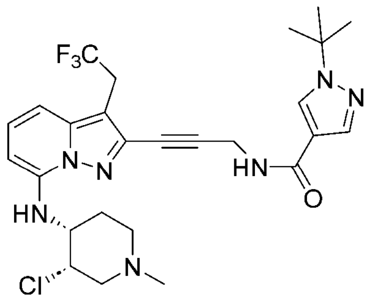
093,
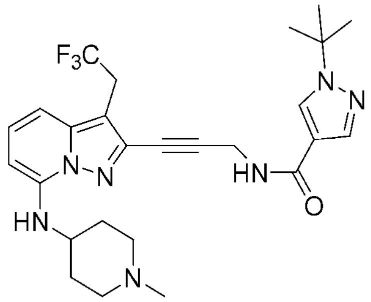
094,
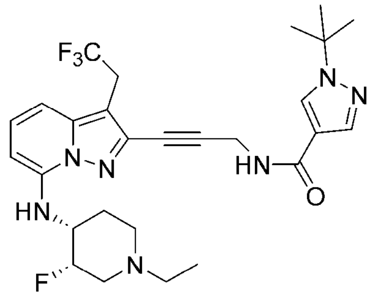
095,
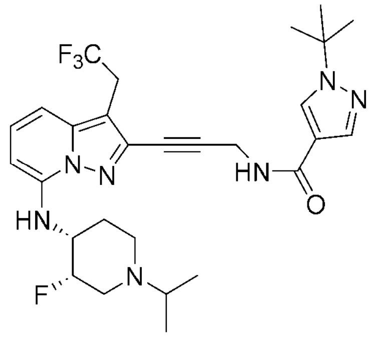
096,

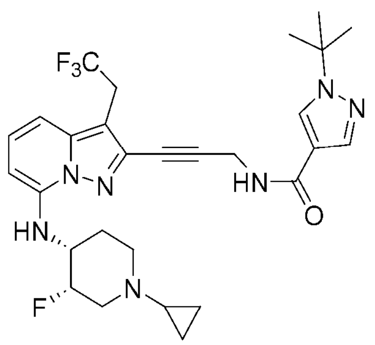

**097** ,

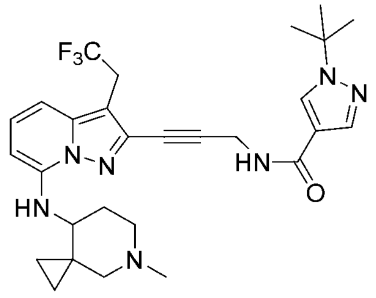

**098** ,

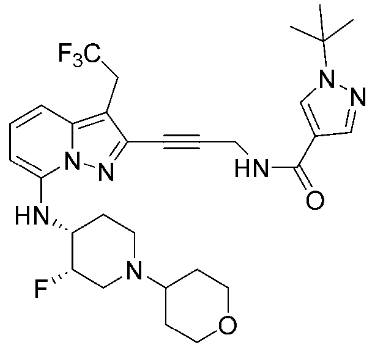

**099** ,

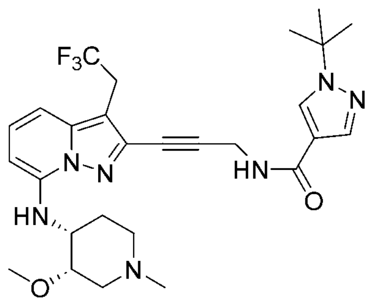

**100** ,

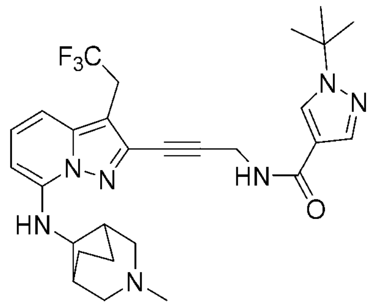

**101** ,

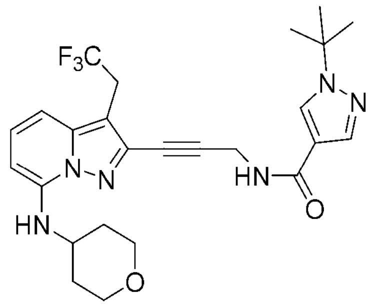

**102** ,

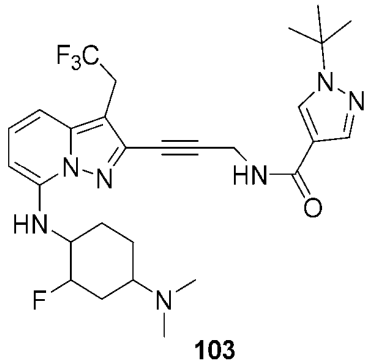

**103** ,

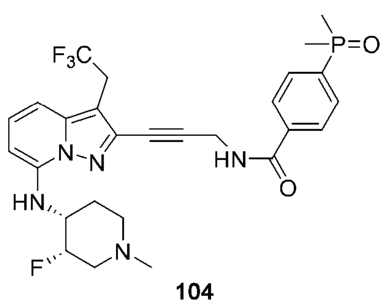

**104** ,

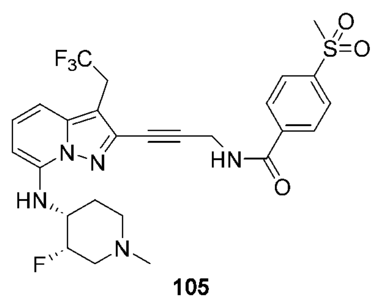

**105** ,

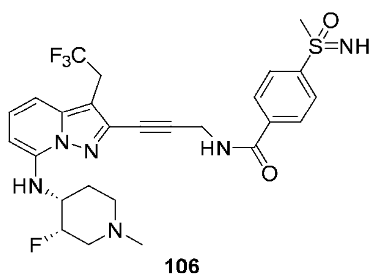

**106** ,

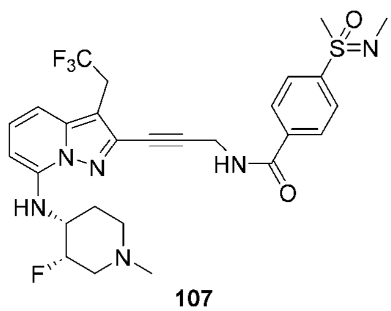

**107** ,

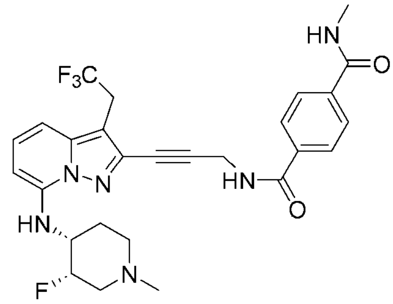

**108** ,

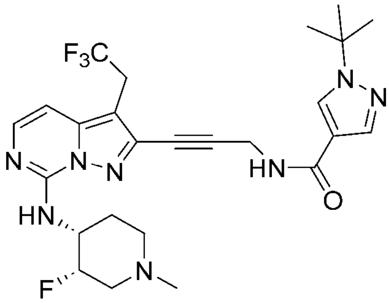

109 ,

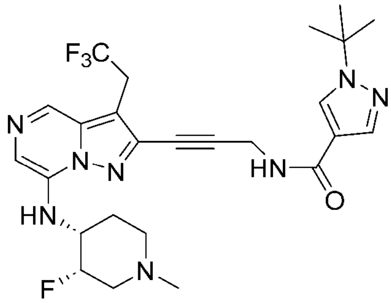

110 ,

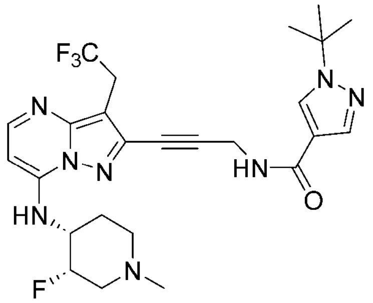

111 ,

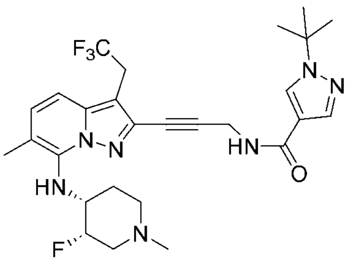

112 ,

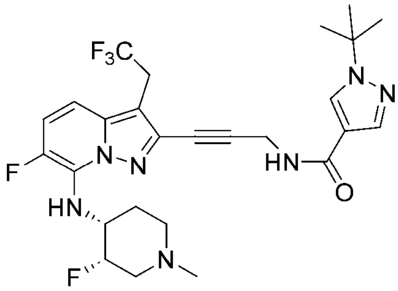

113 ,

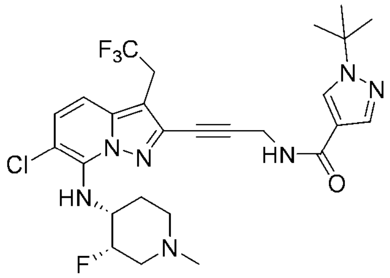

114 ,

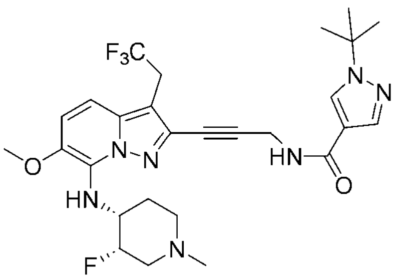

115 ,

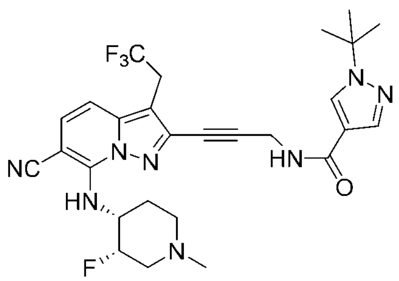

116 ,

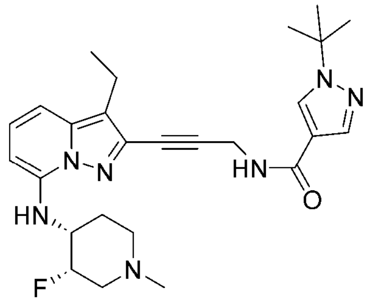

117 ,

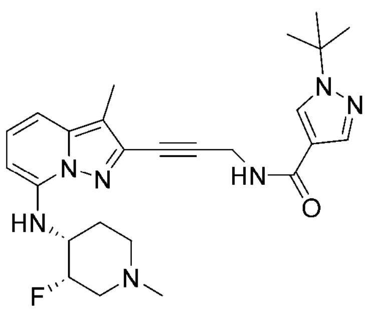

118 ,

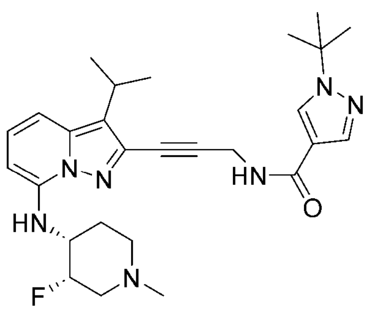

119 ,

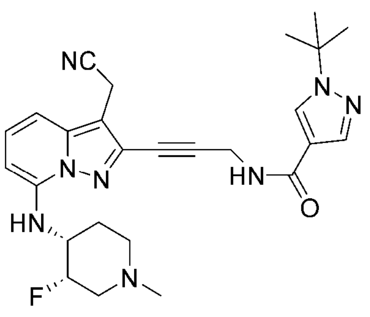

120 ,

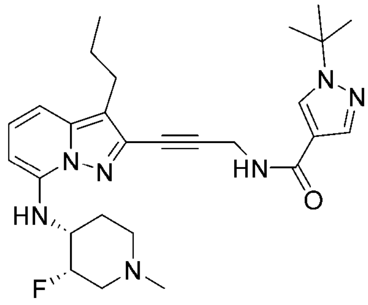
121,

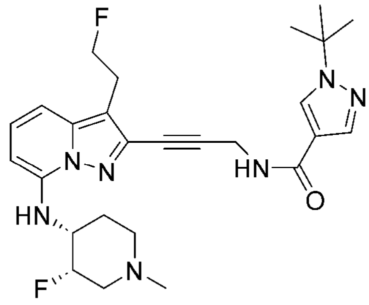
122,

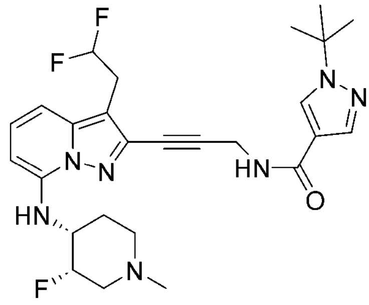
123,

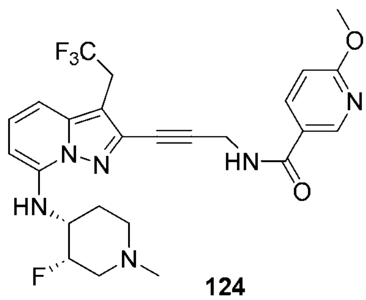
124,

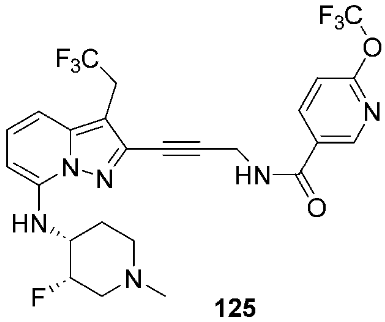
125,

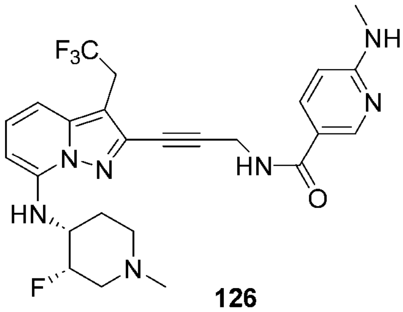
126,

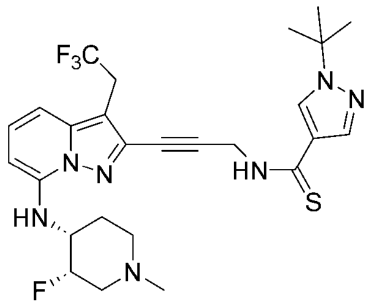
127,

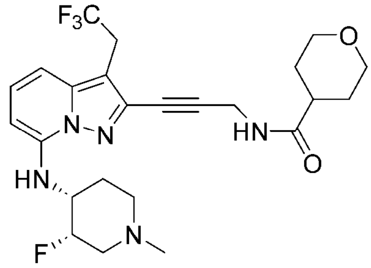
128,

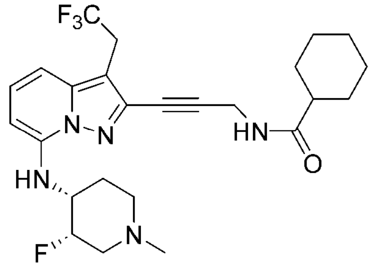
129,

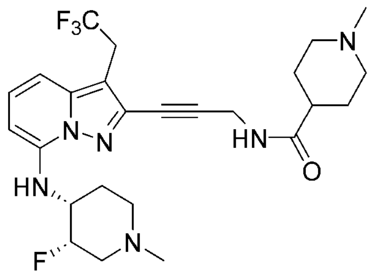
130,

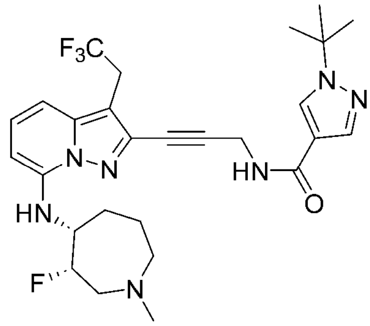
131,

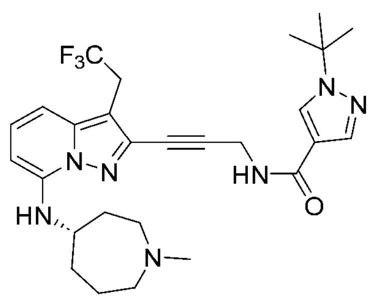
132,

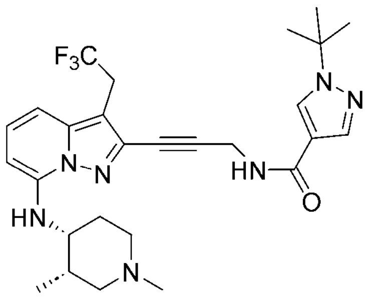
**133** ,

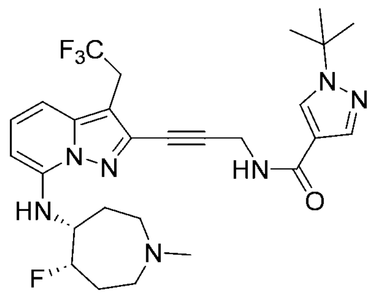
**134** ,

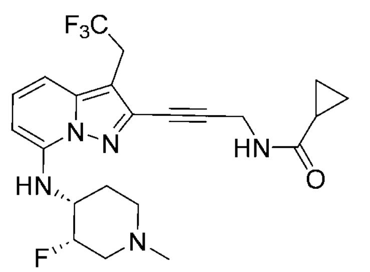
**135** ,

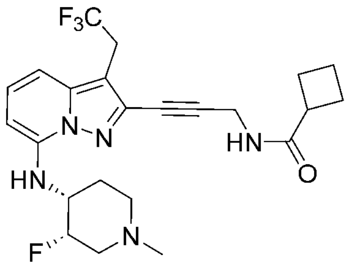
**136** ,

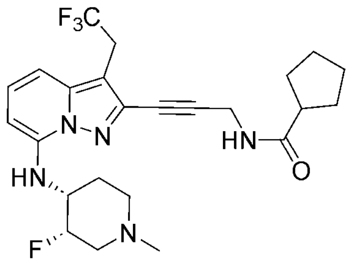
**137** ,

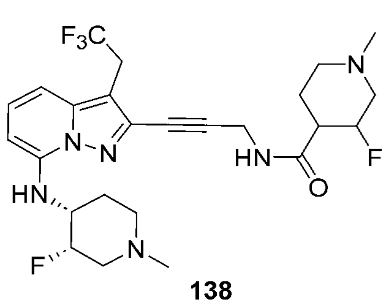
**138** ,

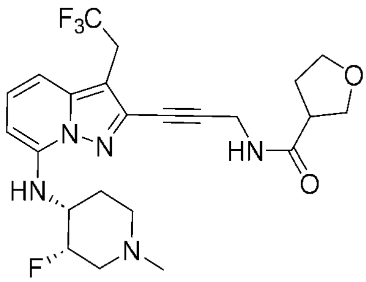
**139** ,

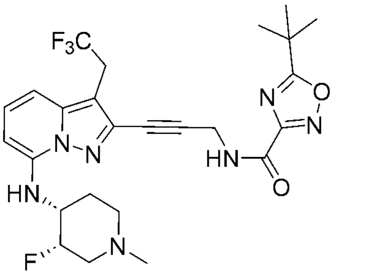
**140** ,

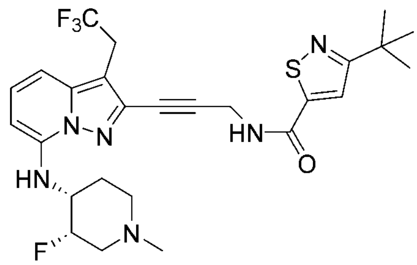
**141** ,

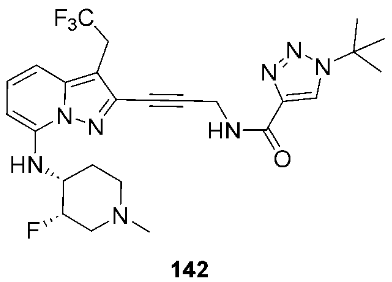
**142** ,

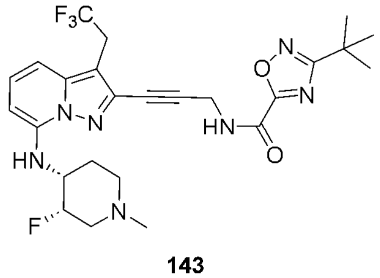
**143** ,

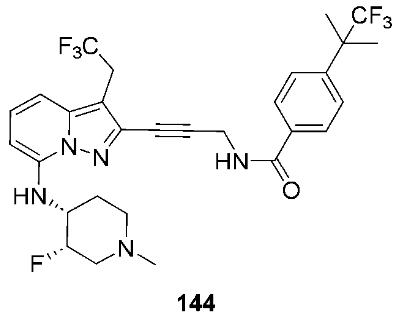
**144** ,

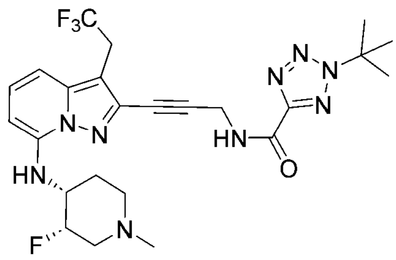
145,
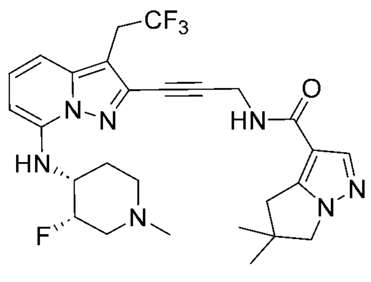
146,
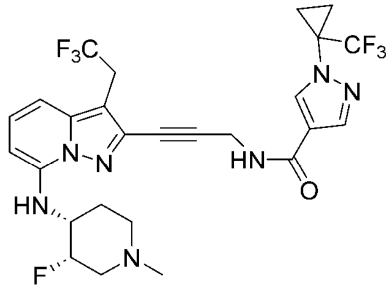
147,
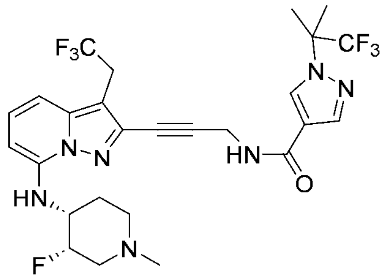
148,
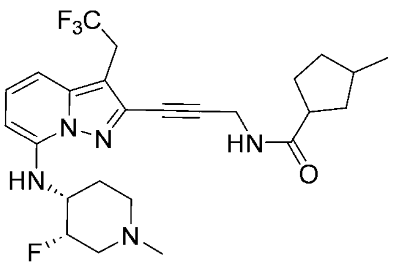
149,
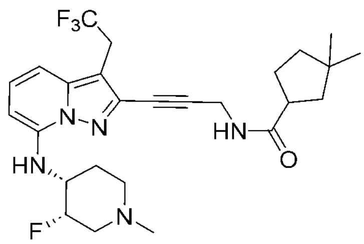
150,
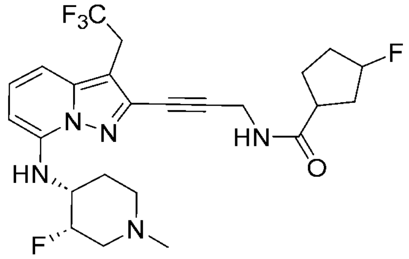
151,
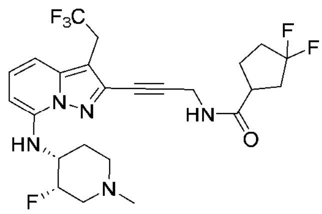
152,
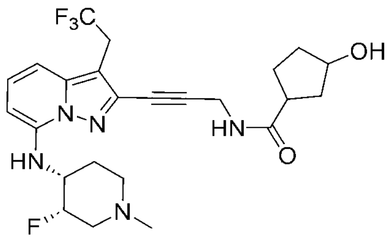
153,
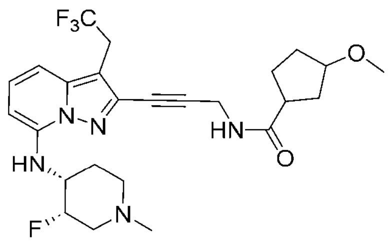
154,
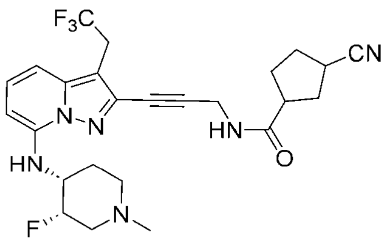
155,
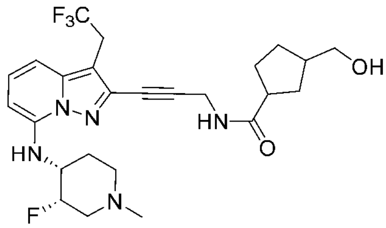
156,
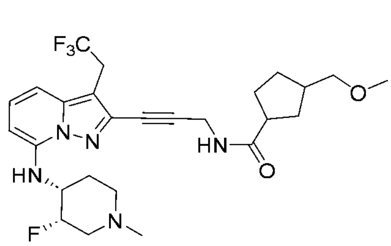
157,
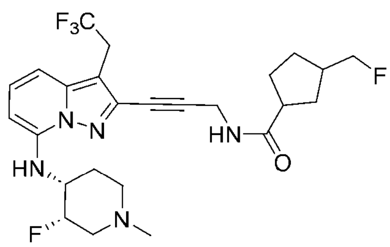
158,
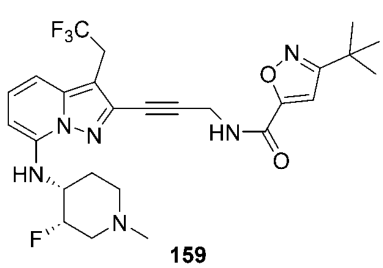
159,

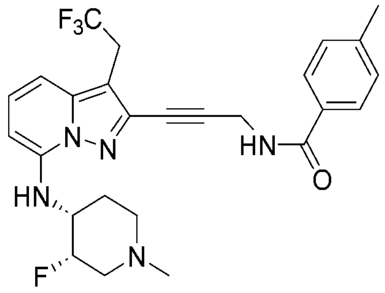
160,
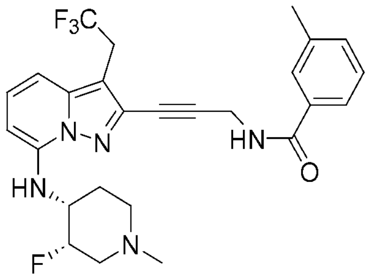
161,
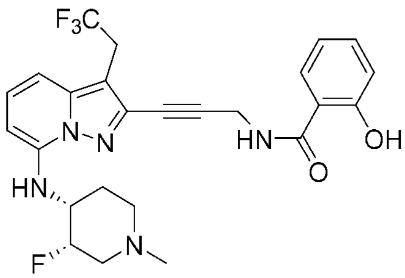
162,

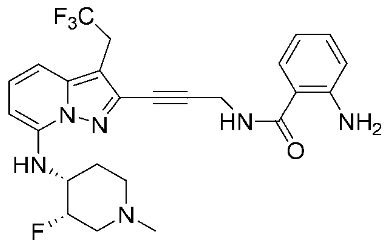
163,
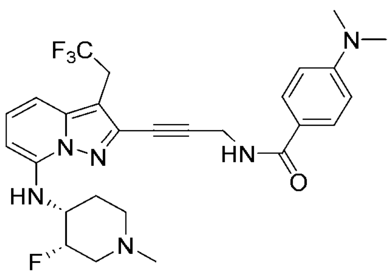
164,
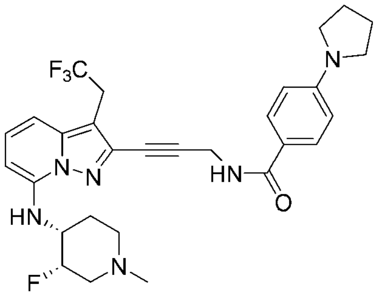
165,

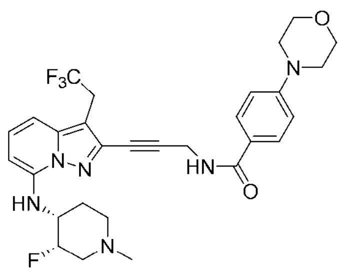
166,
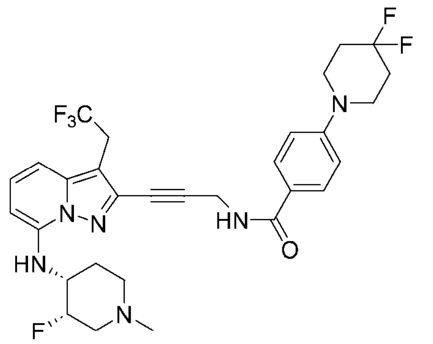
167,
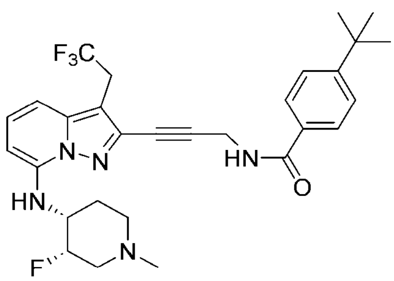
168,

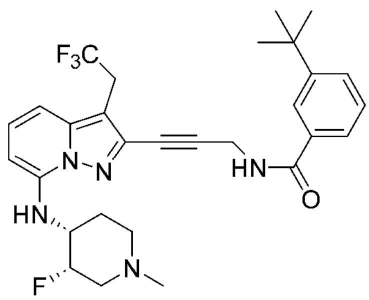
169,
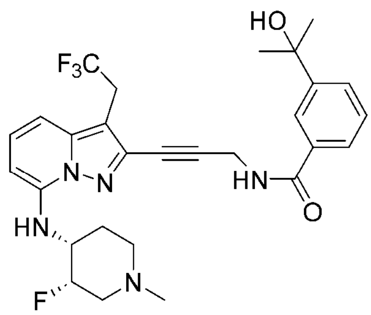
170,
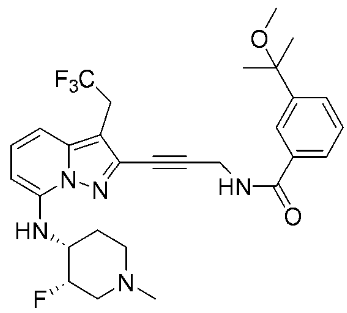
171,

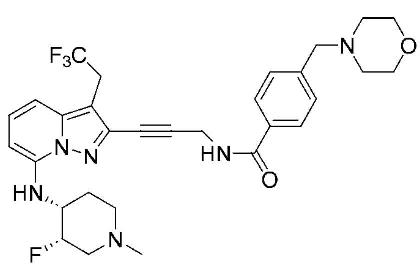

172

,

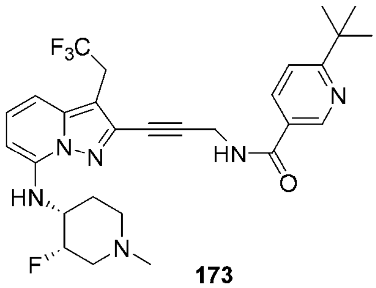

173

,

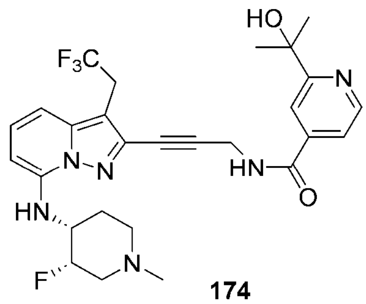

174

,

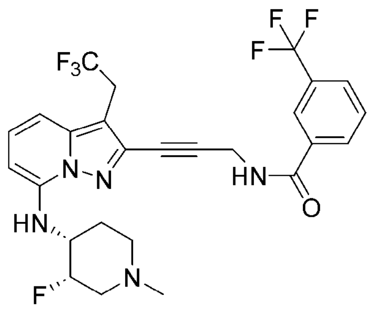

175

,

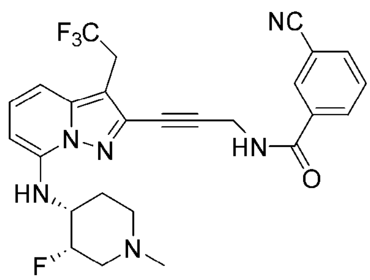

176

,

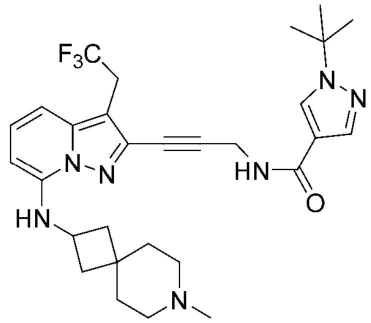

177

,

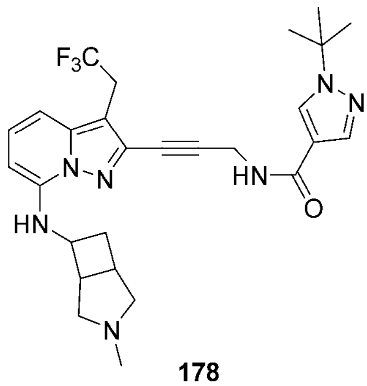

178

,

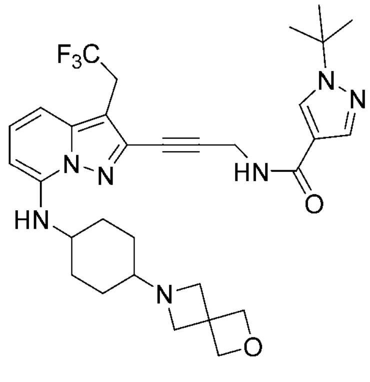

179

,

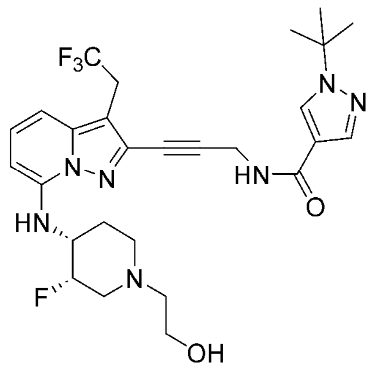

180

,

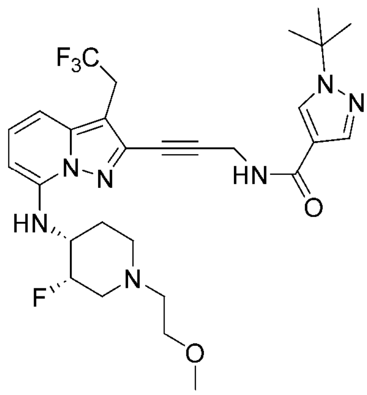

181

,

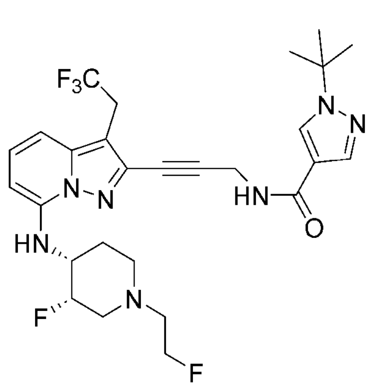

182

,

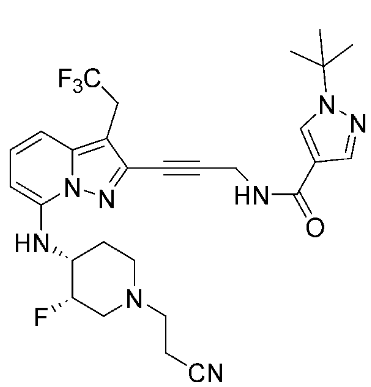

183

,

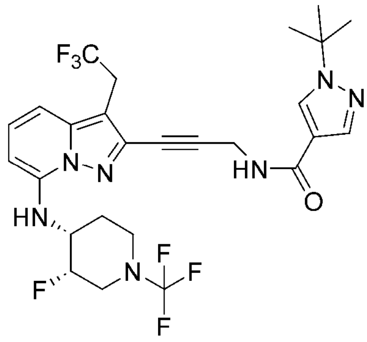
184 ,
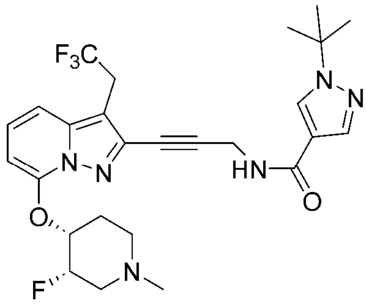
185 ,
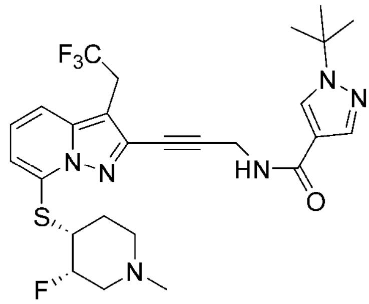
186 ,

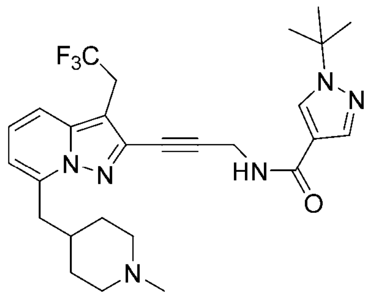
187 ,
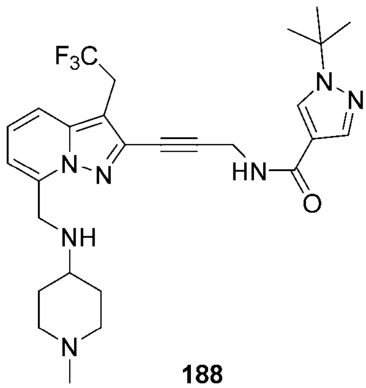
188 ,
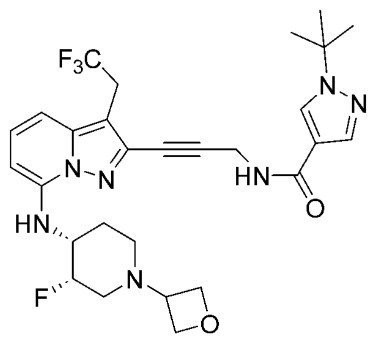
189 ,

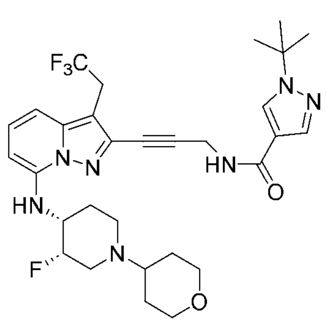
190 ,
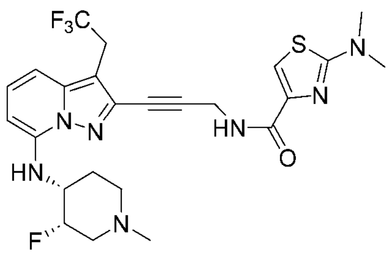
191 ,
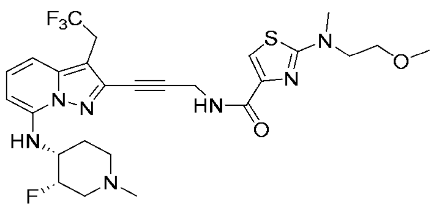
192 ,

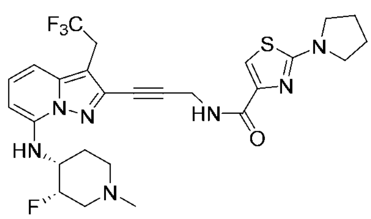
193 ,
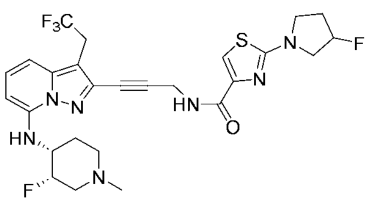
194 ,
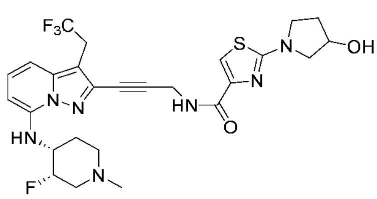
195 ,

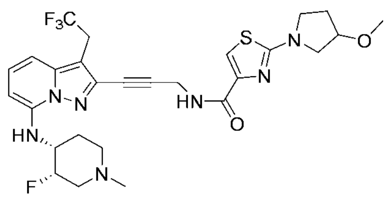
196 ,

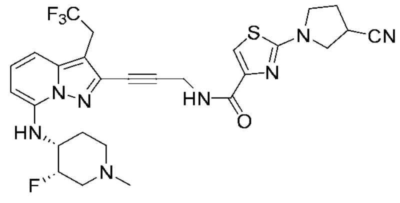
197 ,

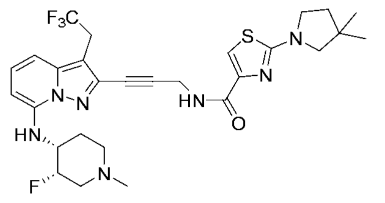
198 ,

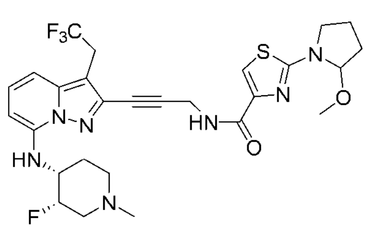
199 ,

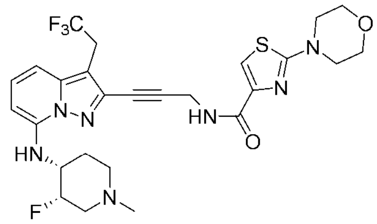
200 ,

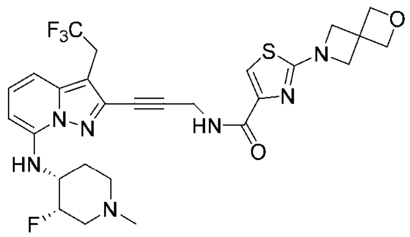
201 ,

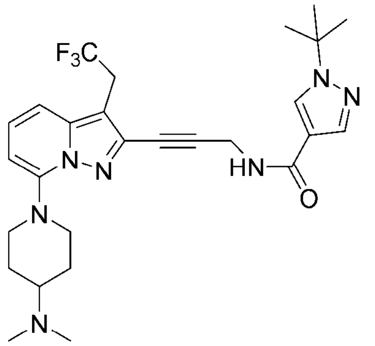
202 ,

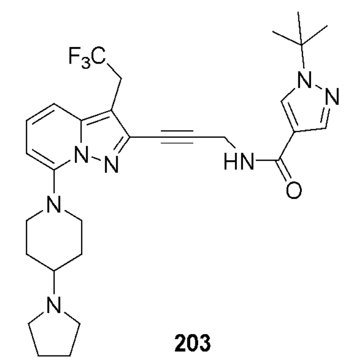
203 ,

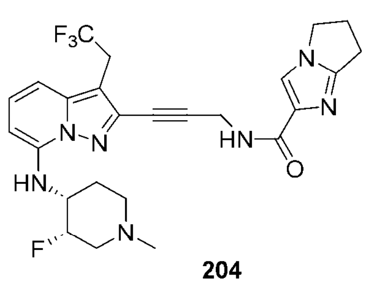
204 ,

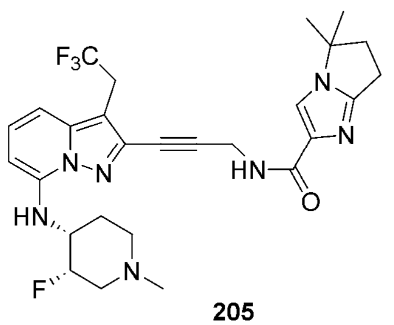
205 ,

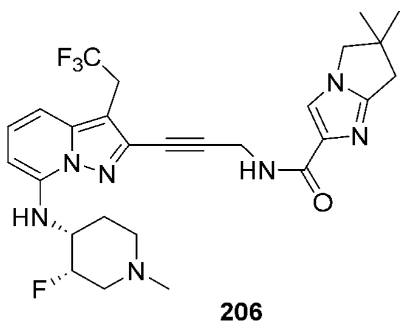
206 ,

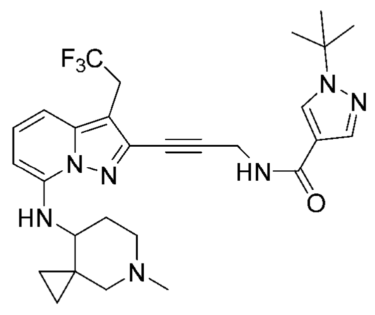
207 ,

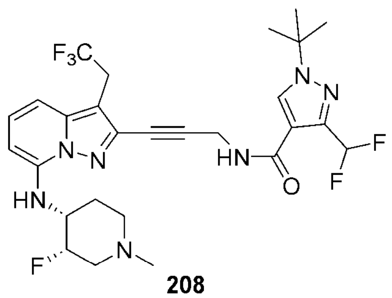
**208** ,

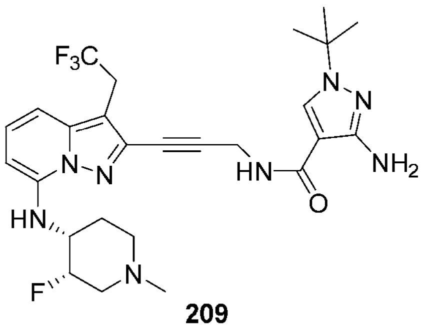
**209** ,

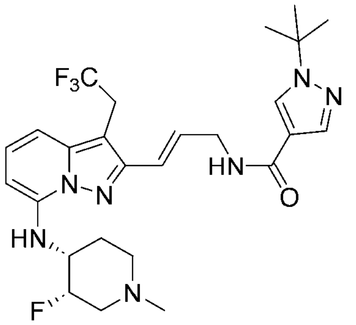
**210** ,

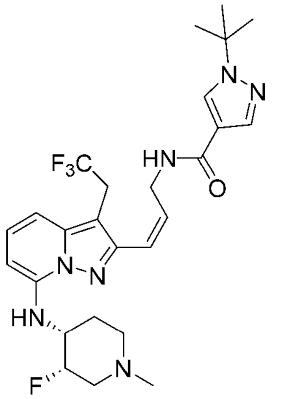
**211** ,

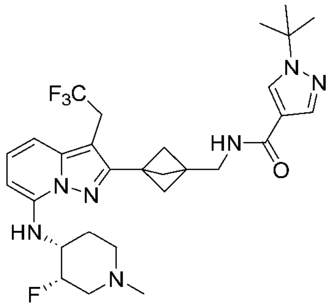
**212** ,

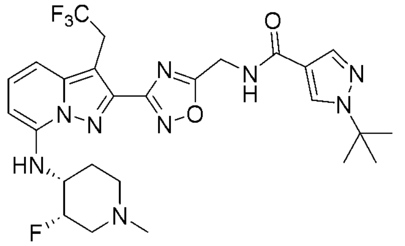
**213** ,

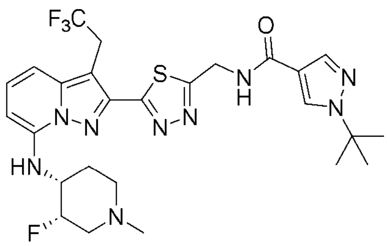
**214** ,

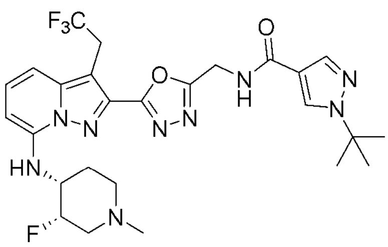
**215** ,

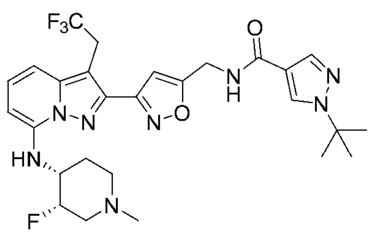
**216** ,

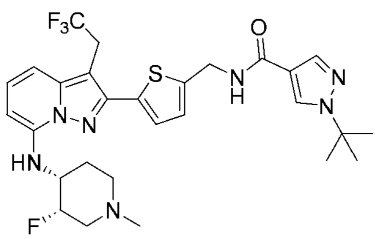
**217** ,

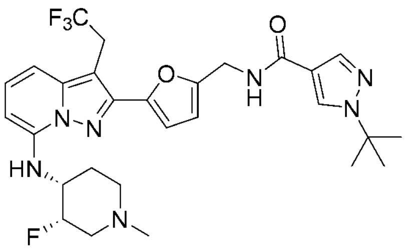
**218** ,

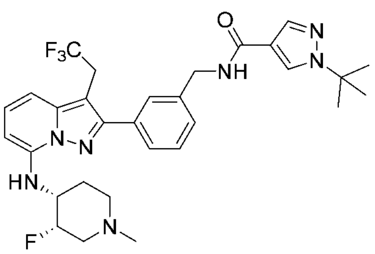
**219** ,

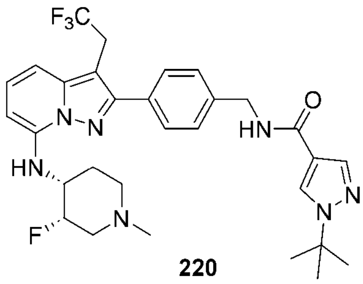
220 ,
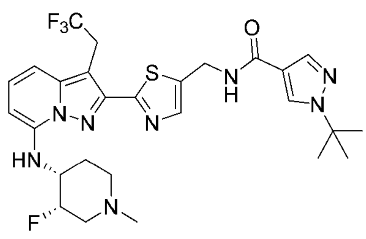
221 ,
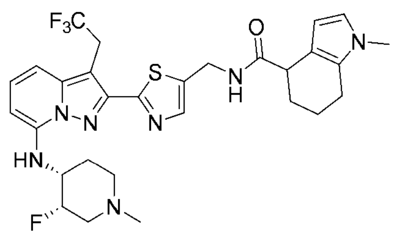
222 ,

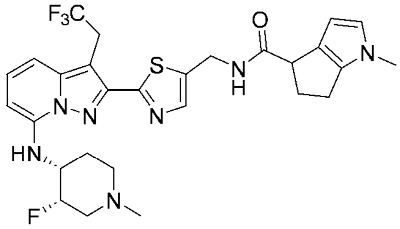
223 ,
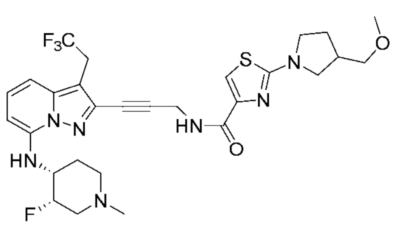
224 ,
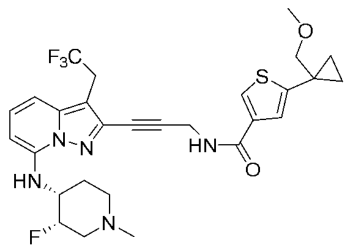
225 ,

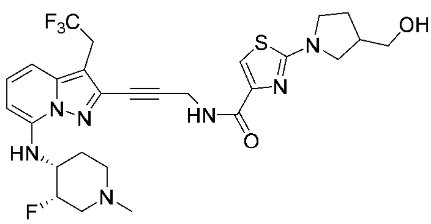
226 ,
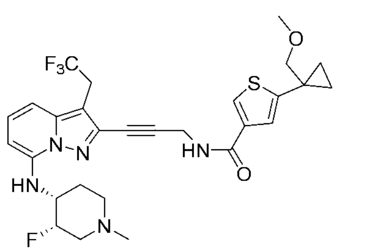
227 ,
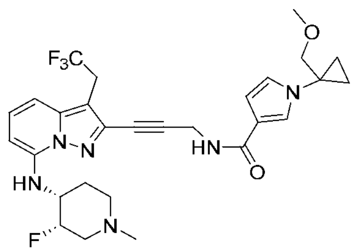
228 ,

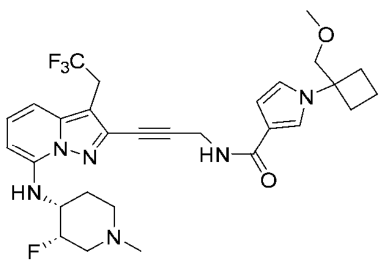
229 ,
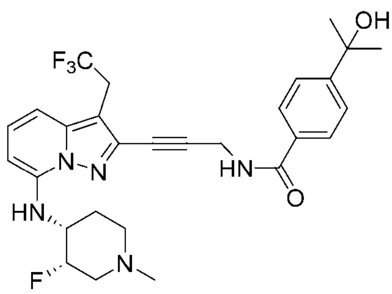
230 ,
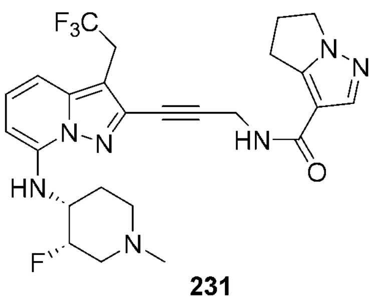
231 ,

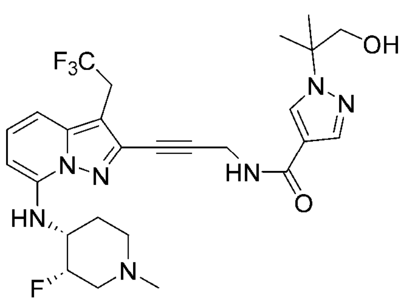
232 ,
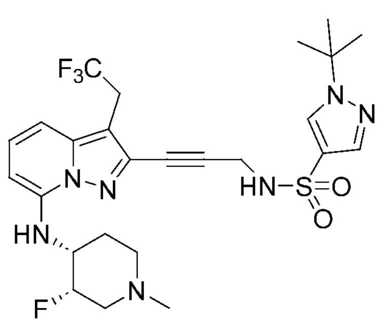
233 ,
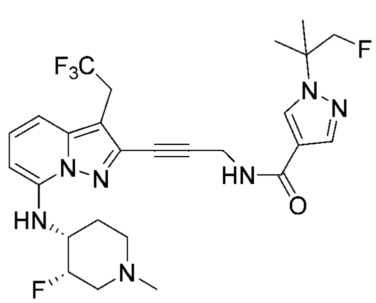
234 ,

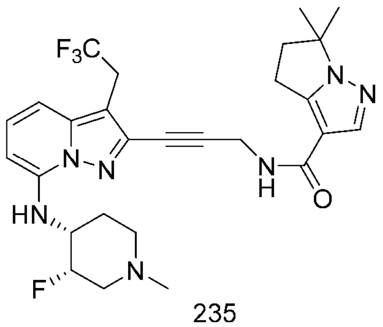
235
,
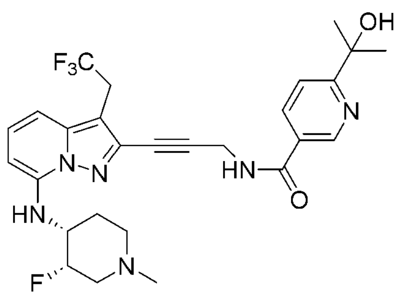
**236**
,
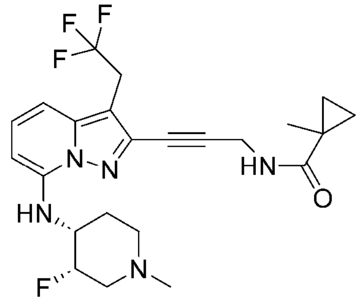
**237**
,

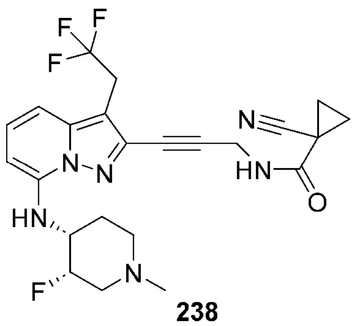
**238**
,
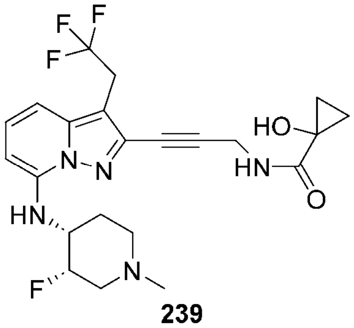
**239**
,
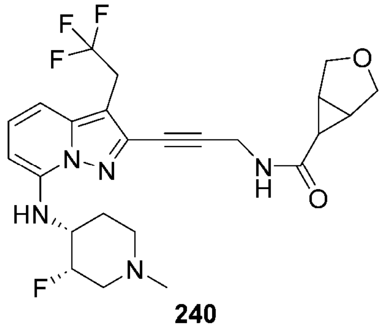
**240**
,

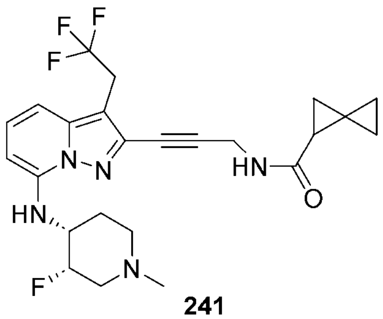
**241**
,
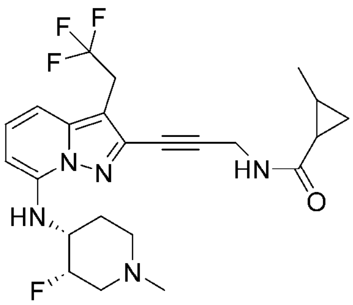
**242**
,
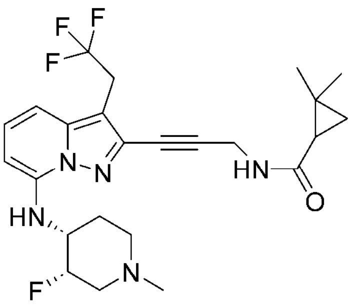
**243**
,

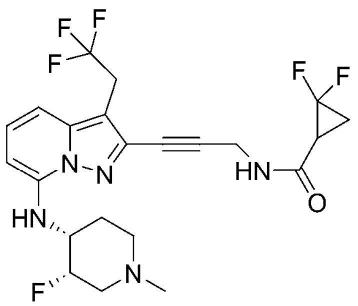
**244**
,
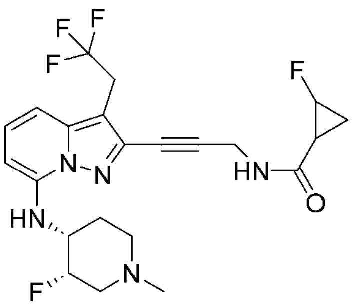
**245**
,
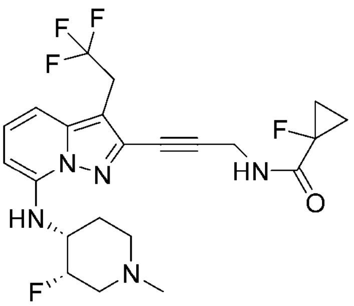
**246**
,

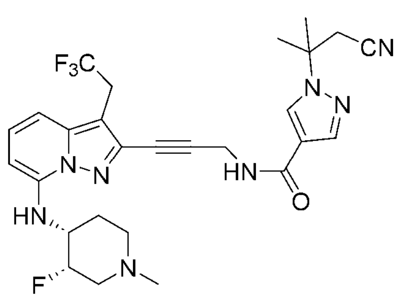

**247** ,

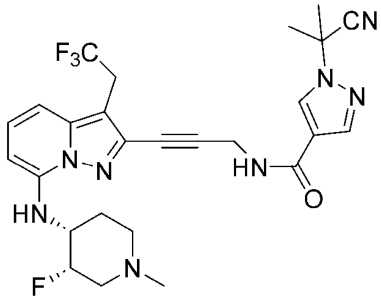

**248** ,

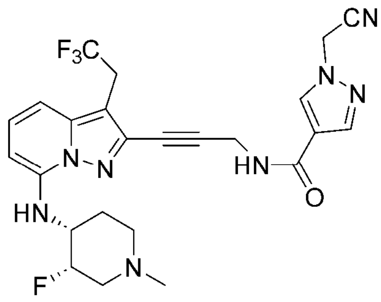

**249** ,

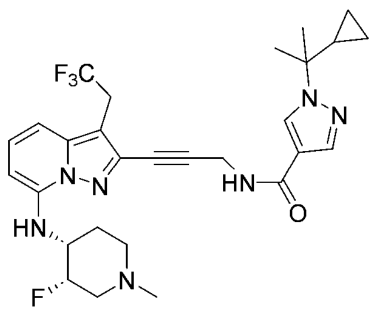

**250** ,

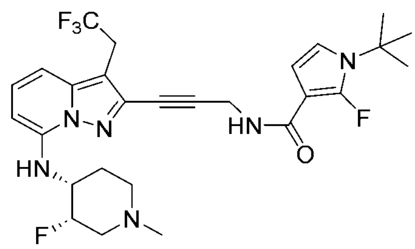

**251** ,

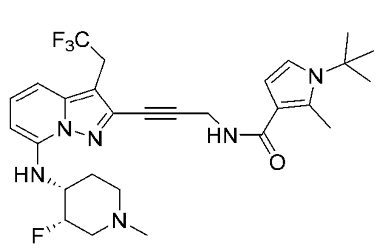

**252** ,

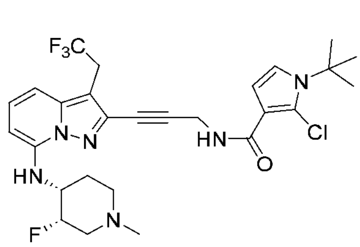

**253** ,

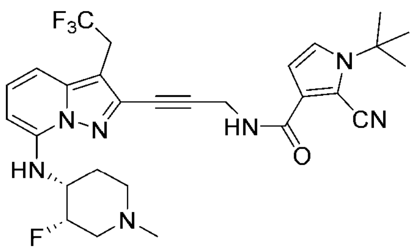

**254** ,

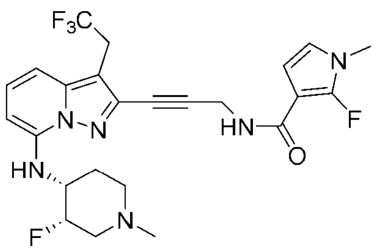

**255** ,

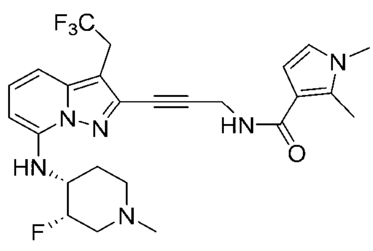

**256** ,

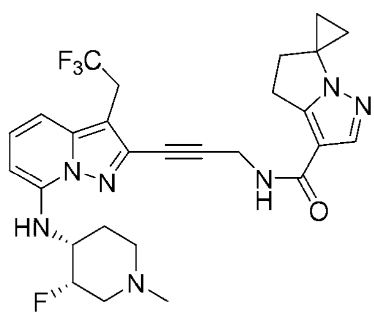

257 ,

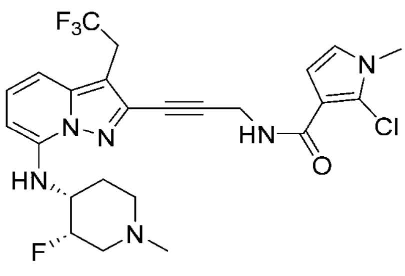

**258** ,

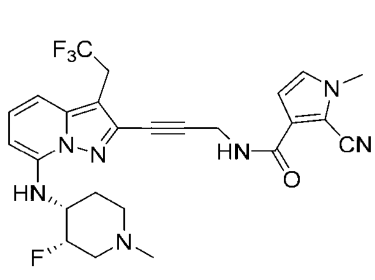
**259** ,

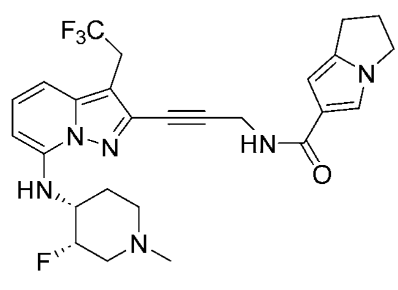
**260** ,

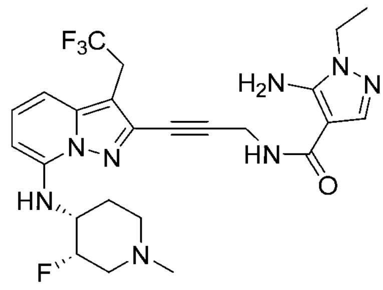
**261** ,

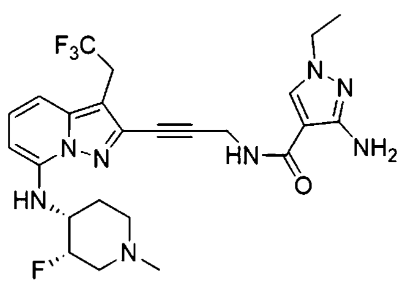
**262** ,

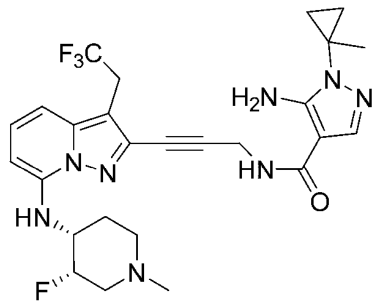
263 ,

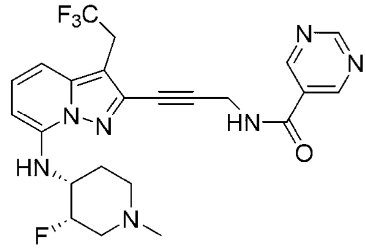
**264** ,

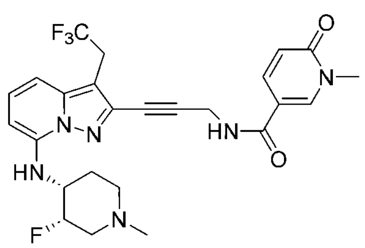
**265** ,

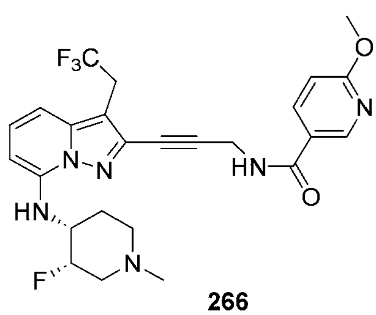
**266** ,

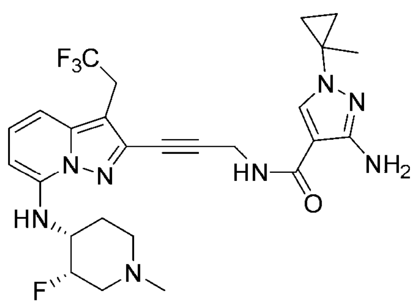
267 ,

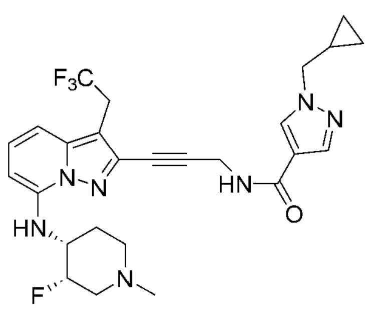
**268** ,

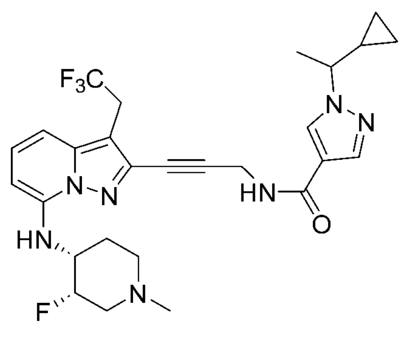
**269** ,

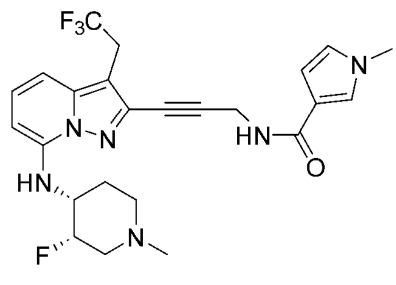
**270** ,

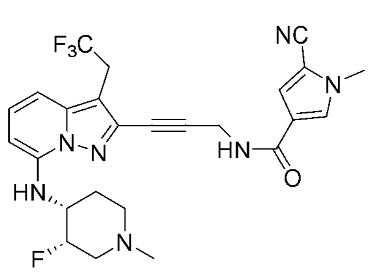
271
,
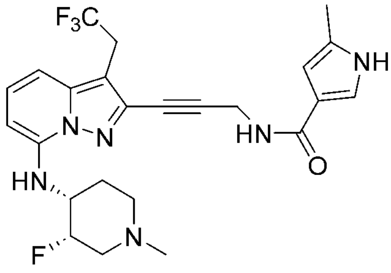
272
,
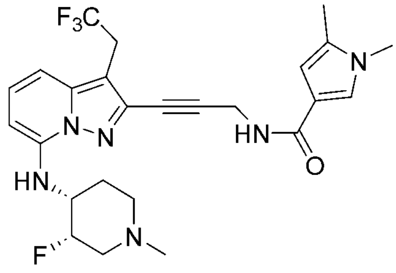
273
,

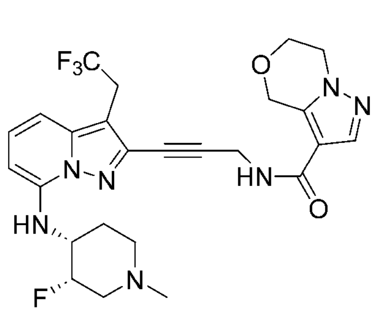
274
,
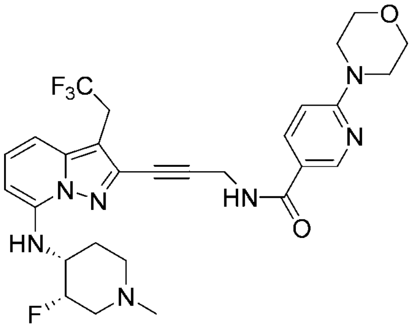
275
,
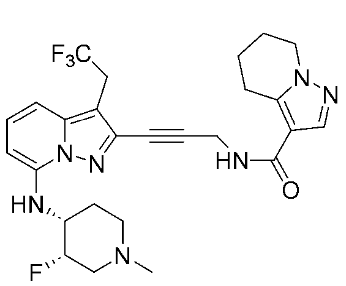
276
,

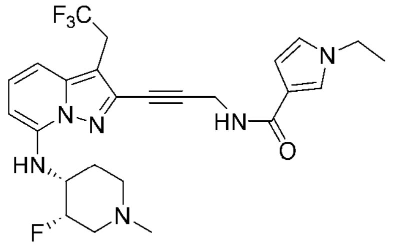
277
,
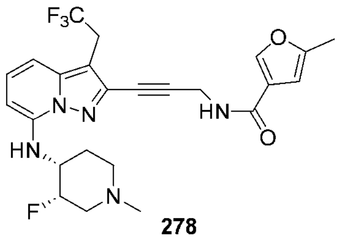
278
,
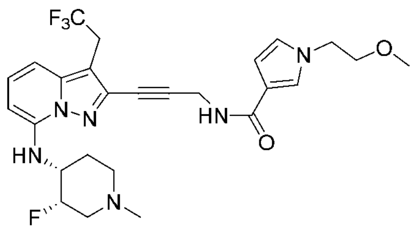
279
,

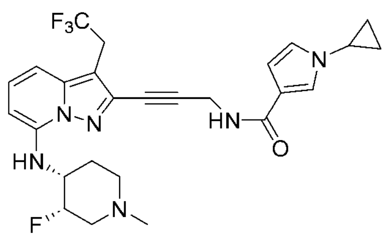
280
,
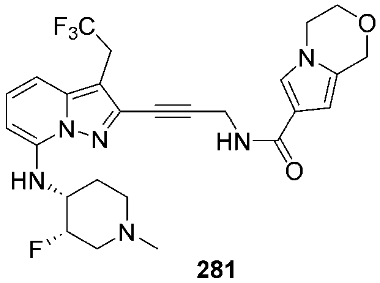
281
,
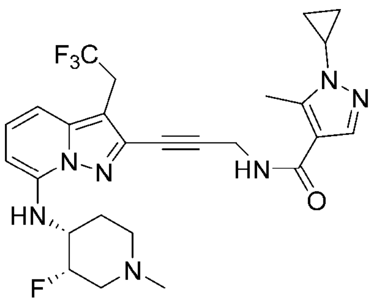
282
,

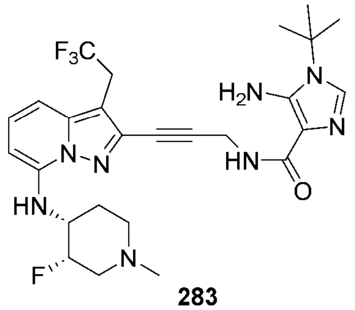
283 ,
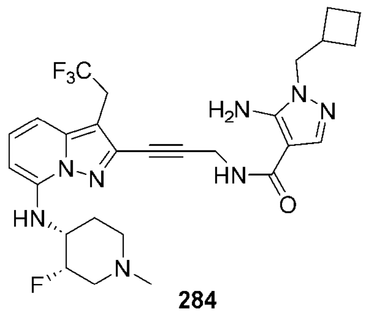
284 ,
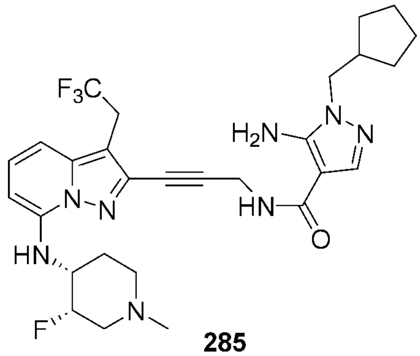
285 ,
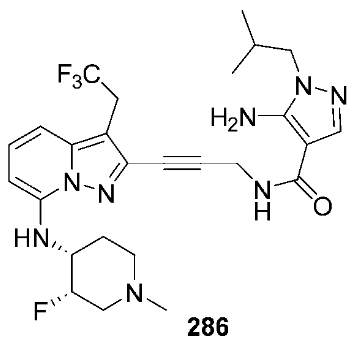
286 ,
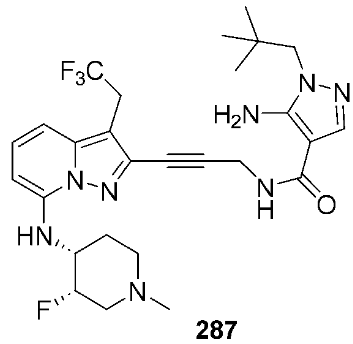
287 ,
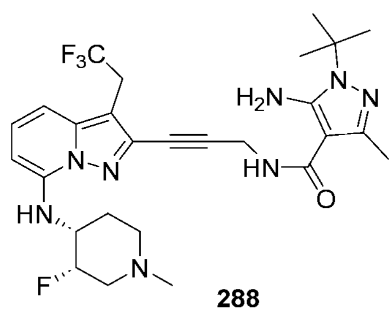
288 ,
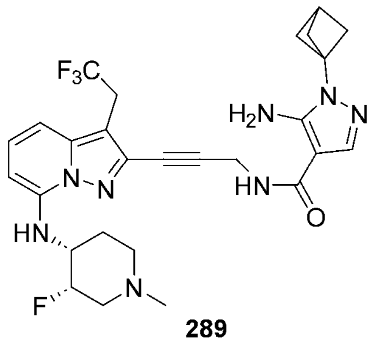
289 ,
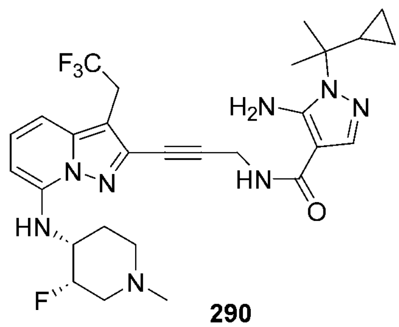
290 ,
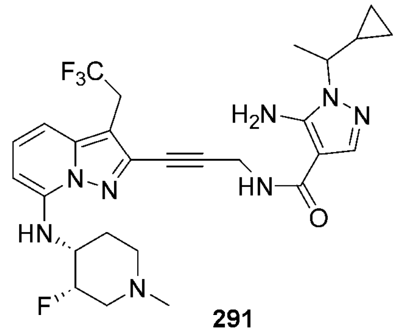
291 ,
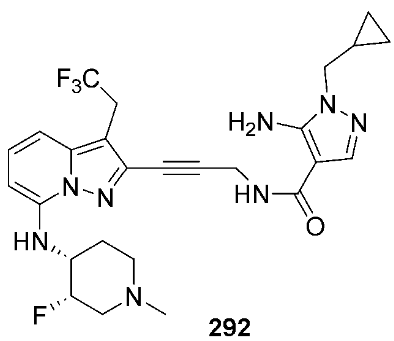
292 ,
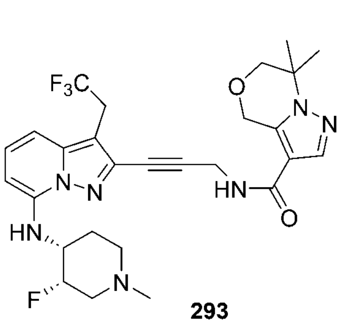
293 ,
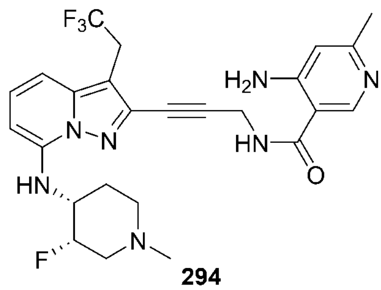
294 ,

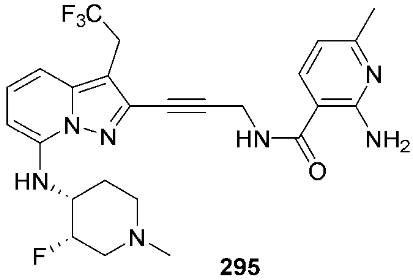
**295** ,
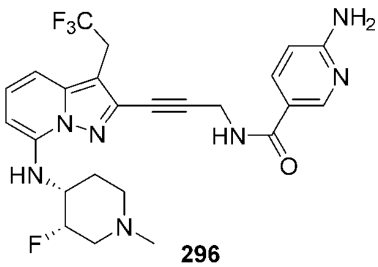
**296** ,
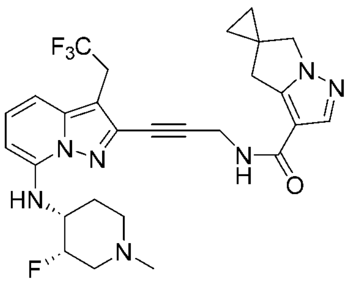
**297** ,

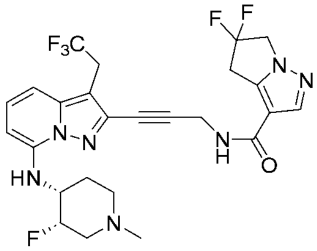
**298** ,
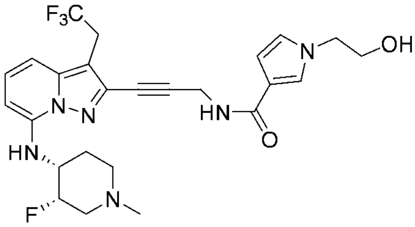
**299** ,
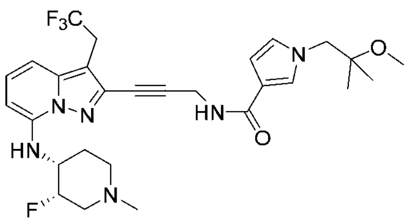
**300** ,

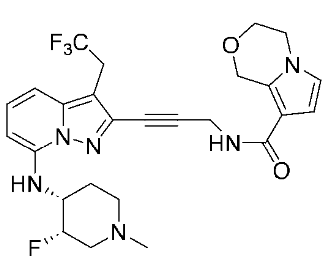
**301** ,
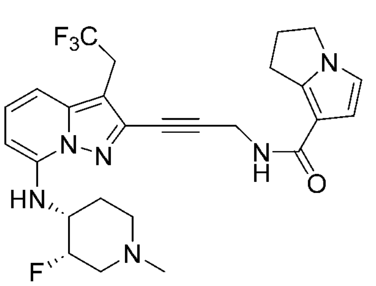
**302** ,
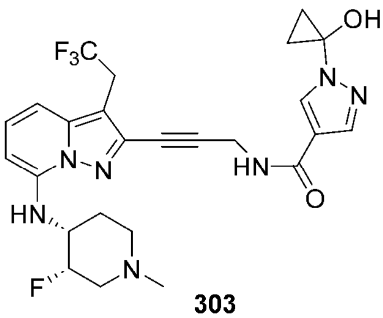
**303** ,

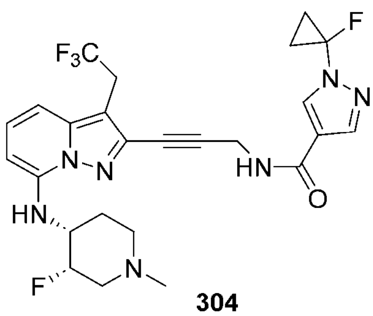
**304** ,
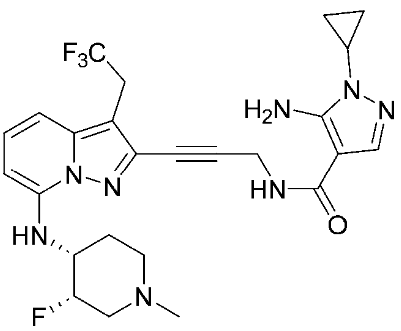
**305** ,
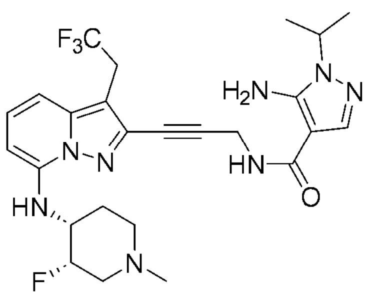
**306** ,

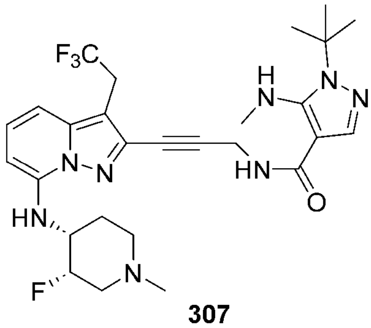
**307** ,

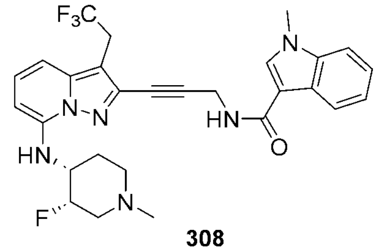
**308** ,

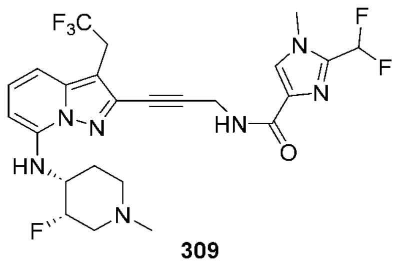
**309** ,

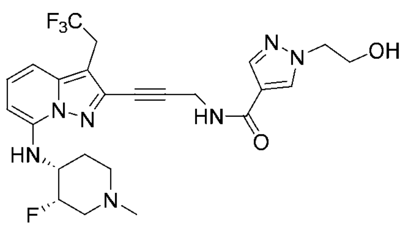
**310** ,

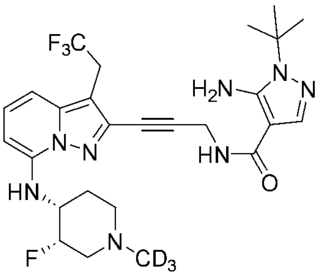
**311** ,

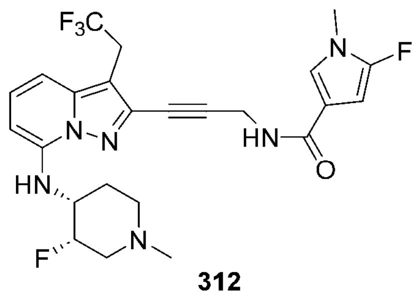
**312** ,

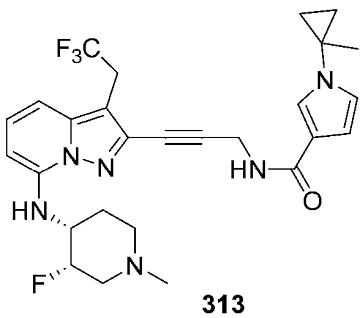
**313** ,

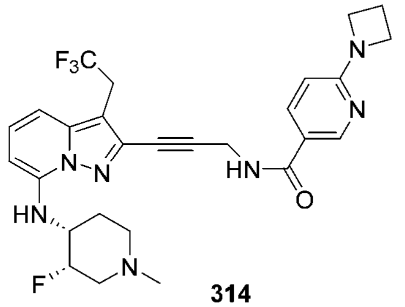
**314** ,

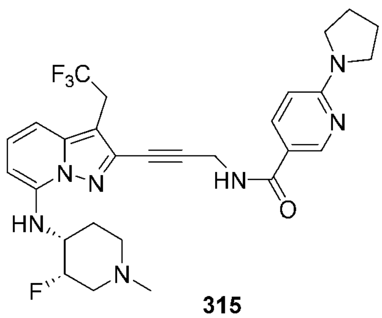
**315** ,

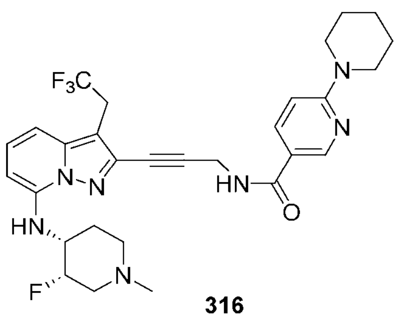
**316** ,

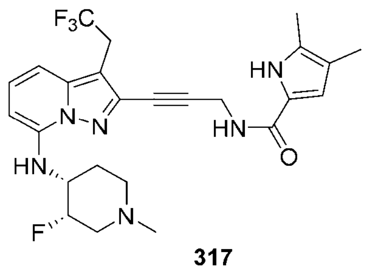
**317** ,

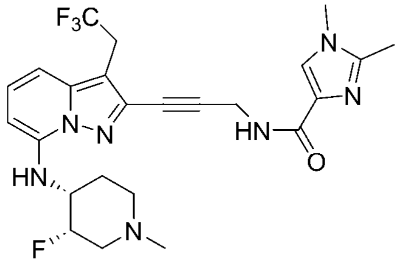
**318** ,

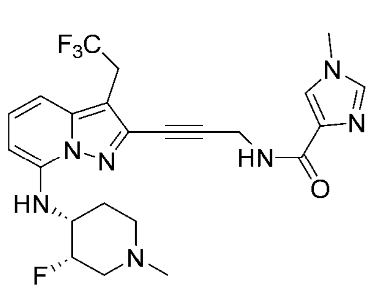

**319** ,

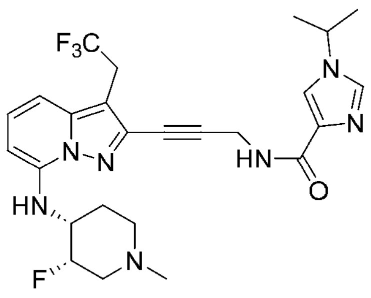

**320** ,

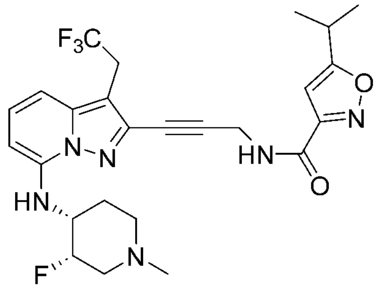

**321** ,

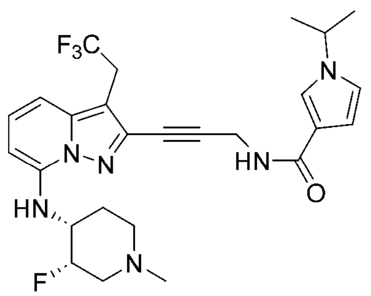

**322** ,

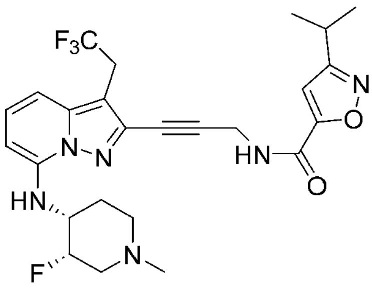

**323** ,

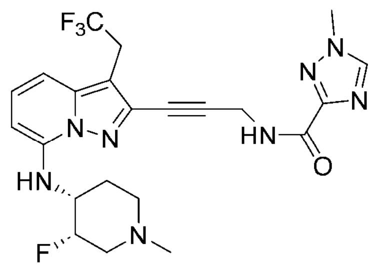

**324** ,

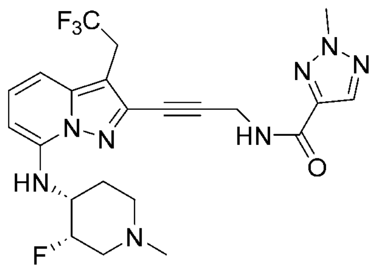

**325** ,

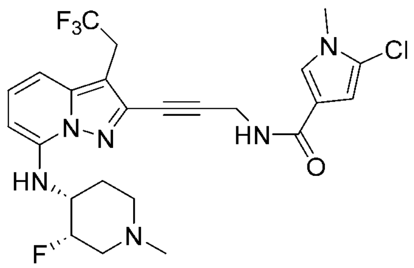

**326** ,

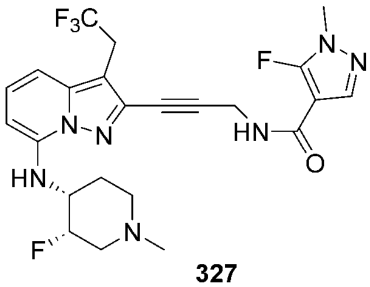

**327** ,

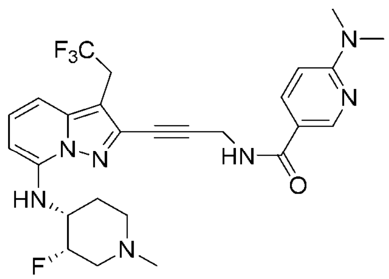

**328** ,

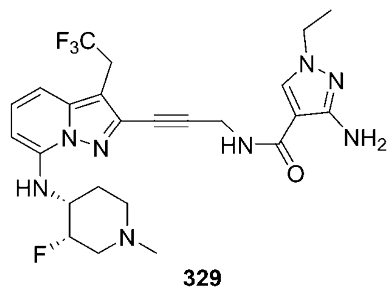

**329** ,

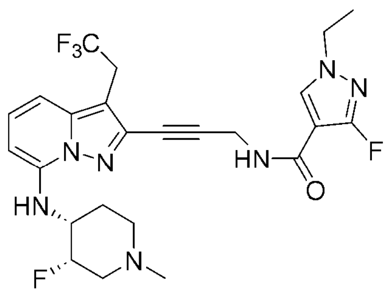

**330** ,

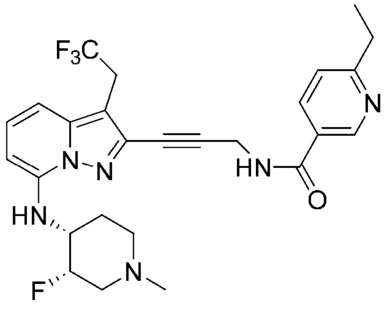
331 ,

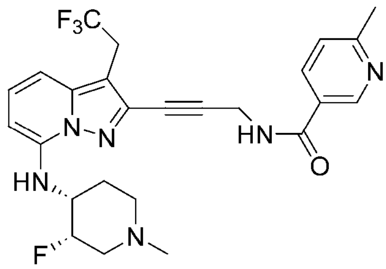
332 ,

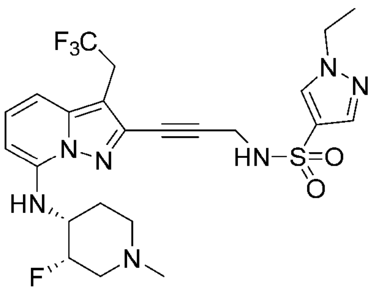
333 ,

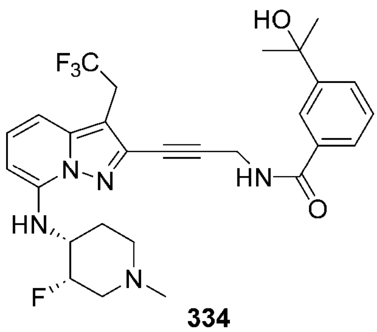
334 ,

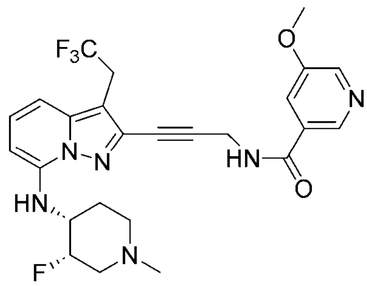
335 ,

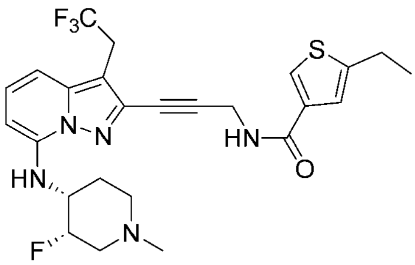
336 ,

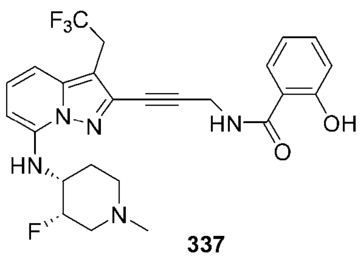
337 ,

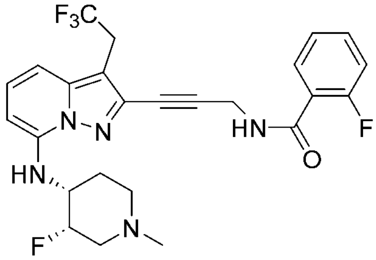
338 ,

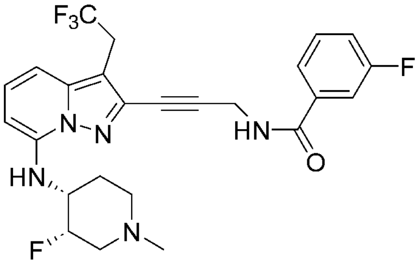
339 ,

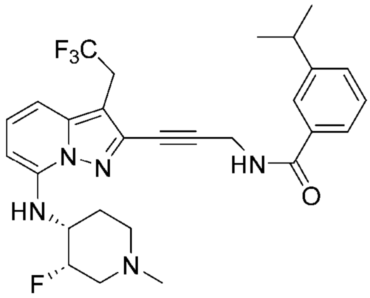
340 ,

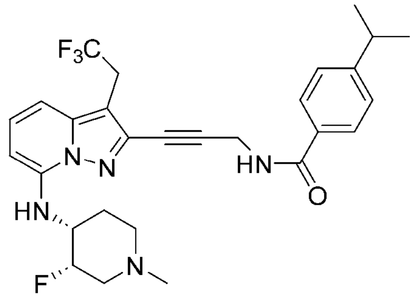
341 ,

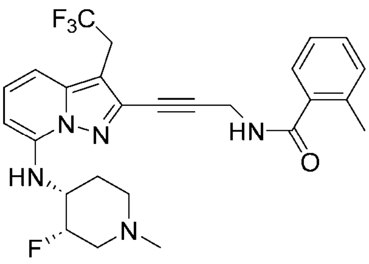
342 ,

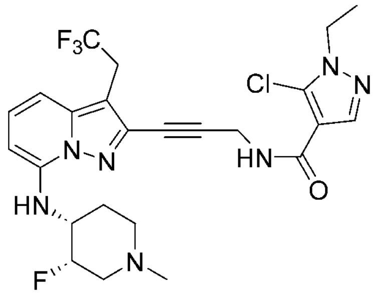
343 ,
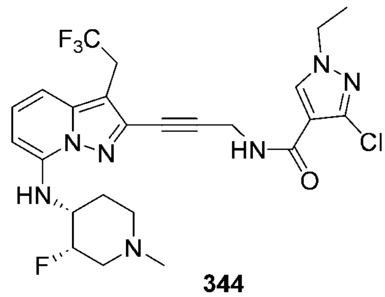
344 ,
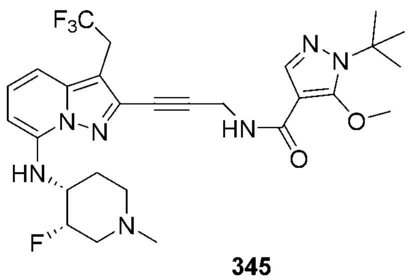
345 ,

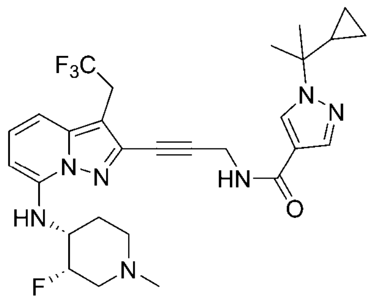
346 ,
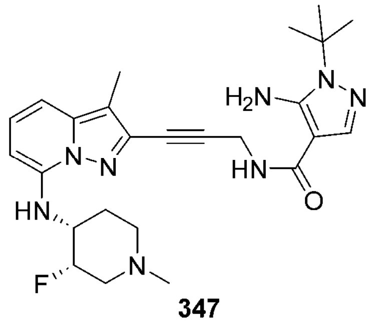
347 ,
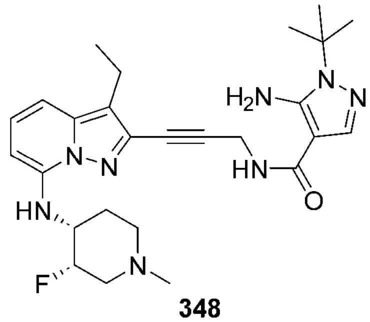
348 ,

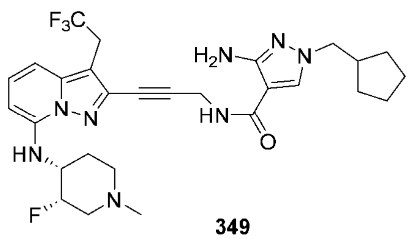
349 ,
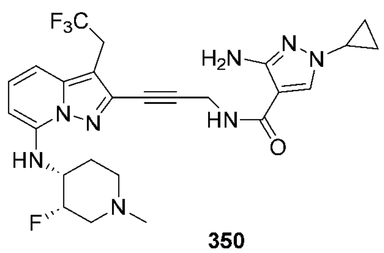
350 ,
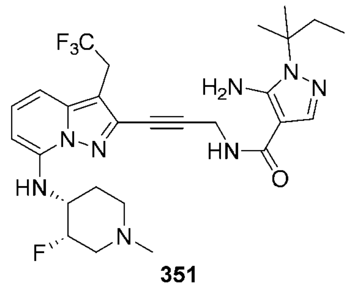
351 ,

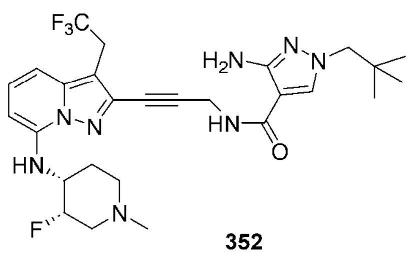
352 ,
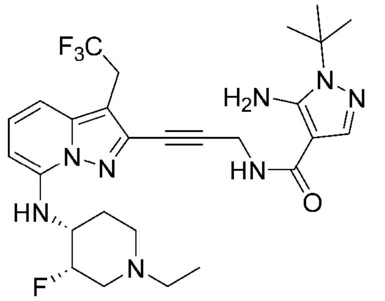
353 ,
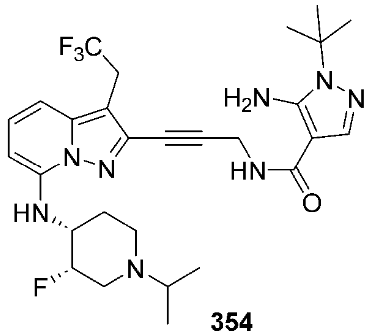
354 ,

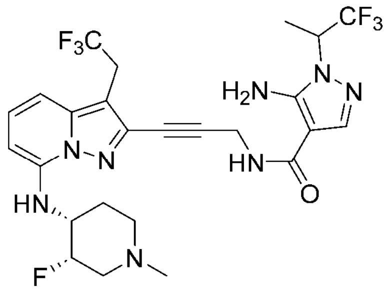

**355** ,

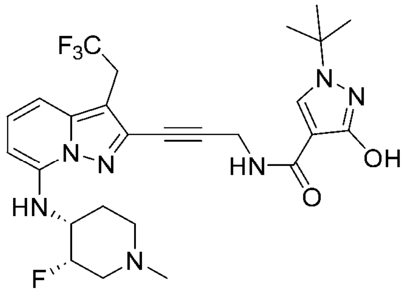

**356** ,

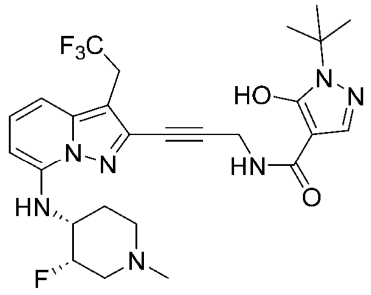

**357** ,

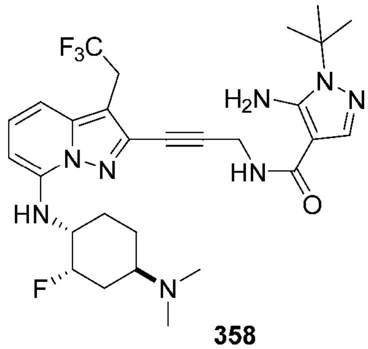

**358** ,

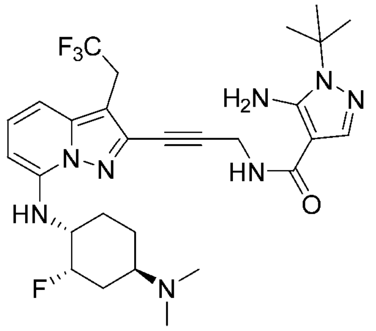

,

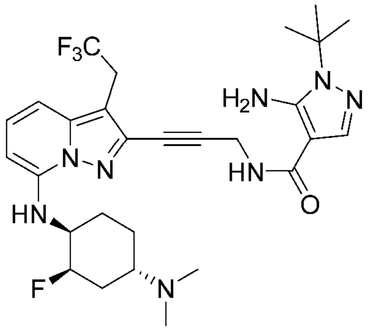

,

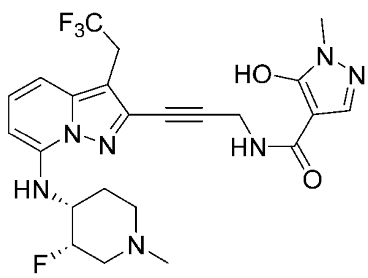

**359** ,

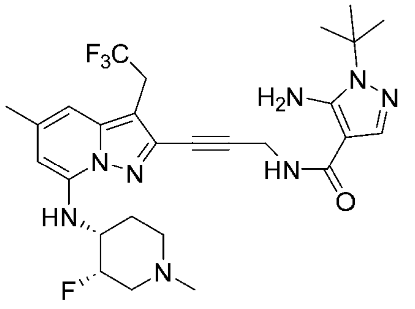

**360** ,

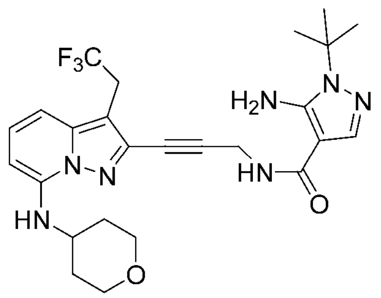

**361** ,

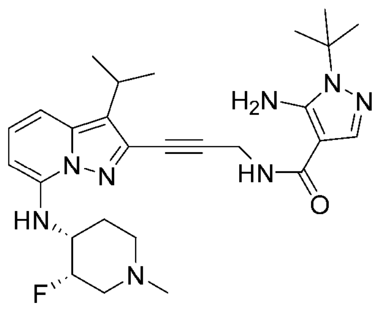

**362** ,

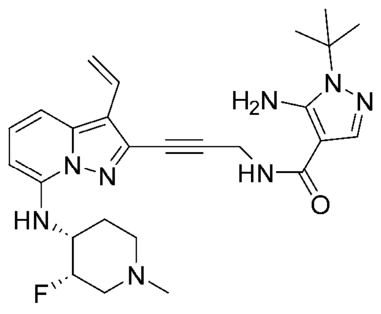

**363** ,

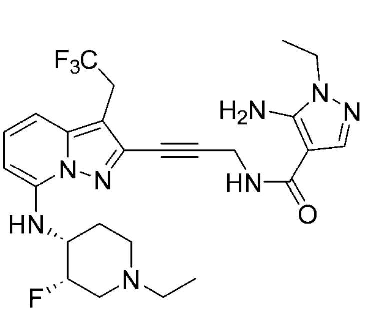

**364** ,

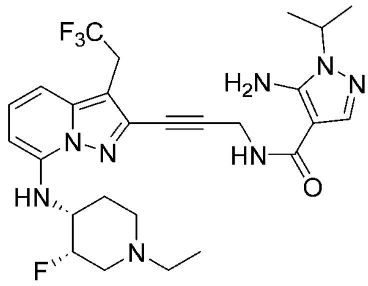

**365** ,

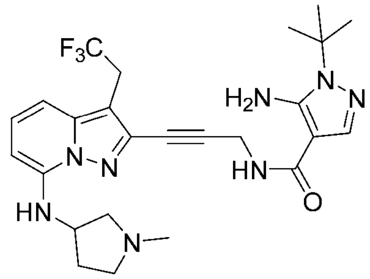

**366** ,

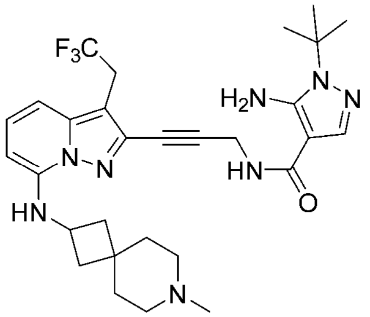

**367** ,

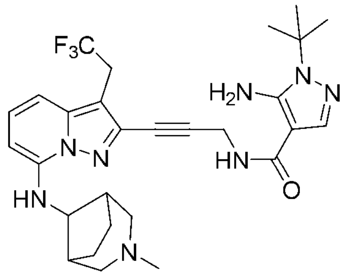

**368** ,

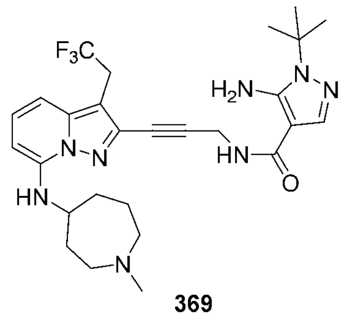

**369** ,

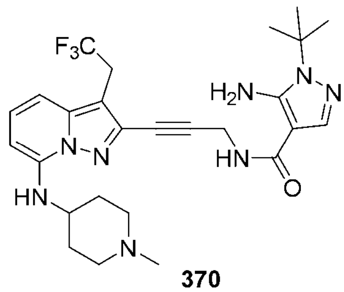

**370** ,

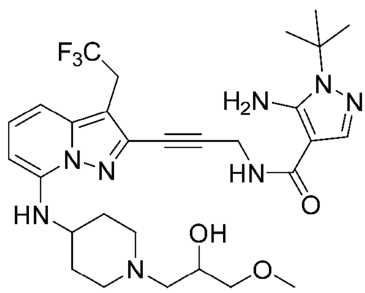

**371** ,

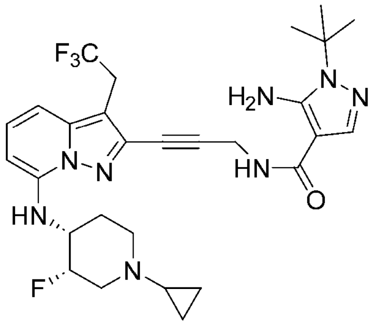

**372** ,

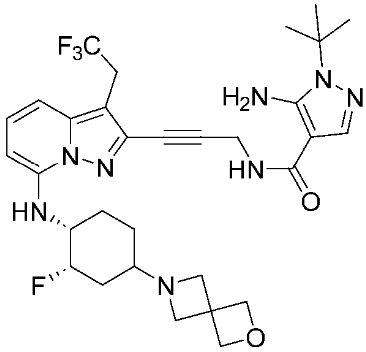

**373** ,

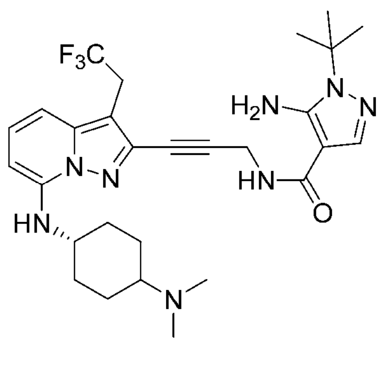

374 ,

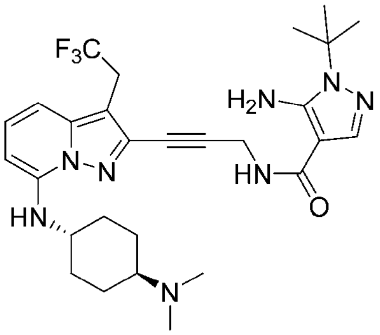

or

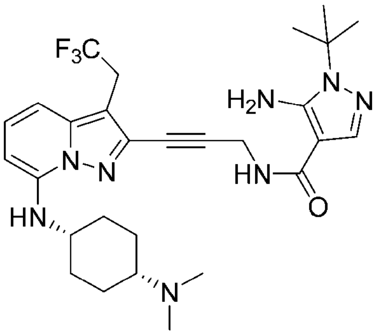

.

**8.** A method for preparing the compound, and the racemate, stereoisomer, tautomer, isotope-labeled counterpart, nitrogen oxide, solvate, polymorph, metabolite, ester, prodrug or pharmaceutically acceptable salt thereof according to any one of claims 1 to 7, comprising the following step: allowing compound 1 to react with compound 2 to give the compound shown as formula (1);

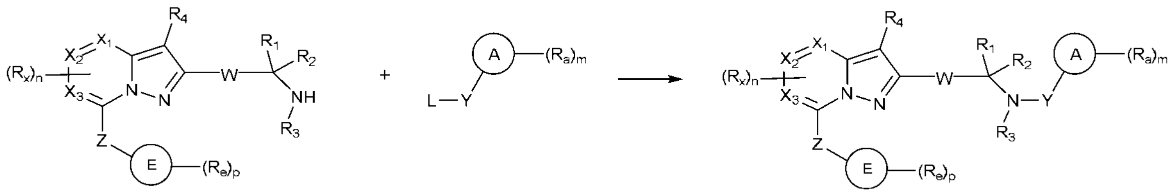

1 2 (I)

wherein ring A, ring E, $X_1$, $X_2$, $X_3$, W, Y, Z, $R_1$, $R_2$, $R_3$, $R_4$, $R_a$, $R_e$, $R_x$, m, n, and p are as defined in any one of claims 1 to 7.

**9.** A pharmaceutical composition, comprising a therapeutically effective amount of at least one of the compound, and the racemate, stereoisomer, tautomer, isotope-labeled counterpart, nitrogen oxide, solvate, polymorph, metabolite, ester, prodrug or pharmaceutically acceptable salt thereof according to any one of claims 1 to 7.

**10.** Use of at least one of the compound, and the racemate, stereoisomer, tautomer, isotope-labeled counterpart, nitrogen oxide, solvate, polymorph, metabolite, ester, prodrug or pharmaceutically acceptable salt thereof according to any one of claims 1 to 7 in the preparation of a drug;

preferably, the use can be use in the preparation of drugs against tumors containing p53-Y220C mutant, such as in the preparation of p53-Y220C reactivator drugs;
preferably, the tumor containing p53-Y220C mutant includes acute lymphocytic leukemia, acute myeloid leukemia, adrenocortical carcinoma, AIDS-related cancer, AIDS-related lymphoma, anal cancer, appendix cancer, astrocytoma, basal cell carcinoma, bile duct cancer, bladder cancer, bone cancer, brain tumors such as cerebellar astrocytoma, cerebral astrocytoma/malignant glioma, ependymoma, medulloblastoma, supratentorial primitive cell tumor, neuroectodermal tumor, visual pathway and hypothalamic glioma, breast cancer, bronchial adenoma, Burkitt's lymphoma, cancer of unknown primary, central nervous system lymphoma, cerebellar astrocytoma, cervical cancer, childhood cancers, chronic lymphocytic leukemia, chronic myelocytic leukemia, chronic myeloproliferative disorders, colon cancer, cutaneous T-cell lymphoma, desmoplastic small round cell tumor, endometrial cancer, ependymoma, esophageal cancer, Ewing's sarcoma, germ cell tumors, gallbladder cancer, gastric cancer, gastrointestinal carcinoid tumors, gastrointestinal stromal tumors, gliomas, hairy cell leukemia, head and neck cancer, heart cancer, hepatocellular carcinoma, Hodgkin lymphoma, hypopharyngeal cancer, intraocular melanoma, islet cell carcinoma, Kaposi's sarcoma, kidney cancer, laryngeal cancer, lip and oral cancer, liposarcoma, liver cancer, lung cancer such as non-small cell lung cancer and small cell lung cancer, lymphoma, leukemia, macroglobulinemia, malignant bone fibrous histiocytoma/osteosarcoma, medulloblastoma, melanoma, mesothelioma, metastatic squamous cell carcinoma with occult primary, oral cancer, multiple endocrine neoplasms syndrome, myelodysplastic syndrome, myeloid leukemia, nasal and paranasal sinus cancer, nasopharyngeal cancer, neuroblastoma, non-Hodgkin lymphoma, non-small cell lung cancer, oral cancer, oropharyngeal cancer, osteosarcoma/malignant fibrous histiocytoma of bone, ovarian cancer, ovarian epithelial cancer, ovarian germ cell tumor, pancreatic cancer, pancreatic islet cell carcinoma, paranasal sinus and nasal cancer, parathyroid cancer, penile cancer, pharyngeal cancer, pheochromocytoma, pineal astrocytoma, pineal germ cell tumor, pituitary adenoma, pleuropulmonary blastoma, plasmacytoma, primary central nervous system lymphoma, prostate cancer, rectal cancer, renal cell carcinoma, transitional cell carcinoma of renal pelvis and ureter, retinoblastoma, rhabdomyosarcoma, salivary gland carcinoma, sarcoma, skin cancer, cutaneous Merkel cell carcinoma, small intestine cancer, soft tissue sarcoma, squamous cell carcinoma, gastric cancer, T-cell lymphoma, laryngeal cancer, thymoma, thymic carcinoma, thyroid cancer, gestational trophoblastic tumor, cancer of unknown primary site, urethral cancer, uterine sarcoma, vaginal cancer, vulvar cancer, Waldenstrom's macroglobulinemia, and nephroblastoma.

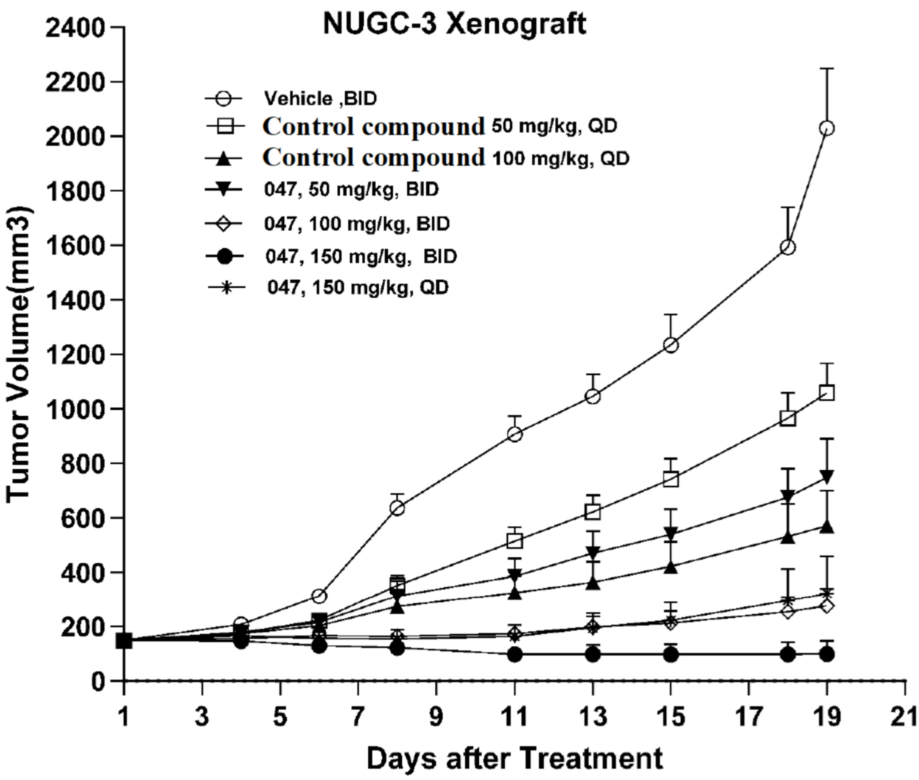
NUGC-3 Xenograft
Vehicle ,BID
Control compound 50 mg/kg, QD
Control compound 100 mg/kg, QD
047, 50 mg/kg, BID
047, 100 mg/kg, BID
047, 150 mg/kg, BID
047, 150 mg/kg, QD
Tumor Volume(mm3)
0
200
400
600
800
1000
1200
1400
1600
1800
2000
2200
2400
1
3
5
7
9
11
13
15
17
19
21
Days after Treatment

FIG. 1

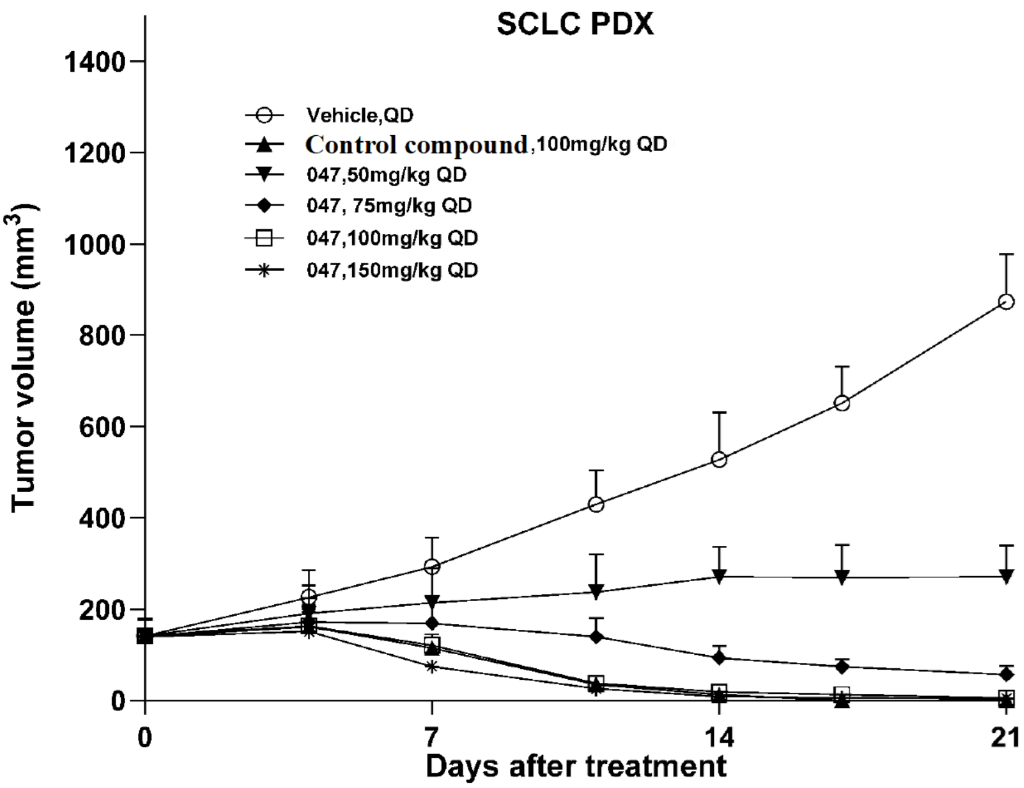
SCLC PDX
Vehicle,QD
Control compound,100mg/kg QD
047,50mg/kg QD
047, 75mg/kg QD
047,100mg/kg QD
047,150mg/kg QD
Tumor volume (mm3)
0
200
400
600
800
1000
1200
1400
0
7
14
21
Days after treatment

FIG. 2

## INTERNATIONAL SEARCH REPORT

International application No.
**PCT/CN2024/101613**

**A. CLASSIFICATION OF SUBJECT MATTER**

C07D 401/12(2006.01)i; C07D 471/04(2006.01)i; A61K 31/454(2006.01)i; A61K 31/4545(2006.01)i; A61P 35/00(2006.01)i; A61P 35/02(2006.01)i

According to International Patent Classification (IPC) or to both national classification and IPC

**B. FIELDS SEARCHED**

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

WPABS; CNABS; CNKI; CJFD; CNTXT; WOTXT; 超星读秀, DUXIU; 万方, WANFANG; STN; USTXT; EPTXT: 长春金赛药业有限责任公司, 耿开骏, 陆标, 杨方龙, 王思勤, 金磊, p53, Y220C, 肿瘤, 癌症, cancer, tumor, 式III结构式, 式IV结构式, 式IV-1结构式

**C. DOCUMENTS CONSIDERED TO BE RELEVANT**

| Category* | Citation of document, with indication, where appropriate, of the relevant passages | Relevant to claim No. |
|---|---|---|
| PX | CN 117986235 A (CHANGCHUN GENESCIENCE PHARMACEUTICAL CO., LTD.) 07 May 2024 (2024-05-07)<br>claims 1-10 | 1-10 |
| A | WO 2022213975 A1 (JACOBIO PHARMACEUTICALS CO., LTD.) 13 October 2022 (2022-10-13)<br>claims 1-23 | 1-10 |
| A | US 2023049952 A1 (PMV PHARMACEUTICALS INC.) 16 February 2023 (2023-02-16)<br>entire document | 1-10 |
| A | CN 116096704 A (PMV PHARMACEUTICALS INC.) 09 May 2023 (2023-05-09)<br>entire document | 1-10 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 July 2024** | **17 September 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

## INTERNATIONAL SEARCH REPORT
**Information on patent family members**

International application No.
**PCT/CN2024/101613**

| Patent document cited in search report | Publication date (day/month/year) | Patent family member(s) | Publication date (day/month/year) |
|---|---|---|---|
| CN 117986235 A | 07 May 2024 | None | |
| WO 2022213975 A1 | 13 October 2022 | US 2023049952 A1 | 16 February 2023 |
| US 2023049952 A1 | 16 February 2023 | WO 2021262483 A1 | 30 December 2021 |
| CN 116096704 A | 09 May 2023 | KR 20230028484 A | 28 February 2023 |
| | | AU 2021297716 A1 | 19 January 2023 |
| | | MX 2022015793 A | 27 February 2023 |
| | | IL 298592 A | 01 January 2023 |
| | | US 2023033324 A1 | 02 February 2023 |
| | | US 11938124 B2 | 26 March 2024 |
| | | EP 4171548 A1 | 03 May 2023 |
| | | EP 4171548 A4 | 19 June 2024 |
| | | CA 3183081 A1 | 30 December 2021 |
| | | JP 2023533447 A | 03 August 2023 |
| | | WO 2021262484 A1 | 30 December 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310767412
- CN 202310972386
- CN 202311158861
- CN 202311378644X
- CN 202311566589
- CN 202410171815X